# EUROPEAN PATENT SPECIFICATION

(11) **EP 4 077 312 B1**
(45) Date of publication and mention of the grant of the patent: **11.11.2026**
(21) Application number: 20902289.6
(22) Date of filing: 17.12.2020
(51) Int. Cl.: C07D 207/08, C07D 487/10, A61K 31/407, A61P 35/00, A61P 3/10

(54) **SUBSTITUTED STRAIGHT CHAIN SPIRO DERIVATIVES**
SUBSTITUIERTE GERADKETTIGE SPIRODERIVATE
DÉRIVÉS SPIRO À CHAÎNE DROITE SUBSTITUÉS

(30) Priority: 19.12.2019 WO PCT/CN2019/126760; 16.01.2020 US 202062961775 P; 04.11.2020 WO PCT/CN2020/126595
(43) Date of publication of application: 26.10.2022
(73) Proprietor: Janssen Pharmaceutica NV, 2340 Beerse (BE)
(72) Inventor: CAI, Wei, Shanghai 200233 (CN); DAI, Xuedong, Shanghai 200233 (CN); QUEROLLE, Olivier Alexis Georges, 92787 Issy-les Moulineaux Cedex 9 (FR); THURING, Johannes Wilhelmus John F, 2340 Beerse (BE); LIU, Yingtao, Shanghai 200233 (CN); LIU, Lianzhu, Shanghai 200233 (CN); XU, Yanping, Shanghai 200233 (CN); FU, Liqiang, Shanghai 200233 (CN); LI, Ming, Shanghai 200233 (CN); FANG, Lichao, Shanghai 200233 (CN); DENG, Xiangjun, Shanghai 200233 (CN); ZHAO, Qiwu, Shanghai 200233 (CN); LI, Kangying, Shanghai 200233 (CN); NG, Alicia Tee Fuay, Shanghai 200233 (CN); DARVILLE, Nicolas Freddy J, 2340 Beerse (BE); CLEATOR, Edward, Wycombe Buckinghamshire HP12 4EG (GB); URBANIETZ, Gregor Thomas, 2340 Beerse (BE); MATON, William, Marc, 2340 Beerse (BE); PANDE, Vineet, 2340 Beerse (BE)
(74) Representative: Lenaerts, Philip
(86) International application number: PCT/CN2020/137266
(87) International publication number: WO 2021/121327

(56) References cited:
- WO-A1-2016/000581
- WO-A1-2016/042452
- WO-A1-2017/214367
- WO-A1-2017/214367
- WO-A1-2018/053267
- WO-A1-2018/175746
- WO-A1-2018/226976
- WO-A1-2019/060365
- WO-A1-2020/069027
- ORR, S.T.M. ET AL.: "One-pot synthesis of chiral azetidines from chloroaldehyde and chiral amines", TETRAHEDRON LETTERS, vol. 52, 14 May 2011 (2011-05-14), pages 3618 - 3620, XP055073241, DOI: 10.1016/j.tetlet.2011.05.027
- ORR, S.T.M. ET AL.: "One-pot synthesis of chiral azetidines from chloroaldehyde and chiral amines", TETRAHEDRON LETTERS, vol. 52, 14 May 2011 (2011-05-14), pages 3618 - 3620, XP055073241, DOI: 10.1016/j.tetlet.2011.05.027
- KRIVTSOV, A.V. ET AL.: "A Menin-MLL Inhibitor Induces Specific Chromatin Changes and Eradicates Disease in Models of MLL-Rearranged Leukemia", CANCER CELL, vol. 36, 9 December 2019 (2019-12-09), pages 660 - 673, XP085945648

## Description

### FIELD OF THE INVENTION

The present disclosure relates to pharmaceutical agents useful for therapy and/or prophylaxis in a mammal, pharmaceutical composition comprising such compounds, and their use as menin/MLL protein/protein interaction inhibitors, useful for treating diseases such as cancer, including but not limited to leukemia, myelodysplastic syndrome (MDS), and myeloproliferative neoplasms (MPN); and diabetes.

The claimed invention is set out in the appended claim set. The (present) disclosure contains the claimed invention as well as other embodiments for illustrative purposes.

### BACKGROUND OF THE INVENTION

Chromosomal rearrangements affecting the mixed lineage leukemia gene (*MLL*; *MLL1*; *KMT2A*) result in aggressive acute leukemias across all age groups and still represent mostly incurable diseases emphasizing the urgent need for novel therapeutic approaches. Acute leukemias harboring these chromosomal translocations of *MLL* represent as lymphoid, myeloid or biphenotypic disease and constitute 5 to 10% of acute leukemias in adults and approximately 70% in infants (Marschalek, Br J Haematol 2011. 152(2), 141-54; Tomizawa et al., Pediatr Blood Cancer 2007. 49(2), 127-32).

MLL is a histone methyltransferase that methylates histone H3 on lysine 4 (H3K4) and functions in multiprotein complexes. Use of inducible loss-of-function alleles of *Mll1* demonstrated that Mll1 plays an essential role in sustaining hematopoietic stem cells (HSCs) and developing B cells although its histone methyltransferase activity is dispensable for hematopoiesis (Mishra et al., Cell Rep 2014. 7(4), 1239-47).

Fusion of MLL with more than 60 different partners has been reported to date and has been associated with leukemia formation/progression (Meyer et al., Leukemia 2013. 27, 2165-2176). Interestingly, the SET (Su(var)3-9, enhancer of zeste, and trithorax) domain of MLL is not retained in chimeric proteins but is replaced by the fusion partner (Thiel et al., Bioessays 2012. 34, 771-80). Recruitment of chromatin modifying enzymes like DotIL and/or the pTEFb complex by the fusion partner leads to enhanced transcription and transcriptional elongation of MLL target genes including *HOXA* genes (e.g. *HOXA9*) and the *HOX* cofactor *MEIS1* as the most prominent ones. Aberrant expression of these genes in turn blocks hematopoietic differentiation and enhances proliferation.

Menin which is encoded by the Multiple Endocrine Neoplasia type 1 (*MEN1*) gene is expressed ubiquitously and is predominantly localized in the nucleus. It has been shown to interact with numerous proteins and is, therefore, involved in a variety of cellular processes. The best understood function of menin is its role as an oncogenic cofactor of MLL fusion proteins. Menin interacts with two motifs within the N-terminal fragment of MLL that is retained in all fusion proteins, MBM1 (menin-binding motif 1) and MBM2 (Thiel et al., Bioessays 2012. 34, 771-80). Menin/MLL interaction leads to the formation of a new interaction surface for lens epithelium-derived growth factor (LEDGF). Although MLL directly binds to LEDGF, menin is obligatory for the stable interaction between MLL and LEDGF and the gene specific chromatin recruitment of the MLL complex via the PWWP domain of LEDGF (Cermakova et al., Cancer Res 2014. 15, 5139-51; Yokoyama & Cleary, Cancer Cell 2008. 8, 36-46). Furthermore, numerous genetic studies have shown that menin is strictly required for oncogenic transformation by MLL fusion proteins suggesting the menin/MLL interaction as an attractive therapeutic target. For example, conditional deletion of *Men1* prevents leukomogenesis in bone marrow progenitor cells ectopically expressing MLL fusions (Chen et al., Proc Natl Acad Sci 2006. 103, 1018-23). Similarly, genetic disruption of menin/MLL fusion interaction by loss-of-function mutations abrogates the oncogenic properties of the MLL fusion proteins, blocks the development of leukemia *in vivo* and releases the differentiation block of MLL-transformed leukemic blasts. These studies also showed that menin is required for the maintenance of *HOX* gene expression by MLL fusion proteins (Yokoyama et al., Cell 2005. 123, 207-18). In addition, small molecule inhibitors of menin/MLL interaction have been developed suggesting druggability of this protein/protein interaction and have also demonstrated efficacy in preclinical models of AML (Borkin et al., Cancer Cell 2015. 27, 589-602; Cierpicki and Grembecka, Future Med Chem 2014. 6, 447-462). Together with the observation that menin is not a requisite cofactor of MLL1 during normal hematopoiesis (Li et al., Blood 2013. 122, 2039-2046), these data validate the disruption of menin/MLL interaction as a promising new therapeutic approach for the treatment of MLL rearranged leukemia and other cancers with an active *HOX*/*MEIS1* gene signature. For example, an internal partial tandem duplication (PTD) within the 5'region of the *MLL* gene represents another major aberration that is found predominantly in de novo and secondary AML as well as myeloid dysplasia syndromes. Although the molecular mechanism and the biological function of MLL-PTD is not well understood, new therapeutic targeting strategies affecting the menin/MLL interaction might also prove effective in the treatment of MLL-PTD-related leukemias. Furthermore, castrationresistant prostate cancer has been shown to be dependent on the menin/MLL interaction (Malik et al., Nat Med 2015. 21, 344-52).

MLL protein is also known as Histone-lysine N-methyltransferase 2A (KMT2A) protein in the scientific field (UniProt Accession # Q03164).

Several references describe inhibitors targeting the menin-MLL interaction: WO2011029054, J Med Chem 2016, 59, 892-913 describe the preparation of thienopyrimidine and benzodiazepine derivatives; WO2014164543 describes thienopyrimidine and thienopyridine derivatives; Nature Chemical Biology March 2012, 8, 277-284 and Ren, J.; et al. Bioorg Med Chem Lett (2016), 26(18), 4472-4476 describe thienopyrimidine derivatives; J Med Chem 2014, 57, 1543-1556 describes hydroxy- and aminomethylpiperidine derivatives; Future Med Chem 2014, 6, 447-462 reviews small molecule and peptidomimetic compounds; WO2016195776 describes furo[2,3-d]pyrimidine, 9H-purine, [1,3]oxazolo[5,4-d]pyrimidine, [1,3]oxazolo[4,5-d]pyrimidine, [1,3]thiazolo[5,4-d]pyrimidine, thieno[2,3-b]pyridine and thieno[2,3-d]pyrimidine derivatives; WO2016197027 describes 5,6,7,8-tetrahydropyrido[3,4-d]pyrimidine, 5,6,7,8-tetrahydropyrido]4,3-d]pyrimidine, pyrido[2,3-d]pyrimidine and quinoline derivatives; and WO2016040330 describes thienopyrimidine and thienopyridine compounds. WO2017192543 describes piperidines as Menin inhibitors. WO2017112768, WO2017207387, WO2017214367, WO2018053267 and WO2018024602 describe inhibitors of the menin-NELL interaction. WO2017161002 and WO2017161028 describe inhibitors of menin-MLL. WO2018050686, WO2018050684 and WO2018109088 describe inhibitors of the menin-MLL interaction. WO2018226976 describes methods and compositions for inhibiting the interaction of menin with MLL proteins. WO2018175746 provides methods of treatment for hematological malignancies and Ewing's sarcoma. WO2018106818 and WO2018106820 provide methods of promoting proliferation of a pancreatic cell. WO2018153312 discloses azaspiro compounds relating to the field of medicinal chemistry. WO2017132398 discloses methods comprising contacting a leukemia cell exhibiting an NPM1 mutation with a pharmacologic inhibitor of interaction between MLL and Menin. WO2019060365 describes substituted inhibitors of menin-MLL. WO2020069027 describes the treatment of hematological malignancies with inhibitors of menin. Krivtsov et al., Cancer Cell 2019. No.6 Vol.36, 660-673 describes a menin-MLL inhibitor. Orr et al., Tetrahedron Letters 2011. Vol.52, 3618-3620 describes the synthesis of chiral azetidines.

### BRIEF DESCRIPTION OF THE DRAWINGS

Fig. 1: Efficacy study in Molm-14 subcutaneous (sc) model.
Fig. 2: Efficacy study in disseminated OCI-AML3 model.

### DESCRIPTION OF THE INVENTION

The claimed invention is set out in the appended claim set. Other embodiments described herein are present for illustrative purposes.

The present disclosure concerns novel compounds of Formula (I), 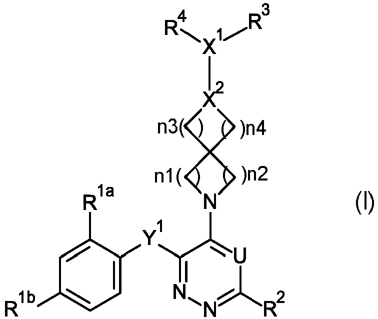 and the tautomers and the stereoisomeric forms thereof, wherein
R^{1a} represents -C(=O)-NR^{xa}R^{xb}; Het; or 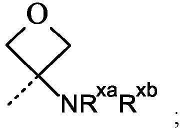
Het represents a 5- or 6-membered monocyclic aromatic ring containing one, two or three nitrogen atoms and optionally a carbonyl moiety;
wherein said 5- or 6-membered monocyclic aromatic ring is optionally substituted with one or two substituents selected from the group consisting of C₃₋₆cycloalkyl and C₁₋₄alkyl;
R^{xa} and R^{xb} are each independently selected from the group consisting of hydrogen, C₁₋₄alkyl and C₃₋₆cycloalkyl;
R^{1b} represents F or Cl;
Y¹ represents -CR^{5a}R^{5b}-, -O- or *-*NR^{5c}*-;*
R² is selected from the group consisting of hydrogen, halo, C₁₋₄alkyl, -O-C₁₋₄alkyl, and - NR^{7a}R^{7b};
U represents N or CH;
n1, n2, n3 and n4 are each independently selected from 1 and 2;
X¹ represents CH, and X² represents N;
R⁴ represents isopropyl;
R^{5a}, R^{5b}, R^{5c}, R^{7a}, and R^{7b}, are each independently selected from the group consisting of hydrogen, C₁₋₄alkyl and C₃₋₆cycloalkyl;
R³ represents -C₁₋₆alkyl-NR^{8a}R^{8b}, -C₁₋₆alkyl-C(=O)-NR^{9a}R^{9b}, -C₁₋₆alkyl-OH, or -C₁₋₆alkyl-NR¹¹-C(=O)-O-C₁₋₄alkyl-O-C(=O)-C₁₋₄alkyl;
wherein each of the C₁₋₄alkyl or C₁₋₆alkyl moieties in the R³ definitions independently of each other may be substituted with one, two or three substituents each independently selected from the group consisting of cyano, halo, -OH, and -O-C₁₋₄alkyl;
R^{8a} and R^{8b} are each independently selected from the group consisting of hydrogen; C₁₋₆alkyl; -C(=O)-C₁₋₄alkyl; -C(=O)-O-C₁₋₄alkyl; -C(=O)-NR^{12a}R^{12b}; and C₁₋₆alkyl substituted with one, two or three substituents each independently selected from the group consisting of - OH, cyano, halo, -S(=O)₂-C₁₋₄alkyl, -O-C₁₋₄alkyl, -C(=O)-NR^{10a}R^{10b}, and -NR^{10c}-C(=O)-C₁₋₄alkyl;
R^{9a}, R^{9b}, R^{10a}, R^{10b}, R^{10c}, R¹¹, R^{12a}, and R^{12b} are each independently selected from the group consisting of hydrogen and C₁₋₆alkyl;
and the pharmaceutically acceptable salts and the solvates thereof.

The present disclosure also relates to a pharmaceutical composition comprising a therapeutically effective amount of a compound of Formula (I), a pharmaceutically acceptable salt, or a solvate thereof, and a pharmaceutically acceptable carrier or excipient.

Additionally, the disclosure relates to a compound of Formula (I), a pharmaceutically acceptable salt, or a solvate thereof, for use as a medicament, and to a compound of Formula (I), a pharmaceutically acceptable salt, or a solvate thereof, for use in the treatment or in the prevention of cancer, including but not limited to leukemia, myelodysplastic syndrome (MDS), and myeloproliferative neoplasms (MPN); and diabetes.

In a particular embodiment, the disclosure relates to a compound of Formula (I), a pharmaceutically acceptable salt, or a solvate thereof, for use in the treatment or in the prevention of cancer.

In a specific embodiment said cancer is selected from leukemias, lymphomas, myelomas or solid tumor cancers (e.g. prostate cancer, lung cancer, breast cancer, pancreatic cancer, colon cancer, liver cancer, melanoma and glioblastoma, etc.). In some embodiments, the leukemias include acute leukemias, chronic leukemias, myeloid leukemias, myelogenous leukemias, lymphoblastic leukemias, lymphocytic leukemias, Acute myelogenous leukemias (AML), Chronic myelogenous leukemias (CML), Acute lymphoblastic leukemias (ALL), Chronic lymphocytic leukemias (CLL), T cell prolymphocytic leukemias (T-PLL), Large granular lymphocytic leukemia, Hairy cell leukemia (HCL), MLL-rearranged leukemias, MLL-PTD leukemias, MLL amplified leukemias, MLL-positive leukemias, leukemias exhibiting *HOX*/*MEIS1* gene expression signatures etc.

In particular, compounds according to the present disclosure and the pharmaceutical compositions thereof may be useful in the treatment or prevention of leukemias, in particular nucleophosmin (NPM1)-mutated leukemias, e.g. NPM1c.

In an embodiment, compounds of Formula (I) and the pharmaceutically acceptable salts, and the solvates thereof, may have improved metabolic stability properties.

In an embodiment, compounds of Formula (I) and the pharmaceutically acceptable salts, and the solvates thereof, may have extended in vivo half-life (T1/2).

In an embodiment, compounds of Formula (I) and the pharmaceutically acceptable salts, and the solvates thereof, may have improved oral bioavailability.

In an embodiment, compounds of Formula (I) and the pharmaceutically acceptable salts, and the solvates thereof, may reduce tumor growth e.g., tumours harbouring MLL (KMT2A) gene rearrangements/alterations and/or NPM1 mutations.

In an embodiment, compounds of Formula (I) and the pharmaceutically acceptable salts, and the solvates thereof, may have improved PD properties *in vivo* during a prolonged period of time, e.g. inhibition of target gene expression such as MEIS1 and upregulation of differentiation marker over a period of at least 16 hours.

In an embodiment, compounds of Formula (I) and the pharmaceutically acceptable salts, and the solvates thereof, may have an improved safety profile (e.g. reduced hERG inhibition; improved cardiovascular safety).

In an embodiment, compounds of Formula (I) and the pharmaceutically acceptable salts, and the solvates thereof, may be suitable for Q.D. dosing (once daily).

The disclosure also relates to the use of a compound of Formula (I), a pharmaceutically acceptable salt, or a solvate thereof, in combination with an additional pharmaceutical agent for use in the treatment or prevention of cancer, including but not limited to leukemia, myelodysplastic syndrome (MDS), and myeloproliferative neoplasms (MPN); and diabetes.

Furthermore, the disclosure relates to a process for preparing a pharmaceutical composition according to the disclosure, characterized in that a pharmaceutically acceptable carrier is intimately mixed with a therapeutically effective amount of a compound of Formula (I), a pharmaceutically acceptable salt, or a solvate thereof.

The disclosure also relates to a product comprising a compound of Formula (I), a pharmaceutically acceptable salt, or a solvate thereof, and an additional pharmaceutical agent, as a combined preparation for simultaneous, separate or sequential use in the treatment or prevention of cancer, including but not limited to leukemia, myelodysplastic syndrome (MDS), and myeloproliferative neoplasms (MPN); and diabetes .

Additionally, the disclosure relates to a compound of Formula (I), a pharmaceutically acceptable salt, or a solvate thereof, as defined herein, or a pharmaceutical composition or combination as defined herein, for use in a method of treating or preventing a cell proliferative disease in a warm-blooded animal.

The following embodiments describe the claimed invention:
1. A compound wherein the compound is 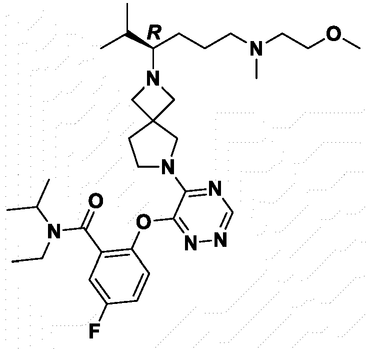 or a pharmaceutically acceptable salt or a solvate thereof.
2. The compound according to embodiment 1, wherein the compound is a pharmaceutically acceptable salt.
3. The compound according to embodiment 1, wherein the compound is a solvate.
4. The compound according to embodiment 1, wherein the compound is a solvate of a pharmaceutically acceptable salt.
5. The compound according to embodiment 1, wherein the compound is 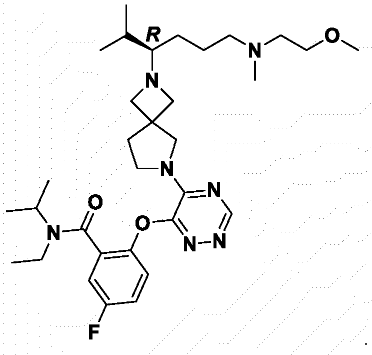
6. The compound according to embodiment 1, wherein the compound is 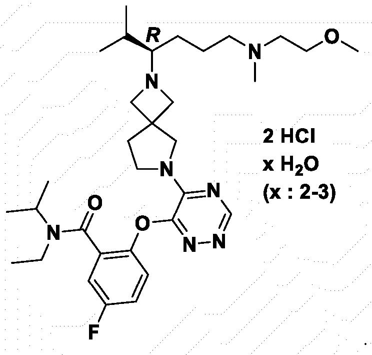
7. The compound according to embodiment 6, wherein the compound is 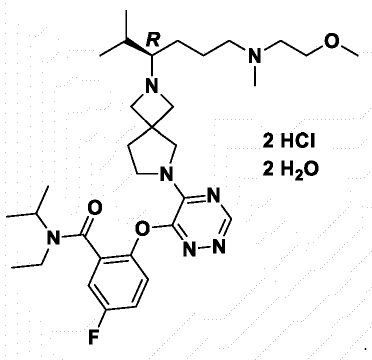
8. The compound according to embodiment 1, wherein the compound is 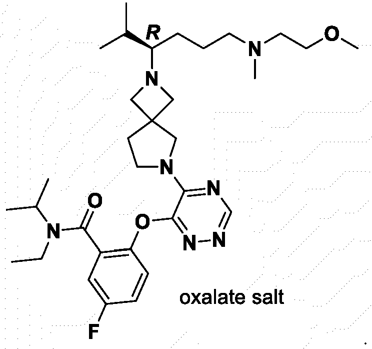
9. A pharmaceutical composition comprising a compound as defined in any one of embodiments 1 to 8 and a pharmaceutically acceptable carrier or diluent.
10. A process for preparing a pharmaceutical composition as defined in embodiment 9 comprising mixing a pharmaceutically acceptable carrier with a therapeutically effective amount of a compound according to any one of embodiments 1 to 8.
11. A compound as defined in any one of embodiments 1 to 8 or a pharmaceutical composition as defined in claim 9 for use as a medicament.
12. A compound as defined in any one of embodiments 1 to 8 or a pharmaceutical composition as defined in embodiment 9 for use in the prevention or treatment of cancer.
13. A compound as defined in any one of embodiments 1 to 8 or a pharmaceutical composition as defined in embodiment 9 for use in the prevention or treatment of leukemia, myelodysplastic syndrome (MDS), or myeloproliferative neoplasms (MPN).
14. The compound or pharmaceutical composition for use according to embodiment 13, in the treatment of leukemia wherein the leukemia is (*NPM1*)-mutated leukemia.
15. The compound or pharmaceutical composition for use according to embodiment 13, in the prevention or treatment of leukemia wherein the leukemia is selected from acute leukemias, chronic leukemias, myeloid leukemias, myelogenous leukemias, lymphoblastic leukemias, and lymphocytic leukemias.
16. The compound or pharmaceutical composition for use according to embodiment 13, in the prevention or treatment of leukemia wherein the leukemia is selected from Acute myelogenous leukemias (AML), Chronic myelogenous leukemias (CML), Acute lymphoblastic leukemias (ALL), Chronic lymphocytic leukemias (CLL), T cell prolymphocytic leukemias (T-PLL), Large granular lymphocytic leukemia, Hairy cell leukemia (HCL), *KMT2A*-rearranged leukemias, *KMT2A*-PTD leukemias, *KMT2A* amplified leukemias, *KMT2A*-positive leukemias, and leukemias exhibiting *HOX*/*MEIS1* gene expression signatures.
17. The compound or pharmaceutical composition for use according to embodiment 13, in the treatment of leukemia wherein the leukemia is an acute leukemia.
18. The compound or pharmaceutical composition for use according to embodiment 17, wherein the acute leukemia is AML.
19. The compound or pharmaceutical composition for use according to embodiment 17, wherein the acute leukemia is ALL.
20. The compound or pharmaceutical composition for use according to any one of embodiments 17-19, wherein the acute leukemia has KMT2A gene alterations or *NPM1* mutations.
21. The compound or pharmaceutical composition for use according to any one of embodiments 17-19, wherein the acute leukemia has KMT2A gene rearrangements.
22. The compound or pharmaceutical composition for use according to any one of embodiments 17-19, wherein the acute leukemia has *NPM1* mutations.
23. The compound or pharmaceutical composition for use according to embodiment 22, wherein the *NPM1* mutation is *NPM1c.*
24. The compound or pharmaceutical composition for use according to embodiment 14, wherein the (*NPM1*)-mutated leukemia is *NPM1c.*

### DETAILED DESCRIPTION OF THE DISCLOSURE

The term 'halo' or 'halogen' as used herein represents fluoro, chloro, bromo and iodo.

The prefix 'C_{x-y}' (where x and y are integers) as used herein refers to the number of carbon atoms in a given group. Thus, a C₁₋₆alkyl group contains from 1 to 6 carbon atoms, and so on.

The term 'C₁₋₄alkyl' as used herein as a group or part of a group represents a straight or branched chain saturated hydrocarbon radical having from 1 to 4 carbon atoms, such as methyl, ethyl, *n*-propyl, isopropyl, *n*-butyl, *s*-butyl, *t*-butyl and the like.

Similar, the term 'C₁₋₆alkyl' as used herein as a group or part of a group represents a straight or branched chain saturated hydrocarbon radical having from 1 to 6 carbon atoms, such as methyl, ethyl, *n*-propyl, isopropyl, *n*-butyl, *s*-butyl, *t*-butyl, *n*-pentyl, *n*-hexyl and the like.

The term 'C₃₋₆cycloalkyl' as used herein as a group or part of a group defines a saturated, cyclic hydrocarbon radical having from 3 to 6 carbon atoms, such as cyclopropyl, cyclobutyl, cyclopentyl and cyclohexyl.

It will be clear for the skilled person that S(=O)₂ or SO₂ represents a sulfonyl moiety.

It will be clear for the skilled person that CO or C(=O) represents a carbonyl moiety.

It will be clear for the skilled person that a group such as -CRR- represents 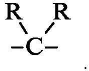 An example of such a group is -CR^{5a}R^{5b}-.

It will be clear for the skilled person that a group such as -NR- represents 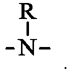 An example of such a group is -NR^{5c}-.

Non-limiting examples of 'monocyclic 5- or 6-membered aromatic rings containing one, two or three nitrogen atoms and optionally a carbonyl moiety', include, but are not limited to pyrazolyl, imidazolyl, pyridinyl, pyridazinyl, pyrimidinyl, pyrazinyl, triazinyl or 1,2-dihydro-2-oxo-4-pyridinyl.

The skilled person will understand that a 5- or 6-membered monocyclic aromatic ring containing one, two or three nitrogen atoms and a carbonyl moiety includes, but is not limited to

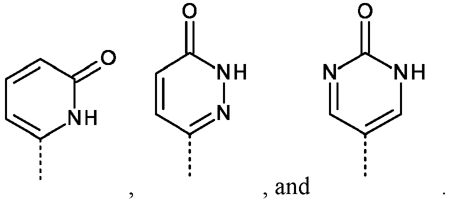

When any variable occurs more than one time in any constituent, each definition is independent.

When any variable occurs more than one time in any formula (e.g. Formula (I)), each definition is independent.

In general, whenever the term 'substituted' is used in the present disclosure, it is meant, unless otherwise indicated or clear from the context, to indicate that one or more hydrogens, in particular from 1 to 4 hydrogens, more in particular from 1 to 3 hydrogens, preferably 1 or 2 hydrogens, more preferably 1 hydrogen, on the atom or radical indicated in the expression using 'substituted' are replaced with a selection from the indicated group, provided that the normal valency is not exceeded, and that the substitution results in a chemically stable compound, i.e. a compound that is sufficiently robust to survive isolation to a useful degree of purity from a reaction mixture (isolation after a reaction e.g. purification by silica gel chromatography). In a particular embodiment, when the number of substituents is not explicitly specified, the number of substituents is one.

Combinations of substituents and/or variables are permissible only if such combinations result in chemically stable compounds. 'Stable compound' is in this context meant to indicate a compound that is sufficiently robust to survive isolation to a useful degree of purity from a reaction mixture (isolation after a reaction e.g. purification by silica gel chromatography).

The skilled person will understand that the term 'optionally substituted' means that the atom or radical indicated in the expression using 'optionally substituted' may or may not be substituted (this means substituted or unsubstituted respectively).

When two or more substituents are present on a moiety they may, where possible and unless otherwise indicated or clear from the context, replace hydrogens on the same atom or they may replace hydrogen atoms on different atoms in the moiety.

Within the context of this disclosure 'saturated' means 'fully saturated', if not otherwise specified.

Unless otherwise specified or clear from the context, aromatic rings goups, can be attached to the remainder of the molecule of Formula (I) through any available ring carbon atom (C-linked) or nitrogen atom (N-linked).

Unless otherwise specified or clear from the context, aromatic rings goups, may optionally be substituted, where possible, on carbon and/or nitrogen atoms according to the embodiments.

The term "subject" as used herein, refers to an animal, preferably a mammal (e.g. cat, dog, primate or human), more preferably a human, who is or has been the object of treatment, observation or experiment.

The term "therapeutically effective amount" as used herein, means that amount of active compound or pharmaceutical agent that elicits the biological or medicinal response in a tissue system, animal or human that is being sought by a researcher, veterinarian, medicinal doctor or other clinician, which includes alleviation or reversal of the symptoms of the disease or disorder being treated.

The term "composition" is intended to encompass a product comprising the specified ingredients in the specified amounts, as well as any product which results, directly or indirectly, from combinations of the specified ingredients in the specified amounts.

The term "treatment", as used herein, is intended to refer to all processes wherein there may be a slowing, interrupting, arresting or stopping of the progression of a disease, but does not necessarily indicate a total elimination of all symptoms.

The term "compound(s) of the (present) disclosure" or "compound(s) according to the (present) disclosure" as used herein, is meant to include the compounds of Formula (I) and the pharmaceutically acceptable salts, and the solvates thereof.

As used herein, any chemical formula with bonds shown only as solid lines and not as solid wedged or hashed wedged bonds, or otherwise indicated as having a particular configuration (e.g. *R, S*) around one or more atoms, contemplates each possible stereoisomer, or mixture of two or more stereoisomers.

Hereinbefore and hereinafter, the term "compound(s) of Formula (I)" is meant to include the tautomers thereof and the stereoisomeric forms thereof.

The terms "stereoisomers", "stereoisomeric forms" or "stereochemically isomeric forms" hereinbefore or hereinafter are used interchangeably.

The disclosure includes all stereoisomers of the compounds of the disclosure either as a pure stereoisomer or as a mixture of two or more stereoisomers.

Enantiomers are stereoisomers that are non-superimposable mirror images of each other. A 1:1 mixture of a pair of enantiomers is a racemate or racemic mixture.

Atropisomers (or atropoisomers) are stereoisomers which have a particular spatial configuration, resulting from a restricted rotation about a single bond, due to large steric hindrance. All atropisomeric forms of the compounds of Formula (I) are intended to be included within the scope of the present disclosure.

Diastereomers (or diastereoisomers) are stereoisomers that are not enantiomers, i.e. they are not related as mirror images. If a compound contains a double bond, the substituents may be in the *E* or the *Z* configuration.

Substituents on bivalent cyclic saturated or partially saturated radicals may have either the cis-or trans-configuration; for example if a compound contains a disubstituted cycloalkyl group, the substituents may be in the cis or trans configuration.

Therefore, the disclosure includes enantiomers, atropisomers, diastereomers, racemates, *E* isomers, *Z* isomers, cis isomers, trans isomers and mixtures thereof, whenever chemically possible.

The meaning of all those terms, i.e. enantiomers, atropisomers, diastereomers, racemates, *E* isomers, *Z* isomers, cis isomers, trans isomers and mixtures thereof are known to the skilled person.

The absolute configuration is specified according to the Cahn-Ingold-Prelog system. The configuration at an asymmetric atom is specified by either *R* or *S*. Resolved stereoisomers whose absolute configuration is not known can be designated by (+) or (-) depending on the direction in which they rotate plane polarized light. For instance, resolved enantiomers whose absolute configuration is not known can be designated by (+) or (-) depending on the direction in which they rotate plane polarized light.

When a specific stereoisomer is identified, this means that said stereoisomer is substantially free, i.e. associated with less than 50%, preferably less than 20%, more preferably less than 10%, even more preferably less than 5%, in particular less than 2% and most preferably less than 1%, of the other stereoisomers. Thus, when a compound of Formula (I) is for instance specified as (*R*), this means that the compound is substantially free of the (*S*) isomer; when a compound of Formula (I) is for instance specified as *E*, this means that the compound is substantially free of the *Z* isomer; when a compound of Formula (I) is for instance specified as cis, this means that the compound is substantially free of the trans isomer.

Some of the compounds according to Formula (I) may also exist in their tautomeric form. Such forms in so far as they may exist, although not explicitly indicated in the above Formula (I) are intended to be included within the scope of the present disclosure. It follows that a single compound may exist in both stereoisomeric and tautomeric form.

Pharmaceutically acceptable salts include acid addition salts and base addition salts. Such salts may be formed by conventional means, for example by reaction of a free acid or a free base form with one or more equivalents of an appropriate base or acid, optionally in a solvent, or in a medium in which the salt is insoluble, followed by removal of said solvent, or said medium, using standard techniques (e.g. *in vacuo,* by freeze-drying or by filtration). Salts may also be prepared by exchanging a counter-ion of a compound of the disclosure in the form of a salt with another counter-ion, for example using a suitable ion exchange resin.

The pharmaceutically acceptable salts as mentioned hereinabove or hereinafter are meant to comprise the therapeutically active non-toxic acid and base salt forms which the compounds of Formula (I) and solvates thereof, are able to form.

Appropriate acids comprise, for example, inorganic acids such as hydrohalic acids, e.g. hydrochloric or hydrobromic acid, sulfuric, nitric, phosphoric and the like acids; or organic acids such as, for example, acetic, propanoic, hydroxyacetic, lactic, pyruvic, oxalic (i.e. ethanedioic), malonic, succinic (i.e. butanedioic acid), maleic, fumaric, malic, tartaric, citric, methanesulfonic, ethanesulfonic, benzenesulfonic, p-toluenesulfonic, cyclamic, salicylic, p-aminosalicylic, pamoic and the like acids. Conversely said salt forms can be converted by treatment with an appropriate base into the free base form.

The compounds of Formula (I) and solvates thereof containing an acidic proton may also be converted into their non-toxic metal or amine salt forms by treatment with appropriate organic and inorganic bases.

Appropriate base salt forms comprise, for example, the ammonium salts, the alkali and earth alkaline metal salts, e.g. the lithium, sodium, potassium, cesium, magnesium, calcium salts and the like, salts with organic bases, e.g. primary, secondary and tertiary aliphatic and aromatic amines such as methylamine, ethylamine, propylamine, isopropylamine, the four butylamine isomers, dimethylamine, diethylamine, diethanolamine, dipropylamine, diisopropylamine, din-butylamine, pyrrolidine, piperidine, morpholine, trimethylamine, triethylamine, tripropylamine, quinuclidine, pyridine, quinoline and isoquinoline; the benzathine, N-methyl-D-glucamine, hydrabamine salts, and salts with amino acids such as, for example, arginine, lysine and the like. Conversely the salt form can be converted by treatment with acid into the free acid form.

The term "prodrug" includes any compound that, following oral or parenteral administration, in particular oral administration, is metabolised *in vivo* to a (more) active form in an experimentally-detectable amount, and within a predetermined time (e.g. within a dosing interval of between 0.5 and 24 hours, or e.g. within a dosing interval of between 6 and 24 hours (i.e. once to four times daily)). For the avoidance of doubt, the term "parenteral" administration includes all forms of administration other than oral administration, in particular intravenous (IV), intramuscular (IM), and subcutaneous (SC) injection.

Prodrugs may be prepared by modifying functional groups present on a compound in such a way that the modifications are cleaved *in vivo* when such prodrug is administered to a mammalian subject. The modifications typically are achieved by synthesising the parent compound with a prodrug substituent. In general, prodrugs include compounds wherein a hydroxyl, amino, sulfhydryl, carboxy or carbonyl group is bonded to any group that may be cleaved *in vivo* to regenerate the free hydroxyl, amino, sulfhydryl, carboxy or carbonyl group, respectively.

Examples of prodrugs include, but are not limited to, esters and carbamates of hydroxy functional groups, esters groups of carboxyl functional groups, N-acyl derivatives and N-Mannich bases. General information on prodrugs may be found e.g. in Bundegaard, H. "Design of Prodrugs" p. l-92, Elesevier, New York-Oxford (1985).

The term solvate comprises the solvent addition forms as well as the salts thereof, which the compounds of Formula (I) are able to form. Examples of such solvent addition forms are e.g. hydrates, alcoholates and the like.

The compounds of the disclosure as prepared in the processes described below may be synthesized in the form of mixtures of enantiomers, in particular racemic mixtures of enantiomers, that can be separated from one another following art-known resolution procedures. A manner of separating the enantiomeric forms of the compounds of Formula (I), and pharmaceutically acceptable salts, and solvates thereof, involves liquid chromatography using a chiral stationary phase. Said pure stereochemically isomeric forms may also be derived from the corresponding pure stereochemically isomeric forms of the appropriate starting materials, provided that the reaction occurs stereospecifically. Preferably if a specific stereoisomer is desired, said compound would be synthesized by stereospecific methods of preparation. These methods will advantageously employ enantiomerically pure starting materials.

The term "enantiomerically pure" as used herein means that the product contains at least 80% by weight of one enantiomer and 20% by weight or less of the other enantiomer. Preferably the product contains at least 90% by weight of one enantiomer and 10% by weight or less of the other enantiomer. In the most preferred embodiment the term "enantiomerically pure" means that the composition contains at least 99% by weight of one enantiomer and 1% or less of the other enantiomer.

The present disclosure also embraces isotopically-labeled compounds of the present disclosure which are identical to those recited herein, but for the fact that one or more atoms are replaced by an atom having an atomic mass or mass number different from the atomic mass or mass number usually found in nature (or the most abundant one found in nature).

All isotopes and isotopic mixtures of any particular atom or element as specified herein are contemplated within the scope of the compounds of the present disclosure, either naturally occurring or synthetically produced, either with natural abundance or in an isotopically enriched form. Exemplary isotopes that can be incorporated into compounds of the present disclosure include isotopes of hydrogen, carbon, nitrogen, oxygen, phosphorus, sulfur, fluorine, chlorine and iodine , such as ²H, ³H, ¹¹C, ¹³C, ¹⁴C , ¹³N, ¹⁵O, ¹⁷O, ¹⁸O, ³²P, ³³P, ³⁵S, ¹⁸F, ³⁶Cl, ¹²²I, ¹²³I, ¹²⁵I, ¹³¹I, ⁷⁵Br, ⁷⁶Br, ⁷⁷Br and ⁸²Br. Preferably, the isotope is selected from the group of ²H, ³H, ¹¹C, ¹³C and ¹⁸F. Preferably, the isotope is selected from the group of ²H, ³H, ¹¹C and ¹⁸F. More preferably, the isotope is ²H, ³H or ¹³C. More preferably, the isotope is ²H or ¹³C. More preferably, the isotope is ²H. In particular, deuterated compounds and ¹³C-enriched compounds are intended to be included within the scope of the present disclosure. In particular, deuterated compounds are intended to be included within the scope of the present disclosure.

Certain isotopically-labeled compounds of the present disclosure (e.g., those labeled with ³H and ¹⁴C) may be useful for example in substrate tissue distribution assays. Tritiated (³H) and carbon-14 (¹⁴C) isotopes are useful for their ease of preparation and detectability. Further, substitution with heavier isotopes such as deuterium (i.e., ²H) may afford certain therapeutic advantages resulting from greater metabolic stability (e.g., increased *in vivo* half-life or reduced dosage requirements) and hence may be preferred in some circumstances. Positron emitting isotopes such as ¹⁵O, ¹³N, ¹¹C and ¹⁸F are useful for positron emission tomography (PET) studies. PET imaging in cancer finds utility in helping locate and identify tumours, stage the disease and determine suitable treatment. Human cancer cells overexpress many receptors or proteins that are potential disease-specific molecular targets. Radiolabelled tracers that bind with high affinity and specificity to such receptors or proteins on tumour cells have great potential for diagnostic imaging and targeted radionuclide therapy (Charron, Carlie L. et al. Tetrahedron Lett. 2016, 57(37), 4119-4127). Additionally, target-specific PET radiotracers may be used as biomarkers to examine and evaluate pathology, by for example, measuring target expression and treatment response (Austin R. et al. Cancer Letters (2016), doi: 10.1016/j.canlet.2016.05.008).

The present disclosure relates in particular to compounds of Formula (I) as defined herein, and the tautomers and the stereoisomeric forms thereof, wherein
R^{1a} represents -C(=O)-NR^{xa}R^{xb}; Het; or 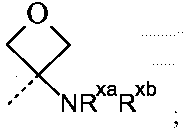
Het represents a 5- or 6-membered monocyclic aromatic ring containing one, two or three nitrogen atoms and optionally a carbonyl moiety;
wherein said 5- or 6-membered monocyclic aromatic ring is optionally substituted with one or two substituents selected from the group consisting of C₃₋₆cycloalkyl and C₁₋₄alkyl;
R^{xa} and R^{xb} are each independently selected from the group consisting of hydrogen, C₁₋₄alkyl and C₃₋₆cycloalkyl;
R^{1b} represents F or Cl;
Y¹ represents -CR^{5a}R^{5b}-, -O- or *-*NR^{5c}*-;*
R² is selected from the group consisting of hydrogen, halo, C₁₋₄alkyl, -O-C₁₋₄alkyl, and - NR^{7a}R^{7b};
U represents N or CH;
n1, n2, n3 and n4 are each independently selected from 1 and 2;
X¹ represents CH, and X² represents N;
R⁴ represents isopropyl;
R^{5a}, R^{5b}, R^{5c}, R^{7a}, and R^{7b}, are each independently selected from the group consisting of hydrogen, C₁₋₄alkyl and C₃₋₆cycloalkyl;
R³ represents -C₁₋₆alkyl-NR^{8a}R^{8b}, -C₁₋₆alkyl-C(=O)-NR^{9a}R^{9b}, -C₁₋₆alkyl-OH, or -C₁₋₆alkyl-NR¹¹-C(=O)-O-C₁₋₄alkyl-O-C(=O)-C₁₋₄alkyl;
wherein each of the C₁₋₄alkyl or C₁₋₆alkyl moieties in the R³ definitions independently of each other may be substituted with one, two or three substituents each independently selected from the group consisting of cyano, halo or -O-C₁₋₄alkyl;
R^{8a} and R^{8b} are each independently selected from the group consisting of hydrogen; C₁₋₆alkyl; -C(=O)-C₁₋₄alkyl; -C(=O)-O-C₁₋₄alkyl; -C(=O)-NR^{12a}R^{12b}; and C₁₋₆alkyl substituted with one, two or three substituents each independently selected from the group consisting of cyano, halo, -S(=O)₂-C₁₋₄alkyl, -O-C₁₋₄alkyl, and -C(=O)-NR^{10a}R^{10b};
R^{9a}, R^{9b}, R^{10a}, R^{10b}, R¹¹, R^{12a}, and R^{12b} are each independently selected from the group consisting of hydrogen and C₁₋₆alkyl;
and the pharmaceutically acceptable salts and the solvates thereof.

The present disclosure relates in particular to compounds of Formula (I) as defined herein, and the tautomers and the stereoisomeric forms thereof, wherein
R^{1a} represents -C(=O)-NR^{xa}R^{xb}; Het; or 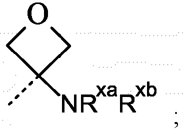
Het represents a 5- or 6-membered monocyclic aromatic ring containing one, two or three nitrogen atoms and optionally a carbonyl moiety;
wherein said 5- or 6-membered monocyclic aromatic ring is optionally substituted with one or two substituents selected from the group consisting of C₃₋₆cycloalkyl and C₁₋₄alkyl;
R^{xa} and R^{xb} are each independently selected from the group consisting of hydrogen, C₁₋₄alkyl and C₃₋₆cycloalkyl;
R^{1b} represents F or Cl;
Y¹ represents -CR^{5a}R^{5b}-, -O- or *-*NR^{5c}*-;*
R² is selected from the group consisting of hydrogen, halo, C₁₋₄alkyl, -O-C₁₋₄alkyl, and - NR^{7a}R^{7b};
U represents N or CH;
n1, n2, n3 and n4 are each independently selected from 1 and 2;
X¹ represents CH, and X² represents N;
R⁴ represents isopropyl;
R^{5a}, R^{5b}, R^{5c}, R^{7a}, and R^{7b}, are each independently selected from the group consisting of hydrogen, C₁₋₄alkyl and C₃₋₆cycloalkyl;
R³ represents -C₁₋₆alkyl-NR^{8a}R^{8b};
wherein the C₁₋₆alkyl moiety in the R³ definition may be substituted with one, two or three substituents each independently selected from the group consisting of cyano, halo, OH, and - O-C₁₋₄alkyl;
R^{8a} and R^{8b} are each independently selected from the group consisting of hydrogen; C₁₋₆alkyl; and C₁₋₆alkyl substituted with one, two or three substituents each independently selected from the group consisting of -OH, cyano, halo, -S(=O)₂-C₁₋₄alkyl, -O-C₁₋₄alkyl, - C(=O)-NR^{10a}R^{10b}, and -NR^{10c}-C(=O)-C₁₋₄alkyl;
R^{10a}, R^{10b}, R^{10c} are each independently selected from the group consisting of hydrogen and C₁₋₆alkyl;
and the pharmaceutically acceptable salts and the solvates thereof.

The present disclosure relates in particular to compounds of Formula (I) as defined herein, and the tautomers and the stereoisomeric forms thereof, wherein
R^{1a} represents -C(=O)-NR^{xa}R^{xb}; Het; or 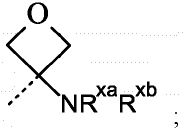
Het represents a 5- or 6-membered monocyclic aromatic ring containing one, two or three nitrogen atoms and optionally a carbonyl moiety;
wherein said 5- or 6-membered monocyclic aromatic ring is optionally substituted with one or two substituents selected from the group consisting of C₃₋₆cycloalkyl and C₁₋₄alkyl;
R^{xa} and R^{xb} are each independently selected from the group consisting of hydrogen, C₁₋₄alkyl and C₃₋₆cycloalkyl;
R^{1b} represents F or Cl;
Y¹ represents -CR^{5a}R^{5b}-, -O- or *-*NR^{5c}*-;*
R² is selected from the group consisting of hydrogen, halo, C₁₋₄alkyl, -O-C₁₋₄alkyl, and - NR^{7a}R^{7b};
U represents N or CH;
n1, n2, n3 and n4 are each independently selected from 1 and 2;
X¹ represents CH, and X² represents N;
R⁴ represents isopropyl;
R^{5a}, R^{5b}, R^{5c}, R^{7a}, and R^{7b}, are each independently selected from the group consisting of hydrogen, C₁₋₄alkyl and C₃₋₆cycloalkyl;
R³ represents -C₁₋₆alkyl-NR^{8a}R^{8b};
wherein the C₁₋₆alkyl moiety in the R³ definition may be substituted with one, two or three substituents each independently selected from the group consisting of cyano, halo and -O-C₁₋₄alkyl;
R^{8a} and R^{8b} are each independently selected from the group consisting of hydrogen; C₁₋₆alkyl; and C₁₋₆alkyl substituted with one, two or three substituents each independently selected from the group consisting of cyano, halo, -S(=O)₂-C₁₋₄alkyl, -O-C₁₋₄alkyl, and - C(=O)-NR^{10a}R^{10b};
R^{10a} and R^{10b} are each independently selected from the group consisting of hydrogen and C₁₋₆alkyl;
and the pharmaceutically acceptable salts and the solvates thereof.

The present disclosure relates in particular to compounds of Formula (I) as defined herein, and the tautomers and the stereoisomeric forms thereof, wherein
R^{1a} represents -C(=O)-NR^{xa}R^{xb} or Het;
Het represents a 6-membered monocyclic aromatic ring containing two nitrogen atoms; wherein said 6-membered monocyclic aromatic ring is substituted with one C₃₋₆cycloalkyl;
R^{xa} and R^{xb} represent C₁₋₄alkyl;
R^{1b} represents F;
Y¹ represents -O-;
R² represents hydrogen;
U represents N or CH;
n1, n2, n3 and n4 are each independently selected from 1 and 2;
X¹ represents CH, and X² represents N;
R⁴ represents isopropyl;
R³ represents -C₁₋₆alkyl-NR^{8a}R^{8b}, -C₁₋₆alkyl-C(=O)-NR^{9a}R^{9b}, -C₁₋₆alkyl-OH, or -C₁₋₆alkyl-NR¹¹-C(=O)-O-C₁₋₄alkyl-O-C(=O)-C₁₋₄alkyl;
wherein each of the C₁₋₄alkyl or C₁₋₆alkyl moieties in the R³ definitions independently of each other may be substituted with one, two or three substituents each independently selected from the group consisting of -OH and -O-C₁₋₄alkyl;
R^{8a} and R^{8b} are each independently selected from the group consisting of hydrogen; C₁₋₆alkyl; -C(=O)-C₁₋₄alkyl; -C(=O)-O-C₁₋₄alkyl; -C(=O)-NR^{12a}R^{12b}; and C₁₋₆alkyl substituted with one, two or three substituents each independently selected from the group consisting of -OH, cyano, halo, -S(=O)₂-C₁₋₄alkyl, -O-C₁₋₄alkyl, -C(=O)-NR^{10a}R^{10b}, and -NR^{10c}-C(=O)-C₁₋₄alkyl;
R^{9a}, R^{9b}, R^{10a}, R^{10b}, R^{10c}, R¹¹, R^{12a}, and R^{12b} are each independently selected from the group consisting of hydrogen and C₁₋₆alkyl;
and the pharmaceutically acceptable salts and the solvates thereof.

The present disclosure relates in particular to compounds of Formula (I) as defined herein, and the tautomers and the stereoisomeric forms thereof, wherein
R^{1a} represents -C(=O)-NR^{xa}R^{xb} or Het;
Het represents a 6-membered monocyclic aromatic ring containing two nitrogen atoms; wherein said 6-membered monocyclic aromatic ring is substituted with one C₃₋₆cycloalkyl;
R^{xa} and R^{xb} represent C₁₋₄alkyl;
R^{1b} represents F;
Y¹ represents -O-;
R² represents hydrogen;
U represents N or CH;
n1, n2, n3 and n4 are each independently selected from 1 and 2;
X¹ represents CH, and X² represents N;
R⁴ represents isopropyl;
R³ represents -C₁₋₆alkyl-NR^{8a}R^{8b};
wherein the C₁₋₆alkyl moiety in the R³ definition may be substituted with one, two or three substituents each independently selected from the group consisting of -OH and -O-C₁₋₄alkyl;
R^{8a} and R^{8b} are each independently selected from the group consisting of hydrogen; C₁₋₆alkyl; and C₁₋₆alkyl substituted with one, two or three substituents each independently selected from the group consisting of -OH, cyano, halo, -S(=O)₂-C₁₋₄alkyl, -O-C₁₋₄alkyl, - C(=O)-NR^{10a}R^{10b}, and -NR^{10c}-C(=O)-C₁₋₄alkyl;
R^{10a}, R^{10b}, and R^{10c} are each independently selected from the group consisting of hydrogen and C₁₋₆alkyl;
and the pharmaceutically acceptable salts and the solvates thereof.

The present disclosure relates in particular to compounds of Formula (I) as defined herein, and the tautomers and the stereoisomeric forms thereof, wherein
R^{1a} represents -C(=O)-NR^{xa}R^{xb};
R^{xa} and R^{xb} represent C₁₋₄alkyl;
R^{1b} represents F;
Y¹ represents -O-;
R² represents hydrogen;
U represents N or CH;
n1, n2, n3 and n4 are each independently selected from 1 and 2;
X¹ represents CH, and X² represents N;
R⁴ represents isopropyl;
R³ represents -C₁₋₆alkyl-NR^{8a}R^{8b};
wherein the C₁₋₆alkyl moiety in the R³ definition may be substituted with one, two or three substituents each independently selected from the group consisting of -OH and -O-C₁₋₄alkyl;
R^{8a} and R^{8b} are each independently selected from the group consisting of hydrogen; C₁₋₆alkyl; and C₁₋₆alkyl substituted with one, two or three substituents each independently selected from the group consisting of -OH, cyano, halo, -S(=O)₂-C₁₋₄alkyl, -O-C₁₋₄alkyl, - C(=O)-NR^{10a}R^{10b}, and -NR^{10c}-C(=O)-C₁₋₄alkyl;
R^{10a}, R^{10b}, and R^{10c} are each independently selected from the group consisting of hydrogen and C₁₋₆alkyl;
and the pharmaceutically acceptable salts and the solvates thereof.

The present disclosure relates in particular to compounds of Formula (I) as defined herein, and the tautomers and the stereoisomeric forms thereof, wherein
R^{1a} represents -C(=O)-NR^{xa}R^{xb} or Het;
Het represents pyrimidinyl substituted with one C₃₋₆cycloalkyl;
R^{xa} and R^{xb} represent C₁₋₄alkyl;
R^{1b} represents F;
Y¹ represents -O-;
R² represents hydrogen;
U represents N;
n1, n2, n3 and n4 are each independently selected from 1 and 2;
X¹ represents CH, and X² represents N;
R⁴ represents isopropyl;
R³ represents -C₁₋₆alkyl-NR^{8a}R^{8b};
wherein the C₁₋₆alkyl moiety in the R³ definition may be substituted with one -OH;
R^{8a} and R^{8b} are each independently selected from the group consisting of hydrogen; C₁₋₆alkyl; and C₁₋₆alkyl substituted with one or two substituents each independently selected from the group consisting of halo, -O-C₁₋₄alkyl, and -NR^{10c}-C(=O)-C₁₋₄alkyl;
R^{10a}, R^{10b}, and R^{10c} are each independently selected from the group consisting of hydrogen and C₁₋₆alkyl;
and the pharmaceutically acceptable salts and the solvates thereof.

The present disclosure relates in particular to compounds of Formula (I) as defined herein, and the tautomers and the stereoisomeric forms thereof, wherein
R^{1a} represents -C(=O)-NR^{xa}R^{xb} or Het;
Het represents pyrimidinyl substituted with one C₃₋₆cycloalkyl;
R^{xa} and R^{xb} represent C₁₋₄alkyl;
R^{1b} represents F;
Y¹ represents -O-;
R² represents hydrogen;
U represents N;
n2 is 2;
n1, n3 and n4 are 1;
X¹ represents CH, and X² represents N;
R⁴ represents isopropyl;
R³ represents -C₁₋₆alkyl-NR^{8a}R^{8b};
wherein the C₁₋₆alkyl moiety in the R³ definition may be substituted with one -OH;
R^{8a} and R^{8b} are each independently selected from the group consisting of hydrogen; C₁₋₆alkyl; and C₁₋₆alkyl substituted with one or two substituents each independently selected from the group consisting of halo, -O-C₁₋₄alkyl, and -NR^{10c}-C(=O)-C₁₋₄alkyl;
R^{10a}, R^{10b}, and R^{10c} are each independently selected from the group consisting of hydrogen and C₁₋₆alkyl;
and the pharmaceutically acceptable salts and the solvates thereof.

The present disclosure relates in particular to compounds of Formula (I) as defined herein, and the tautomers and the stereoisomeric forms thereof, wherein
R^{1a} represents -C(=O)-NR^{xa}R^{xb};
R^{xa} and R^{xb} represent C₁₋₄alkyl;
R^{1b} represents F;
Y¹ represents -O-;
R² represents hydrogen;
U represents N;
n2 is 2;
n1, n3 and n4 are 1;
X¹ represents CH, and X² represents N;
R⁴ represents isopropyl;
R³ represents -C₁₋₆alkyl-NR^{8a}R^{8b};
R^{8a} and R^{8b} are each independently selected from the group consisting of hydrogen; C₁₋₆alkyl; and C₁₋₆alkyl substituted with one or two substituents each independently selected from the group consisting of halo, -O-C₁₋₄alkyl, and -NR^{10c}-C(=O)-C₁₋₄alkyl;
R^{10a}, R^{10b}, and R^{10c} are each independently selected from the group consisting of hydrogen and C₁₋₆alkyl;
and the pharmaceutically acceptable salts and the solvates thereof.

The present disclosure relates in particular to compounds of Formula (I) as defined herein, and the tautomers and the stereoisomeric forms thereof, wherein
R^{1a} represents -C(=O)-NR^{xa}R^{xb};
R^{xa} and R^{xb} represent C₁₋₄alkyl;
R^{1b} represents F;
Y¹ represents -O-;
R² represents hydrogen;
U represents N;
n2 is 2;
n1, n3 and n4 are 1;
X¹ represents CH, and X² represents N;
R⁴ represents isopropyl;
R³ represents -CH₂-CH₂-CH₂-NR^{8a}R^{8b};
R^{8a} and R^{8b} are each independently selected from the group consisting of hydrogen; C₁₋₆alkyl; and C₁₋₆alkyl substituted with one or two substituents each independently selected from the group consisting of halo, -O-C₁₋₄alkyl, and -NR^{10c}-C(=O)-C₁₋₄alkyl;
R^{10a}, R^{10b}, and R^{10c} are each independently selected from the group consisting of hydrogen and C₁₋₆alkyl;
and the pharmaceutically acceptable salts and the solvates thereof.

The present disclosure relates in particular to compounds of Formula (I) as defined herein, and the tautomers and the stereoisomeric forms thereof, wherein
R^{1a} represents -C(=O)-NR^{xa}R^{xb};
R^{xa} and R^{xb} represent C₁₋₄alkyl;
R^{1b} represents F;
Y¹ represents -O-;
R² represents hydrogen;
U represents N;
n1, n2, n3 and n4 are each independently selected from 1 and 2;
X¹ represents CH, and X² represents N;
R⁴ represents isopropyl;
R³ represents -C₁₋₆alkyl-NR^{8a}R^{8b};
R^{8a} and R^{8b} are each independently selected from the group consisting of hydrogen; C₁₋₆alkyl; and C₁₋₆alkyl substituted with one, two or three substituents each independently selected from the group consisting of -OH, cyano, halo, -S(=O)₂-C₁₋₄alkyl, -O-C₁₋₄alkyl, and - C(=O)-NR^{10a}R^{10b};
R^{10a} and R^{10b} are each independently selected from the group consisting of hydrogen and C₁₋₆alkyl;
and the pharmaceutically acceptable salts and the solvates thereof.

The present disclosure relates in particular to compounds of Formula (I) as defined herein, and the tautomers and the stereoisomeric forms thereof, wherein
R^{1a} represents -C(=O)-NR^{xa}R^{xb};
R^{xa} and R^{xb} represent C₁₋₄alkyl;
R^{1b} represents F;
Y¹ represents -O-;
R² represents hydrogen;
U represents N;
n1, n2, n3 and n4 are each independently selected from 1 and 2;
X¹ represents CH, and X² represents N;
R⁴ represents isopropyl;
R³ represents -CH₂-CH₂-CH₂^{_}NR^{8a}R^{8b};
R^{8a} and R^{8b} are each independently selected from the group consisting of hydrogen; C₁₋₆alkyl; and C₁₋₆alkyl substituted with one, two or three substituents each independently selected from the group consisting of -OH, cyano, halo, -S(=O)₂-C₁₋₄alkyl, -O-C₁₋₄alkyl, and - C(=O)-NR^{10a}R^{10b};
R^{10a} and R^{10b} are each independently selected from the group consisting of hydrogen and C₁₋₆alkyl;
and the pharmaceutically acceptable salts and the solvates thereof.

The present disclosure relates in particular to compounds of Formula (I) as defined herein, and the tautomers and the stereoisomeric forms thereof, wherein
R^{1a} represents -C(=O)-NR^{xa}R^{xb};
R^{xa} and R^{xb} represent hydrogen or C₁₋₄alkyl;
R^{1b} represents F;
Y¹ represents -O-;
R² represents hydrogen;
U represents N;
n1, n2, n3 and n4 are each independently selected from 1 and 2;
X¹ represents CH, and X² represents N;
R⁴ represents isopropyl;
R³ represents -CH₂-CH₂-CH₂-NR^{8a}R^{8b};
R^{8a} and R^{8b} are each independently selected from the group consisting of hydrogen; C₁₋₆alkyl; and C₁₋₆alkyl substituted with one, two or three substituents each independently selected from the group consisting of -OH, cyano, halo, -S(=O)₂-C₁₋₄alkyl, -O-C₁₋₄alkyl, and - C(=O)-NR^{10a}R^{10b};
R^{10a} and R^{10b} are each independently selected from the group consisting of hydrogen and C₁₋₆alkyl;
and the pharmaceutically acceptable salts and the solvates thereof.

The present disclosure relates in particular to compounds of Formula (I) as defined herein, and the tautomers and the stereoisomeric forms thereof, wherein
R^{1a} represents -C(=O)-NR^{xa}R^{xb};
R^{xa} and R^{xb} represent hydrogen or C₁₋₄alkyl;
R^{1b} represents F;
Y¹ represents -O-;
R² represents hydrogen;
U represents N;
n1, n2, n3 and n4 are each independently selected from 1 and 2;
X¹ represents CH, and X² represents N;
R⁴ represents isopropyl;
R³ represents -CH₂-CH₂-CH₂-NR^{8a}R^{8b};
R^{8a} and R^{8b} are each independently selected from the group consisting of hydrogen; C₁₋₆alkyl; and C₁₋₆alkyl substituted with one, two or three substituents each independently selected from the group consisting of -OH and -O-C₁₋₄alkyl;
and the pharmaceutically acceptable salts and the solvates thereof.

The present disclosure relates in particular to compounds of Formula (I) as defined herein, and the tautomers and the stereoisomeric forms thereof, wherein
R^{1a} represents -C(=O)-NR^{xa}R^{xb};
R^{xa} and R^{xb} represent C₁₋₄alkyl;
R^{1b} represents F;
Y¹ represents -O-;
R² represents hydrogen;
U represents N;
n1, n2, n3 and n4 are each independently selected from 1 and 2;
X¹ represents CH, and X² represents N;
R⁴ represents isopropyl;
R³ represents -C₁₋₆alkyl-NR^{8a}R^{8b};
R^{8a} and R^{8b} are each independently selected from the group consisting of C₁₋₆alkyl; and C₁₋₆alkyl substituted with one -O-C₁₋₄alkyl;
and the pharmaceutically acceptable salts and the solvates thereof.

The present disclosure relates in particular to compounds of Formula (I) as defined herein, and the tautomers and the stereoisomeric forms thereof, wherein
R^{1a} represents -C(=O)-NR^{xa}R^{xb};
R^{xa} and R^{xb} represent C₁₋₄alkyl;
R^{1b} represents F;
Y¹ represents -O-;
R² represents hydrogen;
U represents N;
n1, n2, n3 and n4 are each independently selected from 1 and 2;
X¹ represents CH, and X² represents N;
R⁴ represents isopropyl;
R³ represents -CH₂-CH₂-CH₂-NR^{8a}R^{8b};
R^{8a} and R^{8b} are each independently selected from the group consisting of C₁₋₆alkyl; and C₁₋₆alkyl substituted with one -O-C₁₋₄alkyl;
and the pharmaceutically acceptable salts and the solvates thereof.

The present disclosure relates in particular to compounds of Formula (I) as defined herein, and the tautomers and the stereoisomeric forms thereof, wherein
R^{1a} represents -C(=O)-NR^{xa}R^{xb}; or Het;
Het represents a 6-membered monocyclic aromatic ring containing two nitrogen atoms;
wherein said 6-membered monocyclic aromatic ring is optionally substituted with one C₃₋₆cycloalkyl;
R^{xa} and R^{xb} represent C₁₋₄alkyl;
R^{1b} represents F;
Y¹ represents -O-;
R² is hydrogen;
U represents N;
n1, n2, n3 and n4 are each independently selected from 1 and 2;
X¹ represents CH, and X² represents N;
R⁴ represents isopropyl;
R³ represents -C₁₋₆alkyl-NR^{8a}R^{8b}, -C₁₋₆alkyl-C(=O)-NR^{9a}R^{9b}, -C₁₋₆alkyl-OH, or -C₁₋₆alkyl-NR¹¹-C(=O)-O-C₁₋₄alkyl-O-C(=O)-C₁₋₄alkyl;
R^{8a} and R^{8b} are each independently selected from the group consisting of hydrogen; C₁₋₆alkyl; -C(=O)-C₁₋₄alkyl; -C(=O)-O-C₁₋₄alkyl; -C(=O)-NR^{12a}R^{12b}; and C₁₋₆alkyl substituted with one, two or three substituents each independently selected from the group consisting of cyano, halo, -S(=O)₂-C₁₋₄alkyl, and -O-C₁₋₄alkyl;
R^{9a}, R^{9b}, R^{12a}, and R^{12b} are each independently selected from the group consisting of hydrogen and C₁₋₆alkyl;
and the pharmaceutically acceptable salts and the solvates thereof.

The present disclosure relates in particular to compounds of Formula (I) as defined herein, and the tautomers and the stereoisomeric forms thereof, wherein
R^{1a} represents -C(=O)-NR^{xa}R^{xb};
R^{xa} and R^{xb} represent C₁₋₄alkyl;
R^{1b} represents F;
Y¹ represents -O-;
R² is hydrogen;
U represents N;
n1, n2, n3 and n4 are each independently selected from 1 and 2;
X¹ represents CH, and X² represents N;
R⁴ represents isopropyl;
R³ represents -C₁₋₆alkyl-NR^{8a}R^{8b}, -C₁₋₆alkyl-C(=O)-NR^{9a}R^{9b}, or -C₁₋₆alkyl-OH;
R^{8a} and R^{8b} are each independently selected from the group consisting of hydrogen; C₁₋₆alkyl; -C(=O)-C₁₋₄alkyl; -C(=O)-O-C₁₋₄alkyl; -C(=O)-NR^{12a}R^{12b}; and C₁₋₆alkyl substituted with one, two or three substituents each independently selected from the group consisting of cyano, halo, -S(=O)₂-C₁₋₄alkyl, and -O-C₁₋₄alkyl;
R^{9a}, R^{9b}, R^{12a}, and R^{12b} are each independently selected from the group consisting of hydrogen and C₁₋₆alkyl;
and the pharmaceutically acceptable salts and the solvates thereof.

In an embodiment, the present disclosure relates to those compounds of Formula (I) and the pharmaceutically acceptable salts, and the solvates thereof, or any subgroup thereof as mentioned in any of the other embodiments, wherein
R^{1b} represents F.

In an embodiment, the present disclosure relates to those compounds of Formula (I) and the pharmaceutically acceptable salts, and the solvates thereof, or any subgroup thereof as mentioned in any of the other embodiments, wherein
R² represents hydrogen.

In an embodiment, the present disclosure relates to those compounds of Formula (I) and the pharmaceutically acceptable salts, and the solvates thereof, or any subgroup thereof as mentioned in any of the other embodiments, wherein n1 is 1, n2 is 2, n3 is 1, and n4 is 1.

In an embodiment, the present disclosure relates to those compounds of Formula (I) and the pharmaceutically acceptable salts, and the solvates thereof, or any subgroup thereof as mentioned in any of the other embodiments, wherein
Y¹ represents -O-.

In an embodiment, the present disclosure relates to those compounds of Formula (I) and the pharmaceutically acceptable salts, and the solvates thereof, or any subgroup thereof as mentioned in any of the other embodiments, wherein
Y¹ represents -O-; and
U represents N.

In an embodiment, the present disclosure relates to those compounds of Formula (I) and the pharmaceutically acceptable salts, and the solvates thereof, or any subgroup thereof as mentioned in any of the other embodiments, wherein
Y¹ represents -O-;
U represents N;
R^{1b} represents F; and
R² represents hydrogen.

In an embodiment, the present disclosure relates to those compounds of Formula (I) and the pharmaceutically acceptable salts, and the solvates thereof, or any subgroup thereof as mentioned in any of the other embodiments, wherein Het represents

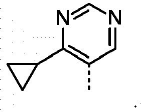

In an embodiment, the present disclosure relates to those compounds of Formula (I) and the pharmaceutically acceptable salts, and the solvates thereof, or any subgroup thereof as mentioned in any of the other embodiments, wherein Het represents a monocyclic 5- or 6-membered aromatic ring containing one or two nitrogen atoms; wherein said monocyclic 5- or 6-membered aromatic ring is substituted with one C₃₋₆cycloalkyl.

In an embodiment, the present disclosure relates to those compounds of Formula (I) and the pharmaceutically acceptable salts, and the solvates thereof, or any subgroup thereof as mentioned in any of the other embodiments, wherein Het represents a monocyclic 5- or 6-membered aromatic ring containing one or two nitrogen atoms; wherein said monocyclic 5- or 6-membered aromatic ring is substituted with one C₃₋₆cycloalkyl; and R^{1b} represents F.

In an embodiment, the present disclosure relates to those compounds of Formula (I) and the pharmaceutically acceptable salts, and the solvates thereof, or any subgroup thereof as mentioned in any of the other embodiments, wherein Het represents a monocyclic 6-membered aromatic ring containing one or two nitrogen atoms; wherein said monocyclic 6-membered aromatic ring is substituted with one C₃₋₆cycloalkyl.

In an embodiment, the present disclosure relates to those compounds of Formula (I) and the pharmaceutically acceptable salts, and the solvates thereof, or any subgroup thereof as mentioned in any of the other embodiments, wherein Het represents a monocyclic 6-membered aromatic ring containing one or two nitrogen atoms; wherein said monocyclic 6-membered aromatic ring is substituted with one C₃₋₆cycloalkyl; and
R^{1b} represents F.

In an embodiment, the present disclosure relates to those compounds of Formula (I) and the pharmaceutically acceptable salts, and the solvates thereof, or any subgroup thereof as mentioned in any of the other embodiments, wherein
R³ represents -C₁₋₆alkyl-NR^{8a}R^{8b};
wherein the C₁₋₆alkyl moiety in the R³ definition may be substituted with one, two or three substituents each independently selected from the group consisting of cyano, halo and -O-C₁₋₄alkyl.

In an embodiment, the present disclosure relates to those compounds of Formula (I) and the pharmaceutically acceptable salts, and the solvates thereof, or any subgroup thereof as mentioned in any of the other embodiments, wherein
R³ represents -C₁₋₆alkyl-NR^{8a}R^{8b};
wherein the C₁₋₆alkyl moiety in the R³ definition may be substituted with one, two or three substituents each independently selected from the group consisting of cyano, halo, -OH, and - O-C₁₋₄alkyl.

In an embodiment, the present disclosure relates to those compounds of Formula (I) and the pharmaceutically acceptable salts, and the solvates thereof, or any subgroup thereof as mentioned in any of the other embodiments, wherein
R³ represents -C₁₋₆alkyl-NR^{8a}R^{8b}.

In an embodiment, the present disclosure relates to those compounds of Formula (I) and the pharmaceutically acceptable salts, and the solvates thereof, or any subgroup thereof as mentioned in any of the other embodiments, wherein
R³ represents -C₁₋₆alkyl-NR^{8a}R^{8b};
wherein the C₁₋₆alkyl moiety in the R³ definition may be substituted with one, two or three substituents each independently selected from the group consisting of cyano, halo and -O-C₁₋₄alkyl;
R^{8a} and R^{8b} are each independently selected from the group consisting of hydrogen; C₁₋₆alkyl; and C₁₋₆alkyl substituted with one, two or three substituents each independently selected from the group consisting of -OH, cyano, halo, -S(=O)₂-C₁₋₄alkyl, -O-C₁₋₄alkyl, - C(=O)-NR^{10a}R^{10b}, and -NR^{10c}-C(=O)-C₁₋₄alkyl.

In an embodiment, the present disclosure relates to those compounds of Formula (I) and the pharmaceutically acceptable salts, and the solvates thereof, or any subgroup thereof as mentioned in any of the other embodiments, wherein
R³ represents -C₁₋₆alkyl-NR^{8a}R^{8b};
wherein the C₁₋₆alkyl moiety in the R³ definition may be substituted with one, two or three substituents each independently selected from the group consisting of cyano, halo, -OH, and - O-C₁₋₄alkyl;
R^{8a} and R^{8b} are each independently selected from the group consisting of hydrogen; C₁₋₆alkyl; and C₁₋₆alkyl substituted with one, two or three substituents each independently selected from the group consisting of -OH, cyano, halo, -S(=O)₂-C₁₋₄alkyl, -O-C₁₋₄alkyl, - C(=O)-NR^{10a}R^{10b}, and -NR^{10c}-C(=O)-C₁₋₄alkyl.

In an embodiment, the present disclosure relates to those compounds of Formula (I) and the pharmaceutically acceptable salts, and the solvates thereof, or any subgroup thereof as mentioned in any of the other embodiments, wherein
R³ represents -C₁₋₆alkyl-NR^{8a}R^{8b};
wherein the C₁₋₆alkyl moiety in the R³ definition may be substituted with one, two or three substituents each independently selected from the group consisting of cyano, halo and -O-C₁₋₄alkyl;
R^{8a} and R^{8b} are each independently selected from the group consisting of hydrogen; C₁₋₆alkyl; and C₁₋₆alkyl substituted with one, two or three substituents each independently selected from the group consisting of cyano, halo, -S(=O)₂-C₁₋₄alkyl, -O-C₁₋₄alkyl, and - C(=O)-NR^{10a}R^{10b}.

In an embodiment, the present disclosure relates to those compounds of Formula (I) and the pharmaceutically acceptable salts, and the solvates thereof, or any subgroup thereof as mentioned in any of the other embodiments, wherein
R³ represents -C₁₋₆alkyl-NR^{8a}R^{8b};
wherein the C₁₋₆alkyl moiety in the R³ definition may be substituted with one, two or three substituents each independently selected from the group consisting of cyano, halo and -O-C₁₋₄alkyl;
R^{8a} and R^{8b} are each independently selected from the group consisting of hydrogen; C₁₋₆alkyl; and C₁₋₆alkyl substituted with one, two or three substituents each independently selected from the group consisting of cyano, halo, -S(=O)₂-C₁₋₄alkyl, -O-C₁₋₄alkyl, -C(=O)-NR^{10a}R^{10b}, and -NR^{10c}-C(=O)-C₁₋₄alkyl.

In an embodiment, the present disclosure relates to those compounds of Formula (I) and the pharmaceutically acceptable salts, and the solvates thereof, or any subgroup thereof as mentioned in any of the other embodiments, wherein
R³ represents -C₁₋₆alkyl-NR^{8a}R^{8b};
wherein the C₁₋₆alkyl moiety in the R³ definition may be substituted with one, two or three substituents each independently selected from the group consisting of cyano, halo, -OH, and - O-C₁₋₄alkyl;
R^{8a} and R^{8b} are each independently selected from the group consisting of hydrogen; C₁₋₆alkyl; and C₁₋₆alkyl substituted with one, two or three substituents each independently selected from the group consisting of cyano, halo, -S(=O)₂-C₁₋₄alkyl, -O-C₁₋₄alkyl, and - C(=O)-NR^{10a}R^{10b}.

In an embodiment, the present disclosure relates to those compounds of Formula (I) and the pharmaceutically acceptable salts, and the solvates thereof, or any subgroup thereof as mentioned in any of the other embodiments, wherein
R³ represents -C₁₋₆alkyl-NR^{8a}R^{8b};
wherein the C₁₋₆alkyl moiety in the R³ definition may be substituted with one, two or three substituents each independently selected from the group consisting of cyano, halo, -OH, and - O-C₁₋₄alkyl;
R^{8a} and R^{8b} are each independently selected from the group consisting of hydrogen; C₁₋₆alkyl; and C₁₋₆alkyl substituted with one, two or three substituents each independently selected from the group consisting of cyano, halo, -S(=O)₂-C₁₋₄alkyl, -O-C₁₋₄alkyl, -C(=O)-NR^{10a}R^{10b}, and -NR^{10c}-C(=O)-C₁₋₄alkyl.

In an embodiment, the present disclosure relates to those compounds of Formula (I) and the pharmaceutically acceptable salts, and the solvates thereof, or any subgroup thereof as mentioned in any of the other embodiments, wherein
R³ represents -C₂₋₆alkyl-NR^{8a}R^{8b};
wherein the C₂₋₆alkyl moiety in the R³ definition may be substituted with one, two or three substituents each independently selected from the group consisting of cyano, halo and -O-C₁₋₄alkyl.

In an embodiment, the present disclosure relates to those compounds of Formula (I) and the pharmaceutically acceptable salts, and the solvates thereof, or any subgroup thereof as mentioned in any of the other embodiments, wherein
R³ represents -C₂₋₆alkyl-NR^{8a}R^{8b};
wherein the C₂₋₆alkyl moiety in the R³ definition may be substituted with one, two or three substituents each independently selected from the group consisting of cyano, halo, -OH, and - O-C₁₋₄alkyl.

In an embodiment, the present disclosure relates to those compounds of Formula (I) and the pharmaceutically acceptable salts, and the solvates thereof, or any subgroup thereof as mentioned in any of the other embodiments, wherein
R³ represents -C₂₋₆alkyl-NR^{8a}R^{8b};
wherein the C₂₋₆alkyl moiety in the R³ definition may be substituted with one, two or three substituents each independently selected from the group consisting of cyano, halo and -O-C₁₋₄alkyl;
R^{8a} and R^{8b} are each independently selected from the group consisting of hydrogen; C₁₋₆alkyl; and C₁₋₆alkyl substituted with one, two or three substituents each independently selected from the group consisting of -OH, cyano, halo, -S(=O)₂-C₁₋₄alkyl, -O-C₁₋₄alkyl, - C(=O)-NR^{10a}R^{10b}, and -NR^{10c}-C(=O)-C₁₋₄alkyl.

In an embodiment, the present disclosure relates to those compounds of Formula (I) and the pharmaceutically acceptable salts, and the solvates thereof, or any subgroup thereof as mentioned in any of the other embodiments, wherein
R³ represents -C₂₋₆alkyl-NR^{8a}R^{8b};
wherein the C₂₋₆alkyl moiety in the R³ definition may be substituted with one, two or three substituents each independently selected from the group consisting of cyano, halo, -OH, and - O-C₁₋₄alkyl;
R^{8a} and R^{8b} are each independently selected from the group consisting of hydrogen; C₁₋₆alkyl; and C₁₋₆alkyl substituted with one, two or three substituents each independently selected from the group consisting of -OH, cyano, halo, -S(=O)₂-C₁₋₄alkyl, -O-C₁₋₄alkyl, - C(=O)-NR^{10a}R^{10b}, and -NR^{10c}-C(=O)-C₁₋₄alkyl.

In an embodiment, the present disclosure relates to those compounds of Formula (I) and the pharmaceutically acceptable salts, and the solvates thereof, or any subgroup thereof as mentioned in any of the other embodiments, wherein
R³ represents -C₂₋₆alkyl-NR^{8a}R^{8b};
wherein the C₂₋₆alkyl moiety in the R³ definition may be substituted with one, two or three substituents each independently selected from the group consisting of cyano, halo, -OH, and - O-C₁₋₄alkyl;
R^{8a} and R^{8b} are each independently selected from the group consisting of hydrogen; C₁₋₆alkyl; and C₁₋₆alkyl substituted with one, two or three substituents each independently selected from the group consisting of cyano, halo, -S(=O)₂-C₁₋₄alkyl, -O-C₁₋₄alkyl, and - C(=O)-NR^{10a}R^{10b}.

In an embodiment, the present disclosure relates to those compounds of Formula (I) and the pharmaceutically acceptable salts, and the solvates thereof, or any subgroup thereof as mentioned in any of the other embodiments, wherein
R³ represents -C₂₋₆alkyl-NR^{8a}R^{8b};
wherein the C₂₋₆alkyl moiety in the R³ definition may be substituted with one, two or three substituents each independently selected from the group consisting of cyano, halo and -O-C₁₋₄alkyl;
R^{8a} and R^{8b} are each independently selected from the group consisting of hydrogen; C₁₋₆alkyl; and C₁₋₆alkyl substituted with one, two or three substituents each independently selected from the group consisting of cyano, halo, -S(=O)₂-C₁₋₄alkyl, -O-C₁₋₄alkyl, and - C(=O)-NR^{10a}R^{10b}.

In an embodiment, the present disclosure relates to those compounds of Formula (I) and the pharmaceutically acceptable salts, and the solvates thereof, or any subgroup thereof as mentioned in any of the other embodiments, wherein
R³ represents -C₁₋₆alkyl-NR^{8a}R^{8b};
R^{8a} and R^{8b} are each independently selected from the group consisting of C₁₋₆alkyl; and C₁₋₆alkyl substituted with one, two or three substituents each independently selected from the group consisting of -OH, cyano, halo, -S(=O)₂-C₁₋₄alkyl, -O-C₁₋₄alkyl, and -C(=O)-NR^{10a}R^{10b}.

In an embodiment, the present disclosure relates to those compounds of Formula (I) and the pharmaceutically acceptable salts, and the solvates thereof, or any subgroup thereof as mentioned in any of the other embodiments, wherein
R³ represents -C₂₋₆alkyl-NR^{8a}R^{8b};
wherein the C₂₋₆alkyl moiety in the R³ definition may be substituted with one, two or three substituents each independently selected from the group consisting of cyano, halo, -OH, and - O-C₁₋₄alkyl;
R^{8a} and R^{8b} are each independently selected from the group consisting of hydrogen; C₁₋₆alkyl; and C₁₋₆alkyl substituted with one, two or three substituents each independently selected from the group consisting of cyano, halo, -S(=O)₂-C₁₋₄alkyl, -O-C₁₋₄alkyl, -C(=O)-NR^{10a}R^{10b}, and -NR^{10c}-C(=O)-C₁₋₄alkyl.

In an embodiment, the present disclosure relates to those compounds of Formula (I) and the pharmaceutically acceptable salts, and the solvates thereof, or any subgroup thereof as mentioned in any of the other embodiments, wherein
R³ represents -C₂₋₆alkyl-NR^{8a}R^{8b};
wherein the C₂₋₆alkyl moiety in the R³ definition may be substituted with one, two or three substituents each independently selected from the group consisting of cyano, halo and -O-C₁₋₄alkyl;
R^{8a} and R^{8b} are each independently selected from the group consisting of hydrogen; C₁₋₆alkyl; and C₁₋₆alkyl substituted with one, two or three substituents each independently selected from the group consisting of cyano, halo, -S(=O)₂-C₁₋₄alkyl, -O-C₁₋₄alkyl, -C(=O)-NR^{10a}R^{10b}, and -NR^{10c}-C(=O)-C₁₋₄alkyl.

In an embodiment, the present disclosure relates to those compounds of Formula (I) and the pharmaceutically acceptable salts, and the solvates thereof, or any subgroup thereof as mentioned in any of the other embodiments, wherein
R³ represents -C₁₋₆alkyl-NR^{8a}R^{8b};
R^{8a} and R^{8b} are each independently selected from the group consisting of C₁₋₆alkyl; and C₁₋₆alkyl substituted with one, two or three substituents each independently selected from the group consisting of -OH, cyano, halo, -S(=O)₂-C₁₋₄alkyl, -O-C₁₋₄alkyl, -C(=O)-NR^{10a}R^{10b}, and -NR^{10c}-C(=O)-C₁₋₄alkyl.

In an embodiment, the present disclosure relates to those compounds of Formula (I) and the pharmaceutically acceptable salts, and the solvates thereof, or any subgroup thereof as mentioned in any of the other embodiments, wherein
R³ represents -C₁₋₆alkyl-NR^{8a}R^{8b};
R^{8a} represents C₁₋₆alkyl; and
R^{8b} represents C₁₋₆alkyl substituted with one -O-C₁₋₄alkyl.

In an embodiment, the present disclosure relates to those compounds of Formula (I) and the pharmaceutically acceptable salts, and the solvates thereof, or any subgroup thereof as mentioned in any of the other embodiments, wherein
R³ represents -C₁₋₆alkyl-NR^{8a}R^{8b}, -C₁₋₆alkyl-C(=O)-NR^{9a}R^{9b}, -C₁₋₆alkyl-OH, or -C₁₋₆alkyl-NR¹¹-C(=O)-O-C₁₋₄alkyl-O-C(=O)-C₁₋₄alkyl;
wherein each of the C₁₋₄alkyl or C₁₋₆alkyl moieties in the R³ definitions independently of each other may be substituted with one, two or three substituents each independently selected from the group consisting of cyano, halo or -O-C₁₋₄alkyl.

In an embodiment, the present disclosure relates to those compounds of Formula (I) and the pharmaceutically acceptable salts, and the solvates thereof, or any subgroup thereof as mentioned in any of the other embodiments, wherein
R³ represents -C₁₋₆alkyl-NR^{8a}R^{8b}, -C₁₋₆alkyl-C(=O)-NR^{9a}R^{9b}, or -C₁₋₆alkyl-NR¹¹-C(=O)-O-C₁₋₄alkyl-O-C(=O)-C₁₋₄alkyl;
wherein each of the C₁₋₄alkyl or C₁₋₆alkyl moieties in the R³ definitions independently of each other may be substituted with one, two or three substituents each independently selected from the group consisting of cyano, halo, -OH, and -O-C₁₋₄alkyl.

In an embodiment, the present disclosure relates to those compounds of Formula (I) and the pharmaceutically acceptable salts, and the solvates thereof, or any subgroup thereof as mentioned in any of the other embodiments, wherein
R³ represents -CH₂-CH₂-CH₂-NR^{8a}R^{8b}.

In an embodiment, the present disclosure relates to those compounds of Formula (I) and the pharmaceutically acceptable salts, and the solvates thereof, or any subgroup thereof as mentioned in any of the other embodiments, wherein
R³ represents -CH₂-CH₂-CH₂-NR^{8a}R^{8b};
R^{8a} represents methyl; and
R^{8b} represents -CH₂-CH₂-OCH₃.

In an embodiment, the present disclosure relates to those compounds of Formula (I) and the pharmaceutically acceptable salts, and the solvates thereof, or any subgroup thereof as mentioned in any of the other embodiments, wherein C₁₋₆alkyl in the R³ definition
-C₁₋₆alkyl-NR^{8a}R^{8b} is limited to -CH₂-CH₂-CH₂-.

In an embodiment, the present disclosure relates to those compounds of Formula (I) and the pharmaceutically acceptable salts, and the solvates thereof, or any subgroup thereof as mentioned in any of the other embodiments, wherein the compounds of Formula (I) are restricted to compounds of Formula (I-y): 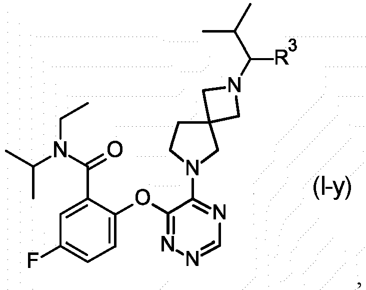 wherein R³ is as defined for the compounds of Formula (I) or any subgroup thereof as mentioned in any of the other embodiments.

In Formula (I-y) n1 is 1, n2 is 2, n3 is 1, and n4 is 1.

In an embodiment the compound of Formula (I) is 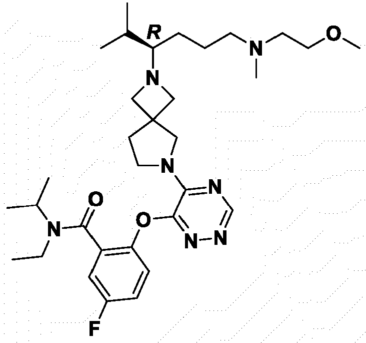 and pharmaceutically acceptable addition salts, and solvates thereof.

In an embodiment the compound of Formula (I) is

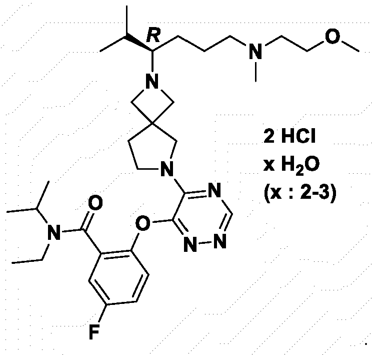

In an embodiment the compound of Formula (I) is

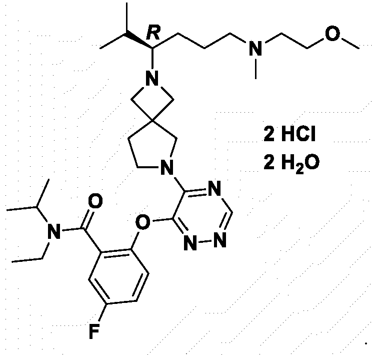

In an embodiment the compound of Formula (I) is

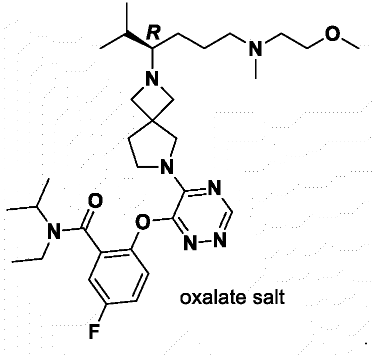

In an embodiment, the present disclosure relates to a subgroup of Formula (I) as defined in the general reaction schemes.

In an embodiment the compound of Formula (I) is selected from the group consisting of any of the exemplified compounds,
tautomers and stereoisomeric forms thereof,
and the free bases, any pharmaceutically acceptable salts, and the solvates thereof.

All possible combinations of the above indicated embodiments are considered to be embraced within the scope of the disclosure.

In another embodiment, the present disclosure relates to the intermediate 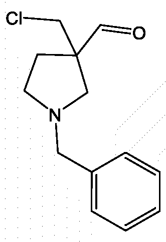
tautomers and stereoisomeric forms thereof,
and any pharmaceutically acceptable salts, and the solvates thereof.

In another embodiment, the present disclosure relates to a process for the preparation of an intermediate comprising the following steps: 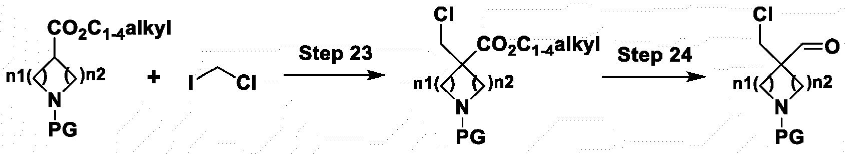
wherein PG is a suitable protecting group such as benzyl;
wherein n1 and n2 are as defined for formula (I);
Step 23: at a suitable temperature such as for example from -78 °C to -25 °C, in the presence of suitable bases such as for example DIEA and n-BuLi, in a suitable solvent such as for example THF;
Step 24: at a suitable temperature such as for example between -55°C and -65 °C, in the presence of suitable reducing agent such as for example DIBAL-H, in a suitable solvent such as for example toluene, conducted in a suitable flow chemistry system.

In another embodiment, the present disclosure relates to a process for the preparation of an intermediate comprising the following steps: 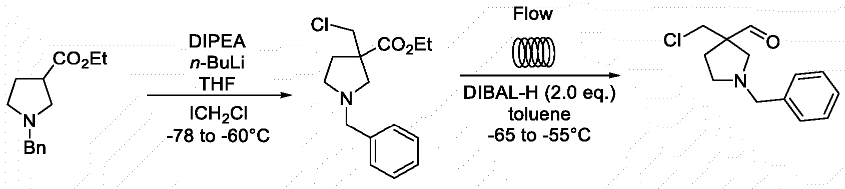
first reaction at a suitable temperature such as for example from -78 °C to -25 °C, in the presence of suitable bases such as for example DIEA and n-BuLi, in a suitable solvent such as for example THF;
then, reaction at a suitable temperature such as for example between -55°C and -65 °C, in the presence of suitable reducing agent such as for example DIBAL-H, in a suitable solvent such as for example toluene, conducted in a suitable flow chemistry system.

In another embodiment, the present disclosure relates to a process for the preparation of an intermediate comprising the following steps: 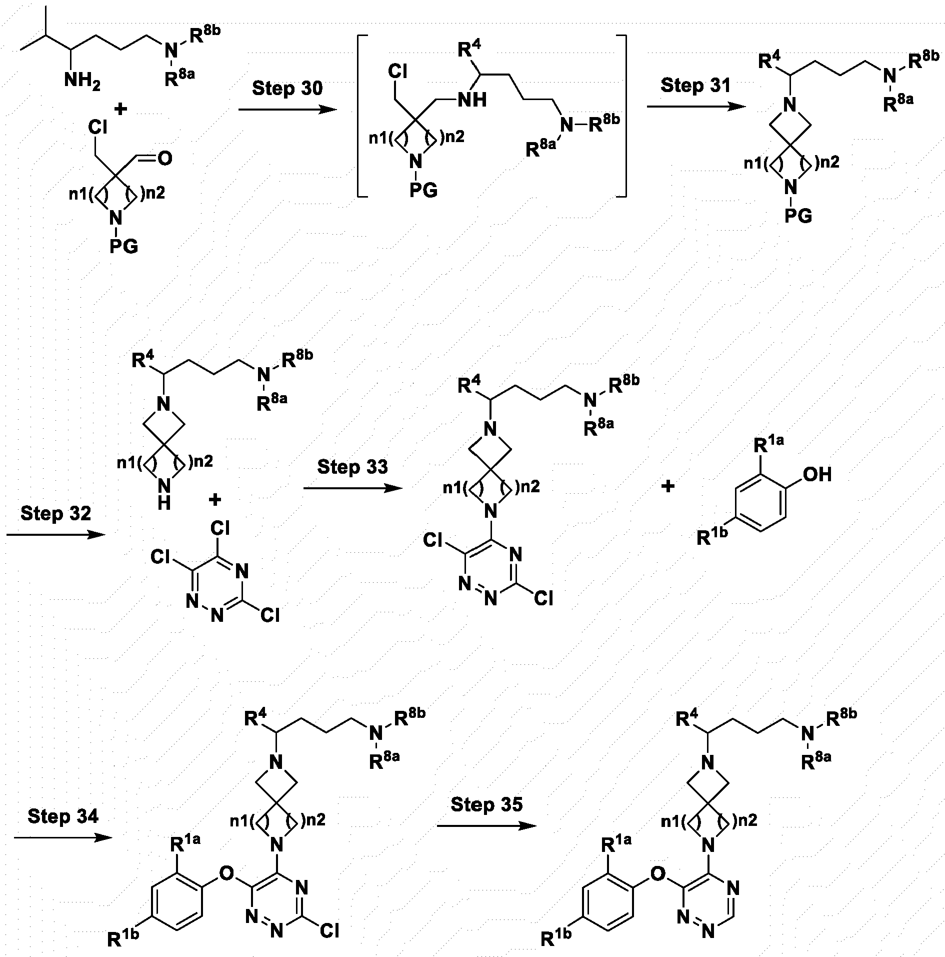
PG is a suitable protecting group such as benzyl;
other variables are as defined for formula (I).

In another embodiment, the present disclosure relates to a process for the preparation of an intermediate comprising the following steps: 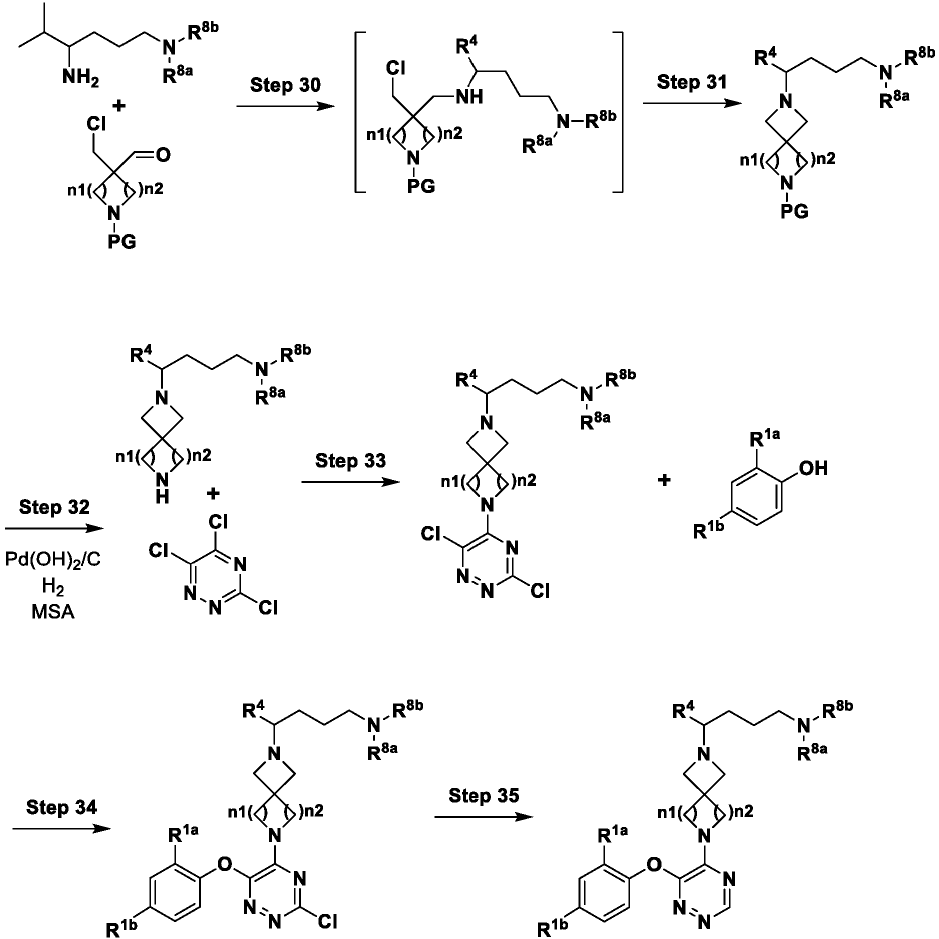
PG is a suitable protecting group such as benzyl;
other variables are as defined for formula (I);
Step 30: at a suitable temperature such as for example from 5 °C to 30 °C, in the presence of a suitable base such as for example TEA, in the presence of suitable reducing agent such as for example NaBH(OAc)₃, in a suitable solvent such as for example toluene;
Step 31: at a suitable temperature such as for example from 50 °C to 55 °C, in the presence of a suitable base such as for example K₂HPO₄, in a suitable solvent such as for example H₂O;
Step 32: at a suitable temperature such as for example from -5 °C to 45 °C, under a hydrogen atmosphere within a suitable pressure range such as for example from 0.27 to 0.40 MPa, in the presence of palladium hydroxide on carbon, in the presence of MSA in a suitable solvent such as EtOH;
Step 33: at a suitable temperature such as for example from -50 °C to -40 °C, in the presence of suitable base such as for example TEA, in a suitable solvent such as 2-methyltetrahydrofuran; Step 34: at a suitable temperature such as for example from 20 °C to 30 °C, in the presence of suitable base such as for example TMG, in a suitable solvent such as 2-methyltetrahydrofuran; Step 35: at a suitable temperature such as for example from 20 °C to 30 °C, under a hydrogen atmosphere within a suitable pressure range such as for example from 0.20 to 0.30 Mpa, in the presence of a suitable catalyst such as for example palladium on carbon, in a suitable solvent such as MeOH.

In another embodiment, the present disclosure relates to a process for the preparation of a compound comprising the following steps:

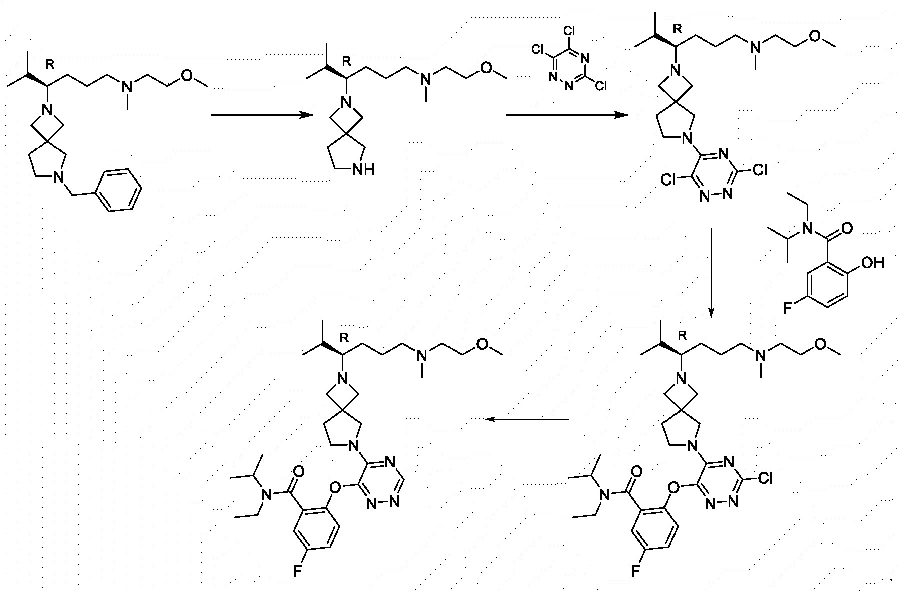

In another embodiment, the present disclosure relates to a process for the preparation of a compound comprising the following steps:

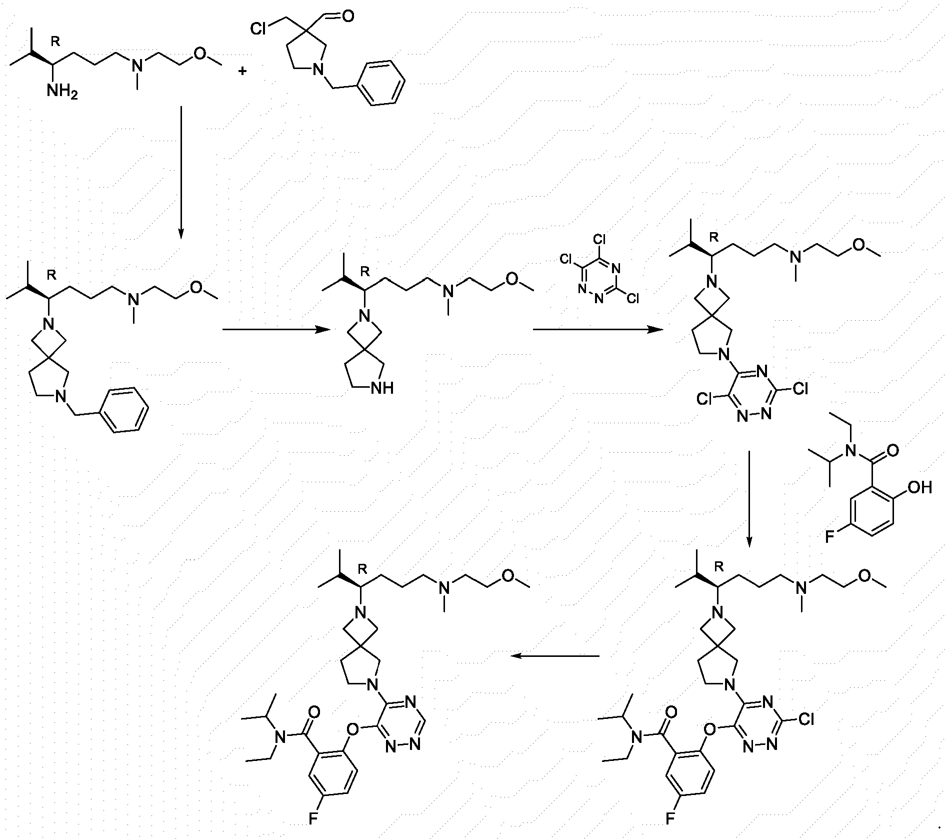

In another embodiment, the present disclosure relates to a process for the preparation of a compound comprising the following steps:

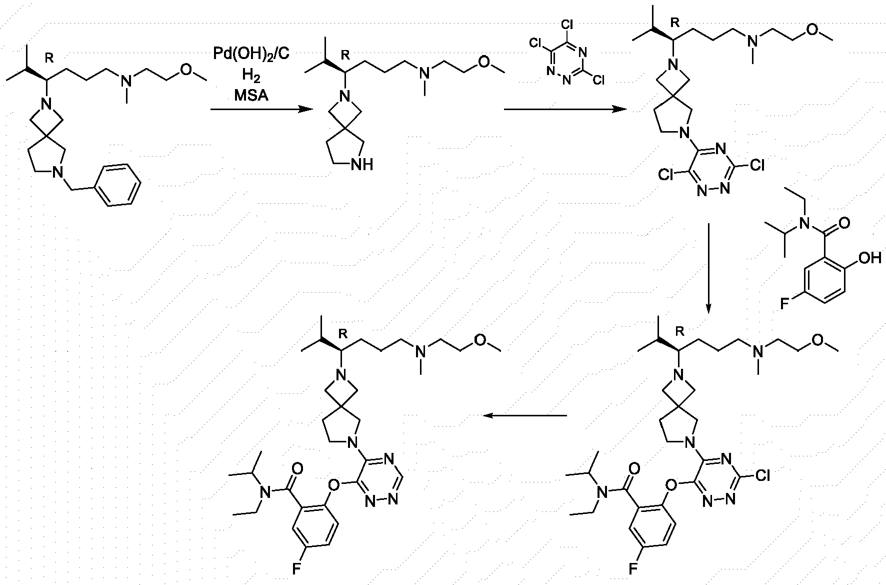

In another embodiment, the present disclosure relates to a process for the preparation of a compound comprising the following steps:

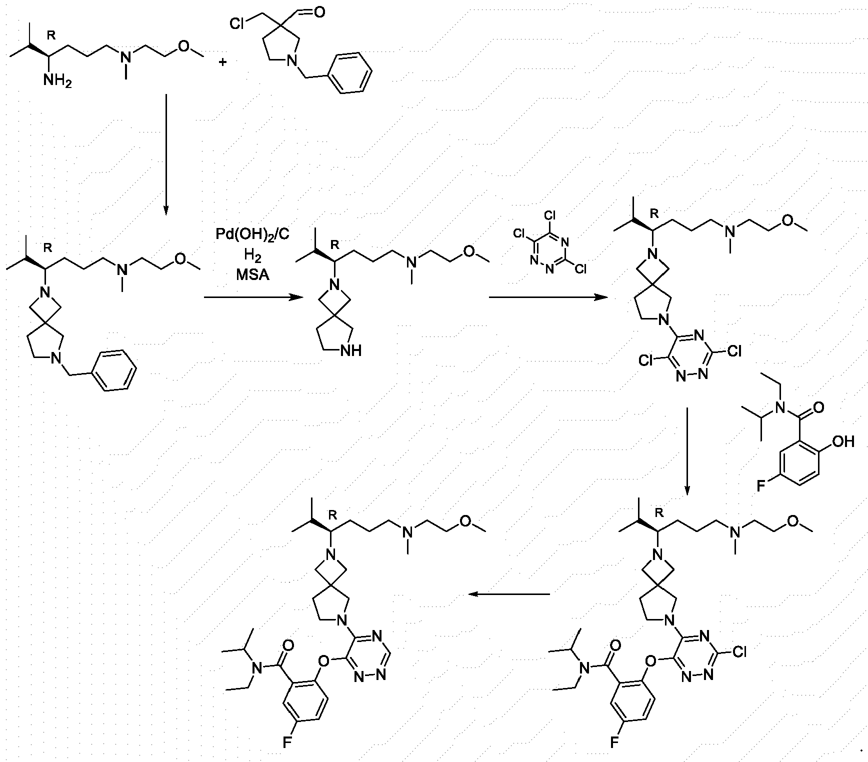

In another embodiment, the present disclosure relates to a process for the preparation of a compound comprising the following steps: 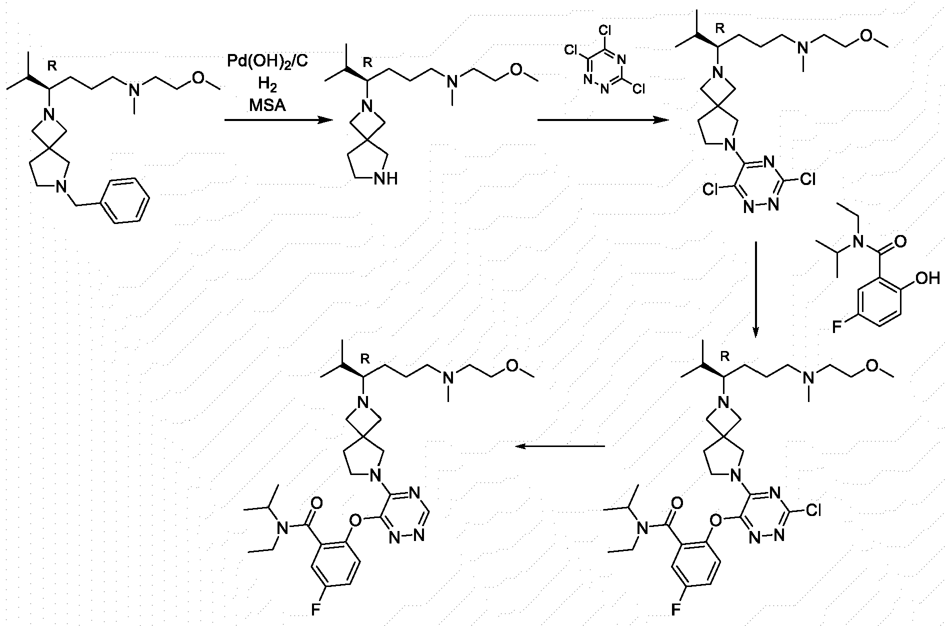
in a first step, at a suitable temperature such as for example from -5 °C to 45 °C, under a hydrogen atmosphere within a suitable pressure range such as for example from 0.27 to 0.40 MPa, in the presence of palladium hydroxide on carbon, in the presence of MSA in a suitable solvent such as EtOH;
in a next step at a suitable temperature such as for example from -50 °C to -40 °C, in the presence of suitable base such as for example TEA, in a suitable solvent such as 2-methyltetrahydrofuran;
in a next step at a suitable temperature such as for example from 20 °C to 30 °C, in the presence of suitable base such as for example TMG, in a suitable solvent such as 2-methyltetrahydrofuran; in a next step at a suitable temperature such as for example from 20 °C to 30 °C, under a hydrogen atmosphere within a suitable pressure range such as for example from 0.20 to 0.30 Mpa, in the presence of a suitable catalyst such as for example palladium on carbon, in a suitable solvent such as MeOH.

In another embodiment, the present disclosure relates to a process for the preparation of a compound comprising the following steps:

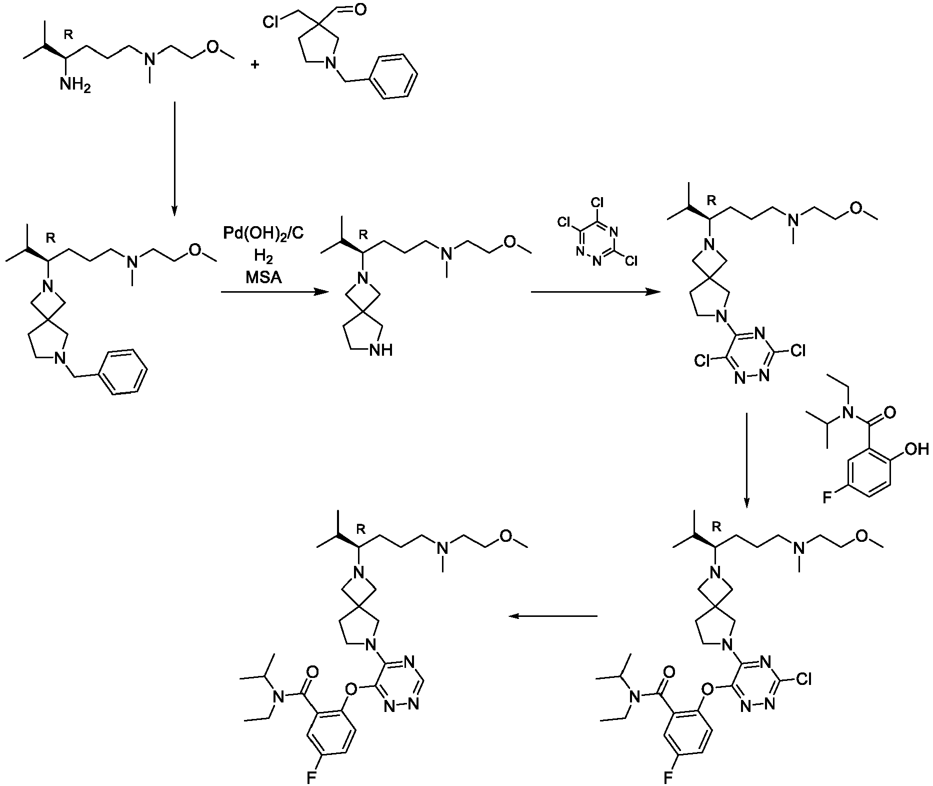

In a first step first at a suitable temperature such as for example from 5 °C to 30 °C, in the presence of a suitable base such as for example TEA, in the presence of suitable reducing agent such as for example NaBH(OAc)₃, in a suitable solvent such as for example toluene; and then at a suitable temperature such as for example from 50 °C to 55 °C, in the presence of a suitable base such as for example K₂HPO₄, in a suitable solvent such as for example H₂O;
in a next step, at a suitable temperature such as for example from -5 °C to 45 °C, under a hydrogen atmosphere within a suitable pressure range such as for example from 0.27 to 0.40 MPa, in the presence of palladium hydroxide on carbon, in the presence of MSA in a suitable solvent such as EtOH;
in a next step at a suitable temperature such as for example from -50 °C to -40 °C, in the presence of suitable base such as for example TEA, in a suitable solvent such as 2-methyltetrahydrofuran;
in a next step at a suitable temperature such as for example from 20 °C to 30 °C, in the presence of suitable base such as for example TMG, in a suitable solvent such as 2-methyltetrahydrofuran; in a next step at a suitable temperature such as for example from 20 °C to 30 °C, under a hydrogen atmosphere within a suitable pressure range such as for example from 0.20 to 0.30 Mpa, in the presence of a suitable catalyst such as for example palladium on carbon, in a suitable solvent such as MeOH.

The following embodiments describe the claimed invention:
1. A compound wherein the compound is 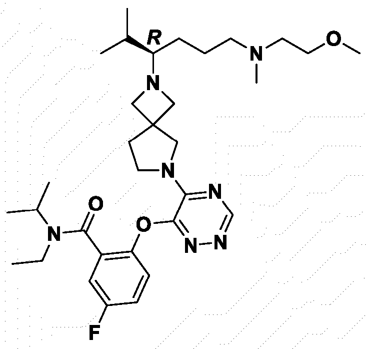 or a pharmaceutically acceptable salt or a solvate thereof.
2. The compound according to embodiment 1, wherein the compound is a pharmaceutically acceptable salt.
3. The compound according to embodiment 1, wherein the compound is a solvate.
4. The compound according to embodiment 1, wherein the compound is a solvate of a pharmaceutically acceptable salt.
5. The compound according to embodiment 1, wherein the compound is 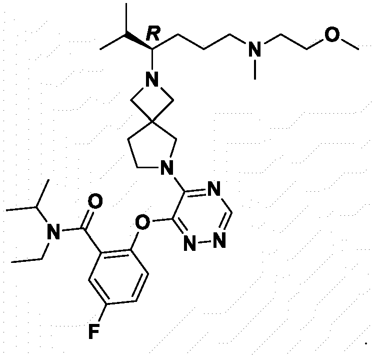
6. The compound according to embodiment 1, wherein the compound is 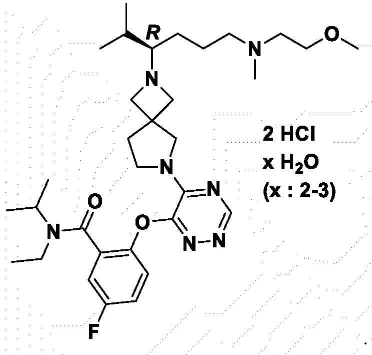
7. The compound according to embodiment 6, wherein the compound is 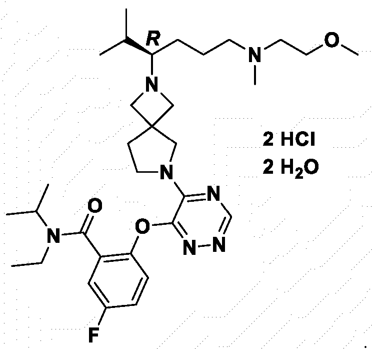
8. The compound according to embodiment 1, wherein the compound is 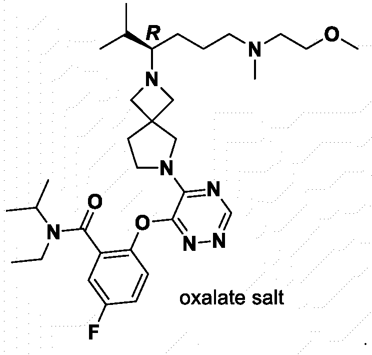
9. A pharmaceutical composition comprising a compound as defined in any one of embodiments 1 to 8 and a pharmaceutically acceptable carrier or diluent.
10. A process for preparing a pharmaceutical composition as defined in embodiment 9 comprising mixing a pharmaceutically acceptable carrier with a therapeutically effective amount of a compound according to any one of embodiments 1 to 8.
11. A compound as defined in any one of embodiments 1 to 8 or a pharmaceutical composition as defined in claim 9 for use as a medicament.
12. A compound as defined in any one of embodiments 1 to 8 or a pharmaceutical composition as defined in embodiment 9 for use in the prevention or treatment of cancer.
13. A compound as defined in any one of embodiments 1 to 8 or a pharmaceutical composition as defined in embodiment 9 for use in the prevention or treatment of leukemia, myelodysplastic syndrome (MDS), or myeloproliferative neoplasms (MPN).
14. The compound or pharmaceutical composition for use according to embodiment 13, in the treatment of leukemia wherein the leukemia is (*NPM1*)-mutated leukemia.
15. The compound or pharmaceutical composition for use according to embodiment 13, in the prevention or treatment of leukemia wherein the leukemia is selected from acute leukemias, chronic leukemias, myeloid leukemias, myelogenous leukemias, lymphoblastic leukemias, and lymphocytic leukemias.
16. The compound or pharmaceutical composition for use according to embodiment 13, in the prevention or treatment of leukemia wherein the leukemia is selected from Acute myelogenous leukemias (AML), Chronic myelogenous leukemias (CML), Acute lymphoblastic leukemias (ALL), Chronic lymphocytic leukemias (CLL), T cell prolymphocytic leukemias (T-PLL), Large granular lymphocytic leukemia, Hairy cell leukemia (HCL), *KMT2A*-rearranged leukemias, *KMT2A*-PTD leukemias, *KMT2A* amplified leukemias, *KMT2A*-positive leukemias, and leukemias exhibiting *HOX*/*MEIS1* gene expression signatures.
17. The compound or pharmaceutical composition for use according to embodiment 13, in the treatment of leukemia wherein the leukemia is an acute leukemia.
18. The compound or pharmaceutical composition for use according to embodiment 17, wherein the acute leukemia is AML.
19. The compound or pharmaceutical composition for use according to embodiment 17, wherein the acute leukemia is ALL.
20. The compound or pharmaceutical composition for use according to any one of embodiments 17-19, wherein the acute leukemia has KMT2A gene alterations or *NPM1* mutations.
21. The compound or pharmaceutical composition for use according to any one of embodiments 17-19, wherein the acute leukemia has KMT2A gene rearrangements.
22. The compound or pharmaceutical composition for use according to any one of embodiments 17-19, wherein the acute leukemia has *NPM1* mutations.
23. The compound or pharmaceutical composition for use according to embodiment 22, wherein the *NPM1* mutation is *NPM1c.*
24. The compound or pharmaceutical composition for use according to embodiment 14, wherein the (*NPM1*)-mutated leukemia is *NPM1c.*

### METHODS FOR THE PREPARATION OF COMPOUNDS OF FORMULA (I)

In this section, as in all other sections unless the context indicates otherwise, references to Formula (I) also include all other sub-groups and examples thereof as defined herein.

The general preparation of some typical examples of the compounds of Formula (I) is described hereunder and in the specific examples, and are generally prepared from starting materials which are either commercially available or prepared by standard synthetic processes commonly used by those skilled in the art of organic chemistry. The following schemes are only meant to represent examples of the disclosure and are in no way meant to be a limit of the disclosure.

Alternatively, compounds of the present disclosure may also be prepared by analogous reaction protocols as described in the general schemes below, combined with standard synthetic processes commonly used by those skilled in the art.

The skilled person will realize that in the reactions described in the Schemes, although this is not always explicitly shown, it may be necessary to protect reactive functional groups (for example hydroxy, amino, or carboxy groups) where these are desired in the final product, to avoid their unwanted participation in the reactions. In general, conventional protecting groups (PG) can be used in accordance with standard practice. The protecting groups may be removed at a convenient subsequent stage using methods known from the art.

The skilled person will realize that in the reactions described in the Schemes, it may be advisable or necessary to perform the reaction under an inert atmosphere, such as for example under N₂-gas atmosphere.

It will be apparent for the skilled person that it may be necessary to cool the reaction mixture before reaction work-up (refers to the series of manipulations required to isolate and purify the product(s) of a chemical reaction such as for example quenching, column chromatography, extraction).

The skilled person will realize that heating the reaction mixture under stirring may enhance the reaction outcome. In some reactions microwave heating may be used instead of conventional heating to shorten the overall reaction time.

The skilled person will realize that another sequence of the chemical reactions shown in the Schemes below, may also result in the desired compound of Formula (I).

The skilled person will realize that intermediates and final compounds shown in the Schemes below may be further functionalized according to methods well-known by the person skilled in the art. The intermediates and compounds described herein can be isolated in free form or as a salt, or a solvate thereof. The intermediates and compounds described herein may be synthesized in the form of mixtures of tautomers and stereoisomeric forms that can be separated from one another following art-known resolution procedures.

### General Synthetic Schemes

All abbreviations used in the general schemes are as defined in the Table in the part Examples. Variables are as defined in the scope or as specifically defined in the general Schemes.

### Part A) Schemes 1a, 1b, 1c, 2a, 2b and 3

### Rn = C₁₋₆alkyl-NR^{8a}PG or C₁₋₆alkyl-OPG or C₁₋₆alkyl-C(=O)OR^{9a}, PG = protecting group

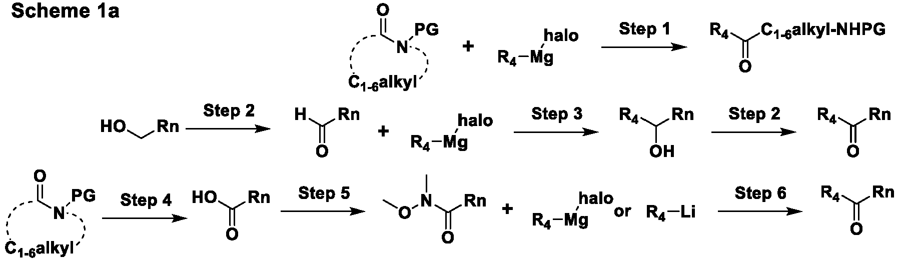

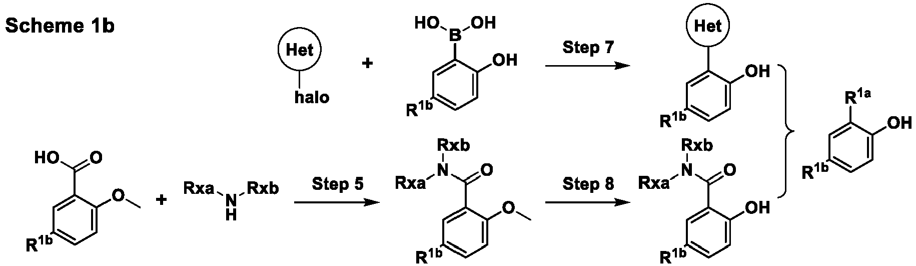

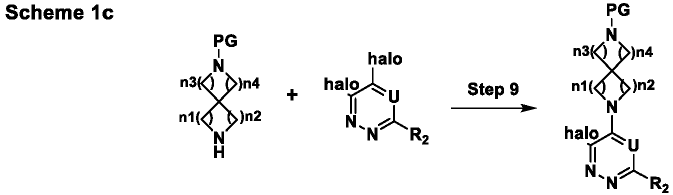

In Scheme 1a, 1b and 1c the following reaction conditions apply:
Step 1: at a suitable temperature such as for example -70°C, in the presence of a suitable base such as for example TMEDA and a suitable organometallic reagent such as for example isopropylmagnesium bromide, in a suitable solvent such as for example THF;
Step 2: at a suitable temperature such as for example from 0 °C to RT, in the presence of a suitable oxidative reagent such as for example DMP, in a suitable solvent such as for example DCM;
Step 3: at a suitable temperature such as for example from -20°C to RT, in the presence of a suitable organometallic reagent such as for example isopropylmagnesium bromide, in a suitable solvent such as for example THF;
Step 4: at a suitable temperature such as for example 80°C, in the presence of a suitable base such as for example NaOH, in suitable solvents such as for example THF and H₂O;
Step 5: at a suitable temperature such as for example RT, in the presence of suitable amide condensation reagents such as for example EDCI and HOBt, in the presence of a suitable base such as for example NMM, in a suitable solvent such as for example DCM;
Step 6: at a suitable temperature such as for example -70°C, in the presence of a suitable organometallic reagent such as for example isopropyllithium, in a suitable solvent such as for example THF;
Step 7: at a suitable temperature such as for example 90 °C, in the presence of a suitable organometallic catalyst such as for example Pd(dppf)Cl₂, in the presence of a suitable base such as for example Na₂CO₃, in suitable solvents such as for example 1,4-dioxane and H₂O; Step 8: at a suitable temperature such as for example from 0 °C to RT, in the presence of a suitable Lewis acid such as for example BBr₃, in a suitable solvent such as for example DCM; Step 9: at a suitable temperature such as for example from -78 °C to 40 °C, in particular from 0 °C to RT, in the presence of a suitable base such as for example TEA, DBU or K₂CO₃, in a suitable solvent such as for example DCM, THF or DMF;

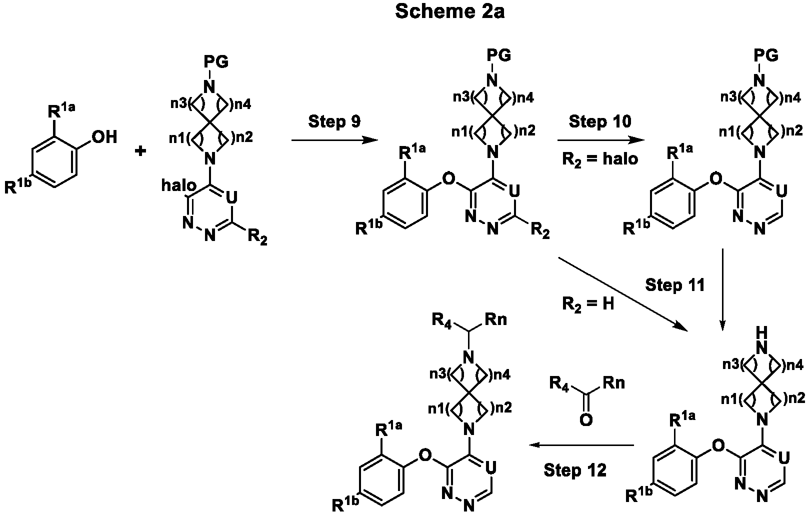

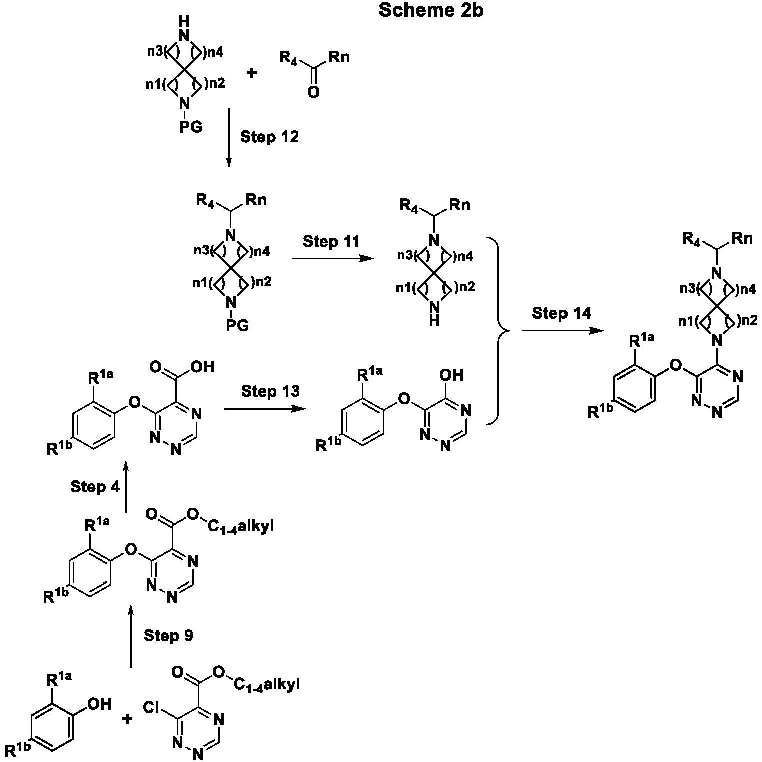

In Scheme 2a and 2b, the following reaction conditions apply:
Step 9: See Step 9 in Scheme 1;
Step 10: at a suitable temperature such as for example RT, in the presence of a suitable catalyst such as for example Pd/C, in the presence of a suitable reductive reagent such as for example H₂, optionally in the presence of a suitable base such as for example TEA, in a suitable solvent such as for example THF;
Alternatively, at a suitable temperature such as RT, in the presence of a suitable catalyst such as for example Pd(dppf)Cl₂·DCM complex, a suitable reducing agent such NaBH₄, a suitable base such as for example TMEDA, in a suitable solvent such as for example THF.

Step 11: for N deprotection, at a suitable temperature such as for example RT, in the presence of a suitable acid as for example TFA, in a suitable solvent such as for example DCM; for O deprotection, at a suitable temperature such as for example RT, in the presence of a suitable acid as for example 4-methylbenzenesulfonic acid, in a suitable solvent such as for example MeOH;
Step 12: at a suitable temperature such as for example 80 °C, optionally in the presence of a suitable Lewis acid such as for example ZnCl₂, in the presence of a suitable reductive reagent such as for example NaBH₃CN, in a suitable solvent such as for example MeOH;
Step 13: at a suitable temperature such as for example RT, in the presence of a suitable organometallic catalyst such as for example Ag(Phen)₂OTf, in the presence of a suitable brominating reagent such as for example 1,3-dibromo-1,3,5-triazinane-2,4,6-trione, in a suitable solvent such as for example DCE;
Step 14: at a suitable temperature such as for example RT, in the presence of a suitable chlorinating reagent such as for example oxalyl chloride, in the presence of DMF, in a suitable solvent such as for example DCM.

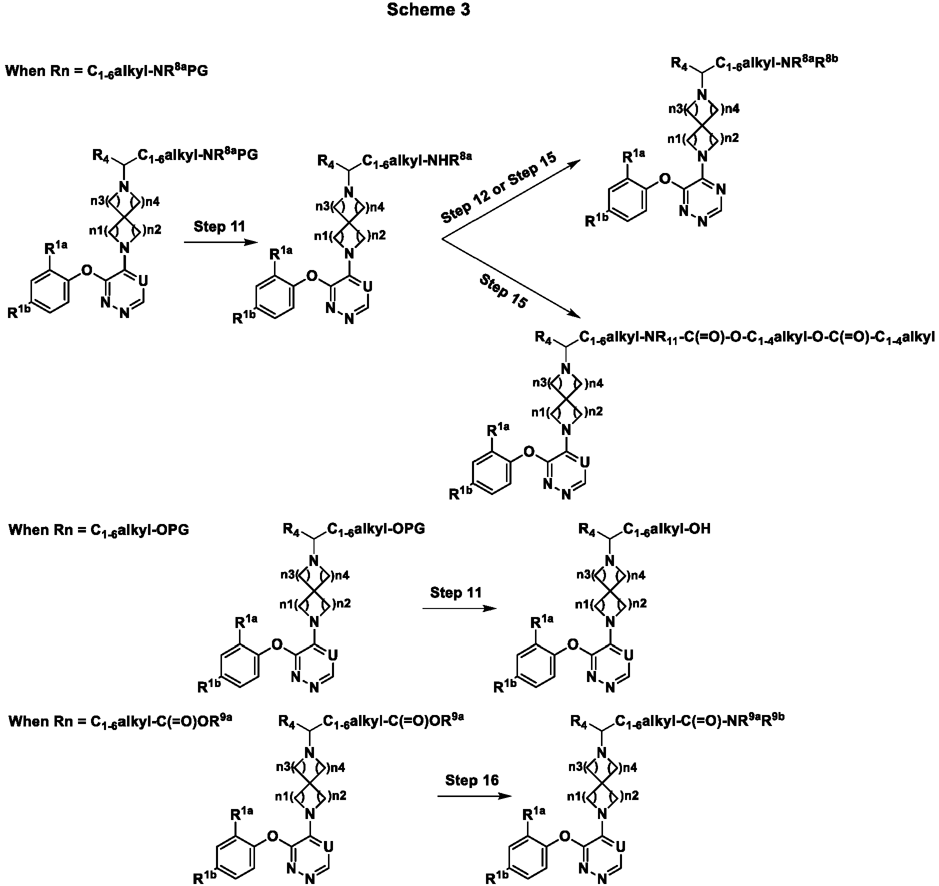

In Scheme 3, the following reaction conditions apply:
Step 11-12: See Step 11-12 in Scheme 2;
Step 15: at a suitable temperature such as for example 80 °C, in the presence of a suitable base such as for example Cs₂CO₃, in suitable solvent such as for example DMF;
Step 16: at a suitable temperature such as for example 40 °C, in the presence of a suitable base such as for example ammonia, in suitable solvent such as for example 1,4-dioxane.

### Part B) Schemes 4, 5, 6, 7, 8, 9, 10, 11 and 12

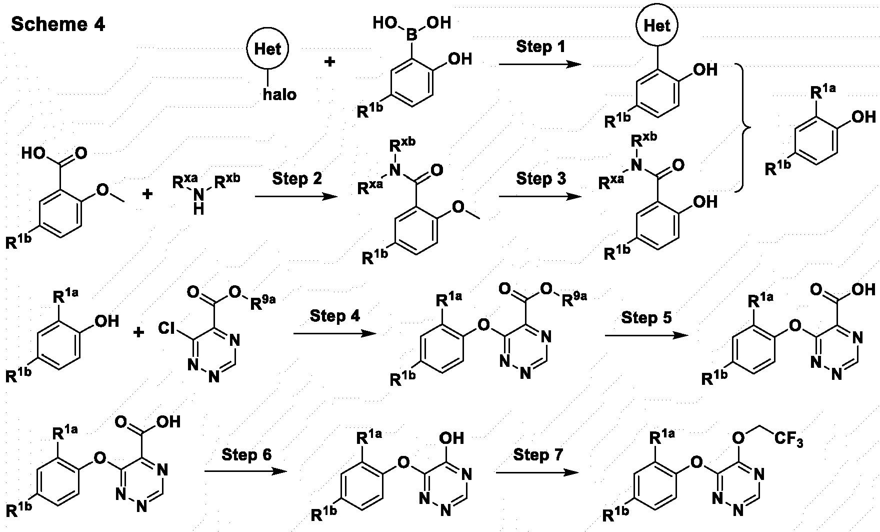

In Scheme 4, the following reaction conditions apply:
Step 1: at a suitable temperature such as for example 90 °C, in the presence of a suitable organometallic catalyst such as for example Pd(dppf)Cl₂, in the presence of a suitable base such as for example Na₂CO₃, in suitable solvents such as for example 1,4-dioxane and H₂O;
Step 2: at a suitable temperature such as for example RT, in the presence of suitable amide condensation reagent such as for example HATU, in the presence of a suitable base such as for example DIEA, in a suitable solvent such as for example DCM;
Step 3: at a suitable temperature such as for example from -78 °C to RT, in the presence of a suitable Lewis acid such as for example BBr₃, in a suitable solvent such as for example DCM;
Step 4: at a suitable temperature such as for example from -78 °C to 40 °C, in particular from 0 °C to RT, in the presence of a suitable base such as for example TEA, DBU or K₂CO₃, in a suitable solvent such as for example DCM, THF or DMF;
Step 5: at a suitable temperature such as for example RT, in the presence of a suitable base such as for example LiOH·H₂O, in suitable solvents such as for example THF and H₂O;
Step 6: at a suitable temperature such as for example RT, in the presence of a suitable organometallic catalyst such as for example Ag(Phen)₂OTf, in the presence of a suitable brominating reagent such as for example 1,3-dibromo-1,3,5-triazinane-2,4,6-trione, in a suitable solvent such as for example DCE;
Step 7: at a suitable temperature such as for example RT, in the presence of a suitable brominating reagent such as 1,3-dibromo-1,3,5-triazinane-2,4,6-trione, in the presence of 2,2,2-trifluoroethan-1-ol as solvent.

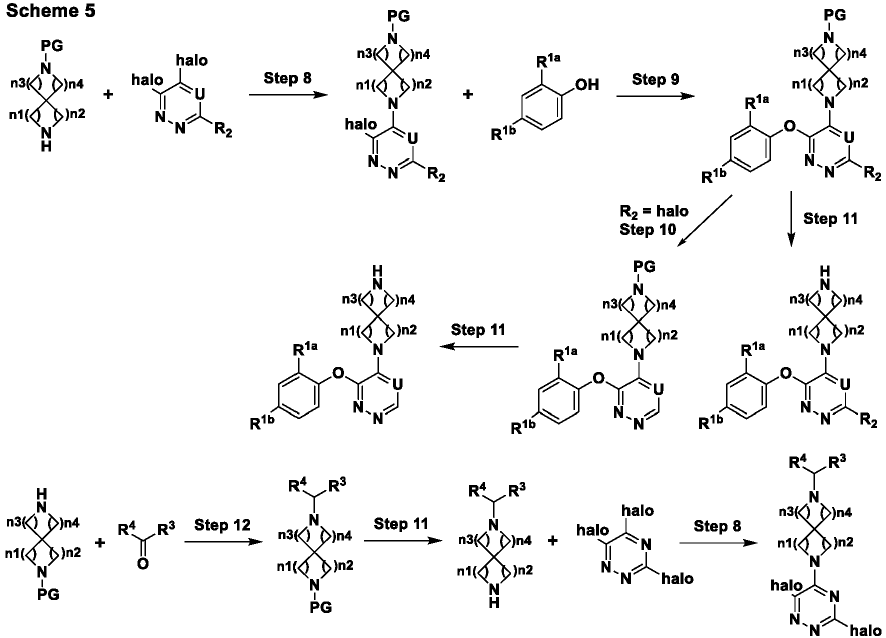

In Scheme 5, the following reaction conditions apply:
Step 8: at a suitable temperature such as for example from -78 °C to 40 °C, in particular from 0 °C to RT, in the presence of a suitable base such as for example TEA, DBU or K₂CO₃, in a suitable solvent such as for example DCM, THF or DMF;
Step 9: at a suitable temperature such as for example from -78 °C to 40 °C, in particular from 0 °C to RT, in the presence of a suitable base such as for example TEA, DBU or K₂CO₃, in a suitable solvent such as for example DCM, THF or DMF;
Step 10: at a suitable temperature such as for example RT, in the presence of a suitable organometallic catalyst as for example Pd/C and a suitable base as for example TEA, in a suitable solvent such as for example MeOH under H₂ atmosphere;
Step 11: When PG is Boc, at a suitable temperature such as for example RT, in the presence of a suitable acid as for example TFA, in a suitable solvent such as for example DCM.

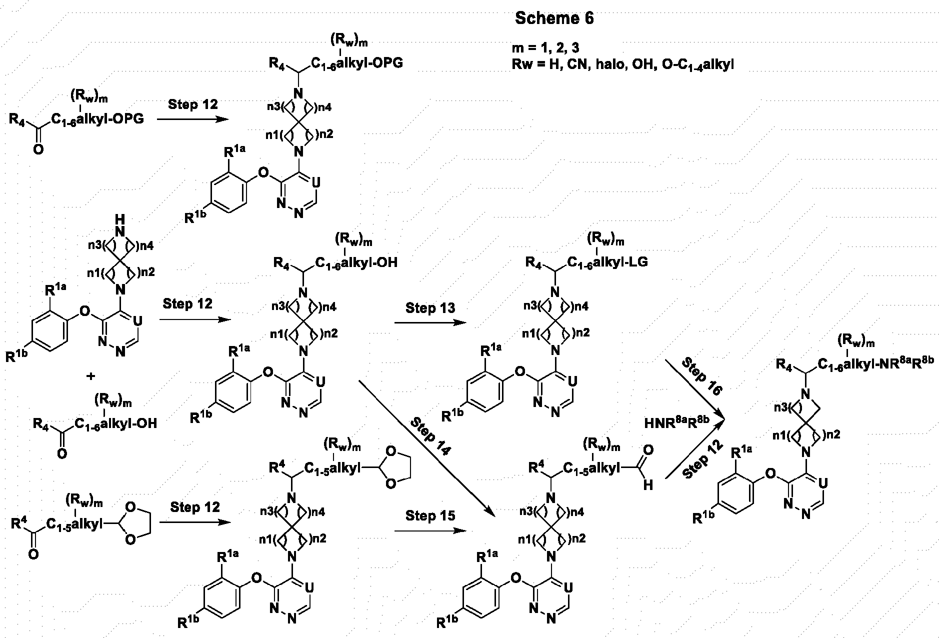

In Scheme 6, the following reaction conditions apply:
Step 12: reductive amination condition, at a suitable temperature such as for example from RT to 80 °C, in the presence or absence of a suitable Lewis acid such as for example ZnCl₂ or an acid for example AcOH, in the presence of a suitable reducing agent such as for example NaBH₃CN, in a suitable solvent such as for example MeOH;
Step 13: at a suitable temperature such as for example 0 °C, in the presence of a suitable electrophile as for example MsCl, in the presence of a suitable base such as for example TEA, in a suitable solvent such as for example DCM;
Step 14: at a suitable temperature such as for example from 0 °C to RT, in the presence of a suitable oxidizing agent as for example DMP, in a suitable solvent such as for example DCM; Step 15: at a suitable temperature such as for example 50 °C, in the presence of a suitable acid as for example HCl, in a suitable solvent such as for example ACN;
Step 16: at a suitable temperature such as for example RT, in the presence or absence of a suitable base as for example TEA, in a suitable solvent such as for example THF.

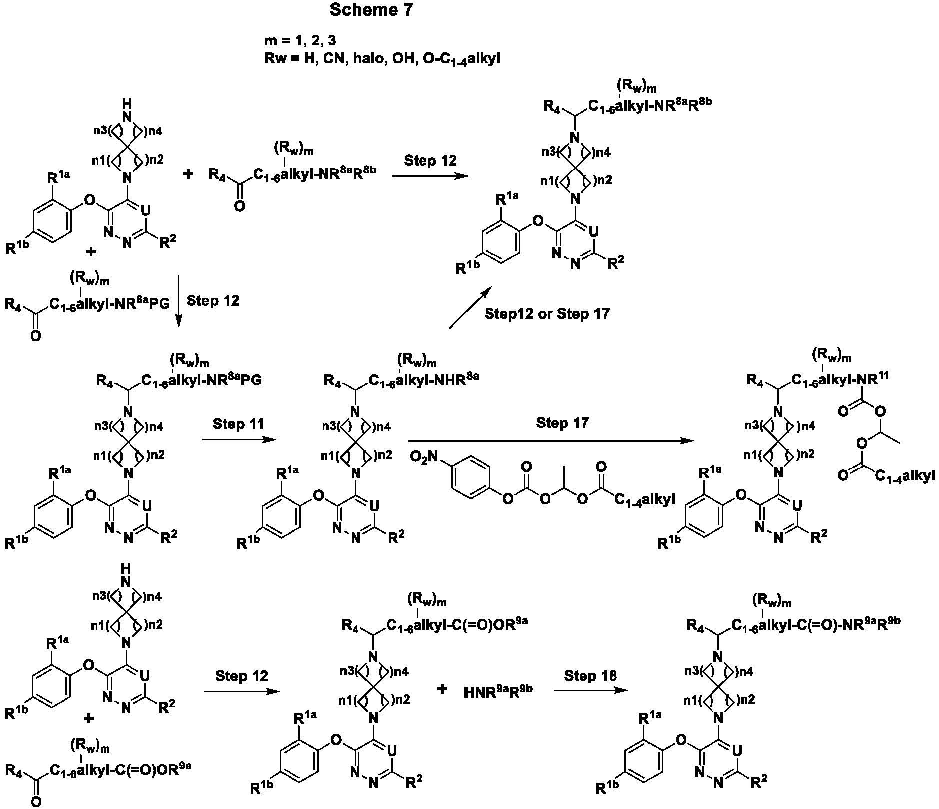

In Scheme 7, the following reaction conditions apply:
Step 11: When PG is Boc, at a suitable temperature such as for example RT, in the presence of a suitable acid as for example TFA, in a suitable solvent such as for example DCM;
Step 12: reductive amination condition, at a suitable temperature such as for example from RT to 80 °C, in the presence or absence of a suitable Lewis acid such as for example ZnCl₂ or an acid for example AcOH, in the presence of a suitable reducing agent such as for example NaBH₃CN, in a suitable solvent such as for example MeOH;
Step 17: at a suitable temperature such as for example from RT to 80 °C, in the presence of a suitable base such as for example DIEA or Cs₂CO₃, in suitable solvent such as for example DCM or DMF;
Step 18: at a suitable temperature such as for example 40 °C, in the presence of a suitable base such as for example ammonia, in suitable solvent such as for 1,4-dioxane.

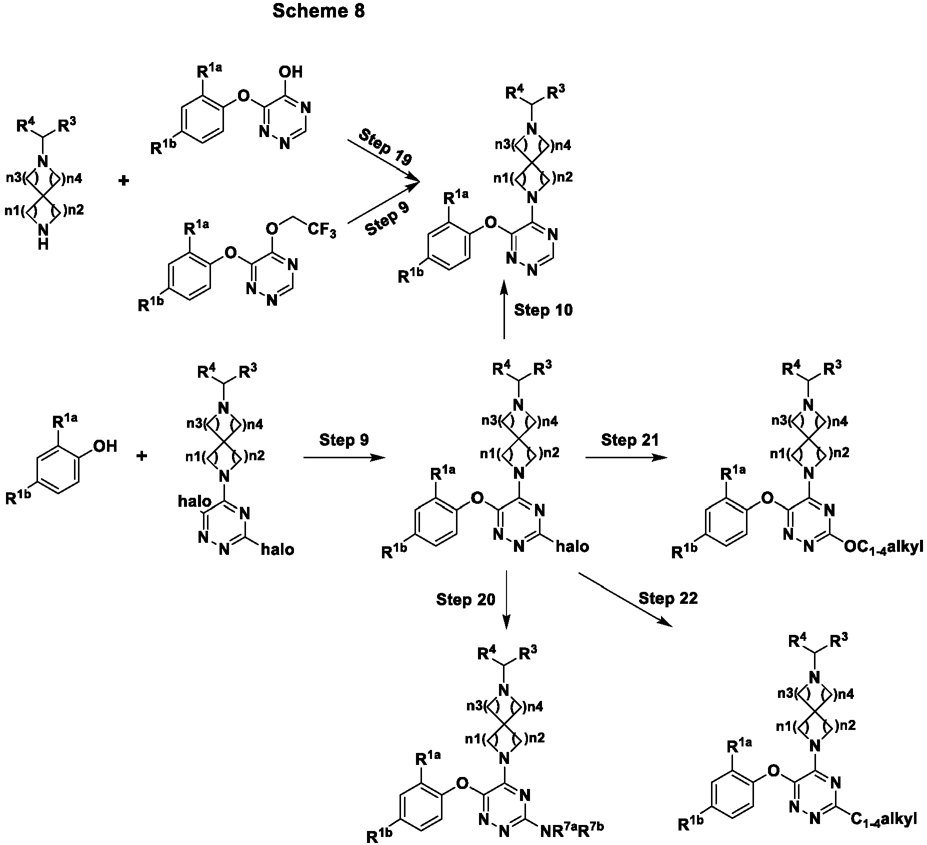

In Scheme 8, the following reaction conditions apply:
Step 9: at a suitable temperature such as for example from -78 °C to 40 °C, in particular from 0 °C to RT, in the presence of a suitable base such as for example TEA, DBU or K₂CO₃, in a suitable solvent such as for example DCM, THF or DMF;
Step 10: at a suitable temperature such as for example RT, in the presence of a suitable organometallic catalyst as for example Pd/C, optionally in the presence of a suitable base as for example TEA, in a suitable solvent such as for example MeOH under H₂ atmosphere;
Step 19: at a suitable temperature such as for example RT, in the presence of a suitable chlorinating reagent such as for example oxalyl chloride, in the presence of DMF, in a suitable solvent such as for example DCM;
Step 20: at a suitable temperature such as for example 90 °C, in the presence of a suitable nucleophilic amine, in a suitable solvent such as for example EtOH;
Step 21: at a suitable temperature such as for example RT, in the presence of a suitable acid such as for example HCl in dioxane, in a suitable solvent such as for example MeOH;
Step 22: at a suitable temperature such as for example 110 °C, in the presence of a suitable boron reagent such as for example trimethylboroxine, in the presence of a suitable organometallic catalyst such as for example tetrakis(triphenylphosphine)palladium(0), in the presence of a suitable base such as for example K₂CO₃, in a suitable solvent such as for example 1,4-dioxane;

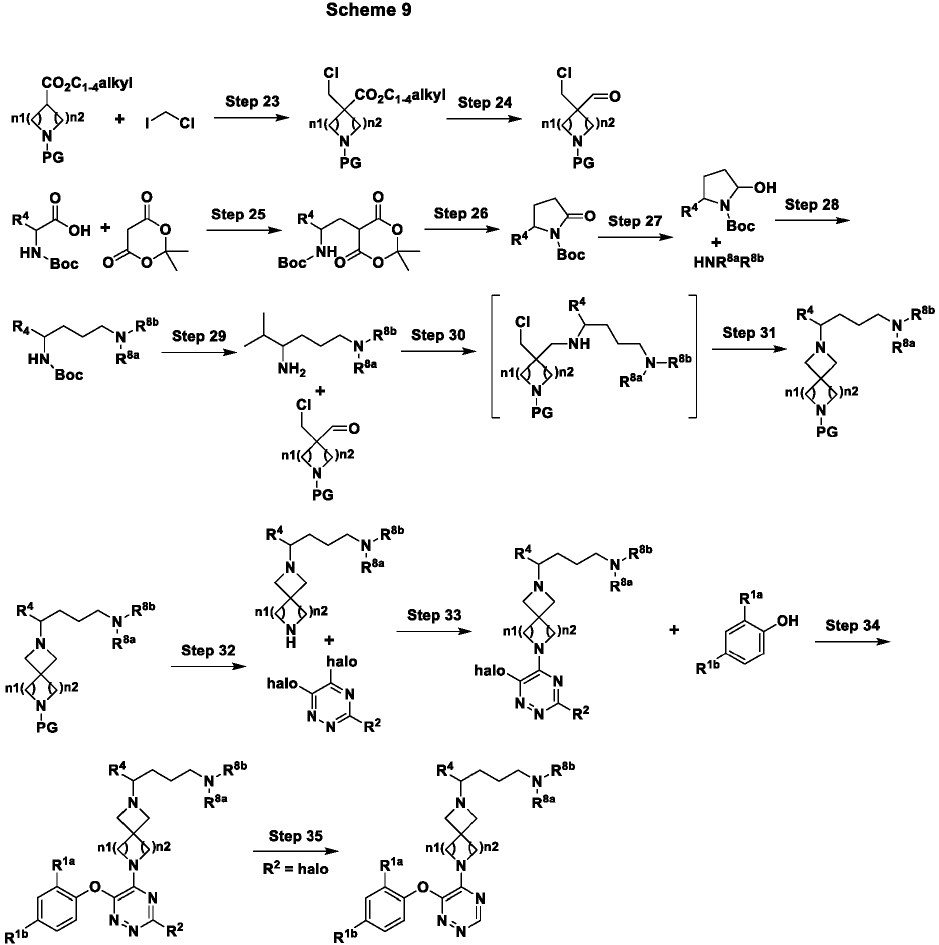

In Scheme 9, the following reaction conditions apply:
Step 23: at a suitable temperature such as for example from -78 °C to -25 °C, in the presence of suitable bases such as for example DIEA and n-BuLi, in a suitable solvent such as for example THF;s
Step 24: at a suitable temperature such as for example between -65 °C and - 55°C, in the presence of suitable reducing agent such as for example DIBAL-H, in a suitable solvent such as for example toluene, preferably conducted in a suitable flow chemistry system;
Step 25: first at a suitable temperature such as for example from -10 °C to 10 °C, in the presence of a suitable base such as for example DMAP, in the presence of a suitable condensation agent such as for example DCC, in a suitable solvent such as for example DCM; then at a suitable temperature such as for example from -10 °C to 0 °C, in the presence of a suitable acid such as for example AcOH, in the presence of a suitable reducing agent such as for example NaBH₄, in a suitable solvent such as for example DCM;
Step 26: in a suitable solvent such as for example toluene and heated to reflux;
Step 27: at a suitable temperature such as for example from -5 °C to 5 °C, in the presence of suitable reducing agent such as for example LiBH₄, in a suitable solvent such as for example 2-methyltetrahydrofuran;
Step 28: at a suitable temperature such as for example from 15 °C to 25 °C, in the presence of a suitable reducing agent such as for example NaBH(OAc)₃, in a suitable solvent such as for example DCM;
Step 29: at a suitable temperature such as for example from 15 °C to 25 °C, in the presence of a suitable acid such as for HCl, in a suitable solvent such as for example IPA;
Step 30: at a suitable temperature such as for example from 5 °C to 30 °C, in the presence of a suitable base such as for example TEA, in the presence of suitable reducing agent such as for example NaBH(OAc)₃, in a suitable solvent such as for example toluene;
Step 31: at a suitable temperature such as for example from 50 °C to 55 °C, in the presence of a suitable base such as for example K₂HPO₄, in a suitable solvent such as for example H₂O;
Step 32: When PG is Bn at a suitable temperature such as for example from -5 °C to 45 °C, under a hydrogen atmosphere within a suitable pressure range such as for example from 0.27 to 0.40 MPa, in the presence of a suitable catalyst such as for example palladium hydroxide on carbon, in the presence of a suitable acid as for example MSA in a suitable solvent such as EtOH;
Step 33: at a suitable temperature such as for example from -50 °C to -40 °C, in the presence of suitable base such as for example TEA, in a suitable solvent such as 2-methyltetrahydrofuran;
Step 34: at a suitable temperature such as for example from 20 °C to 30 °C, in the presence of suitable base such as for example TMG, in a suitable solvent such as 2-methyltetrahydrofuran;
Step 35: at a suitable temperature such as for example from 20 °C to 30 °C, under a hydrogen atmosphere within a suitable pressure range such as for example from 0.20 to 0.30 Mpa, in the presence of a suitable catalyst such as for example palladium on carbon, in a suitable solvent such as MeOH;
alternatively, at a suitable temperature such as room temperature, in the presence of a suitable catalyst such as for example 1,1'-Bis(diphenylphosphino)ferrocene-palladium(II)dichloride dichloromethane complex, a suitable reducing agent such sodium borohydride , a suitable base such as for example *N*,*N*,*N'*,*N'*-tetramethylethylenediamine, in a suitable solvent such as for example tetrahydrofuran.

### SCHEME 10

In general, compounds of Formula (I) wherein Y¹ is limited to -CH₂-, and R² is limited to W¹, hereby named compounds of Formula (Ia), can be prepared according to the following reaction Scheme 10. In Scheme 10, W¹ represents chloro, bromo or iodo; all other variables are defined according to the scope of the present disclosure.

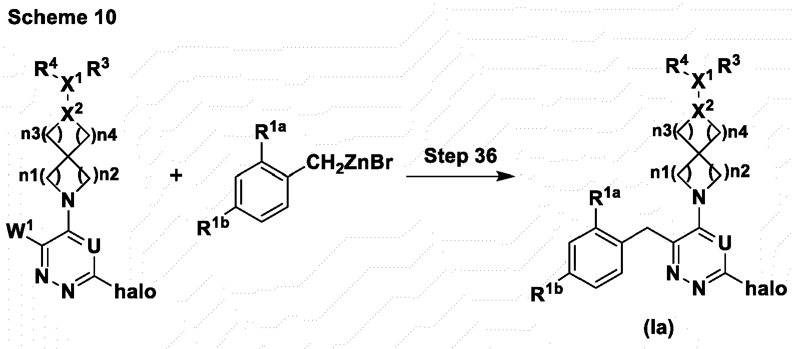

In Scheme 10, the following reaction conditions apply:
Step 36: at a suitable temperature ranged from 60 °C to 100 °C, in presence of a suitable catalyst such as palladium acetate (Pd(OAc)₂) or tris(dibenzylideneacetone)dipalladium(0) (Pd₂(dba)₃) or tetrakis(triphenylphosphine)palladium(0), in a suitable solvent such as for example tetrahydrofuran or dioxane.

The skilled person will realize that starting from compound (Ia), analogous chemistry as reported in step 10 in scheme 5 and in steps 20, 21 and 22 in scheme 8 could be performed.

### SCHEME 11

In general, compounds of Formula (I) wherein Y¹ is limited to -CR^{5a}R^{5b}- and R² is limited to W¹, hereby named compounds of Formula (Ib), can be prepared according to the following reaction Scheme 11. In Scheme 11 at least one of R^{5a} and R^{5b} is other than hydrogen. All other variables are defined according to the scope of the present disclosure.

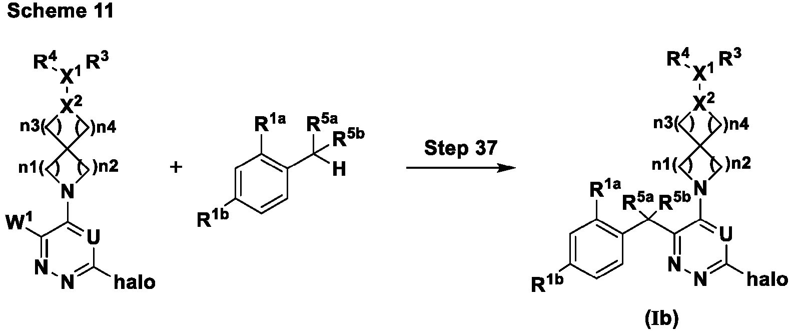

In Scheme 11, the following reaction condition apply:
Step 37: at a suitable temperature ranged from 80°C to 200°C, in presence of a suitable catalyst such as palladium acetate (Pd(OAc)₂), in the presence of a suitable ligand such as for example triphenylphosphine or tricyclohexylphosphine, in a suitable solvent such as for example dioxane, preferably in sealed conditions, optionally under microwave irradiation.

The skilled person will realize that starting from compound (Ib), analogous chemistry as reported in step 10 in scheme 5 and in steps 20, 21 and 22 in scheme 8 could be performed.

### SCHEME 12

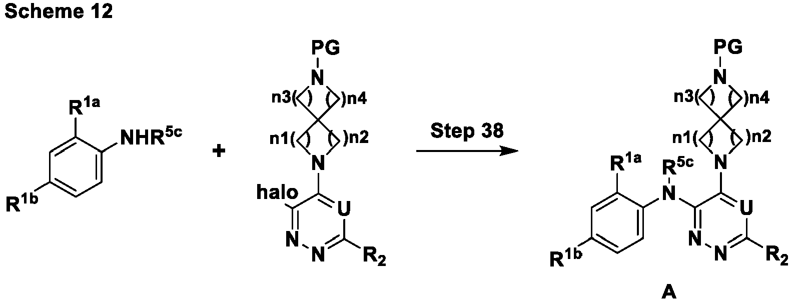

In Scheme 12, the following reaction condition apply:
Step 38: at a suitable temperature such as for example from RT to 80 °C, in the presence of a suitable base such as for example DIEA, Cs₂CO₃ or DBU, in suitable solvent such as for example DCM, THF or DMF;
Alternatively, at a suitable temperature such as for example RT to 100 °C, in the presence of a suitable catalyst such as for example Pd₂dba₃, in the presence of a suitable ligand such as for example Xantphos, in the presence of a suitable base such as Cs₂CO₃ or Na₂CO₃, in a suitable solvent such dioxane or a mixture of dioxane and water

The skilled person will realize that starting from intermediate A, analogous chemistry as reported in case Y¹ represents O can be performed.

It will be appreciated that where appropriate functional groups exist, compounds of various formulae or any intermediates used in their preparation may be further derivatized by one or more standard synthetic methods employing condensation, substitution, oxidation, reduction, or cleavage reactions. Particular substitution approaches include conventional alkylation, arylation, heteroarylation, acylation, sulfonylation, halogenation, nitration, formylation and coupling procedures.

The compounds of Formula (I) may be synthesized in the form of racemic mixtures of enantiomers which can be separated from one another following art-known resolution procedures. The racemic compounds of Formula (I) containing a basic nitrogen atom may be converted into the corresponding diastereomeric salt forms by reaction with a suitable chiral acid. Said diastereomeric salt forms are subsequently separated, for example, by selective or fractional crystallization and the enantiomers are liberated therefrom by alkali. An alternative manner of separating the enantiomeric forms of the compounds of Formula (I) involves liquid chromatography using a chiral stationary phase. Said pure stereochemically isomeric forms may also be derived from the corresponding pure stereochemically isomeric forms of the appropriate starting materials, provided that the reaction occurs stereospecifically.

In the preparation of compounds of the present disclosure, protection of remote functionality (e.g., primary or secondary amine) of intermediates may be necessary. The need for such protection will vary depending on the nature of the remote functionality and the conditions of the preparation methods. Suitable amino-protecting groups (NH-Pg) include acetyl, trifluoroacetyl, t-butoxycarbonyl (Boc), benzyloxycarbonyl (CBz) and 9-fluorenylmethyleneoxycarbonyl (Fmoc). The need for such protection is readily determined by one skilled in the art. For a general description of protecting groups and their use, see T. W. Greene and P. G. M. Wuts, Protective Groups in Organic Synthesis, 4th ed., Wiley, Hoboken, New Jersey, 2007.

### PHARMACOLOGY

It has been found that the compounds of the present disclosure block the interaction of menin with MLL proteins and oncogenic MLL fusion proteins per se, or can undergo metabolism to a (more) active form in vivo (prodrugs). Therefore the compounds according to the present disclosure and the pharmaceutical compositions comprising such compounds may be useful for the treatment or prevention, in particular treatment, of diseases such as cancer, including but not limited to leukemia, myelodysplastic syndrome (MDS), and myeloproliferative neoplasms (MPN); and diabetes.

In particular, the compounds according to the present disclosure and the pharmaceutical compositions thereof may be useful in the treatment or prevention of cancer. According to one embodiment, cancers that may benefit from a treatment with menin/MLL inhibitors of the disclosure comprise leukemias, lymphomas, myelomas or solid tumor cancers (e.g. prostate cancer, lung cancer, breast cancer, pancreatic cancer, colon cancer, liver cancer, melanoma and glioblastoma, etc.). In some embodiments, the leukemias include acute leukemias, chronic leukemias, myeloid leukemias, myelogenous leukemias, lymphoblastic leukemias, lymphocytic leukemias, Acute myelogenous leukemias (AML), Chronic myelogenous leukemias (CML), Acute lymphoblastic leukemias (ALL), Chronic lymphocytic leukemias (CLL), T cell prolymphocytic leukemias (T-PLL), Large granular lymphocytic leukemia, Hairy cell leukemia (HCL), MLL-rearranged leukemias, MLL-PTD leukemias, MLL amplified leukemias, MLL-positive leukemias, leukemias exhibiting *HOX*/*MEIS1* gene expression signatures etc.

In particular, the compounds according to the present disclosure and the pharmaceutical compositions thereof may be useful in the treatment or prevention of myelodysplastic syndrome (MDS) or myeloproliferative neoplasms (MPN).

In particular, compounds according to the present disclosure and the pharmaceutical compositions thereof may be useful in the treatment or prevention of leukemias, in particular nucleophosmin (NPM1)-mutated leukemias, e.g. NPM1c.

In particular, compounds according to the present disclosure and the pharmaceutical compositions thereof may be useful in the treatment or prevention of AML, in particular nucleophosmin (NPM1)-mutated AML (i.e., NPM1^{mut} AML), more in particular abstract NPM1-mutated AML.

In particular, compounds according to the present disclosure and the pharmaceutical compositions thereof may be useful in the treatment or prevention of MLL-rearranged leukemias, in particular MLL-rearranged AML or ALL.

In particular, compounds according to the present disclosure and the pharmaceutical compositions thereof may be useful in the treatment or prevention of leukemias with MLL gene alterations, in particular AML or ALL with MLL gene alterations.

In particular, compounds according to the present disclosure and the pharmaceutical compositions thereof may be suitable for Q.D. dosing (once daily).

In particular, compounds according to the present disclosure and the pharmaceutical compositions thereof may be useful in the treatment or prevention of hematological cancer in a subject exhibiting NPM1 gene mutations and/or mixed lineage leukemia gene (*MLL; MLL1*; *KMT2A*) alterations, mixed lineage leukemia (MLL), MLL-related leukemia, MLL-associated leukemia, MLL-positive leukemia, MLL-induced leukemia, rearranged mixed lineage leukemia, leukemia associated with a MLL, rearrangement/alteration or a rearrangement/alteration of the MLL gene, acute leukemia, chronic leukemia, myelodysplastic syndrome (MDS), myeloproliferative neoplasms (MPN), insulin resistance, pre-diabetes, diabetes, or risk of diabetes, hyperglycemia, chromosomal rearrangement on chromosome 11q23, type-1 diabetes, type-2 diabetes; promoting proliferation of a pancreatic cell, where pancreatic cell is an islet cell, beta cell, the beta cell proliferation is evidenced by an increase in beta cell production or insulin production; and for inhibiting a menin-MLL interaction, where the MLL fusion protein target gene is HOX or MEIS1 in human.

Hence, the disclosure relates to compounds of Formula (I), the tautomers and the stereoisomeric forms thereof, and the pharmaceutically acceptable salts, and the solvates thereof, for use as a medicament.

The disclosure also relates to the use of a compound of Formula (I), a tautomer or a stereoisomeric form thereof, or a pharmaceutically acceptable salt, or a solvate thereof, or a pharmaceutical composition according to the disclosure, for the manufacture of a medicament.

The present disclosure also relates to a compound of Formula (I), a tautomer or a stereoisomeric form thereof, or a pharmaceutically acceptable salt, or a solvate thereof, or a pharmaceutical composition according to the disclosure, for use in the treatment, prevention, amelioration, control or reduction of the risk of disorders associated with the interaction of menin with MLL proteins and oncogenic MLL fusion proteins in a mammal, including a human, the treatment or prevention of which is affected or facilitated by blocking the interaction of menin with MLL proteins and oncogenic MLL fusion proteins.

Also, the present disclosure relates to the use of a compound of Formula (I), a tautomer or a stereoisomeric form thereof, or a pharmaceutically acceptable salt, or a solvate thereof, or a pharmaceutical composition according to the disclosure, for the manufacture of a medicament for treating, preventing, ameliorating, controlling or reducing the risk of disorders associated with the interaction of menin with MLL proteins and oncogenic MLL fusion proteins in a mammal, including a human, the treatment or prevention of which is affected or facilitated by blocking the interaction of menin with MLL proteins and oncogenic MLL fusion proteins.

The disclosure also relates to a compound of Formula (I), a tautomer or a stereoisomeric form thereof, or a pharmaceutically acceptable salt, or a solvate thereof, for use in the treatment or prevention of any one of the diseases mentioned hereinbefore.

The disclosure also relates to a compound of Formula (I), a tautomer or a stereoisomeric form thereof, or a pharmaceutically acceptable salt, or a solvate thereof, for use in treating or preventing any one of the diseases mentioned hereinbefore.

The disclosure also relates to the use of a compound of Formula (I), a tautomer or a stereoisomeric form thereof, or a pharmaceutically acceptable salt, or a solvate thereof, for the manufacture of a medicament for the treatment or prevention of any one of the disease conditions mentioned hereinbefore.

The compounds of the present disclosure can be administered to mammals, preferably humans, for the treatment or prevention of any one of the diseases mentioned hereinbefore.

In view of the utility of the compounds of Formula (I), the tautomers and the stereoisomeric forms thereof, and the pharmaceutically acceptable salts, and the solvates thereof, there is provided use in a method of treating warm-blooded animals, including humans, suffering from any one of the diseases mentioned hereinbefore.

Said use in a method comprises the administration, i.e. the systemic or topical administration, of a therapeutically effective amount of a compound of Formula (I), a tautomer or a stereoisomeric form thereof, or a pharmaceutically acceptable salt, or a solvate thereof, to warm-blooded animals, including humans.

Therefore, the disclosure also relates to use in a method for the treatment or prevention of any one of the diseases mentioned hereinbefore comprising administering a therapeutically effective amount of compound according to the disclosure to a patient in need thereof.

One skilled in the art will recognize that a therapeutically effective amount of the compounds of the present disclosure is the amount sufficient to have therapeutic activity and that this amount varies *inter alias,* depending on the type of disease, the concentration of the compound in the therapeutic formulation, and the condition of the patient. An effective therapeutic daily amount would be from about 0.005 mg/kg to 100 mg/kg. The amount of a compound according to the present disclosure, also referred to herein as the active ingredient, which is required to achieve a therapeutically effect may vary on case-by-case basis, for example with the particular compound, the route of administration, the age and condition of the recipient, and the particular disorder or disease being treated. Use in a method of treatment may also include administering the active ingredient on a regimen of between one and four intakes per day. In these uses in methods of treatment the compounds according to the disclosure are preferably formulated prior to administration.

The present disclosure also provides compositions for preventing or treating the disorders referred to herein. Said compositions comprising a therapeutically effective amount of a compound of Formula (I), a tautomer or a stereoisomeric form thereof, or a pharmaceutically acceptable salt, or a solvate thereof, and a pharmaceutically acceptable carrier or diluent.

While it is possible for the active ingredient to be administered alone, it is preferable to present it as a pharmaceutical composition. Accordingly, the present disclosure further provides a pharmaceutical composition comprising a compound according to the present disclosure, together with a pharmaceutically acceptable carrier or diluent. The carrier or diluent must be "acceptable" in the sense of being compatible with the other ingredients of the composition and not deleterious to the recipients thereof.

The pharmaceutical compositions may be prepared by any methods well known in the art of pharmacy, for example, using methods such as those described in Gennaro et al. Remington's Pharmaceutical Sciences (18th ed., Mack Publishing Company, 1990, see especially Part 8 : Pharmaceutical preparations and their Manufacture).

The compounds of the present disclosure may be administered alone or in combination with one or more additional therapeutic agents. Combination therapy includes administration of a single pharmaceutical dosage formulation which contains a compound according to the present disclosure and one or more additional therapeutic agents, as well as administration of the compound according to the present disclosure and each additional therapeutic agent in its own separate pharmaceutical dosage formulation.

Therefore, an embodiment of the present disclosure relates to a product containing as first active ingredient a compound according to the disclosure and as further active ingredient one or more anticancer agent, as a combined preparation for simultaneous, separate or sequential use in the treatment of patients suffering from cancer.

The one or more other medicinal agents and the compound according to the present disclosure may be administered simultaneously (e.g. in separate or unitary compositions) or sequentially in either order. In the latter case, the two or more compounds will be administered within a period and in an amount and manner that is sufficient to ensure that an advantageous or synergistic effect is achieved. It will be appreciated that the preferred method and order of administration and the respective dosage amounts and regimes for each component of the combination will depend on the particular other medicinal agent and compound of the present disclosure being administered, their route of administration, the particular condition, in particular tumour, being treated and the particular host being treated.

The following examples further illustrate the present disclosure.

### EXAMPLES

Several methods for preparing the compounds of this disclosure are illustrated in the following examples. Unless otherwise noted, all starting materials were obtained from commercial suppliers and used without further purification, or alternatively can be synthesized by a skilled person by using well-known methods.

| **Abbreviation** | **Meaning** |
|---|---|
| **Ag(Phen)₂OTf** | silver triflate-bis(1,10-phenanthroline) complex |
| **2-MeTHF** | 2-methyltetrahydrofuran |
| **ACN** | acetonitrile |
| **AcCl** | acetyl chloride |
| **AcOH** | acetic acid |
| **Ac₂O** | acetic anhydride |
| **aq.** | aqueous |
| **Ar** | argon |
| **BBr₃** | tribromoborane |
| **bn** | benzyl |
| **Boc** | *tert*-butyloxycarbonyl |
| **Boc₂O** | di-*tert*-butyl dicarbonate |
| ***n*-BuLi** | *n*-butyllithium |
| **Cbz** | benzyloxycarbonyl |
| **CD₃OD** | Methanol-d₄ |
| **CHCl₃** | chloroform |
| **Cs₂CO₃** | cesium carbonate |
| **conc.** | concentrated |
| **DBU** | 1,8-diazabicyclo[5.4.0]undec-7-ene |
| **DCC** | dicyclohexylcarbodiimide |
| **DCE** | dichloroethane |
| **DCM** | dichloromethane |
| **DDQ** | 4,5-dichloro-3,6-dioxocyclohexa-1,4-diene-1,2-dicarbonitrile |
| **DEA** | diethylamine |
| **DIBAL-H** | diisobutylaluminum hydride |
| **DIEA or DIPEA** | *N,N*-diisopropylethylamine |
| **DMAP** | *N,N*-dimethylpyridin-4-amine |
| **DMF** | *N,N*-dimethylformamide |
| **DMP** | Dess-Martin periodinane |
| **DMSO** | dimethyl sulfoxide |
| **dppf** | 1,1'-ferrocenediyl-bis(diphenylphosphine) |
| **EDCI** | *N*-(3-Dimethylaminopropyl)-*N'*-ethylcarbodiimide hydrochloride |
| **EA or EtOAc** | ethyl acetate |
| **EtOH** | ethanol |
| **eq.** | equivalent(s) |
| **FA** | formic acid |
| **FCC** | flash column chromatography |
| **h** | hour(s) |
| **H₂** | hydrogen |
| **HATU** | 1-[bis(dimethylamino)methylene]-1*H*-1,2,3-triazolo[4,5-b]pyridinium 3-oxid hexafluorophosphate |
| **H₂O** | water |
| **HCl** | hydrochloric acid |
| **HOBt** | 1-Hydroxybenzotriazole |
| **HPLC** | high performance liquid chromatography |
| **ICH₂Cl** | chloroiodomethane |
| **IPA** | isopropyl alcohol |
| **IPAc** | isopropyl acetate |
| **K₂CO₃** | potassium carbonate |
| **KI** | potassium iodide |
| **K₂HPO₄** | dipotassium phosphate |
| **K₃PO₄** | tripotassium phosphate |
| **LiAlD₄** | lithium aluminum deuteride |
| **LAH** | lithium aluminum hydride |
| **LiBH₄** | lithium borohydride |
| **LDA** | lithium diisopropylamide |
| **LiCl** | lithium chloride |
| **LG** | leaving group |
| **Me** | methyl |
| **MeOH** | methanol |
| **2-MeTHF** | 2-methyltetrahydrofuran |
| **min** | minute(s) |
| **mL** | milliliters |
| **mmol** | millimoles |
| **mg** | milligram |
| **MgSO₄** | magnesium sulfate |
| **MSA** | methanesulfonic acid |
| **MsCl** | methanesulfonyl chloride |
| **MS** | molecular sieve |
| **MTBE** | methyl *tert*-butyl ether |
| **N₂** | nitrogen |
| **NA** | not available |
| **NaBH₃CN** | sodium cyanoborohydride |
| **NaBH(OAc)₃** | sodium triacetoxyborohydride |
| **NaBD₃CN** | sodium cyanoborodeuteride |
| **Na₂CO₃** | sodium carbonate |
| **NaH** | sodium hydride |
| **NaHCO₃** | sodium bicarbonate |
| **NaI** | sodium iodide |
| **NaOAc** | sodium acetate |
| **NaOH** | sodium hydroxide |
| **Na₂SO₃** | sodium sulfite |
| **Na₂SO₄** | sodium sulfate |
| **NH₄Cl** | ammonium chloride |
| **NMM** | 1-4-Methylmorpholine |
| **Pd₂dba₃** | tris(dibenzylideneacetone)dipalladium(0) |
| **Pd(dppf)Cl₂·DCM** | [1,1'-bis(diphenylphosphino)ferrocene]dichloropalladium(II), complex with dichloromethane |
| **Pd(PPh₃)₄** | tetrakis(triphenylphosphine)palladium(0) |
| **PE** | petroleum ether |
| **PG** | protecting group |
| **Phen** | phenanthroline |
| **psi** | pound per square inch |
| ***p*-TsOH** | *p*-toluenesulfonic acid |
| ***p*-TsOH·H₂O** | *p*-toluenesulfonic acid monohydrate |
| **Rₜ** | retention time |
| **Rochelle's salt** | potassium sodium tartrate tetrahydrate |
| **RT** | room temperature |
| **sat.** | saturated |
| **SFC** | supercritical fluid chromatography |
| **TBAF** | tetrabutylammonium fluoride |
| **TBDMS** | *tert*-butyldimethylsilyl |
| **TBDPS** | *tert*-butyldiphenylsilyl |
| ***t*-BuOK** | potassium *tert*-butoxide |
| **TEA** | triethylamine |
| **Tf** | trifluoromethanesulfonyl |
| **TFA** | trifluoroacetic acid |
| **THF** | tetrahydrofuran |
| **Ti(OiPr)₄** | titanium(IV) isopropoxide |
| **TLC** | thin layer chromatography |
| **TMEDA** | *N,N,N',N*'-tetramethylethylenediamine |
| **TMG** | 1,1,3,3-tetramethylguanidine |
| **TMSI** | iodotrimethylsilane |
| **Ts** | *p*-toluenesulfonyl |
| **TsCl** | *p*-toluenesulfonyl chloride |
| **v/v** | volume per volume |
| **vol.** | volume(s) |
| **wt** | weight |
| **Xantphos** | 4,5-bis(diphenylphosphino)-9,9-dimethylxanthene |

As understood by a person skilled in the art, compounds synthesized using the protocols as indicated may exist as a solvate e.g. hydrate, and/or contain residual solvent or minor impurities. Compounds or intermediates isolated as a salt form, may be integer stoichiometric i.e. mono- or di-salts, or of intermediate stoichiometry. When an intermediate or compound in the experimental part below is indicated as 'HCl salt' without indication of the number of equivalents of HCl, this means that the number of equivalents of HCl was not determined. The same principle will also apply to all other salt forms referred to in the experimental part, such as e.g. 'oxalate salt', 'formate salt' or '

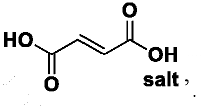

The stereochemical configuration for centers in some compounds may be designated "*R*" or "*S*" when the mixture(s) was separated and absolute stereochemistry was known, or when only one enantiomer was obtained and absolute stereochemistry was known; for some compounds, the stereochemical configuration at indicated centers has been designated as "**R*" (first eluted from the column in case the column conditions of the separation are described in the synthesis protocol and when only one stereocenter present or indicated) or "**S*" (second eluted from the column in case the column conditions of the separation are described in the synthesis protocol and when only one stereocenter present or indicated) when the absolute stereochemistry is undetermined (even if the bonds are drawn stereo specifically) although the compound itself has been isolated as a single stereoisomer and is enantiomerically pure. In case a compound designated as "*R" is converted into another compound, the "*R" indication of the resulting compound is derived from its starting material.

For example, it will be clear that Compound 25 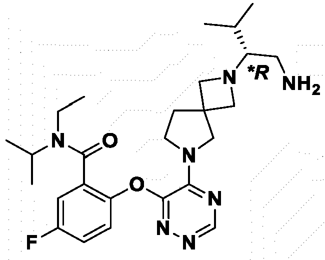 is

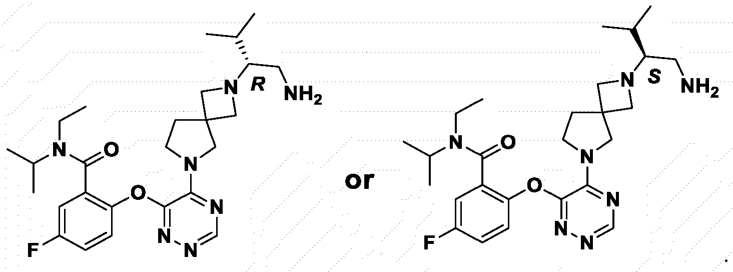

When "**R*" or "**S*" occurs together with a 2^{nd} stereocentre which is designated "*R*" or "*S*" (known absolute stereochemistry for 2^{nd} stereocentre) in the same molecule, the absolute stereochemistry of the stereocentre designated "**R*" or "**S*" is undetermined (even if the bonds are drawn stereo specifically) although the compound itself has been isolated as a single stereoisomer and is enantiomerically pure. "**R*" or "**S*" is assigned randomly for such molecules. For example, it will be clear that Compound 340 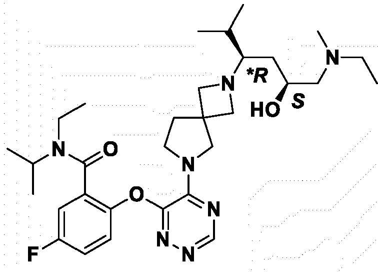 is

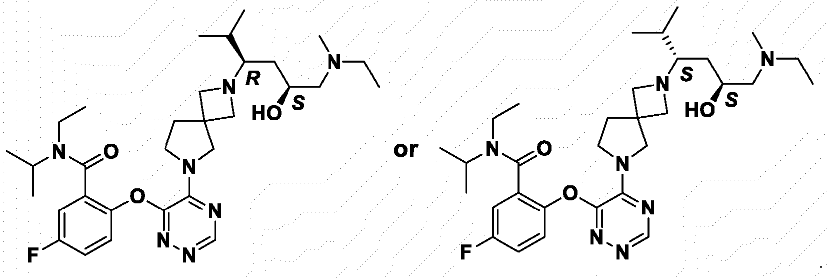

For compounds wherein the stereochemical configuration of two stereocentres is indicated by * (e.g. *R or *S), the absolute stereochemistry of the stereocentres is undetermined (even if the bonds are drawn stereospecifically), although the compound itself has been isolated as a single stereoisomer and is enantiomerically pure. In this case, the configuration of the first stereocentre is independent of the configuration of the second stereocentre in the same compound. "**R*" or "**S*" is assigned randomly for such molecules.

For example, for Compound 306 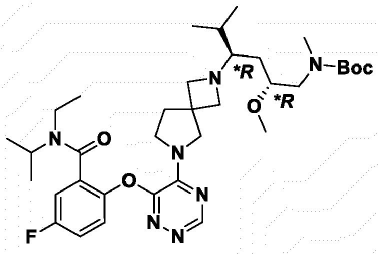 this means that the compound is 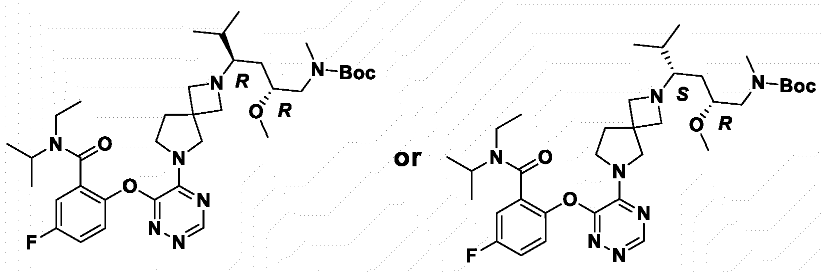 or

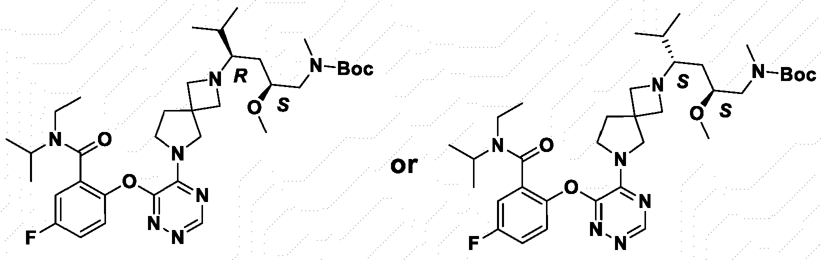

A skilled person will realize that the paragraphs above about stereochemical configurations, also apply to intermediates.

A skilled person will realize that, even where not mentioned explicitly in the experimental protocols below, typically after a column chromatography purification, the desired fractions were collected and the solvent was evaporated.

In case no stereochemistry is indicated, this means it is a mixture of stereoisomers, unless otherwise is indicated or is clear from the context.

When a stereocenter is indicated with 'RS' this means that a racemic mixture was obtained at the indicated centre, unless otherwise indicated.

### Preparation of intermediates

For intermediates that were used in a next reaction step as a crude or as a partially purified intermediate, in some cases no mol amounts are mentioned for such intermediate in the next reaction step or alternatively estimated mol amounts or theoretical mol amounts for such intermediate in the next reaction step are indicated in the reaction protocols described below.

### Preparation of intermediate 27

### N-ethyl-5-fluoro-N-isopropyl-2-methoxybenzamide

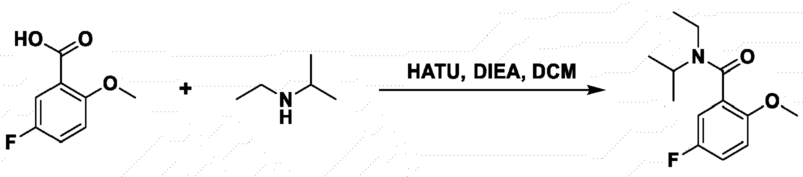

To the mixture of 5-fluoro-2-methoxybenzoic acid (8.00 g, 47.0 mmol) and *N*-ethylpropan-2-amine (8.19 g, 94.0 mmol) in dry DCM (150 mL) cooled at 0 °C, were slowly added HATU (21.5 g, 56.5 mmol) and DIEA (9.10 g, 70.4 mmol) in portions. The resulting mixture was slowly warmed to RT and stirred for 8 h. The organic layer was washed with water (20 mL x 3) and dried over anhydrous Na₂SO₄. After filtration, the solvent was removed under reduced pressure and the crude product was purified by FCC (EtOAc/PE = 0% to 20%) to afford the title intermediate (12.0 g, 96% yield) as a white solid.

### Preparation of intermediate 67, 235, 246

### 5-fluoro-N,N-diisopropyl-2-methoxybenzamide

### 5-fluoro-2-methoxy-N-(propan-2-yl-¹³C₃)benzamide

### 5-fluoro-N-isopropyl-2-methoxy-N-methylbenzamide

**The following intermediate was synthesized by an analogous method as described above for intermediate 27**

| **Int. No.** | **Structure** | **Starting Materials** |
|---|---|---|
| 67 | 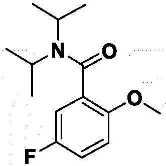 | 5-fluoro-2-methoxybenzoic acid, diisopropylamine |
| 235 | 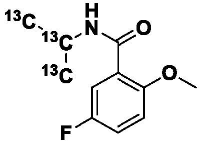 | 5-fluoro-2-methoxybenzoic acid, propan-2-amine-1,2,3-¹³*C*₃ |
| 246 | 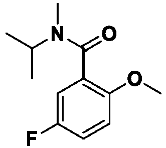 | 5-fluoro-2-methoxybenzoic acid, *N*-methylpropan-2-amine |

### Preparation of intermediate 28

### N-ethyl-5-fluoro-2-hydroxy-N-isopropylbenzamide

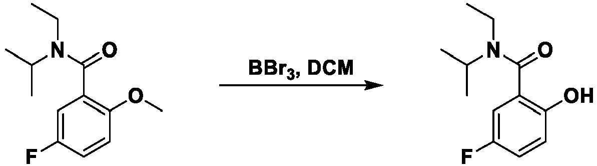

### To the solution of N-ethyl-5-fluoro-N-isopropyl-2-methoxybenzamide (intermediate 27)

(12.0 g, 50.1 mmol) in dry DCM (100 mL) cooled at -78 °C was slowly added BBr₃ (14.4 mL, 152 mmol), the resulting mixture was slowly warmed to RT and stirred for 8 h. The mixture was cooled to -78 °C again and MeOH (5 mL) was added dropwise to quench the reaction. The resulting mixture was slowly warmed to RT and the pH value was adjusted to about 8 by adding sat. aq. NaHCO₃ solution. The aqueous layer was extracted by DCM (50 mL x 3) and the combined organic layers were dried over anhydrous Na₂SO₄, filtered and concentrated under reduced pressure to give the crude product which was purified by FCC (EtOAc/PE = 0% to 20%) to afford the title intermediate (9.0 g, 78% yield) as a white solid.

### Preparation of intermediate 68, 237, 247

### 5-fluoro-2-hydroxy-N,N-diisopropylbenzamide

### N-(ethyl-¹³C₂)-5-fluoro-2-hydroxy-N-(propan-2-yl-¹³C₃)benzamide

### 5-fluoro-2-hydroxy-N-isopropyl-N-methylbenzamide

**The following intermediate was synthesized by an analogous method as described above for intermediate 28**

| **Int. No.** | **Structure** | **Starting Materials** |
|---|---|---|
| 68 | 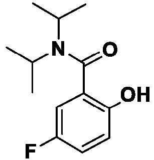 | intermediate 67 |
| 237 | 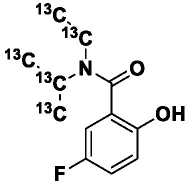 | intermediate 236 |
| 247 | 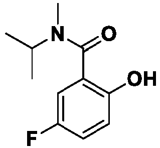 | intermediate 246 |

### Preparation of intermediate 60

### 5-bromo-4-cyclopropylpyrimidine

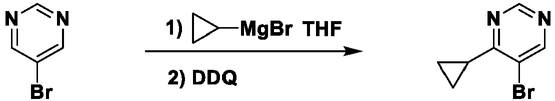

To a solution of 5-bromopyrimidine (30 g, 189 mmol) in THF (1000 mL) was added cyclopropylmagnesium bromide (396 mL, 198 mmol, 0.5 M in THF) at 0 °C under N₂ atmosphere. After addition, the reaction mixture was stirred at RT for 4 h, then a solution of DDQ (42.8 g, 189 mmol) in THF (500 mL) was added dropwise into the reaction mixture at 0 °C. After addition, the reaction mixture was stirred at RT for 16 h. The reaction mixture was concentrated *in vacuo* and the residue was partitioned between EtOAc (200 mL) and water (200 mL), and the aqueous layer was extracted by EtOAc (200 mL x 3). The combined organic layers were washed with 1N NaOH (200 mL x 2), brine (200 mL), dried over Na₂SO₄, filtered. The filtrate was concentrated *in vacuo* and the residue was purified by FCC (EtOAc/PE = 0% to 15%) to afford the title intermediate (21.4 g, 55% yield) as white solid.

### Preparation of intermediate 61

### 2-(4-cyclopropylpyrimidin-5-yl)-4-fluorophenol

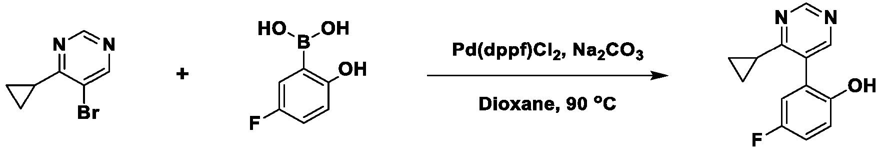

The mixture of 5-bromo-4-cyclopropylpyrimidine (**intermediate 60**) (20.0 g, 100 mmol), (5-fluoro-2-hydroxyphenyl)boronic acid (18.7 g, 120 mmol), Pd(dppf)Cl₂ (3.68 g, 5.03 mmol) and Na₂CO₃ (2 M in H₂O, 101 mL, 202 mmol) in 1,4-dioxane (350 mL) was heated at 90 °C for 12 h under N₂ atmosphere. After cooled to RT, the reaction mixture was filtered through a celite pad, the filtrate was suspended into water (400 mL) and further extracted with EtOAc (200 mL x 3). The combined organic layers were dried over anhydrous Na₂SO₄, filtered and concentrated under reduced pressure to give the crude product, which was purified by FCC on silica gel (PE/EtOAc = 1:0 to 3:1) to afford the title intermediate (24.0 g, 95% purity, 98.6% yield) as a brown solid.

### Preparation of intermediate 13

### tert-butyl 6-(3,6-dichloro-1,2,4-triazin-5-yl)-2,6-diazaspiro[3.4]octane-2-carboxylate

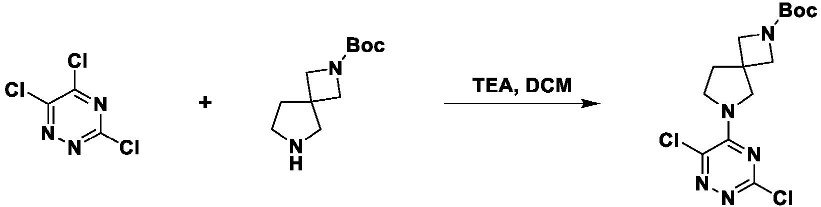

To the solution of 3,5,6-trichloro-1,2,4-triazine (10.0 g, 54.2 mmol) and TEA (15.2 mL, 109 mmol) in DCM (100 mL) cooled at 0 °C was added *tert*-butyl 2,6-diazaspiro[3.4]octane-2-carboxylate (9.21 g, 43.4 mmol), the mixture was warmed to RT and stirred for 1 h. The mixture was diluted with water (20 mL) and extracted with DCM (30 mL x 3). The combined organic layers were washed with brine, dried over Na₂SO₄, filtered and concentrated under reduced pressure to give the crude product which was purified by FCC on silica gel (PE/EtOAc = 1:0 to 3:1) to afford the title intermediate (12.0 g, 58% yield) as a yellow solid.

### Preparation of intermediate 69

### tert-butyl 6-(3-chloropyridazin-4-yl)-2,6-diazaspiro[3.4]octane-2-carboxylate

**The following intermediate was synthesized by an analogous method as described above for intermediate 13**

| **Int. No.** | **Structure** | **Starting Materials** |
|---|---|---|
| 69 | 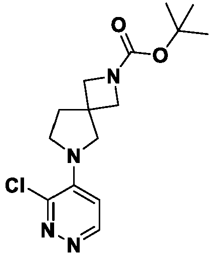 | 3,4-dichloropyridazine, *tert*-butyl 2,6-diazaspiro[3.4]octane-2-carboxylate |

### Preparation of intermediate 14

### tert-butyl 6-(3-chloro-6-(2-(ethyl(isopropyl)carbamoyl)-4-fluorophenoxy)-1,2,4-triazin-5-yl)-2,6-diazaspiro[3.4]octane-2-carboxylate

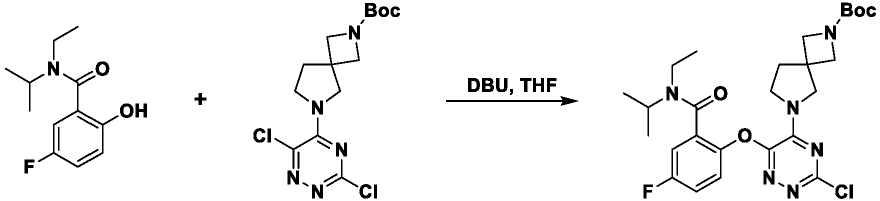

The mixture of *tert*-butyl 6-(3,6-dichloro-1,2,4-triazin-5-yl)-2,6-diazaspiro[3.4]octane-2-carboxylate (**intermediate 13**) (12.0 g, 33.3 mmol), *N*-ethyl-5-fluoro-2-hydroxy-*N-*isopropylbenzamide (**intermediate 28**) (7.5 g, 33.3 mmol) and DBU (6.1 g, 40.1 mmol) in THF (120 mL) was stirred at 25 °C for 8 h. The mixture was diluted with water (30 mL) and extracted with DCM (30 mL x 3). The combined organic layers were washed with brine, dried over Na₂SO₄, filtered and concentrated under reduced pressure to give the crude product which was purified by FCC (PE/EtOAc = 1:0 to 3:1) to afford the title intermediate (14.0 g, 73% yield) as green solid.

### Preparation of intermediates 57, 74, 70, and 83

### tert-butyl 6-(3-chloro-6-(2-(4-cyclopropylpyrimidin-5-yl)-4-fluorophenoxy)-1,2,4-triazin-5-yl)-2,6-diazaspiro[3.4]octane-2-carboxylate

### tert-butyl 6-(3-(2-(ethyl(isopropyl)carbamoyl)-4-fluorophenoxy)pyridazin-4-yl)-2,6-diazaspiro[3.4]octane-2-carboxylate

### tert-butyl 6-(3-(2-(diisopropylcarbamoyl)-4-fluorophenoxy)pyridazin-4-yl)-2,6-diazaspiro[3.4]octane-2-carboxylate

### tert-butyl 6-(3-chloro-6-(2-(diisopropylcarbamoyl)-4-fluorophenoxy)-1,2,4-triazin-5-yl)-2,6-diazaspiro [3.4] octane-2-carboxylate

**The following intermediates were synthesized by an analogous method as described above for intermediate 14**

| **Int. No.** | **Structure** | **Starting Materials** |
|---|---|---|
| 57 | 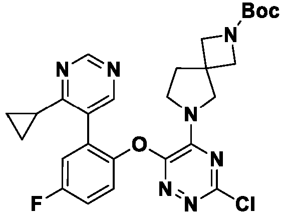 | intermediate 61, intermediate 13 |
| 74 | 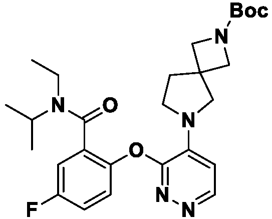 | intermediate 28, intermediate 69 |
| 70 | 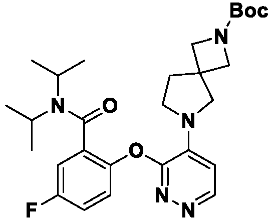 | intermediate 68, intermediate 69 |
| 83 | 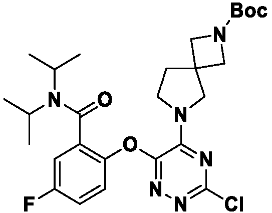 | intermediate 68, intermediate 13 |

### Preparation of intermediate 2

### tert-butyl 6-(6-(2-(ethyl(isopropyl)carbamoyl)-4-fluorophenoxy)-1,2,4-triazin-5-yl)-2,6-diazaspiro[3.4]octane-2-carboxylate

### Method A:

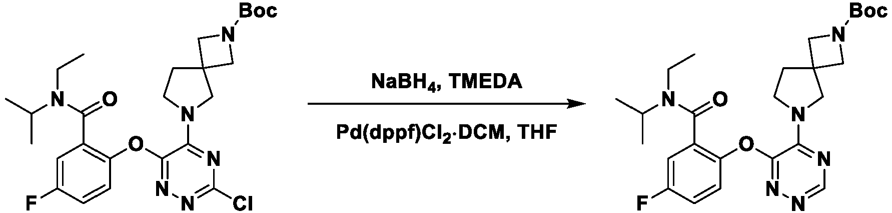

To the mixture of *tert-*butyl 6-(3-chloro-6-(2-(ethyl(isopropyl)carbamoyl)-4-fluorophenoxy)-1,2,4-triazin-5-yl)-2,6-diazaspiro[3.4]octane-2-carboxylate (**intermediate 14**) (20 g, 36.4 mmol), NaBH₄ (2.48 g, 65.7 mmol) and TMEDA (8.54 g, 73.5 mmol) in THF (500 mL) was added Pd(dppf)Cl₂·DCM (1.70 g, 2.08 mmol) under N₂ atmosphere. After addition, the reaction mixture was stirred at 25 °C for 14 h. The reaction mixture was filtered and the filtrate was concentrated, the residue was purified by FCC on silica gel (EtOAc) to afford the title intermediate (15 g, 93% purity, 74% yield) as brown solid.

### Method B:

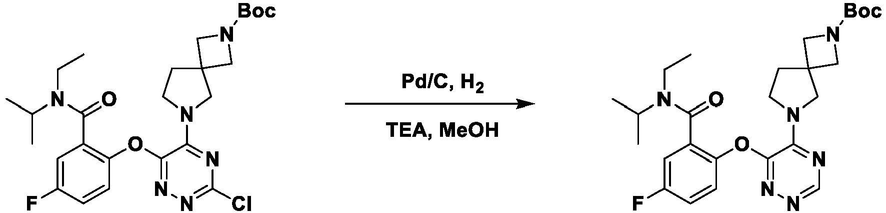

To the solution of *tert*-butyl 6-(3-chloro-6-(2-(ethyl(isopropyl)carbamoyl)-4-fluorophenoxy)-1,2,4-triazin-5-yl)-2,6-diazaspiro[3.4]octane-2-carboxylate (**intermediate 14**) (22.0 g, 40.1 mmol), TEA (15 mL) in MeOH (100 mL) was added Pd/C (wet, 5.0 g, 10%) The resulting mixture was stirred under H₂ atmosphere (30 psi) at 25 °C for 8 h. The reaction mixture was filtered through a celite pad and the filtrate was concentrated *in vacuo* to afford the title intermediate (25.0 g, crude), which was used directly in next step without further purification.

### Preparation of intermediate 58, 84

### tert-butyl 6-(6-(2-(4-cyclopropylpyrimidin-5-yl)-4-fluorophenoxy)-1,2,4-triazin-5-yl)-2,6-diazaspiro[3.4]octane-2-carboxylate

### tert-butyl 6-(6-(2-(diisopropylcarbamoyl)-4-fluorophenoxy)-1,2,4-triazin-5-yl)-2,6-diazaspiro[3.4]octane-2-carboxylate

**The following intermediates were synthesized by an analogous method described above for intermediate 2**

| **Int. No.** | **Structure** | **Starting Material** | **Conditions** |
|---|---|---|---|
| 58 | 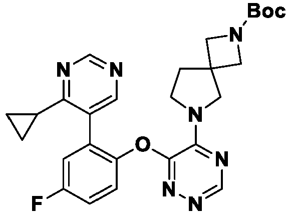 | intermediate 57 | NaBH₄, TMEDA, Pd(dppf)Cl₂·DCM, THF |
| 84 | 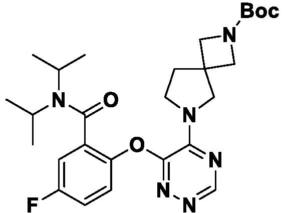 | intermediate 83 | Pd/C, H₂, TEA, MeOH |

### Preparation of intermediate 3

### 2-((5-(2,6-diazaspiro[3.4]octan-6-yl)-1,2,4-triazin-6-yl)oxy)-N-ethyl-5-fluoro-N-isopropylbenzamide

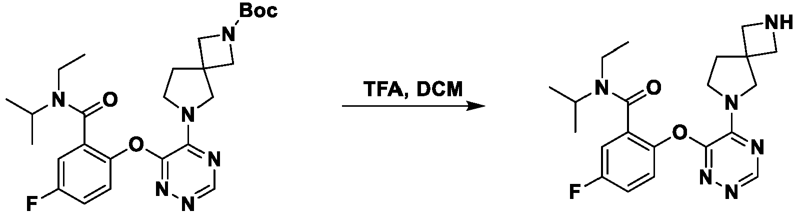

To the solution of *tert*-butyl 6-(6-(2-(ethyl(isopropyl)carbamoyl)-4-fluorophenoxy)-1,2,4-triazin-5-yl)-2,6-diazaspiro[3.4]octane-2-carboxylate (**intermediate 2**) (300 mg, 0.583 mmol) in DCM (5 mL) was added TFA (0.5 mL, 6.4 mmol), the resulting mixture was stirred at RT for 3 h. Then 10% NaOH (5 mL) solution was slowly added into the mixture to adjust the pH value to about 12, the resulting mixture was extracted with DCM (10 mL x 3). The combined organic layers were dried over anhydrous Na₂SO₄, filtered, and concentrated *in vacuo* to afford the title intermediate (220 mg, 90% yield) as a white solid.

### Preparation of intermediate 59, 75, 85

### 6-(6-(2-(4-cyclopropylpyrimidin-5-yl)-4-fluorophenoxy)-1,2,4-triazin-5-yl)-2,6-diazaspiro[3.4]octane

### 2-((4-(2,6-diazaspiro[3.4]octan-6-yl)pyridazin-3-yl)oxy)-N-ethyl-5-fluoro-N-isopropylbenzamide

### 2-((5-(2,6-diazaspiro[3.4]octan-6-yl)-1,2,4-triazin-6-yl)oxy)-5-fluoro-N,N-diisopropylbenzamide

**The following intermediates were synthesized by an analogous method described above for intermediate 3**

| **Int. No.** | **Structure** | **Starting Material** |
|---|---|---|
| 59 | 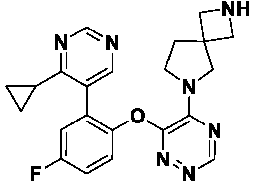 | intermediate 58 |
| 75 | 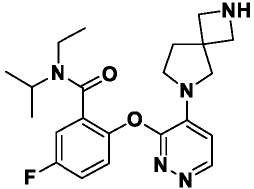 | intermediate 74 |
| 85 | 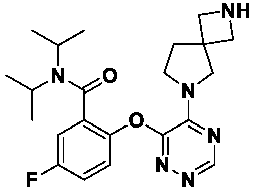 | intermediate 84 |

### Preparation of intermediate 160

### N-methoxy-N-methyl-4-(methylamino)butanamide hydrochloride

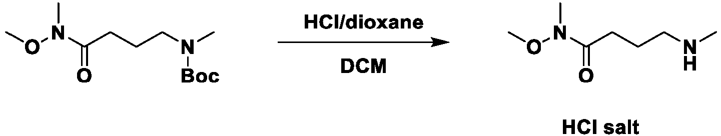

To a solution of *tert*-butyl (4-(methoxy(methyl)amino)-4-oxobutyl)(methyl)carbamate (**intermediate 8**) (220 g, crude) in DCM (200 mL) was slowly added HCl/1,4-dioxane (750 mL, 3 mol) at 0 °C. The resulting mixture was slowly warmed to RT and stirred at this temperature for 2 h. The mixture was concentrated *in vacuo* to afford the title intermediate (197 g, crude) which was used directly in next step without further purification.

### Intermediate 164, 238, 243, 244

### N-(2-methoxyethyl)-N,5-dimethyl-4-(2,6-diazaspiro[3.4]octan-6-yl)hexan-1-amine hydrochloride

### 2-((3-chloro-5-(2,6-diazaspiro[3.4]octan-6-yl)-1,2,4-triazin-6-yl)oxy)-N-ethyl-5-fluoro-N-isopropylbenzamide hydrochloride

### 2-((5-(2,6-diazaspiro[3.4]octan-6-yl)-1,2,4-triazin-6-yl)oxy)-5-fluoro-N,N-diisopropylbenzamide hydrochloride

### 2-((5-(2,6-diazaspiro[3.4]octan-6-yl)-1,2,4-triazin-6-yl)oxy)-N-ethyl-5-fluoro-N-isopropylbenzamide hydrochloride

**The following intermediates were synthesized by an analogous method described above for intermediate 160**

| **Int. No.** | **Structure** | **Starting Material** |
|---|---|---|
| 164 | 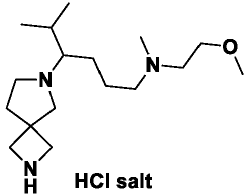 | intermediate 163 |
| 238 | 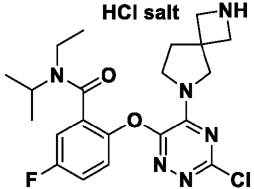 | intermediate 14 |
| 243 | 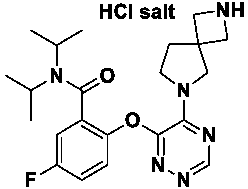 | intermediate 84 |
| 244 | 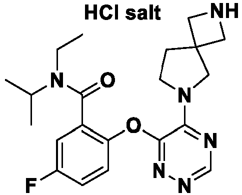 | intermediate 2 |

### Preparation of intermediate 71

### 2-((4-(2,6-diazaspiro[3.4]octan-6-yl)pyridazin-3-yl)oxy)-5-fluoro-N,N-diisopropylbenzamide

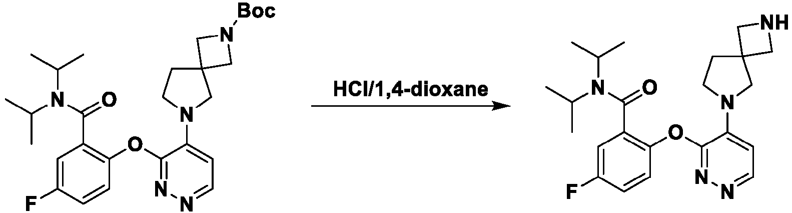

To the solution of *tert*-butyl 6-(3-(2-(diisopropylcarbamoyl)-4-fluorophenoxy)pyridazin-4-yl)-2,6-diazaspiro[3.4]octane-2-carboxylate (**intermediate 70**) (5.0 g, 9.4 mmol) in 1,4-dioxane (30 mL) cooled at 0 °C was slowly added HCl in 1.4-dioxane (20 mL, 4 M, 80 mmol) The resulting mixture was stirred at RT for 2 h. Then, the mixture was concentrated and the residue was re-dissolved in DCM (50 mL), to which 1 M NaOH (20 mL) was slowly added and the pH value was adjusted to 12, the resulting mixture was extracted by DCM (30 mL x 3). The combined organic layers were dried over anhydrous Na₂SO₄, filtered, and concentrated *in vacuo* to afford the title intermediate (4 g, crude) as a yellow solid, which was used in the next step without further purification.

### Preparation of intermediate 29

### tert-butyl 2,2-dimethyl-5-oxopyrrolidine-1-carboxylate

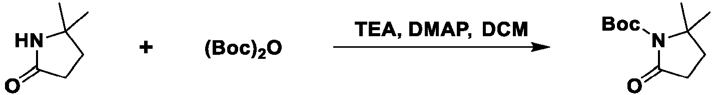

To a solution of 5,5-dimethylpyrrolidin-2-one (3.00 g, 26.5 mmol) in DCM (30 mL) were added TEA (8.10 g, 80.0 mmol) and DMAP (325 mg, 2.66 mmol), and followed by addition of di-*tert*-butyl dicarbonate (8.70 g, 39.8 mmol). The reaction was stirred at 40 °C overnight. After cooled to RT, the reaction mixture was washed with brine (30 mL x 2), dried over Na₂SO₄, filtered and concentrated under reduced pressure to give a crude product. The crude product was further purified by FCC on silica gel (PE/EtOAc = 100:0 to 3:1) to afford the title intermediate (2.8 g, 50% yield) as a yellow powder.

### Preparation of intermediate 1

### tert-butyl (5-methyl-4-oxohexyl)carbamate

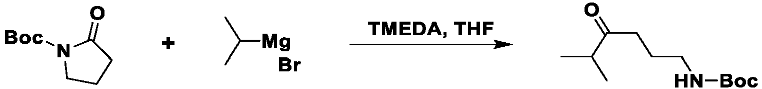

To a solution of *tert*-butyl 2-oxopyrrolidine-1-carboxylate (5.0 g, 27 mmol) and TMEDA (5.0 mL, 33 mmol) in THF (60 mL) cooled at -70 °C was slowly added isopropylmagnesium bromide solution (19 mL, 55 mmol, 2.9 M in 2-methyltetrahydrofuran), the resulting mixture was slowly warmed to RT and stirred for 12 h. The mixture was poured into sat. aq. NH₄Cl (50 mL) solution and extracted with EtOAc (50 mL x 3). The combined organic layers were dried over anhydrous Na₂SO₄, filtered, and concentrated under reduced pressure to give the crude product, which was further purified by FCC (PE/EtOAc = 1:0 to 100:1) to afford the title intermediate (3.7 g, 60% yield) as a yellow oil.

### Preparation of intermediate 30, 110, 141

### tert-butyl (2,6-dimethyl-5-oxoheptan-2-yl)carbamate

### tert-butyl (6-methyl-5-oxoheptyl)carbamate

### 6-hydroxy-2,4-dimethylhexan-3-one

**The following intermediates were synthesized by an analogous method described above for intermediate 1**

| **Int. No.** | **Structure** | **Starting Materials** |
|---|---|---|
| 30 | 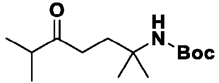 | isopropylmagnesium bromide, intermediate 29 |
| 110 | 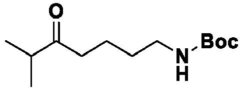 | isopropylmagnesium bromide, *tert*-butyl 2-oxopiperidine-1-carboxylate |
| 141 | 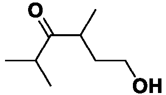 | isopropylmagnesium chloride, 3-methyldihydrofuran-2(3*H*)-one |

### Preparation of intermediate 34

### benzyl 2,2-dimethyl-5-oxopyrrolidine-1-carboxylate

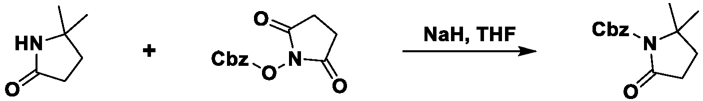

To a solution of 5,5-dimethylpyrrolidin-2-one (5.00 g, 44.2 mmol) in THF (150 mL) cooled at 0 °C was added NaH (1.94 g, 48.5 mmol, 60%), the resulting mixture was stirred at this temperature for 30 min. Subsequently *N*-(benzyloxycarbonyloxy)succinimide (12.1 g, 48.6 mmol) was added and the reaction mixture was slowly warmed to RT and stirred for additional 16 h. The solvent was evaporated under reduced pressure, sat. aq. NH₄Cl solution (30 mL) was added and extracted with EtOAc (2 x 30 mL). The combined organic layers were washed with brine (40 mL), dried over Na₂SO₄, filtered, and concentrated under reduced pressure to afford the crude product, which was further purified by FCC (PE/EtOAc = 1:0 to 3: 1) to afford the title intermediate (5.16 g, 39% yield) as colorless oil.

### Preparation of intermediate 35

### 4-(((benzyloxy)carbonyl)amino)-4-methylpentanoic acid

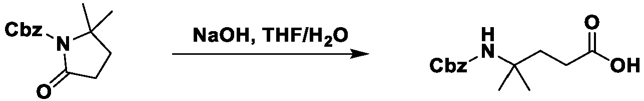

NaOH (4.18 g, 16.9 mmol) was added to a solution of benzyl 2,2-dimethyl-5-oxopyrrolidine-1-carboxylate (**intermediate 34**) (5.16 g, 20.9 mmol) in THF (60 mL) and H₂O (15 mL). The mixture was stirred at 80 °C for 16 h. The reaction mixture was cooled to 25 °C and acidified by 1 M HCl to adjust the pH value to about 3, then the mixture was extracted by EtOAc (20 x 2 mL). The combined organic layers were washed with brine (20 mL), dried over Na₂SO₄, filtered, and concentrated *in vacuo* to afford the title intermediate (4.48 g, crude) as colorless oil, which was used directly in next step without further purification.

### Preparation of intermediate 7

### 4-((tert-butoxycarbonyl)(methyl)amino)butanoic acid

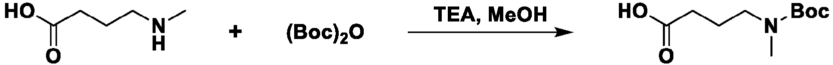

To a solution of 4-(methylamino)butanoic acid hydrochloride (3.0 g, 19.5 mmol) and TEA (7.78 mL, 58.6 mmol) in MeOH (30 mL) was added Boc₂O (4.69 g, 21.5 mmol) dropwise. The mixture was stirred at RT for 2 h. The mixture was concentrated under reduced pressure and the residue was diluted with EtOAc (100 mL), washed with cooled 0.1 N HCl (70 mL x 2), H₂O (50 mL x 2) and brine (50 mL), dried over Na₂SO₄, filtered and concentrated to afford the title intermediate (1.80 g, crude) as colorless oil.

### Preparation of intermediate 8

### tert-butyl (4-(methoxy(methyl)amino)-4-oxobutyl)(methyl)carbamate

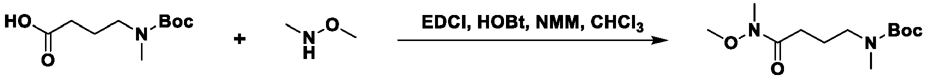

To a solution of 4-((*tert*-butoxycarbonyl)(methyl)amino)butanoic acid (**intermediate 7**) (1.80 g, crude) in CHCl₃ (30 mL) was added *N,O*-dimethylhydroxylamine hydrochloride (960 mg, 9.84 mmol), HOBt (1.24 g, 9.18 mmol) and NMM (2.80 mL, 25.1 mmol). And, then EDCI (2.23 g, 11.6 mmol) was added and the reaction mixture was stirred at RT for 4 h. The reaction mixture was diluted with DCM (100 mL), washed with 1N HCl (30 mL x 3), sat. aq. NaHCO₃ (30 mL x 3) and brine (30 mL), dried over Na₂SO₄, filtered and concentrated under *in vacuo* to afford the title intermediate (1.70 g, crude) as colorless oil.

### Preparation of intermediates 19, 36, 189, 190, 203, 204

### tert-butyl (3-(methoxy(methyl)amino)-3-oxopropyl)carbamate

### benzyl (5-(methoxy(methyl)amino)-2-methyl-5-oxopentan-2-yl)carbamate

### (S)-3-((tert-butyldiphenylsilyl)oxy)-4-(ethyl(methyl)amino)-N-methoxy-N-methylbutanamide

### (R)-3-((tert-butyldiphenylsilyl)oxy)-4-(ethyl(methyl)amino)-N-methoxy-N-methylbutanamide

### (S)-3-((tert-butyldiphenylsilyl)oxy)-N-methoxy-4-((2-methoxyethyl)(methyl)amino)-N-methylbutanamide

### (R)-3-((tert-butyldiphenylsilyl)oxy)-N-methoxy-4-((2-methoxyethyl)(methyl)amino)-N-methylbutanamide

**The following intermediates were synthesized by an analogous method described above for intermediate 8**

| **Int. No.** | **Structure** | **Starting Materials** |
|---|---|---|
| 19 | 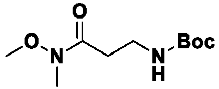 | 3-((*tert*-butoxycarbonyl)amino)propanoic acid *N,O*-dimethylhydroxylamine hydrochloride |
| 36 | 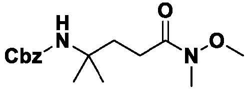 | intermediate 35 *N,O*-dimethylhydroxylamine hydrochloride |
| 189 | 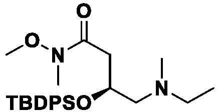 | intermediate 187, *N,O*-dimethylhydroxylamine hydrochloride |
| 190 | 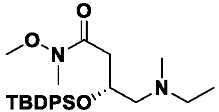 | intermediate 188, *N,O*-dimethylhydroxylamine hydrochloride |
| 203 | 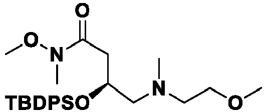 | intermediate 201, *N*,*O*-dimethylhydroxylamine hydrochloride |
| 204 | 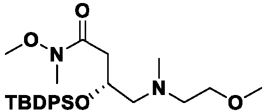 | intermediate 202, *N*,*O*-dimethylhydroxylamine hydrochloride |

### Preparation of intermediate 37

### benzyl (5-(methoxy(methyl)amino)-2-methyl-5-oxopentan-2-yl)(methyl)carbamate

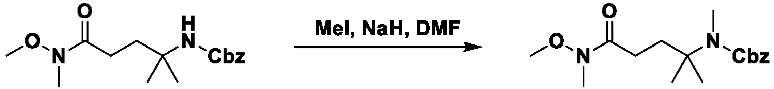

To a solution of benzyl (5-(methoxy(methyl)amino)-2-methyl-5-oxopentan-2-yl)carbamate **(intermediate 36)** (2.30 g, 7.46 mmol) in DMF (30 mL) cooled at 0 °C under N₂ atmosphere was added NaH (358 mg, 8.95 mmol, 60%). Then, MeI (8.87 g, 62.5 mmol) was added and the mixture was stirred at 25 °C for 12 h. The mixture was quenched with sat. aq. NH₄Cl (30 mL) and extracted with EtOAc (30 mL x 2). The combined organic layers washed with brine (40 mL), dried over Na₂SO₄, filtered and concentrated *in vacuo* to give the crude product, which was further purified by FCC on silica gel (PE/EtOAc = 1:0 to 3:1) to afford the title intermediate (2.15 g, 76% yield) as yellow oil.

### Preparation of intermediate 236

### N-(ethyl-¹³C₂)-5-fluoro-2-methoxy-N-(propan-2-yl-¹³C₃)benzamide

**The following intermediate was synthesized by an analogous method as described above for intermediate 37**

| **Int. No.** | **Structure** | **Starting Materials** | **Conditions** |
|---|---|---|---|
| 236 | 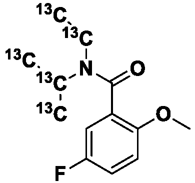 | intermediate 235, iodoethane-1,2-¹³*C*₂ | NaH, DMF, from 0 °C to 90 °C |

### Preparation of intermediate 9

### tert-butyl methyl(5-methyl-4-oxohexyl)carbamate

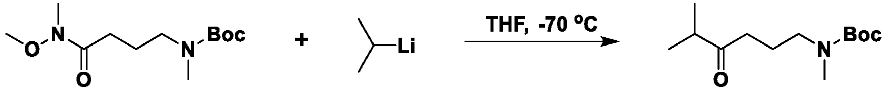

To a solution of *tert*-butyl (4-(methoxy(methyl)amino)-4-oxobutyl)(methyl)carbamate **(intermediate 8)** (200 mg, crude) in THF (5 mL) cooled at -70°C under N₂ atmosphere was added dropwise isopropyllithium (3.2 mL, 2.24 mmol, 0.7M in pentane). The resulting mixture was stirred at -70 °C for 2 h. The mixture was quenched with sat. aq. NH₄Cl (15 mL), extracted with EtOAc (30 mL x 2). The combined organic layers were washed with brine (30 mL), dried over Na₂SO₄, filtered and concentrated under reduced pressure to give a crude product. The crude product was further purified by FCC (PE/EtOAc = 10 : 1) to afford the title intermediate (60 mg) as colorless oil.

### Preparation of intermediates 20, 38, 162, 191, 192, 205, 206

### tert-butyl (4-methyl-3-oxopentyl)carbamate

### benzyl (2,6-dimethyl-5-oxoheptan-2-yl)(methyl)carbamate

### 6-((2-methoxyethyl)(methyl)amino)-2-methylhexan-3-one

### (S)-5-((tert-butyldiphenylsilyl)oxy)-6-(ethyl(methyl)amino)-2-methylhex-1-en-3-one

### (R)-5-((tert-butyldiphenylsilyl)oxy)-6-(ethyl(methyl)amino)-2-methylhex-1-en-3-one

### (S)-5-((tert-butyldiphenylsilyl)oxy)-6-((2-methoxyethyl)(methyl)amino)-2-methylhex-1-en-3-one

### (R)-5-((tert-butyldiphenylsilyl)oxy)-6-((2-methoxyethyl)(methyl)amino)-2-methylhex-1-en-3-one

**The following intermediates were synthesized by an analogous method described above for intermediate 9**

| **Int. No.** | **Structure** | **Starting Materials** |
|---|---|---|
| 20 | 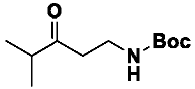 | intermediate 19, isopropylmagnesium chloride |
| 38 | 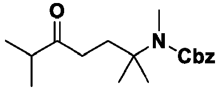 | intermediate 37 isopropylmagnesium chloride |
| 162 | 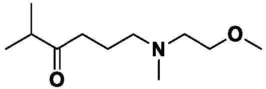 | intermediate 161, isopropyllithium |
| 191 | 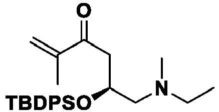 | intermediate 189, isopropenylmagnesium bromide |
| 192 | 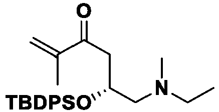 | intermediate 190, isopropenylmagnesium bromide |
| 205 | 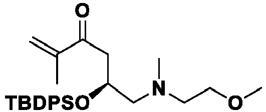 | intermediate 203, isopropenylmagnesium bromide |
| 206 | 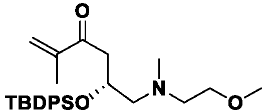 | intermediate 204, isopropenylmagnesium bromide |

### Preparation of intermediate 15

### 2-(3-methyl-2-oxobutyl)isoindoline-1,3-dione

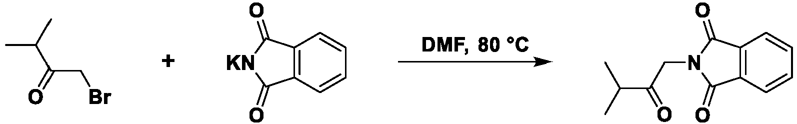

To the solution of 1-bromo-3-methylbutan-2-one (200 mg, 1.21 mmol) in DMF (4 mL) was added potassium phthalimide (1.12 g, 6.05 mmol) and the mixture was stirred at 80 °C for 12 h. After cooled to RT, water (15 mL) was added and the mixture was extracted with EtOAc (40 mL x 3). The combined organic layers were dried over anhydrous Na₂SO₄, filtered and concentrated under reduced pressure to afford the crude product, which was further purified by preparative TLC (PE/EtOAc = 3:1) to afford the title intermediate (200 mg, 69% yield) as a white solid.

### Preparation of intermediate 46

### methyl 5-methyl-4-oxohexanoate

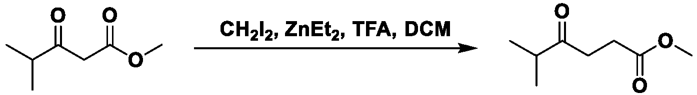

To a solution of ZnEt₂ (104 mL, 104 mmol) in DCM (150 mL) at 0 °C under N₂ was added dropwise TFA (11.9 g, 104 mmol) slowly via syringe and the mixture was stirred at 0 °C for 30 min. Then, methylene iodide (27.9 g, 104 mmol) was added dropwise with stirring and the suspension was stirred for another 30 min. And, then methyl 4-methyl-3-oxopentanoate (5.00 g, 34.7 mmol) was added rapidly by syringe and the resulting mixture was stirred at RT for 16 h and refluxed at 50 °C for 20 h. After cooled to RT, the reaction mixture was quenched with sat. aq. NH₄Cl (50 mL) and extracted with EtOAc (30 mL x 3). The combined organic layers were washed with brine, dried over MgSO₄, and concentrated under reduced pressure to an oil residue which was purified by FCC (PE/EtOAc = 1:0 to 20:1) to afford the title intermediate (300 mg, 5% yield) as a yellow oil.

### Preparation of intermediate 22

### tert-butyl (4-methyl-3-(2,6-diazaspiro[3.4]octan-2-yl)pentyl)carbamate hydrochloride

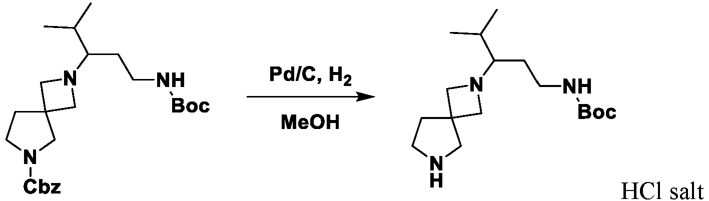

To a solution of benzyl 2-(1-((*tert*-butoxycarbonyl)amino)-4-methylpentan-3-yl)-2,6-diazaspiro[3.4]octane-6-carboxylate **(intermediate** 2**1)** (0.580 g, 1.30 mmol) in MeOH (50 mL) were added 1,1,2-trichloroethane (0.260 g, 1.95 mmol) and Pd/C (0.05 g, 10%) under Ar and the reaction was stirred at 35 °C for 8 h under H₂ (15 psi) atmosphere. The reaction mixture was filtered. The filtrate was concentrated *in vacuo* to afford the title intermediate (280 mg, crude) as colorless oil.

### Preparation of intermediate 23

### ethyl 6-(2-(ethyl(isopropyl)carbamoyl)-4-fluorophenoxy)-1,2,4-triazine-5-carboxylate

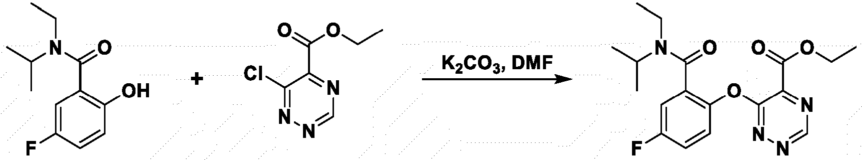

To the mixture of ethyl 6-chloro-1,2,4-triazine-5-carboxylate (13 g, 69 mmol) and *N*-ethyl-5-fluoro-2-hydroxy-*N*-isopropylbenzamide **(intermediate 28)** (15.6 g, 69.3 mmol) in DMF (150 mL) was added K₂CO₃ (28.6 g, 204 mmol). The resulting mixture was stirred at RT for 2 h. The reaction mixture was filtered and the filtrate was concentrated under reduced pressure to give the crude residue, which was diluted with water (100 mL) and extracted with EtOAc (100 mL x 2). The combined organic layers were dried over anhydrous Na₂SO₄, filtered, and concentrated under reduced pressure to give the crude product, which was further purified by FCC (PE/EtOAc = 1:0 to 1:1) to afford the title intermediate (30 g, 81% purity, 92% yield) as a yellow solid.

### Preparation of intermediate 24

### 6-(2-(ethyl(isopropyl)carbamoyl)-4-fluorophenoxy)-1,2,4-triazine-5-carboxylic acid

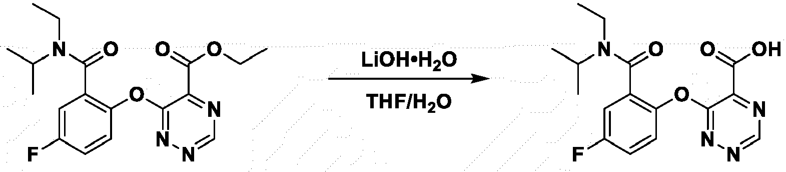

To the mixture of ethyl 6-(2-(ethyl(isopropyl)carbamoyl)-4-fluorophenoxy)-1,2,4-triazine-5-carboxylate **(intermediate 23)** (8.6 g, 23 mmol) in THF (100 mL) and H₂O (25 mL) was added LiOH•H₂O (2.0 g, 48 mmol) and the reaction mixture was stirred at RT for 1 h. The mixture was acidified with 0.5M HCl to adjust the pH value to 5~6, and further extracted with EtOAc (150 mL). The aqueous phase was purified by preparative HPLC over Boston Prime (column: C18 150x30 mm 5 um; eluent: ACN/H₂O (0.225% FA) from 19% to 49%, v/v) to afford the title intermediate (5.0 g, 62% yield).

### Preparation of intermediates 187, 188, 201, 202

### (S)-3-((tert-butyldiphenylsilyl)oxy)-4-(ethyl(methyl)amino)butanoic acid

### (R)-3-((tert-butyldiphenylsilyl)oxy)-4-(ethyl(methyl)amino)butanoic acid

### (S)-3-((tert-butyldiphenylsilyl)oxy)-4-((2-methoxyethyl)(methyl)amino)butanoic acid

### (R)-3-((tert-butyldiphenylsilyl)oxy)-4-((2-methoxyethyl)(methyl)amino)butanoic acid

**The following intermediates were synthesized by an analogous method as described above for intermediate 24**

| **Int. No.** | **Structure** | **Starting Material** | **Conditions** |
|---|---|---|---|
| 187 | 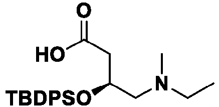 | intermediate 185 | NaOH, THF/MeOH/H₂O, RT |
| 188 | 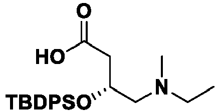 | intermediate 186 | NaOH, THF/MeOH/H₂O, RT |
| 201 | 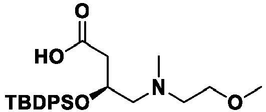 | intermediate 199 | NaOH, THF/EtOH/H₂O, RT |
| 202 | 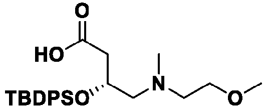 | intermediate 200 | NaOH, THF/MeOH/H₂O, RT |

### Preparation of intermediate 25

### N-ethyl-5-fluoro-2-((5-hydroxy-1,2,4-triazin-6-yl)oxy)-N-isopropylbenzamide

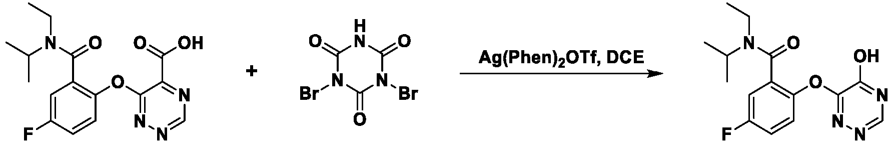

To the solution of 6-(2-(ethyl(isopropyl)carbamoyl)-4-fluorophenoxy)-1,2,4-triazine-5-carboxylic acid **(intermediate 24)** (50 mg, 0.14 mmol) and 1,3-dibromo-1,3,5-triazinane-2,4,6-trione (50 mg, 0.17 mmol) in DCE (1 mL) was added Ag(Phen)₂OTf (30 mg, 0.049 mmol) and the resulting mixture was stirred at RT for 2 h. The reaction mixture was filtered through a celite pad and washed with ACN (10 mL). The filtrate was concentrated under reduced pressure to afford the crude product, which was further purified by preparative HPLC using a Xtimate (column: C18 150x40 mm 10 µm; eluent: ACN/H₂O (0.2% FA) from 20% to 50% v/v) to afford the title intermediate (20 mg, 41%) as a white solid.

### Preparation of intermediate 159

### N-ethyl-5-fluoro-N-isopropyl-2-((5-(2,2,2-trifluoroethoxy)-1,2,4-triazin-6-yl)oxy)benzamide

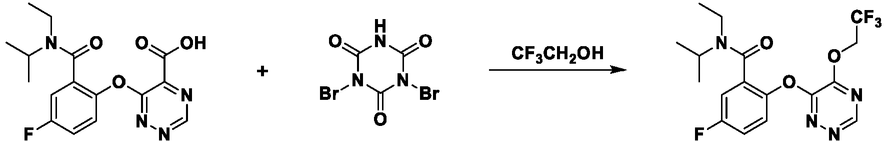

4Å molecular sieve (8 g) was added to the mixture of 6-(2-(ethyl(isopropyl)carbamoyl)-4-fluorophenoxy)-1,2,4-triazine-5-carboxylic acid **(intermediate 24)** (8.0 g, 23.0 mmol) in 2,2,2-trifluoroethan-1-ol (100 mL). The resulting mixture was stirred under N₂ atmosphere at 70 °C for 1 h. Then cooled to RT and 1,3-dibromo-1,3,5-triazinane-2,4,6-trione (13.1 g, 45.7 mmol) was added to above mixture. The resulting mixture was further stirred under N₂ atmosphere at RT overnight. The reaction mixture was filtered over a celite pad. The filtrate was concentrated under reduced pressure and the crude residue was purified by FCC (PE : EtOAc from 1:0 to 2:1) to afford the title intermediate (3.1 g, purity 84%, yield 28%) as a yellow solid.

### Preparation of intermediate 51

### 4-((tert-butyldimethylsilyl)oxy)butan-1-ol

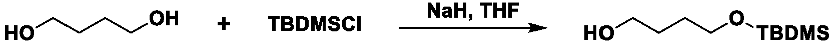

To the solution of butane-1,4-diol (5.00 g, 55.5 mmol) in THF (100 mL) cooled at 0 °C was added NaH (1.55 g, 38.8 mmol, 60%), the resulting mixture was stirred at 0 °C for 20 min. Then TBDMSCl (5.85 g, 38.8 mmol) was added to the reaction mixture and the reaction was further stirred at 0 °C for additional 1 h. The mixture was quenched with water (80 mL) and extracted with EtOAc (80 mL x 3). The combined organic layers were dried over anhydrous Na₂SO₄, filtered and concentrated under reduced pressure to afford the crude product which was further purified by FCC (PE/EtOAc = 1:0 to 10: 1) to afford the title intermediate (7.2 g, 63%) as a colorless liquid.

### Preparation of intermediates 183, 184

### ethyl (S)-3-((tert-butyldiphenylsilyl)oxy)-4-iodobutanoate

### ethyl (R)-3-((tert-butyldiphenylsilyl)oxy)-4-iodobutanoate

**The following intermediates were synthesized by an analogous method as described above for intermediate 51**

| **Int. No.** | **Structure** | **Starting Materials** | **Conditions** |
|---|---|---|---|
| 183 | 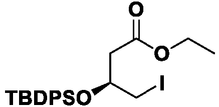 | TBDPSCl, intermediate 181 | imidazole, DCM, RT |
| 184 | 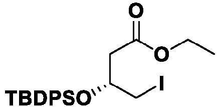 | TBDPSCl, intermediate 182 | imidazole, DCM, RT |

### Preparation of intermediate 52

### 4-((tert-butyldimethylsilyl)oxy)butanal

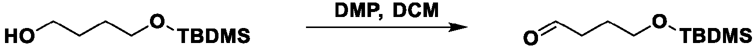

To the solution of 4-((*tert*-butyldimethylsilyl)oxy)butan-1-ol **(intermediate 51)** (7.20 g, 35.2 mmol) in DCM (200 mL) cooled at 0 °C was added DMP (22.4 g, 52.8 mmol) and the reaction mixture was slowly warmed to RT and stirred for 2 h. The reaction mixture was diluted with DCM (100 mL) and stirred with of sat. aq. (NaHCO₃/Na₂SO₃ = 1/1, 100 mL) for 2 min, the separated organic layer was washed with brine (100 mL x 3), dried over anhydrous Na₂SO₄, filtered, and concentrated under reduced pressure to give the crude product which was further purified by FCC (PE/EtOAc = 1:0 to 12: 1) to afford the title intermediate (2.95 g, 41%) as a colorless liquid.

### Preparation of intermediate 54, 145, 146, 158

### 6-((tert-butyldimethylsilyl)oxy)-2-methylhexan-3-one

### 2-((5-(2-(2,4-dimethyl-6-oxohexan-3-yl)-2,6-diazaspiro[3.4]octan-6-yl)-1,2,4-triazin-6-yl)oxy)-N-ethyl-5-fluoro-N-isopropylbenzamide (mixture of R,S and S,R; or mixture of R,R and S,S)

### 2-((5-(2-(2,4-dimethyl-6-oxohexan-3-yl)-2,6-diazaspiro [3.4] octan-6-yl)-1,2,4-triazin-6-yl)oxy)-N-ethyl-5-fluoro-N-isopropylbenzamide (mixture of R,R and S,S; or mixture of R,S and S,R)

### (R)-N-ethyl-5-fluoro-N-isopropyl-2-((5-(2-(2-methyl-6-oxoheptan-3-yl)-2,6-diazaspiro[3.4]octan-6-yl)-1,2,4-triazin-6-yl)oxy)benzamide

**The following intermediates were synthesized by an analogous method described above for intermediate 52**

| **Int. No.** | **Structure** | **Starting Material** |
|---|---|---|
| 54 | 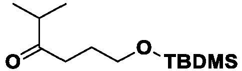 | intermediate 53 |
| 145 | **mixture of R,S and S,R or mixture of R,R and S,S** | Compound 261 |
| | 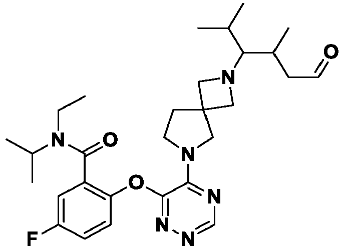 | |
| 146 | **mixture of R,R and S,S or mixture of R,S and *S,R*** | Compound 262 |
| | 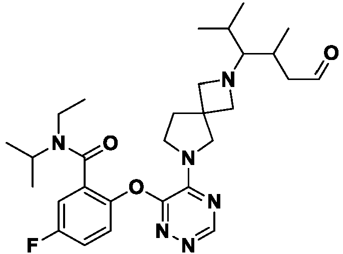 | |
| 158 | 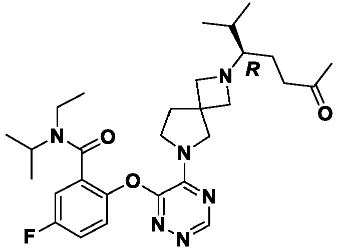 | Compound 298 |

### Preparation of intermediate 53

### 6-((tert-butyldimethylsilyl)oxy)-2-methylhexan-3-ol

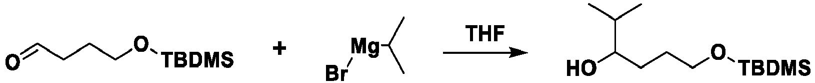

To the solution of 4-((*tert*-butyldimethylsilyl)oxy)butanal **(intermediate 52)** (1.00 g, 4.94 mmol) in THF (4.9 mL) cooled at -20 °C under N₂ atmosphere was added dropwise isopropylmagnesium bromide (4.94 mL, 14.8 mmol, 3 M in THF) and the reaction mixture was slowly warmed to RT and stirred for 2 h. The mixture was quenched with sat. aq. NH₄Cl (20 mL), and extracted with EtOAc (50 mL x 3). The combined organic layers were dried over anhydrous Na₂SO₄, filtered, and concentrated *in vacuo* to give the crude product which was further purified by FCC (PE/EtOAc = 1:0 to 20: 1) to afford the title intermediate (580 mg, 48%) as a white oil.

### Preparation of intermediates 16, 21, 39, 47, 55, 94, 98, 161, 163

### 2-((5-(2-(1-(1,3-dioxoisoindolin-2-yl)-3-methylbutan-2-yl)-2,6-diazaspiro[3.4]octan-6-yl)-1,2,4-triazin-6-yl)oxy)-N-ethyl-5-fluoro-N-isopropylbenzamide

### benzyl 2-(1-((tert-butoxycarbonyl)amino)-4-methylpentan-3-yl)-2,6-diazaspiro [3.4] octane-6-carboxylate

### benzyl (5-(6-(6-(2-(ethyl(isopropyl)carbamoyl)-4-fluorophenoxy)-1,2,4-triazin-5-yl)-2,6-diazaspiro[3.4]octan-2-yl)-2,6-dimethylheptan-2-yl)(methyl)carbamate

### methyl 4-(6-(6-(2-(4-cyclopropylpyrimidin-5-yl)-4-fluorophenoxy)-1,2,4-triazin-5-yl)-2,6-diazaspiro[3.4]octan-2-yl)-5-methylhexanoate

### 2-((5-(2-(6-((tert-butyldimethylsilyl)oxy)-2-methylhexan-3-yl)-2,6-diazaspiro[3.4]octan-6-yl)-1,2,4-triazin-6-yl)oxy)-N-ethyl-5-fluoro-N-isopropylbenzamide

### 2-((5-(2-(1-(1,3-dioxolan-2-yl)-4-methylpentan-3-yl)-2,6-diazaspiro[3.4]octan-6-yl)-1,2,4-triazin-6-yl)oxy)-N-ethyl-5-fluoro-N-isopropylbenzamide

### 2-((5-(2-(1-(1,3-dioxolan-2-yl)-4-methylpentan-3-yl)-2,6-diazaspiro[3.4]octan-6-yl)-1,2,4-triazin-6-yl)oxy)-5-fluoro-N,N-diisopropylbenzamide

### N-methoxy-4-((2-methoxyethyl)(methyl)amino)-N-methylbutanamide

### tert-butyl 6-(6-((2-methoxyethyl)(methyl)amino)-2-methylhexan-3-yl)-2,6-diazaspiro[3.4]octane-2-carboxylate

**The following intermediates were synthesized by an analogous method as described for Compound 60 and Compound 61**

| **Int. No.** | **Structure** | **Starting Materials** | **Conditions** |
|---|---|---|---|
| 16 | 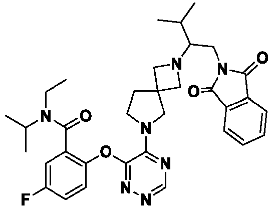 | intermediate 3, intermediate 15 | ZnCl₂, NaBH₃CN, MeOH, 65 °C |
| 21 | 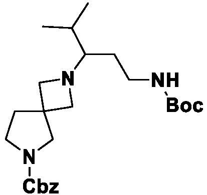 | intermediate 20, benzyl 2,6-diazaspiro[3.4]octan e-6-carboxylate | AcOH, NaBH₃CN, MeOH, 45 °C |
| 39 | 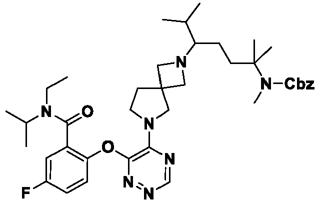 | intermediate 3, intermediate 38 | ZnCl₂, NaBH₃CN, MeOH, 65 °C |
| 47 | 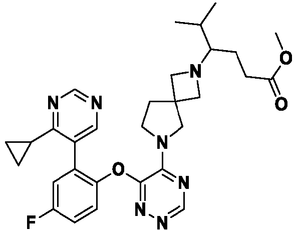 | intermediate 59 intermediate 46 | ZnCl₂, NaBH₃CN, MeOH, 80 °C |
| 55 | 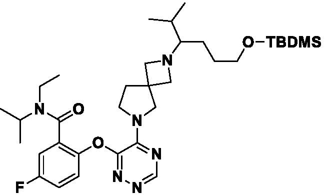 | intermediate 3, intermediate 54 | ZnCl₂, NaBH₃CN, MeOH, 80 °C |
| 94 | 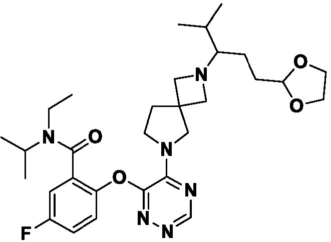 | intermediate 3, intermediate 93 | AcOH, NaBH₃CN, MeOH, 45 °C |
| 98 | 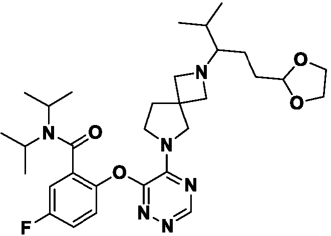 | intermediate 85, intermediate 93 | AcOH, NaBH₃CN, MeOH, 60 °C |
| 161 | 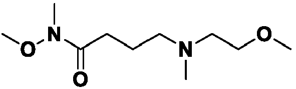 | intermediate 160, 1,1,2-trimethoxyethane, HCl | AcOH, NaBH₃CN, EtOH, RT |
| 163 | 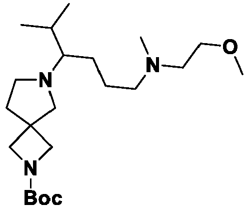 | intermediate 162, tert-butyl 2,6-diazaspiro[3.4]octan e-2-carboxylate | NaOAc, NaBH₃CN, MeOH, 55 °C |

### Preparation of intermediate 17 and 18

### (*R)-2-((5-(2-(1-(1,3-dioxoisoindolin-2-yl)-3-methylbutan-2-yl)-2,6-diazaspiro[3.4]octan-6-yl)-1,2,4-triazin-6-yl)oxy)-N-ethyl-5-fluoro-N-isopropylbenzamide

### (*S)-2-((5-(2-(1-(1,3-dioxoisoindolin-2-yl)-3-methylbutan-2-yl)-2,6-diazaspiro[3.4]octan-6-yl)-1,2,4-triazin-6-yl)oxy)-N-ethyl-5-fluoro-N-isopropylbenzamide

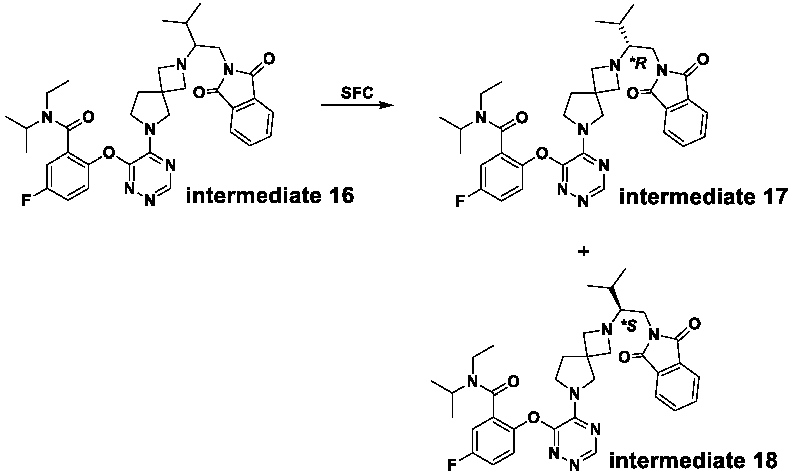

2-((5-(2-(1-(1,3-dioxoisoindolin-2-yl)-3-methylbutan-2-yl)-2,6-diazaspiro[3.4]octan-6-yl)-1,2,4-triazin-6-yl)oxy)-*N*-ethyl-5-fluoro-*N*-isopropylbenzamide **(intermediate 16)** (200 mg, 0.254 mmol) was purified by SFC over DAICEL CHIRALCEL OD (column: 250x50 mm 10µm; Mobile phase: A: Supercritical CO₂, B: IPA (0.1% ammonia), A:B =65:35 at 70 mL/min; Column Temp: 38 °C; Nozzle Pressure: 100 Bar; Nozzle Temp: 60 °C; Evaporator Temp: 20 °C; Trimmer Temp: 25 °C; Wavelength: 220 nm) to afford the title intermediates **intermediate 17** (100 mg, 95% purity, 42% yield) and **intermediate 18** (100 mg, 99% purity, 44% yield) both as colorless oil.

### Preparation of intermediate 40 and 41

### benzyl (*R)-(5-(6-(6-(2-(ethyl(isopropyl)carbamoyl)-4-fluorophenoxy)-1,2,4-triazin-5-yl)-2,6-diazaspiro[3.4]octan-2-yl)-2,6-dimethylheptan-2-yl)(methyl)carbamate

### benzyl (*S)-(5-(6-(6-(2-(ethyl(isopropyl)carbamoyl)-4-fluorophenoxy)-1,2,4-triazin-5-yl)-2,6-diazaspiro[3.4]octan-2-yl)-2,6-dimethylheptan-2-yl)(methyl)carbamate

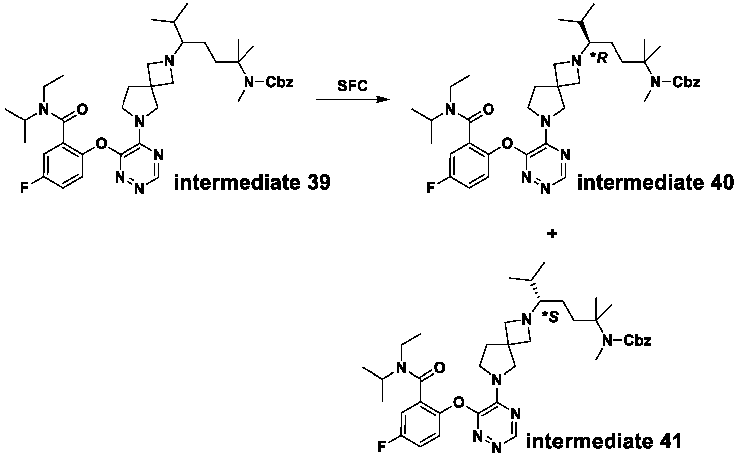

benzyl (5-(6-(6-(2-(ethyl(isopropyl)carbamoyl)-4-fluorophenoxy)-1,2,4-triazin-5-yl)-2,6-diazaspiro[3.4]octan-2-yl)-2,6-dimethylheptan-2-yl)(methyl)carbamate **(intermediate 39)** (650 mg, 0.923 mmol) was separated by SFC over DAICEL CHIRALPAK AD-H (column: 250x30mm 5µm; eluent: 30% (v/v) super critical CO₂ in EtOH (0.1% ammonia), flow rate: 60 mL/min) to afford the title intermediates **intermediate 40** (250 mg, 96% purity, 37% yield) and **intermediate 41** (220 mg, 99.9% purity, 34% yield) both as a colorless oil.

### Preparation of intermediate 48 and 49

### methyl (*R)-4-(6-(6-(2-(4-cyclopropylpyrimidin-5-yl)-4-fluorophenoxy)-1,2,4-triazin-5-yl)-2,6-diazaspiro[3.4]octan-2-yl)-5-methylhexanoate

### methyl (*S)-4-(6-(6-(2-(4-cyclopropylpyrimidin-5-yl)-4-fluorophenoxy)-1,2,4-triazin-5-yl)-2,6-diazaspiro[3.4]octan-2-yl)-5-methylhexanoate

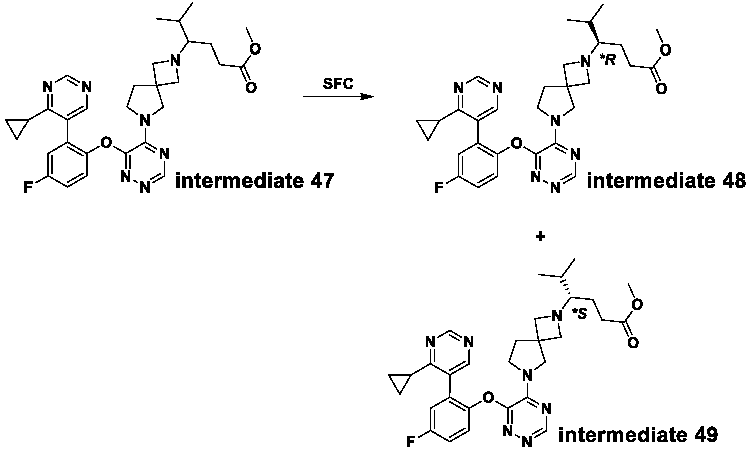

methyl 4-(6-(6-(2-(4-cyclopropylpyrimidin-5-yl)-4-fluorophenoxy)-1,2,4-triazin-5-yl)-2,6-diazaspiro[3.4]octan-2-yl)-5-methylhexanoate **(intermediate 47)** (360 mg, 0.513 mmol) was purified by SFC over Phenomenex-Cellulose-2 (column: 250x30mm, 10µm; eluent: 35%

(v/v) supercritical CO₂ in MeOH with 0.1% ammonia) to afford the title intermediates **intermediate 48** (110 mg, 35% yield) and **intermediate 49** (90 mg, 31% yield) both as white solid.

### Preparation of intermediate 93

### 1-(1,3-dioxolan-2-yl)-4-methylpentan-3-one

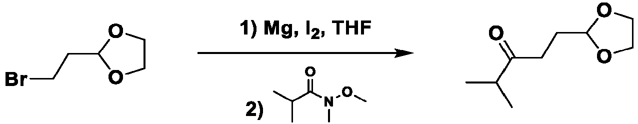

To the mixture of magnesium (6.0 g, 247 mmol) and iodine (100 mg, 0.394 mmol) in THF (70 mL) at 25 °C was slowly added a solution of 2-(2-bromoethyl)-1,3-dioxolane (20.0 g, 110 mmol) in THF (30 mL), the resulting mixture was stirred at 25 °C for 1 h. Then, the mixture was slowly added to the solution of N-methoxy-N-methylisobutyramide (10 g, 76.2 mmol) in THF (100 mL) cooled at 0 °C. The reaction mixture was slowly warmed to 25 °C and stirred at this temperature for 8 h. The mixture was quenched by sat. aq. NH₄Cl (300 mL), extracted with MTBE (200 mL x 3). The combined organic layers were dried over Na₂SO₄, filtered and concentrated under reduced pressure to give the crude product, which was purified by FCC (PE:EtOAc = 1:0 to 20:1) to afford the title intermediate (13 g, crude) as colorless oil which was used directly in next step without further purification.

### Preparation of intermediate 95 and 96

### (R)-2-((5-(2-(1-(1,3-dioxolan-2-yl)-4-methylpentan-3-yl)-2,6-diazaspiro[3.4]octan-6-yl)-1,2,4-triazin-6-yl)oxy)-N-ethyl-5-fluoro-N-isopropylbenzamide

### (S)-2-((5-(2-(1-(1,3-dioxolan-2-yl)-4-methylpentan-3-yl)-2,6-diazaspiro[3.4]octan-6-yl)-1,2,4-triazin-6-yl)oxy)-N-ethyl-5-fluoro-N-isopropylbenzamide

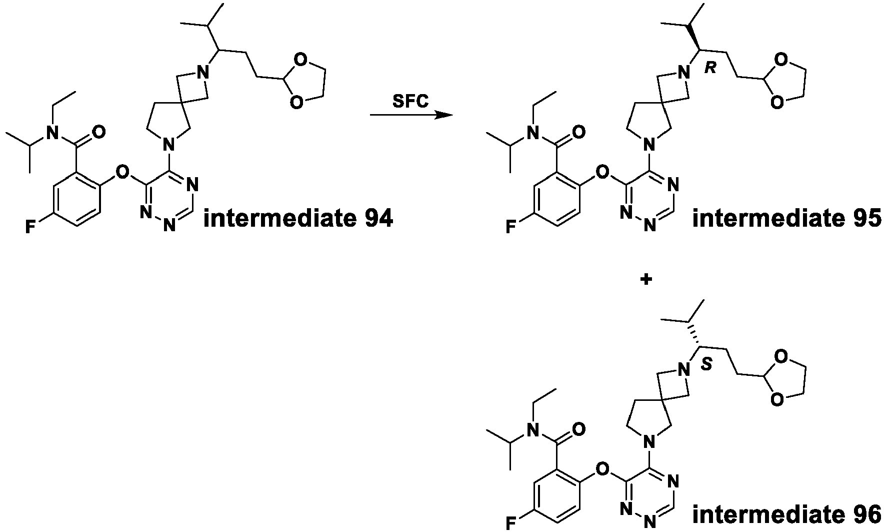

2-((5-(2-(1-(1,3-dioxolan-2-yl)-4-methylpentan-3-yl)-2,6-diazaspiro[3.4]octan-6-yl)-1,2,4-triazin-6-yl)oxy)-*N*-ethyl-5-fluoro-*N*-isopropylbenzamide **(intermediate 94)** (4.00 g, 7.01 mmol) separated by SFC over DAICEL CHIRALCEL OD (column: 250x50mm 10um; Mobile phase: A: Supercritical CO₂, B: MeOH (0.1% ammonia), A:B = 75:25 at 200 mL/min; Column Temp: 38 °C; Nozzle Pressure: 100 Bar; Nozzle Temp: 60 °C; Evaporator Temp: 20 °C; Trimmer Temp: 25 °C; Wavelength: 220 nm) to afford the title intermediates **intermediate 95** (1.72 g, 98.76% purity, 42.5% yield) and **intermediate 96** (1.57 g, 98.09% purity, 38.5% yield) as white solid.

### Preparation of intermediate 99 and 100

### (*R)-2-((5-(2-(1-(1,3-dioxolan-2-yl)-4-methylpentan-3-yl)-2,6-diazaspiro[3.4]octan-6-yl)-1,2,4-triazin-6-yl)oxy)-5-fluoro-N,N-diisopropylbenzamide

### (*S)-2-((5-(2-(1-(1,3-dioxolan-2-yl)-4-methylpentan-3-yl)-2,6-diazaspiro[3.4]octan-6-yl)-1,2,4-triazin-6-yl)oxy)-5-fluoro-N,N-diisopropylbenzamide

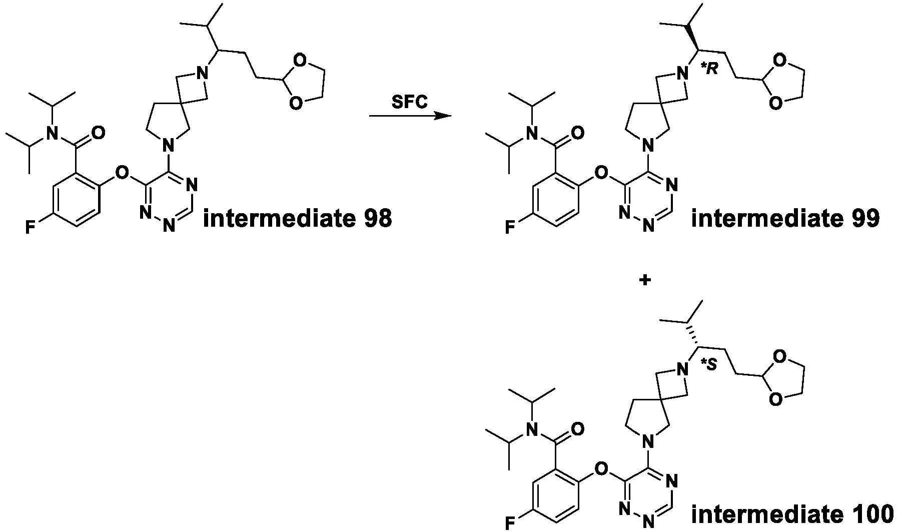

2-((5-(2-(1-(1,3-dioxolan-2-yl)-4-methylpentan-3-yl)-2,6-diazaspiro[3.4]octan-6-yl)-1,2,4-triazin-6-yl)oxy)-5-fluoro-*N,N*-diisopropylbenzamide **(intermediate 98)** (6.5 g) was separated by SFC over DAICEL CHIRALPAK IG (column: 250x50mm 10um; Mobile phase: A: Supercritical CO₂, B: MeOH (0.1% ammonia), A:B = 65:35 at 200 mL/min; Column Temp: 38 ; Nozzle Pressure: 100Bar; Nozzle Temp: 60 °C; Evaporator Temp: 20 °C; Trimmer Temp: 25 °C; Wavelength: 220nm) to afford the title intermediates **intermediate 99** (2.7 g) and **intermediate 100** (2.8 g).

### Preparation of intermediate 97

### (R)-N-ethyl-5-fluoro-N-isopropyl-2-((5-(2-(2-methyl-6-oxohexan-3-yl)-2,6-diazaspiro[3.4]octan-6-yl)-1,2,4-triazin-6-yl)oxy)benzamide

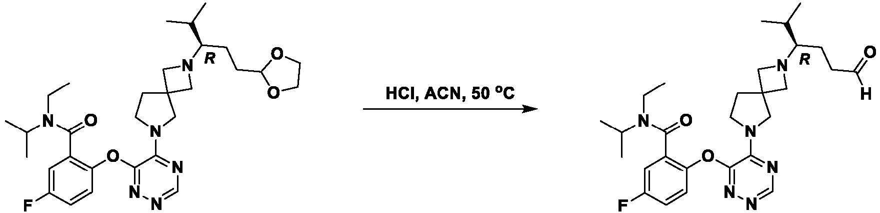

To a solution of (*R*)-2-((5-(2-(1-(1,3-dioxolan-2-yl)-4-methylpentan-3-yl)-2,6-diazaspiro[3.4]octan-6-yl)-1,2,4-triazin-6-yl)oxy)-*N*-ethyl-5-fluoro-*N*-isopropylbenzamide **(intermediate 95)** (1.00 g, 1.75 mmol) in ACN (10 mL) was added 1M HCl (10.0 mL, 10.0 mmol) and the resulting mixture was stirred at 50 °C for 1 h. After cooling to RT, the reaction mixture was concentrated under reduced pressure. The resulting residue was diluted with DCM (50 mL) and basified to pH = 14 by 10% aq. NaOH. The mixture was further extracted by DCM (30 mL x 3) and the combined organic layers were dried over anhydrous Na₂SO₄, filtered and concentrated *in vacuo* to afford the title intermediate (900 mg, 87% purity, 85% yield) as a white solid, which was used directly in next step without further purification.

### Preparation of intermediates 101, 102, 103

### (*R)-5-fluoro-N,N-diisopropyl-2-((5-(2-(2-methyl-6-oxohexan-3-yl)-2,6-diazaspiro[3.4]octan-6-yl)-1,2,4-triazin-6-yl)oxy)benzamide

### (*S)-5-fluoro-N,N-diisopropyl-2-((5-(2-(2-methyl-6-oxohexan-3-yl)-2,6-diazaspiro[3.4]octan-6-yl)-1,2,4-triazin-6-yl)oxy)benzamide

### N-ethyl-5-fluoro-N-isopropyl-2-((5-(2-(2-methyl-6-oxohexan-3-yl)-2,6-diazaspiro[3.4]octan-6-yl)-1,2,4-triazin-6-yl)oxy)benzamide

**The following intermediates were synthesized by an analogous method as described** for **intermediate 97**

| **Int. No.** | **Structure** | **Starting Material** |
|---|---|---|
| 101 | 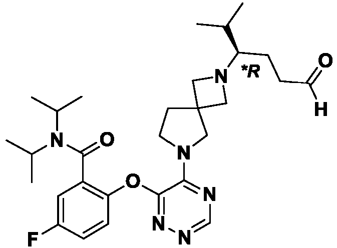 | intermediate 99 |
| 102 | 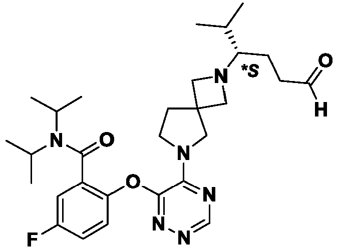 | intermediate 100 |
| 103 | 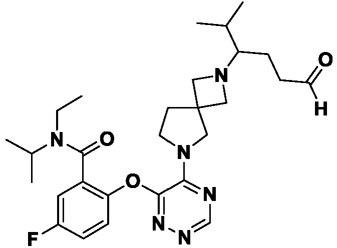 | intermediate 94 |

### Preparation of intermediate 114

### methyl 2-(2-isopropyl-1,3-dioxolan-2-yl)acetate

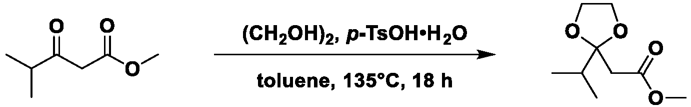

In a 1000 mL flask equipped with a Dean-Stark apparatus, methyl 4-methyl-3-oxopentanoate (50 g, 347 mmol) was added to a solution consisting of ethane-1,2-diol (43 g, 693 mmol), p-toluenesulfonic acid monohydrate (597 mg, 3.47 mmol) and toluene (500 mL). The mixture was stirred at 135°C for 18 h. After cooling to RT, 1M Na₂CO₃ (300 mL) aqueous solution was added to the reaction mixture. The organic layer was separated and washed with H₂O (100 mL), dried over anhydrous Na₂SO₄, filtered, and concentrated *in vacuo* to afford the title intermediate (41 g, crude) as a yellow oil which was used directly in next step without further purification.

### Preparation of intermediate 115

### 2-(2-isopropyl-1,3-dioxolan-2-yl)ethan-1-ol

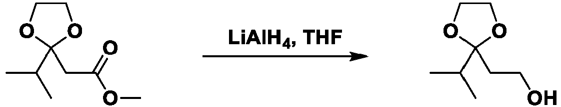

LiAlH₄ (2.5 g, 66 mmol) was added in portions to THF (250 mL) cooled at 0°C under N₂ atmosphere. A solution of methyl 2-(2-isopropyl-1,3-dioxolan-2-yl)acetate **(intermediate 114)** (10 g, crude) in THF (20 mL) was added drop-wise to above mixture at 0°C under N₂ atmosphere. The resulting mixture was slowly warmed to RT and stirred at this temperature for 18 h under N₂ atmosphere. Then 2.5 mL H₂O was slowly added to above mixture, followed with addition of aq. NaOH solution (15%, 7.5 mL). The resulting mixture was stirred at RT for 0.5 h. Then anhydrous MgSO₄ was added to above mixture. The suspension was filtered through a celite pad and washed with THF (200 mL). The filtrate was concentrated *in vacuo* to afford the title intermediate (6.8 g, crude) as a yellow oil which was used directly in next step without further purification.

### Preparation of intermediate 116

### 1-hydroxy-4-methylpentan-3-one

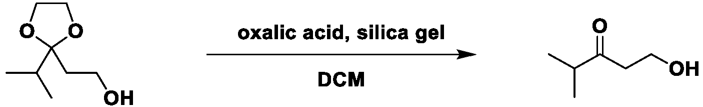

Oxalic acid (4.2 mL, 10% in water, 4.7 mmol) was added to a mixture of silica gel (27 g, 449 mmol) in DCM (230 mL). Once the aqueous layer vanished, a solution of 2-(2-isopropyl-1,3-dioxolan-2-yl)ethan-1-ol **(intermediate 115)** (3.7 g, crude) in DCM (7 mL) was added and the reaction mixture was stirred at RT for 5 h. Then NaHCO₃ (800 mg) was added. The resulting mixture was filtered and washed with DCM (50 mL x 3). The filtrate was concentrated *in vacuo* to afford the title intermediate (2.4 g, crude) as a colorless oil which was used directly in next step without further purification.

### Preparation of intermediate 124

### (*R)-3-(6-(6-(2-(ethyl(isopropyl)carbamoyl)-4-fluorophenoxy)-1,2,4-triazin-5-yl)-2,6-diazaspiro[3.4]octan-2-yl)-4-methylpentyl methanesulfonate

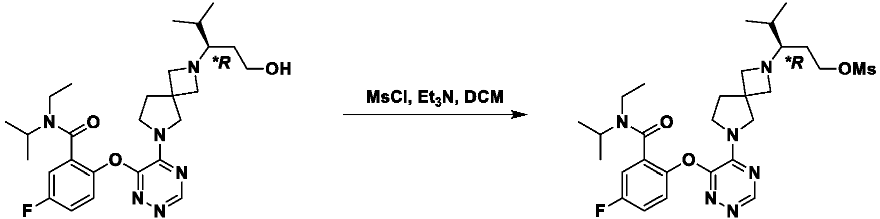

MsCl (250 mg, 2.18 mmol) was added dropwise to a solution of *N*-ethyl-5-fluoro-2-((5-(2-(1-hydroxy-4-methylpentan-3-yl)-2,6-diazaspiro[3.4]octan-6-yl)-1,2,4-triazin-6-yl)oxy)-*N-*isopropylbenzamide **(Compound 213)** (500 mg, 0.972 mmol) and TEA (0.27 mL, 1.9 mmol) in DCM (10 mL) cooled at 0 °C under N₂ atmosphere. The resulting mixture was stirred at 0 °C under N₂ for 45 min. Then the reaction mixture was quenched with H₂O (5 mL) and extracted with DCM (10 mL x 3). The combined organic layers were washed with brine (5 mL), dried over anhydrous Na₂SO₄ and filtered. The filtrate was concentrated *in vacuo* to afford the title intermediate (400 mg, crude) as a yellow oil which was used directly in next step without further purification.

### Preparation of intermediate 130, 139

### methyl 3-methyl-4-(tosyloxy)butanoate

### 2-methoxypropyl 4-methylbenzenesulfonate

**The following intermediates were synthesized by an analogous method as described above for intermediate 124**

| **Int. No.** | **Structure** | **Starting Materials** | **Conditions** |
|---|---|---|---|
| 130 | 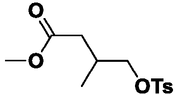 | intermediate 129, TsCl | DMAP, TEA, DCM, RT |
| 139 | 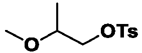 | 2-methoxypropan-1-ol, TsCl | DMAP, TEA, DCM, RT |

### Preparation of intermediate 125

### N-benzyl-2-methoxy-N-methylacetamide

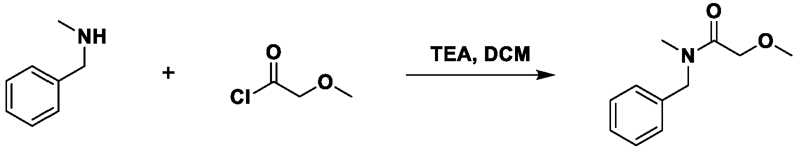

To a solution of *N*-methyl-1-phenylmethanamine (5.5 g, 45.4 mmol) and TEA (14g, 138.4 mmol) in DCM (60 mL) cooled at 0 °C was dropwise added 2-methoxyacetyl chloride (5 g, 46.073 mmol). The resulting mixture was slowly warmed to 25 °C and stirred at this temperature for 1 h. Then, aq. sat. NaHCO₃ solution (50 mL) was added to above mixture and extracted with DCM (50 mL x 3). The combined organic layers were washed with brine (100 mL), dried over Na₂SO₄, filtered and concentrated *in vacuo* give a crude residue which was purified by FCC (EA:PE = from 0 to 80%) to afford the title intermediate (3.4 g, 34% yield) as a colorless oil.

### Preparation of intermediate 126

### N-benzyl-2-methoxy-N-methylethan-1-amine-1,1-d₂

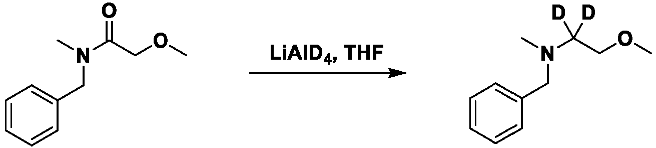

To the mixture of LiAlD₄ (1.5 g, 35.732 mmol) in THF (25 mL) cooled at 0 °C under N₂ atmosphere was added dropwise a solution of N-benzyl-2-methoxy-*N*-methylacetamide **(intermediate 125)** (3.4 g, 17.6 mmol) in THF (25 mL). The reaction mixture was first stirred at 25 °C for 1 h and at 50 °C for additional 2 h. Then the reaction mixture was cooled to 0 °C and quenched with aq. NaOH (1 M, 10 mL) dropwise. The resulting mixture was filtered and the filter cake was washed with EtOAc (100 mL). The filtrate was washed with H₂O (50 mL) and brine (50 mL), dried over Na₂SO₄, and filtered. The solvent was concentrated under reduced pressure to afford a residue which was purified by FCC (EtOAc:PE = from 0 to 100%) to afford the title intermediate (2.0 g, 60% yield) as a colorless oil.

### Preparation of intermediate 127

### 2-methoxy-N-methylethan-1,1-d₂-1-amine, hydrochloride

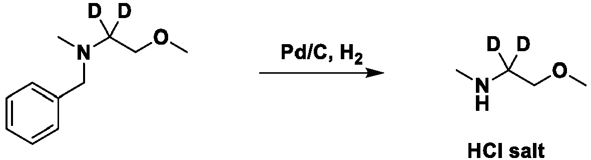

To the solution of *N*-benzyl-2-methoxy-*N*-methylethan-1-amine-1,1-*d*₂ (800 mg, 4.413 mmol) in MeOH (20 mL) and THF (60 mL) was added 1, 1,2-trichloroethane (1.2 g, 9.0 mmol) and Pd/C (wet, 10%, 0.5 g). The resulting mixture was stirred under H₂ atmosphere (50 psi) at 50 °C for 18 h. After cooling to RT, the reaction mixture was filtered by celite and the filtrate was concentrated *in vacuo* to afford the title intermediate (600 mg, crude) as yellow oil which was used directly in next step without further purification.

### Preparation of intermediate 128

### methyl 4-hydroxy-3-methylbut-2-enoate

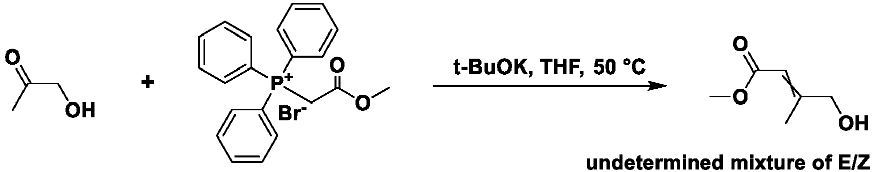

t-BuOK (16.0 g, 143 mmol) was added to a solution of (2-methoxy-2-oxoethyl)triphenylphosphonium bromide (59.0 g, 142 mmol) in THF (220 mL). The resulting mixture was stirred at 50 °C for 1 h. Then 1-hydroxypropan-2-one (7.2 g, 97 mmol) in THF (30 mL) was added to above mixture and the reaction mixture was stirred at 50 °C for another 16 h. After cooling to RT, H₂O (200 mL) was added and the mixture was extracted with EtOAc (200 mL x 3). The combined organic layers were washed with H₂O (300 mL), dried over anhydrous Na₂SO₄ and filtered. The filtrate was concentrated to *in vacuo* to afford a crude compound which was purified by FCC (PE: EtOAc = 1:0 to 1:1) to afford the title intermediate (3.4 g, 27% yield ) as a light yellow oil.

### Preparation of intermediate 129

### methyl 4-hydroxy-3-methylbutanoate

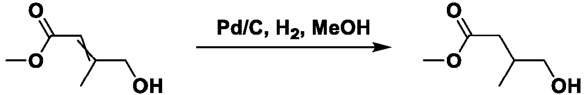

To the solution of methyl 4-hydroxy-3-methylbut-2-enoate **(intermediate 128)** (3.4 g, 26 mmol) in MeOH (100 mL) was added dry Pd/C (500 mg, 10%) and the suspension was stirred at RT under H₂ (15 psi) atmosphere for 4 h. Then the reaction mixture was filtered through a celite pad and washed with MeOH (200 mL). The filtrate was concentrated *in vacuo* afford the title intermediate (2.3 g, 67% yield) as a yellow oil which was used directly in the next step without further purification.

### Preparation of intermediates 193, 194, 207, 208

### (S)-5-((tert-butyldiphenylsilyl)oxy)-6-(ethyl(methyl)amino)-2-methylhexan-3-one

### (R)-5-((tert-butyldiphenylsilyl)oxy)-6-(ethyl(methyl)amino)-2-methylhexan-3-one

### (S)-5-((tert-butyldiphenylsilyl)oxy)-6-((2-methoxyethyl)(methyl)amino)-2-methylhexan-3-one

### (R)-5-((tert-butyldiphenylsilyl)oxy)-6-((2-methoxyethyl)(methyl)amino)-2-methylhexan-3-one

**The following intermediates were synthesized by an analogous method as described for intermediate 129**

| **Int. No.** | **Structure** | **Starting Material** |
|---|---|---|
| 193 | 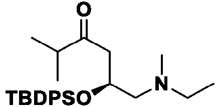 | intermediate 191 |
| 194 | 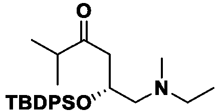 | intermediate 192 |
| 207 | 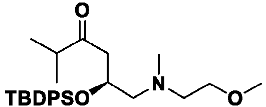 | intermediate 205 |
| 208 | 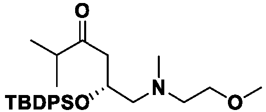 | intermediate 206 |

### Preparation of intermediate 131 and 132

### methyl (*R)-3-methyl-4-(tosyloxy)butanoate

### methyl (*S)-3-methyl-4-(tosyloxy)butanoate

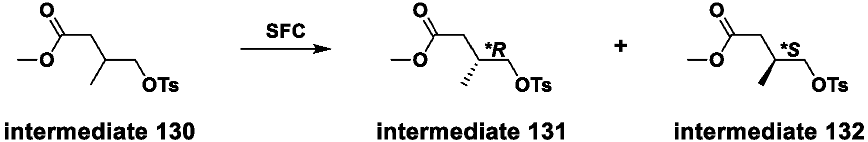

Methyl 3-methyl-4-(tosyloxy)butanoate **(intermediate 130)** (3.3 g) was purified by SFC over DAICEL CHIRALPAK AY-H (column: 250x30mm 5um; Mobile phase: A: Supercritical CO₂, B: EtOH (0.1% ammonia), A:B = 90:10 at 60 mL/min) to afford the title intermediates **(intermediate 131)** (1.28 g, 97% purity, 36 % yield) and **(intermediate 132)** (1.27 g, 85% purity, 33 % yield) both as white solid.

### Preparation of intermediate 134

### methyl (*S)-4-((2-methoxyethyl)(methyl)amino)-3-methylbutanoate

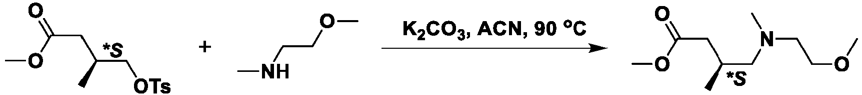

A mixture of methyl (**S*)-3-methyl-4-(tosyloxy)butanoate **(intermediate 132)** (1.27 g, 4.44 mmol), 2-methoxy-*N*-methylethan-1-amine (593 mg, 6.65 mmol), and K₂CO₃ (1.23 mg, 8.87 mmol) in ACN (5 mL) was stirred at 90 °C overnight. After cooling to RT, the reaction mixture was filtered and the filtrate was concentrated *in vacuo* to afford the title intermediate (670 mg, crude) as a brown oil which was used directly in next step without further purification.

### Preparation of intermediates 133, 185, 186, 199, 200, 219

### methyl (*R)-4-((2-methoxyethyl)(methyl)amino)-3-methylbutanoate

### ethyl (S)-3-((tert-butyldiphenylsilyl)oxy)-4-(ethyl(methyl)amino)butanoate

### ethyl (R)-3-((tert-butyldiphenylsilyl)oxy)-4-(ethyl(methyl)amino)butanoate

### ethyl (S)-3-((tert-butyldiphenylsilyl)oxy)-4-((2-methoxyethyl)(methyl)amino)butanoate

### ethyl (R)-3-((tert-butyldiphenylsilyl)oxy)-4-((2-methoxyethyl)(methyl)amino)butanoate N-(2-methoxyethyl)-N,2-dimethylprop-2-en-1-amine

**The following intermediates were synthesized by an analogous method as described for intermediate 134**

| **Int. No.** | **Structure** | **Starting Materials** | **Conditions** |
|---|---|---|---|
| 133 | 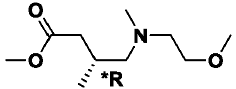 | intermediate 131, 2-methoxy-*N*-methylethan-1-amine | K₂CO₃, ACN, 90 °C |
| 185 | 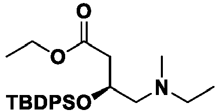 | intermediate 183, *N*-methylethanamine | K₂CO₃, ACN, 80 °C |
| 186 | 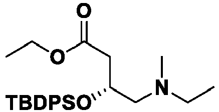 | intermediate 184, *N*-methylethanamine | K₂CO₃, ACN, 80 °C |
| 199 | 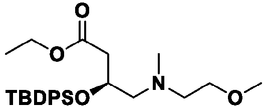 | intermediate 183, 2-methoxy-*N*-methylethan-1-amine | K₂CO₃, ACN, 85 °C |
| 200 | 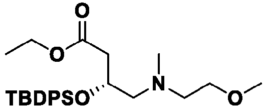 | intermediate 184, 2-methoxy-*N*-methylethan-1-amine | K₂CO₃, ACN, 85 °C |
| 219 | 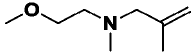 | 3-bromo-2-methylprop-1-ene, 2-methoxy-*N*-methylethan-1-amine | K₂CO₃, H₂O, RT |

### Preparation of intermediate 136

### (*S)-6-((2-methoxyethyl)(methyl)amino)-2,5-dimethylhexan-3-one

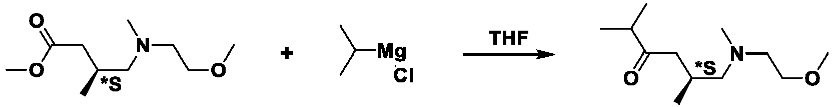

To the solution of methyl (*S)-4-((2-methoxyethyl)(methyl)amino)-3-methylbutanoate **(intermediate 134)** (670 mg, crude) in THF (5 mL) cooled at 0 °C under N₂ was added dropwise isopropylmagnesium chloride (4.94 mL, 9.88 mmol, 2 M, in THF). The resulting mixture was stirred at 50 °C for 5 h under N₂. After cooling to RT, the reaction mixture was quenched with sat. aq. NH₄Cl solution (1.5 mL) and filtered. The filtrate was concentrated *in vacuo* to afford the title intermediate (507.1 mg, crude) as a yellow oil which was used directly in next step without further purification.

### Preparation of intermediate 135

### (*R)-6-((2-methoxyethyl)(methyl)amino)-2,5-dimethylhexan-3-one

**The following intermediate was synthesized by an analogous method as described** for **intermediate 136**

| **Int. No.** | **Structure** | **Starting Materials** | **Conditions** |
|---|---|---|---|
| 135 | 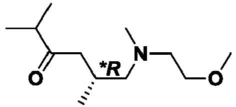 | intermediate 133, isopropylmagnesium chloride | THF, 50 °C |

### Preparation of intermediate 165

### tert-butyl (2-hydroxy-5-methyl-4-oxohexyl)(methyl)carbamate

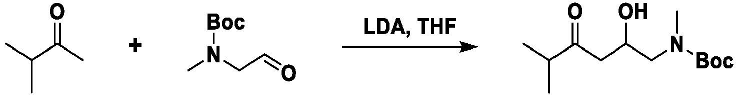

To the solution of 3-methylbutan-2-one (6.0 g, 70.0 mmol) in THF (150 mL) cooled at -40 °C under N₂ atmosphere was added dropwise LDA (40 mL, 2 M in THF, 80.0 mmol). The resulting mixture was stirred at -40 °C for 1 h. Then a solution of *tert*-butyl methyl(2-oxoethyl)carbamate (8.0 g, 46.2 mmol) in THF (50 mL) was added dropwise to above mixture and the reaction was further stirred at -40 °C for 2 h. The reaction was quenched by the dropwise addition of H₂O (20 mL) at -40 °C. Then the mixture was warmed to RT and concentrated under reduced pressure. The crude residue was diluted with H₂O (200 mL) and extracted with EtOAc (200 mL × 2). The combined organic layers were washed with brine (200 mL), dried over anhydrous Na₂SO₄, filtered and concentrated. The crude product was purified by FCC (PE/EtOAc = 20/1 to 3/1) to afford the title intermediate (8.8 g, 85% purity, 62% yield) as colorless oil.

**The following intermediate was synthesized by an analogous method as described for intermediate 165**

| **Int. No.** | **Structure** | **Starting Materials** |
|---|---|---|
| 174 | 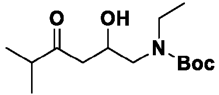 | 3-methylbutan-2-one, *tert*-butyl ethyl(2-oxoethyl)carbamate |

### Preparation of intermediate 166

### tert-butyl (2-methoxy-5-methyl-4-oxohexyl)(methyl)carbamate

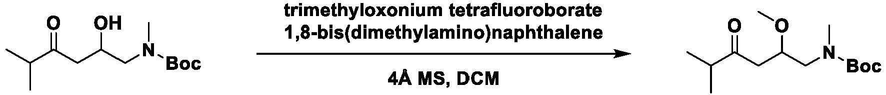

To a solution of *tert*-butyl (2-hydroxy-5-methyl-4-oxohexyl)(methyl)carbamate **(intermediate 165)** (4.00 g, 15.4 mmol) in DCM (200 mL) was added 4 Å molecular sieve (4 g) under N₂ atmosphere and the mixture was stirred at 25 °C for 10 min. Then 1,8-bis(dimethylamino)naphthalene (8.26 g, 38.6 mmol) was added and the mixture was cooled to 0 °C, followed with addition of trimethyloxonium tetrafluoroborate (5.93 g, 40.1 mmol). The reaction mixture was first stirred at 0 °C for 2 h, then warmed up to 25 °C and stirred at this temperature for additional 16 h. The suspension was filtered and washed with DCM (40 mL × 2). The filtrate was concentrated *in vacuo* and the residue was purified by FCC (PE/EtOAc = 5/1 to 4/1) to afford the title intermediate (2.00 g, 44% yield) as colorless oil.

### Preparation of intermediate 181

### ethyl (S)-3-hydroxy-4-iodobutanoate

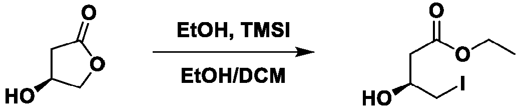

To a solution of (S)-4-hydroxydihydrofuran-2(3H)-one (5 g, 50.0 mmol) in EtOH (8.6 mL) in DCM (20 mL) under N₂ atmosphere was slowly added TMSI (14.8 g, 74.0 mmol). The resulting mixture was stirred at RT for 16 h. A solution of sat. Na₂SO₃ (40 mL) was added. The organic layer was separated and concentrated *in vacuo* to afford the title intermediate (8.8 g, crude) as yellow oil which was used directly in next step without further purification.

### Preparation of intermediate 182

### ethyl (R)-3-hydroxy-4-iodobutanoate

**The following intermediate was synthesized by an analogous method as described above for intermediate 181**

| **Int. No.** | **Structure** | **Starting Material** |
|---|---|---|
| 182 | 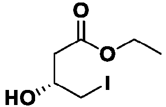 | (R)-4-hydroxydihydrofuran-2(3H)-one |

### Preparation of intermediate 195

### (S)-6-(ethyl(methyl)amino)-5-hydroxy-2-methylhexan-3-one

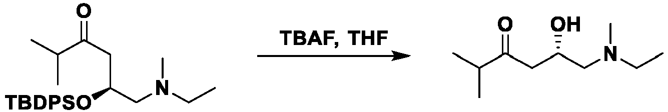

To a solution of (*S*)-5-((*tert*-butyldiphenylsilyl)oxy)-6-(ethyl(methyl)amino)-2-methylhexan-3-one **(intermediate 193)** (2.33 g, 5.04 mmol) in THF (3 mL) was added TBAF (0.65 mL, 1.0 M in THF, 0.65 mmol) under N₂ atmosphere. The resulting mixture was stirred at RT for 16 h. The reaction mixture was concentrated under reduced pressure and the crude residue was diluted with H₂O (25 mL) and extracted with DCM (60 mL × 3). The combined organic layers were washed with brine (40 mL × 2), dried over Na₂SO₄ and filtered. The filtrate was concentrated *in vacuo* to afford the title intermediate (2.2 g, crude) as yellow oil which was used directly in next step without further purification.

### Preparation of intermediate 196, 209, 210

### (R)-6-(ethyl(methyl)amino)-5-hydroxy-2-methylhexan-3-one

### (S)-5-hydroxy-6-((2-methoxyethyl)(methyl)amino)-2-methylhexan-3-one

### (R)-5-hydroxy-6-((2-methoxyethyl)(methyl)amino)-2-methylhexan-3-one

**The following intermediates were synthesized by an analogous method as described above for intermediate 195**

| **Int. No.** | **Structure** | **Starting Material** |
|---|---|---|
| 196 | 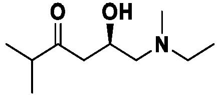 | intermediate 194 |
| 209 | 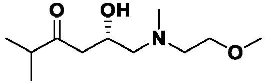 | intermediate 207 |
| 210 | 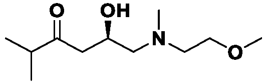 | intermediate 208 |

### Preparation of intermediate 220

### N-((3-isopropyl-5-methyl-4,5-dihydroisoxazol-5-yl)methyl)-2-methoxy-N-methylethan-1-amine

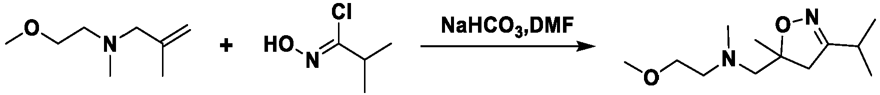

To a solution of *N*-(2-methoxyethyl)-*N*,2-dimethylprop-2-en-1-amine **(intermediate 219)** (2.90 g, 20.2 mmol) in DMF (50 mL) cooled at 0 °C were added NaHCO₃ (6.82 g, 81.2 mmol) and (*Z*)-*N*-hydroxyisobutyrimidoyl chloride (2.47 g, 20.3 mmol). The reaction mixture was stirred at 0 °C for 30 min and then at RT for 16 h. The reaction mixture was quenched by H₂O (50 mL) and extracted with EtOAc (30 mL × 2). The combined organic layers were washed with sat. aq. LiCl solution (50 mL), dried over anhydrous Na₂SO₄ and filtered. The filtrate was concentrated *in vacuo* to give the crude product, which was purified by FCC (MeOH: DCM = 1:10) to afford the title intermediate (1.20 g, 89.9% purity, 25.9% yield) as brown oil.

### Preparation of intermediate 221

### 5-hydroxy-6-((2-methoxyethyl)(methyl)amino)-2,5-dimethylhexan-3-one

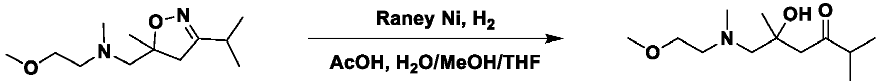

To a solution of *N*-((3-isopropyl-5-methyl-4,5-dihydroisoxazol-5-yl)methyl)-2-methoxy-N-methylethan-1-amine **(intermediate 220)** (1.20 g, 5.26 mmol) in MeOH and THF (40 mL, MeOH/THF = 1/2) were added AcOH (3.15 g, 52.5 mmol) and H₂O (9.50 mL, 572.3 mmol). Raney-Ni (750 mg) was added to the solution under N₂ atmosphere at 0 °C. The suspension was degassed and purged with H₂ for 3 times and the mixture was stirred under H₂ atmosphere (30 Psi) at 25 °C overnight.

The reaction mixture was filtered through a celite pad and the filtrate was extracted with DCM. The combined organic layers were washed with NaHCO₃ (20 mL × 2) and brine (20 mL × 2), dried over Na₂SO₄ and filtered. The filtrate was concentrated in vacuo to afford the title intermediate (1.10 g, crude) as brown oil, which was used directly in next step without further purification.

### Preparation of intermediate 227

### tert-butyl (R)-(1-(2,2-dimethyl-4,6-dioxo-1,3-dioxan-5-yl)-3-methylbutan-2-yl)carbamate

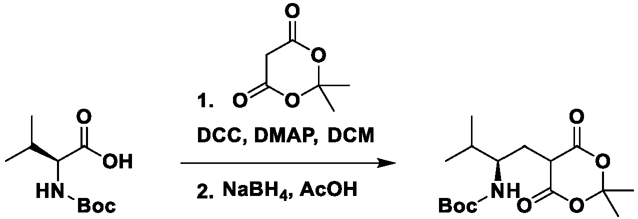

Boc-L-valine (44.9 kg), 2,2-dimethyl-1,3-dioxane-4,6-dione (32.9 kg) and DMAP (35.5 kg) in DCM (607 kg) pre-cooled at -10 to 0°C were added to a solution of DCC (55.5 kg) in DCM (613 kg) over 3 h and aged for 16 h at -10 to 0°C. 10% citric acid aqueous solution (449 kg) was added whilst maintaining a temperature below 10°C. The resulting slurry was aged for 2 h at 0 to 10°C. then filtered. The filter cake was washed with DCM (91 kg). The filtrate was separated and the organic layer was washed with 10% citric acid aqueous solution (two times 450 kg) and 10% NaCl aqueous solution (449 kg). To organic phase (1200 kg), was added acetic acid (75.0 kg) whilst maintaining a temperature between -10 to 0°C. Sodium Borohydride (18.0 kg) was added in portions over 5 h whilst maintaining a temperature in the range -10 to 0°C and then resulting mixture was aged at -10 to 0°C for an additional 16 h. The mixture was warmed to 15 to 25°C, and aged for 2 h. The mixture was then washed with 14% NaCl aqueous solution (450 kg) followed by a second wash with 14% NaCl aqueous solution (432 kg) and a final water wash (444 kg). The organic phase was concentrated under reduced pressure to 2-4 vol. Iso-propanol (143 kg) was added to the residue and concentrated to 4-5 vol. under reduced pressure. After cooling to -10 to 0°C and aging for 8 h, the resulting slurry was filtered, washed with IPA (38 kg) and dried to afford the title intermediate (46.7 kg, 69% yield) as a white solid.

### Preparation of intermediate 228

### tert-butyl (R)-2-isopropyl-5-oxopyrrolidine-1-carboxylate

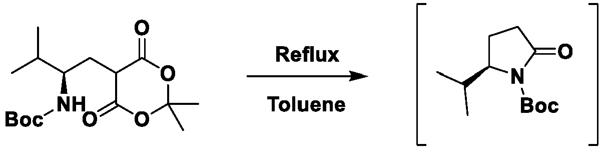

*tert-*butyl (*R*)-(1-(2,2-dimethyl-4,6-dioxo-1,3-dioxan-5-yl)-3-methylbutan-2-yl)carbamate **(intermediate 227)** (46.7 kg) in toluene (333 kg) was heated to reflux and aged for 4 h. The mixture was cooled to ambient temperature, filtered and washed with toluene (20 kg). The combined filtrates were concentrated to dryness at reduced pressure to afford the desired compound (31.05 kg, 96% yield) as an oil which was used directly without further purification.

### Preparation of intermediate 229

### tert-butyl (5R)-2-hydroxy-5-isopropylpyrrolidine-1-carboxylate

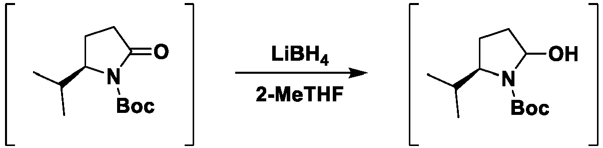

*tert-*butyl (*R*)-2-isopropyl-5-oxopyrrolidine-1-carboxylate **(intermediate 228)** (30.9 kg) in 2-MeTHF (26.7 kg) was cooled to -5 to 5°C. A solution of LiBH₄ in 2-MeTHF (1M, 45.2 kg, 54.4 mol) was added over 3 h and the mixture was aged for 4 h. A cold aqueous solution of 5% NaHCO₃ (163 kg) was added at -5 to 5°C over 3h and aged for an additional 2 h. The mixture was warmed to ambient temperature and aged for a further 2 h. The aqueous layer was separated and the organic layer was washed with 10% NaCl aqueous solution (170 kg) and water (155 kg). During the water wash, an emulsion formed and solid NaCl (3.1 kg) was added to affect the separation. After removal of the aqueous layer, the organic layer was concentrated under reduced pressure to dryness to afford the desired compound (28.5 kg, 91% yield) as an oil, which was used directly without further purification.

### Preparation of intermediate 230

### tert-butyl (R)-(6-((2-methoxyethyl)(methyl)amino)-2-methylhexan-3-yl)carbamate

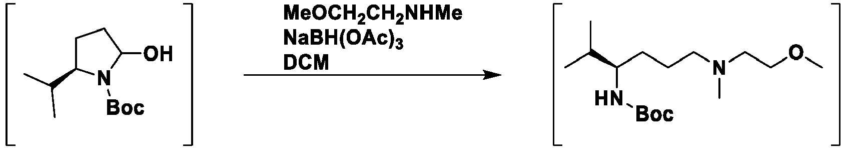

*tert*-butyl (5*R*)-2-hydroxy-5-isopropylpyrrolidine-1-carboxylate **(intermediate 229)** (28.55 kg) in DCM (344 kg), at 15 to 25°C was treated with 2-methoxy-*N*-methylethan-1-amine (12.3 kg, 138.0 mol) and the resulting mixture was aged for 1 h. Sodium triacetoxyborohydride (40.12 kg) was added in portions over 5h whilst maintaining a temperature between 15 to 25°C and the resulting mixture was aged for 48 h. The reaction mixture was quenched by the addition of 8% NaOH aqueous solution (184 kg) over 2 h whilst maintaining a temperature between 15 to 25°C and the mixture was aged for a further 2 h. The water layer was separated, and the organic layer was washed with water (169 kg). The organic layer was then concentrated under reduced pressure to dryness to afford the title intermediate (33.26 kg, 88% yield) as an oil which was used directly without further purification.

### Preparation of intermediate 231

### (R)-N¹-(2-methoxyethyl)-N¹,5-dimethylhexane-1,4-diamine, dihydrochloride

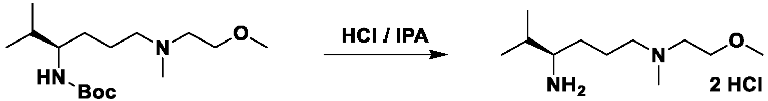

To 4 molar solution of HCl in iso-propanol (84.80 kg) at ambient temperature was added a solution of *tert*-butyl (*R*)-(6-((2-methoxyethyl)(methyl)amino)-2-methylhexan-3-yl)carbamate **(intermediate 230)** (32.38 kg) in iso-propanol (25.6 kg) over 3 h and the mixture was aged at ambient temperature for an additional 19 h. Methyl *tert*-butyl ether (95.25 kg) was then added over 1 h and the mixture was aged for 2.5 h. The resulting slurry was filtered and washed with MTBE (53 kg). The filter cake was dried to afford the title compound (23.92 kg, 81% yield) as a white solid.

### Preparation of intermediate 232

### ethyl 1-benzyl-3-(chloromethyl)pyrrolidine-3-carboxylate

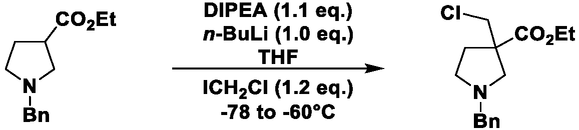

To a solution of DIPEA (952 g, 1.1 eq.) in THF (6 L) which was cooled to -35 to -25°C was added *n*-BuLi (2.33 kg, 2.5 M in hexane, 1.0 eq.) whilst maintaining a temperature below - 25°C. The resulting mixture was aged at -35 to -25°C for an additional 30 min then cooled to between -78 to -60°C. A solution of ethyl 1-benzylpyrrolidine-3-carboxylate (2 kg, 1.0 eq.) in THF (2 L) at -78 to -60°C was added and stirred for an addition 30 min. Chloroiodomethane (1.81 kg, 1.2 eq.) was then charged at -78 to -60°C. The reaction mixture was aged at -60 to - 40°C for 2 h. To the reaction mixture was added to citric acid aqueous solution (660 g in 6 L H₂O) at a temperature between 0 to 10°C and the resulting mixture was aged at 20 to 30°C for an additional 20 min. After separating the layers, the aqueous layer was extracted with EtOAc (6 L) and the combined organic layers washed with brine (6 L) then warmed to 50 to 60°C. Oxalic acid (2.22 kg) was charged at 50 to 60°C. The resulting mixture was stirred at 50 to 60°C for 3 h then cooled to 20 to 30°C and aged overnight. The resulting solid was filtered and the cake was washed with ethyl acetate (2 L). The wet cake was added to toluene (4 L), H₂O (8 L) and K₃PO₄ (1.5 eq.) and the resulting mixture was aged at 20 to 30°C for 20 min. After separating the layers, the aqueous layer was extracted with toluene (2 L). The organic layers were combined and washed twice with water (2 L ). The organic phase was concentrated under reduced pressure to afford 4.2 kg of the desired compound as a toluene solution (46 wt % by assay, giving an assay yield of 80%).

### Preparation of intermediate 233

### 1-benzyl-3-(chloromethyl)pyrrolidine-3-carbaldehyde

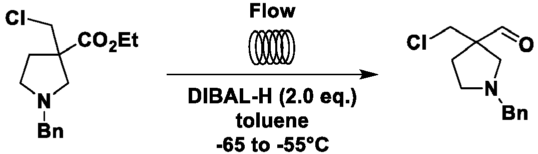

Reaction conducted in a flow chemistry system: A solution of ethyl 1-benzyl-3-(chloromethyl)pyrrolidine-3-carboxylate **(intermediate 232)** (4.4 kg) in toluene (26 L) was pumped at 26.7 mL/min and cooled to -60°C. After cooling, it was then mixed with a cooled solution of DIBAL-H (28.1 mol) in toluene at -60°C (28 L) with a pumping rate of 32.1 mL/min. The mixture was passed through a Perfluoroalkoxy (PFA) coil tube reactor at -60°C (total flow rate of 58.8 mL/min with a residence time of 5 seconds). The resulting mixture was mixed with cooled MeOH (-60°C) which was pumped at the rate of 15.2 mL/min. This mixed solution was pumped to another PFA coil tube reactor at -60°C (total flow rate of 74 mL/min with a residence time of 5 seconds). The resulting mixture was collected into a receiver which contained 20 wt % aq. solution Rochelle's salt (20 V). The layers were separated, and the organic phase was twice washed with water (2 x 44 L). The organic phase was combined with another 3.0 kg batch prepared in an analogous manner and concentrated under reduced pressure to afford 20.8 kg of a toluene solution of the desired compound (25.5 wt % assay by HPLC, giving an assay yield of 85%) which was used directly without further purification.

**¹H NMR (300 MHz, Chloroform-d):** δ 9.62 (s, 1H), 7.39 - 7.20 (m, 5H), 3.83 - 3.57 (m, 4H), 2.96 (d, J = 10.2 Hz, 1H), 2.80 - 2.55 (m, 3H), 2.17 (ddd, J = 13.9, 7.9, 6.1 Hz, 1H), 1.83 (ddd, J = 13.4, 7.8, 5.5 Hz, 1H).

### Preparation of intermediate 234

### (R)-4-(6-benzyl-2,6-diazaspiro[3.4]octan-2-yl)-N-(2-methoxyethyl)-N,5-dimethylhexan-1-amine

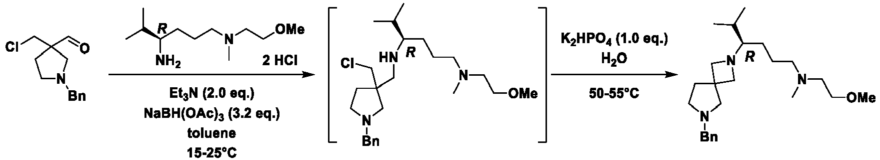

To a solution of 1-benzyl-3-(chloromethyl)pyrrolidine-3-carbaldehyde **(intermediate 233)** in toluene (3.0 kg, 10 wt %) diluted with toluene (30 L) and (*R*)-*N*¹-(2-methoxyethyl)-*N*¹,5-dimethylhexane-1,4-diamine, dihydrochloride **(intermediate 231)** (3.47 kg) was added triethylamine (2.55 kg, 25.2 mol) at 20 to 30°C. The resulting mixture was aged for 2 h at 20 to 30°C. Then sodium triacetoxyborohydride (9.0 kg) was charged at 20 to 30°C and the mixture was aged for 12 h. The reaction mixture was cooled to 5 to 15°C and 25 wt % NaOH aqueous solution (25 L, ~16.75 eq.) was added maintaining a temperature below 35°C. The resulting mixture was aged at 20 to 30°C for 25 mins and the layers were separated. The organic layer was washed with 15 wt % aq. NaCl (10 L) and the layers were again separated and water (18 L) was charged to the organic phase. The pH of the aqueous phase was adjusted to 6~7 with 4M aq. HCl whilst maintaining an internal temperature below 35°C. The organic phase was then discarded and the aqueous phase was separated and basified to pH 8~9 with K₂HPO₄.

The resulting mixture was warmed to 50 to 55°C and aged for 3 h. The reaction mixture was then cooled to ambient temperature and combined with other two batches (2.4 kg + 3.0 kg). The combined streams were washed with methyl *tert*-butyl ether three times (3 x 40 L). To the resulting aqueous layer was added additional methyl *tert*-butyl ether (83 L) and the aqueous phase was basified to pH 9~10 using 8 wt % aq. NaOH whilst maintaining a temperature between 15 to 35°C. The aqueous layer was separated, and the organic layer was washed with three times water (3 x 30 L). The organic layer was then concentrated under reduced pressure to approximately 3 volumes and then flushed with methanol three times (3 x 30 L) and concentrated to dryness to afford the desired compound (12.4 kg, 90% isolated yield) as light-yellow oil, which was used directly without further purification.

### Preparation of intermediate 224

### (R)-N-(2-methoxyethyl)-N,5-dimethyl-4-(2,6-diazaspiro[3.4]octan-2-yl)hexan-1-amine

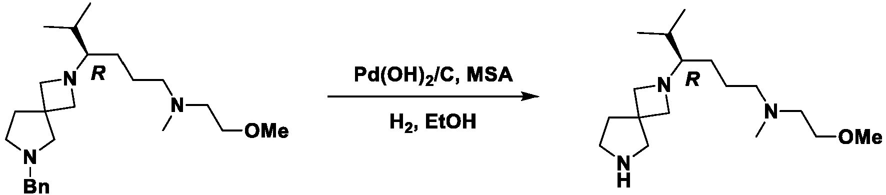

To palladium hydroxide on carbon (1.2kg) in EtOH (1.47 kg) cooled to -5 to 5°C were added methanesulfonic acid (MSA) (11kg), (*R*)-4-(6-benzyl-2,6-diazaspiro[3.4]octan-2-yl- *N-(2-*methoxyethyl)-*N*,5-dimethylhexan-1-amine **(intermediate 234)** (10kg) and EtOH (250L). The mixture was warmed to 35-45°C and stirred under a hydrogen atmosphere (0.27 to 0.40 MPa) for 16-20h. The mixture was filtered over diatomite (20kg) and the pad was washed with EtOH (24L). The filtrate was concentrated under reduced pressure (<40°C) to 2~3 vol. and then flushed twice with 2-MeTHF (73kg and 47kg) to give a 2~3 vol. solution. After dilution with 2-MeTHF (65kg), 10% aq. sodium sulfate (30kg) was added and the mixture was cooled to 0 to 10°C, followed by the addition of 16% aq. NaOH (50kg) to adjust the pH to 13~14. The temperature was adjusted to 15 to 25°C and stirred for 30 to 60 min. The aqueous layer was separated and extracted twice with 2-MeTHF (47kg x 2). The combined organic layers were concentrated under reduced pressure (<40°C) to 3~4 vol. and 2-MeTHF (950g) was added. After concentration under reduced pressure (<40°C) to 3~4 vol., the resulting solution was diluted with 2-MeTHF (30kg), dried by passing through 4A molecular sieves (25kg) and washed with 2-MeTHF (30kg). The final solution was concentrated to afford the desired compound (6.7kg) as an oil with 90.1% assay purity in a 79% corrected yield.

### Preparation of intermediate 225

### (R)-4-(6-(3,6-dichloro-1,2,4-triazin-5-yl)-2,6-diazaspiro[3.4]octan-2-yl)-N-(2-methoxyethyl)-N,5-dimethylhexan-1-amine

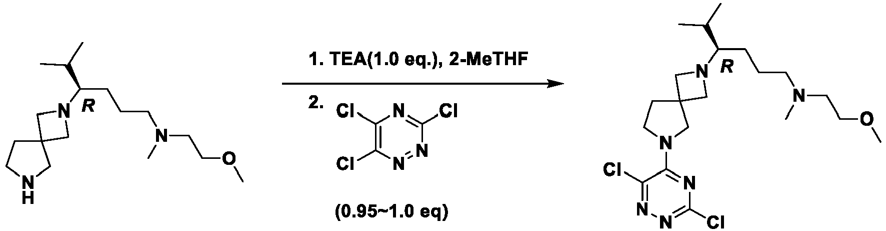

To (*R*)-*N*-(2-methoxyethyl)-*N*,5-dimethyl-4-(2,6-diazaspiro[3.4]octan-2-yl)hexan-1-amine **(intermediate 224)** (100 g) was added 2-MeTHF (430 g) and TEA (68 g) and the mixture was cooled to -50 to -40°C. 3,5,6-trichloro-1,2,4-triazine (62 g) in 2-MeTHF (172 g) was added and the mixture was stirred for 1 to 3 h. The resulting mixture was warmed to -20 to - 10°C and a 7% NaHCO₃ aqueous solution was added, the mixture was warmed to 20 to 30°C and stirred for 30 to 60 min. The aqueous layer was removed and the organic layer was washed with 10% Na₂SO₄ (500 g). The organic layer was dried by passing through 4Å molecular sieves (220 g) and washed with 2-MeTHF (180 g). The title intermediate was afforded in 90% assay yield as a solution 14.8 wt% in 2-MeTHF.

### Preparation of intermediate 245

### (R)-2-((5-(2-(6-((2-((tert-butyldimethylsilyl)oxy)ethyl)(methyl)amino)-2-methylhexan-3-yl)-2,6-diazaspiro[3.4]octan-6-yl)-1,2,4-triazin-6-yl)oxy)-N-ethyl-5-fluoro-N-isopropylbenzamide

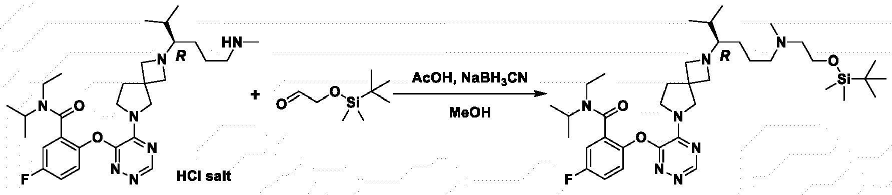

NaBH₃CN (23.2 mg, 0.37 mmol) was added to a solution of (*R*)-*N*-ethyl-5-fluoro-*N-*isopropyl-2-((5-(2-(2-methyl-6-(methylamino)hexan-3-yl)-2,6-diazaspiro[3.4]octan-6-yl)-1,2,4-triazin-6-yl)oxy)benzamide hydrochloride **(Compound 19)** (100 mg, 0.18 mmol), 2-((*tert*-butyldimethylsilyl)oxy)acetaldehyde (71 µL, 0.37 mmol) and AcOH (11 µL, 0.18 mmol) in MeOH (2 mL). Then, the reaction mixture was stirred at RT for 24 h. The reaction mixture was poured into water, basified with an aqueous solution of K₂CO₃ and DCM was added. The organic layer was separated, dried over MgSO₄, filtered and evaporated till dryness to give a crude (152 mg) which was purified by silica gel chromatography (Stationary phase: irregular bare silica 4g, Mobile phase: 0.5% NH4OH, 95% DCM, 5% MeOH). The fractions containing the product were mixed and concentrated to afford the title intermediate (46 mg, 36% yield).

### Preparation of Compounds

The below examples describe the preparation of Compounds according to the claimed invention as well as other Compounds provided for illustrative purposes.

### Preparation of Compound 61

### tert-butyl (4-(6-(6-(2-(ethyl(isopropyl)carbamoyl)-4-fluorophenoxy)-1,2,4-triazin-5-yl)-2,6-diazaspiro[3.4]octan-2-yl)-5-methylhexyl)carbamate

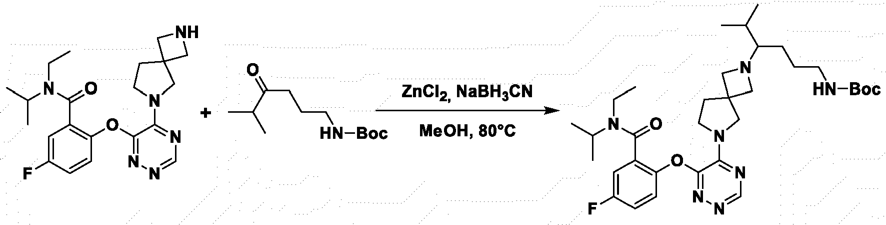

The mixture 2-((5-(2,6-diazaspiro[3.4]octan-6-yl)-1,2,4-triazin-6-yl)oxy)-*N*-ethyl-5-fluoro-*N-*isopropylbenzamide **(intermediate 3)** (1.0 g, 2.4 mmol), *tert*-butyl (5-methyl-4-oxohexyl)carbamate **(intermediate 1)** (830 mg, 3.62 mmol) and ZnCl₂ (660 mg, 4.84 mmol) in MeOH (15 mL) was stirred at 80 °C for 0.5 h. Then NaBH₃CN (310 mg, 4.93 mmol) was added and the resulting mixture was stirred at 80 °C for 6 h. After cooled to RT, the mixture was concentrated under reduced pressure to give the crude product, which was further purified by preparative HPLC using a Waters Xbridge Prep OBD (column: C18 150x40 mm 10 um; eluent: ACN/H₂O (0.05% ammonia) from 45% to 75% v/v) to afford the title compound (700 mg, 46% yield) as colorless oil.

### Preparation of Compounds 62 and 63

### tert-butyl (R)-(4-(6-(6-(2-(ethyl(isopropyl)carbamoyl)-4-fluorophenoxy)-1,2,4-triazin-5-yl)-2,6-diazaspiro[3.4]octan-2-yl)-5-methylhexyl)carbamate

### tert-butyl (S)-(4-(6-(6-(2-(ethyl(isopropyl)carbamoyl)-4-fluorophenoxy)-1,2,4-triazin-5-yl)-2,6-diazaspiro[3.4]octan-2-yl)-5-methylhexyl)carbamate

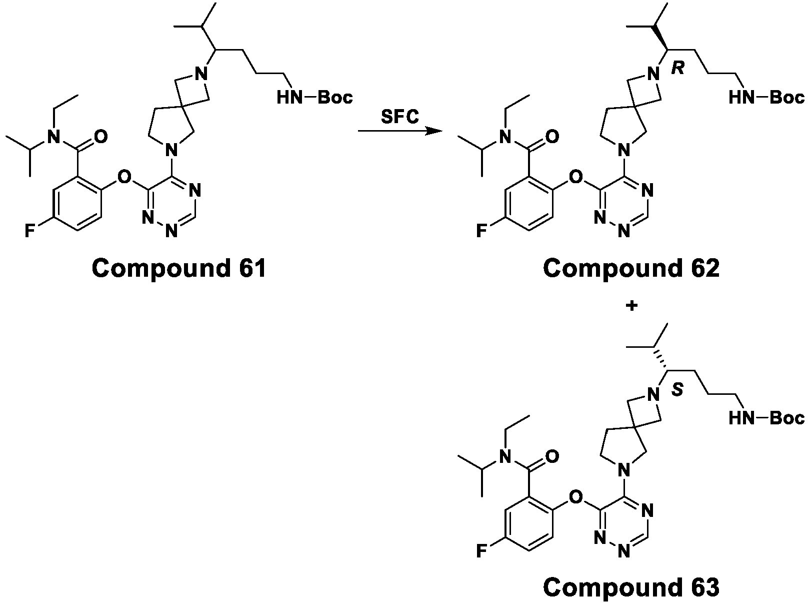

*tert*-butyl (4-(6-(6-(2-(ethyl(isopropyl)carbamoyl)-4-fluorophenoxy)-1,2,4-triazin-5-yl)-2,6-diazaspiro[3.4]octan-2-yl)-5-methylhexyl)carbamate **(Compound 61)** (200 mg, 0.319 mmol) was purified by SFC over DAICEL CHIRALPAK IG (column: 250x30 mm 10 um; isocratic elution: EtOH (containing 0.1% of 25% ammonia): supercritical CO₂, 40% : 60% (v/v)) to afford the title compounds **(Compound 62)** (85 mg, 42% yield) and (Compound **63)** (80 mg, 40% yield) both as light yellow oil.

### Compound 207 and 208

### tert-butyl (*R)-(5-(6-(6-(2-(ethyl(isopropyl)carbamoyl)-4-fluorophenoxy)-1,2,4-triazin-5-yl)-2,6-diazaspiro[3.4]octan-2-yl)-6-methylheptyl)carbamate

### tert-butyl (*S)-(5-(6-(6-(2-(ethyl(isopropyl)carbamoyl)-4-fluorophenoxy)-1,2,4-triazin-5-yl)-2,6-diazaspiro[3.4]octan-2-yl)-6-methylheptyl)carbamate

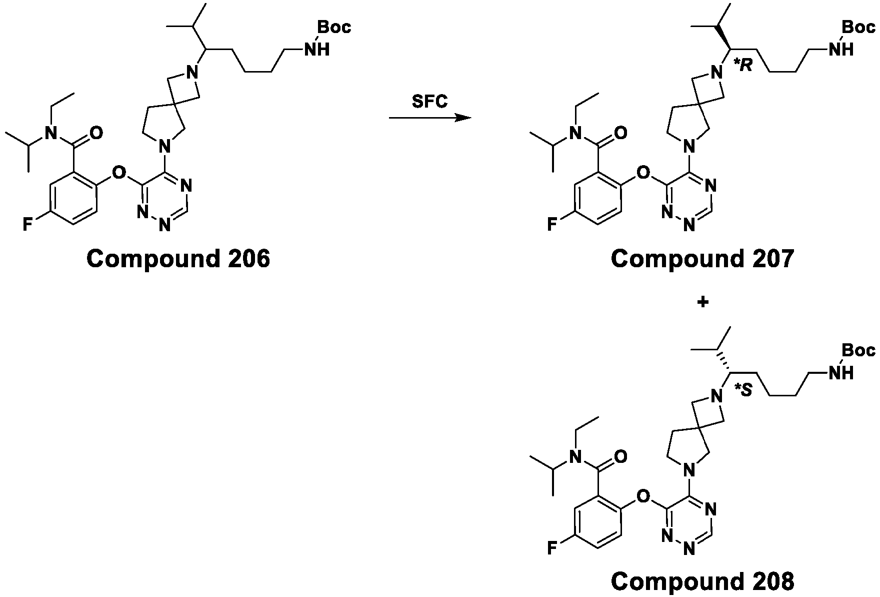

*Tert-*butyl (5-(6-(6-(2-(ethyl(isopropyl)carbamoyl)-4-fluorophenoxy)-1,2,4-triazin-5-yl)-2,6-diazaspiro[3.4]octan-2-yl)-6-methylheptyl)carbamate **(Compound 206)** (1.4 g) was purified by SFC over DAICEL CHIRALPAK IG (column: 250×30 mm, 10 µm; Mobile phase: A: Supercritical CO₂, B: MeOH (0.1% ammonia), A:B = 55:45 at 200 mL/min) to afford the title compounds **(Compound 207)** (700 mg) and **(Compound 208)** (700 mg) both as white solid.

### Compound 304 and 305

### tert-butyl ((4*R)-4-(6-(6-(2-(ethyl(isopropyl)carbamoyl)-4-fluorophenoxy)-1,2,4-triazin-5-yl)-2,6-diazaspiro[3.4]octan-2-yl)-2-methoxy-5-methylhexyl)(methyl)carbamate

### tert-butyl ((4*S)-4-(6-(6-(2-(ethyl(isopropyl)carbamoyl)-4-fluorophenoxy)-1,2,4-triazin-5-yl)-2,6-diazaspiro[3.4]octan-2-yl)-2-methoxy-5-methylhexyl)(methyl)carbamate

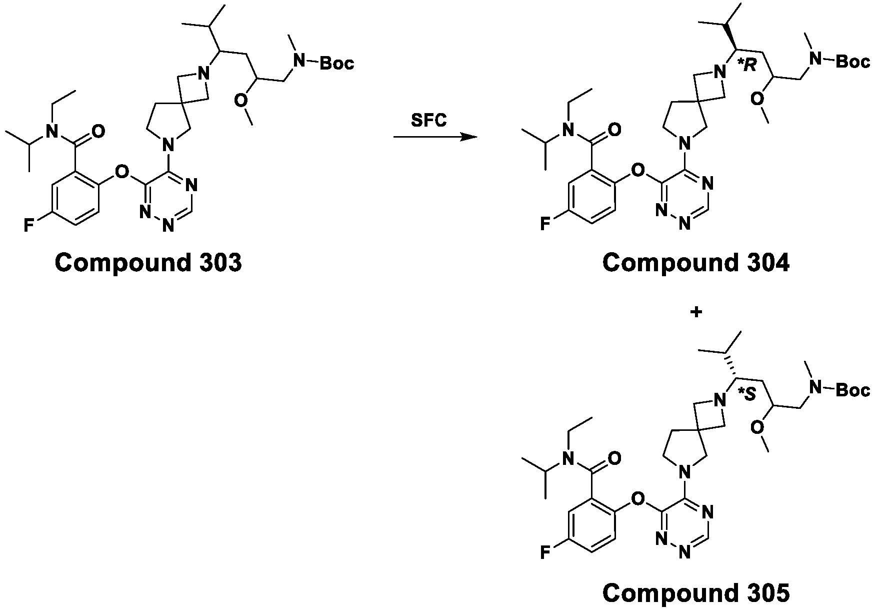

*tert*-butyl (4-(6-(6-(2-(ethyl(isopropyl)carbamoyl)-4-fluorophenoxy)-1,2,4-triazin-5-yl)-2,6-diazaspiro[3.4]octan-2-yl)-2-methoxy-5-methylhexyl)(methyl)carbamate **(Compound 303)** (250 mg) was separated by SFC over DAICEL CHIRALPAK IG (column: 250×30 mm, 10 µm; Mobile phase: A: Supercritical CO₂, B: MeOH (0.1% ammonia), A:B = 60:40; Flow rate: 80 mL/min) to afford the title compounds **(Compound 304)** (124 mg) and **(Compound 305)** (124 mg) both as colorless sticky oil.

### Compound 306 and 307

### tert-butyl ((2*R,4*R)-4-(6-(6-(2-(ethyl(isopropyl)carbamoyl)-4-fluorophenoxy)-1,2,4-triazin-5-yl)-2,6-diazaspiro[3.4]octan-2-yl)-2-methoxy-5-methylhexyl)(methyl)carbamate

### tert-butyl ((2 *S,4 *R)-4-(6-(6-(2-(ethyl(isopropyl)carbamoyl)-4-fluorophenoxy)-1,2,4-triazin-5-yl)-2,6-diazaspiro[3.4]octan-2-yl)-2-methoxy-5-methylhexyl)(methyl)carbamate

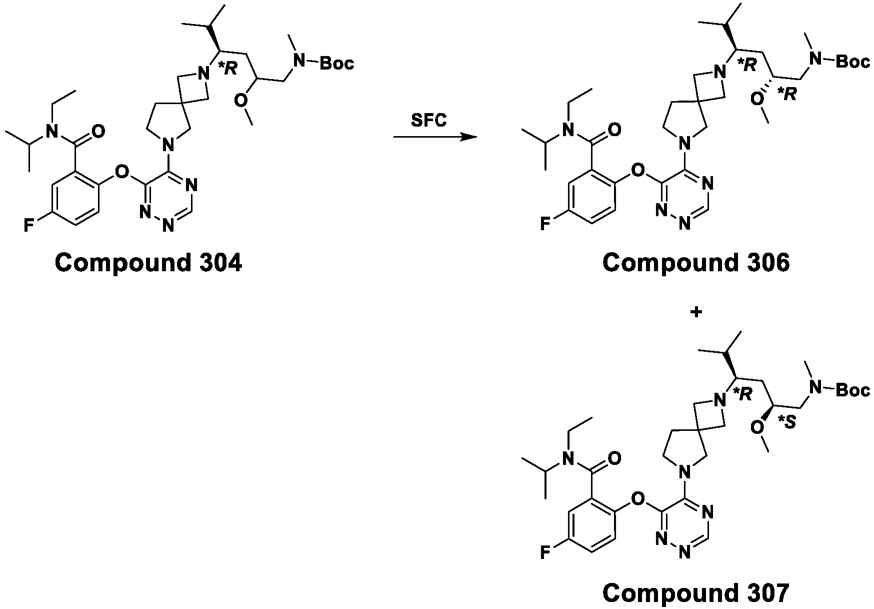

*Tert*-butyl ((4**R*)-4-(6-(6-(2-(ethyl(isopropyl)carbamoyl)-4-fluorophenoxy)-1,2,4-triazin-5-yl)-2,6-diazaspiro[3.4]octan-2-yl)-2-methoxy-5-methylhexyl)(methyl)carbamate **(Compound 304)** (120 mg) was separated by SFC over DAICEL CHIRALPAK IG (column: 250×30 mm,10 um; Mobile phase: A: Supercritical CO₂, B: MeOH (0.1% ammonia), A:B = 70:30 at 80 mL/min) to afford the title compounds **(Compound 306) (45** mg) and **(Compound 307)** (46 mg) both as colorless sticky oil.

### Compound 371 and 372

### tert-butyl ((2 *S,4 *S)-4-(6-(6-(2-(ethyl(isopropyl)carbamoyl)-4-fluorophenoxy)-1,2,4-triazin-5-yl)-2,6-diazaspiro[3.4]octan-2-yl)-2-methoxy-5-methylhexyl)(methyl)carbamate

### tert-butyl ((2 *R,4 *S)-4-(6-(6-(2-(ethyl(isopropyl)carbamoyl)-4-fluorophenoxy)-1,2,4-triazin-5-yl)-2,6-diazaspiro[3.4]octan-2-yl)-2-methoxy-5-methylhexyl)(methyl)carbamate

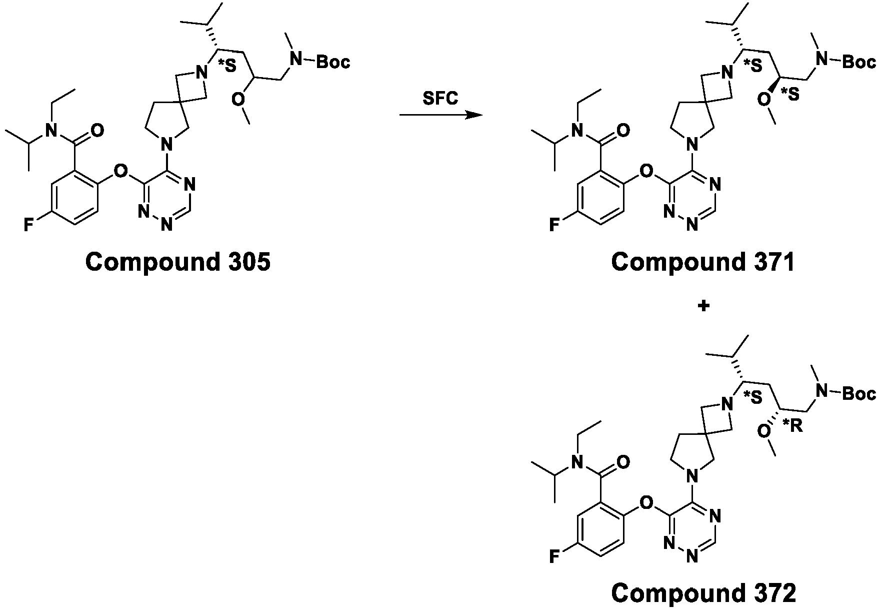

*Tert*-butyl ((4**S*)-4-(6-(6-(2-(ethyl(isopropyl)carbamoyl)-4-fluorophenoxy)-1,2,4-triazin-5-yl)-2,6-diazaspiro[3.4]octan-2-yl)-2-methoxy-5-methylhexyl)(methyl)carbamate **(Compound 305)** (120 mg) was separated by SFC over DAICEL CHIRALPAK IG (column: 250×30 mm, 10 µm; Mobile phase: A: Supercritical CO₂, B: IPA (0.1% ammonia), A:B = 60:40; Flow rate: 80 mL/min) to afford the title compounds **(Compound 371)** (45 mg) and **(Compound 372)** (46 mg) both as colorless sticky oil.

### Compound 404 and 405

### tert-butyl (R)-(4-(6-(3-chloro-6-(2-(ethyl(isopropyl)carbamoyl)-4-fluorophenoxy)-1,2,4-triazin-5-yl)-2,6-diazaspiro[3.4]octan-2-yl)-5-methylhexyl)carbamate

### tert-butyl (S)-(4-(6-(3-chloro-6-(2-(ethyl(isopropyl)carbamoyl)-4-fluorophenoxy)-1,2,4-triazin-5-yl)-2,6-diazaspiro[3.4]octan-2-yl)-5-methylhexyl)carbamate

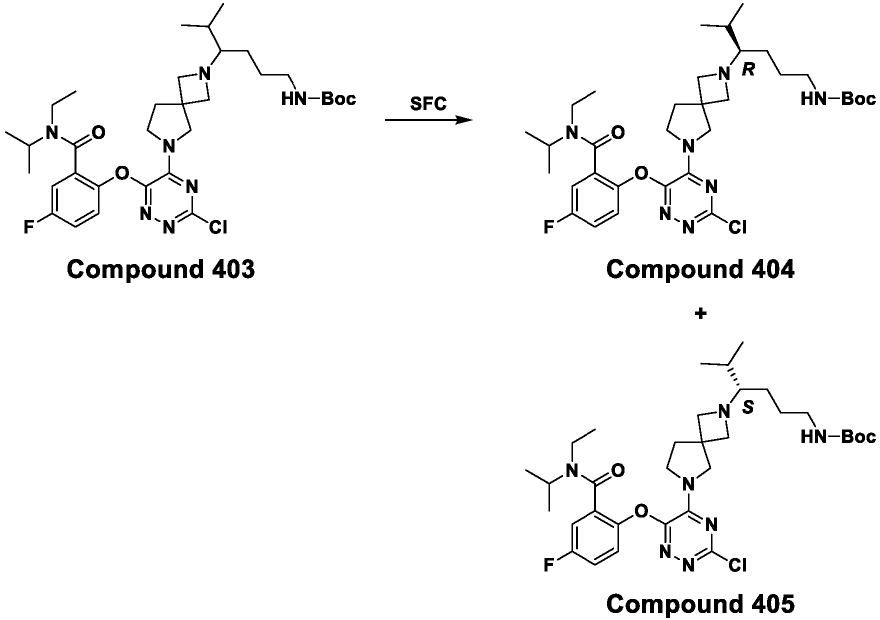

*Tert*-butyl (4-(6-(3-chloro-6-(2-(ethyl(isopropyl)carbamoyl)-4-fluorophenoxy)-1,2,4-triazin-5-yl)-2,6-diazaspiro[3.4]octan-2-yl)-5-methylhexyl)carbamate **(Compound 403)** (19.5 g) was separated by SFC over DAICEL CHIRALPAK IG (column: 250×30 mm, 10 um; Mobile phase: A: Supercritical CO₂, B: MeOH (0.1% ammonia), A:B =55:45 at 80 mL/min; Column Temp: 38 °C; Nozzle Pressure: 100 Bar; Nozzle Temp: 60 °C; Evaporator Temp: 20 °C; Trimmer Temp: 25 °C; Wavelength: 220 nm) to afford the title compounds **(Compound 404)** (8.00 g) and **(Compound 405)** (7.00 g) both as sticky oil.

### Compound 1

### (R)-2-((5-(2-(6-amino-2-methylhexan-3-yl)-2,6-diazaspiro[3.4]octan-6-yl)-1,2,4-triazin-6-yl)oxy)-N-ethyl-5-fluoro-N-isopropylbenzamide formate

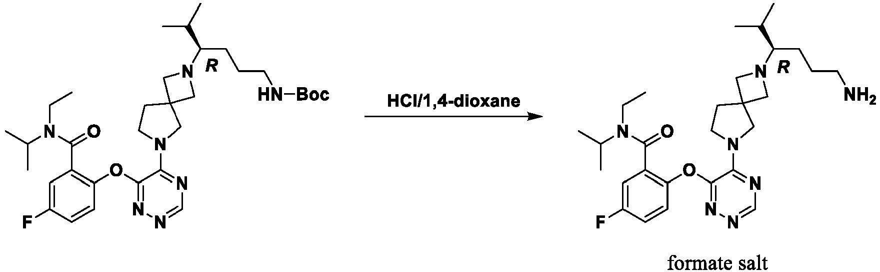

HCl/1,4-dioxane (0.5 mL, 2.0 mmol) was added to a solution of *tert*-butyl (*R*)-(4-(6-(6-(2-(ethyl(isopropyl)carbamoyl)-4-fluorophenoxy)-1,2,4-triazin-5-yl)-2,6-diazaspiro[3.4]octan-2-yl)-5-methylhexyl)carbamate **(Compound 62)** (85 mg, 0.14 mmol) in 1,4-dioxane (2 mL). The reaction mixture was sitrred at RT for 4 h. The mixture was concentrated under reduced pressure and the residue was first neutralized by ammonia (5 mL) and further purified by preparative HPLC using a Welch Xtimate C18 (column: 150x25 mm 5µm; eluent: ACN/H₂O (0.225%FA) from 1% to 31% (v/v)) to afford the title compound (32 mg, 41% yield) as a colorless oil.

**¹H NMR (400 MHz, Methanol-*d*₄):** δ = 8.45-8.41 (m, 3H), 7.48-7.13 (m, 3H), 4.50-4.01 (m, 6H), 3.98-3.66 (m, 3H), 3.56-3.38 (m, 1H), 3.25-3.12 (m, 1H), 3.10-3.01 (m, 1H), 2.99-2.87 (m, 2H), 2.43-2.18 (m, 2H), 2.13-1.96 (m, 1H), 1.84-1.44 (m, 4H), 1.25-0.92 (m, 13H), 0.87-0.69 (m, 2H).

**LC-MS (ESI) (Method 1):** Rt = 2.957 min, m/z found 528.3 [M+H]⁺.

**SFC (Method 12):** Rt = 1.151 min.

### Preparation of Compound 60

### tert-butyl (4-(6-(6-(2-(ethyl(isopropyl)carbamoyl)-4-fluorophenoxy)-1,2,4-triazin-5-yl)-2,6-diazaspiro[3.4]octan-2-yl)-5-methylhexyl)(methyl)carbamate

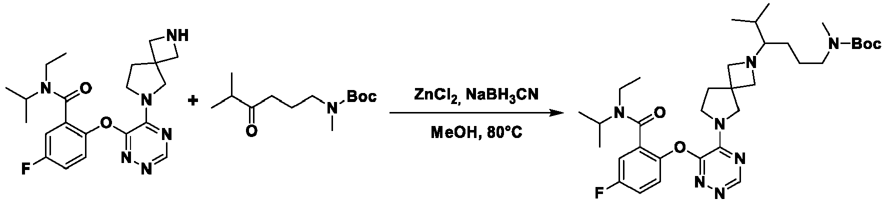

To a solution of 2-((5-(2,6-diazaspiro[3.4]octan-6-yl)-1,2,4-triazin-6-yl)oxy)-*N-*ethyl-5-fluoro-*N*-isopropylbenzamide **(intermediate 3)** (600 mg, 1.45 mmol) and *tert*-butyl methyl(5-methyl-4-oxohexyl)carbamate **(intermediate 9)** (330 mg, 1.37 mmol) in MeOH (50 mL) was added ZnCl₂ (789 mg, 5.79 mmol). The resulting mixture was stirred at 80 °C for 2 h. Then NaBH₃CN (729 mg, 11.6 mmol) was added and the reaction mixture was stirred at 80°C overnight. After cooling to RT, the mixture was concentrated under reduced pressure to give a crude residue, which was diluted with DCM (50 mL), quenched with sat. aq. NH₄Cl (50 mL) and extracted with DCM (50 mL x 3). The combined organic layers were washed with brine (50 mL), dried over Na₂SO₄, filtered and the filtrate was concentrated under reduced pressure to give a crude product which was further purified by FCC (DCM/MeOH = 10:1) to afford the title compound (400 mg, 42% yield) as white solid.

### Compound 56 and 57

### tert-butyl (R)-(4-(6-(6-(2-(ethyl(isopropyl)carbamoyl)-4-fluorophenoxy)-1,2,4-triazin-5-yl)-2,6-diazaspiro[3.4]octan-2-yl)-5-methylhexyl)(methyl)carbamate

### tert-butyl (S)-(4-(6-(6-(2-(ethyl(isopropyl)carbamoyl)-4-fluorophenoxy)-1,2,4-triazin-5-yl)-2,6-diazaspiro[3.4]octan-2-yl)-5-methylhexyl)(methyl)carbamate

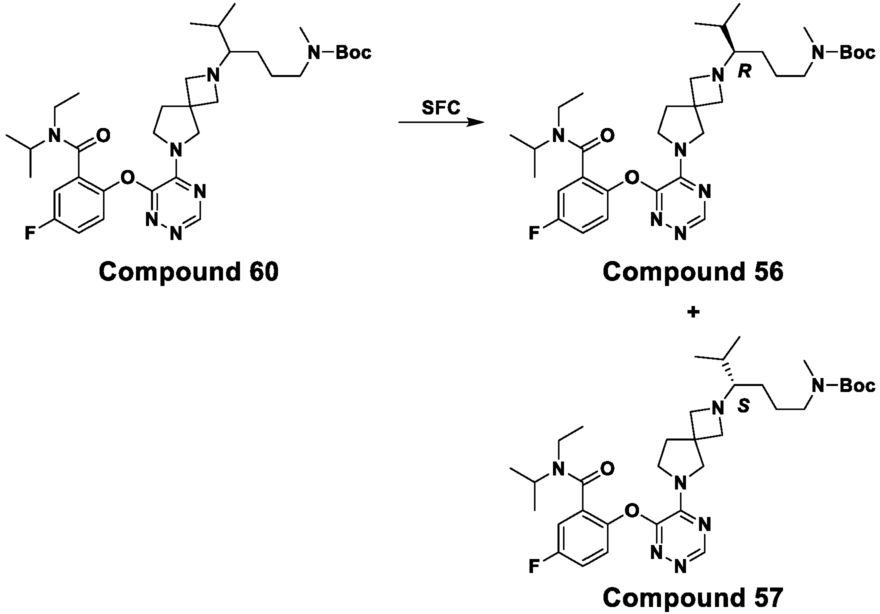

*Tert-*butyl (4-(6-(6-(2-(ethyl(isopropyl)carbamoyl)-4-fluorophenoxy)-1,2,4-triazin-5-yl)-2,6-diazaspiro[3.4]octan-2-yl)-5-methylhexyl)(methyl)carbamate **(Compound 60)** (419 mg, 0.653 mmol) was purified by SFC over DAICEL CHIRALPAK AD (column: 250x30 mm 10 µm; Mobile phase: A: Supercritical CO₂, B: IPA (0.1% ammonia), A:B = 80:20 at 60 mL/min; Column Temp: 38 °C; Nozzle Pressure: 100 Bar; Nozzle Temp: 60 °C; Evaporator Temp: 20 °C; Trimmer Temp: 25 °C; Wavelength: 220 nm) to afford the title compounds **(Compound 56)** (146 mg, 34% yield) and **(Compound 57)** (149 mg, 36% yield) both as white solid.

### Compound 19

### (R)-N-ethyl-5-fluoro-N-isopropyl-2-((5-(2-(2-methyl-6-(methylamino)hexan-3-yl)-2,6-diazaspiro[3.4]octan-6-yl)-1,2,4-triazin-6-yl)oxy)benzamide hydrochloride

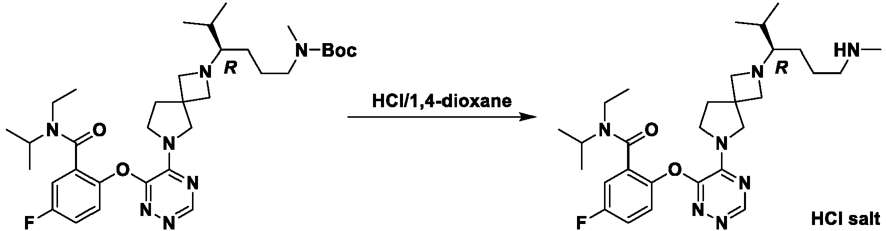

To a solution of *tert*-butyl (*R*)-(4-(6-(6-(2-(ethyl(isopropyl)carbamoyl)-4-fluorophenoxy)-1,2,4-triazin-5-yl)-2,6-diazaspiro[3.4]octan-2-yl)-5-methylhexyl)(methyl)carbamate **(Compound 56)** (130 mg, 0.203 mmol) in 1,4-dioxane (3 mL) was added HCl/1,4-dioxane (5 mL, 20.0 mmol), and the reaction mixture was stirred at RT for 1 h. The reaction mixture was concentrated *in vacuo* and the residue was purified by preparative HPLC over Phenomenex Gemini-NX (column: 150x30 mm 5um, Mobile Phase A: water (0.05%HCl), Mobile Phase B: ACN, Flow rate: 25 mL/min, gradient condition B/A from 0% B to 26% (0% B to 26% B)) to afford the title compound (105 mg, 84% yield) as colorless oil.

**LC-MS (ESI) (Method 1):** Rt = 2.939 min, m/z found 542.4 [M+H]⁺.

**SFC (Method** 1): Rt = 1.201 min.

### Compound 398

### (R)-N-ethyl-5-fluoro-N-isopropyl-2-((5-(2-(2-methyl-6-(methylamino)hexan-3-yl)-2,6-diazaspiro[3.4]octan-6-yl)-1,2,4-triazin-6-yl)oxy)benzamide

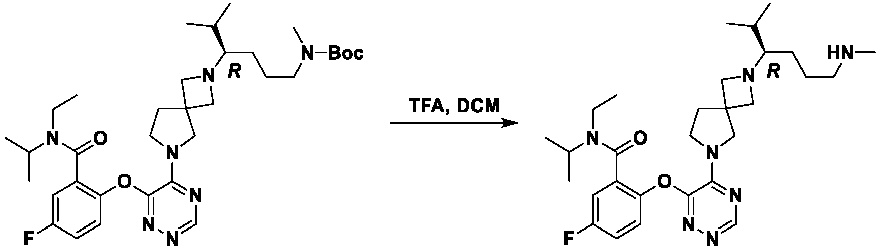

At 5 °C, TFA (0.51 mL, 6.7 mmol) was added dropwise to a solution of *tert*-butyl (*R*)-(4-(6-(6-(2-(ethyl(isopropyl)carbamoyl)-4-fluorophenoxy)-1,2,4-triazin-5-yl)-2,6-diazaspiro[3.4]octan-2-yl)-5-methylhexyl)(methyl)carbamate **(Compound 56)** (287 mg, 0.45 mmol) in DCM (7.5 mL) and the reaction mixture was stirred overnight. The reaction mixture was evaporated to dryness to give a crude mixture (540 mg) which was purified by silica gel chromatography (Stationary phase: irregular bare silica 12g, Mobile phase: Gradient from 95% DCM, 5% MeOH (+10% NH₄OH) to 90% DCM, 10% MeOH (+10% NH₄OH)). The pure fractions were mixed and concentrated to afford 173 mg of an intermediate fractions which was freeze-dried with ACN/H₂O (20/80, v/v) to afford of the title compound (170 mg, 70% yield).

**LC-MS (ESI) (Method 4):** Rt = 2.08 min, m/z found 542.6 [M+H]⁺.

### Compound 51

### tert-butyl (3-(6-(6-(2-(ethyl(isopropyl)carbamoyl)-4-fluorophenoxy)-1,2,4-triazin-5-yl)-2,6-diazaspiro[3.4]octan-2-yl)-4-methylpentyl)carbamate

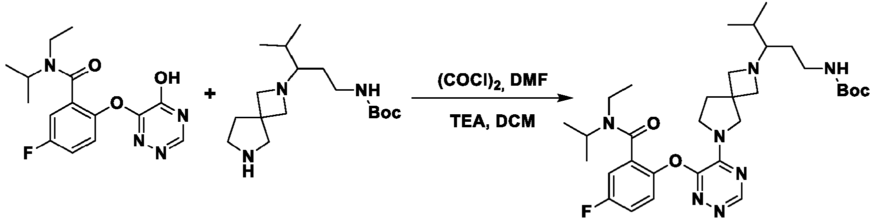

To a solution of *N*-ethyl-5-fluoro-2-((5-hydroxy-1,2,4-triazin-6-yl)oxy)-N-isopropylbenzamide **(intermediate 25)** (0.100 g, 0.312 mmol) in DCM (12 mL) was added oxalyl chloride (0.079 g, 0.624 mmol), followed by DMF (0.046 g, 0.624 mmol) at RT. The mixture was stirred at this temperature for 1 h. Then the mixture was added to a solution of *tert*-butyl (4-methyl-3-(2,6-diazaspiro[3.4]octan-2-yl)pentyl)carbamate hydrochloride **(intermediate 22)** (0.272 g, crude) and TEA (0.158 g, 1.56 mmol) in DCM (3 mL). The resulting mixture was stirred at 25 °C for 0.5 h. The reaction mixture was concentrated under reduced pressure and the residue was partitioned between DCM (35 mL) and H₂O (35 mL), extracted with DCM (35 mL x 3). The combined organic layers were dried over Na₂SO₄, filtered and concentrated. The residue was purified by FCC (PE/EtOAc (0.5% ammonia) = 1/1) to afford the title compound (100 mg, 89% purity, 46% yield) as colorless oil.

### Compound 52 and 53

### tert-butyl (*R)-(5-(6-(6-(2-(4-cyclopropylpyrimidin-5-yl)-4-fluorophenoxy)-1,2,4-triazin-5-yl)-2,6-diazaspiro[3.4]octan-2-yl)-2,6-dimethylheptan-2-yl)carbamate

### tert-butyl (*S)-(5-(6-(6-(2-(4-cyclopropylpyrimidin-5-yl)-4-fluorophenoxy)-1,2,4-triazin-5-yl)-2,6-diazaspiro[3.4]octan-2-yl)-2,6-dimethylheptan-2-yl)carbamate

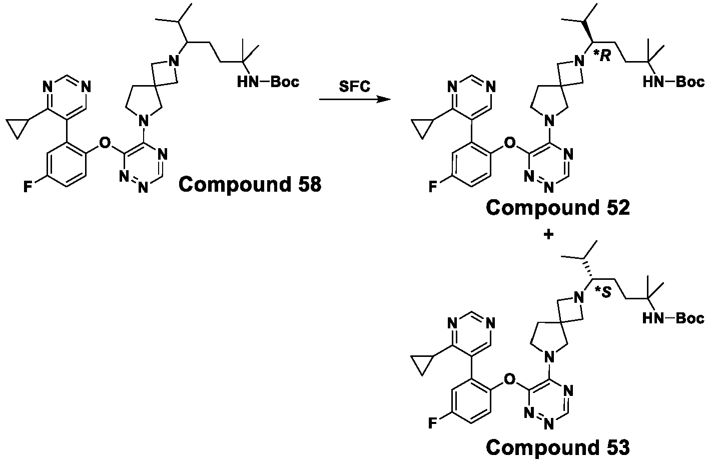

*tert*-butyl (5-(6-(6-(2-(4-cyclopropylpyrimidin-5-yl)-4-fluorophenoxy)-1,2,4-triazin-5-yl)-2,6-diazaspiro[3.4]octan-2-yl)-2,6-dimethylheptan-2-yl)carbamate **(Compound 58)** (150 mg, 0.227 mmol) was purified by SFC over DAICEL CHIRALPAK AD-H (column: 250x30 mm 5 µm; Mobile phase: A: Supercritical CO₂, B: IPA (0.1% ammonia), A: B = 4:1 at 60 mL/min) to afford the title compounds **Compound 52** (47 mg, 96.3% purity, 30.2% yield) and **Compound 53** (56 mg, 97.7% purity, 36.5% yield) both as white solids.

### Compound 54 and 55

### tert-butyl (*R)-(5-(6-(6-(2-(ethyl(isopropyl)carbamoyl)-4-fluorophenoxy)-1,2,4-triazin-5-yl)-2,6-diazaspiro[3.4]octan-2-yl)-2,6-dimethylheptan-2-yl)carbamate

### tert-butyl (*S)-(5-(6-(6-(2-(ethyl(isopropyl)carbamoyl)-4-fluorophenoxy)-1,2,4-triazin-5-yl)-2,6-diazaspiro[3.4]octan-2-yl)-2,6-dimethylheptan-2-yl)carbamate

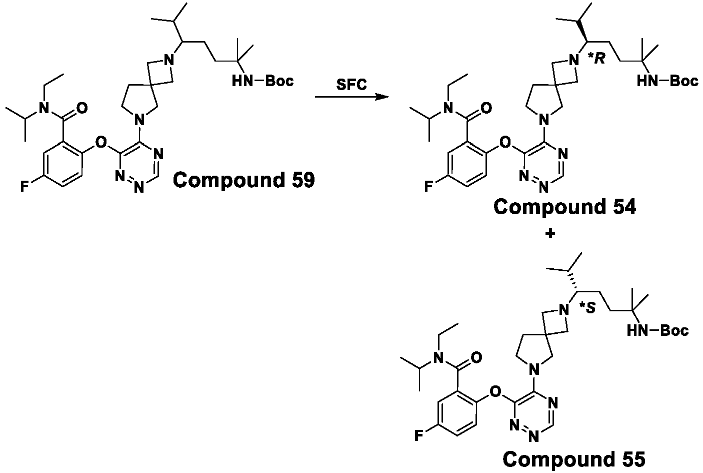

*tert*-butyl (5-(6-(6-(2-(ethyl(isopropyl)carbamoyl)-4-fluorophenoxy)-1,2,4-triazin-5-yl)-2,6-diazaspiro[3.4]octan-2-yl)-2,6-dimethylheptan-2-yl)carbamate **(Compound 59)** (1.70 g, 2.59 mmol) was separated by SFC over DAICEL CHIRALPAK IG (column: 250x50 mm 10 µm)); Mobile phase: A: Supercritical CO₂, B: EtOH (0.1% ammonia), A: B = 3:2 at 150 mL/min) to afford the title compounds **Compound 54** (700 mg, 90% purity, 37% yield) and **Compound 55** (700 mg, purity: 96% purity, 40% yield) both as a white solid.

### Compound 408

### tert-butyl (R)-(4-(6-(6-(2-(ethyl(isopropyl)carbamoyl)-4-fluorophenoxy)-3-(methylamino)-1,2,4-triazin-5-yl)-2,6-diazaspiro[3.4]octan-2-yl)-5-methylhexyl)carbamate

**The following compounds was synthesized by an analogous method as described above for Compound 395**

| **Co. No.** | **Structure** | **Starting Material** | **Conditions** |
|---|---|---|---|
| 408 | 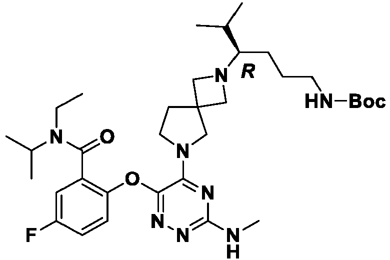 | Compound 404 | methanamine in EtOH (33%), 90°C, 1 h |

### Compound 412

### tert-butyl (R)-(4-(6-(6-(2-(ethyl(isopropyl)carbamoyl)-4-fluorophenoxy)-3-methyl-1,2,4-triazin-5-yl)-2,6-diazaspiro[3.4]octan-2-yl)-5-methylhexyl)carbamate

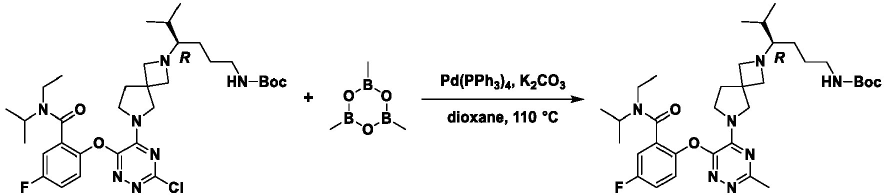

To the mixture of *tert*-butyl (*R*)-(4-(6-(3-chloro-6-(2-(ethyl(isopropyl)carbamoyl)-4-fluorophenoxy)-1,2,4-triazin-5-yl)-2,6-diazaspiro[3.4]octan-2-yl)-5-methylhexyl)carbamate **(Compound 404)** (50.0 mg, 0.076 mmol), 2,4,6-trimethyl-1,3,5,2,4,6-trioxatriborinane (76.0 mg, 0.303 mmol, 50% in THF) and K₂CO₃ (21.0 mg, 0.152 mmol) in anhydrous dioxane (1 mL) was added Pd(PPh₃)₄ (8.7 mg, 0.008 mmol) and the resulting mixture was stirred at 110 °C for 8 h under N₂ atmosphere. After cooled to RT, the mixture was diluted with H₂O (40 mL) and extracted with EtOAc (20 mL × 3). The combined organic layers were dried over anhydrous Na₂SO₄ and filtered. The filtrate was concentrated under reduced pressure to give the crude product which was purified by preparative TLC (DCM/MeOH = 10/1) to afford the title compound (30.0 mg, 59.7% yield) as yellow solid.
**Compounds 2, 3, 20, 30, 31, 37, 38, 26, 80, 209, 210, 218, 220, 221, 308, 309, 317, 328, 359, 373, 374, 409, 413**
**(*S*)-2-((5-(2-(6-amino-2-methylhexan-3-yl)-2,6-diazaspiro[3.4]octan-6-yl)-1,2,4-triazin-6-yl)oxy)-*N*-ethyl-5-fluoro-*N*-isopropylbenzamide formate**
**2-((5-(2-(6-amino-2-methylhexan-3-yl)-2,6-diazaspiro[3.4]octan-6-yl)-1,2,4-triazin-6-yl)oxy)-*N*-ethyl-5-fluoro-*N*-isopropylbenzamide formate**
**(*S*)-*N*-ethyl-5-fluoro-*N*-isopropyl-2-((5-(2-(2-methyl-6-(methylamino)hexan-3-yl)-2,6-diazaspiro[3.4]octan-6-yl)-1,2,4-triazin-6-yl)oxy)benzamide hydrochloride**
**(**R*)-2-((5-(2-(6-amino-2,6-dimethylheptan-3-yl)-2,6-diazaspiro[3.4]octan-6-yl)-1,2,4-triazin-6-yl)oxy)-*N*-ethyl-5-fluoro-*N*-isopropylbenzamide**
**(**S*)-2-((5-(2-(6-amino-2,6-dimethylheptan-3-yl)-2,6-diazaspiro[3.4]octan-6-yl)-1,2,4-triazin-6-yl)oxy)-*N*-ethyl-5-fluoro-*N*-isopropylbenzamide**
**(**R*)-5-(6-(6-(2-(4-cyclopropylpyrimidin-5-yl)-4-fluorophenoxy)-1,2,4-triazin-5-yl)-2,6-diazaspiro[3.4]octan-2-yl)-2,6-dimethylheptan-2-amine**
**(**S*)-5-(6-(6-(2-(4-cyclopropylpyrimidin-5-yl)-4-fluorophenoxy)-1,2,4-triazin-5-yl)-2,6-diazaspiro[3.4]octan-2-yl)-2,6-dimethylheptan-2-amine**
**2-((5-(2-(1-amino-4-methylpentan-3-yl)-2,6-diazaspiro[3.4]octan-6-yl)-1,2,4-triazin-6-yl)oxy)-*N*-ethyl-5-fluoro-*N*-isopropylbenzamide**
***N*-ethyl-5-fluoro-*N*-isopropyl-2-((4-(2-(2-methyl-6-(methylamino)hexan-3-yl)-2,6-diazaspiro[3.4]octan-6-yl)pyridazin-3-yl)oxy)benzamide**
**(**R*)-2-((5-(2-(7-amino-2-methylheptan-3-yl)-2,6-diazaspiro[3.4]octan-6-yl)-1,2,4-triazin-6-yl)oxy)-*N*-ethyl-5-fluoro-*N*-isopropylbenzamide formate**
**(**S*)-2-((5-(2-(7-amino-2-methylheptan-3-yl)-2,6-diazaspiro[3.4]octan-6-yl)-1,2,4-triazin-6-yl)oxy)-*N*-ethyl-5-fluoro-*N*-isopropylbenzamide**
**(**R*)-2-((5-(2-(1-amino-4-methylpentan-3-yl)-2,6-diazaspiro[3.4]octan-6-yl)-1,2,4-triazin-6-yl)oxy)-*N*-ethyl-5-fluoro-*N*-isopropylbenzamide**
***(*R)-2-((5-(2-(1-amino-4-methylpentan-3-yl)-2,6-diazaspiro[3.4]octan-6-yl)-1,2,4-triazin-*6-yl)oxy)-*N*-ethyl-5-fluoro-*N*-isopropylbenzamide hydrochloride**
**(**S*)-2-((5-(2-(1-amino-4-methylpentan-3-yl)-2,6-diazaspiro[3.4]octan-6-yl)-1,2,4-triazin-6-yl)oxy)-*N*-ethyl-5-fluoro-*N*-isopropylbenzamide hydrochloride**
***N*-ethyl-5-fluoro-N-isopropyl-2-((5-(2-((3 *R,5 *R)-5-methoxy-2-methyl-6-(methylamino)hexan-3-yl)-2,6-diazaspiro[3.4]octan-6-yl)-1,2,4-triazin-6-yl)oxy)benzamide hydrochloride**
***N*-ethyl-5-fluoro-N-isopropyl-2-((5-(2-((3 *R,5 *S)-5-methoxy-2-methyl-6-(methylamino)hexan-3-yl)-2,6-diazaspiro[3.4]octan-6-yl)-1,2,4-triazin-6-yl)oxy)benzamide hydrochloride**
**N-ethyl-5-fluoro-2-((5-(2-(5-hydroxy-2-methyl-6-(methylamino)hexan-3-yl)-2,6-diazaspiro[3.4]octan-6-yl)-1,2,4-triazin-6-yl)oxy)-*N*-isopropylbenzamide hydrochloride**
***N*-ethyl-2-((5-(2-(6-(ethylamino)-5-hydroxy-2-methylhexan-3-yl)-2,6-diazaspiro[3.4]octan-6-yl)-1,2,4-triazin-6-yl)oxy)-5-fluoro-*N*-isopropylbenzamide hydrochloride**
**5-fluoro-2-((5-(2-(5-hydroxy-2-methyl-6-(methylamino)hexan-3-yl)-2,6-diazaspiro[3.4]octan-6-yl)-1,2,4-triazin-6-yl)oxy)-*N,N*-diisopropylbenzamide hydrochloride**
***N*-ethyl-5-fluoro-*N*-isopropyl-2-((5-(2-((3**S*,5**S*)-5-methoxy-2-methyl-6-(methylamino)hexan-3-yl)-2,6-diazaspiro[3.4]octan-6-yl)-1,2,4-triazin-6-yl)oxy)benzamide hydrochloride**
***N-ethyl-5-fluoro-N-isopropyl-2-((5-(2-((3 *S,5 *R)-5-methoxy-2-methyl-6-*(methylamino)hexan-3-yl)-2,6-diazaspiro[3.4]octan-6-yl)-1,2,4-triazin-6-yl)oxy)benzamide hydrochloride**
**(*R*)-2-((5-(2-(6-amino-2-methylhexan-3-yl)-2,6-diazaspiro[3.4]octan-6-yl)-3-(methylamino)-1,2,4-triazin-6-yl)oxy)-*N*-ethyl-5-fluoro-*N*-isopropylbenzamide hydrochloride**
**(*R*)-2-((5-(2-(6-amino-2-methylhexan-3-yl)-2,6-diazaspiro[3.4]octan-6-yl)-3-methyl-1,2,4-triazin-6-yl)oxy)-*N*-ethyl-5-fluoro-*N*-isopropylbenzamide formate**

**The following Compounds were synthesized by an analogous method described above for Compound 1 and 19**

| **Co. No.** | **Structure** | **Starting Material** | **Conditions** | **Spectra Details** |
|---|---|---|---|---|
| 2 | 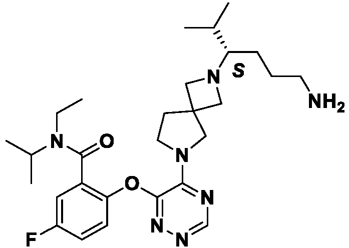 formate salt | Compound 63 | HCl | **LC-MS (ESI) (Method 1):** Rt = 3.028 min, m/z found 528.3 [M+H]⁺. |
| | | | 1,4-dioxane | **SFC (Method 12):** Rt = 1.502 min. |
| 3 | 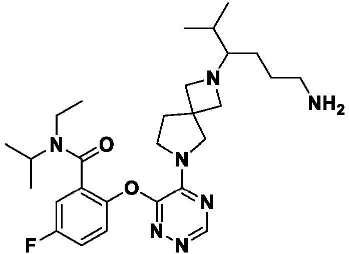 formate salt | Compound 61 | HCl | **LC-MS (ESI) (Method** 1): Rt = 2.977 min, m/z found 528.4 [M+H]⁺. |
| | | | 1,4-dioxane | |
| 20 | 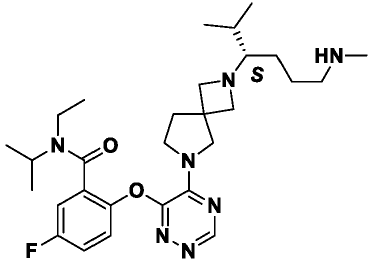 HCl salt | Compound 57 | HCl | **LC-MS (ESI) (Method 1):** Rₜ = 2.890 min, m/z found 542.3 [M+H]⁺. |
| | | | 1,4-dioxane | **SFC (Method 1):** Rₜ = 1.697 min. |
| 30 | 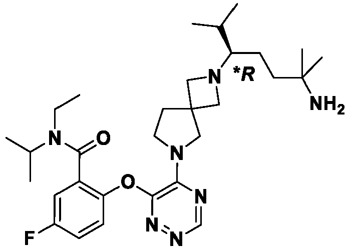 | Compound 54 | TFA | **LC-MS (ESI) (Method 1):** Rt = 2.931 min, m/z found 556.3 [M+H]⁺. |
| | | | DCM | **SFC (Method 2):** Rₜ = 4.431 min. |
| 31 | 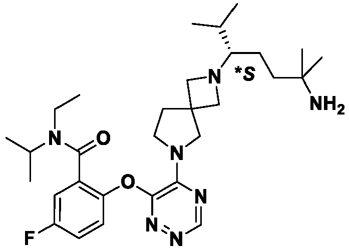 | Compound 55 | TFA | LC-MS (ESI) (Method 1): Rt = 2.897 min, m/z found 556.3 [M+H]⁺. |
| | | | DCM | SFC (Method 2): Rt = 4.997 **min.** |
| 37 | 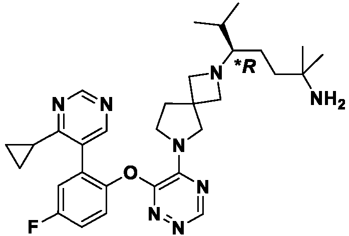 | Compound 52 | TFA | **¹H NMR (400 MHz, Methanol-*d*₄):** *δ* 8.88 (brs, 1H), 8.46-8.36 (m, 2H), 7.58-7.45 (m, 1H), 7.44-7.26 (m, 2H), 4.07-3.52 (m, 4H), 3.31-3.11 (m, 4H), 2.24-2.02 (m, 3H), 1.99-1.78 (m, 2H), 1.55-1.38 (m, 3H), 1.37-1.20 (m, 2H), 1.14-1.06 (m, 8H), 0.99-0.83 (m, 7H). |
| | | | DCM | |
| | | | | **LC-MS (ESI) (Method** 1): Rt = 3.21 min, m/z found 561.3 [M+H]⁺. |
| | | | | **SFC (Method 3):** Rt = 5.566 min. |
| 38 | 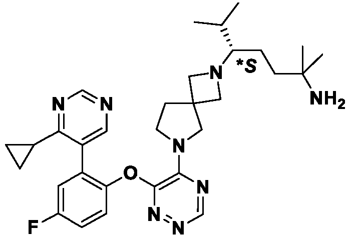 | Compound 53 | TFA | **LC-MS (ESI) (Method 1):** Rt = 3.26 min, m/z found 561.3 [M+H]⁺. |
| | | | DCM | **SFC (Method 3):** Rₜ = 5.929 min. |
| 26 | 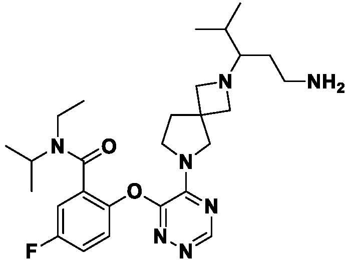 | Compound 51 | TFA | **LC-MS (ESI) (Method 1):** Rt = 2.98 min, m/z found 514.4 M+H]⁺. |
| | | | DCM | |
| 80 | 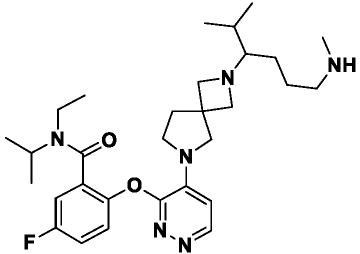 | Compound 79 | TFA | |
| | | | DCM | |
| 209 | 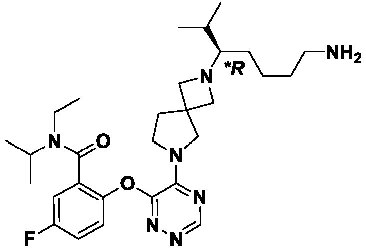 formate salt | Compound 207 | HCl | **LC-MS (ESI) (Method 1):** Rₜ = 2.950 min, m/z found 542.3 [M+H]⁺. |
| | | | 1,4-dioxane | |
| | | | | **SFC (Method 18):** Rₜ = 2.021 min. |
| 210 | 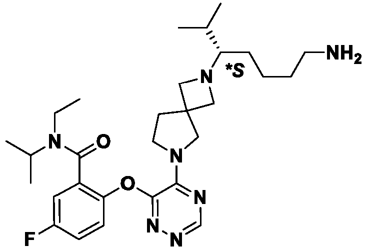 | Compound 208 | HCl | **LC-MS (ESI) (Method 1):** Rₜ = 2.919 min, m/z found 542.3 [M+H]⁺. |
| | | | 1,4-dioxane | **SFC (Method 18):** Rₜ = 2.201 min. |
| 218 | 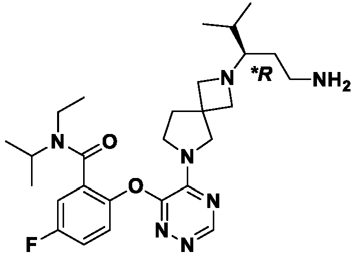 | Compound 216 | TFA | |
| | | | DCM | |
| 220 | 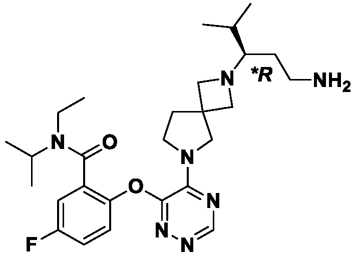 HCl salt | Compound 216 | HCl | |
| | | | 1,4-dioxane | |
| 221 | 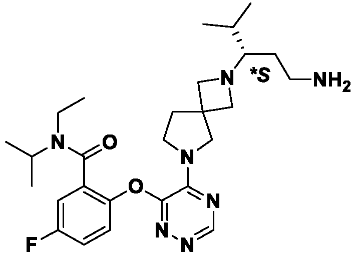 HCl salt | Compound 217 | HCl | |
| | | | 1,4-dioxane | |
| 308 | 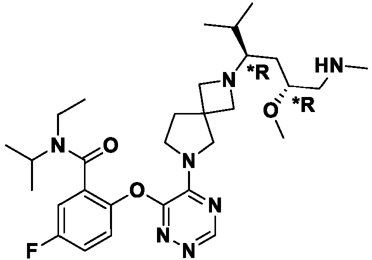 HCl salt | Compound 306 | HCl/1,4-dioxane, MeOH | |
| 309 | 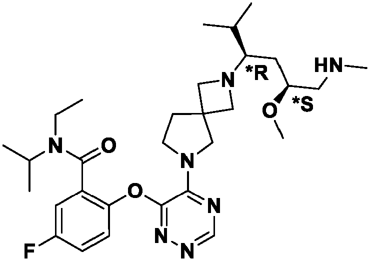 HCl salt | Compound 307 | HCl/1,4-dioxane, MeOH | |
| 317 | 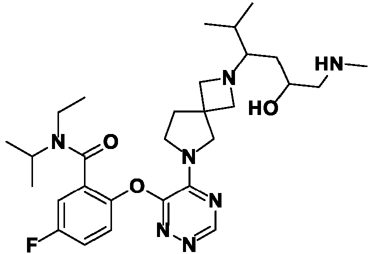 HCl salt | Compound 316 | HCl/1,4-dioxane, MeOH | |
| 328 | 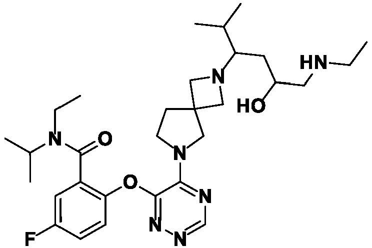 HCl salt | Compound 327 | HCl/1,4-dioxane, MeOH | |
| 359 | 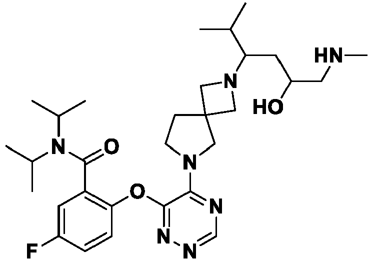 HCl salt | Compound 358 | HCl/1,4-dioxane, MeOH | |
| 373 | 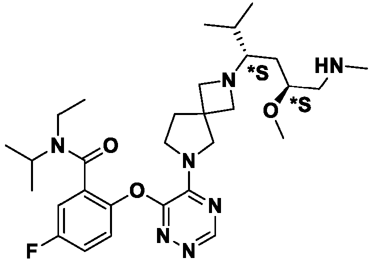 HCl salt | Compound 371 | HCl/1,4-dioxane, MeOH | |
| 374 | 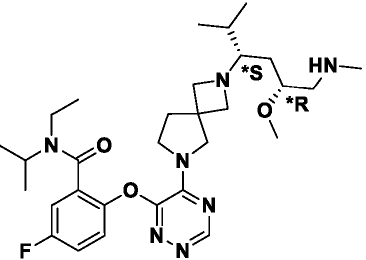 HCl salt | Compound 372 | HCl/1,4-dioxane, MeOH | |
| 409 | 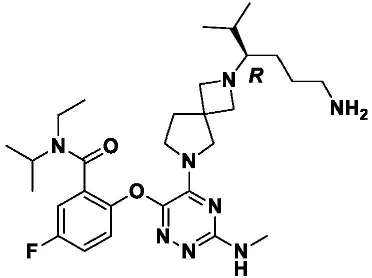 HCl salt | Compound 408 | HCl/1,4-dioxane, ACN | **LC-MS (ESI) (Method 2):** Rt = 1.94 min, m/z found 557.3 [M+H]⁺. |
| | | | | SFC (Method 13): Rₜ = 2.75 min. |
| 413 | 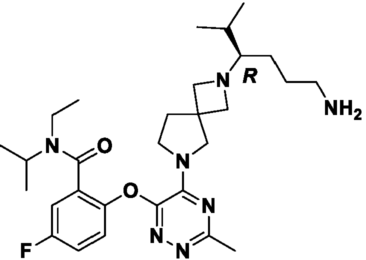 formate salt | Compound 412 | HCl/1,4-dioxane, MeOH | **LC-MS (ESI) (Method 1):** Rt = 2.885 min, m/z found 542.3 [M+H]⁺. |
| | | | | **SFC (Method 13):** Rₜ = 2.347 min. |

### Compound 4

### (R)-2-((5-(2-(6-(dimethylamino)-2-methylhexan-3-yl)-2,6-diazaspiro[3.4]octan-6-yl)-1,2,4-triazin-6-yl)oxy)-N-ethyl-5-fluoro-N-isopropylbenzamide

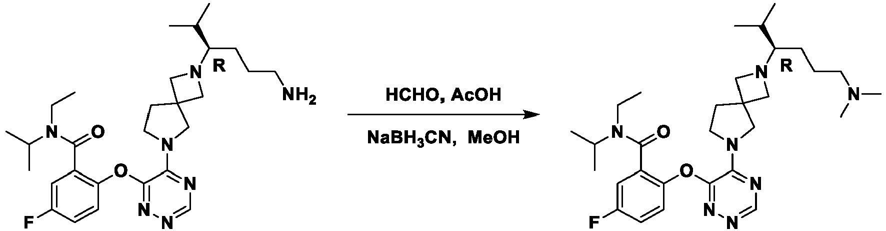

To the mixture of (*R*)-2-((5-(2-(6-amino-2-methylhexan-3-yl)-2,6-diazaspiro[3.4]octan-6-yl)-1,2,4-triazin-6-yl)oxy)-N-ethyl-5-fluoro-N-isopropylbenzamide hydrochloride **(Compound 65)** (180 mg, crude), formaldehyde (0.085 mL, 1.1 mmol) and AcOH (0.043 mL, 0.76 mmol) in MeOH (10 mL) was added NaBH₃CN (72.0 mg, 1.14 mmol), the resulting mixture was stirred at RT for 2 h. The mixture was filtered and the filtrate was purified by preparative HPLC over Welch Xtimate (column: C18 150x30mm 5um; eluent: ACN/H₂O (0.225% FA) from 5% to 25%, v/v) and the desired fractions were collected and freeze dried. The resulting solid was further neutralized by 25% ammonia (15 mL) and extracted with DCM (20 mL x 2). The combined organic layers were dried over anhydrous Na₂SO₄, filtered, and concentrated under reduced pressure to give a residue, which was further dissolved in ACN/water and freeze dried to afford the title compound (37.65 mg) as yellow solid.

**LC-MS (ESI) (Method 1):** Rt = 2.95 min, m/z found 556.3 [M+H]⁺.

**SFC (Method 4):** Rt = 1.772 min.
**Compound 5, 32, 33, 74, 81, 101, 211, 212, 222, 224, 231, 410**
**(*S*)-2-((5-(2-(6-(dimethylamino)-2-methylhexan-3-yl)-2,6-diazaspiro[3.4]octan-6-yl)-1,2,4-triazin-6-yl)oxy)-*N*-ethyl-5-fluoro-*N*-isopropylbenzamide formate**
**(**R*)-2-((5-(2-(6-(dimethylamino)-2,6-dimethylheptan-3-yl)-2,6-diazaspiro[3.4]octan-6-yl)-1,2,4-triazin-6-yl)oxy)-*N*-ethyl-5-fluoro-*N*-isopropylbenzamide**
**(**S*)-2-((5-(2-(6-(dimethylamino)-2,6-dimethylheptan-3-yl)-2,6-diazaspiro[3.4]octan-6-yl)-1,2,4-triazin-6-yl)oxy)-*N*-ethyl-5-fluoro-*N*-isopropylbenzamide**
**2-((4-(2-(6-(dimethylamino)-2-methylhexan-3-yl)-2,6-diazaspiro[3.4]octan-6-yl)pyridazin-3-yl)oxy)-5-fluoro-*N,N*-diisopropylbenzamide**
**2-((4-(2-(6-(dimethylamino)-2-methylhexan-3-yl)-2,6-diazaspiro[3.4]octan-6-yl)pyridazin-3-yl)oxy)-*N*-ethyl-5-fluoro-*N*-isopropylbenzamide**
**2-((5-(2-(6-(dimethylamino)-2-methylhexan-3-yl)-2,6-diazaspiro[3.4]octan-6-yl)-1,2,4-triazin-6-yl)oxy)-5-fluoro-*N*,*N*-diisopropylbenzamide**
**(**R*)-2-((5-(2-(7-(dimethylamino)-2-methylheptan-3-yl)-2,6-diazaspiro[3.4]octan-6-yl)-1,2,4-triazin-6-yl)oxy)-*N*-ethyl-5-fluoro-*N*-isopropylbenzamide**
**(**S*)-2-((5-(2-(7-(dimethylamino)-2-methylheptan-3-yl)-2,6-diazaspiro[3.4]octan-6-yl)-1,2,4-triazin-6-yl)oxy)-*N*-ethyl-5-fluoro-*N*-isopropylbenzamide**
**(**R*)-2-((5-(2-(1-(dimethylamino)-4-methylpentan-3-yl)-2,6-diazaspiro[3.4]octan-6-yl)-1,2,4-triazin-6-yl)oxy)-*N*-ethyl-5-fluoro-*N*-isopropylbenzamide**
**(**S*)-2-((5-(2-(1-(dimethylamino)-4-methylpentan-3-yl)-2,6-diazaspiro[3.4]octan-6-yl)-1,2,4-triazin-6-yl)oxy)-*N*-ethyl-5-fluoro-*N*-isopropylbenzamide**
**(*R)-2-((5-(2-(1-((2-amino-2-oxoethyl)(methyl)amino)-4-methylpentan-3-yl)-2,6-diazaspiro[3.4]octan-6-yl)-1,2,4-triazin-6-yl)oxy)-N-ethyl-5-fluoro-N-isopropylbenzamide**
**(*R*)-2-((5-(2-(6-(dimethylamino)-2-methylhexan-3-yl)-2,6-diazaspiro[3.4]octan-6-yl)-3-methoxy-1,2,4-triazin-6-yl)oxy)-*N*-ethyl-5-fluoro-*N*-isopropylbenzamide formate**

**The following Compounds were synthesized by an analogous method described above for Compound 4**

| **Co. No.** | **Structure** | **Starting Materials** | **Spectra Details** |
|---|---|---|---|
| 5 | 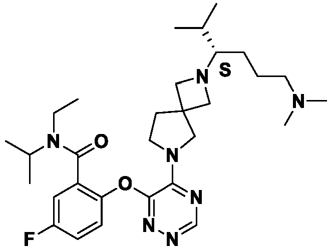 formate salt | Compound 66 | **LC-MS (Method 1):** Rt = 2.977 min, m/z found 556.4 [M+H]⁺. |
| | | | **SFC (Method 4):** Rt = 1.402 min. |
| 32 | 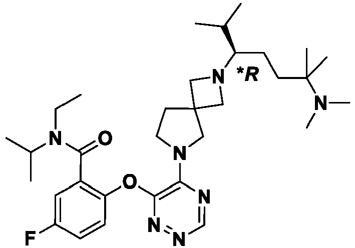 | Compound 30 | **LC-MS (ESI) (Method 2):** Rₜ = 2.043 min, m/z found 584.3 [M+H]⁺. |
| | | | **SFC (Method 2):** Rₜ = 4.431 min. |
| 33 | 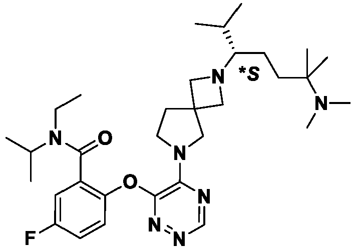 | Compound 31 | **LC-MS (ESI) (Method 2):** Rₜ = 2.008 min, *m*/*z* found 584.3 [M+H]⁺. |
| | | | **SFC (Method 2):** Rt = 4.997 min. |
| 74 | 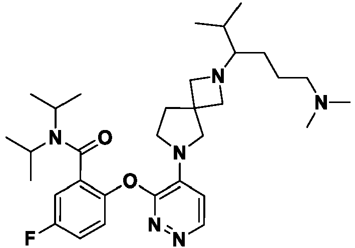 | Compound 73 | **LC-MS (ESI) (Method 2):** Rₜ = 1.933 min, m/z found 569.4 [M+H]⁺. |
| 81 | 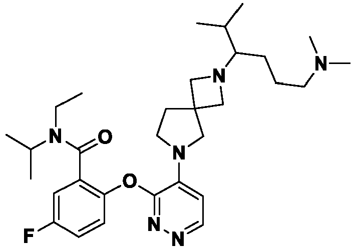 | Compound 80 | |
| 101 | 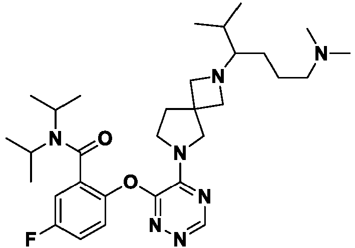 | Compound 97 | |
| 211 | 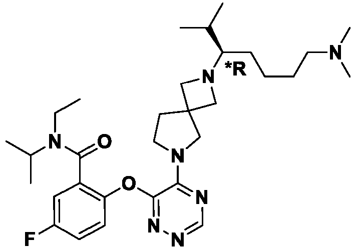 | Compound 209 | **LC-MS (ESI) (Method 2):** Rₜ = 1.946 min, m/z found 570.3 [M+H]⁺. |
| | | | **SFC (Method 8):** Rt = 2.243 **min.** |
| 212 | 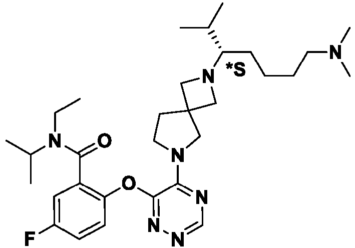 | Compound 210 | **LC-MS (ESI) (Method 1):** Rₜ = 3.021 min, m/z found 570.3 [M+H]⁺. |
| | | | **SFC (Method 8):** Rₜ = 2.431 min. |
| 222 | 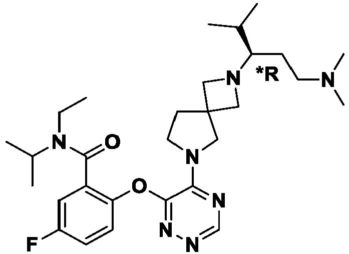 | Compound 220 | |
| 224 | 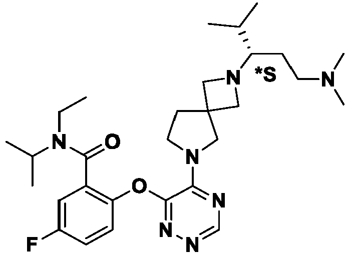 | Compound 221 | |
| 231 | 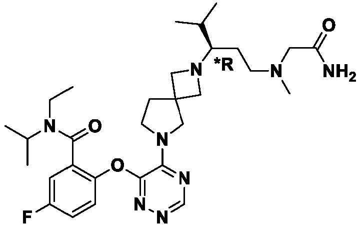 | Compound 230 | **LC-MS (ESI) (Method 1):** Rₜ = 2.858 min, m/z found 585.3 [M+H]⁺. |
| | | | **SFC (Method 6):** Rt = 1.454 min. |
| 410 | 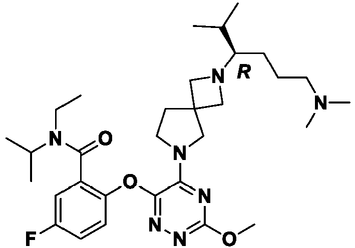 formate salt | Compound 407 | **LC-MS (ESI) (Method 2):** Rₜ = 2.066 min, m/z found 586.3 [M+H]⁺. |
| | | | **SFC (Method 14):** Rt = 2.582 min. |

### Compound 75, 76

### (*R)-2-((4-(2-(6-(dimethylamino)-2-methylhexan-3-yl)-2,6-diazaspiro[3.4]octan-6-yl)pyridazin-3-yl)oxy)-5-fluoro-N,N-diisopropylbenzamide

### (*S)-2-((4-(2-(6-(dimethylamino)-2-methylhexan-3-yl)-2,6-diazaspiro[3.4]octan-6-yl)pyridazin-3-yl)oxy)-5-fluoro-N,N-diisopropylbenzamide

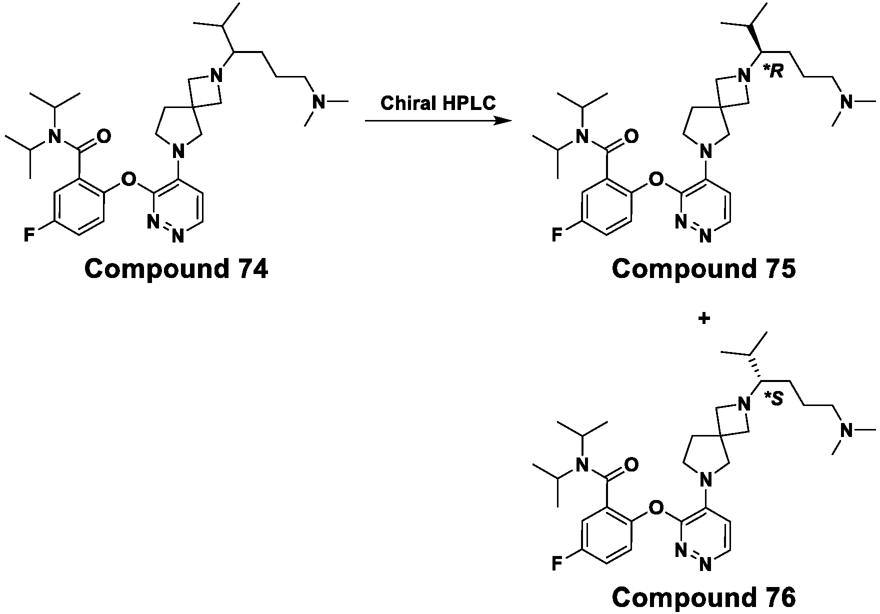

2-((4-(2-(6-(dimethylamino)-2-methylhexan-3-yl)-2,6-diazaspiro[3.4]octan-6-yl)pyridazin-3-yl)oxy)-5-fluoro-*N*,*N*-diisopropylbenzamide **(Compound 74)** (600 mg) was separated by chiral HPLC over DAICEL CHIRALPAK IG (column: 250x30mm 10um; Mobile phase: A: Heptane, B: EtOH, A:B from 20% to 70% (v/v); flowrate: 25 mL/min) to afford the title compounds **Compound 75** (92 mg, 15%) and **Compound 76** (84 mg) as white solid.

### Compound 75

**LC-MS (ESI) (Method 2):** Rₜ = 1.915 min, m/z found 569.3 [M+H]⁺.
**Chiral HPLC (Method 4):** Rt = 4.842 min.

### Compound 76

**LC-MS (ESI) (Method 2):** Rt = 1.924 min, m/z found 569.3 [M+H]⁺.
**Chiral HPLC (Method 4):** Rt = 6.200 min.

### Compound 77, 78

### (*R)-2-((4-(2-(6-(dimethylamino)-2-methylhexan-3-yl)-2,6-diazaspiro[3.4]octan-6-yl)pyridazin-3-yl)oxy)-N-ethyl-5-fluoro-N-isopropylbenzamide

### (*S)-2-((4-(2-(6-(dimethylamino)-2-methylhexan-3-yl)-2,6-diazaspiro[3.4]octan-6-yl)pyridazin-3-yl)oxy)-N-ethyl-5-fluoro-N-isopropylbenzamide

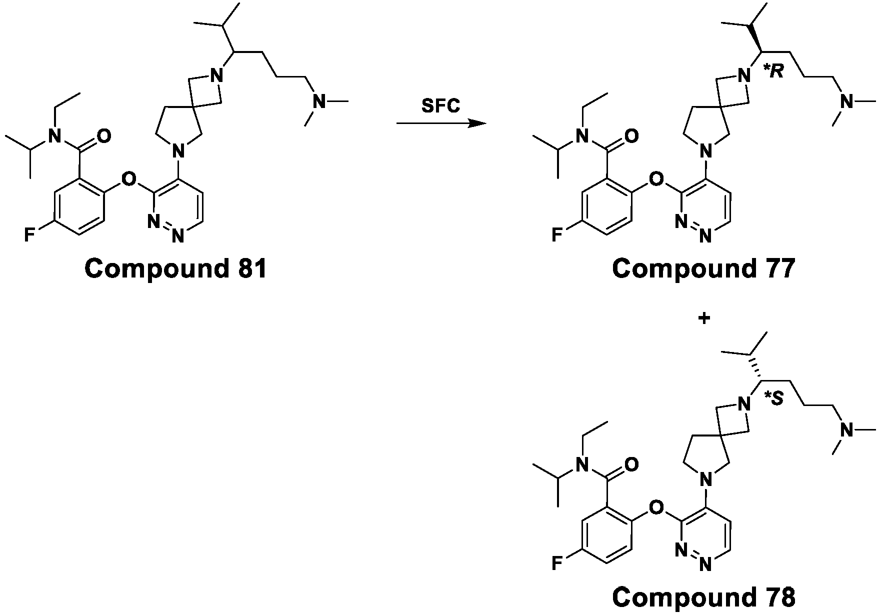

2-((4-(2-(6-(dimethylamino)-2-methylhexan-3-yl)-2,6-diazaspiro[3.4]octan-6-yl)pyridazin-3-yl)oxy)-*N*-ethyl-5-fluoro-*N*-isopropylbenzamide **(Compound 81)** (31.0 mg) was separated by SFC over DAICEL CHIRALPAK IE (column: 250x30mm 10um; eluent: 100% MeOH (0.1% ammonia); flowrate: 25 mL/min) to afford the title compounds **Compound 77** (4.2 mg) and **Compound 78** (1.3 mg) as white solid.

### Compound 77

**LC-MS (ESI) (Method 3):** Rₜ = 5.039 min, m/z found 555.3 [M+H]⁺.
**Chiral HPLC (Method 2):** Rt = 7.719 min.

### Compound 78

**LC-MS (ESI) (Method 3):** Rt = 4.870 min, m/z found 555.3 [M+H]⁺.
**Chiral HPLC (Method 2):** Rt = 8.754 min.

### Compound 105, 106

### (*R)-2-((5-(2-(6-(dimethylamino)-2-methylhexan-3-yl)-2,6-diazaspiro[3.4]octan-6-yl)-1,2,4-triazin-6-yl)oxy)-5-fluoro-N,N-diisopropylbenzamide

### (*S)-2-((5-(2-(6-(dimethylamino)-2-methylhexan-3-yl)-2,6-diazaspiro[3.4]octan-6-yl)-1,2,4-triazin-6-yl)oxy)-5-fluoro-N,N-diisopropylbenzamide

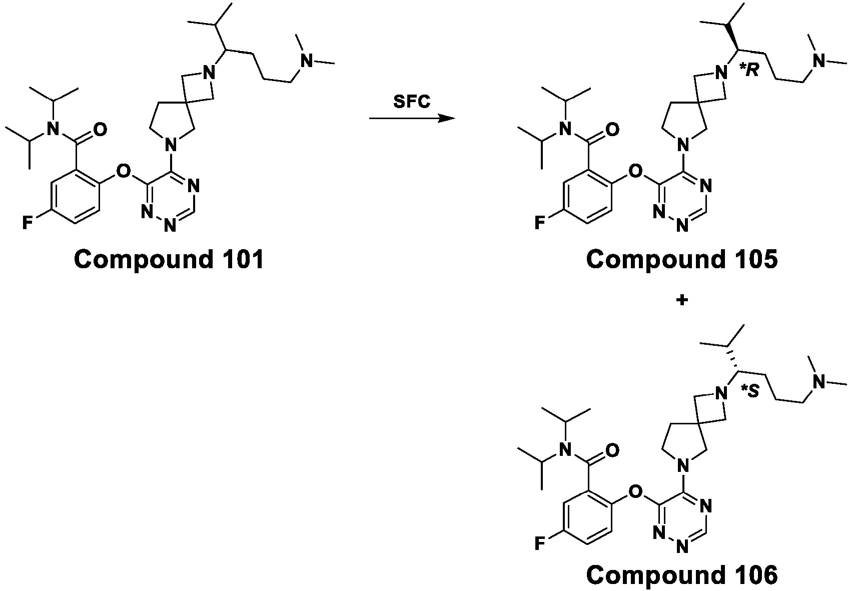

2-((5-(2-(6-(dimethylamino)-2-methylhexan-3-yl)-2,6-diazaspiro[3.4]octan-6-yl)-1,2,4-triazin-6-yl)oxy)-5-fluoro-*N,N*-diisopropylbenzamide **(Compound 101)** (1.5 g) was obtained by SFC over DAICEL CHIRALPAK IG (column: 250x50mm 10um; Mobile phase: A: Supercritical CO₂, B: MeOH (0.1% ammonia), A:B = 55:45 at 200 mL/min; Column Temp: 38 ; Nozzle Pressure: 100Bar; Nozzle Temp: 60 ; Evaporator Temp: 20 ; Trimmer Temp: 25 ; Wavelength: 220nm) to afford the title compounds **Compound 105** (600 mg, 40.0 % yield) and **Compound 106** (600 mg, 40.0 % yield) as white solid.

### Compound 102

### (*R)-2-((5-(2-(6-(dimethylamino)-2-methylhexan-3-yl)-2,6-diazaspiro[3.4]octan-6-yl)-1,2,4-triazin-6-yl)oxy)-5-fluoro-N,N-diisopropylbenzamide fumarate

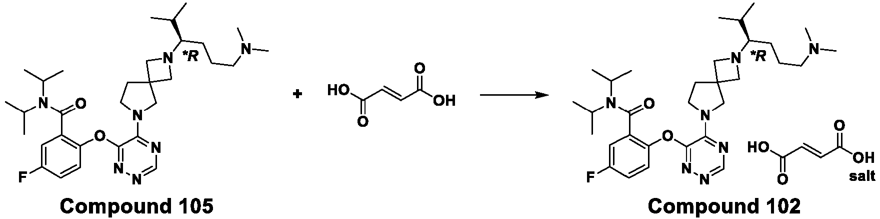

To a solution of (**R*)-2-((5-(2-(6-(dimethylamino)-2-methylhexan-3-yl)-2,6-diazaspiro[3.4]octan-6-yl)-1,2,4-triazin-6-yl)oxy)-5-fluoro-*N,N*-diisopropylbenzamide **(Compound 105)** (300 mg, 0.527 mmol) in ACN (12 mL) and water (4 mL) was added fumaric acid (123 mg, 1.06 mmol). After a clear solution was formed, the mixture was concentrated under reduced pressure, the resulting residue was added to a mixture of ACN (3 mL) and water (10 mL). The mixture was lyophilized to dryness to afford the title compound (422 mg) as a white solid.

**¹H NMR (400 MHz, Methanol-*d*₄):** δ = 8.50 (s, 1H), 7.50-7.15 (m, 3H), 6.72 (s, 4H), 4.51-3.89 (m, 7H), 3.86-3.69 (m, 2H), 3.61-3.49 (m, 1H), 3.25-3.07 (m, 3H), 2.88 (s, 6H), 2.50-2.20 (m, 2H), 2.19-2.06 (m, 1H), 1.97-1.77 (m, 2H), 1.75-1.57 (m, 2H), 1.51 (d, J=6.8 Hz, 3H), 1.37-1.14 (m, 6H), 1.11-0.97 (m, 6H), 0.78 (d, J=6.0 Hz, 3H).

**LC-MS (ESI) (Method 2):** Rt = 2.08 min, m/z found 570.3 [M+H]⁺.

**SFC (Method 4):** Rt = 1.284 min.
**Compound 103, 112, 114, 122, 123, 127, 128, 132, 133, 135, 137, 140, 142, 145, 146, 148, 150, 152, 154, 157, 159, 161, 165, 167, 170, 172, 176, 177, 179, 181, 184, 185, 188, 189, 191, 193, 195, 197, 199, 201, 203, 205, 219, 223, 225, 227, 233, 240, 241, 242, 243, 245, 256, 265, 266, 268, 270, 278, 280, 283, 259, 104, 229, 300, 302, 314, 315, 323, 324, 325, 326, 334, 335, 336, 337, 342, 343, 346, 352, 353, 356, 357, 365, 366, 369, 370, 377, 378, 382, 386, 387, 391, 392, 394, 397**
**(**S*)-2-((5-(2-(6-(dimethylamino)-2-methylhexan-3-yl)-2,6-diazaspiro[3.4]octan-6-yl)-1,2,4-triazin-6-yl)oxy)-5-fluoro-*N*,*N*-diisopropylbenzamide fumarate**
***N*-ethyl-5-fluoro-*N*-isopropyl-2-((5-(2-((*R*)-6-(((*R*)-1-methoxypropan-2-yl)amino)-2-methylhexan-3-yl)-2,6-diazaspiro[3.4]octan-6-yl)-1,2,4-triazin-6-yl)oxy)benzamide fumarate**
**(*R*)-2-((5-(2-(6-((3,3-difluoropropyl)amino)-2-methylhexan-3-yl)-2,6-diazaspiro[3.4]octan-6-yl)-1,2,4-triazin-6-yl)oxy)-*N*-ethyl-5-fluoro-*N-*isopropylbenzamide fumarate**
**(**R*)-*N*-ethyl-5-fluoro-*N*-isopropyl-2-((5-(2-(6-(isopropyl(methyl)amino)-2-methylhexan-3-yl)-2,6-diazaspiro[3.4]octan-6-yl)-1,2,4-triazin-6-yl)oxy)benzamide fumarate**
**(**S*)-*N*-ethyl-5-fluoro-*N*-isopropyl-2-((5-(2-(6-(isopropyl(methyl)amino)-2-methylhexan-3-yl)-2,6-diazaspiro[3.4]octan-6-yl)-1,2,4-triazin-6-yl)oxy)benzamide fumarate**
**(**R*)-*N*-ethyl-5-fluoro-*N*-isopropyl-2-((5-(2-(2-methyl-6-(methyl(propyl)amino)hexan-3-yl)-2,6-diazaspiro[3.4]octan-6-yl)-1,2,4-triazin-6-yl)oxy)benzamide fumarate**
**(**S*)-*N*-ethyl-5-fluoro-*N*-isopropyl-2-((5-(2-(2-methyl-6-(methyl(propyl)amino)hexan-3-yl)-2,6-diazaspiro[3.4]octan-6-yl)-1,2,4-triazin-6-yl)oxy)benzamide fumarate**
**(**R*)*-N*-ethyl-2-((5-(2-(6-(ethyl(methyl)amino)-2-methylhexan-3-yl)-2,6-diazaspiro[3.4]octan-6-yl)-1,2,4-triazin-6-yl)oxy)-5-fluoro-*N*-isopropylbenzamide fumarate**
**(**S*)-*N*-ethyl-2-((5-(2-(6-(ethyl(methyl)amino)-2-methylhexan-3-yl)-2,6-diazaspiro[3.4]octan-6-yl)-1,2,4-triazin-6-yl)oxy)-5-fluoro-*N*-isopropylbenzamide fumarate**
**(*R*)-*N*-ethyl-5-fluoro-*N*-isopropyl-2-((5-(2-(6-((2-methoxy-2-methylpropyl)amino)-2-methylhexan-3-yl)-2,6-diazaspiro[3.4]octan-6-yl)-1,2,4-triazin-6-yl)oxy)benzamide fumarate**
**(*R*)-*N*-ethyl-5-fluoro-*N*-isopropyl-2-((5-(2-(6-((2-methoxy-2-methylpropyl)(methyl)amino)-2-methylhexan-3-yl)-2,6-diazaspiro[3.4]octan-6-yl)-1,2,4-triazin-6-yl)oxy)benzamide fumarate**
**(R)-N-ethyl-5-fluoro-2-((5-(2-(6-((2-hydroxy-2-methylpropyl)(methyl)amino)-2-methylhexan-3-yl)-2,6-diazaspiro[3.4]octan-6-yl)-1,2,4-triazin-6-yl)oxy)-*N-*isopropylbenzamide fumarate**
**(*R*)-*N*-ethyl-5-fluoro-*N*-isopropyl-2-((5-(2-(6-((3-methoxypropyl)(methyl)amino)-2-methylhexan-3-yl)-2,6-diazaspiro[3.4]octan-6-yl)-1,2,4-triazin-6-yl)oxy)benzamide fumarate**
**(**R*)-2-((5-(2-(6-((3-(dimethylamino)-3-oxopropyl)(methyl)amino)-2-methylhexan-3-yl)-2,6-diazaspiro[3.4]octan-6-yl)-1,2,4-triazin-6-yl)oxy)-5-fluoro-*N*,*N*-diisopropylbenzamide fumarate**
**(**S*)-2-((5-(2-(6-((3-(dimethylamino)-3-oxopropyl)(methyl)amino)-2-methylhexan-3-yl)-2,6-diazaspiro[3.4]octan-6-yl)-1,2,4-triazin-6-yl)oxy)-5-fluoro-*N*,*N*-diisopropylbenzamide fumarate**
***(R)-N-ethyl-5-fluoro-N-isopropyl-2-((5-(2-(2-methyl-6-(methyl(2-(N-*methylacetamido)ethyl)amino)hexan-3-yl)-2,6-diazaspiro[3.4]octan-6-yl)-1,2,4-triazin-6-yl)oxy)benzamide fumarate**
**(*R*)-2-((5-(2-(6-((2,2-dimethoxyethyl)(methyl)amino)-2-methylhexan-3-yl)-2,6-diazaspiro[3.4]octan-6-yl)-1,2,4-triazin-6-yl)oxy)-N-ethyl-5-fluoro-N-isopropylbenzamide fumarate**
**(*R*)-2-((5-(2-(6-((4-(dimethylamino)-4-oxobutyl)(methyl)amino)-2-methylhexan-3-yl)-2,6-diazaspiro[3.4]octan-6-yl)-1,2,4-triazin-6-yl)oxy)-N-ethyl-5-fluoro-N-isopropylbenzamide fumarate**
**N-ethyl-5-fluoro-N-isopropyl-2-((5-(2-((R)-6-(((R)-1-methoxypropan-2-yl)(methyl)amino)-2-methylhexan-3-yl)-2,6-diazaspiro[3.4]octan-6-yl)-1,2,4-triazin-6-yl)oxy)benzamide fumarate**
***N*-ethyl-5-fluoro-*N*-isopropyl-2-((5-(2-((R)-6-(((*S*)-1-methoxypropan-2-yl)(methyl)amino)-2-methylhexan-3-yl)-2,6-diazaspiro[3.4]octan-6-yl)-1,2,4-triazin-6-yl)oxy)benzamide fumarate**
**(*R*)-2-((5-(2-(6-((1,3-dimethoxypropan-2-yl)amino)-2-methylhexan-3-yl)-2,6-diazaspiro[3.4]octan-6-yl)-1,2,4-triazin-6-yl)oxy)-*N*-ethyl-5-fluoro-*N-*isopropylbenzamide fumarate**
**(*R*)-2-((5-(2-(6-((1,3-dimethoxypropan-2-yl)(methyl)amino)-2-methylhexan-3-yl)-2,6-diazaspiro[3.4]octan-6-yl)-1,2,4-triazin-6-yl)oxy)-*N*-ethyl-5-fluoro-*N-*isopropylbenzamide fumarate**
**N-ethyl-5-fluoro-2-((5-(2-((R)-6-(((R)-1-hydroxy-3-methoxypropan-2-yl)(methyl)amino)-2-methylhexan-3-yl)-2,6-diazaspiro[3.4]octan-6-yl)-1,2,4-triazin-6-yl)oxy)-*N-*isopropylbenzamide fumarate**
***N*-ethyl-5-fluoro-2-((5-(2-((*R*)-6-(((*S*)-1-hydroxy-3-methoxypropan-2-yl)(methyl)amino)-2-methylhexan-3-yl)-2,6-diazaspiro[3.4]octan-6-yl)-1,2,4-triazin-6-yl)oxy)-*N-*isopropylbenzamide fumarate**
**N-ethyl-5-fluoro-2-((5-(2-((3R)-6-((3-hydroxy-2-methoxypropyl)(methyl)amino)-2-methylhexan-3-yl)-2,6-diazaspiro[3.4]octan-6-yl)-1,2,4-triazin-6-yl)oxy)-*N-*isopropylbenzamide fumarate**
**2-((5-(2-((3*R*)-6-((2,3-dimethoxypropyl)amino)-2-methylhexan-3-yl)-2,6-diazaspiro[3.4]octan-6-yl)-1,2,4-triazin-6-yl)oxy)-N-ethyl-5-fluoro-N-isopropylbenzamide fumarate**
**2-((5-(2-((*R*)-6-(((**R*)-2,3-dimethoxypropyl)(methyl)amino)-2-methylhexan-3-yl)-2,6-diazaspiro[3.4]octan-6-yl)-1,2,4-triazin-6-yl)oxy)-*N*-ethyl-5-fluoro-*N-*isopropylbenzamide fumarate**
**2-((5-(2-((*R*)-6-(((**S*)-2,3-dimethoxypropyl)(methyl)amino)-2-methylhexan-3-yl)-2,6-diazaspiro[3.4]octan-6-yl)-1,2,4-triazin-6-yl)oxy)-*N*-ethyl-5-fluoro-*N-*isopropylbenzamide fumarate**
**2-((5-(2-((3*R*)-6-((4-(dimethylamino)-4-oxobutan-2-yl)(methyl)amino)-2-methylhexan-3-yl)-2,6-diazaspiro[3.4]octan-6-yl)-1,2,4-triazin-6-yl)oxy)-N-ethyl-5-fluoro-N-isopropylbenzamide fumarate**
**2-((5-(2-((3R)-6-((3-(dimethylamino)-2-methyl-3-oxopropyl)(methyl)amino)-2-methylhexan-3-yl)-2,6-diazaspiro[3.4]octan-6-yl)-1,2,4-triazin-6-yl)oxy)-*N*-ethyl-5-fluoro-N-isopropylbenzamide fumarate**
***2-((5-(2-((R)-6-(((*R)-4-(dimethylamino)-4-oxobutan-2-yl)(methyl)amino)-2-*methylhexan-3-yl)-2,6-diazaspiro[3.4]octan-6-yl)-1,2,4-triazin-6-yl)oxy)-*N*-ethyl-5-fluoro-*N*-isopropylbenzamide fumarate**
**2-((5-(2-((*R*)-6-(((**S*)-4-(dimethylamino)-4-oxobutan-2-yl)(methyl)amino)-2-methylhexan-3-yl)-2,6-diazaspiro[3.4]octan-6-yl)-1,2,4-triazin-6-yl)oxy)-*N*-ethyl-5-fluoro-*N*-isopropylbenzamide fumarate**
***2-((5-(2-((R)-6-(((*R)-3-(dimethylamino)-2-methyl-3-oxopropyl)(methyl)amino)-2-*methylhexan-3-yl)-2,6-diazaspiro[3.4]octan-6-yl)-1,2,4-triazin-6-yl)oxy)-*N*-ethyl-5-fluoro-N-isopropylbenzamide fumarate**
***2-((5-(2-((R)-6-(((*S)-3-(dimethylamino)-2-methyl-3-oxopropyl)(methyl)amino)-2-*methylhexan-3-yl)-2,6-diazaspiro[3.4]octan-6-yl)-1,2,4-triazin-6-yl)oxy)-*N*-ethyl-5-fluoro-N-isopropylbenzamide fumarate**
**N-ethyl-5-fluoro-N-isopropyl-2-((5-(2-((R)-2-methyl-6-(methyl((R)-4-(methylamino)-4-oxobutan-2-yl)amino)hexan-3-yl)-2,6-diazaspiro[3.4]octan-6-yl)-1,2,4-triazin-6-yl)oxy)benzamide fumarate**
**N-ethyl-5-fluoro-N-isopropyl-2-((5-(2-((R)-2-methyl-6-(methyl((S)-4-(methylamino)-4-oxobutan-2-yl)amino)hexan-3-yl)-2,6-diazaspiro[3.4]octan-6-yl)-1,2,4-triazin-6-yl)oxy)benzamide fumarate**
**N-ethyl-5-fluoro-N-isopropyl-2-((5-(2-((R)-2-methyl-6-(methyl((R)-2-methyl-3-(methylamino)-3-oxopropyl)amino)hexan-3-yl)-2,6-diazaspiro[3.4]octan-6-yl)-1,2,4-triazin-6-yl)oxy)benzamide fumarate**
**N-ethyl-5-fluoro-N-isopropyl-2-((5-(2-((R)-2-methyl-6-(methyl((S)-2-methyl-3-(methylamino)-3-oxopropyl)amino)hexan-3-yl)-2,6-diazaspiro[3.4]octan-6-yl)-1,2,4-triazin-6-yl)oxy)benzamide fumarate**
**2-((5-(2-((**R*)-6-(((*R*)-4-amino-4-oxobutan-2-yl)(methyl)amino)-2-methylhexan-3-yl)-2,6-diazaspiro[3.4]octan-6-yl)-1,2,4-triazin-6-yl)oxy)-5-fluoro-*N,N*-diisopropylbenzamide fumarate**
**2-((5-(2-((**R*)-6-(((S)-4-amino-4-oxobutan-2-yl)(methyl)amino)-2-methylhexan-3-yl)-2,6-diazaspiro[3.4]octan-6-yl)-1,2,4-triazin-6-yl)oxy)-5-fluoro-*N,N*-diisopropylbenzamide fumarate**
**2-((5-(2-((**R*)-6-(((*R*)-3-amino-2-methyl-3-oxopropyl)(methyl)amino)-2-methylhexan-3-yl)-2,6-diazaspiro[3.4]octan-6-yl)-1,2,4-triazin-6-yl)oxy)-5-fluoro-*N,N-*diisopropylbenzamide fumarate**
**2-((5-(2-((**R*)-6-(((S)-3-amino-2-methyl-3-oxopropyl)(methyl)amino)-2-methylhexan-3-yl)-2,6-diazaspiro[3.4]octan-6-yl)-1,2,4-triazin-6-yl)oxy)-5-fluoro-*N,N-*diisopropylbenzamide fumarate**
**(*R)-2-((5-(2-(1-amino-4-methylpentan-3-yl)-2,6-diazaspiro[3.4]octan-6-yl)-1,2,4-triazin-6-yl)oxy)-*N*-ethyl-5-fluoro-*N*-isopropylbenzamide fumarate**
**(**R*)-2-((5-(2-(1-(dimethylamino)-4-methylpentan-3-yl)-2,6-diazaspiro[3.4]octan-6-yl)-1,2,4-triazin-6-yl)oxy)-*N*-ethyl-5-fluoro-*N*-isopropylbenzamide fumarate**
**(*S)-2-((5-(2-(1-(dimethylamino)-4-methylpentan-3-yl)-2,6-diazaspiro[3.4]octan-6-yl)-1,2,4-triazin-6-yl)oxy)-*N*-ethyl-5-fluoro-*N*-isopropylbenzamide fumarate**
**(**R*)-*N*-ethyl-5-fluoro-*N*-isopropyl-2-((5-(2-(1-((2-methoxyethyl)amino)-4-methylpentan-3-yl)-2,6-diazaspiro[3.4]octan-6-yl)-1,2,4-triazin-6-yl)oxy)benzamide fumarate**
**(*R*)-*N*-ethyl-5-fluoro-*N*-isopropyl-2-((5-(2-(6-((2-methoxyethyl-1,1-*d*₂)(methyl)amino)-2-methylhexan-3-yl)-2,6-diazaspiro[3.4]octan-6-yl)-1,2,4-triazin-6-yl)oxy)benzamide fumarate**
**N-ethyl-5-fluoro-N-isopropyl-2-((5-(2-((3 *R,5 *R)-6-((2-methoxyethyl)(methyl)amino)-2,5-dimethylhexan-3-yl)-2,6-diazaspiro[3.4]octan-6-yl)-1,2,4-triazin-6-yl)oxy)benzamide fumarate**
**N-ethyl-5-fluoro-N-isopropyl-2-((5-(2-((3 *S,5 *R)-6-((2-methoxyethyl)(methyl)amino)-2,5-dimethylhexan-3-yl)-2,6-diazaspiro[3.4]octan-6-yl)-1,2,4-triazin-6-yl)oxy)benzamide fumarate**
***N*-ethyl-5-fluoro-*N*-isopropyl-2-((5-(2-((3**R*,5**S*)-6-((2-methoxyethyl)(methyl)amino)-2,5-dimethylhexan-3-yl)-2,6-diazaspiro[3.4]octan-6-yl)-1,2,4-triazin-6-yl)oxy)benzamide fumarate**
**N-ethyl-5-fluoro-N-isopropyl-2-((5-(2-((3 *S,5 *S)-6-((2-methoxyethyl)(methyl)amino)-2,5-dimethylhexan-3-yl)-2,6-diazaspiro[3.4]octan-6-yl)-1,2,4-triazin-6-yl)oxy)benzamide fumarate**
**(*R*)-2-((5-(2-(6-((2-acetamidoethyl)(methyl)amino)-2-methylhexan-3-yl)-2,6-diazaspiro[3.4]octan-6-yl)-1,2,4-triazin-6-yl)oxy)-N-ethyl-5-fluoro-N-isopropylbenzamide fumarate**
**(*R*)-2-((5-(2-(6-((1,3-dihydroxypropan-2-yl)(methyl)amino)-2-methylhexan-3-yl)-2,6-diazaspiro[3.4]octan-6-yl)-1,2,4-triazin-6-yl)oxy)-N-ethyl-5-fluoro-N-isopropylbenzamide fumarate**
**N-ethyl-5-fluoro-N-isopropyl-2-((5-(2-(6-((2-methoxyethyl)(methyl)amino)-2,4-dimethylhexan-3-yl)-2,6-diazaspiro[3.4]octan-6-yl)-1,2,4-triazin-6-yl)oxy)benzamide (mixture of *R,S* and *S,R;* or mixture of *R,R* and S,S) fumarate**
**N-ethyl-5-fluoro-N-isopropyl-2-((5-(2-(6-((2-methoxyethyl)(methyl)amino)-2,4-dimethylhexan-3-yl)-2,6-diazaspiro[3.4]octan-6-yl)-1,2,4-triazin-6-yl)oxy)benzamide (mixture of *R,R* and *S,S;* or mixture of *R,S* and *S,R)* fumarate**
***(*R)-N-ethyl-5-fluoro-2-((5-(2-(1-((2-hydroxyethyl)amino)-4-methylpentan-3-yl)-2,6-*diazaspiro[3.4]octan-6-yl)-1,2,4-triazin-6-yl)oxy)-*N*-isopropylbenzamide fumarate**
***(*R)-N-ethyl-5-fluoro-2-((5-(2-(1-((2-hydroxyethyl)(methyl)amino)-4-methylpentan-3-*yl)-2,6-diazaspiro[3.4]octan-6-yl)-1,2,4-triazin-6-yl)oxy)-*N*-isopropylbenzamide fumarate**
**(*R)-2-((5-(2-(1-((3-amino-3-oxopropyl)amino)-4-methylpentan-3-yl)-2,6-diazaspiro[3.4]octan-6-yl)-1,2,4-triazin-6-yl)oxy)-N-ethyl-5-fluoro-N-isopropylbenzamide fumarate**
**(*R)-2-((5-(2-(1-((3-amino-3-oxopropyl)(methyl)amino)-4-methylpentan-3-yl)-2,6-diazaspiro[3.4]octan-6-yl)-1,2,4-triazin-6-yl)oxy)-N-ethyl-5-fluoro-N-isopropylbenzamide fumarate**
**N-ethyl-5-fluoro-2-((5-(2-((R)-6-(((R)-2-hydroxy-3-methoxypropyl)(methyl)amino)-2-methylhexan-3-yl)-2,6-diazaspiro[3.4]octan-6-yl)-1,2,4-triazin-6-yl)oxy)-*N-*isopropylbenzamide fumarate**
**(*R*)*-N*-ethyl-5-fluoro-2-((5-(2-(6-((2-hydroxyethyl)(methyl)amino)-2-methylhexan-3-yl)-2,6-diazaspiro[3.4]octan-6-yl)-1,2,4-triazin-6-yl)oxy)-*N*-isopropylbenzamide fumarate**
**(*R*)-2-((5-(2-(6-((2,2-dimethoxyethyl)amino)-2-methylhexan-3-yl)-2,6-diazaspiro[3.4]octan-6-yl)-1,2,4-triazin-6-yl)oxy)-N-ethyl-5-fluoro-N-isopropylbenzamide fumarate**
***(*R)-N-ethyl-5-fluoro-N-isopropyl-2-((5-(2-(1-(isopropylamino)-4-methylpentan-3-yl)-*2,6-diazaspiro[3.4]octan-6-yl)-1,2,4-triazin-6-yl)oxy)benzamide fumarate**
**N-ethyl-5-fluoro-N-isopropyl-2-((5-(2-((3R)-6-((2-methoxyethyl)(methyl)amino)-2-methylheptan-3-yl)-2,6-diazaspiro[3.4]octan-6-yl)-1,2,4-triazin-6-yl)oxy)benzamide fumarate**
**N-ethyl-5-fluoro-N-isopropyl-2-((5-(6-(6-((2-methoxyethyl)(methyl)amino)-2-methylhexan-3-yl)-2,6-diazaspiro[3.4]octan-2-yl)-1,2,4-triazin-6-yl)oxy)benzamide fumarate**
**2-((5-(2-((3**R,5***R*)-6-(dimethylamino)-5-methoxy-2-methylhexan-3-yl)-2,6-diazaspiro[3.4]octan-6-yl)-1,2,4-triazin-6-yl)oxy)-N-ethyl-5-fluoro-N-isopropylbenzamide fumarate**
**2-((5-(2-((3**R,*5**S*)-6-(dimethylamino)-5-methoxy-2-methylhexan-3-yl)-2,6-diazaspiro[3.4]octan-6-yl)-1,2,4-triazin-6-yl)oxy)-N-ethyl-5-fluoro-N-isopropylbenzamide fumarate**
**N-ethyl-5-fluoro-2-((5-(2-((3 **R,5* *R)-5-hydroxy-6-(isopropyl(methyl)amino)-2-methylhexan-3-yl)-2,6-diazaspiro[3.4]octan-6-yl)-1,2,4-triazin-6-yl)oxy)-*N-*isopropylbenzamide fumarate**
***N-ethyl-5-fluoro-2-((5-(2-((3*S,5*S)-5-hydroxy-6-(isopropyl(methyl)amino)-2-*methylhexan-3-yl)-2,6-diazaspiro[3.4]octan-6-yl)-1,2,4-triazin-6-yl)oxy)-*N-*isopropylbenzamide fumarate**
**N-ethyl-5-fluoro-2-((5-(2-((3 **R,5* *S)-5-hydroxy-6-(isopropyl(methyl)amino)-2-methylhexan-3-yl)-2,6-diazaspiro[3.4]octan-6-yl)-1,2,4-triazin-6-yl)oxy)-*N-*isopropylbenzamide fumarate**
**N-ethyl-5-fluoro-2-((5-(2-((3 **S,5* *R)-5-hydroxy-6-(isopropyl(methyl)amino)-2-methylhexan-3-yl)-2,6-diazaspiro[3.4]octan-6-yl)-1,2,4-triazin-6-yl)oxy)-*N-*isopropylbenzamide fumarate**
**2-((5-(2-((3**R,*5**R*)-6-(diethylamino)-5-hydroxy-2-methylhexan-3-yl)-2,6-diazaspiro[3.4]octan-6-yl)-1,2,4-triazin-6-yl)oxy)-*N*-ethyl-5-fluoro-*N-*isopropylbenzamide fumarate**
**2-((5-(2-((3**S,*5**S*)-6-(diethylamino)-5-hydroxy-2-methylhexan-3-yl)-2,6-diazaspiro[3.4]octan-6-yl)-1,2,4-triazin-6-yl)oxy)-*N*-ethyl-5-fluoro-*N-*isopropylbenzamide fumarate**
**2-((5-(2-((3**S,*5**R*)-6-(diethylamino)-5-hydroxy-2-methylhexan-3-yl)-2,6-diazaspiro[3.4]octan-6-yl)-1,2,4-triazin-6-yl)oxy)-*N*-ethyl-5-fluoro-*N-*isopropylbenzamide fumarate**
**2-((5-(2-((3 **R,*5**S*)-6-(diethylamino)-5-hydroxy-2-methylhexan-3-yl)-2,6-diazaspiro[3.4]octan-6-yl)-1,2,4-triazin-6-yl)oxy)-*N*-ethyl-5-fluoro-*N-*isopropylbenzamide fumarate**
***N*-ethyl-2-((5-(2-((3**R*,5*S*)-6-(ethyl(methyl)amino)-5-hydroxy-2-methylhexan-3-yl)-2,6-diazaspiro[3.4]octan-6-yl)-1,2,4-triazin-6-yl)oxy)-5-fluoro-*N*-isopropylbenzamide fumarate**
***N*-ethyl-2-((5-(2-((3**S*,5*S*)-6-(ethyl(methyl)amino)-5-hydroxy-2-methylhexan-3-yl)-2,6-diazaspiro[3.4]octan-6-yl)-1,2,4-triazin-6-yl)oxy)-5-fluoro-*N*-isopropylbenzamide fumarate**
***N*-ethyl-2-((5-(2-((3**R*,5*R*)-6-(ethyl(methyl)amino)-5-hydroxy-2-methylhexan-3-yl)-2,6-diazaspiro[3.4]octan-6-yl)-1,2,4-triazin-6-yl)oxy)-5-fluoro-*N*-isopropylbenzamide fumarate**
***N*-ethyl-5-fluoro-2-((5-(2-((3**R*,5*S*)-5-hydroxy-6-((2-methoxyethyl)(methyl)amino)-2-methylhexan-3-yl)-2,6-diazaspiro[3.4]octan-6-yl)-1,2,4-triazin-6-yl)oxy)-*N-*isopropylbenzamide fumarate**
***N*-ethyl-5-fluoro-2-((5-(2-((3**S*,5*S*)-5-hydroxy-6-((2-methoxyethyl)(methyl)amino)-2-methylhexan-3-yl)-2,6-diazaspiro[3.4]octan-6-yl)-1,2,4-triazin-6-yl)oxy)-*N-*isopropylbenzamide fumarate**
***N*-ethyl-5-fluoro-2-((5-(2-((3**R*,5*R*)-5-hydroxy-6-((2-methoxyethyl)(methyl)amino)-2-methylhexan-3-yl)-2,6-diazaspiro[3.4]octan-6-yl)-1,2,4-triazin-6-yl)oxy)-*N-*isopropylbenzamide fumarate**
***N*-ethyl-5-fluoro-2-((5-(2-((3**S*,5*R*)-5-hydroxy-6-((2-methoxyethyl)(methyl)amino)-2-methylhexan-3-yl)-2,6-diazaspiro[3.4]octan-6-yl)-1,2,4-triazin-6-yl)oxy)-*N-*isopropylbenzamide fumarate**
**2-((5-(2-((3 **R,*5**R*)-6-(dimethylamino)-5-hydroxy-2-methylhexan-3-yl)-2,6-diazaspiro[3.4]octan-6-yl)-1,2,4-triazin-6-yl)oxy)-5-fluoro-*N,N*-diisopropylbenzamide fumarate**
**2-((5-(2-((3**R,*5**S*)-6-(dimethylamino)-5-hydroxy-2-methylhexan-3-yl)-2,6-diazaspiro[3.4]octan-6-yl)-1,2,4-triazin-6-yl)oxy)-5-fluoro-*N,N*-diisopropylbenzamide fumarate**
**2-((5-(2-((3**S,*5**S*)-6-(dimethylamino)-5-hydroxy-2-methylhexan-3-yl)-2,6-diazaspiro[3.4]octan-6-yl)-1,2,4-triazin-6-yl)oxy)-5-fluoro-*N,N*-diisopropylbenzamide fumarate**
**2-((5-(2-((3**S,*5**R*)-6-(dimethylamino)-5-hydroxy-2-methylhexan-3-yl)-2,6-diazaspiro[3.4]octan-6-yl)-1,2,4-triazin-6-yl)oxy)-5-fluoro-*N,N*-diisopropylbenzamide fumarate**
**2-((5-(2-((3**S*,5**S*)-6-(dimethylamino)-5-methoxy-2-methylhexan-3-yl)-2,6-diazaspiro[3.4]octan-6-yl)-1,2,4-triazin-6-yl)oxy)-N-ethyl-5-fluoro-N-isopropylbenzamide fumarate**
**2-((5-(2-((3**S*,5**R*)-6-(dimethylamino)-5-methoxy-2-methylhexan-3-yl)-2,6-diazaspiro[3.4]octan-6-yl)-1,2,4-triazin-6-yl)oxy)-N-ethyl-5-fluoro-N-isopropylbenzamide fumarate**
**N-ethyl-5-fluoro-2-((5-(2-(5-hydroxy-6-((2-methoxyethyl)(methyl)amino)-2,5-dimethylhexan-3-yl)-2,6-diazaspiro[3.4]octan-6-yl)-1,2,4-triazin-6-yl)oxy)-*N-*isopropylbenzamide fumarate**
**N-ethyl-5-fluoro-2-((5-(2-((3 **R,5* *R)-5-hydroxy-2-methyl-6-(methyl(propyl)amino)hexan-3-yl)-2,6-diazaspiro[3.4]octan-6-yl)-1,2,4-triazin-6-yl)oxy)-N-isopropylbenzamide fumarate**
**N-ethyl-5-fluoro-2-((5-(2-((3 *S,5 *S)-5-hydroxy-2-methyl-6-(methyl(propyl)amino)hexan-3-yl)-2,6-diazaspiro[3.4]octan-6-yl)-1,2,4-triazin-6-yl)oxy)-N-isopropylbenzamide fumarate**
**2-((5-(2-((3**R,*5**S*)-6-(ethyl(methyl)amino)-5-hydroxy-2-methylhexan-3-yl)-2,6-diazaspiro[3.4]octan-6-yl)-1,2,4-triazin-6-yl)oxy)-5-fluoro-*N,N*-diisopropylbenzamide fumarate**
**2-((5-(2-((3**S*,5**S*)-6-(ethyl(methyl)amino)-5-hydroxy-2-methylhexan-3-yl)-2,6-diazaspiro[3.4]octan-6-yl)-1,2,4-triazin-6-yl)oxy)-5-fluoro-*N,N*-diisopropylbenzamide fumarate**
**(*R*)-2-((3-chloro-5-(2-(6-((2-methoxyethyl)(methyl)amino)-2-methylhexan-3-yl)-2,6-diazaspiro[3.4]octan-6-yl)-1,2,4-triazin-6-yl)oxy)-N-ethyl-5-fluoro-N-isopropylbenzamide fumarate**
**(*R*)*-N*-ethyl-5-fluoro-*N*-isopropyl-2-((5-(2-(6-((2-methoxyethyl)amino)-2-methylhexan-3-yl)-2,6-diazaspiro[3.4]octan-6-yl)-1,2,4-triazin-6-yl)oxy)benzamide fumarate**

**The following Compounds were synthesized by an analogous method described above for Compound 102**

| **Co. No.** | **Structure** | **Starting Materials** | **Spectra Details** |
|---|---|---|---|
| 103 | 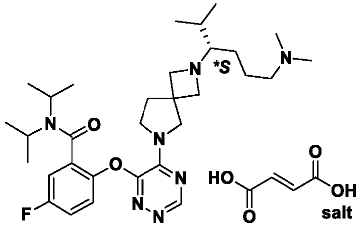 | Compound 106, fumaric acid | **LC-MS (ESI) (Method 2):** Rₜ = 2.041 min, m/z found 570.3 [M+H]⁺. |
| | | | **SFC (Method 13):** Rₜ = 1.722 min. |
| 112 | 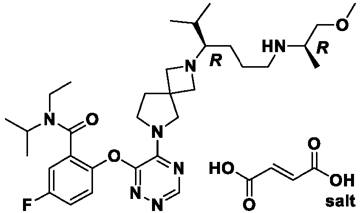 | Compound 111, fumaric acid | **¹H NMR (400 MHz, Methanol-*d*₄):** δ = 8.49 (s, 1H), 7.45-7.22 (m, 3H), 6.71 (s, 4H), 4.20-3.63 (m, 9H), 3.51-3.40 (m, 6H), 3.31-2.95 (m, 5H), 2.47-2.23 (m, 2H), 2.19-1.98 (m, 1H), 1.94-1.54 (m, 4H), 1.35 (d, *J*=5.6 Hz, 3H), 1.19-0.98 (m, 13H), 0.89-0.73 (m, 2H). |
| | | | **LC-MS (ESI) (Method 1):** Rₜ = 3.063 min, m/z found 600.5 [M+H]⁺. |
| | | | **SFC (Method 6):** Rₜ = 1.214 min. |
| 114 | 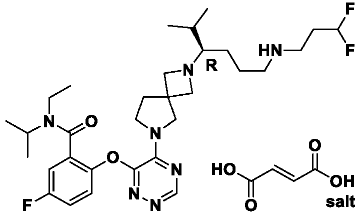 | Compound 113, fumaric acid | **¹H NMR (400 MHz, Methanol-*d*₄): δ** = 8.47 (s, 1H), 7.52-7.07 (m, 3H), 6.69 (s, 4H), 6.30-5.90 (m, 1H), 4.50-3.39 (m, 10H), 3.25-2.83 (m, 6H), 2.43-1.99 (m, 5H), 1.90-1.49 (m, 4H), 1.23-0.71 (m, 15H). |
| | | | **LC-MS (ESI) (Method 1):** Rt = 3.056 min, m/z found 606.3 [M+H]⁺. |
| | | | **SFC** (Method 13): Rₜ = 1.944 min. |
| 122 | 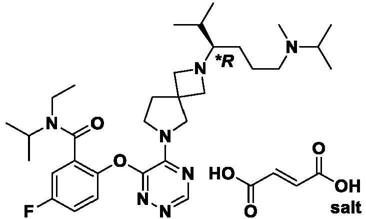 | Compound 120, fumaric acid | **LC-MS (ESI) (Method 2):** Rₜ = 2.030 min, m/z found 584.3 [M+H]⁺. |
| | | | **SFC (Method 18):** Rₜ = 2.312 min. |
| 123 | 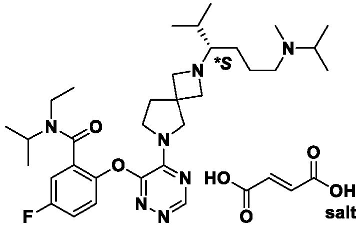 | Compound 121, fumaric acid | **LC-MS (ESI) (Method 2):** Rₜ = 2.020 min, m/z found 584.3 [M+H]⁺. |
| | | | **SFC (Method 18):** Rₜ = 2.557 min. |
| 127 | 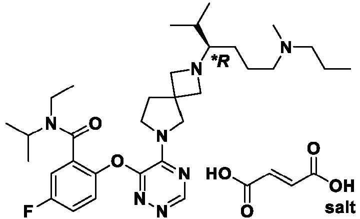 | Compound 125, fumaric acid | **LC-MS (ESI) (Method 2):** Rₜ = 2.065 min, m/z found 584.3 [M+H]⁺. |
| | | | **Chiral HPLC (Method 7):** Rₜ = 3.197 min. |
| 128 | 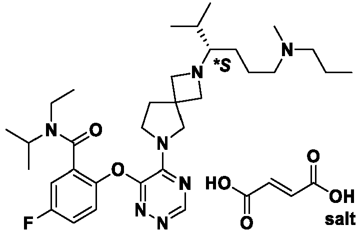 | Compound 126, fumaric acid | **LC-MS (ESI) (Method 2):** Rₜ = 2.074 min, m/z found 584.3 [M+H]⁺. |
| | | | **Chiral HPLC (Method 7):** Rₜ = 3.805 min. |
| 132 | 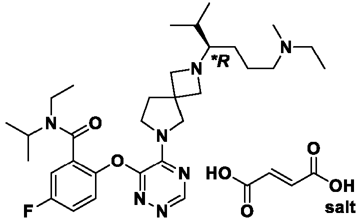 | Compound 130, fumaric acid | **LC-MS (ESI) (Method 2):** Rₜ = 1.954 min, m/z found 570.3 [M+H]⁺. |
| | | | **Chiral HPLC (Method 7):** Rₜ = **3.702 min.** |
| 133 | 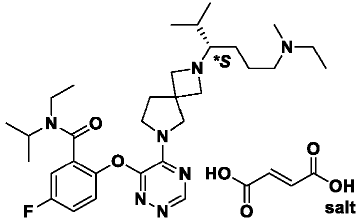 | Compound 131, fumaric acid | **LC-MS (ESI) (Method 2):** Rₜ = 1.955 min, m/z found 570.3 [M+H]⁺. |
| | | | **Chiral HPLC (Method 7):** Rₜ = 4.808 min. |
| 135 | 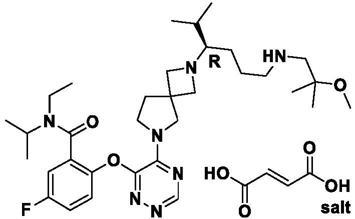 | Compound 134, fumaric acid | **LC-MS (ESI) (Method 2):** Rₜ = 2.083 min, m/z found 614.4 [M+H]⁺. |
| | | | **SFC (Method 6):** Rₜ = 1.346 min. |
| 137 | 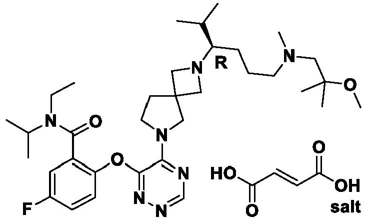 | Compound 136, fumaric acid | **LC-MS (ESI) (Method 2):** Rₜ = 2.115 min, m/z found 628.4 [M+H]⁺. |
| | | | **SFC (Method 6):** Rₜ = 0.938 min. |
| 140 | 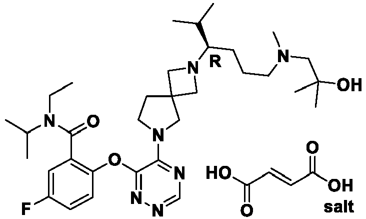 | Compound 139, fumaric acid | **LC-MS (ESI) (Method 2):** Rₜ = 1.986 min, m/z found 614.4 [M+H]⁺. |
| | | | **SFC (Method 13):** Rₜ = 1.749 min. |
| 142 | 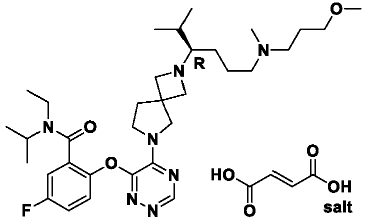 | Compound 141, fumaric acid | **LC-MS (ESI) (Method 2):** Rₜ = 2.039 min, m/z found 614.4 [M+H]⁺. |
| | | | **SFC (Method 6):** Rₜ = 1.171 min. |
| 145 | 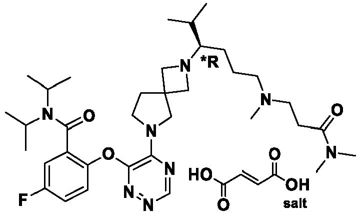 | Compound 143, fumaric acid | **LC-MS (ESI) (Method 2):** Rₜ = 2.096 min, m/z found 655.6 [M+H]⁺. |
| | | | **SFC (Method 19):** Rₜ = 3.861 min. |
| 146 | 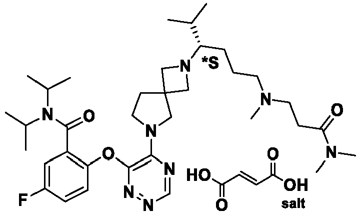 | Compound 144, fumaric acid | **LC-MS (ESI) (Method 2):** Rₜ = 2.096 min, m/z found 655.6 [M+H]⁺. |
| | | | **SFC (Method 19):** Rₜ = 4.578 min. |
| 148 | 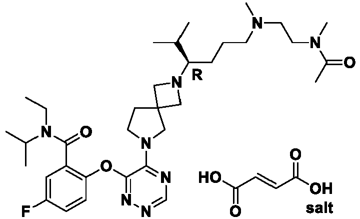 | Compound 147, fumaric acid | **LC-MS (ESI) (Method 3):** Rₜ = 4.480 min, m/z found 641.6 [M+H]⁺. |
| | | | **SFC (Method 6):** Rₜ = 1.356 min. |
| 150 | 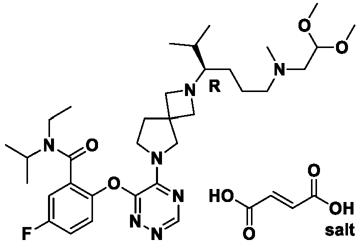 | Compound 149, fumaric acid | **LC-MS (ESI) (Method 3):** Rₜ = 4.836 min, m/z found 630.4 [M+H]⁺. |
| | | | **SFC (Method 6):** Rₜ = 1.067 min. |
| 152 | 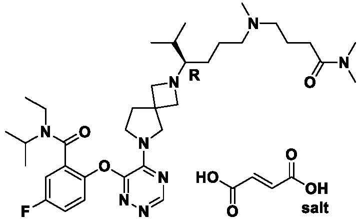 | Compound 151, fumaric acid | **LC-MS (ESI) (Method 2):** Rₜ = 2.332 min, m/z found 655.5 [M+H]⁺. |
| | | | **SFC (Method 6):** Rₜ = 1.449 min. |
| 154 | 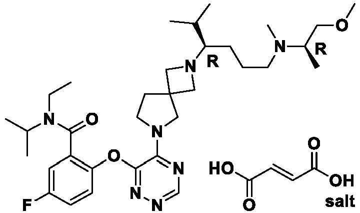 | Compound 153, fumaric acid | **LC-MS (ESI) (Method 2):** Rₜ = 2.022 min, m/z found 614.4 [M+H]⁺. |
| | | | **SFC (Method 6):** Rₜ = 1.137 min. |
| 157 | 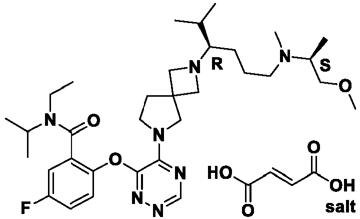 | Compound 156, fumaric acid | **LC-MS (ESI) (Method** 2): Rₜ = 2.049 min, m/z found 614.4 [M+H]⁺. |
| | | | **SFC (Method 20):** Rₜ = 1.001 min. |
| 159 | 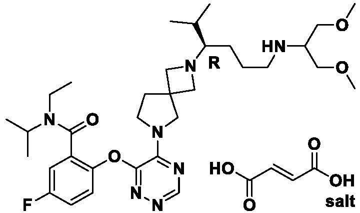 | Compound 158, fumaric acid | **LC-MS (ESI) (Method 1):** Rₜ = 3.102 min, m/z found 630.4 [M+H]⁺. |
| | | | **SFC (Method 6):** Rₜ = 1.194 min. |
| 161 | 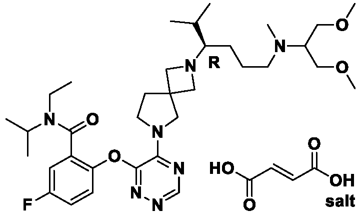 | Compound 160, fumaric acid | **LC-MS (ESI) (Method 2):** Rₜ = 2.112 min, m/z found 644.3 [M+H]⁺. |
| | | | **SFC (Method 6):** Rₜ = 1.073 min. |
| 165 | 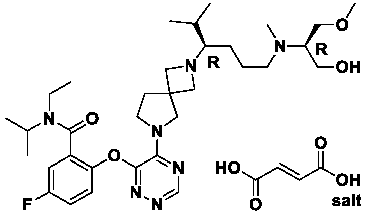 | Compound 164, fumaric acid | **LC-MS (ESI) (Method 3):** Rₜ = 4.766 min, m/z found 630.3 [M+H]⁺. |
| | | | **SFC (Method 6):** Rₜ = 1.271 min. |
| 167 | 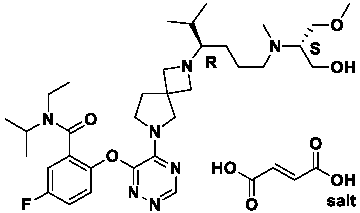 | Compound 166, fumaric acid | **LC-MS (ESI) (Method 3):** Rₜ = 4.730 min, m/z found 630.3 [M+H]⁺. |
| | | | **SFC (Method 6):** Rt = 1.373 min. |
| 170 | 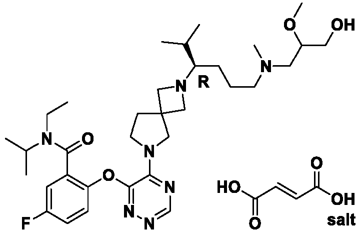 | Compound 169, fumaric acid | **LC-MS (ESI) (Method 3):** Rₜ = 4.652 min, m/z found 630.4 [M+H]⁺. |
| 172 | 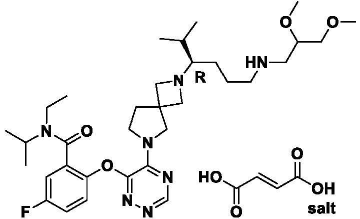 | Compound 171, fumaric acid | **LC-MS (ESI) (Method 3):** Rₜ = 5.073 min, m/z found 630.4 [M+H]⁺. |
| 176 | 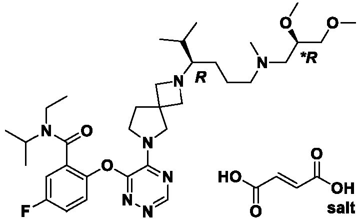 | Compound 174, fumaric acid | **LC-MS (ESI) (Method 3):** Rₜ = 5.035 min, m/z found 644.5 [M+H]⁺. |
| | | | **SFC (Method** 23): Rₜ = 4.662 min. |
| 177 | 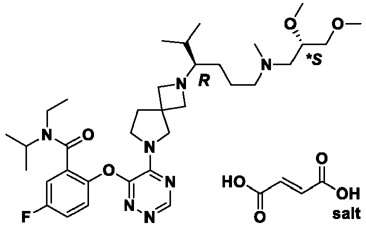 | Compound 175, fumaric acid | **LC-MS (ESI) (Method 3):** Rₜ = 5.031 min, m/z found 644.5 [M+H]⁺. |
| | | | **SFC (Method 23):** Rₜ = 4.977 min. |
| 179 | 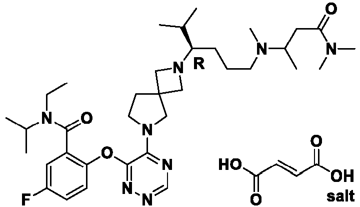 | Compound 178, fumaric acid | **LC-MS (ESI) (Method 5):** Rₜ = 1.72 min, m/z found 655.5 [M+H]⁺. |
| 181 | 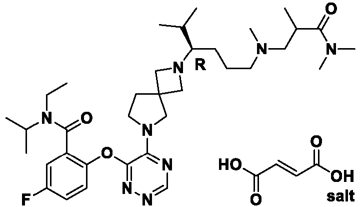 | Compound 180, fumaric acid | **LC-MS (ESI) (Method 5):** Rₜ = 1.61 min, m/z found 655.5 [M+H]⁺. |
| 184 | 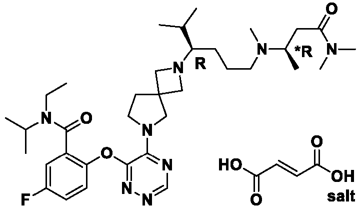 | Compound 182, fumaric acid | **LC-MS (ESI) (Method 2):** Rₜ = 2.041 min, m/z found 655.4 [M+H]⁺. |
| | | | **SFC (Method 8):** Rₜ = 2.752 min. |
| 185 | 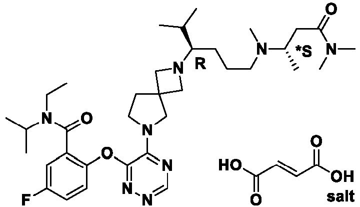 | Compound 183, fumaric acid | **LC-MS (ESI) (Method 2):** Rₜ = 2.058 min, m/z found 655.4 [M+H]⁺. |
| | | | **SFC (Method 8):** Rₜ = 3.09 min. |
| 188 | 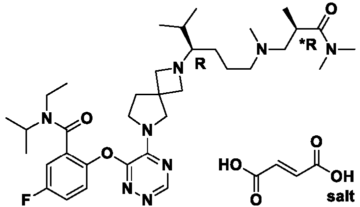 | Compound 186, fumaric acid | **LC-MS (ESI) (Method 3):** Rₜ = 4.940 min, m/z found 655.4 [M+H]⁺. |
| | | | **SFC (Method 23):** Rₜ = 5.055 min. |
| 189 | 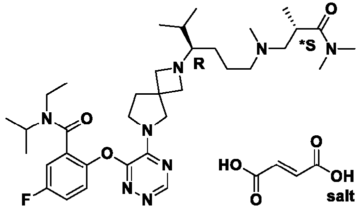 | Compound 187, fumaric acid | **LC-MS (ESI) (Method 3):** Rₜ = 4.907 min, m/z found 655.4 [M+H]⁺. |
| | | | **SFC (Method 23):** Rₜ = 5.287 min. |
| 191 | 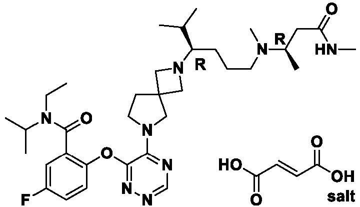 | Compound 190, fumaric acid | **LC-MS (ESI) (Method 5):** Rₜ = 1.66 min, m/z found 641.3 [M+H]⁺. |
| 193 | 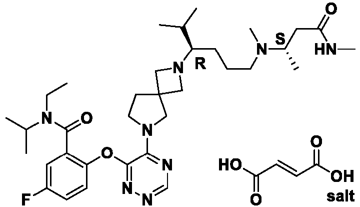 | Compound 192, fumaric acid | **LC-MS (ESI) (Method 5):** Rₜ = 1.70 min, m/z found 641.3 [M+H]⁺. |
| 195 | 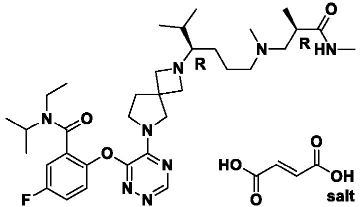 | Compound 194, fumaric acid | **LC-MS (ESI) (Method 5):** Rₜ = 1.67 min, m/z found 641.3 [M+H]⁺. |
| 197 | 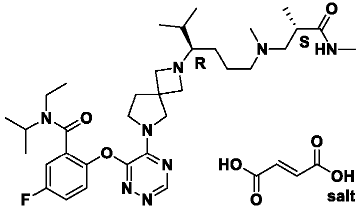 | Compound 196, fumaric acid | **LC-MS (ESI) (Method 5):** Rₜ = 1.85 min, m/z found 641.3 [M+H]⁺. |
| 199 | 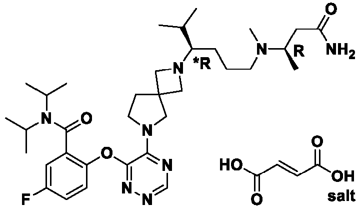 | Compound 198, fumaric acid | **LC-MS (ESI) (Method 5):** Rₜ = 1.62 min, m/z found 641.3 [M+H]⁺. |
| 201 | 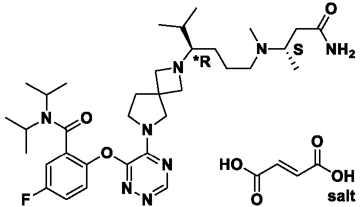 | Compound 200, fumaric acid | **LC-MS (ESI) (Method 5):** Rₜ = 1.63 min, m/z found 641.3 [M+H]⁺. |
| 203 | 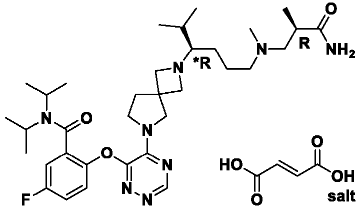 | Compound 202, fumaric acid | **LC-MS (ESI) (Method 5):** Rₜ = 1.72 min, m/z found 641.3 [M+H]⁺. |
| 205 | 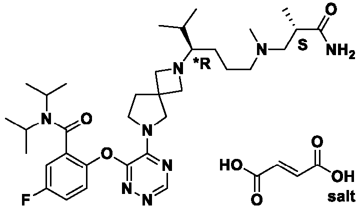 | Compound 204, fumaric acid | **LC-MS (ESI) (Method 5):** Rₜ = 1.66 min, m/z found 641.2 [M+H]⁺. |
| 219 | 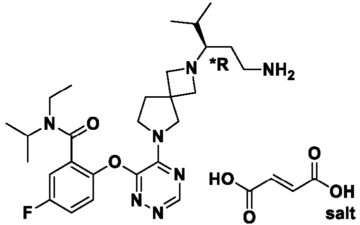 | Compound 218, fumaric acid | **LC-MS (ESI) (Method 6):** Rₜ = 2.67 min, m/z found 514.2 [M+H]⁺. |
| 223 | 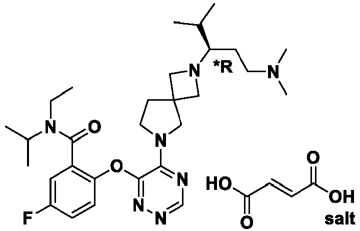 | Compound 222, fumaric acid | **LC-MS (ESI) (Method 3):** Rₜ = 5.047 min, m/z found 542.3 [M+H]⁺. |
| | | | **SFC (Method 18):** Rₜ = 1.991 min. |
| 225 | 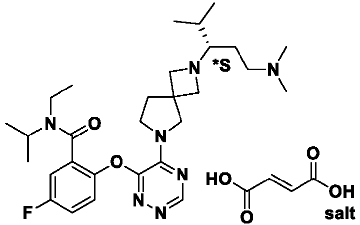 | Compound 224, fumaric acid | **LC-MS (ESI) (Method 3):** Rₜ = 4.890 min, m/z found 542.3 [M+H]⁺. |
| | | | **SFC (Method 18):** Rₜ = 2.189 min. |
| 227 | 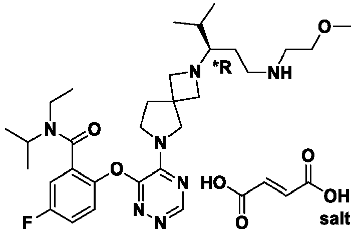 | Compound 226, fumaric acid | **LC-MS (ESI) (Method 6):** Rₜ = 3.00 min, m/z found 572.3 [M+H]⁺. |
| 233 | 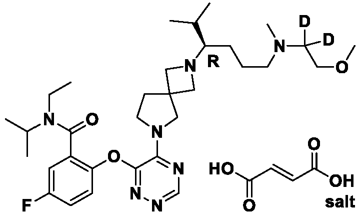 | Compound 232, fumaric acid | **LC-MS (ESI) (Method 1):** Rₜ = 3.031 min, m/z found 602.3 [M+H]⁺. |
| | | | **SFC (Method 6):** Rₜ = 1.134 min. |
| 240 | 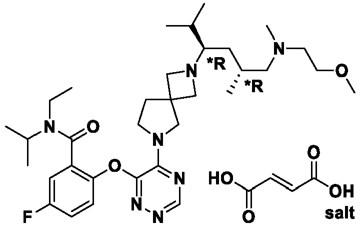 | Compound 236, fumaric acid | **LC-MS (ESI) (Method 3):** Rₜ = 5.301 min, m/z found 614.4 [M+H]⁺. |
| | | | **SFC (Method 4):** Rₜ = 1.241 min. |
| 241 | 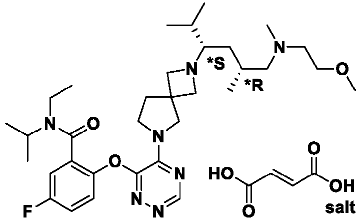 | Compound 237, fumaric acid | **LC-MS (ESI) (Method 3):** Rₜ = 5.194 min, m/z found 614.4 [M+H]⁺. |
| | | | **SFC (Method 4):** Rₜ = 1.347 min. |
| 242 | 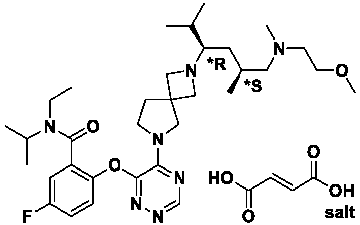 | Compound 238, fumaric acid | **LC-MS (ESI) (Method 3):** Rₜ = 5.284 min, m/z found 614.4 [M+H]⁺. |
| | | | **SFC (Method 14):** Rₜ = 2.358 min. |
| 243 | 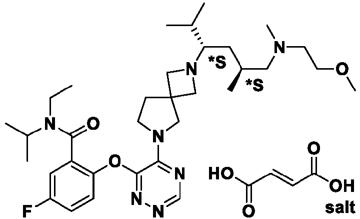 | Compound 239, fumaric acid | **LC-MS (ESI) (Method 3):** Rₜ = 5.244 min, m/z found 614.4 [M+H]⁺. |
| | | | **SFC (Method 14):** Rₜ = 2.450 min. |
| 245 | 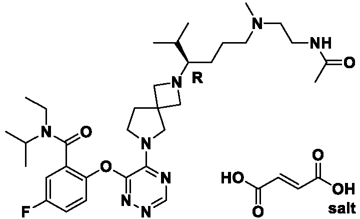 | Compound 244, fumaric acid | **¹H NMR (400 MHz, Methanol-*d*₄):** δ = 8.51 (brs, 1H), 7.56-7.16 (m, 3H), 6.74 (s, 4H), 4.57-3.67 (m, 9H), 3.63-3.40 (m, 3H), 3.30-3.08 (m, 6H), 2.87 (s, 3H), 2.48-2.28 (m, 2H), 2.20-2.07 (m, 1H), 1.98 (s, 3H), 1.92-1.79 (m, 2H), 1.76-1.52 (m, 2H), 1.26-0.94 (m, 13H), 0.89-0.74 (m, 2H). |
| | | | **LC-MS (ESI) (Method 1):** Rₜ = 2.916 min, m/z found 627.4 [M+H]⁺. |
| | | | **SFC (Method 25):** Rₜ = 1.707 min. |
| 256 | 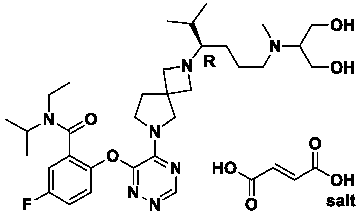 | Compound 255, fumaric acid | **LC-MS (ESI) (Method 1):** Rₜ = 2.932 min, m/z found 616.3 [M+H]⁺. |
| | | | **SFC (Method 6):** Rₜ = 1.383 min. |
| 265 | 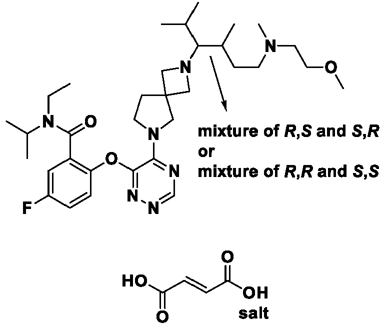 | Compound 263, fumaric acid | **LC-MS (ESI) (Method 2):** Rₜ = 2.043 min, m/z found 614.3 [M+H]⁺. |
| 266 | 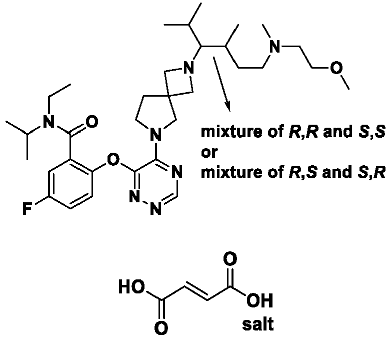 | Compound 264, fumaric acid | **LC-MS (ESI) (Method 2):** Rₜ = 1.988 min, m/z found 614.5 [M+H]⁺. |
| 268 | 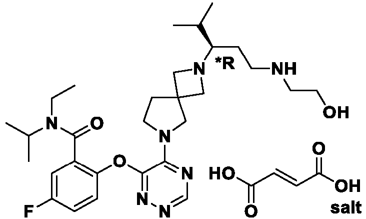 | Compound 267, fumaric acid | **LC-MS (ESI) (Method 5):** Rₜ = 1.45 min, m/z found 558.2 [M+H]⁺. |
| 270 | 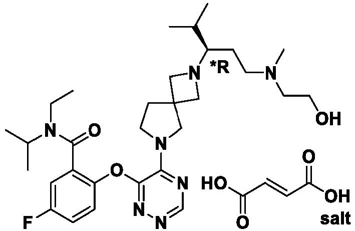 | Compound 269, fumaric acid | **LC-MS (ESI) (Method 5):** Rₜ = 1.49 min, m/z found 572.3 [M+H]⁺. |
| 278 | 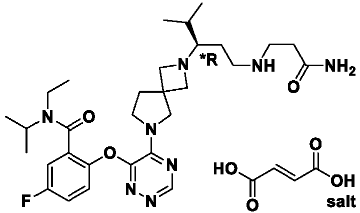 | Compound 277, fumaric acid | **LC-MS (ESI) (Method 5):** Rₜ = 1.54 min, m/z found 585.2 [M+H]⁺. |
| 280 | 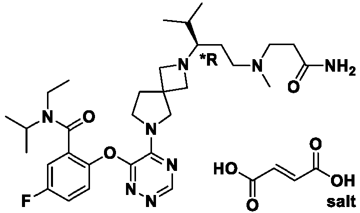 | Compound 279, fumaric acid | **LC-MS (ESI) (Method 5):** Rₜ = 1.54 min, m/z found 599.3 [M+H]⁺. |
| 283 | 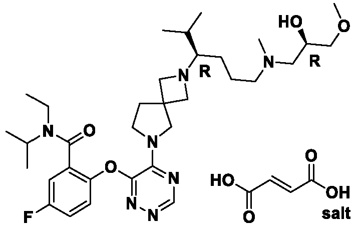 | Compound 282, fumaric acid | **LC-MS (ESI) (Method 1):** Rₜ = 2.605 min, m/z found 630.3 [M+H]⁺. |
| | | | **SFC (Method 6):** Rₜ = 1.303 min. |
| 259 | 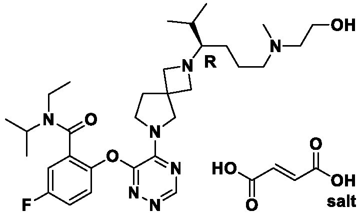 | Compound 286, fumaric acid | **LC-MS (ESI) (Method 1):** Rₜ = 2.900 min, m/z found 586.6 [M+H]⁺. |
| | | | **SFC (Method 6):** Rₜ = 1.301 min. |
| 104 | 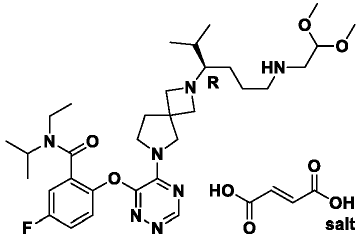 | Compound 287, fumaric acid | **¹H NMR (400 MHz, Methanol-*d*₄):** δ = 8.45 (s, 1H), 7.50-7.09 (m, 3H), 6.67 (s, 4H), 4.48-3.60 (m, 10H), 3.45 (s, 6H), 3.23-2.87 (m, 6H), 2.44-2.18 (m, 2H), 2.16-1.96 (m, 1H), 1.89-1.50 (m, 4H), 1.29-0.91 (m, 14H), 0.87-0.70 (m, 2H). |
| | | | **LC-MS (ESI) (Method 1):** Rₜ = 3.025 min, m/z found 616.3 [M+H]⁺. |
| | | | **SFC (Method 6):** Rₜ = 1.305 min. |
| 229 | 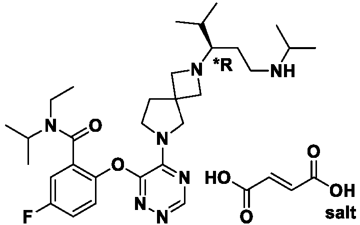 | Compound 228, fumaric acid | **LC-MS (ESI) (Method 6):** Rₜ = 2.95 min, m/z found 556.3 [M+H]⁺. |
| 300 | 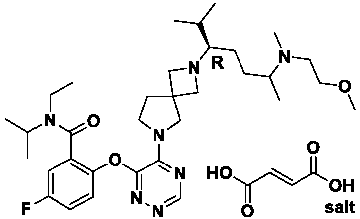 | Compound 299, fumaric acid | **LC-MS (ESI) (Method 2):** Rₜ = 2.017 min, m/z found 614.4 [M+H]⁺. |
| | | | **Chiral HPLC (Method 8):** Rₜ = 5.212 min. |
| 302 | 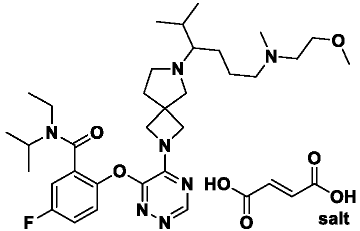 | Compound 301, fumaric acid | **LC-MS (ESI) (Method 2):** Rₜ = 2.021 min, m/z found 600.7 [M+H]⁺. |
| 314 | 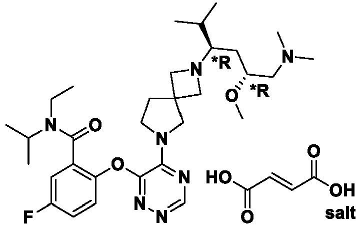 | Compound 310, fumaric acid | **LC-MS (ESI) (Method 3):** Rₜ = 4.900 min, m/z found 586.3 [M+H]⁺. |
| | | | **SFC (Method 11):** Rₜ = 4.457 min. |
| 315 | 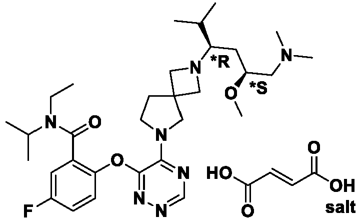 | Compound 312, fumaric acid | **LC-MS (ESI) (Method 3):** Rₜ = 4.966 min, m/z found 586.3 [M+H]⁺. |
| | | | **SFC (Method 11):** Rₜ = 4.273 min. |
| 323 | 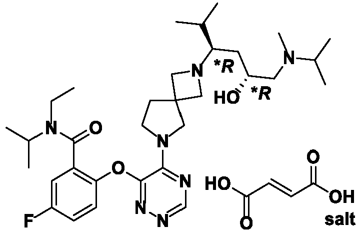 | Compound 319, fumaric acid | **LC-MS (ESI) (Method 2):** Rₜ = 2.006 min, m/z found 600.3 [M+H]⁺. |
| | | | **SFC (Method 27):** Rₜ = 2.598 min. |
| 324 | 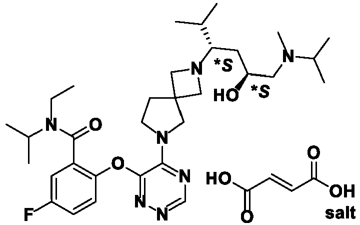 | Compound 320, fumaric acid | **LC-MS (ESI) (Method 2):** Rₜ = 2.012 min, m/z found 600.4 [M+H]⁺. |
| | | | **SFC (Method 27)**: Rₜ = 4.487 min. |
| 325 | 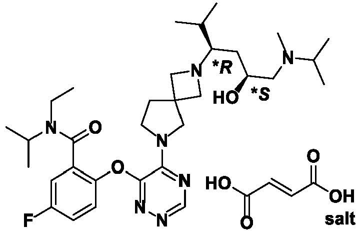 | Compound 321, fumaric acid | **LC-MS (ESI) (Method 2):** Rₜ = 2.012 min, m/z found 600.3 [M+H]⁺. |
| | | | **SFC (Method 28):** Rₜ = 2.196 min. |
| 326 | 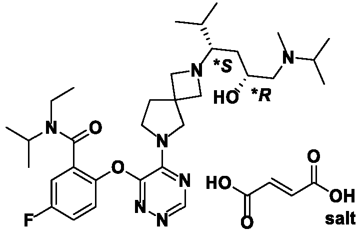 | Compound 322, fumaric acid | **LC-MS (ESI) (Method 1):** Rₜ = 3.045 min, m/z found 600.3 [M+H]⁺. |
| | | | **SFC (Method 28):** Rₜ = 2.677 min. |
| 334 | 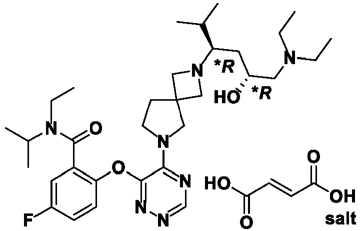 | Compound 330, fumaric acid | **LC-MS (ESI) (Method 2):** Rₜ = 2.023 min, m/z found 600.3 [M+H]⁺. |
| | | | **Chiral HPLC (Method 9):** Rₜ = 4.014 min. |
| 335 | 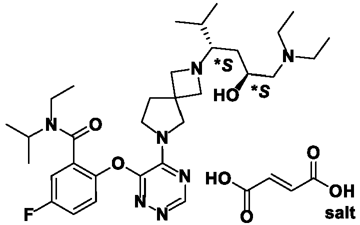 | Compound 331, fumaric acid | **LC-MS (ESI) (Method 2):** Rₜ = 2.028 min, m/z found 600.3 [M+H]⁺. |
| | | | **Chiral HPLC (Method 9):** Rₜ = 4.265 min. |
| 336 | 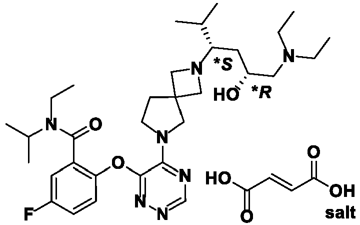 | Compound 332, fumaric acid | **LC-MS (ESI) (Method 2):** Rₜ = 1.967 min, m/z found 600.3 [M+H]⁺. |
| | | | **SFC (Method 29):** Rₜ = 4.190 min. |
| 337 | 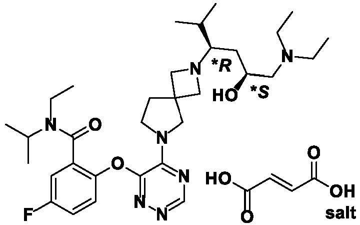 | Compound 333, fumaric acid | **LC-MS (ESI) (Method 2):** Rₜ = 1.973 min, m/z found 600.3 [M+H]⁺. |
| | | | **SFC (Method 29):** Rₜ = 4.444 min. |
| 342 | 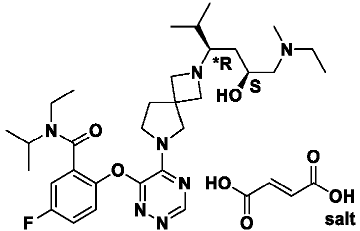 | Compound 340, fumaric acid | **LC-MS (ESI) (Method 1):** Rₜ = 2.934 min, m/z found 586.5 [M+H]⁺. |
| | | | **SFC (Method 6):** Rₜ = 1.326 min. |
| 343 | 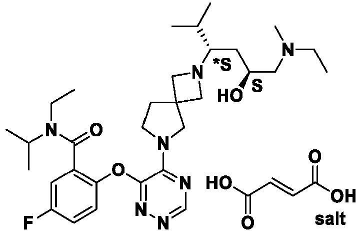 | Compound 341, fumaric acid | **LC-MS (ESI) (Method 1):** Rₜ = 2.976 min, m/z found 586.5 [M+H]⁺. |
| | | | **SFC (Method 6):** Rₜ = 1.285 min**.** |
| 346 | 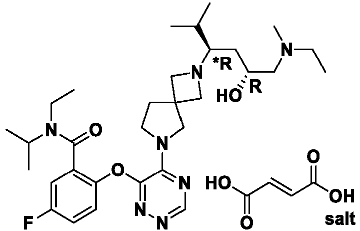 | Compound 344, fumaric acid | **¹H NMR (400 MHz, Methanol-*d*₄):** δ = 8.47 (s, 1H), 7.53-7.16 (m, 3H), 6.68 (s, 4H), 4.49-3.65 (m, 10H), 3.42 (brs, 2H), 3.28-2.99 (m, 5H), 2.88 (s, 3H), 2.34 (brs, 2H), 2.23-2.11 (m, 1H), 1.85-1.63 (m, 2H), 1.40-0.74 (m, 18H). |
| | | | **LC-MS (ESI) (Method 1):** Rₜ = 2.968 min, m/z found 586.3 [M+H]⁺. |
| | | | **SFC (Method 8):** Rₜ = 2.265 min. |
| 352 | 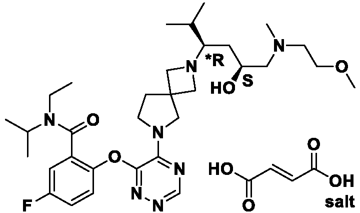 | Compound 350, fumaric acid | **LC-MS (ESI) (Method 1):** Rₜ = 3.010 min, m/z found 616.3 [M+H]⁺. |
| | | | **SFC (Method 6):** Rₜ = 1.235 min. |
| 353 | 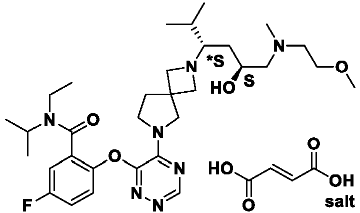 | Compound 351, fumaric acid | **LC-MS (ESI) (Method 1):** Rₜ = 2.967 min, m/z found 616.3 [M+H]⁺. |
| | | | **SFC (Method 6):** Rₜ = 1.261 min. |
| 356 | 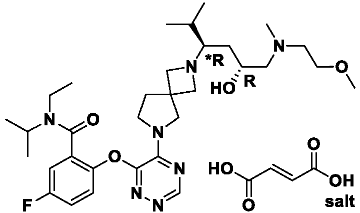 | Compound 354, fumaric acid | **LC-MS (ESI) (Method 1):** Rₜ = 2.959 min, m/z found 616.4 [M+H]⁺. |
| | | | **SFC (Method 28):** Rₜ = 2.014 min. |
| 357 | 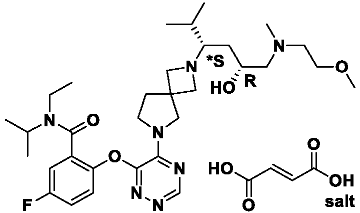 | Compound 355, fumaric acid | **LC-MS (ESI) (Method 1):** Rₜ = 2.906 min, m/z found 616.3 [M+H]⁺. |
| | | | **SFC (Method 28):** Rₜ = 2.973 min. |
| 365 | 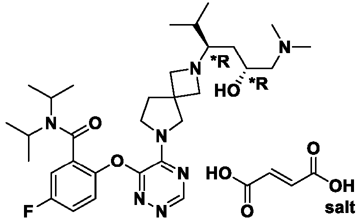 | Compound 363, fumaric acid | **LC-MS (ESI) (Method 1):** Rₜ = 3.150 min, m/z found 586.3 [M+H]⁺. |
| | | | **SFC (Method 30):** Rₜ = 2.491 min. |
| 366 | 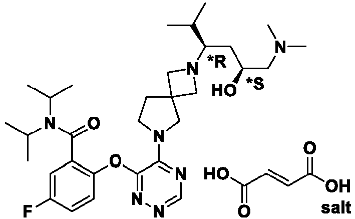 | Compound 364, fumaric acid | **LC-MS (ESI) (Method 1):** Rₜ = 3.093 min, m/z found 586.3 [M+H]⁺. |
| | | | **SFC (Method 30):** Rₜ = 3.517 min. |
| 369 | 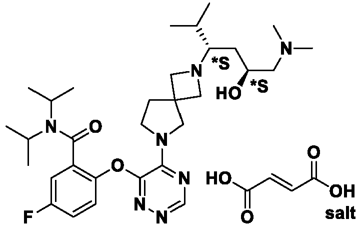 | Compound 367, fumaric acid | **LC-MS (ESI) (Method 1):** Rₜ = 3.170 min, m/z found 586.3 [M+H]⁺. |
| | | | **SFC (Method 31):** Rₜ = 1.863 min. |
| 370 | 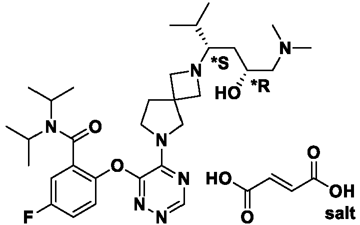 | Compound 368, fumaric acid | **LC-MS (ESI) (Method 1):** Rₜ = 3.137 min, m/z found 586.3 [M+H]⁺. |
| | | | **SFC (Method 31):** Rₜ = 2.165 min. |
| 377 | 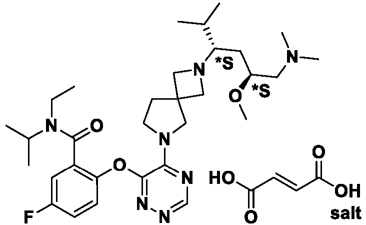 | Compound 375, fumaric acid | **LC-MS (ESI) (Method 2):** Rₜ = 1.997 min, m/z found 586.3 [M+H]⁺. |
| | | | **SFC (Method 11):** Rₜ = 4.749 min. |
| 378 | 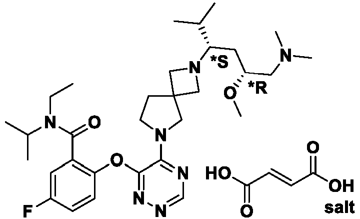 | Compound 376, fumaric acid | **LC-MS (ESI) (Method 3):** Rₜ = 4.923 min, m/z found 586.3 [M+H]⁺. |
| | | | **SFC (Method 11):** Rₜ = 4.663 min. |
| 382 | 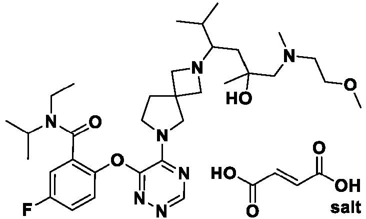 | Compound 381, fumaric acid | **LC-MS (ESI) (Method 2):** Rₜ = 2.019/2.055 min, m/z found 630.5 [M+H]⁺. |
| 386 | 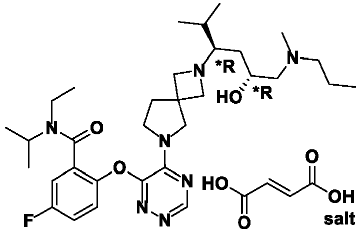 | Compound 384, fumaric acid | **LC-MS (ESI) (Method 2):** Rₜ = 2.062 min, m/z found 600.3 [M+H]⁺. |
| | | | **SFC (Method 27):** Rₜ = 2.111 min. |
| 387 | 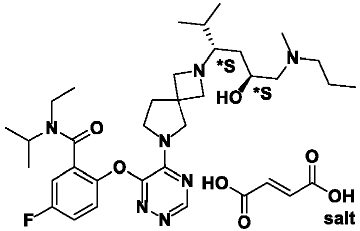 | Compound 385, fumaric acid | **LC-MS (ESI) (Method 2):** Rₜ = 2.059 min, m/z found 600.3 [M+H]⁺. |
| | | | **SFC (Method 27):** Rₜ = 3.466 min. |
| 391 | 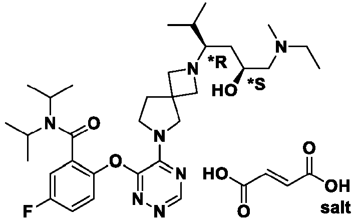 | Compound 389, fumaric acid | **LC-MS (ESI) (Method 2):** Rₜ = 2.159 min, m/z found 600.3 [M+H]⁺. |
| | | | **Chiral HPLC (Method 9):** Rt = 3.862 min. |
| 392 | 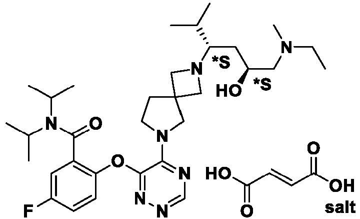 | Compound 390, fumaric acid | **LC-MS (ESI) (Method 2):** Rₜ = 2.119 min, m/z found 600.3 [M+H]⁺. |
| | | | **SFC (Method 27):** Rₜ = 2.386 min. |
| 394 | 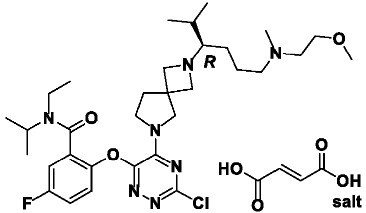 | Compound 393, fumaric acid | **LC-MS (ESI) (Method 2):** Rₜ = 2.442 min, m/z found 634.3 [M+H]⁺. |
| | | | **SFC (Method 6):** Rₜ = 1.232 min. |
| 397 | 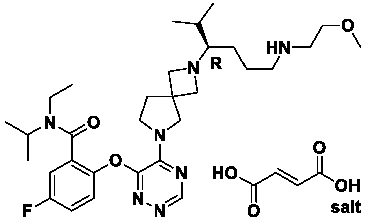 | Compound 11, fumaric acid | **LC-MS (ESI) (Method 5):** Rₜ = 1.661 min, m/z found 586.2 [M+H]⁺. |

### Compound 6

### (R)-2-((5-(2-(6-acetamido-2-methylhexan-3-yl)-2,6-diazaspiro[3.4]octan-6-yl)-1,2,4-triazin-6-yl)oxy)-N-ethyl-5-fluoro-N-isopropylbenzamide formate

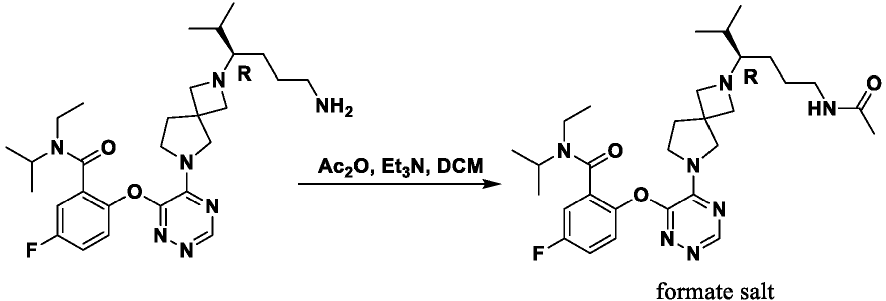

To the solution of (*R*)-2-((5-(2-(6-amino-2-methylhexan-3-yl)-2,6-diazaspiro[3.4]octan-6-yl)-1,2,4-triazin-6-yl)oxy)-*N*-ethyl-5-fluoro-*N*-isopropylbenzamide formate **(Compound 1)** (30 mg, 0.057 mmol) and TEA (60 uL, 0.43 mmol) in DCM (1 mL) cooled at 0 °C was added Ac₂O (20 uL, 0.21 mmol), the resulting mixture was stirred at RT under N₂ atmosphere for 0.5 h. The reaction mixture was concentrated under reduced pressure and the residue was purified by preparative HPLC using a Welch Xtimate (column: C18 150x25mm 5um; eluent: ACN/H₂O (0.225% FA) from 30% to 50% (v/v)) to afford the title compound (3.31 mg, 9% yield) as a white solid.

**LC-MS (ESI) (Method 5):** Rₜ = 0.633 min, m/z found 570.4 [M+H]⁺.

**SFC (Method 5):** Rt = 1.191 min.

### Compound 7, 29, 34

### (S)-2-((5-(2-(6-acetamido-2-methylhexan-3-yl)-2,6-diazaspiro[3.4]octan-6-yl)-1,2,4-triazin-6-yl)oxy)-N-ethyl-5-fluoro-N-isopropylbenzamide

### 2-((5-(2-(1-acetamido-4-methylpentan-3-yl)-2,6-diazaspiro[3.4]octan-6-yl)-1,2,4-triazin-6-yl)oxy)-N-ethyl-5-fluoro-N-isopropylbenzamide

### (*R)-2-((5-(2-(6-acetamido-2,6-dimethylheptan-3-yl)-2,6-diazaspiro[3.4]octan-6-yl)-1,2,4-triazin-6-yl)oxy)-N-ethyl-5-fluoro-N-isopropylbenzamide

**The following Compounds were synthesized by an analogous method described above for Compound 6**

| **Co. No.** | **Structure** | **Starting Materials** | **Conditions** | **Spectra Details** |
|---|---|---|---|---|
| 7 | 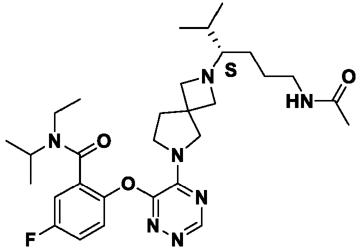 | Compound 2 | Ac₂O, TEA, DCM | **LC-MS (ESI) (Method 5):** Rt = 0.646 min, m/z found 570.3 [M+H]⁺. |
| | | | | **SFC (Method 5):** Rt = 1.657 min. |
| 29 | 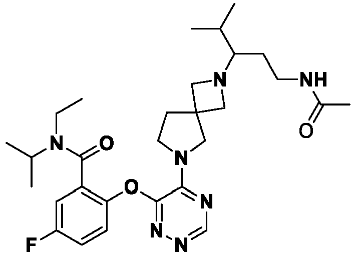 | Compound 26 | Ac₂O, TEA, DCM | LC-MS (ESI) **(Method 1):** Rt = 3.250 min, m/z found 556.4 [M+H]⁺. |
| 34 | 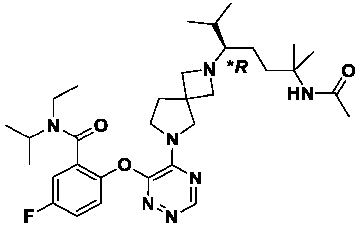 | Compound 30 | AcCl, TEA, DCM | **LC-MS (ESI) (Method 3):** Rₜ = 4.573 min, m/z found 598.3 [M+H]⁺. |

### Compound 8

### (R)-N-ethyl-5-fluoro-N-isopropyl-2-((5-(2-(2-methyl-6-(3-methylureido)hexan-3-yl)-2,6-diazaspiro[3.4]octan-6-yl)-1,2,4-triazin-6-yl)oxy)benzamide

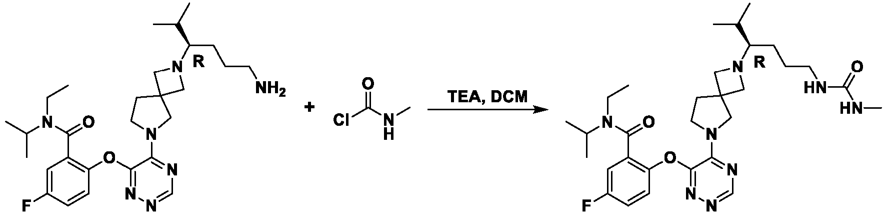

To the solution of (*R*)-2-((5-(2-(6-amino-2-methylhexan-3-yl)-2,6-diazaspiro[3.4]octan-6-yl)-1,2,4-triazin-6-yl)oxy)-*N*-ethyl-5-fluoro-*N*-isopropylbenzamide formate **(Compound 1)** (70 mg, 0.12 mmol) and TEA (0.35 mL, 2.5 mmol) in DCM (10 mL) cooled at 0 °C was added methylcarbamic chloride (18 mg, 0.19 mmo) and the resulting mixture was stirred for 2 h at 0 °C. The reaction mixure was concentrated under reduced pressure and the residue was purified by preparative HPLC over Phenomenex Gemini-NX (column: 150x30mm 5um; eluent: ACN/H₂O (0.04% ammonia+10mM NH₄HCO₃) from 35% to 65%, v/v) to afford the title compound (50 mg, 70% yield) as a white solid.

**LC-MS (ESI) (method 1):** Rₜ = 3.34 min, m/z found 585.3 [M+H]⁺.

**SFC (Method 6):** Rt = 2.222 min.

### Compound 9

### (S)-N-ethyl-5-fluoro-N-isopropyl-2-((5-(2-(2-methyl-6-(3-methylureido)hexan-3-yl)-2,6-diazaspiro[3.4]octan-6-yl)-1,2,4-triazin-6-yl)oxy)benzamide

**The following Compound was synthesized by an analogous method described above for Compound 8**

| **Co. No.** | **Structure** | **Starting Materials** | **Spectra Details** |
|---|---|---|---|
| 9 | 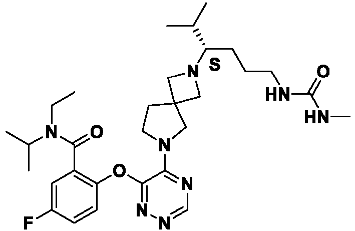 | Compound 2 | **LC-MS (ESI) (method 1):** Rt = 3.38 min, m/z found 585.3 [M+H]⁺. |
| | | | **SFC (Method 6):** Rₜ = 2.418 min. |

### Compound 10

### methyl (R)-(4-(6-(6-(2-(ethyl(isopropyl)carbamoyl)-4-fluorophenoxy)-1,2,4-triazin-5-yl)-2,6-diazaspiro[3.4]octan-2-yl)-5-methylhexyl)carbamate

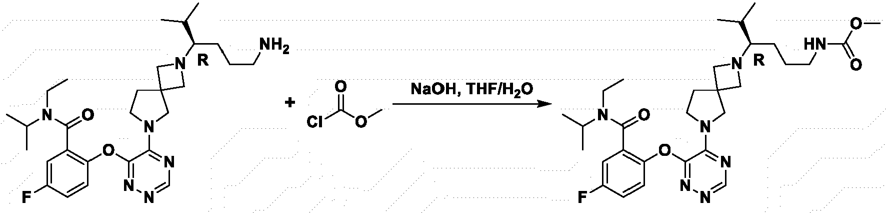

To the mixture of (*R*)-2-((5-(2-(6-amino-2-methylhexan-3-yl)-2,6-diazaspiro[3.4]octan-6-yl)-1,2,4-triazin-6-yl)oxy)-*N*-ethyl-5-fluoro-*N*-isopropylbenzamide hydrochloride **(Compound 65)** (0.100 g, crude) in THF/H₂O (2 mL/2 mL) cooled at 0 °C were added 2 M NaOH (0.15 mL, 0.30 mmol) and methyl carbonochloridate (0.030 g, 0.317 mmol, in 0.1 mL DCM). The resulting mixture was stirred at 0 °C for 0.5 h. The mixture was diluted with water (10 mL) and sat. aq. NaHCO₃ (15 mL), further extracted with EtOAc (15 mL x 3). The combined organic layers were dried over (Na₂SO₄), filtered and evaporated *in vacuo* to give the crude product, which was further purified by preparative HPLC using Phenomenex Gemini NX (column: C18 75x30mm 3um; eluent: ACN/H₂O (0.05% ammonia+10mM NH₄HCO₃) 35% to 65% (v/v)) to afford the title compound (11.53 mg) as sticky oil.

**LC-MS (ESI) (Method 1):** Rt = 3.283 min, m/z found 586.3 [M+H]⁺.

### Compound 22

### methyl (R)-(4-(6-(6-(2-(ethyl(isopropyl)carbamoyl)-4-fluorophenoxy)-1,2,4-triazin-5-yl)-2,6-diazaspiro[3.4]octan-2-yl)-5-methylhexyl)(methyl)carbamate

**The following Compound was synthesized by an analogous method described above for Compound 10**

| **Co. No.** | **Structure** | **Starting Materials** | **Spectra Details** |
|---|---|---|---|
| 22 | 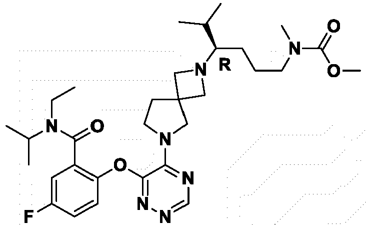 | Compound 19 | **LC-MS (ESI) (Method 2):** Rₜ = 2.472 min, m/z found 600.3 [M+H]⁺. |

### Compound 11

### (R)-N-ethyl-5-fluoro-N-isopropyl-2-((5-(2-(6-((2-methoxyethyl)amino)-2-methylhexan-3-yl)-2,6-diazaspiro[3.4]octan-6-yl)-1,2,4-triazin-6-yl)oxy)benzamide

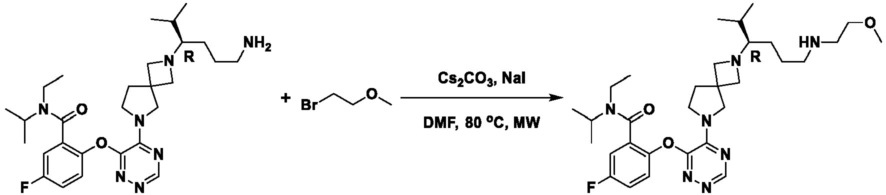

The mixture of (*R*)-2-((5-(2-(6-amino-2-methylhexan-3-yl)-2,6-diazaspiro[3.4]octan-6-yl)-1,2,4-triazin-6-yl)oxy)-*N*-ethyl-5-fluoro-*N*-isopropylbenzamide **(Compound 64)** (120 mg, crude), 1-bromo-2-methoxyethane (32 mg, 0.23 mmol), Cs₂CO₃ (222 mg, 0.681 mmol), NaI (102 mg, 0.680 mmol) in DMF (1 mL) was stirred at 80 °C via microwave irradiation for 1 h. After cooling to RT, the mixture was diluted with H₂O (10 mL) and extracted with EtOAc (3 x 10 mL). The combined organic layers were washed with H₂O (10 mL), dried over Na₂SO₄, filtered and concentrated under reduced pressure to afford the crude product which was further purified by HPLC over a Phenomenex Gemini-NX (column: 150x30 mm 5 µm; eluent: ACN/H₂O (10mM NH₄HCO₃) from 51% to 71% (v/v)) and further purified by SFC over DAICEL CHIRALCEL OD-H (column: 250x30 mm 5 um; eluent: supercritical CO₂ in EtOH (0.1% v/v ammonia) 25/25, v/v) to afford the title compound (5.13 mg, 96% purity) as yellow solid.

**LC-MS (ESI) (Method 1):** Rt = 2.997 min, m/z found 586.3 [M+H]⁺.
**Compound 28, 90, 93, 287, 149, 226, 257, 228**
**(*S*)-*N*-ethyl-5-fluoro-*N*-isopropyl-2-((5-(2-(6-((2-methoxyethyl)(methyl)amino)-2-methylhexan-3-yl)-2,6-diazaspiro[3.4]octan-6-yl)-1,2,4-triazin-6-yl)oxy)benzamide**
**(*R*)-2-((5-(2-(6-(bis(2-methoxyethyl)amino)-2-methylhexan-3-yl)-2,6-diazaspiro[3.4]octan-6-yl)-1,2,4-triazin-6-yl)oxy)-*N*-ethyl-5-fluoro-*N-*isopropylbenzamide formate**
**5-fluoro-*N,N-*diisopropyl-2-((5-(2-(6-((2-methoxyethyl)(methyl)amino)-2-methylhexan-3-yl)-2,6-diazaspiro[3.4]octan-6-yl)-1,2,4-triazin-6-yl)oxy)benzamide formate**
**(*R*)-2-((5-(2-(6-((2,2-dimethoxyethyl)amino)-2-methylhexan-3-yl)-2,6-diazaspiro[3.4]octan-6-yl)-1,2,4-triazin-6-yl)oxy)-*N*-ethyl-5-fluoro-*N-*isopropylbenzamide**
**(*R*)-2-((5-(2-(6-((2,2-dimethoxyethyl)(methyl)amino)-2-methylhexan-3-yl)-2,6-diazaspiro[3.4]octan-6-yl)-1,2,4-triazin-6-yl)oxy)-*N*-ethyl-5-fluoro-*N-*isopropylbenzamide**
***(*R)-N-ethyl-5-fluoro-N-isopropyl-2-((5-(2-(1-((2-methoxyethyl)amino)-4-methylpentan-*3-yl)-2,6-diazaspiro[3.4]octan-6-yl)-1,2,4-triazin-6-yl)oxy)benzamide**
**(*R*)-2-((5-(2-(6-((2-ethoxyethyl)(methyl)amino)-2-methylhexan-3-yl)-2,6-diazaspiro[3.4]octan-6-yl)-1,2,4-triazin-6-yl)oxy)-*N*-ethyl-5-fluoro-*N-*isopropylbenzamide**
***(*R)-N-ethyl-5-fluoro-N-isopropyl-2-((5-(2-(1-(isopropylamino)-4-methylpentan-3-yl)-*2,6-diazaspiro[3.4]octan-6-yl)-1,2,4-triazin-6-yl)oxy)benzamide**

**The following Compounds were synthesized by an analogous method described above for Compound 11**

| **Co. No.** | **Structure** | **Starting Materials** | **Conditions** | **Spectra Details** |
|---|---|---|---|---|
| 28 | 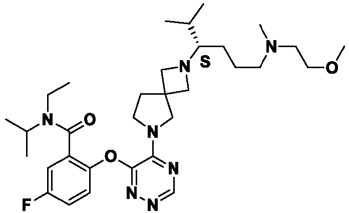 | Compound 20, 1-bromo-2-methoxyethane | K₂CO₃, NaI, DMF, 50 °C | **LC-MS (ESI) (Method 2):** Rt = 2.047 min, m/z found 600.3 [M+H]⁺. |
| | | | | **SFC (Method 11):** Rt = 5.404 min |
| 90 | 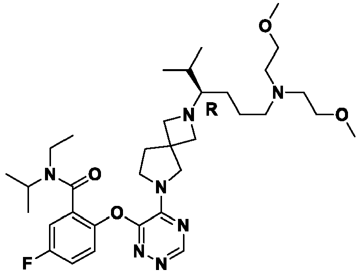 formate salt | Compound 1, 1-bromo-2-methoxyethane | Cs₂CO₃, DMF, 80 °C, microwave | **LC-MS (ESI) (Method 2):** Rt = 2.105 min, m/z found 644.4 [M+H]⁺. |
| | | | | **SFC (Method 15):** Rt = 1.105 min. |
| 93 | 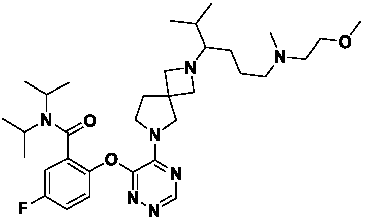 formate salt | Compound 92, 1-bromo-2-methoxyethane | K₂CO₃, NaI, DMF, 50 °C | |
| 287 | 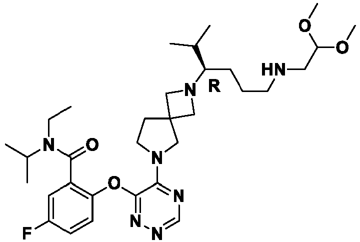 | Compound 1, 2-bromo-1,1-dimethoxyethane | K₂CO₃, NaI, DMF, 70 °C | |
| 149 | 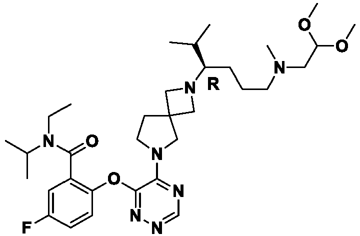 | Compound 19, 2-bromo-1,1-dimethoxyethane | K₂CO₃, KI, DMF, 80 °C | |
| 226 | 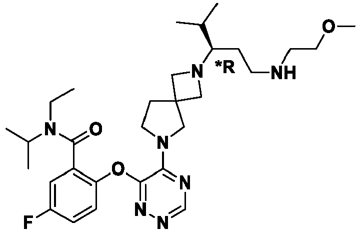 | Compound 218, 1-bromo-2-methoxyethane | DIEA, ACN, 50 °C | |
| 257 | 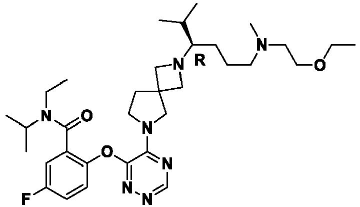 | Compound 19, 1-bromo-2-ethoxyethane | K₂CO₃, NaI, DMF, 50 °C | |
| 228 | 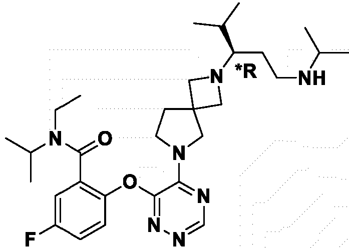 | Compound 218, 2-iodopropane | DIEA, ACN, RT | |

### Compound 12

### (R)-2-((5-(2-(6-((2-cyanoethyl)amino)-2-methylhexan-3-yl)-2,6-diazaspiro[3.4]octan-6-yl)-1,2,4-triazin-6-yl)oxy)-N-ethyl-5-fluoro-N-isopropylbenzamide

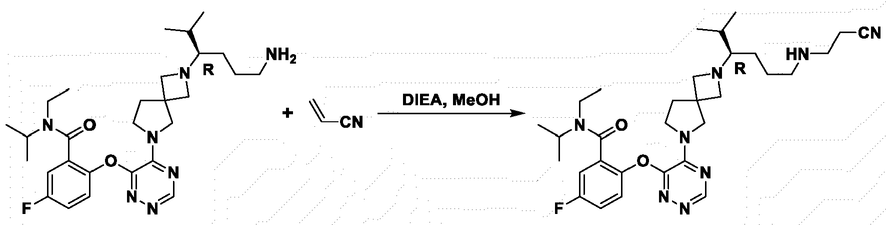

To a solution of (*R*)-2-((5-(2-(6-amino-2-methylhexan-3-yl)-2,6-diazaspiro[3.4]octan-6-yl)-1,2,4-triazin-6-yl)oxy)-N-ethyl-5-fluoro-N-isopropylbenzamide hydrochloride **(Compound 65)** (260 mg, crude) and DIEA (200 mg, 1.98 mmol) in MeOH (15 mL) was added acrylonitrile (580 mg, 10.9 mmol) at 0 °C. After addition, the reaction mixture was stirred at RT for 18 h. The reaction mixture was concentrated *in vacuo* and the residue was purified by preparative HPLC over Boston Prime (column: C18 150x30mm 5um, Mobile Phase A: water (0.04% ammonia+10mM NH₄HCO₃), Mobile Phase B: ACN, Flow rate: 25 mL/min, gradient condition B/A from 40% to 70%) to afford the title compound (120 mg) as colorless oil. **LC-MS (ESI) (Method 1):** Rₜ = 2.938 min, m/z found 581.3 [M+H]⁺.

### Compound 18, 246

### N-ethyl-5-fluoro-N-isopropyl-2-((5-(2-(2-methyl-6-((2-(methylsulfonyl)ethyl)amino)hexan-3-yl)-2,6-diazaspiro[3.4]octan-6-yl)-1,2,4-triazin-6-yl)oxy)benzamide

### (R)-2-((5-(2-(6-((3-(dimethylamino)-3-oxopropyl)(methyl)amino)-2-methylhexan-3-yl)-2,6-diazaspiro[3.4]octan-6-yl)-1,2,4-triazin-6-yl)oxy)-N-ethyl-5-fluoro-N-isopropylbenzamide formate

**The following Compounds were synthesized by an analogous method described above for Compound 12**

| **Co. No.** | **Structure** | **Starting Materials** | **Conditions** | **Spectra Details** |
|---|---|---|---|---|
| 18 | 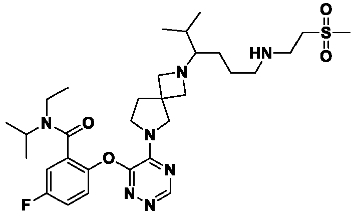 | Compound 3, (methylsulfonyl)-ethene | TEA, MeOH, RT | **LC-MS (ESI) (Method 4):** Rt = 2.24 min, m/z found 634.7 [M+H]⁺ |
| 246 | 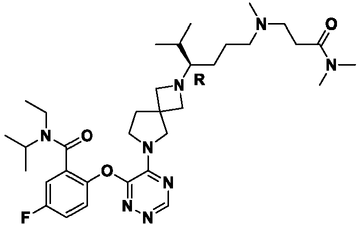 formate salt | Compound 19, *N,N-*dimethylacrylami de | TEA, MeOH, reflux | **LC-MS (ESI) (Method 5):** Rt = 1.53 min, m/z found 641.5 [M+H]⁺. |

### Compound 27

### (R)-N-ethyl-5-fluoro-N-isopropyl-2-((5-(2-(6-((2-methoxyethyl)(methyl)amino)-2-methylhexan-3-yl)-2,6-diazaspiro[3.4]octan-6-yl)-1,2,4-triazin-6-yl)oxy)benzamide

### Preparation Method A:

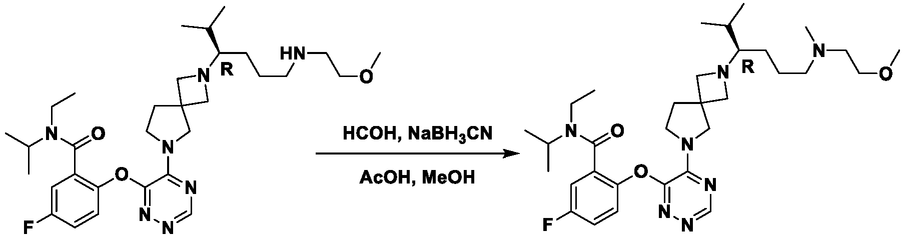

The mixture of (*R*)-*N*-ethyl-5-fluoro-*N*-isopropyl-2-((5-(2-(6-((2-methoxyethyl)amino)-2-methylhexan-3-yl)-2,6-diazaspiro[3.4]octan-6-yl)-1,2,4-triazin-6-yl)oxy)benzamide **(Compound 11)** (40.0 mg, 0.068 mmol), formaldehyde (55.4 mg, 0.683 mol, 37% in water) and AcOH (8.2 mg, 0.137 mmol) in anhydrous MeOH (2 mL) was stirred at 45 °C for 1 h. Then, NaBH₃CN (8.6 mg, 0.137 mmol) was added to the mixture and the resulting mixture was stirred at 45 °C for another 1 h. After cooling to RT, the reaction mixture was treated with sat. aq. NaHCO₃ (40 mL) to adjust the pH value to about 8 and further extracted with DCM (20 mL x 3). The combined organic layers were dried over anhydrous Na₂SO₄, filtered and concentrated under reduced pressure to give the crude which was purified by preparative HPLC over Boston Prime (column: C18 150x30mm 5um, Mobile Phase A: H₂O (0.04% ammonia+10mM NH₄HCO₃), Mobile Phase B: ACN, Flow rate: 25 mL/min, gradient condition B/A from 50% to 80% (50%B to 80% B)) to afford the title compound (9.62 mg, 99.10% purity, 23.3% yield) as yellow oil.

### Preparation Method B:

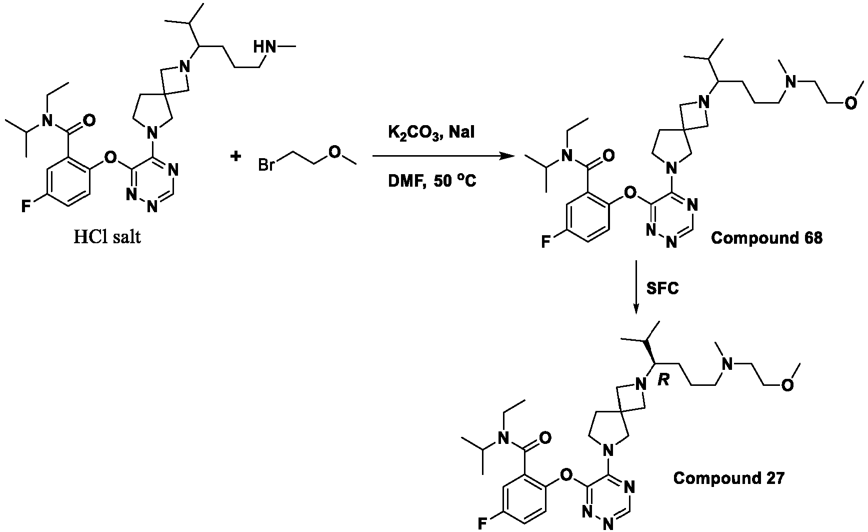

To the mixture of*N*-ethyl-5-fluoro-*N*-isopropyl-2-((5-(2-(2-methyl-6-(methylamino)hexan-3-yl)-2,6-diazaspiro[3.4]octan-6-yl)-1,2,4-triazin-6-yl)oxy)benzamide hydrochloride **(Compound** 67) (480 mg, crude), K₂CO₃ (700 mg, 5.07 mmol) and NaI (400 mg, 2.67 mmol) in DMF (5 mL) was added 1-bromo-2-methoxyethane (230 mg, 1.65 mmol). The resulting mixture was stirred at 50 °C overnight. After cooled to RT, the reaction mixture was quenched with H₂O (30 mL) and extracted with DCM (30 mL x 3). The combined organic layers were washed with brine (30 mL x 3), dried over Na₂SO₄, filtered and concentrated to give a crude residue. The residue was purified by FCC (DCM/MeOH = 10:1) to afford N-ethyl-5-fluoro-*N*-isopropyl-2-((5-(2-(6-((2-methoxyethyl)(methyl)amino)-2-methylhexan-3-yl)-2,6-diazaspiro[3.4]octan-6-yl)-1,2,4-triazin-6-yl)oxy)benzamide **(Compound 68)** (250 mg, 48% yield) as yellow oil.

The *N*-ethyl-5-fluoro-*N*-isopropyl-2-((5-(2-(6-((2-methoxyethyl)(methyl)amino)-2-methylhexan-3-yl)-2,6-diazaspiro[3.4]octan-6-yl)-1,2,4-triazin-6-yl)oxy)benzamide **(Compound 68)** (960 mg, combined from several batches obtained by Method B) was first separated by SFC using DAICEL CHIRALPAK IG (column: 250x30mm 10um; Mobile phase: A: Supercritical CO₂, B: EtOH (0.1% ammonia), A:B=40:60 at 60 mL/min) and further purified by preparative HPLC using Boston Prime (column: 150x30mm 5um, Mobile Phase A: H₂O (10mM NH₄HCO₃), Mobile Phase B: ACN, Flow rate: 25 mL/min, gradient condition B/A from 55% to 85%) to afford the title compound (270 mg) as colorless oil.

**¹H NMR (400 MHz, Methanol-*d*₄):** δ = 8.40 (s, 1H), 7.47-7.32 (m, 1H), 7.30-7.10 (m, 2H), 4.24-4.01 (m, 2H), 3.89-3.60 (m, 3H), 3.48 (br s, 3H), 2.63-2.51 (m, 2H), 2.43-2.32 (m, 2H), 2.29-2.07 (m, 6H), 1.86-1.72 (m, 1H), 1.62-1.44 (m, 2H), 1.39-1.02 (m, 10H), 0.99-0.66 (m, 9H). Some protons were hidden by the solvent peak and are not reported.

LCMS (ESI) (Method 2): Rt = 1.965 min, m/z found 600.3 [M+H]⁺.

SFC (Method 11): Rt = 4.904 min.

### Preparation Method C:

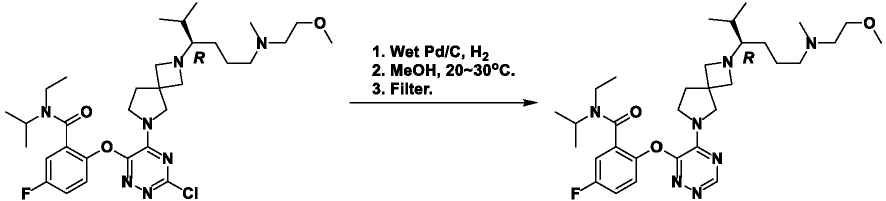

A methanol solution of (*R*)-2-((3-chloro-5-(2-(6-((2-methoxyethyl)(methyl)amino)-2-methylhexan-3-yl)-2,6-diazaspiro[3.4]octan-6-yl)-1,2,4-triazin-6-yl)oxy-*N*-ethyl-5-fluoro-*N-*isopropylbenzamide **(Compound 393)** (163.93g of a 60.1 wt % solution in MeOH, 100g corrected of **Compound 393),** palladium on carbon (10 g) and MeOH (316 g) was stirred at 20 to 30°C under a hydrogen atmosphere (0.20 to 0.30 Mpa) for 18 h. The mixture was filtered over diatomite (75 g) and the cake was washed with MeOH (158 g). The filtrate was concentrated under reduced pressure (≤ 40°C) to ~3 vol., then flushed with isopropyl acetate (IPAc, 870 g) concentrating to ~3 vol. The mixture was then diluted with IPAc (696 g) and a 20% Na₂CO₃ aqueous solution was added (500 g). The mixture was stirred for 30 to 60 min. The aqueous layer was removed. The organic layer was washed with water (500 g) then concentrated under reduced pressure <45°C to ~3 vol. The title intermediate was afforded in approximately 90% assay yield as a 48.1 wt% solution in IPAc.

### Compound 70

### (R)-N-ethyl-5-fluoro-N-isopropyl-2-((5-(2-(6-((2-methoxyethyl)(methyl)amino)-2-methylhexan-3-yl)-2,6-diazaspiro[3.4]octan-6-yl)-1,2,4-triazin-6-yl)oxy)benzamide oxalate

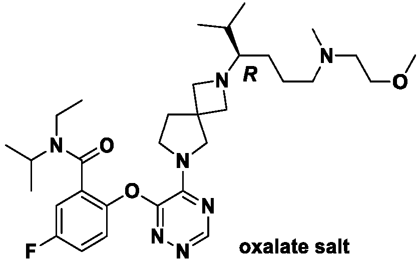

To a solution of (R)-N-ethyl-5-fluoro-N-isopropyl-2-((5-(2-(6-((2-methoxyethyl)(methyl)amino)-2-methylhexan-3-yl)-2,6-diazaspiro[3.4]octan-6-yl)-1,2,4-triazin-6-yl)oxy)benzamide **(Compound** 27) (270 mg, 0.450 mmol) in 20 mL of ACN (20 mL) was added oxalic acid (81.0 mg, 0.900 mmol). After addition, the reaction mixture was stirred at RT for 1 h. Then the reaction mixture was concentrated, the residue was redissolved in ACN and deionized water, and lyophilized to afford the title compound (350 mg) as white solid.

**¹H NMR (400 MHz, Methanol-*d*₄):** δ = 8.48 (s, 1H), 7.52-7.11 (m, 3H), 4.54-3.64 (m, 12H), 3.40-3.34 (m, 5H), 3.23-3.13 (m, 2H), 2.90 (s, 3H), 2.54-2.27 (m, 2H), 2.19-2.03 (m, 1H), 1.97-1.77 (m, 2H), 1.75-1.50 (m, 2H), 1.35-0.65 (m, 17H).

**¹H NMR (400 MHz, DMSO-*d*₆):** δ = 8.51 (s, 1H), 7.51-7.29 (m, 3H), 4.29-3.34 (m, 12H), 3.23-2.84 (m, 7H), 2.70 (s, 3H), 2.35-2.09 (m, 2H), 2.05-1.85 (m, 1H), 1.81-1.58 (m, 2H), 1.56-1.33 (m, 2H), 1.18-0.60 (m, 17H).

**LCMS (ESI) (Method 2):** Rt = 1.969 min, m/z found 600.4 [M+H]⁺.

### Preparation of Compound 70a

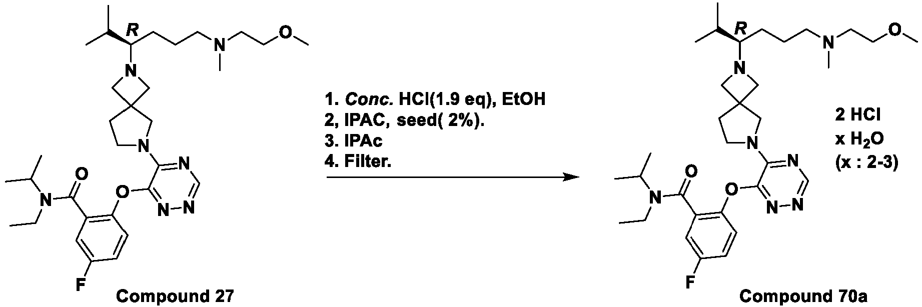

To a solution of **Compound** 27 (207.90 g of a 48 wt% solution in IPAc, 100g of active **compound** 27) in IPAc (360 g) was added EtOH (63 g) at 20 to 25°C. The solution was then treated with conc. HCl (32.9 g) in EtOH (49.5 g) over ~15 min. The mixture was seeded with crystalline **Compound** 70a seed (2 g, 2% seed load) then aged for 18 h. IPAc (870 g) was added slowly over 4 h at between 20 to 25°C and the slurry was stirred for an additional 18 h. After cooling to ~5°C, the product was filtered, washed with IPAc (522 g) and dried under vac at 20-30 °C to afford the weakly crystalline **Compound** 70a as a white solid (91.0% yield, 115.4 g). (Note: A small amount of seed material used in the reaction was obtained via an analogous reaction protocol on small-scale.)

Recrystallisation: A solution of weakly crystalline **Compound** 70a (100 g), EtOH (166 g), purified water (21.5 g) and IPAc (178 g) was stirred at 20 to 30°C for 0.5-2 h to get a clear solution. Extra IPAc (522 g) was added dropwise over 1~2 h, and then the mixture was seeded with crystalline **Compound** 70a seed (2 g, 2% seed load). Then the mixture was aged for 18 ~20 h, IPAc (348 g) was added slowly over 12 h at between 20 to 30°C, and the slurry was stirred for an additional 55~60 h. The product was filtered, washed with IPAc (158 g) and dried *in vacuo* at 20~30°C to afford **Compound** 70a as a white solid (85% yield, 85.0 g, net).

**¹HNMR (DMSO-*d*₆, 400MHz):** δ = 11.60 (1H, brs), 10.8 (1H, brs), 8.52 (1H, s), 7.36 (3H, m), 3.97-4.20 (7H, m), 3.64-3.71 (4H, m), 3.47 (7H, m), 3.25 (2H, m), 3.05 (3H, m), 2.73 (3H, s), 2.10-2.45 (1H, m), 1.99 (1H, m), 1.78 (2H, m), 1.55 (2H, m), 0.83-1.12 (12H, m), 0.70 (2H, m).

**LCMS (Method** 7): Rₜ = 0.669 min, m/z found 600.5 [M+H]⁺.
Compound 83, 84, 94, 95, 88, 89, 99, 100, 250, 251, 252, 254, 258, 396, 402
(*R)-N-ethyl-5-fluoro-N-isopropyl-2-((4-(2-(6-((2-methoxyethyl)(methyl)amino)-2-**methylhexan-3-yl)-2,6-diazaspiro[3.4]octan-6-yl)pyridazin-3-yl)oxy)benzamide oxalate**
(*S)-N-ethyl-5-fluoro-N-isopropyl-2-((4-(2-(6-((2-methoxyethyl)(methyl)amino)-2-**methylhexan-3-yl)-2,6-diazaspiro[3.4]octan-6-yl)pyridazin-3-yl)oxy)benzamide** oxalate
(*R)-5-fluoro-N,N-diisopropyl-2-((5-(2-(6-((2-methoxyethyl)(methyl)amino)-2-**methylhexan-3-yl)-2,6-diazaspiro[3.4]octan-6-yl)-1,2,4-triazin-6-yl)oxy)benzamide** oxalate
**(**S*)-5-fluoro**-***N*,*N*-diisopropyl-2-((5-(2-(6-((2-methoxyethyl)(methyl)amino)-2-methylhexan-3-yl)-2,6-diazaspiro[3.4]octan-6-yl)-1,2,4-triazin-6-yl)oxy)benzamide** oxalate
(*R)-5-fluoro-N,N-diisopropyl-2-((4-(2-(6-((2-methoxyethyl)(methyl)amino)-2-**methylhexan-3-yl)-2,6-diazaspiro[3.4]octan-6-yl)pyridazin-3-yl)oxy)benzamide** oxalate
(*S)-5-fluoro-N,N-diisopropyl-2-((4-(2-(6-((2-methoxyethyl)(methyl)amino)-2-**methylhexan-3-yl)-2,6-diazaspiro[3.4]octan-6-yl)pyridazin-3-yl)oxy)benzamide** oxalate
**(**R*)-5-fluoro-*N*,*N*-diisopropyl-2-((5-(2-(6-((2-methoxyethyl)amino)-2-methylhexan-3-yl)-**2,6-diazaspiro[3.4]octan-6-yl)-1,2,4-triazin-6-yl)oxy)benzamide oxalate
**(**S*)-5-fluoro-*N*,*N*-diisopropyl-2-((5-(2-(6-((2-methoxyethyl)amino)-2-methylhexan-3-yl)-**2,6-diazaspiro[3.4]octan-6-yl)-1,2,4-triazin-6-yl)oxy)benzamide oxalate
N-ethyl-5-fluoro-N-isopropyl-2-((5-(2-((R)-6-(((*R)-2-methoxypropyl)(methyl)amino)-2-**methylhexan-3-yl)-2,6-diazaspiro[3.4]octan-6-yl)-1,2,4-triazin-6-yl)oxy)benzamide** oxalate
N-ethyl-5-fluoro-N-isopropyl-2-((5-(2-((R)-6-(((*S)-2-methoxypropyl)(methyl)amino)-2-**methylhexan-3-yl)-2,6-diazaspiro[3.4]octan-6-yl)-1,2,4-triazin-6-yl)oxy)benzamide** oxalate
N-ethyl-5-fluoro-N-isopropyl-2-((5-(2-((3R)-6-((2-methoxypropyl)(methyl)amino)-2-**methylhexan-3-yl)-2,6-diazaspiro[3.4]octan-6-yl)-1,2,4-triazin-6-yl)oxy)benzamide** oxalate
N-ethyl-5-fluoro-N-isopropyl-2-((5-(2-((3S)-6-((2-methoxypropyl)(methyl)amino)-2-**methylhexan-3-yl)-2,6-diazaspiro[3.4]octan-6-yl)-1,2,4-triazin-6-yl)oxy)benzamide oxalate**
**(*R*)-2-((5-(2-(6-((2-ethoxyethyl)(methyl)amino)-2-methylhexan-3-yl)-2,6-diazaspiro[3.4]octan-6-yl)-1,2,4-triazin-6-yl)oxy)-*N*-ethyl-5-fluoro-*N-*isopropylbenzamide oxalate**
**(**R*)-2-((5-(2-(6-(dimethylamino)-2-methylhexan-3-yl)-2,6-diazaspiro[3.4]octan-6-yl)-1,2,4-triazin-6-yl)oxy)-5-fluoro-*N*,*N*-diisopropylbenzamide oxalate**
**(*R*)*-N-*(ethyl-¹³*C*₂)-5-fluoro-2-((5-(2-(6-((2-methoxyethyl)(methyl)amino)-2-methylhexan-3-yl)-2,6-diazaspiro[3.4]octan-6-yl)-1,2,4-triazin-6-yl)oxy)-*N*-(propan-2-yl-¹³*C*₃)benzamide oxalate**

**The following Compounds were synthesized by an analogous method described above for Compound 70**

| **Co. No.** | **Structure** | **Starting Materials** | **Spectra Details** |
|---|---|---|---|
| 83 | 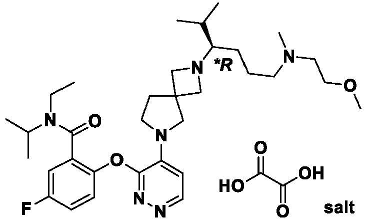 | Compound 107, oxalic acid | **LC-MS (ESI) (Method 3):** Rt = 5.034 min, m/z found 599.3 [M+H]⁺. |
| | | | **Chiral HPLC (Method 2):** Rₜ **=** 8.596 min. |
| 84 | 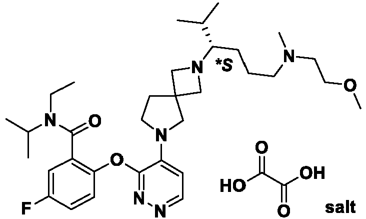 | Compound 108, oxalic acid | **LC-MS (ESI) (Method 3):** Rt = 4.957 min, m/z found 599.3 [M+H]⁺. |
| | | | **Chiral HPLC (Method 2):** Rₜ = 9.726 min. |
| 94 | 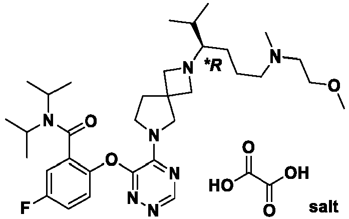 | Compound 109, oxalic acid | **LC-MS (ESI) (Method 2):** Rt = 2.431 min, m/z found 614.5 [M+H]⁺. |
| | | | **Chiral HPLC (Method 3):** Rₜ = 4.967 min. |
| 95 | 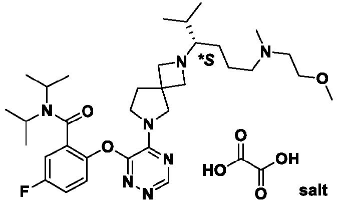 | Compound 110, oxalic acid | **LC-MS (ESI) (Method 2):** Rt = 2.471 min, m/z found 614.5 [M+H]⁺. |
| | | | **Chiral HPLC (Method 3):** Rₜ = 5.947 min. |
| 88 | 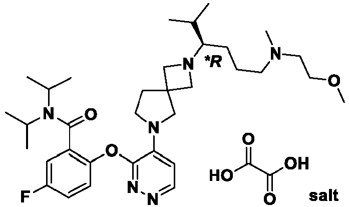 | Compound 117, oxalic acid | **LC-MS (ESI) (Method 1):** Rt = 2.243 min, m/z found 613.4 [M+H]⁺. |
| | | | **Chiral HPLC (Method 5):** Rₜ = 4.873 min. |
| 89 | 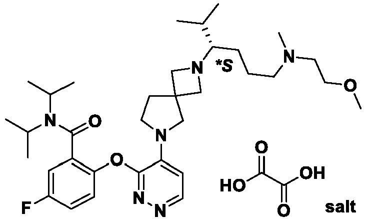 | Compound 118, oxalic acid | **LC-MS (ESI) (Method 1):** Rt = 2.271 min, m/z found 613.3 [M+H]⁺. |
| | | | **Chiral HPLC (Method 5):** Rₜ = 5.947 min. |
| 99 | 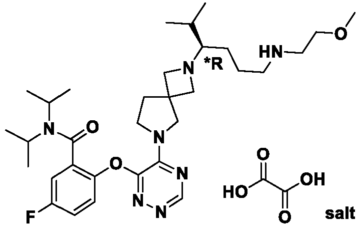 | Compound 115, oxalic acid | **LC-MS (ESI) (Method 2):** Rt = 2.224 min, m/z found 600.3 [M+H]⁺. |
| | | | **Chiral HPLC (Method 6):** Rₜ = 3.810 min. |
| 100 | 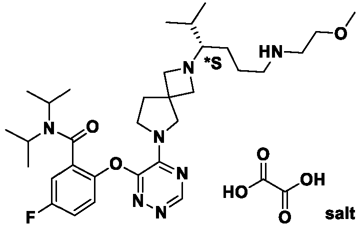 | Compound 116, oxalic acid | **LC-MS (ESI) (Method 2):** Rt = 2.21 min, m/z found 600.4 [M+H]⁺. |
| | | | **Chiral HPLC (Method 6):** Rₜ = 5.322 min. |
| 250 | 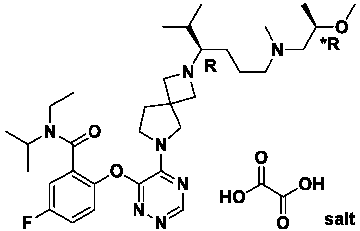 | Compound 248, oxalic acid | **LC-MS (ESI) (Method 1):** Rt = 3.107 min, m/z found 614.4 [M+H]⁺. |
| | | | **SFC (Method 16):** Rt **=** 4.082 min. |
| 251 | 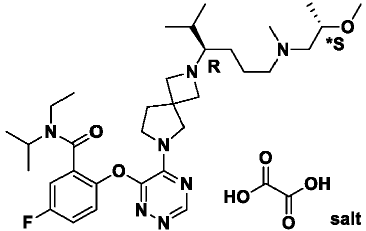 | Compound 249, oxalic acid | **LC-MS (ESI) (Method 1):** Rt = 3.141 min, m/z found 614.4 [M+H]⁺. |
| | | | **SFC (Method 16): Rt =** 4.287 min. |
| 252 | 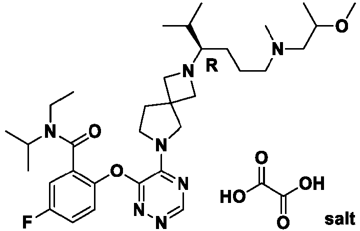 | Compound 247, oxalic acid | **LC-MS (ESI) (Method 1):** Rt = 3.011 min, m/z found 614.4 [M+H]⁺. |
| 254 | 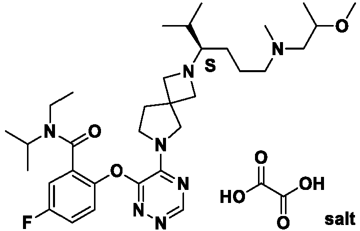 | Compound 253, oxalic acid | **LC-MS (ESI) (Method 1):** Rt = 3.054 min, m/z found 614.4 [M+H]⁺. |
| 258 | 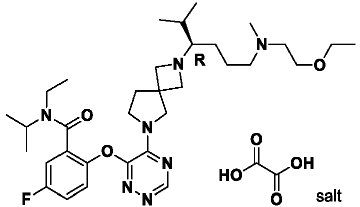 | Compound 257, oxalic acid | **LC-MS (ESI) (Method 2):** Rt = 2.047 min, m/z found 614.4 [M+H]⁺. |
| | | | **SFC (Method 16): Rt =** 4.345 min. |
| 396 | 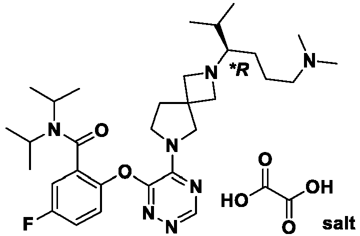 | Compound 105, oxalic acid | **LC-MS (ESI) (Method 2):** Rt = 2.071 min, m/z found 570.3 [M+H]⁺. |
| | | | **SFC (Method 4):** Rt = 1.364 min. |
| 402 | 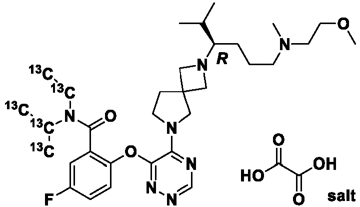 | Compound 401, oxalic acid | **LC-MS (ESI) (Method 5):** Rt = 1.500 min, m/z found 605.3 [M+H]⁺. |

**Compound 13, 16, 71, 136, 139, 153, 156, 160, 164, 166, 169, 173, 274, 275, 276, 279, 282, 285, 178, 180, 190, 192, 194, 196, 198, 200, 202, 204, 310, 311, 312, 313, 318, 329, 360, 375, 376, 379, 380, 383, 388, 411**
**(*R*)-2-((5-(2-(6-((2-cyanoethyl)(methyl)amino)-2-methylhexan-3-yl)-2,6-diazaspiro[3.4]octan-6-yl)-1,2,4-triazin-6-yl)oxy)-N-ethyl-5-fluoro-N-isopropylbenzamide**
**(*R*)-2-((5-(2-(6-((2,2-difluoroethyl)(methyl)amino)-2-methylhexan-3-yl)-2,6-diazaspiro[3.4]octan-6-yl)-1,2,4-triazin-6-yl)oxy)-N-ethyl-5-fluoro-N-isopropylbenzamide**
**(*R*)*-N*-ethyl-2-((5-(2-(6-(ethyl(2-methoxyethyl)amino)-2-methylhexan-3-yl)-2,6-diazaspiro[3.4]octan-6-yl)-1,2,4-triazin-6-yl)oxy)-5-fluoro-*N*-isopropylbenzamide**
***(R)-N-ethyl-5-fluoro-N-isopropyl-2-((5-(2-(6-((2-methoxy-2-*methylpropyl)(methyl)amino)-2-methylhexan-3-yl)-2,6-diazaspiro[3.4]octan-6-yl)-1,2,4-triazin-6-yl)oxy)benzamide**
***(R)-N-ethyl-5-fluoro-2-((5-(2-(6-((2-hydroxy-2-methylpropyl)(methyl)amino)-2-*methylhexan-3-yl)-2,6-diazaspiro[3.4]octan-6-yl)-1,2,4-triazin-6-yl)oxy)-*N-*isopropylbenzamide**
***N*-ethyl-5-fluoro-*N*-isopropyl-2-((5-(2-((*R*)-6-(((*R*)-1-methoxypropan-2-yl)(methyl)amino)-2-methylhexan-3-yl)-2,6-diazaspiro[3.4]octan-6-yl)-1,2,4-triazin-6-yl)oxy)benzamide**
***N*-ethyl-5-fluoro-*N*-isopropyl-2-((5-(2-((*R*)-6-(((*S*)-1-methoxypropan-2-yl)(methyl)amino)-2-methylhexan-3-yl)-2,6-diazaspiro[3.4]octan-6-yl)-1,2,4-triazin-6-yl)oxy)benzamide**
**(*R*)-2-((5-(2-(6-((1,3-dimethoxypropan-2-yl)(methyl)amino)-2-methylhexan-3-yl)-2,6-diazaspiro[3.4]octan-6-yl)-1,2,4-triazin-6-yl)oxy)-N-ethyl-5-fluoro-N-isopropylbenzamide**
***N*-ethyl-5-fluoro-2-((5-(2-((*R*)-6-(((*R*)-1-hydroxy-3-methoxypropan-2-yl)(methyl)amino)-2-methylhexan-3-yl)-2,6-diazaspiro[3.4]octan-6-yl)-1,2,4-triazin-6-yl)oxy)-*N-*isopropylbenzamide**
***N*-ethyl-5-fluoro-2-((5-(2-((*R*)-6-(((*S*)-1-hydroxy-3-methoxypropan-2-yl)(methyl)amino)-2-methylhexan-3-yl)-2,6-diazaspiro[3.4]octan-6-yl)-1,2,4-triazin-6-yl)oxy)-*N-*isopropylbenzamide**
***N*-ethyl-5-fluoro-2-((5-(2-((3*R*)-6-((3-hydroxy-2-methoxypropyl)(methyl)amino)-2-methylhexan-3-yl)-2,6-diazaspiro[3.4]octan-6-yl)-1,2,4-triazin-6-yl)oxy)-*N-*isopropylbenzamide**
**2-((5-(2-((3*R*)-6-((2,3-dimethoxypropyl)(methyl)amino)-2-methylhexan-3-yl)-2,6-diazaspiro[3.4]octan-6-yl)-1,2,4-triazin-6-yl)oxy)-N-ethyl-5-fluoro-N-isopropylbenzamide**
***(*R)-N-ethyl-5-fluoro-2-((5-(2-(1-((3-hydroxypropyl)(methyl)amino)-4-methylpentan-3-*yl)-2,6-diazaspiro[3.4]octan-6-yl)-1,2,4-triazin-6-yl)oxy)-*N*-isopropylbenzamide**
***(*R)-N-ethyl-5-fluoro-N-isopropyl-2-((5-(2-(1-((3-methoxypropyl)(methyl)amino)-4-*methylpentan-3-yl)-2,6-diazaspiro [3.4] octan-6-yl)-1,2,4-triazin-6-yl)oxy)benzamide**
***(*R)-N-ethyl-5-fluoro-N-isopropyl-2-((5-(2-(1-((2-methoxyethyl)(methyl)amino)-4-*methylpentan-3-yl)-2,6-diazaspiro [3.4] octan-6-yl)-1,2,4-triazin-6-yl)oxy)benzamide**
**(*R)-2-((5-(2-(1-((3-amino-3-oxopropyl)(methyl)amino)-4-methylpentan-3-yl)-2,6-diazaspiro[3.4]octan-6-yl)-1,2,4-triazin-6-yl)oxy)-*N*-ethyl-5-fluoro-*N-*isopropylbenzamide**
***N*-ethyl-5-fluoro-2-((5-(2-((*R*)-6-(((*R*)-2-hydroxy-3-methoxypropyl)(methyl)amino)-2-methylhexan-3-yl)-2,6-diazaspiro[3.4]octan-6-yl)-1,2,4-triazin-6-yl)oxy)-*N-*isopropylbenzamide**
***N*-ethyl-5-fluoro-2-((5-(2-((*R*)-6-(((*S*)-2-hydroxy-3-methoxypropyl)(methyl)amino)-2-methylhexan-3-yl)-2,6-diazaspiro[3.4]octan-6-yl)-1,2,4-triazin-6-yl)oxy)-*N-*isopropylbenzamide formate**
**2-((5-(2-((3*R*)-6-((4-(dimethylamino)-4-oxobutan-2-yl)(methyl)amino)-2-methylhexan-3-yl)-2,6-diazaspiro[3.4]octan-6-yl)-1,2,4-triazin-6-yl)oxy)-*N*-ethyl-5-fluoro-N-isopropylbenzamide**
**2-((5-(2-((3*R*)-6-((3-(dimethylamino)-2-methyl-3-oxopropyl)(methyl)amino)-2-methylhexan-3-yl)-2,6-diazaspiro[3.4]octan-6-yl)-1,2,4-triazin-6-yl)oxy)-*N*-ethyl-5-fluoro-*N*-isopropylbenzamide**
***N*-ethyl-5-fluoro-*N*-isopropyl-2-((5-(2-((*R*)-2-methyl-6-(methyl((R)-4-(methylamino)-4-oxobutan-2-yl)amino)hexan-3-yl)-2,6-diazaspiro[3.4]octan-6-yl)-1,2,4-triazin-6-yl)oxy)benzamide**
***N*-ethyl-5-fluoro-*N*-isopropyl-2-((5-(2-((*R*)-2-methyl-6-(methyl((*S*)-4-(methylamino)-4-oxobutan-2-yl)amino)hexan-3-yl)-2,6-diazaspiro[3.4]octan-6-yl)-1,2,4-triazin-6-yl)oxy)benzamide**
***N*-ethyl-5-fluoro-*N*-isopropyl-2-((5-(2-((*R*)-2-methyl-6-(methyl((*R*)-2-methyl-3-(methylamino)-3-oxopropyl)amino)hexan-3-yl)-2,6-diazaspiro[3.4]octan-6-yl)-1,2,4-triazin-6-yl)oxy)benzamide**
***N*-ethyl-5-fluoro-*N*-isopropyl-2-((5-(2-((*R*)-2-methyl-6-(methyl((*S*)-2-methyl-3-(methylamino)-3-oxopropyl)amino)hexan-3-yl)-2,6-diazaspiro [3.4]octan-6-yl)-1,2,4-triazin-6-yl)oxy)benzamide**
**2-((5-(2-((**R*)-6-(((*R*)-4-amino-4-oxobutan-2-yl)(methyl)amino)-2-methylhexan-3-yl)-2,6-diazaspiro[3.4]octan-6-yl)-1,2,4-triazin-6-yl)oxy)-5-f1uoro-*N*,*N*-diisopropylbenzamide**
**2-((5-(2-((**R*)-6-(((*S*)-4-amino-4-oxobutan-2-yl)(methyl)amino)-2-methylhexan-3-yl)-2,6-diazaspiro[3.4]octan-6-yl)-1,2,4-triazin-6-yl)oxy)-5-fluoro-*N*,*N*-diisopropylbenzamide**
**2-((5-(2-((**R*)-6-(((*R*)-3-amino-2-methyl-3-oxopropyl)(methyl)amino)-2-methylhexan-3-yl)-2,6-diazaspiro [3.4] octan-6-yl)-1,2,4-triazin-6-yl)oxy)-5-fluoro-*N*,*N-*diisopropylbenzamide**
**2-((5-(2-((**R*)-6-(((*S*)-3-amino-2-methyl-3-oxopropyl)(methyl)amino)-2-methylhexan-3-yl)-2,6-diazaspiro[3.4]octan-6-yl)-1,2,4-triazin-6-yl)oxy)-5-fluoro-*N*,*N-*diisopropylbenzamide**
**2-((5-(2-((3**R*,5**R*)-6-(dimethylamino)-5-methoxy-2-methylhexan-3-yl)-2,6-diazaspiro[3.4]octan-6-yl)-1,2,4-triazin-6-yl)oxy)-N-ethyl-5-fluoro-N-isopropylbenzamide**
***N*-ethyl-2-((5-(2-((3**R,*5**R*)-6-(ethyl(methyl)amino)-5-methoxy-2-methylhexan-3-yl)-2,6-diazaspiro[3.4]octan-6-yl)-1,2,4-triazin-6-yl)oxy)-5-fluoro-*N*-isopropylbenzamide**
**2-((5-(2-((3**R*,5**S*)-6-(dimethylamino)-5-methoxy-2-methylhexan-3-yl)-2,6-diazaspiro[3.4]octan-6-yl)-1,2,4-triazin-6-yl)oxy)-*N*-ethyl-5-fluoro-*N-*isopropylbenzamide**
***N*-ethyl-2-((5-(2-((3**R,*5 **S*)-6-(ethyl(methyl)amino)-5-methoxy-2-methylhexan-3-yl)-2,6-diazaspiro[3.4]octan-6-yl)-1,2,4-triazin-6-yl)oxy)-5-fluoro-*N*-isopropylbenzamide**
***N*-ethyl-5-fluoro-2-((5-(2-(5-hydroxy-6-(isopropyl(methyl)amino)-2-methylhexan-3-yl)-2,6-diazaspiro[3.4]octan-6-yl)-1,2,4-triazin-6-yl)oxy)-*N*-isopropylbenzamide**
**2-((5-(2-(6-(diethylamino)-5-hydroxy-2-methylhexan-3-yl)-2,6-diazaspiro[3.4]octan-6-yl)-1,2,4-triazin-6-yl)oxy)-*N*-ethyl-5-fluoro-*N*-isopropylbenzamide**
**2-((5-(2-(6-(dimethylamino)-5-hydroxy-2-methylhexan-3-yl)-2,6-diazaspiro[3.4]octan-6-yl)-1,2,4-triazin-6-yl)oxy)-5-fluoro-*N*,*N*-diisopropylbenzamide**
**2-((5-(2-((3**S*,5**S*)-6-(dimethylamino)-5-methoxy-2-methylhexan-3-yl)-2,6-diazaspiro[3.4]octan-6-yl)-1,2,4-triazin-6-yl)oxy)-*N*-ethyl-5-fluoro-*N-*isopropylbenzamide**
**2-((5-(2-((3**S*,5**R*)-6-(dimethylamino)-5-methoxy-2-methylhexan-3-yl)-2,6-diazaspiro[3.4]octan-6-yl)-1,2,4-triazin-6-yl)oxy)-*N*-ethyl-5-fluoro-*N-*isopropylbenzamide**
***N*-ethyl-2-((5-(2-((3 **S*,5**S*)-6-(ethyl(methyl)amino)-5-methoxy-2-methylhexan-3-yl)-2,6-diazaspiro[3.4]octan-6-yl)-1,2,4-triazin-6-yl)oxy)-5-fluoro-*N*-isopropylbenzamide**
***N*-ethyl-2-((5-(2-((3 **S*,5**R*)-6-(ethyl(methyl)amino)-5-methoxy-2-methylhexan-3-yl)-2,6-diazaspiro[3.4]octan-6-yl)-1,2,4-triazin-6-yl)oxy)-5-fluoro-*N*-isopropylbenzamide**
**N-ethyl-5-fluoro-2-((5-(2-(5-hydroxy-2-methyl-6-(methyl(propyl)amino)hexan-3-yl)-2,6-diazaspiro[3.4]octan-6-yl)-1,2,4-triazin-6-yl)oxy)-*N*-isopropylbenzamide**
**2-((5-(2-(6-(ethyl(methyl)amino)-5-hydroxy-2-methylhexan-3-yl)-2,6-diazaspiro[3.4]octan-6-yl)-1,2,4-triazin-6-yl)oxy)-5-fluoro-*N*,*N*-diisopropylbenzamide**
**(*R*)-2-((3-chloro-5-(2-(6-(dimethylamino)-2-methylhexan-3-yl)-2,6-diazaspiro[3.4]octan-6-yl)-1,2,4-triazin-6-yl)oxy)-*N*-ethyl-5-fluoro-*N*-isopropylbenzamide formate**

**The following Compounds were synthesized by an analogous method described above for Compound 27 by method A**

| **Co. No.** | **Structure** | **Starting Material** | **Spectra Details** |
|---|---|---|---|
| 13 | 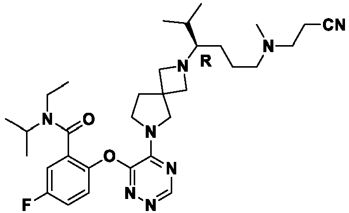 | Compound 12 | **LC-MS (ESI) (Method 1):** Rₜ = 2.897 min, m/z found 595.3 [M+H]⁺. |
| 16 | 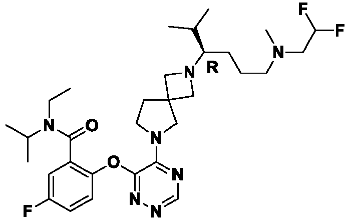 | Compound 15 | **LC-MS (ESI) (Method 2):** Rₜ = 1.893 min, m/z found 606.3 [M+H]⁺. |
| 71 | 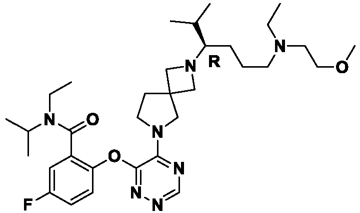 | Compound 11 | **LC-MS (ESI) (Method 2):** Rₜ = 2.002 min, m/z found 614.4 [M+H]⁺. |
| | | | **SFC (Method 6)**: Rₜ = 1.382 min. |
| 136 | 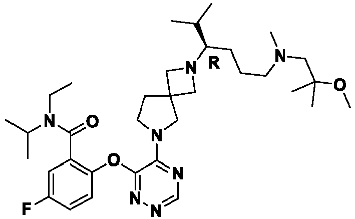 | Compound 134 | |
| 139 | 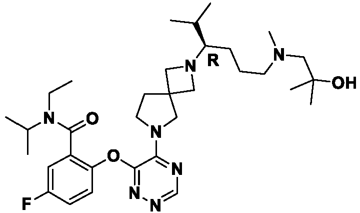 | Compound 138 | |
| 153 | 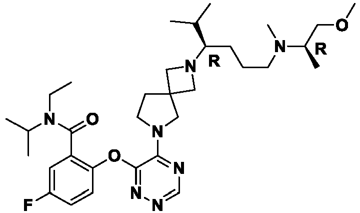 | Compound 111 | |
| 156 | 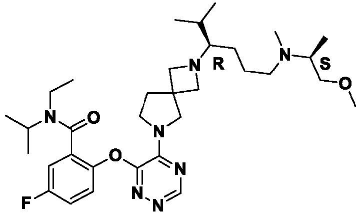 | Compoun 155 | |
| 160 | 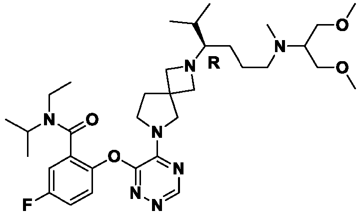 | Compound 158 | |
| 164 | 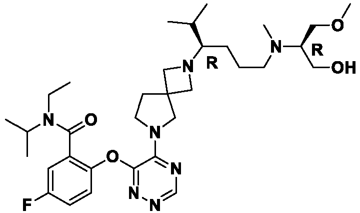 | Compound 162 | |
| 166 | 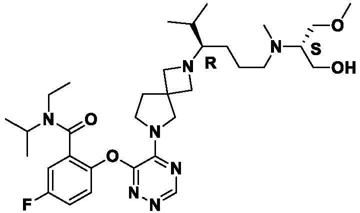 | Compound 163 | |
| 169 | 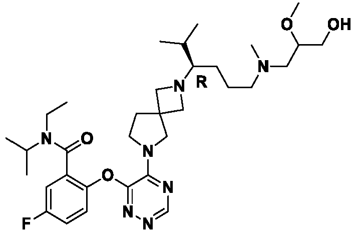 | Compound 168 | |
| 173 | 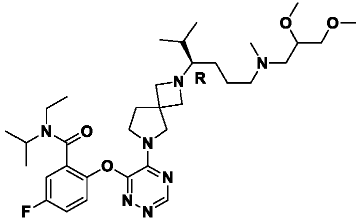 | Compound 171 | |
| 274 | 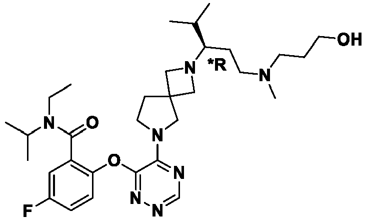 | Compound 273 | **LC-MS (ESI) (Method 1):** Rₜ = 2.969 min, m/z found 586.3 [M+H]⁺. |
| | | | **SFC (Method 13):** Rₜ = 2.031 min. |
| 275 | 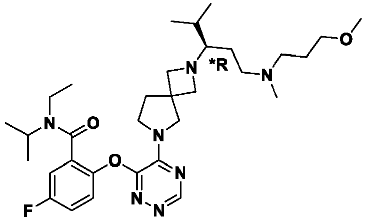 | Compound 271 | **LC-MS (ESI) (Method 2):** Rₜ = 2.031 min, m/z found 600.3 [M+H]⁺. |
| | | | **SFC (Method 3)**: Rₜ = 3.479 min. |
| 276 | 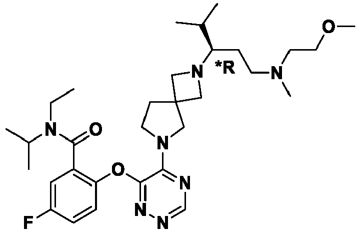 | Compound 227 | **LC-MS (ESI) (Method 6):** Rₜ = 2.98 min, m/z found 586.2 [M+H]⁺. |
| 279 | 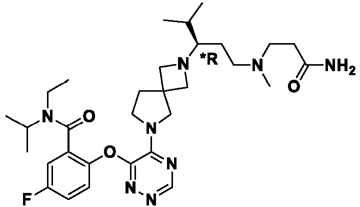 | Compound 277 | |
| 282 | 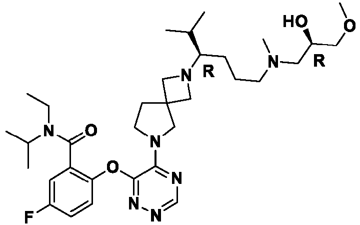 | Compound 281 | |
| 285 | 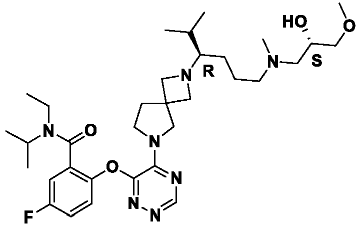 formate salt | Compound 284 | **LC-MS (ESI) (Method 3):** Rₜ = 4.980 min, m/z found 630.3 [M+H]⁺. |
| | | | **SFC (Method 13):** Rₜ = 1.993 min. |
| 178 | 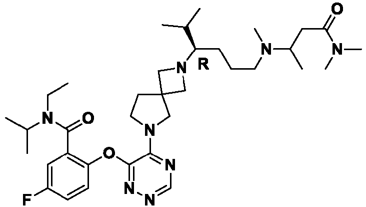 | Compound 288 | |
| 180 | 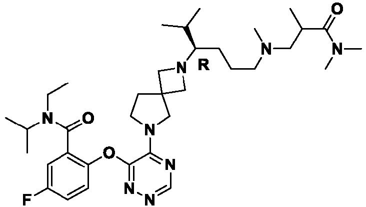 | Compound 289 | |
| 190 | 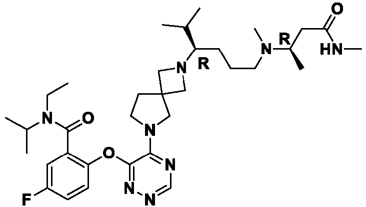 | Compound 290 | |
| 192 | 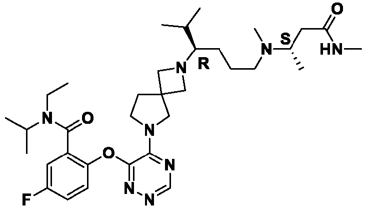 | Compound 291 | |
| 194 | 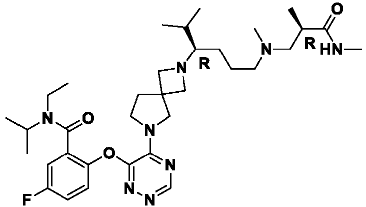 | Compound 292 | |
| 196 | 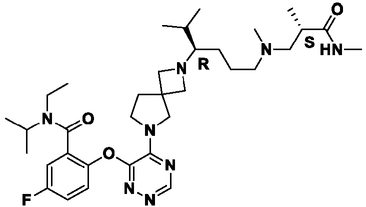 | Compound 293 | |
| 198 | 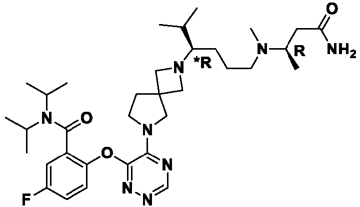 | Compound 294 | |
| 200 | 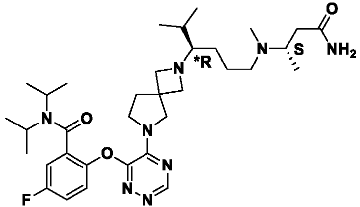 | Compound 295 | |
| 202 | 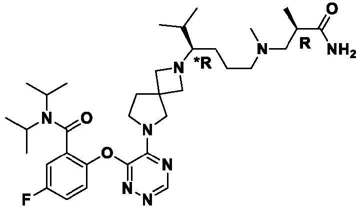 | Compound 296 | |
| 204 | 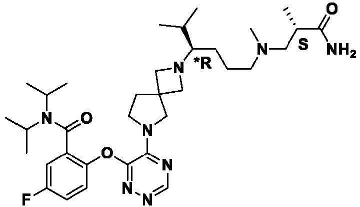 | Compound 297 | |
| 310 | 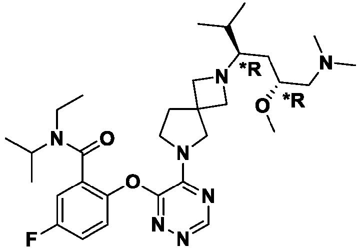 | Compound 308 | |
| 311 | 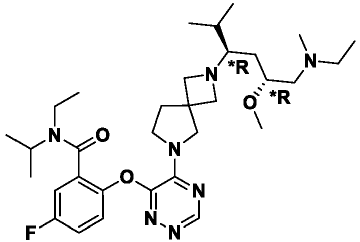 | Compound 308 | **LC-MS (ESI) (Method 2):** Rₜ = 2.032 min, m/z found 630.3 [M+H]⁺. |
| | | | **SFC (Method 24):** Rₜ = 1.955 min. |
| 312 | 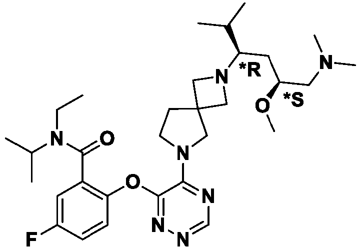 | Compound 309 | |
| 313 | 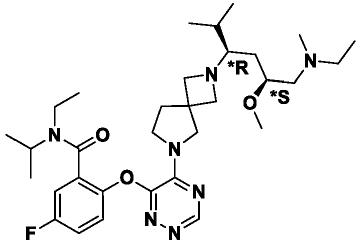 | Compound 309 | **LC-MS (ESI) (Method 2):** Rₜ = 2.048 min, m/z found 630.3 [M+H]⁺. |
| | | | **SFC (Method 24):** Rₜ = 1.937 min. |
| 318 | 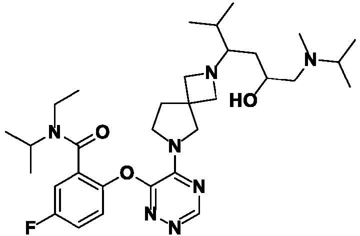 | Compound 317 | |
| 329 | 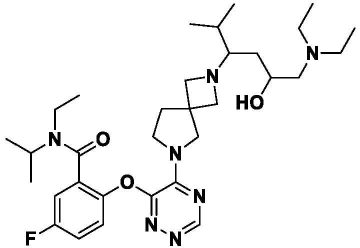 | Compound 328 | |
| 360 | 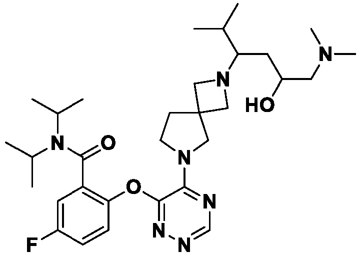 | Compound 359 | |
| 375 | 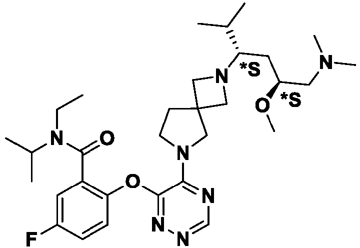 | Compound 373 | |
| 376 | 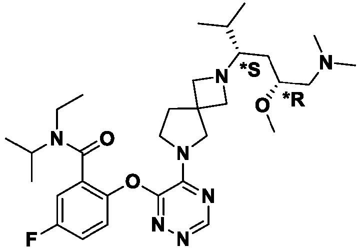 | Compound 374 | |
| 379 | 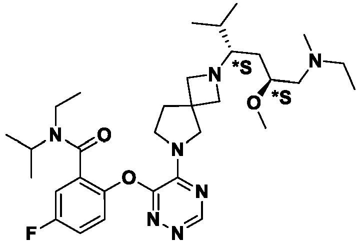 | Compound 373 | **LC-MS (ESI) (Method 2):** Rₜ = 2.039 min, m/z found 600.3 [M+H]⁺. |
| | | | **SFC (Method 24):** Rₜ = 1.907 min. |
| 380 | 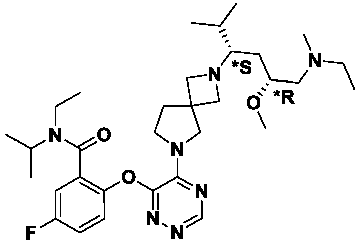 | Compound 374 | **LC-MS (ESI) (Method 2):** Rₜ = 2.047 min, m/z found 600.3 [M+H]⁺. |
| | | | **SFC (Method 24):** Rₜ = 1.922 min. |
| 383 | 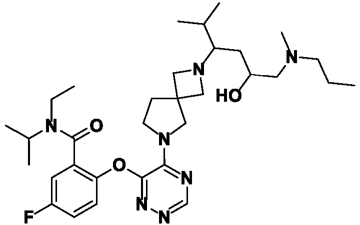 | Compound 317 | |
| 388 | 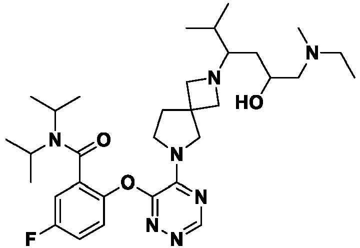 | Compound 359 | |
| 411 | 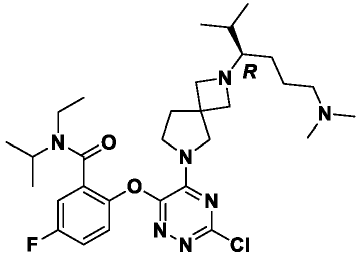 formate salt | Compound 406 | **LC-MS (ESI) (Method 2):** Rₜ = 2.376 min, m/z found 590.3 [M+H]⁺. |
| | | | **SFC (Method 13):** Rₜ = 1.823 min. |

### Compound 401, 415

### (R)-N-(ethyl-¹³C₂)-5-fluoro-2-((5-(2-(6-((2-methoxyethyl)(methyl)amino)-2-methylhexan-3-yl)-2,6-diazaspiro[3.4]octan-6-yl)-1,2,4-triazin-6-yl)oxy)-N-(propan-2-yl-¹³C₃)benzamide

### (R)-5-fluoro-N-isopropyl-2-((5-(2-(6-((2-methoxyethyl)(methyl)amino)-2-methylhexan-3-yl)-2,6-diazaspiro[3.4]octan-6-yl)-1,2,4-triazin-6-yl)oxy)-N-methylbenzamide

**The following compounds were synthesized by an analogous method described above for Compound 27 by method C**

| **Co. No.** | **Structure** | **Starting Material** | **Spectra Details** |
|---|---|---|---|
| 401 | 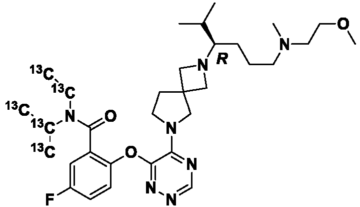 | Compound 400 | |
| 415 | 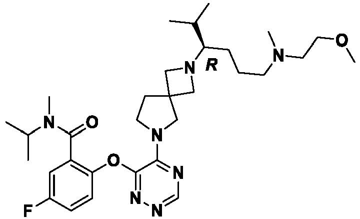 | Compound 414 | **LC-MS (ESI) (Method 1):** Rt = 2.851 min, m/z found 586.5 [M+H]⁺. |
| | | | **SFC (Method 13):** Rt = 1.772 min. |

### Compound 107, 108

### (*R)-N-ethyl-5-fluoro-N-isopropyl-2-((4-(2-(6-((2-methoxyethyl)(methyl)amino)-2-methylhexan-3-yl)-2,6-diazaspiro[3.4]octan-6-yl)pyridazin-3-yl)oxy)benzamide

### (*S)-N-ethyl-5-fluoro-N-isopropyl-2-((4-(2-(6-((2-methoxyethyl)(methyl)amino)-2-methylhexan-3-yl)-2,6-diazaspiro[3.4]octan-6-yl)pyridazin-3-yl)oxy)benzamide

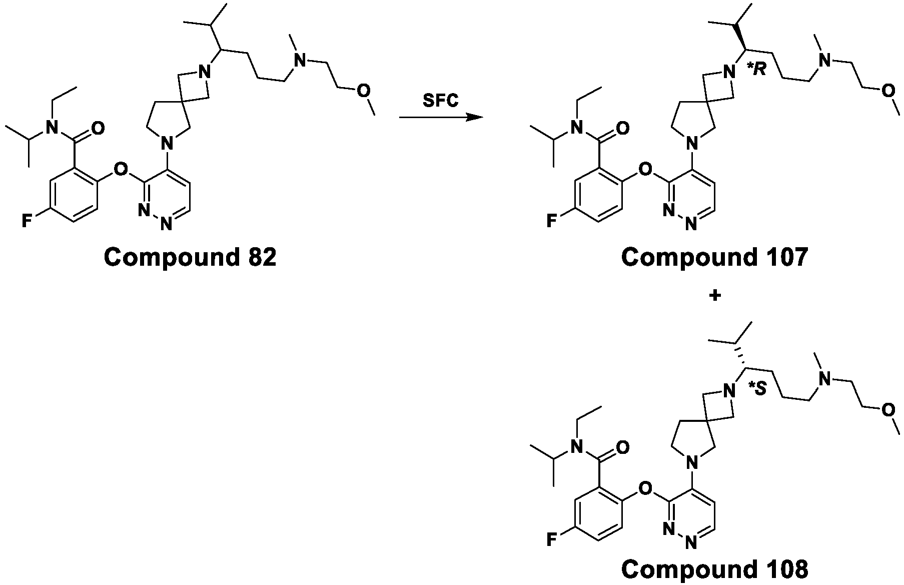

*N*-ethyl-5-fluoro-*N*-isopropyl-2-((4-(2-(6-((2-methoxyethyl)(methyl)amino)-2-methylhexan-3-yl)-2,6-diazaspiro[3.4]octan-6-yl)pyridazin-3-yl)oxy)benzamide **(Compound 82)** (47.0 mg) was purified by SFC over DAICEL CHIRALPAK IE (column: 250x30mm 10um; eluent: 100% MeOH (0.1% ammonia); flowrate: 25 ml/min) to afford the title compounds **Compound 107** (19.0 mg, 40%) and **Compound 108** (21.2 mg, 45%) as white solid.

### Compound 117, 118

### (*R)-5-fluoro-N,N-diisopropyl-2-((4-(2-(6-((2-methoxyethyl)(methyl)amino)-2-methylhexan-3-yl)-2,6-diazaspiro[3.4]octan-6-yl)pyridazin-3-yl)oxy)benzamide

### (*S)-5-fluoro-N,N-diisopropyl-2-((4-(2-(6-((2-methoxyethyl)(methyl)amino)-2-methylhexan-3-yl)-2,6-diazaspiro[3.4]octan-6-yl)pyridazin-3-yl)oxy)benzamide

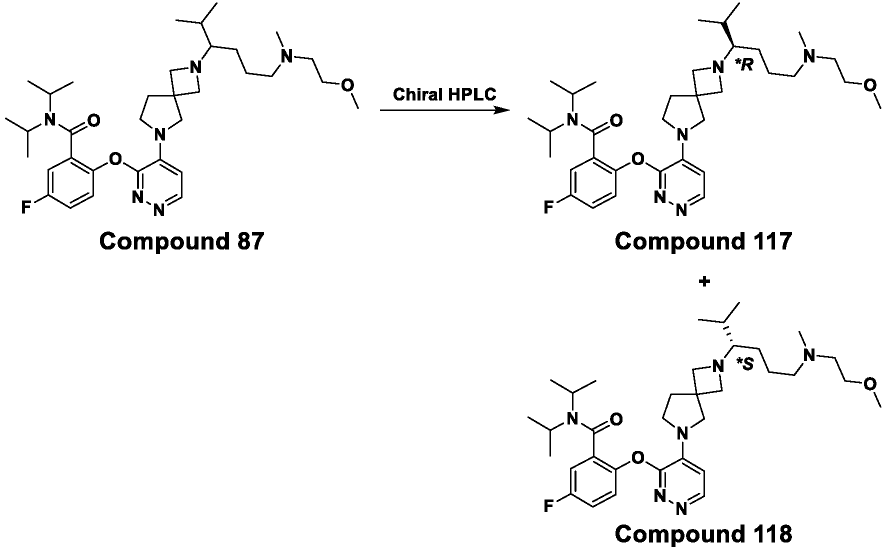

5-fluoro-*N*,*N*-diisopropyl-2-((4-(2-(6-((2-methoxyethyl)(methyl)amino)-2-methylhexan-3-yl)-2,6-diazaspiro[3.4]octan-6-yl)pyridazin-3-yl)oxy)benzamide **(Compound 87)** (300 mg) was purified by chiral HPLC over CHIRALPAK AD-H (column: 5×25 cm, 10 um; Isocratic elution: n-Hexane/EtOH/DEA =90/10/0.1 (v/v/v); Flow rate: 60 mL/min, Temperature: 35 °C) to afford the title compounds **Compound 117** (122.8 mg) and **Compound 118** (137.0 mg) both as white solid.

### Compound 109, 110

### (*R)-5-fluoro-N,N-diisopropyl-2-((5-(2-(6-((2-methoxyethyl)(methyl)amino)-2-methylhexan-3-yl)-2,6-diazaspiro[3.4]octan-6-yl)-1,2,4-triazin-6-yl)oxy)benzamide

### (*S)-5-fluoro-N,N-diisopropyl-2-((5-(2-(6-((2-methoxyethyl)(methyl)amino)-2-methylhexan-3-yl)-2,6-diazaspiro[3.4]octan-6-yl)-1,2,4-triazin-6-yl)oxy)benzamide

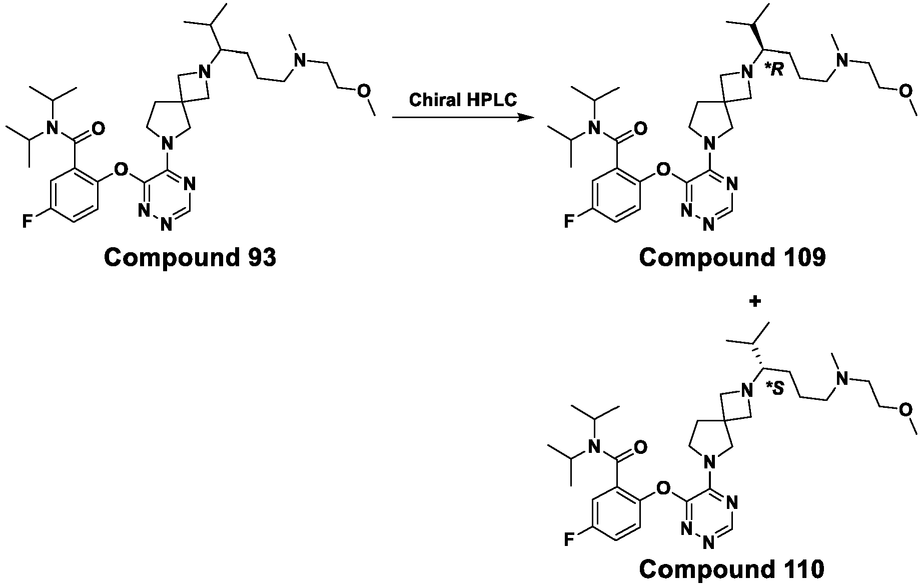

5-fluoro-*N*,*N*-diisopropyl-2-((5-(2-(6-((2-methoxyethyl)(methyl)amino)-2-methylhexan-3-yl)-2,6-diazaspiro[3.4]octan-6-yl)-1,2,4-triazin-6-yl)oxy)benzamide **(Compound** 93) (110 mg) was first separated by preparative chiral HPLC over DAICEL CHIRALPAK AD (column: 5×25 cm 10 um; Mobile phase: A: n-Hexane, B: Ethanol/DEA=10/0.1(v/v), A:B=90:10 at 60 mL/min; Column Temp: 38 °C) and further purified by preparative HPLC using Phenomenex Gemini NX (column: 75x30 mm 3um; Mobile Phase A: water (0.05% NH₃H₂O+10mM NH₄HCO₃), B: ACN, gradient from 50% B to 80% B; Flow rate: 25 mL/min) to afford the title compounds **Compound 109** (27 mg) and **Compound 110** (27 mg).

### Compound 69

### N-ethyl-5-fluoro-N-isopropyl-2-((5-(2-(6-((2-methoxy-2-methylpropyl)amino)-2-methylhexan-3-yl)-2,6-diazaspiro[3.4]octan-6-yl)-1,2,4-triazin-6-yl)oxy)benzamide

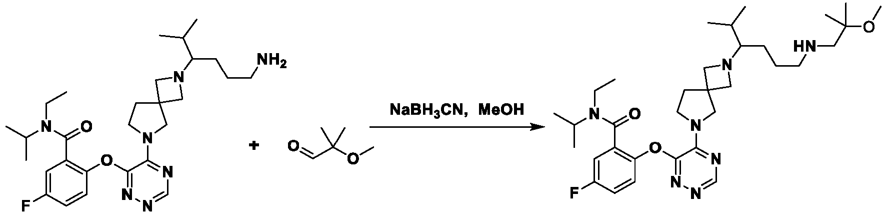

NaBH₃CN (42 mg, 0.666 mmol) was added to a mixture of 2-((5-(2-(6-amino-2-methylhexan-3-yl)-2,6-diazaspiro[3.4]octan-6-yl)-1,2,4-triazin-6-yl)oxy)-N-ethyl-5-fluoro-N-isopropylbenzamide (Compound 3) (200 mg, 0.333 mmol) and 2-methoxy-2-methylpropanal (72 mg, 0.333 mmol) in MeOH (5 mL) and the reaction mixture was stirred at RT overnight. The reaction mixture was diluted with DCM and basified with 10% aq. K₂CO₃ solution. The organic layer was decanted, filtered through Chromabond^{®} and evaporated to dryness. The residue was purified twice by chromatography over silica gel (iregular SiOH, 24g; mobile phase: gradient from 0.3% NH4OH, 3% MeOH, 97% DCM to 1% NH4OH, 10% MeOH, 90% DCM). The pure fractions were collected and evaporated to dryness to afford the title compound (68 mg, 33% yield).

LC-MS (ESI) (Method 4): Rt = 2.39 min, m/z found 614.8 [M+H]⁺.
Compound 14, 17, 255, 82, 87
*(R)-N-ethyl-5-fluoro-N-isopropyl-2-((5-(2-(2-methyl-6-((3,3,3-*trifluoropropyl)amino)hexan-3-yl)-2,6-diazaspiro [3.4] octan-6-yl)-1,2,4-triazin-6-yl)oxy)benzamide
*(R)-N-ethyl-5-fluoro-N-isopropyl-2-((5-(2-(2-methyl-6-((2,2,2-*trifluoroethyl)amino)hexan-3-yl)-2,6-diazaspiro [3.4] octan-6-yl)-1,2,4-triazin-6-yl)oxy)benzamide
**(*R*)-2-((5-(2-(6-((1,3-dihydroxypropan-2-yl)(methyl)amino)-2-methylhexan-3-yl)-2,6-**diazaspiro[3.4]octan-6-yl)-1,2,4-triazin-6-yl)oxy)-N-ethyl-5-fluoro-N-isopropylbenzamide
N-ethyl-5-fluoro-N-isopropyl-2-((4-(2-(6-((2-methoxyethyl)(methyl)amino)-2-**methylhexan-3-yl)-2,6-diazaspiro[3.4]octan-6-yl)pyridazin-3-yl)oxy)benzamide**
**5-fluoro-*N*,*N-*diisopropyl-2-((4-(2-(6-((2-methoxyethyl)(methyl)amino)-2-methylhexan-3-yl)-2,6-diazaspiro[3.4]octan-6-yl)pyridazin-3-yl)oxy)benzamide**

The following Compounds were synthesized by an analogous method described above for Compound 69

| **Ex. No.** | **Structure** | **Starting Materials** | **Conditions** | **Spectra Details** |
|---|---|---|---|---|
| 14 | 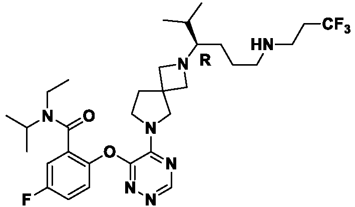 | Compound 64, 3,3,3-trifluoro-propanal | ZnCl₂, NaBH₃CN, MeOH, RT | **LC-MS (ESI) (Method 2):** Rₜ = 2.145 min, m/z found 624.3 [M+H]⁺. |
| 17 | 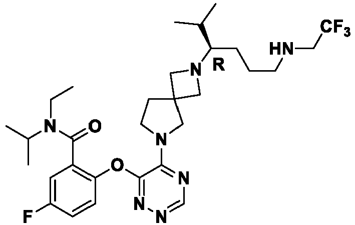 | Compound 64, 2,2,2-trifluoro-acetaldehyde | ZnCl₂, NaBH₃CN, MeOH, 80 °C | **LC-MS (ESI) (Method 2):** Rₜ = 2.085 min, m/z found 610.3 [M+H]⁺. |
| 255 | 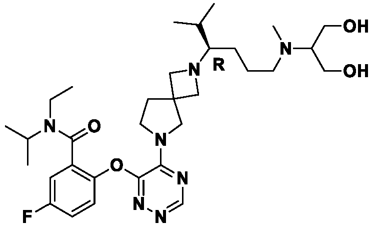 | Compound 19, 1,3-dihydroxy-propan-2-one | ZnCl₂, NaBH₃CN, MeOH, 45 °C | |
| 82 | 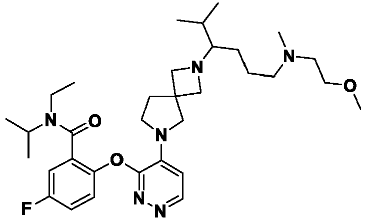 | Compound 80, 1,1,2-trimethoxy-ethane, HCl | NaBH₃CN, AcOH, EtOH | |
| 87 | 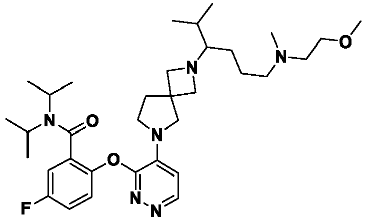 | Compound 86, 1,1,2-trimethoxyet hane, HCl | NaBH₃CN, AcOH, EtOH | |

### Compound 21

### (R)-N-ethyl-5-fluoro-N-isopropyl-2-((5-(2-(2-methyl-6-(methyl(2,2,2-trifluoroethyl)amino)hexan-3-yl)-2,6-diazaspiro[3.4]octan-6-yl)-1,2,4-triazin-6-yl)oxy)benzamide

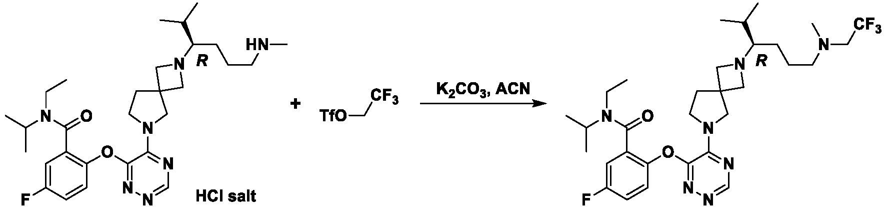

The mixture of (R)-N-ethyl-5-fluoro-N-isopropyl-2-((5-(2-(2-methyl-6-(methylamino)hexan-3-yl)-2,6-diazaspiro[3.4]octan-6-yl)-1,2,4-triazin-6-yl)oxy)benzamide hydrochloride **(Compound 19)** (50 mg, 0.086 mmol), 2,2,2-trifluoroethyl trifluoromethanesulfonate (60.2 mg, 0.259 mmol) and K₂CO₃ (112 mg, 0.865 mmol) in ACN (1 mL) was stirred at RT for 16 h. The reaction mixture was filtered and the filtrate was purified by preparative HPLC over Phenomenex Gemini-NX (column: 80x40mm 3um, Mobile Phase A: water (0.05% ammonia + 10mM NH₄HCO₃), Mobile Phase B: ACN, Flow rate: 25 mL/min, gradient condition B/A from 52% B to 82%) to afford the title compound (12.06 mg, 97% purity, 22% yield) as brown oil.

**LC-MS (ESI) (Method 2):** Rt = 2.345 min, m/z found 624.3 [M+H]⁺.
**Compound 15, 23, 247, 253**
**(*R*)-2-((5-(2-(6-((2,2-difluoroethyl)amino)-2-methylhexan-3-yl)-2,6-diazaspiro[3.4]octan-6-yl)-1,2,4-triazin-6-yl)oxy)-*N*-ethyl-5-fluoro-*N*-isopropylbenzamide**
**(*R*)-2-((5-(2-(6-((2-(dimethylamino)-2-oxoethyl)(methyl)amino)-2-methylhexan-3-yl)-2,6-**diazaspiro[3.4]octan-6-yl)-1,2,4-triazin-6-yl)oxy)-N-ethyl-5-fluoro-N-isopropylbenzamide
N-ethyl-5-fluoro-N-isopropyl-2-((5-(2-((3R)-6-((2-methoxypropyl)(methyl)amino)-2-**methylhexan-3-yl)-2,6-diazaspiro[3.4]octan-6-yl)-1,2,4-triazin-6-yl)oxy)benzamide**
N-ethyl-5-fluoro-N-isopropyl-2-((5-(2-((3S)-6-((2-methoxypropyl)(methyl)amino)-2-**methylhexan-3-yl)-2,6-diazaspiro[3.4]octan-6-yl)-1,2,4-triazin-6-yl)oxy)benzamide**

The following Compounds were synthesized by an analogous method described above for Compound 21

| **Co. No.** | **Structure** | **Starting Materials** | **Conditions** | **Spectra Details** |
|---|---|---|---|---|
| 15 | 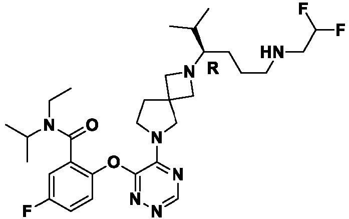 | Compound 65, 2,2-difluoroethyl trifluoromethanesulfonate | DIEA, DMF, 40 °C | **LC-MS (ESI) (Method 2):** Rt = 3.025 min, m/z found 592.3 [M+H]⁺. |
| 23 | 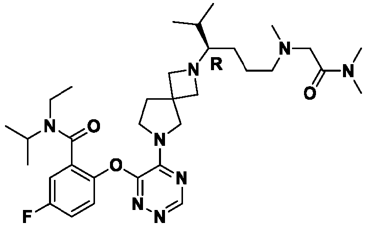 | Compound 19, 2-chloro-*N*,*N-*dimethylacetamide | K₂CO₃, MeOH | **LC-MS (ESI) (Method 1)**: Rt = 2.875 min, m/z found 627.3 [M+H]⁺. |
| 247 | 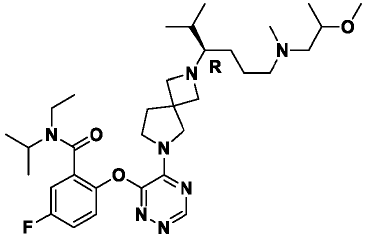 | Compound 19, intermediate 139 | Cs₂CO₃, NaI, DMF | |
| 253 | 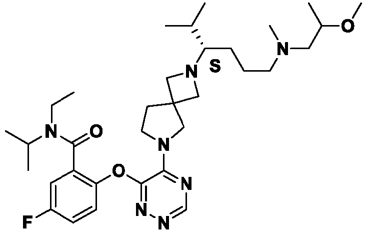 | Compound 20, intermediate 139 | Cs₂CO₃, NaI, DMF | |

### Compound 24

### (*S)-2-((5-(2-(1-amino-3-methylbutan-2-yl)-2,6-diazaspiro[3.4]octan-6-yl)-1,2,4-triazin-6-yl)oxy)-N-ethyl-5-fluoro-N-isopropylbenzamide

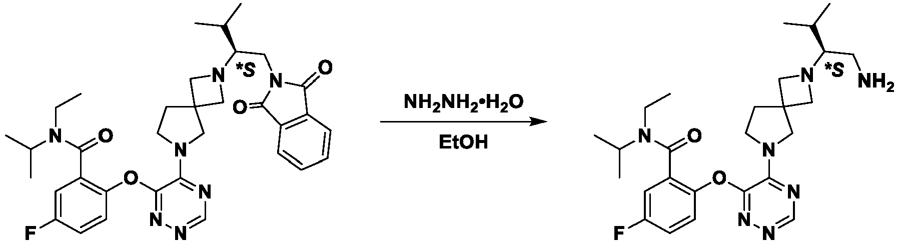

To a solution of (*S)-2-((5-(2-(1-(1,3-dioxoisoindolin-2-yl)-3-methylbutan-2-yl)-2,6-diazaspiro[3.4]octan-6-yl)-1,2,4-triazin-6-yl)oxy)-*N*-ethyl-5-fluoro-*N*-isopropylbenzamide **(intermediate 18)** (0.05 g, 0.079 mmol) in EtOH (2 mL) was added hydrazinium hydroxide (0.127 g, 3.97 mmol). The resulting mixture was stirred at 25 °C for 8 h. The reaction was concentrated under reduced pressure and the residue was purified by preparative HPLC over Boston Prime (column: C18 150x30mm 5um, Mobile Phase A: water (0.04% ammonia+10mM NH₄HCO₃), Mobile Phase B: ACN, Flow rate: 30 mL/min, gradient condition B/A from 25% to 55%) to afford the title compound (5.74 mg, 99.5% purity, 14.4% yield) as a white solid.

**LC-MS (ESI) (Method 1):** Rt = 2.94 min, m/z found 500.4 [M+H]⁺.

**SFC (Method** 7): Rt = 5.183 min.

### Compound 25

### (*R)-2-((5-(2-(1-amino-3-methylbutan-2-yl)-2,6-diazaspiro[3.4]octan-6-yl)-1,2,4-triazin-6-yl)oxy)-N-ethyl-5-fluoro-N-isopropylbenzamide

The following Compound was synthesized by an analogous method described above for Compound 24

| **Co. No.** | **Structure** | **Starting Materials** | **Spectra Details** |
|---|---|---|---|
| 25 | 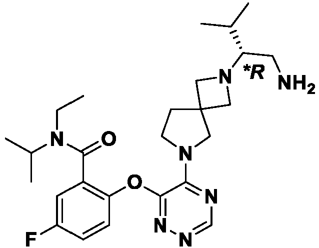 | intermediate 17 | **LC-MS (ESI) (Method 1):** Rₜ = 2.91 min, m/z found 500.4 [M+H]⁺. |
| | | | **SFC (Method 7):** Rt = 3.879 min. |

### Compound 35

### (*R)-2-((5-(2-(2,6-dimethyl-6-(methylamino)heptan-3-yl)-2,6-diazaspiro[3.4]octan-6-yl)-1,2,4-triazin-6-yl)oxy)-N-ethyl-5-fluoro-N-isopropylbenzamide hydrochloride

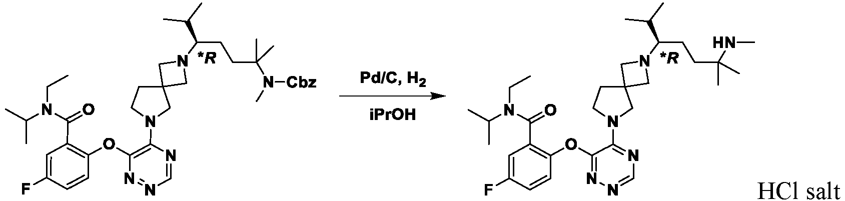

To the mixture of benzyl (*R)-(5-(6-(6-(2-(ethyl(isopropyl)carbamoyl)-4-fluorophenoxy)-1,2,4-triazin-5-yl)-2,6-diazaspiro[3.4]octan-2-yl)-2,6-dimethylheptan-2-yl)(methyl)carbamate **(intermediate 40)** (210 mg, 0.298 mmol) and HCl (18 µL, 0.22 mmol ) in i-PrOH (5 mL) was added Pd/C (20 mg, 10%) under Ar. The resulting mixture was stirred at 25 °C for 12 h under H₂ (15 PSI) atmosphere. The mixture was filtered and the filtrate was concentrated under reduced pressure to give a crude product, which was further purified by preparative HPLC over Phenomenex Gemini-NX (column: 150x30mm 5um, Mobile Phase A: H₂O (0.05% HCl), Mobile Phase B: ACN, Flow rate: 35 mL/min, gradient condition B/A from 3% to 29%) to afford the title compound (170 mg, 98% purity, 92% yield) as a white solid.

**LC-MS (ESI) (Method 2):** Rt = 2.040 min, m/z found 570.3 [M+H]⁺.

**SFC (Method** 8): Rt = 2.145 min.

### Compound 36

### (*S)-2-((5-(2-(2,6-dimethyl-6-(methylamino)heptan-3-yl)-2,6-diazaspiro[3.4]octan-6-yl)-1,2,4-triazin-6-yl)oxy)-N-ethyl-5-fluoro-N-isopropylbenzamide hydrochloride The following Compound was synthesized by an analogous method described above for

### Compound 35

| **Co. No.** | **Structure** | **Starting Materials** | **Spectra Details** |
|---|---|---|---|
| 36 | 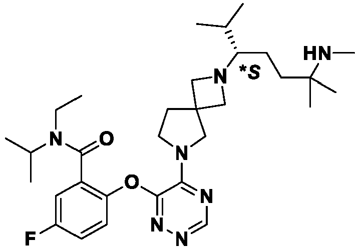 HCl salt | intermediate 41 | **LC-MS (ESI) (Method 2):** Rt = 1.970 min, m/z found 570.3 [M+H]⁺. |
| | | | **SFC (Method 8):** Rt = 2.347 min. |

### Compound 39

### 1-((((R)-4-(6-(6-(2-(ethyl(isopropyl)carbamoyl)-4-fluorophenoxy)-1,2,4-triazin-5-yl)-2,6-diazaspiro[3.4]octan-2-yl)-5-methylhexyl)carbamoyl)oxy)ethyl isobutyrate

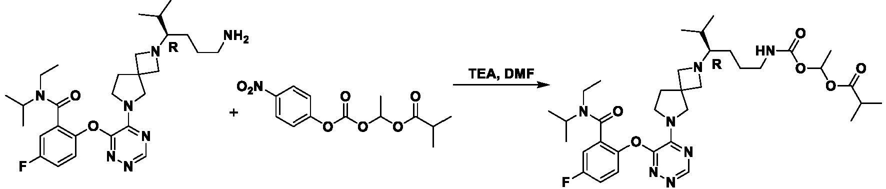

The mixture of (*R*)-2-((5-(2-(6-amino-2-methylhexan-3-yl)-2,6-diazaspiro[3.4]octan-6-yl)-1,2,4-triazin-6-yl)oxy)-*N*-ethyl-5-fluoro-*N*-isopropylbenzamide **(Compound 64)** (150 mg, crude), 1-(((4-nitrophenoxy)carbonyl)oxy)ethyl isobutyrate (102 mg, 0.343 mmol) and TEA (144 mg, 1.42 mmol) in anhydrous DMF (5 mL) was stirred at 25 °C for 2 h. The mixture was concentrated under reduced pressure to give the crude product which was further purified by preparative HPLC over Boston Prime (column: C18 150x30mm 5um, Mobile Phase A: H₂O (0.04% ammonia+10mM NH₄HCO₃), Mobile Phase B: ACN, Flow rate: 25 mL/min, gradient condition B/A from 55% to 85%) to afford the title compound (82.20 mg) as a yellow solid. **LC-MS (ESI) (Method 1):** Rt = 3.901 min, m/z found 686.3 [M+H]⁺.

### Compound 40, 41, 42

### 1-((((R)-4-(6-(6-(2-(ethyl(isopropyl)carbamoyl)-4-fluorophenoxy)-1,2,4-triazin-5-yl)-2,6-diazaspiro[3.4]octan-2-yl)-5-methylhexyl)(methyl)carbamoyl)oxy)ethyl isobutyrate

### 1-((((*R)-5-(6-(6-(2-(ethyl(isopropyl)carbamoyl)-4-fluorophenoxy)-1,2,4-triazin-5-yl)-2,6-diazaspiro[3.4]octan-2-yl)-2,6-dimethylheptan-2-yl)carbamoyl)oxy)ethyl isobutyrate formate

### 1-((((*S)-5-(6-(6-(2-(ethyl(isopropyl)carbamoyl)-4-fluorophenoxy)-1,2,4-triazin-5-yl)-2,6-diazaspiro[3.4]octan-2-yl)-2,6-dimethylheptan-2-yl)carbamoyl)oxy)ethyl isobutyrate

The following Compounds were synthesized by an analogous method described above for Compound 39

| **Co. No.** | **Structure** | **Starting Materials** | **Spectra Details** |
|---|---|---|---|
| 40 | 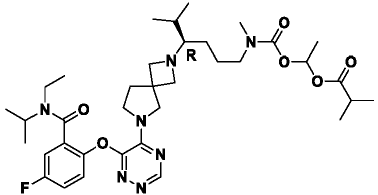 | Compound 19 | **LC-MS (ESI) (method 2)**: Rt = 2.990 min, m/z found 700.3 [M+H]⁺. |
| 41 | 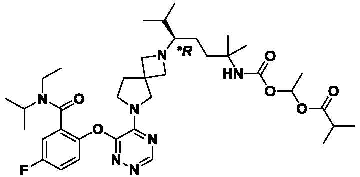 formate salt | Compound 30 | **LC-MS (ESI) (method 3)** Rt = 5.523 min, m/z found 714.3 [M+H]⁺. |
| 42 | 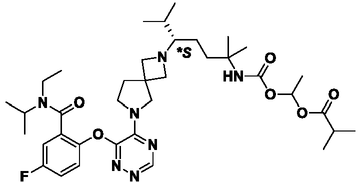 | Compound 31 | **LC-MS (ESI) (Method 3)**: Rt = 5.516 min, *m*/*z* found 714.4 [M+H]⁺. |

### Compound 43

### (*R)-4-(6-(6-(2-(4-cyclopropylpyrimidin-5-yl)-4-fluorophenoxy)-1,2,4-triazin-5-yl)-2,6-diazaspiro[3.4]octan-2-yl)-5-methylhexanamide

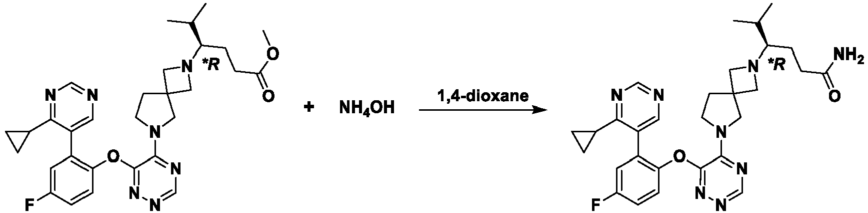

To the mixture of methyl (*R)-4-(6-(6-(2-(4-cyclopropylpyrimidin-5-yl)-4-fluorophenoxy)-1,2,4-triazin-5-yl)-2,6-diazaspiro[3.4]octan-2-yl)-5-methylhexanoate **(intermediate 48)** (110 mg, 0.178 mmol) in NH₄OH (10 mL) and 1,4-dioxane (5 mL) was added NH₄Cl (95 mg, 1.78 mmol). The resulting mixture was stirred at 40 °C for 16 h. After cooling to RT, the reaction mixture was concentrated *in vacuo* and the residue was purified by preparative HPLC using a Boston Prime (column: C18 150x30mm Sum; eluent: ACN/H₂O (0.04% ammonia+10mM NH₄HCO₃) from 30% to 60% (v/v)) to afford the title compound (34 mg, 34%) as a white solid.

**LC-MS (ESI) (Method 1):** Rₜ = 3.287 min, m/z found 547.2 [M+H]⁺.

**SFC (Method** 9): Rt = 6.275 min.

### Compound 44

**The following Compound was synthesized by an analogous method described above for Compound 43**

| **Co. No.** | **Structure** | **Starting Materials** | **Spectra Details** |
|---|---|---|---|
| 44 | 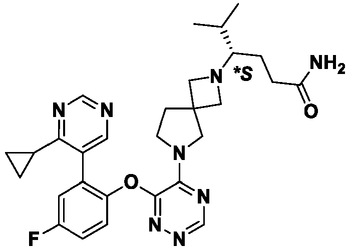 | intermediate 49 | **LC-MS (ESI) (Method 1)**: Rt = 3.292 min, m/z found 547.2 [M+H]⁺. |
| | | | **SFC (Method 9):** Rt = 7.506 min. |

### Compound 50

### 4-(6-(6-(2-(4-cyclopropylpyrimidin-5-yl)-4-fluorophenoxy)-1,2,4-triazin-5-yl)-2,6-diazaspiro[3.4]octan-2-yl)-N,5-dimethylhexanamide

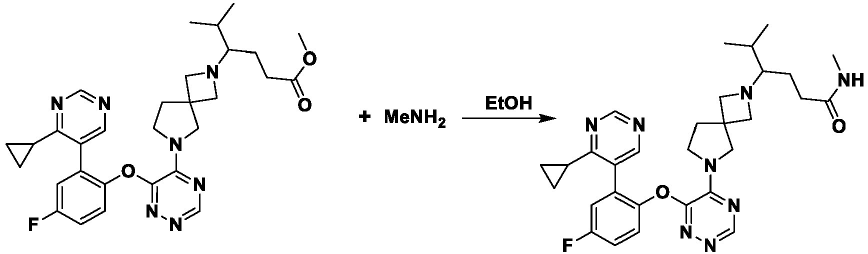

Methanamine hydrochloride (600 mg, 8.89 mmol) was added to a solution consisting of methyl 4-(6-(6-(2-(4-cyclopropylpyrimidin-5-yl)-4-fluorophenoxy)-1,2,4-triazin-5-yl)-2,6-diazaspiro[3.4]octan-2-yl)-5-methylhexanoate **(intermediate** 47) (500 mg, 0.890 mmol) in MeNH₂/EtOH (33%, 20 mL). The reaction mixture was stirred at 80 °C for 5 h. After cooling to RT, the reaction mixture was concentrated under reduced pressure to afford the crude product which was further purified by FCC (DCM/MeOH = 10:1) to afford the title compound (100 mg, 18% yield) as a yellow solid.

### Compound 45 and 46

### (*S)-4-(6-(6-(2-(4-cyclopropylpyrimidin-5-yl)-4-fluorophenoxy)-1,2,4-triazin-5-yl)-2,6-diazaspiro[3.4]octan-2-yl)-N,5-dimethylhexanamide

### (*R)-4-(6-(6-(2-(4-cyclopropylpyrimidin-5-yl)-4-fluorophenoxy)-1,2,4-triazin-5-yl)-2,6-diazaspiro[3.4]octan-2-yl)-N,5-dimethylhexanamide

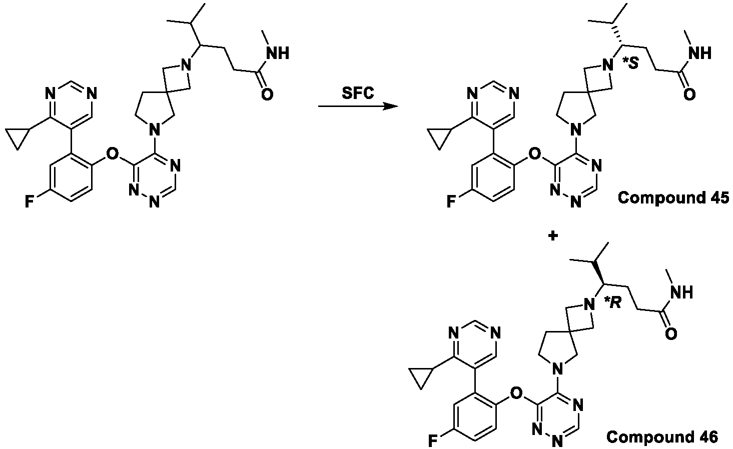

4-(6-(6-(2-(4-cyclopropylpyrimidin-5-yl)-4-fluorophenoxy)-1,2,4-triazin-5-yl)-2,6-diazaspiro[3.4]octan-2-yl)-*N*,5-dimethylhexanamide (Compound 50) (250 mg, 0.446 mmol) was purified by SFC over DAICEL CHIRALPAK AS (250x30 mm 10 um) (eluent: supercritical CO₂ in EtOH (0.1% v/v ammonia) 20/20, v/v) to afford the title compounds **Compound 45** (81.10 mg, 98% purity, 32% yield) and Compound 46 (72.53 mg, 98% purity, 28% yield) both as white solid.

### Compound 45

LC-MS (ESI) (Method 1): Rt = 3.323 min, m/z found 561.2 [M+H]⁺.
SFC (Method 10): Rₜ = 3.880 min.

### Compound 46

LC-MS (ESI) (Method 1): Rt = 3.353 min, m/z found 561.2 [M+H]⁺.
SFC (Method 10): Rₜ = 3.707 min.

### Compound 49

### N-ethyl-5-fluoro-2-((5-(2-(6-hydroxy-2-methylhexan-3-yl)-2,6-diazaspiro[3.4]octan-6-yl)-1,2,4-triazin-6-yl)oxy)-N-isopropylbenzamide

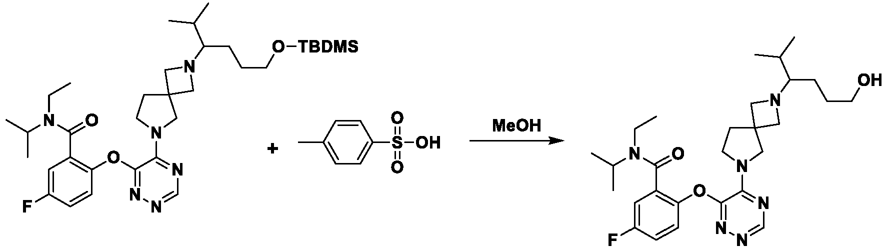

To the solution of 2-((5-(2-(6-((*tert*-butyldimethylsilyl)oxy)-2-methylhexan-3-yl)-2,6-diazaspiro[3.4]octan-6-yl)-1,2,4-triazin-6-yl)oxy)-*N*-ethyl-5-fluoro-*N*-isopropylbenzamide **(intermediate** 55) (217 mg, 0.338 mmol) in MeOH (2 mL) was added 4-methylbenzenesulfonic acid (203 mg, 1.18 mmol). The reaction mixture was stirred at RT overnight. The mixture was concentrated under reduced pressure to give the crude product which was further purified by preparative HPLC using a Phenomenex Gemini NX-C18 (column: 75x30mm 3µm; eluent: ACN/H₂O (0.04% ammonia+10mM NH₄HCO₃) from 35% to 60% (v/v)) to afford the title compound (45 mg, 25% yield) as a white solid.

### Compound 47 and 48

### (*R)-N-ethyl-5-fluoro-2-((5-(2-(6-hydroxy-2-methylhexan-3-yl)-2,6-diazaspiro[3.4]octan-6-yl)-1,2,4-triazin-6-yl)oxy)-N-isopropylbenzamide

### (*S)-N-ethyl-5-fluoro-2-((5-(2-(6-hydroxy-2-methylhexan-3-yl)-2,6-diazaspiro[3.4]octan-6-yl)-1,2,4-triazin-6-yl)oxy)-N-isopropylbenzamide

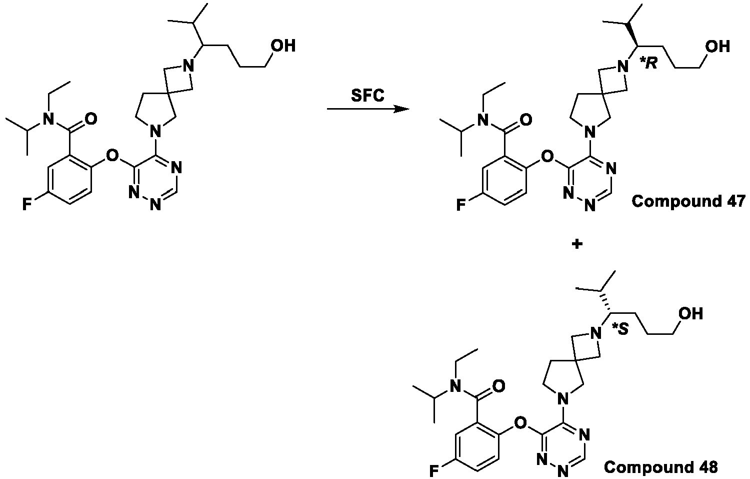

*N*-ethyl-5-fluoro-2-((5-(2-(6-hydroxy-2-methylhexan-3-yl)-2,6-diazaspiro[3.4]octan-6-yl)-1,2,4-triazin-6-yl)oxy)-*N*-isopropylbenzamide **(Compound 49)** (45.0 mg, 0.0850 mmol) was further purified by SFC over DAICEL CHIRALPAK IG (250x30mm 10um) (eluent: 40% to 40% (v/v) supercritical CO₂ in EtOH with 0.1% ammonia) to afford the title compounds **Compound 47** (17.38 mg, 39% yield) and **Compound 48** (15.79 mg, 35% yield) both as a white solid.

### Compound 47

**LCMS (ESI) (Method 1):** Rt = 3.240 min, m/z found 529.2 [M+H]⁺.
**SFC (Method** 11): Rt = 4.778 min

### Compound 48

**LCMS (ESI) (Method 1):** Rt = 3.212 min, m/z found 529.3 [M+H]⁺.
**SFC (Method** 11): Rₜ= 5.161min.

### Compound 64

### (R)-2-((5-(2-(6-amino-2-methylhexan-3-yl)-2,6-diazaspiro[3.4]octan-6-yl)-1,2,4-triazin-6-yl)oxy)-N-ethyl-5-fluoro-N-isopropylbenzamide

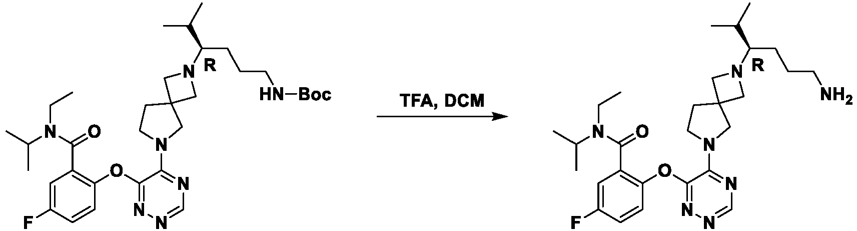

To the solution of tert-butyl (R)-(4-(6-(6-(2-(ethyl(isopropyl)carbamoyl)-4-fluorophenoxy)-1,2,4-triazin-5-yl)-2,6-diazaspiro[3.4]octan-2-yl)-5-methylhexyl)carbamate (Compound 62) (550 mg, 0.876 mmol) in DCM (4 mL) was slowly added TFA (4 mL), and the resulting mixture was stirred at 25 °C for 1 h. The reaction mixture was concentrated under reduced pressure to give a residue. The residue was diluted in DCM (40 mL) and the pH value was adjusted to around 12 by aq. NaOH (2 M, 16 mL) solution. The aqueous layer was extracted with DCM (10 mL x 2). The combined organic layers were dried over anhydrous Na₂SO₄, filtered and concentrated *in vacuo* to afford the title compound (460 mg, crude) as yellow solid, which was used directly in next step without further purification.

### Compound 97

### 2-((5-(2-(6-amino-2-methylhexan-3-yl)-2,6-diazaspiro[3.4]octan-6-yl)-1,2,4-triazin-6-yl)oxy)-5-fluoro-N,N-diisopropylbenzamide

The following compound was synthesized by an analogous method as described above for Compound 64

| **Co. No.** | **Structure** | **Starting Material** |
|---|---|---|
| 97 | 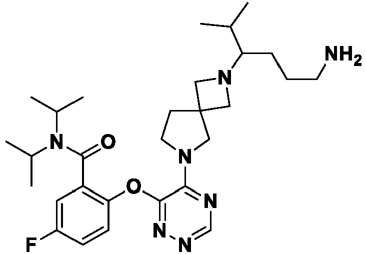 | Compound 96 |

### Compound 65

### (R)-2-((5-(2-(6-amino-2-methylhexan-3-yl)-2,6-diazaspiro[3.4]octan-6-yl)-1,2,4-triazin-6-yl)oxy)-N-ethyl-5-fluoro-N-isopropylbenzamide hydrochloride

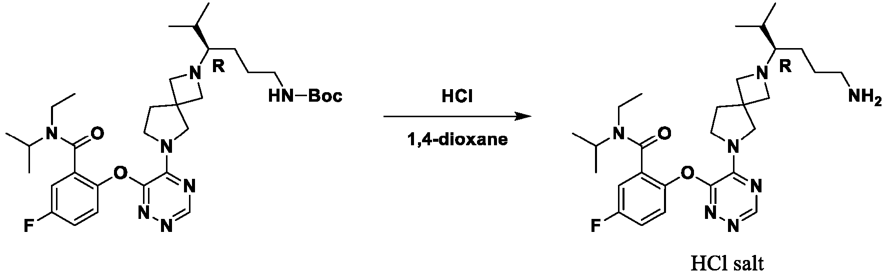

To the solution of tert-butyl (R)-(4-(6-(6-(2-(ethyl(isopropyl)carbamoyl)-4-fluorophenoxy)-1,2,4-triazin-5-yl)-2,6-diazaspiro[3.4]octan-2-yl)-5-methylhexyl)carbamate **(Compound** 62) (250 mg, 0.398 mmol) in 1,4-dioxane (5 mL) was added a solution of 4M HCl in dioxane (10 mL, 40 mmol), the resulting mixture was stirred at RT for 16 h. The reaction mixture was concentrated *in vacuo* to afford the title compound (220 mg, crude, HCl salt) as yellow oil, which was used directly in next step without further purification.

### Compound 67

### N-ethyl-5-fluoro-N-isopropyl-2-((5-(2-(2-methyl-6-(methylamino)hexan-3-yl)-2,6-diazaspiro[3.4]octan-6-yl)-1,2,4-triazin-6-yl)oxy)benzamide hydrochloride

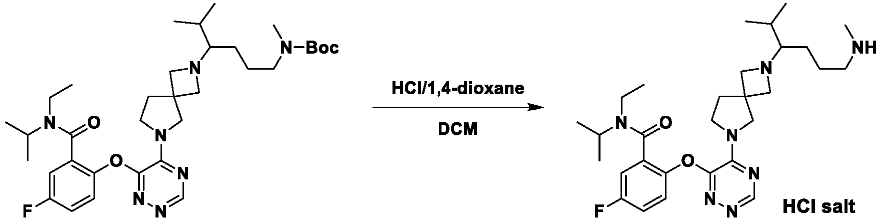

To a solution of tert-butyl (4-(6-(6-(2-(ethyl(isopropyl)carbamoyl)-4-fluorophenoxy)-1,2,4-triazin-5-yl)-2,6-diazaspiro[3.4]octan-2-yl)-5-methylhexyl)(methyl)carbamate (Compound 60) (1 g, 1.56 mmol) in DCM (10 mL) was added 4M HCl in dioxane (5 mL, 20 mmol), the resulting mixture was stirred at RT for 1 h. The reaction mixture was concentrated *in vacuo* to afford the title compound (960 mg, crude, HCl salt) which was used directly in next step without further purification.

### Compound 66, 73, 92

### (S)-2-((5-(2-(6-amino-2-methylhexan-3-yl)-2,6-diazaspiro[3.4]octan-6-yl)-1,2,4-triazin-6-yl)oxy)-N-ethyl-5-fluoro-N-isopropylbenzamide hydrochloride

### 2-((4-(2-(6-amino-2-methylhexan-3-yl)-2,6-diazaspiro[3.4]octan-6-yl)pyridazin-3-yl)oxy)-5-fluoro-N,N-diisopropylbenzamide hydrochloride

### 5-fluoro-N,N-diisopropyl-2-((5-(2-(2-methyl-6-(methylamino)hexan-3-yl)-2,6-diazaspiro[3.4]octan-6-yl)-1,2,4-triazin-6-yl)oxy)benzamide hydrochloride

The following compounds were synthesized by an analogous method as described above for Compound 65 and Compound 67

| **Co. No.** | **Structure** | **Starting Material** |
|---|---|---|
| 66 | 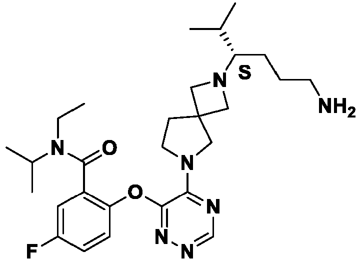 HCl salt | Compound 63 |
| 73 | 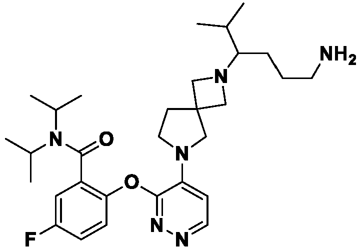 HCl salt | Compound 72 |
| 92 | 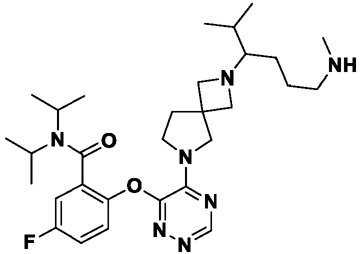 HCl salt | Compound 91 |

### Compound 86

### 5-fluoro-N,N-diisopropyl-2-((4-(2-(2-methyl-6-(methylamino)hexan-3-yl)-2,6-diazaspiro[3.4]octan-6-yl)pyridazin-3-yl)oxy)benzamide

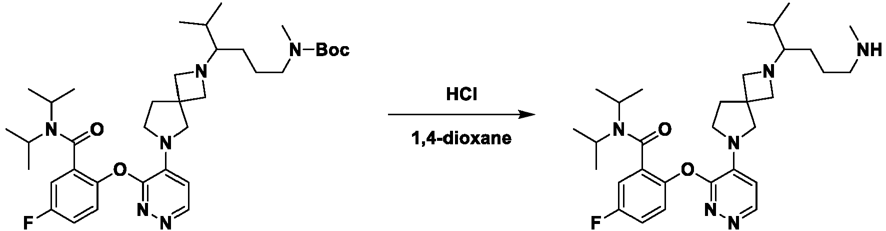

To the solution of *tert*-butyl (4-(6-(3-(2-(diisopropylcarbamoyl)-4-fluorophenoxy)pyridazin-4-yl)-2,6-diazaspiro[3.4]octan-2-yl)-5-methylhexyl)(methyl)carbamate **(Compound 85)** (1.0 g, 1.5 mmol) in 1,4-dioxane (10 mL) cooled at 0 °C was added a solution of 4M HCl in 1,4-dioxane (5 mL, 20 mmol) in portions. The resulting mixture was slowly warmed to 25 °C and stirred for 2 h. The reaction mixture was concentrated under reduced pressure to give a residue, which was re-dissolved in DCM (30 mL). Then, 1 M NaOH (20 mL) was added to adjust the pH value to about 12. The resulting mixture was further extracted with DCM (30 mL x 3). The combined organic layers were dried over anhydrous Na₂SO₄, filtered, and concentrated *in vacuo* to afford the title compound (1.26 g, crude) as a yellow solid, which was used directly in next step without further purification.
**Compound 58, 59, 213, 234, 235, 260, 303, 79, 85, 91, 72, 96, 206, 316, 327, 338, 339, 348, 349, 358, 381, 399, 403**
***tert-butyl* (5-(6-(6-(2-(4-cyclopropylpyrimidin-5-yl)-4-fluorophenoxy)-1,2,4-triazin-5-yl)-2,6-diazaspiro[3.4]octan-2-yl)-2,6-dimethylheptan-2-yl)carbamate**
***tert-butyl* (5-(6-(6-(2-(ethyl(isopropyl)carbamoyl)-4-fluorophenoxy)-1,2,4-triazin-5-yl)-2,6-diazaspiro[3.4]octan-2-yl)-2,6-dimethylheptan-2-yl)carbamate**
**N-ethyl-5-fluoro-2-((5-(2-(1-hydroxy-4-methylpentan-3-yl)-2,6-diazaspiro[3.4]octan-6-yl)-1,2,4-triazin-6-yl)oxy)-*N*-isopropylbenzamide**
***N*-ethyl-5-fluoro-*N*-isopropyl-2-((5-(2-((5 *R)-6-((2-methoxyethyl)(methyl)amino)-2,5-dimethylhexan-3-yl)-2,6-diazaspiro[3.4]octan-6-yl)-1,2,4-triazin-6-yl)oxy)benzamide**
***N*-ethyl-5-fluoro-*N*-isopropyl-2-((5-(2-((*5*S*)-6-((2-methoxyethyl)(methyl)amino)-2,5-dimethylhexan-3-yl)-2,6-diazaspiro[3.4]octan-6-yl)-1,2,4-triazin-6-yl)oxy)benzamide**
***N*-ethyl-5-fluoro-2-((5-(2-(6-hydroxy-2,4-dimethylhexan-3-yl)-2,6-diazaspiro[3.4]octan-6-yl)-1,2,4-triazin-6-yl)oxy)-*N*-isopropylbenzamide**
***tert-butyl* (4-(6-(6-(2-(ethyl(isopropyl)carbamoyl)-4-fluorophenoxy)-1,2,4-triazin-5-yl)-2,6-diazaspiro[3.4]octan-2-yl)-2-methoxy-5-methylhexyl)(methyl)carbamate**
***tert-butyl* (4-(6-(3-(2-(ethyl(isopropyl)carbamoyl)-4-fluorophenoxy)pyridazin-4-yl)-2,6-diazaspiro[3.4]octan-2-yl)-5-methylhexyl)(methyl)carbamate**
***tert-butyl* (4-(6-(3-(2-(diisopropylcarbamoyl)-4-fluorophenoxy)pyridazin-4-yl)-2,6-diazaspiro[3.4]octan-2-yl)-5-methylhexyl)(methyl)carbamate**
***tert-butyl* (4-(6-(6-(2-(diisopropylcarbamoyl)-4-fluorophenoxy)-1,2,4-triazin-5-yl)-2,6-diazaspiro[3.4]octan-2-yl)-5-methylhexyl)(methyl)carbamate**
***tert-butyl* (4-(6-(3-(2-(diisopropylcarbamoyl)-4-fluorophenoxy)pyridazin-4-yl)-2,6-diazaspiro[3.4]octan-2-yl)-5-methylhexyl)carbamate**
***tert-butyl* (4-(6-(6-(2-(diisopropylcarbamoyl)-4-fluorophenoxy)-1,2,4-triazin-5-yl)-2,6-diazaspiro[3.4]octan-2-yl)-5-methylhexyl)carbamate**
***tert-butyl* (5-(6-(6-(2-(ethyl(isopropyl)carbamoyl)-4-fluorophenoxy)-1,2,4-triazin-5-yl)-2,6-diazaspiro[3.4]octan-2-yl)-6-methylheptyl)carbamate**
***tert-butyl* (4-(6-(6-(2-(ethyl(isopropyl)carbamoyl)-4-fluorophenoxy)-1,2,4-triazin-5-yl)-2,6-diazaspiro[3.4]octan-2-yl)-2-hydroxy-5-methylhexyl)(methyl)carbamate**
***tert-butyl* ethyl(4-(6-(6-(2-(ethyl(isopropyl)carbamoyl)-4-fluorophenoxy)-1,2,4-triazin-5-yl)-2,6-diazaspiro[3.4]octan-2-yl)-2-hydroxy-5-methylhexyl)carbamate**
***N*-ethyl-2-((5-(2-((5*S)*-6-(ethyl(methyl)amino)-5-hydroxy-2-methylhexan-3-yl)-2,6-diazaspiro[3.4]octan-6-yl)-1,2,4-triazin-6-yl)oxy)-5-fluoro-*N*-isopropylbenzamide**
***N*-ethyl-2-((5-(2-((5R)-6-(ethyl(methyl)amino)-5-hydroxy-2-methylhexan-3-yl)-2,6-diazaspiro[3.4]octan-6-yl)-1,2,4-triazin-6-yl)oxy)-5-fluoro-*N*-isopropylbenzamide**
***N*-ethyl-5-fluoro-2-((5-(2-((5S)-5-hydroxy-6-((2-methoxyethyl)(methyl)amino)-2-methylhexan-3-yl)-2,6-diazaspiro[3.4]octan-6-yl)-1,2,4-triazin-6-yl)oxy)-*N-*isopropylbenzamide**
***N*-ethyl-5-fluoro-2-((5-(2-((5R)-5-hydroxy-6-((2-methoxyethyl)(methyl)amino)-2-methylhexan-3-yl)-2,6-diazaspiro[3.4]octan-6-yl)-1,2,4-triazin-6-yl)oxy)-*N* isopropylbenzamide**
***tert-butyl* (4-(6-(6-(2-(diisopropylcarbamoyl)-4-fluorophenoxy)-1,2,4-triazin-5-yl)-2,6-diazaspiro[3.4]octan-2-yl)-2-hydroxy-5-methylhexyl)(methyl)carbamate**
***N*-ethyl-5-fluoro-2-((5-(2-(5-hydroxy-6-((2-methoxyethyl)(methyl)amino)-2,5-dimethylhexan-3-yl)-2,6-diazaspiro[3.4]octan-6-yl)-1,2,4-triazin-6-yl)oxy)-*N-*isopropylbenzamide**
***N*-ethyl-5-fluoro-N-isopropyl-2-((5-(2-(6-((2-methoxyethyl)(methyl)amino)-2-methylhexan-3-yl-3-*d*)-2,6-diazaspiro[3.4]octan-6-yl)-1,2,4-triazin-6-yl)oxy)benzamide**
***tert-butyl* (4-(6-(3-chloro-6-(2-(ethyl(isopropyl)carbamoyl)-4-fluorophenoxy)-1,2,4-triazin-5-yl)-2,6-diazaspiro[3.4]octan-2-yl)-5-methylhexyl)carbamate**

**The following compounds were synthesized by an analogous method as described for Compound 60 and Compound 61**

| **Co. No.** | **Structure** | **Starting Material** | **Conditions** |
|---|---|---|---|
| 58 | 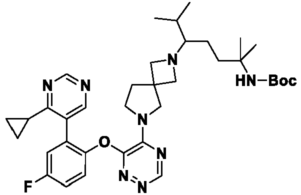 | intermediate 59, intermediate 30 | ZnCl₂, NaBH₃CN, MeOH, 65 °C |
| 59 | 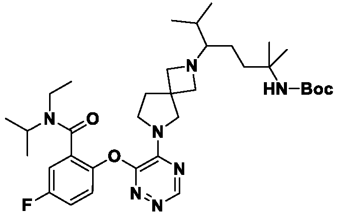 | intermediate 3, intermediate 30 | ZnCl₂, NaBH₃CN, MeOH, 65 °C |
| 213 | 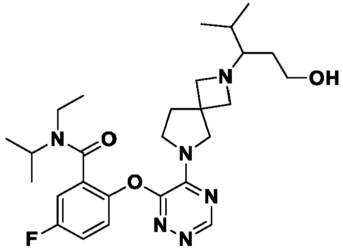 | intermediate 3, intermediate 116 | AcOH, NaBH₃CN, MeOH, RT |
| 234 | 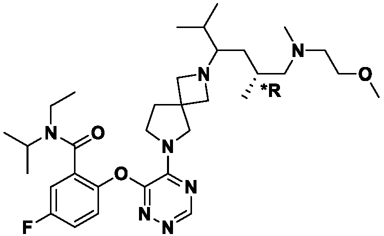 | intermediate 3, intermediate 135 | ZnCl₂, NaBH₃CN, MeOH, 60 °C |
| 235 | 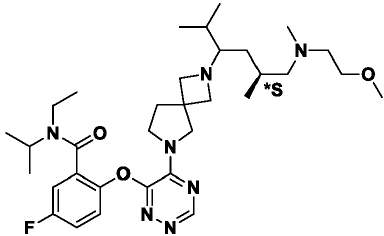 | intermediate 3, intermediate 136 | ZnCl₂, NaBH₃CN, MeOH, 60 °C |
| 260 | 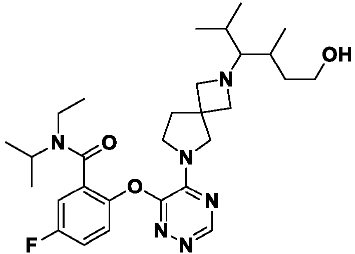 | intermediate 3, intermediate 141 | Ti(OiPr)₄, NaBH₃CN, MeOH, 80 °C |
| 303 | 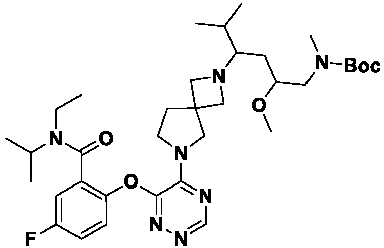 | intermediate 244, intermediate 166 | NaOAc, NaBH₃CN, MeOH, RT |
| 79 | 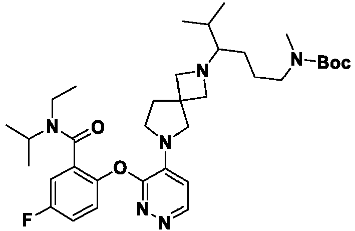 | intermediate 9, intermediate 75 | ZnCl₂, NaBH₃CN, MeOH, 80 °C |
| 85 | 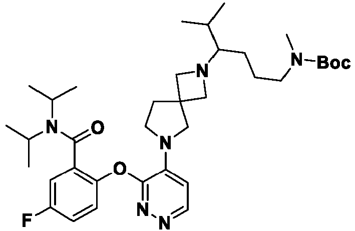 | intermediate 9, intermediate 71 | AcOH, NaBH₃CN, MeOH, 70 °C |
| 91 | 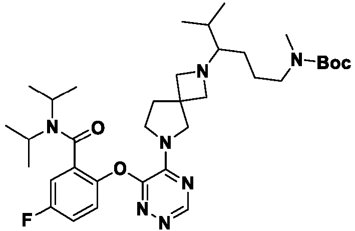 | intermediate 9, intermediate 85 | ZnCl₂, NaBH₃CN, MeOH, 80 °C |
| 72 | 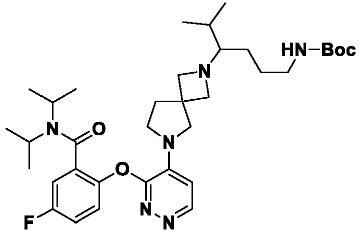 | intermediate 1, intermediate 71 | AcOH, NaBH₃CN, MeOH, 80 °C |
| 96 | 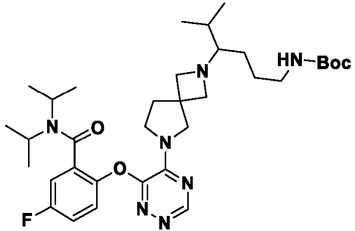 | intermediate 1, intermediate 85 | ZnCl₂, NaBH₃CN, MeOH, 80 °C |
| 206 | 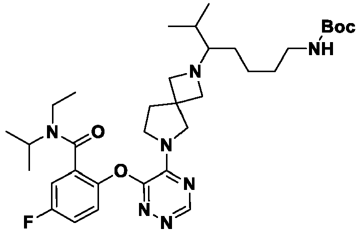 | intermediate 110, intermediate 3 | ZnCl₂, NaBH₃CN, MeOH, 65 °C |
| 316 | 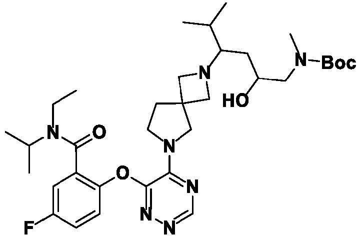 | intermediate 165, intermediate 244 | NaOAc, NaBH₃CN, MeOH, 26 °C |
| 327 | 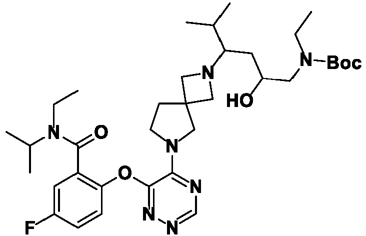 | intermediate 174, intermediate 244 | NaOAc, NaBH₃CN, MeOH, RT |
| 338 | 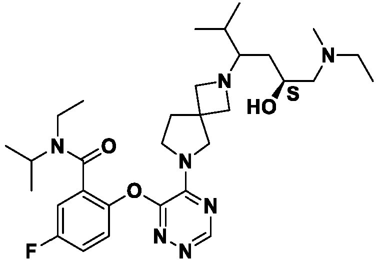 | intermediate 195, intermediate 244 | NaOAc, NaBH₃CN, MeOH, RT |
| 339 | 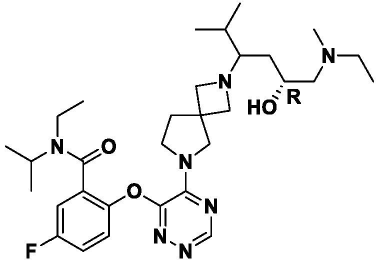 | intermediate 196, intermediate 244 | NaOAc, NaBH₃CN, MeOH, RT |
| 348 | 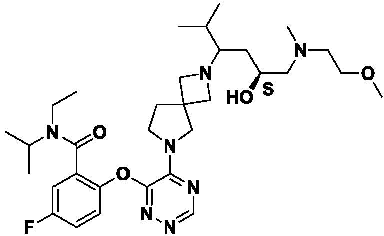 | intermediate 209, intermediate 244 | TEA, NaBH₃CN, DCM, 30 °C |
| 349 | 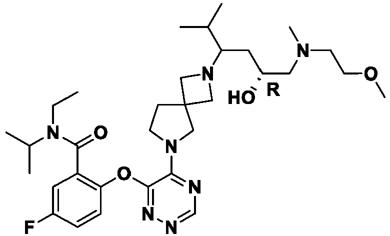 | intermediate 210, intermediate 244 | TEA, NaBH(OAc)₃, DCM, 35 °C |
| 358 | 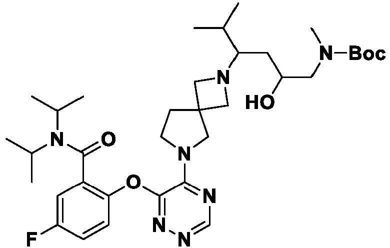 | intermediate 165, intermediate 243 | NaOAc, NaBH₃CN, MeOH, RT |
| 381 | 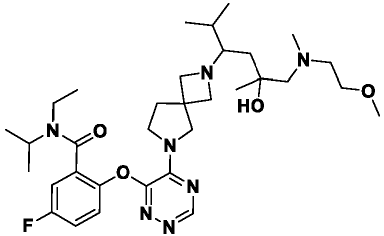 | intermediate 221, intermediate 244 | NaOAc, NaBH₃CN, MeOH, 60 °C |
| 399 | 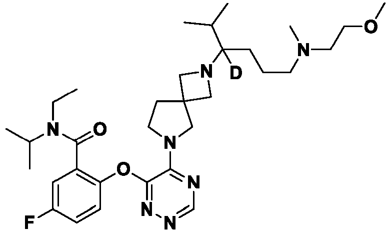 | intermediate 162, intermediate 3 | NaBD₃CN, CD₃OD, RT |
| 403 | 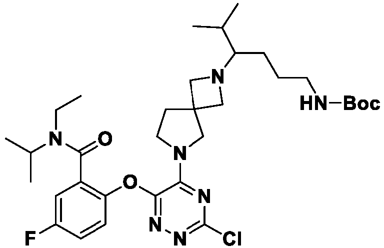 | intermediate 1, intermediate 238 | NaOAc, NaBH₃CN, MeOH, 45 °C |

**For Co. No. 399: LC-MS (ESI) (Method 8):** Rt = 1.21 min, m/z found 601.6 [M+H]⁺

### Compound 111

### N-ethyl-5-fluoro-N-isopropyl-2-((5-(2-((R)-6-(((R)-1-methoxypropan-2-yl)amino)-2-methylhexan-3-yl)-2,6-diazaspiro[3.4]octan-6-yl)-1,2,4-triazin-6-yl)oxy)benzamide

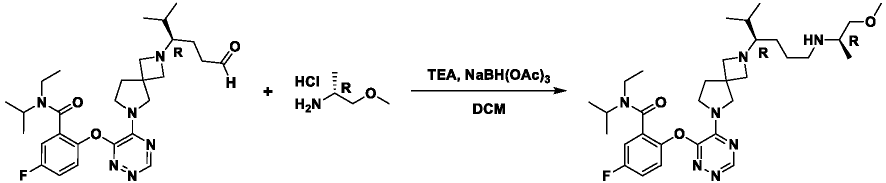

The mixture of (R)-N-ethyl-5-fluoro-N-isopropyl-2-((5-(2-(2-methyl-6-oxohexan-3-yl)-2,6-diazaspiro[3.4]octan-6-yl)-1,2,4-triazin-6-yl)oxy)benzamide **(intermediate 97)** (150 mg, 0.285 mmol) and (R)-1-methoxypropan-2-amine hydrochloride (71.5 mg, 0.569 mmol) and TEA (288 mg, 2.85 mmol) in DCM (2 mL) was stirred at 25 °C for 2 h. Then NaBH(OAc)₃ (181 mg, 0.854 mmol) was added to above mixture and the reaction was further stirred at 25 °C for additional 8 h. The mixture was quenched with H₂O (20 mL) and extracted with DCM (30 mL*3). The combined organic layers were dried over anhydrous Na₂SO₄, filtered and concentrated under reduced pressure to afford a crude product, which was purified by preparative HPLC (column: Boston Green ODS 150x30mm 5um; Mobile Phase: A: H₂O (0.05% ammonia)), B: ACN, flow rate: 30 mL/min, gradient condition: from 45% B to 85% B) to afford the title compound **Compound 111** (63 mg, 98.5% purity, 36.3% yield) as a colorless sticky oil.

### Compound 113

### (R)-2-((5-(2-(6-((3,3-difluoropropyl)amino)-2-methylhexan-3-yl)-2,6-diazaspiro[3.4loctan-6-yl)-1,2,4-triazin-6-yl)oxy)-N-ethyl-5-fluoro-N-isopropylbenzamide

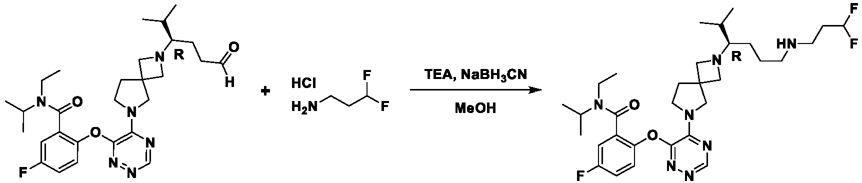

The mixture of (R)-N-ethyl-5-fluoro-N-isopropyl-2-((5-(2-(2-methyl-6-oxohexan-3-yl)-2,6-diazaspiro[3.4]octan-6-yl)-1,2,4-triazin-6-yl)oxy)benzamide **(intermediate 97)** (160 mg, 0.304 mmol), 3,3-difluoropropan-1-amine hydrochloride (160 mg, 1.22 mmol) and TEA (128 mg, 1.27 mmol) in MeOH (5 ml) was first stirred at RT for 10 min. Then AcOH (39 mg, 0.649 mmol) and NaBH₃CN (77 mg, 1.26 mmol) were added and the resulting mixture was stirred at RT for additional 16 h. The mixture was concentrated under reduced pressure to remove MeOH. The resulting residue was diluted with H₂O (30 mL) and extracted with DCM (20 mL x 3). The combined organic layers were washed with brine (10 mL x 2), dried over Na₂SO₄, filtered and concentrated to afford a crude product, which was purified by preparative HPLC (column: Boston Prime C18 150x30mm 5µm; Mobile phase: A: water (0.05% ammonia), B: ACN; gradient condition: 46% B to 76% B (v/v)) to afford the title compound **Compound 113** (32 mg, 17% yield) as a white solid.
**Compound 115, 116, 119, 124, 129, 134, 138, 141, 143, 144, 147, 151, 155, 158, 162, 163, 168, 171, 288, 289, 290, 291, 292, 293, 294, 295, 296, 297, 232, 244, 263, 264, 281, 284, 299**
**(**R*)-5-fluoro-*N,N*-diisopropyl-2-((5-(2-(6-((2-methoxyethyl)amino)-2-methylhexan-3-yl)-2,6-diazaspiro[3.4]octan-6-yl)-1,2,4-triazin-6-yl)oxy)benzamide**
**(**S*)-5-fluoro-*N,N*-diisopropyl-2-((5-(2-(6-((2-methoxyethyl)amino)-2-methylhexan-3-yl)-2,6-diazaspiro[3.4]octan-6-yl)-1,2,4-triazin-6-yl)oxy)benzamide**
***N*-ethyl-5-fluoro-N-isopropyl-2-((5-(2-(6-(isopropyl(methyl)amino)-2-methylhexan-3-yl)-2,6-diazaspiro[3.4]octan-6-yl)-1,2,4-triazin-6-yl)oxy)benzamide**
***N*-ethyl-5-fluoro-*N*-isopropyl-2-((5-(2-(2-methyl-6-(methyl(propyl)amino)hexan-3-yl)-2,6-diazaspiro[3.4]octan-6-yl)-1,2,4-triazin-6-yl)oxy)benzamide**
***N*-ethyl-2-((5-(2-(6-(ethyl(methyl)amino)-2-methylhexan-3-yl)-2,6-diazaspiro[3.4]octan-6-yl)-1,2,4-triazin-6-yl)oxy)-5-fluoro-*N*-isopropylbenzamide**
***(R)-N-ethyl-5-fluoro-N-isopropyl-2-((5-(2-(6-((2-methoxy-2-methylpropyl)amino)-2-*methylhexan-3-yl)-2,6-diazaspiro[3.4]octan-6-yl)-1,2,4-triazin-6-yl)oxy)benzamide**
**(*R*)-*N*-ethyl-5-fluoro-2-((5-(2-(6-((2-hydroxy-2-methylpropyl)amino)-2-methylhexan-3-yl)-2,6-diazaspiro[3.4]octan-6-yl)-1,2,4-triazin-6-yl)oxy)-*N*-isopropylbenzamide**
***(R)-N-ethyl-5-fluoro-N-isopropyl-2-((5-(2-(6-((3-methoxypropyl)(methyl)amino)-2-*methylhexan-3-yl)-2,6-diazaspiro[3.4]octan-6-yl)-1,2,4-triazin-6-yl)oxy)benzamide**
**(**R*)-2-((5-(2-(6-((3-(dimethylamino)-3-oxopropyl)(methyl)amino)-2-methylhexan-3-yl)-2,6-diazaspiro[3.4]octan-6-yl)-1,2,4-triazin-6-yl)oxy)-5-fluoro-*N,N-*diisopropylbenzamide**
**(**S*)-2-((5-(2-(6-((3-(dimethylamino)-3-oxopropyl)(methyl)amino)-2-methylhexan-3-yl)-2,6-diazaspiro[3.4]octan-6-yl)-1,2,4-triazin-6-yl)oxy)-5-fluoro-*N,N*-diisopropylbenzamide**
**(*R*)-*N*-ethyl-5-fluoro-*N*-isopropyl-2-((5-(2-(2-methyl-6-(methyl(2-(*N-*methylacetamido)ethyl)amino)hexan-3-yl)-2,6-diazaspiro [3.4]octan-6-yl)-1,2,4-triazin-6-yl)oxy)benzamide**
**(*R*)-2-((5-(2-(6-((4-(dimethylamino)-4-oxobutyl)(methyl)amino)-2-methylhexan-3-yl)-2,6-diazaspiro[3.4]octan-6-yl)-1,2,4-triazin-6-yl)oxy)-*N*-ethyl-5-fluoro-*N-*isopropylbenzamide**
***N*-ethyl-5-fluoro-*N*-isopropyl-2-((5-(2-((*R*)-6-(((*S*)-1-methoxypropan-2-yl)amino)-2-methylhexan-3-yl)-2,6-diazaspiro[3.4]octan-6-yl)-1,2,4-triazin-6-yl)oxy)benzamide**
**(*R*)-2-((5-(2-(6-((1,3-dimethoxypropan-2-yl)amino)-2-methylhexan-3-yl)-2,6-diazaspiro[3.4]octan-6-yl)-1,2,4-triazin-6-yl)oxy)-*N*-ethyl-5-fluoro-*N-*isopropylbenzamide**
***N*-ethyl-5-fluoro-2-((5-(2-((*R*)-6-(((*R*)-1-hydroxy-3-methoxypropan-2-yl)amino)-2-methylhexan-3-yl)-2,6-diazaspiro[3.4]octan-6-yl)-1,2,4-triazin-6-yl)oxy)-*N-*isopropylbenzamide**
***N*-ethyl-5-fluoro-2-((5-(2-((*R*)-6-(((*S*)-1-hydroxy-3-methoxypropan-2-yl)amino)-2-methylhexan-3-yl)-2,6-diazaspiro[3.4]octan-6-yl)-1,2,4-triazin-6-yl)oxy)-*N-*isopropylbenzamide**
***N*-ethyl-5-fluoro-2-((5-(2-((3*R*)-6-((3-hydroxy-2-methoxypropyl)amino)-2-methylhexan-3-yl)-2,6-diazaspiro[3.4]octan-6-yl)-1,2,4-triazin-6-yl)oxy)-*N*-isopropylbenzamide**
**2-((5-(2-((3*R*)-6-((2,3-dimethoxypropyl)amino)-2-methylhexan-3-yl)-2,6-diazaspiro[3.4]octan-6-yl)-1,2,4-triazin-6-yl)oxy)-*N*-ethyl-5-fluoro-*N-*isopropylbenzamide**
**2-((5-(2-((3*R*)-6-((4-(dimethylamino)-4-oxobutan-2-yl)amino)-2-methylhexan-3-yl)-2,6-diazaspiro[3.4]octan-6-yl)-1,2,4-triazin-6-yl)oxy)-*N*-ethyl-5-fluoro-*N-*isopropylbenzamide**
**2-((5-(2-((3*R*)-6-((3-(dimethylamino)-2-methyl-3-oxopropyl)amino)-2-methylhexan-3-yl)-2,6-diazaspiro[3.4]octan-6-yl)-1,2,4-triazin-6-yl)oxy)-*N*-ethyl-5-fluoro-*N-*isopropylbenzamide**
***N*-ethyl-5-fluoro-*N*-isopropyl-2-((5-(2-((*R*)-2-methyl-6-(((*R*)-4-(methylamino)-4-oxobutan-2-yl)amino)hexan-3-yl)-2,6-diazaspiro[3.4]octan-6-yl)-1,2,4-triazin-6-yl)oxy)benzamide**
***N*-ethyl-5-fluoro-*N*-isopropyl-2-((5-(2-((*R*)-2-methyl-6-(((*S*)-4-(methylamino)-4-oxobutan-2-yl)amino)hexan-3-yl)-2,6-diazaspiro[3.4]octan-6-yl)-1,2,4-triazin-6-yl)oxy)benzamide**
***N*-ethyl-5-fluoro-*N*-isopropyl-2-((5-(2-((*R*)-2-methyl-6-(((*R*)-2-methyl-3-(methylamino)-3-oxopropyl)amino)hexan-3-yl)-2,6-diazaspiro[3.4]octan-6-yl)-1,2,4-triazin-6-yl)oxy)benzamide**
***N*-ethyl-5-fluoro-*N*-isopropyl-2-((5-(2-((*R*)-2-methyl-6-(((*S*)-2-methyl-3-(methylamino)-3-oxopropyl)amino)hexan-3-yl)-2,6-diazaspiro[3.4]octan-6-yl)-1,2,4-triazin-6-yl)oxy)benzamide**
**2-((5-(2-((**R*)-6-(((*R*)-4-amino-4-oxobutan-2-yl)amino)-2-methylhexan-3-yl)-2,6-diazaspiro[3.4]octan-6-yl)-1,2,4-triazin-6-yl)oxy)-5-fluoro-*N,N*-diisopropylbenzamide**
**2-((5-(2-((**R*)-6-(((*S*)-4-amino-4-oxobutan-2-yl)amino)-2-methylhexan-3-yl)-2,6-diazaspiro[3.4]octan-6-yl)-1,2,4-triazin-6-yl)oxy)-5-fluoro-*N,N-*diisopropylbenzamide**
**2-((5-(2-((**R*)-6-(((*R*)-3-amino-2-methyl-3-oxopropyl)amino)-2-methylhexan-3-yl)-2,6-diazaspiro[3.4]octan-6-yl)-1,2,4-triazin-6-yl)oxy)-5-fluoro-*N,N*-diisopropylbenzamide**
**2-((5-(2-((**R*)-6-(((*S*)-3-amino-2-methyl-3-oxopropyl)amino)-2-methylhexan-3-yl)-2,6-diazaspiro[3.4]octan-6-yl)-1,2,4-triazin-6-yl)oxy)-5-fluoro-*N,N*-diisopropylbenzamide**
**(*R*)-*N*-ethyl-5-fluoro-N-isopropyl-2-((5-(2-(6-((2-methoxyethyl-1,1-*d*₂)(methyl)amino)-2-methylhexan-3-yl)-2,6-diazaspiro[3.4]octan-6-yl)-1,2,4-triazin-6-yl)oxy)benzamide**
**(*R*)-2-((5-(2-(6-((2-acetamidoethyl)(methyl)amino)-2-methylhexan-3-yl)-2,6-diazaspiro[3.4]octan-6-yl)-1,2,4-triazin-6-yl)oxy)-*N*-ethyl-5-fluoro-*N-*isopropylbenzamide**
***N*-ethyl-5-fluoro-*N*-isopropyl-2-((5-(2-(6-((2-methoxyethyl)(methyl)amino)-2,4-dimethylhexan-3-yl)-2,6-diazaspiro[3.4]octan-6-yl)-1,2,4-triazin-6-yl)oxy)benzamide (mixture of *R,S* and *S,R;* or mixture of *R,R* and *S,S*)**
***N*-ethyl-5-fluoro-*N*-isopropyl-2-((5-(2-(6-((2-methoxyethyl)(methyl)amino)-2,4-dimethylhexan-3-yl)-2,6-diazaspiro[3.4]octan-6-yl)-1,2,4-triazin-6-yl)oxy)benzamide (mixture of *R,R* and *S,S;* or mixture of *R,S* and *S,R*)**
***N*-ethyl-5-fluoro-2-((5-(2-((*R*)-6-(((*R*)-2-hydroxy-3-methoxypropyl)amino)-2-methylhexan-3-yl)-2,6-diazaspiro[3.4]octan-6-yl)-1,2,4-triazin-6-yl)oxy)-*N-*isopropylbenzamide formate**
***N*-ethyl-5-fluoro-2-((5-(2-((*R*)-6-(((*S*)-2-hydroxy-3-methoxypropyl)amino)-2-methylhexan-3-yl)-2,6-diazaspiro[3.4]octan-6-yl)-1,2,4-triazin-6-yl)oxy)-*N-*isopropylbenzamide formate**
***N*-ethyl-5-fluoro-*N*-isopropyl-2-((5-(2-((3*R*)-6-((2-methoxyethyl)(methyl)amino)-2-methylheptan-3-yl)-2,6-diazaspiro[3.4]octan-6-yl)-1,2,4-triazin-6-yl)oxy)benzamide**

**The following compounds were synthesized by an analogous method as described above for Compound 111 and 113**

| **Co. No.** | **Structure** | **Starting Material** | **Conditions** | **Spectra Details** |
|---|---|---|---|---|
| 115 | 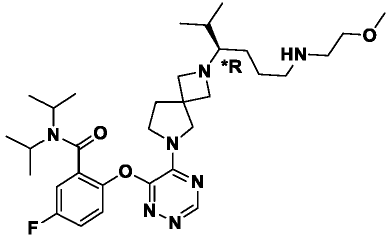 | intermediate 101, 2-methoxyethan-1-amine | NaOAc, NaBH₃CN, MeOH, 60 °C | |
| 116 | 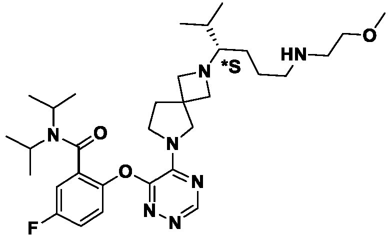 | intermediate 102, 2-methoxyethan-1-amine | NaOAc, NaBH₃CN, MeOH, 60 °C | |
| 119 | 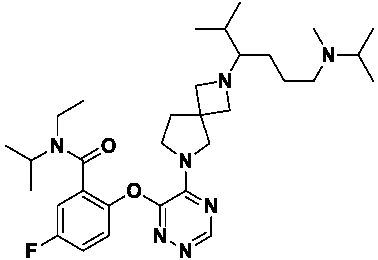 | intermediate 103, N-methylpropan-2-amine | NaBH(OAc) ₃, TEA, DCM, 25 °C | |
| 124 | 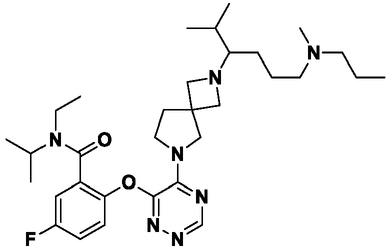 | intermediate 103, N-methylpropan-1-amine | NaBH(OAc) ₃, DCM, 25 °C | |
| 129 | 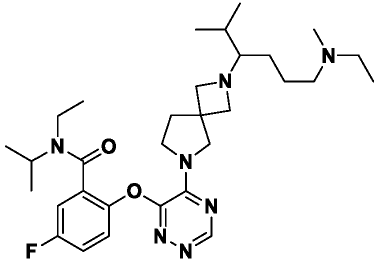 | intermediate 103, *N-*methylethanamin e | NaBH(OAc) ₃, DCM, 25 °C | |
| 134 | 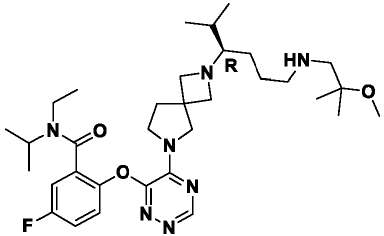 | intermediate 97, 2-methoxy-2-methylpropan-1-amine | NaOAc, NaBH₃CN, MeOH, 45 °C | |
| 138 | 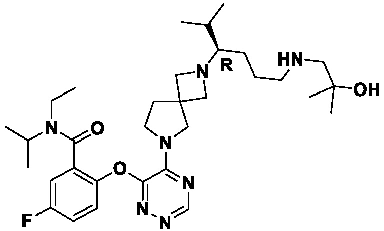 | intermediate 97, 1-amino-2-methylpropan-2-ol | NaBH₃CN, AcOH, MeOH, 45 °C | |
| 141 | 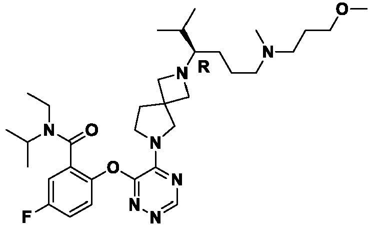 | intermediate 97, 3-methoxy-N-methylpropan-1-amine | NaOAc, NaBH₃CN, MeOH, RT | |
| 143 | 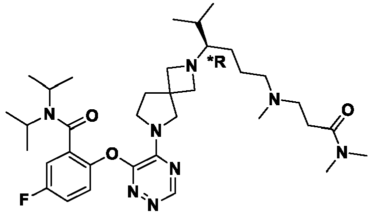 | intermediate 101, N,N-dimethyl-3-(methylamino)pr opanamide | NaBH(OAc) ₃, TEA, DCM, 25 °C | |
| 144 | 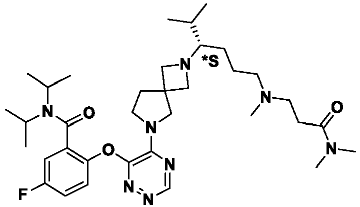 | intermediate 102, N,N-dimethyl-3-(methylamino)pr opanamide | NaBH(OAc) ₃, TEA, DCM, 25 °C | |
| 147 | 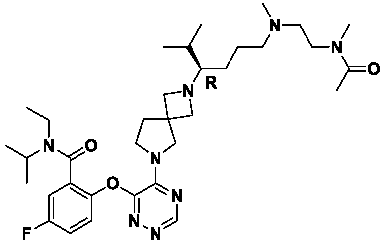 | intermediate 97, N-methyl-N-(2-(methylamino)et hyl)acetamide | NaBH(OAc) ₃, TEA, DCM, 45 °C | |
| 151 | 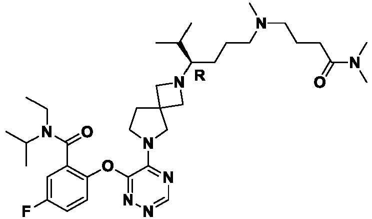 | intermediate 97, N,N-dimethyl-4-(methylamino)bu tanamide | NaOAc, NaBH₃CN, MeOH, RT | |
| 155 | 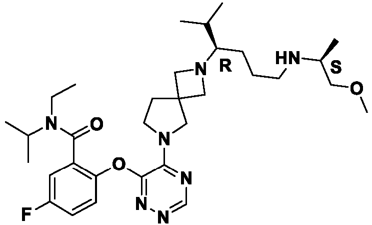 | intermediate 97, (S)-1-methoxypropan-2-amine | NaBH(OAc) ₃, DCM, 25 °C | |
| 158 | 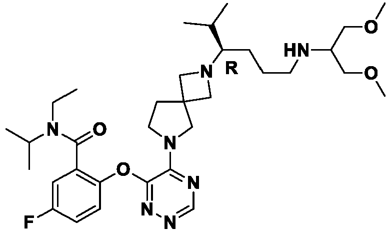 | intermediate 97, 1,3-dimethoxypropa n-2-amine | NaBH₃CN, TEA, DCM, RT | |
| 162 | 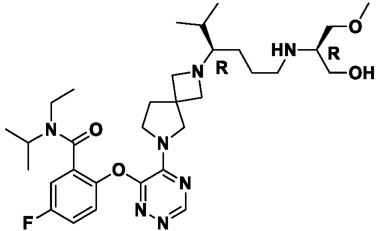 | intermediate 97, (R)-2-amino-3 - methoxypropan-1-ol | NaBH₃CN, AcOH, MeOH, 50 °C | **LC-MS (ESI) (Method 1):** Rₜ = 2.912 min, m/z found 616.3 [M+H]**⁺.** |
| | | | | **SFC (Method 3):** Rₜ = 4.465 min. |
| 163 | 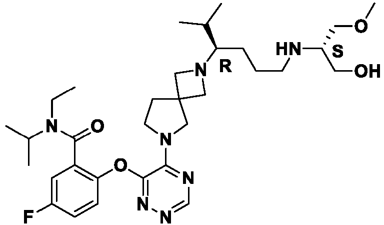 | intermediate 97, (S)-2-amino-3 - methoxypropan-1-ol | NaBH₃CN, AcOH, MeOH, 40 °C | **LC-MS (ESI) (Method 3):** Rₜ = 4.462 min, m/z found 616.4 [M+H]⁺. |
| | | | | **SFC (Method 3):** Rₜ = 4.812 min. |
| 168 | 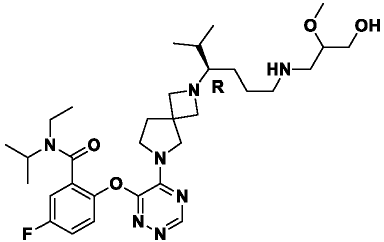 | intermediate 97, 3-amino-2-methoxypropan-1-ol | NaBH₃CN, AcOH, MeOH, 60 °C | **LC-MS (ESI) (Method 3):** Rₜ = 4.791 min, m/z found 616.5 [M+H]⁺. |
| 171 | 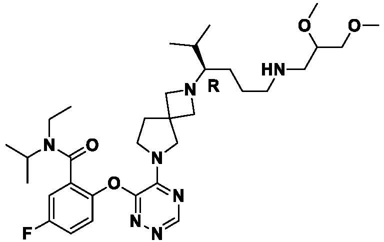 | intermediate 97, 2,3-dimethoxypropa n-1-amine | NaBH₃CN, AcOH, MeOH, 25 °C | |
| 288 | 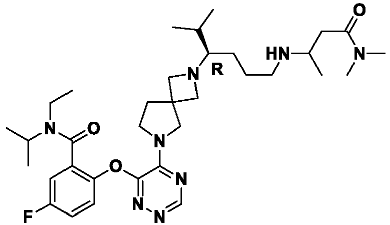 | intermediate 97, 3-amino-N,N-dimethylbutanam ide | NaOAc, NaBH₃CN, MeOH, 20 °C | |
| 289 | 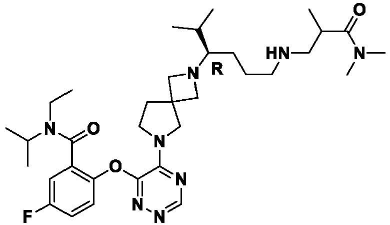 | intermediate 97, 3-amino-N,N,2-trimethylpropana mide | NaOAc, NaBH₃CN, MeOH, 20 °C | |
| 290 | 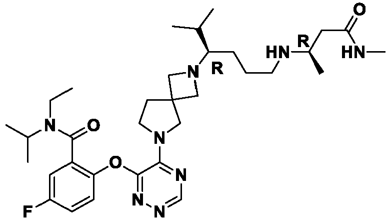 | intermediate 97, (R)-3-amino-N-methylbutanamid e | NaOAc, NaBH₃CN, MeOH, 20 °C | |
| 291 | 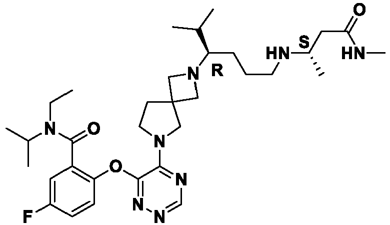 | intermediate 97, (*S*)-3-amino-*N-*methylbutanamid e | NaOAc, NaBH₃CN, MeOH, 20 °C | |
| 292 | 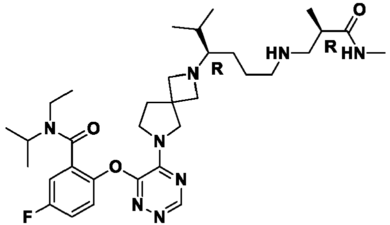 | intermediate 97, (*R*)-3-amino-*N*,2-dimethylpropana mide | NaOAc, NaBH₃CN, MeOH, 20 °C | |
| 293 | 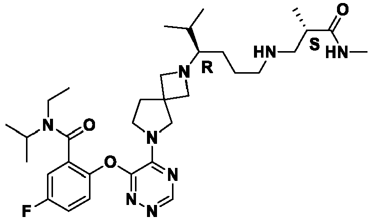 | intermediate 97, (*S*)-3-amino-*N*,2-dimethylpropana mide | NaOAc, NaBH₃CN, MeOH, 20 °C | |
| 294 | 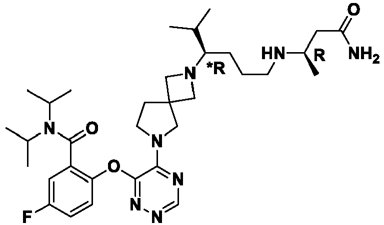 | intermediate 101, (*R*)-3-aminobutanamid e | NaOAc, NaBH₃CN, MeOH, 20 °C | |
| 295 | 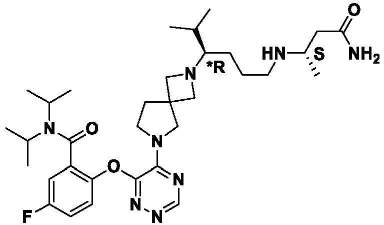 | intermediate 101, (*S*)-3-aminobutanamid e | NaOAc, NaBH₃CN, MeOH, 20 °C | |
| 296 | 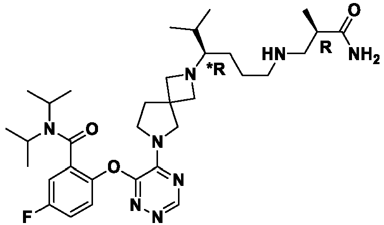 | intermediate 101, (*R*)-3-amino-2-methylpropanam ide | NaOAc, NaBH₃CN, MeOH, 20 °C | |
| 297 | 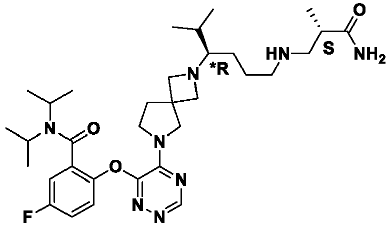 | intermediate 101, (*S*)-3-amino-2-methylpropanam ide | NaOAc, NaBH₃CN, MeOH, 20 °C | |
| 232 | 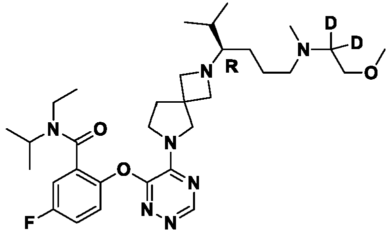 | intermediate 97, intermediate 127 | NaBH₃CN, MeOH, 25 °C | |
| 244 | 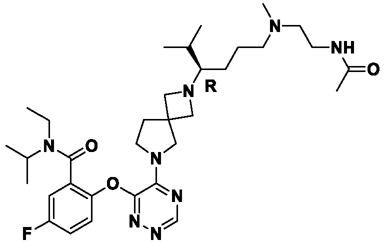 | intermediate 97, *N-(2-*(methylamino)et hyl)acetamide | NaBH₃CN, TEA, MeOH, RT | |
| 263 | 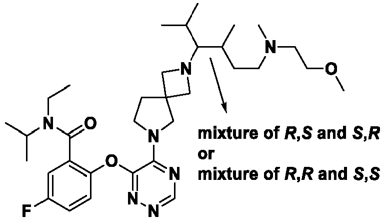 | intermediate 145, 2-methoxy-N-methylethan-1-amine | NaBH₃CN, MeOH, 40 °C | |
| 264 | 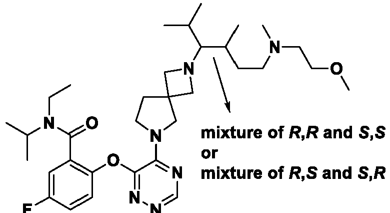 | intermediate 146, 2-methoxy-N-methylethan-1-amine | NaBH₃CN, MeOH, 40 °C | |
| 281 | 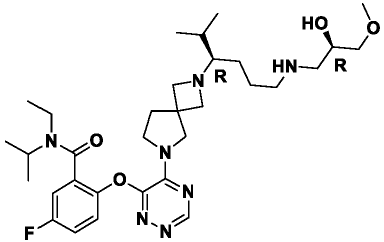 formate salt | intermediate 97, (*R*)-1-amino-3-methoxypropan-2-ol | NaBH₃CN, ZnCl₂, MeOH, 25 °C | **LC-MS (ESI) (Method 1):** Rₜ = 3.016 min, m/z found 616.3 [M+H]⁺. |
| | | | | **SFC (Method 13):** Rₜ = 2.306 min. |
| 284 | 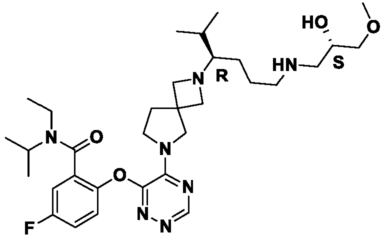 formate salt | intermediate 97, (*S*)-1-amino-3-methoxypropan-2-ol | NaBH₃CN, ZnCl₂, MeOH, 25 °C | **LC-MS (ESI) (Method 1):** Rₜ = 3.048 min, m/z found 616.3 [M+H]⁺. |
| | | | | **SFC (Method 13):** Rₜ = 2.333 min. |
| 299 | 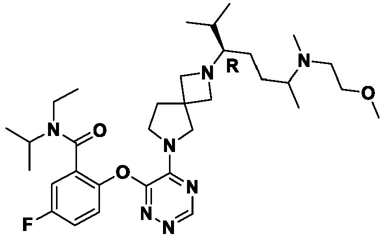 | intermediate 158, 2-methoxy-N-methylethan-1-amine | NaOAc, NaBH₃CN, MeOH, RT | |

### Compound 120 and 121

### (*R)-N-ethyl-5-fluoro-N-isopropyl-2-((5-(2-(6-(isopropyl(methyl)amino)-2-methylhexan-3-yl)-2,6-diazaspiro[3.4]octan-6-yl)-1,2,4-triazin-6-yl)oxy)benzamide

### (*S)-N-ethyl-5-fluoro-N-isopropyl-2-((5-(2-(6-(isopropyl(methyl)amino)-2-methylhexan-3-yl)-2,6-diazaspiro[3.4]octan-6-yl)-1,2,4-triazin-6-yl)oxy)benzamide

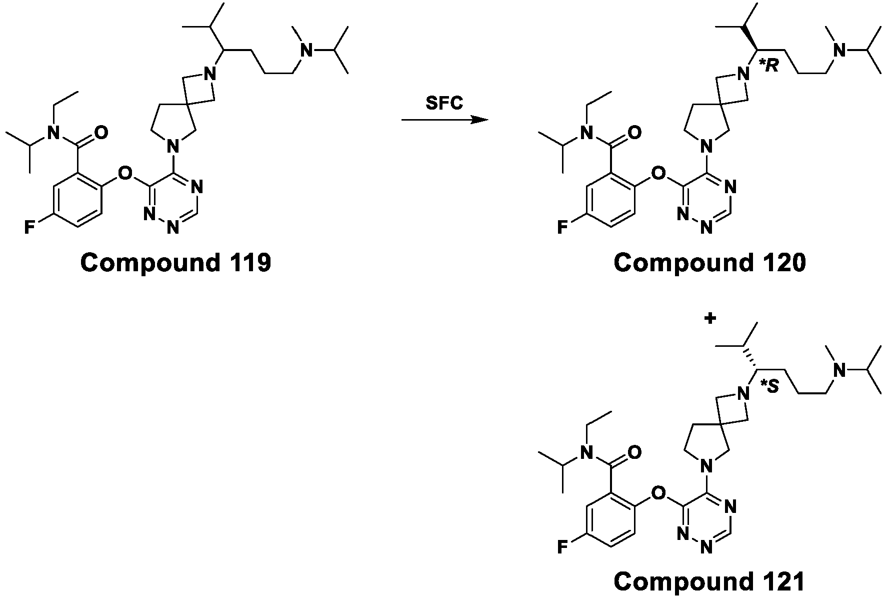

*N*-ethyl-5-fluoro-*N*-isopropyl-2-((5-(2-(6-(isopropyl(methyl)amino)-2-methylhexan-3-yl)-2*,*6-diazaspiro[3.4]octan-6-yl)-1,2,4-triazin-6-yl)oxy)benzamide **(Compound 119)** (100 mg) was separated by SFC over DAICEL CHIRALPAK IG (column: 250x30mm 10um; Mobile phase: A: Supercritical CO₂, B: MeOH (0.1% ammonia), A:B = 55:45 at 70 mL/min; Column Temp: 38 °C; Nozzle Pressure: 100 Bar; Nozzle Temp: 60 °C; Evaporator Temp: 20 °C; Trimmer Temp: 25 °C; Wavelength: 220nm) to afford the title compounds **(Compound 120)** (22.1 mg) and **(Compound 121)** (32.5 mg) both as light yellow solid.

### Compound 125 and 126

### (*R)-N-ethyl-5-fluoro-N-isopropyl-2-((5-(2-(2-methyl-6-(methyl(propyl)amino)hexan-3-yl)-2,6-diazaspiro[3.4]octan-6-yl)-1,2,4-triazin-6-yl)oxy)benzamide

### (*S)-N-ethyl-5-fluoro-N-isopropyl-2-((5-(2-(2-methyl-6-(methyl(propyl)amino)hexan-3-yl)-2,6-diazaspiro[3.4]octan-6-yl)-1,2,4-triazin-6-yl)oxy)benzamide

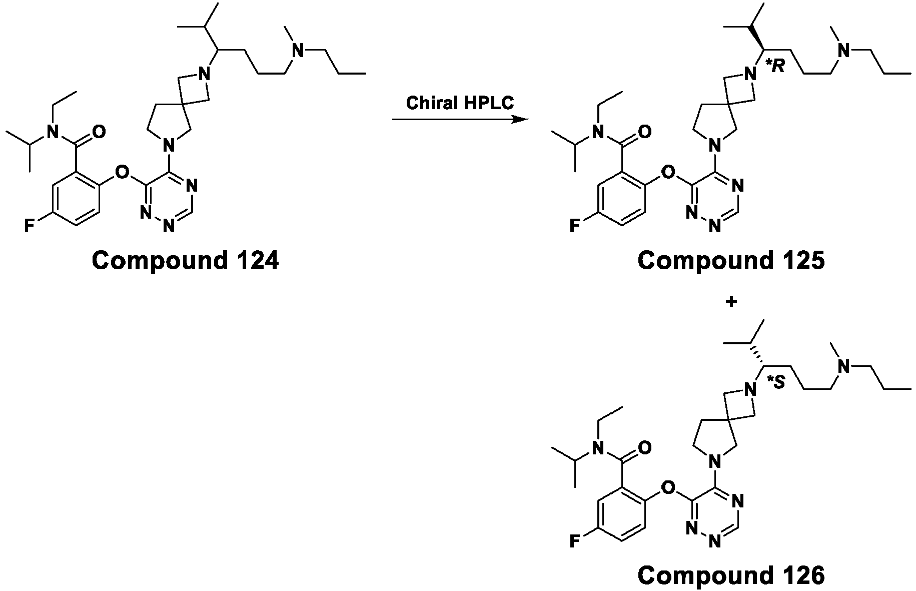

*N-ethyl-5-fluoro-N-isopropyl-2-((5-(2-(2-methyl-6-(methyl(propyl)amino)hexan-3-yl)-2,6-*diazaspiro[3.4]octan-6-yl)-1,2,4-triazin-6-yl)oxy)benzamide **(Compound 124)** (150 mg) was separated by chiral HPLC over Daicel ChiralPak IG (column: 250x30mm 10um; Mobile Phase A: Hexane; Mobile Phase B: EtOH; Flow rate: 20 mL/min; gradient condition from 20% B to 100% B) to afford the title compounds **(Compound 125)** (38.0 mg) and **(Compound 126)** (27.2 mg) both as light yellow solid.

### Compound 130 and 131

### (*R)-N-ethyl-2-((5-(2-(6-(ethyl(methyl)amino)-2-methylhexan-3-yl)-2,6-diazaspiro[3.4]octan-6-yl)-1,2,4-triazin-6-yl)oxy)-5-fluoro-N-isopropylbenzamide

### (*S)-N-ethyl-2-((5-(2-(6-(ethyl(methyl)amino)-2-methylhexan-3-yl)-2,6-diazaspiro[3.4]octan-6-yl)-1,2,4-triazin-6-yl)oxy)-5-fluoro-N-isopropylbenzamide

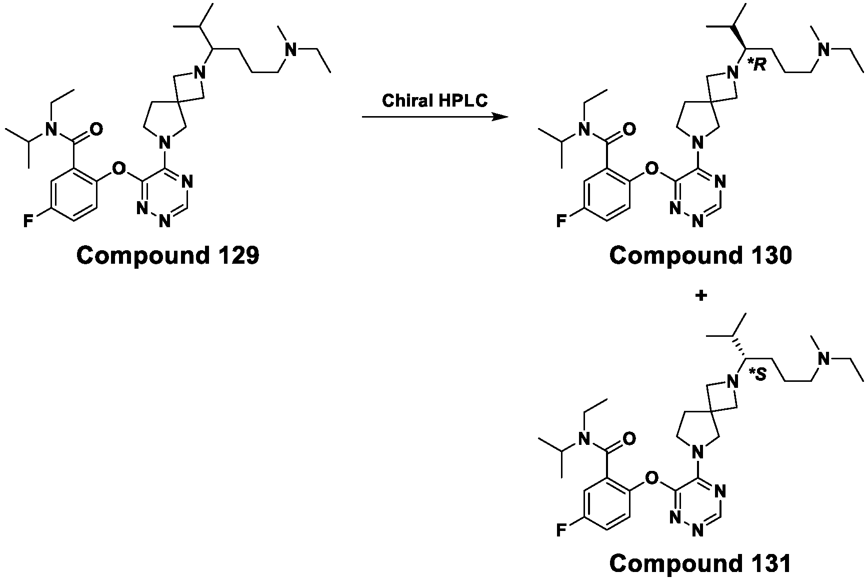

*N*-ethyl-2-((5-(2-(6-(ethyl(methyl)amino)-2-methylhexan-3-yl)-2,6-diazaspiro[3.4]octan-6-yl)-1,2,4-triazin-6-yl)oxy)-5-fluoro-*N*-isopropylbenzamide **(Compound 129)** (300 mg) was separated by chiral HPLC over Daicel ChiralPak IG (column: 250x30mm 10um; Mobile Phase A: Hexane; Mobile Phase B: EtOH; Flow rate: 20 mL/min; gradient condition from 20% B to 100% B) to afford the title compounds **(Compound 130)** (68.4 mg) and **(Compound 131)** (54.8 mg) both as light yellow solid.

### Compound 174 and 175

### 2-((5-(2-((R)-6-(((*R)-2,3-dimethoxypropyl)(methyl)amino)-2-methylhexan-3-yl)-2,6-diazaspiro[3.4loctan-6-yl)-1,2,4-triazin-6-yl)oxy)-N-ethyl-5-fluoro-N-isopropylbenzamide

### 2-((5-(2-((R)-6-(((*S)-2,3-dimethoxypropyl)(methyl)amino)-2-methylhexan-3-yl)-2,6-diazaspiro[3.4]octan-6-yl)-1,2,4-triazin-6-yl)oxy)-N-ethyl-5-fluoro-N-isopropylbenzamide

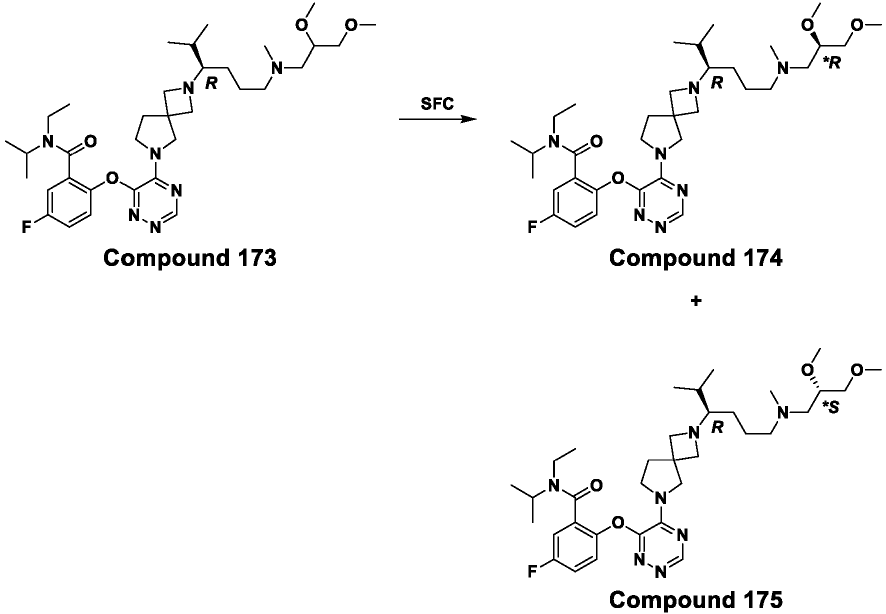

2-((5-(2-((3*R*)-6-((2,3-dimethoxypropyl)(methyl)amino)-2-methylhexan-3-yl)-2,6-diazaspiro[3.4]octan-6-yl)-1,2,4-triazin-6-yl)oxy)-*N*-ethyl-5-fluoro-*N*-isopropylbenzamide **(Compound 173)** (60 mg) was purified by SFC over DAICEL CHIRALPAK AD (column: 250x30mm 10um; Mobile phase: A: Supercritical CO₂, B: IPA (0.1% ammonia), A: B=70%:30% isocratic (v/v) at 70 mL/min) to afford the title compounds **(Compound 174)** (10 mg) and **(Compound 175)** (10 mg) both as colorless sticky oil.

### Compound 182 and 183

### 2-((5-(2-((R)-6-(((*R)-4-(dimethylamino)-4-oxobutan-2-yl)(methyl)amino)-2-methylhexan-3-yl)-2,6-diazaspiro[3.4]octan-6-yl)-1,2,4-triazin-6-yl)oxy)-N-ethyl-5-fluoro-N-isopropylbenzamide

### 2-((5-(2-((R)-6-(((*S)-4-(dimethylamino)-4-oxobutan-2-yl)(methyl)amino)-2-methylhexan-3-yl)-2,6-diazaspiro[3.4]octan-6-yl)-1,2,4-triazin-6-yl)oxy)-N-ethyl-5-fluoro-N-isopropylbenzamide

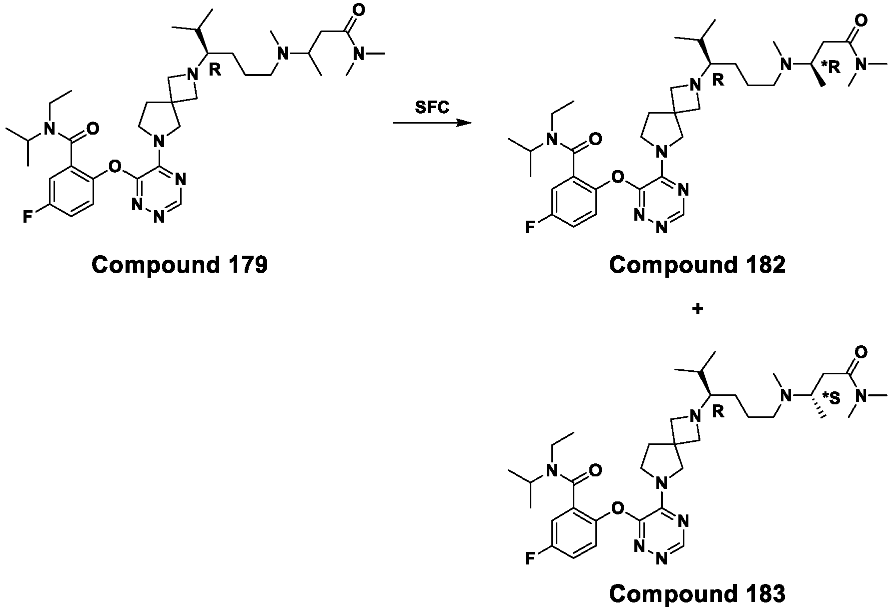

2-((5-(2-((3*R*)-6-((4-(dimethylamino)-4-oxobutan-2-yl)(methyl)amino)-2-methylhexan-3-yl)-2,6-diazaspiro[3.4]octan-6-yl)-1,2,4-triazin-6-yl)oxy)-*N*-ethyl-5-fluoro-*N*-isopropylbenzamide fumarate **(Compound 179)** (58.0 mg) was separated by SFC over DAICEL CHIRALPAK IG (column: 250x30mm 10um; Mobile phase: A: Supercritical CO₂, B: EtOH (0.1% ammonia), A:B = 45:55 at 80 mL/min; Column Temp: 38 °C; Nozzle Pressure: 100 Bar; Nozzle Temp: 60 °C; Evaporator Temp: 20 °C; Trimmer Temp: 25 °C; Wavelength: 220 nm) to afford the title compounds **(Compound 182)** (12.0 mg) and **(Compound 183)** (16.0 mg) both as colorless sticky oil.

### Compound 186 and 187

### 2-((5-(2-((R)-6-(((*R)-3-(dimethylamino)-2-methyl-3-oxopropyl)(methyl)amino)-2-methylhexan-3-yl)-2,6-diazaspiro[3.4]octan-6-yl)-1,2,4-triazin-6-yl)oxy)-N-ethyl-5-fluoro-N-isopropylbenzamide

### 2-((5-(2-((R)-6-(((*S)-3-(dimethylamino)-2-methyl-3-oxopropyl)(methyl)amino)-2-methylhexan-3-yl)-2,6-diazaspiro[3.4]octan-6-yl)-1,2,4-triazin-6-yl)oxy)-N-ethyl-5-fluoro-N-isopropylbenzamide

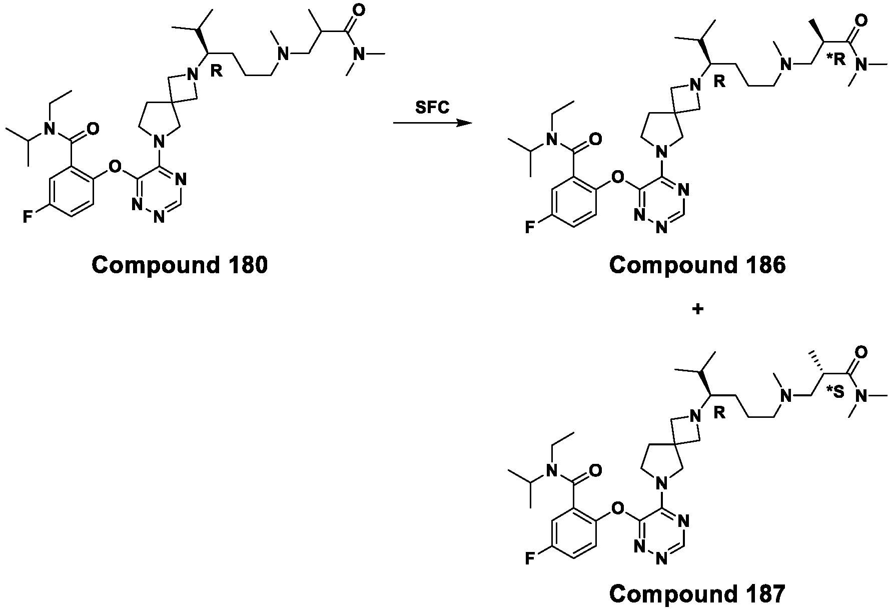

2-((5-(2-((3*R*)-6-((3-(dimethylamino)-2-methyl-3-oxopropyl)(methyl)amino)-2-methylhexan-3-yl)-2,6-diazaspiro[3.4]octan-6-yl)-1,2,4-triazin-6-yl)oxy)-N-ethyl-5-fluoro-N-isopropylbenzamide **(Compound 180)** (42.0 mg) was separated by SFC over DAICEL CHIRALPAK AD-H (column: 250x30mm 5um; Mobile phase: A: Supercritical CO₂, B: IPA (0.1% ammonia), A:B =70:30 at 60 mL/min; Column Temp: 38 °C; Nozzle Pressure: 100Bar; Nozzle Temp: 60 °C; Evaporator Temp: 20 °C; Trimmer Temp: 25 °C; Wavelength: 220nm) to afford the title compounds **(Compound 186)** (20.0 mg) and **(Compound 187)** (20.0 mg) both as light yellow sticky oil.

### Compound 214 and 215

### (*R)-N-ethyl-5-fluoro-2-((5-(2-(1-hydroxy-4-methylpentan-3-yl)-2,6-diazaspiro[3.4]octan-6-yl)-1,2,4-triazin-6-yl)oxy)-N-isopropylbenzamide

### (*S)-N-ethyl-5-fluoro-2-((5-(2-(1-hydroxy-4-methylpentan-3-yl)-2,6-diazaspiro[3.4]octan-6-yl)-1,2,4-triazin-6-yl)oxy)-N-isopropylbenzamide

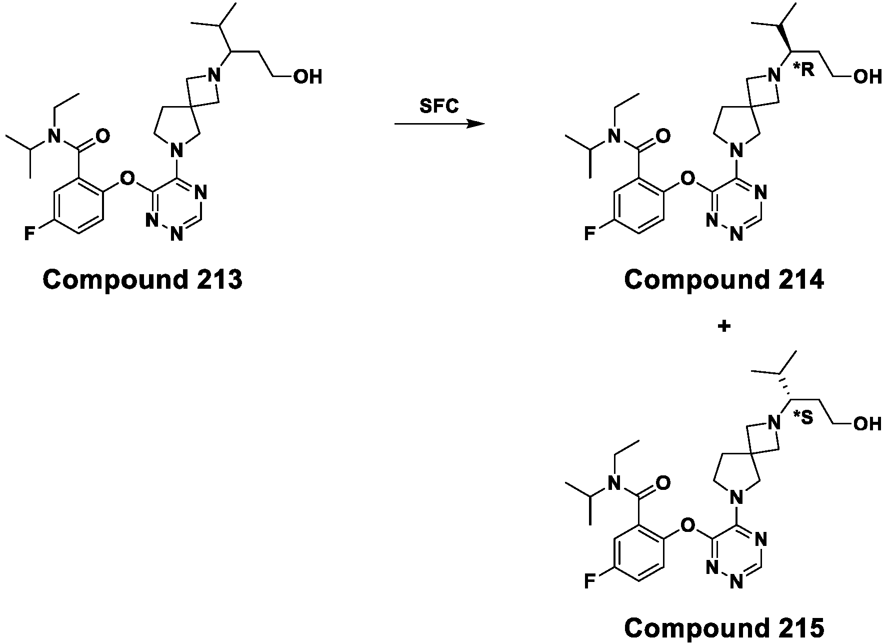

*N*-ethyl-5-fluoro-2-((5-(2-(1-hydroxy-4-methylpentan-3-yl)-2,6-diazaspiro[3.4]octan-6-yl)-1,2,4-triazin-6-yl)oxy)-*N*-isopropylbenzamide **(Compound 213)** (300 mg, crude) was first purified by preparative HPLC over Phenomenex Gemini-NX (column: C18 75x30 mm 3um; eluent: ACN/H₂O (0.05% ammonia+10mM NH₄HCO₃) from 30% to 60%, v/v) to afford a pure product (100 mg). This pure product was further purified by SFC over DAICEL CHIRALPAK IG (column: 250x30 mm 10 µm; Mobile phase: A: supercritical CO₂, B: MeOH (containing 0.1% ammonia), A:B = 45%:55% isocratic elution) to afford the title compounds **(Compound 214)** (38.8 mg) and **(Compound 215)** (40.7 mg) both as white solid.

### Compound 214

**LC-MS (ESI) (Method 1):** Rt = 3.000 min, m/z found 515.2 [M+H]⁺.
**SFC (Method 22):** Rt = 4.406 min.

### Compound 215

**LC-MS (ESI) (Method 1):** Rt = 3.145 min, m/z found 515.2 [M+H]⁺.
**SFC (Method 22):** Rt = 4.925 min.

### Compound 216 and 217

### tert-butyl (*R)-(3-(6-(6-(2-(ethyl(isopropyl)carbamoyl)-4-fluorophenoxy)-1,2,4-triazin-5-yl)-2,6-diazaspiro[3.4]octan-2-yl)-4-methylpentyl)carbamate

### tert-butyl (*S)-(3-(6-(6-(2-(ethyl(isopropyl)carbamoyl)-4-fluorophenoxy)-1,2,4-triazin-5-yl)-2,6-diazaspiro[3.4]octan-2-yl)-4-methylpentyl)carbamate

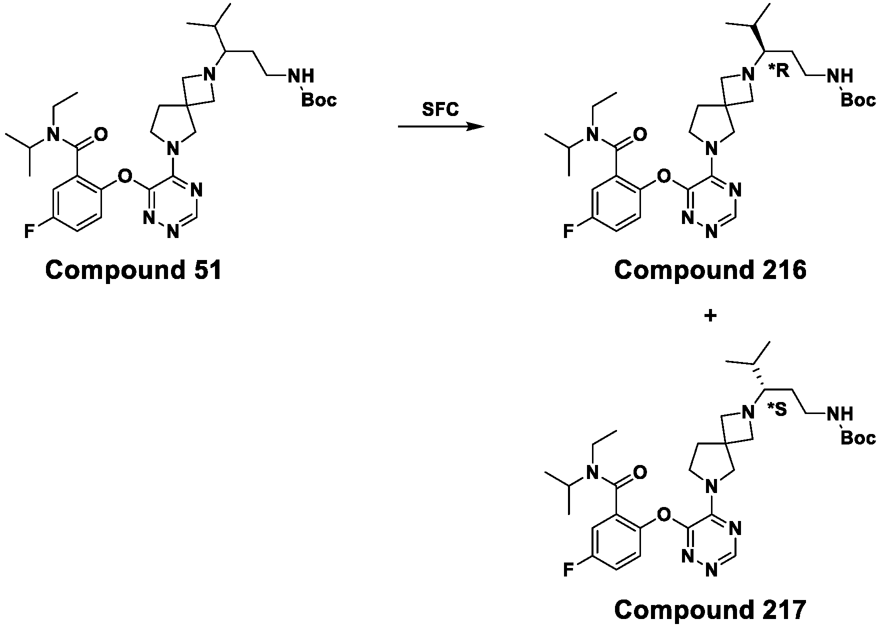

Tert-butyl (3-(6-(6-(2-(ethyl(isopropyl)carbamoyl)-4-fluorophenoxy)-1,2,4-triazin-5-yl)-2,6-diazaspiro[3.4]octan-2-yl)-4-methylpentyl)carbamate **(Compound 51)** (1.00 g) was purified by SFC over DAICEL CHIRALPAK IG (column: 250x30mm 10um; Mobile phase: A: Supercritical CO₂, B: MeOH (0.1% ammonia), A:B = 60:40 (v/v)) to afford the title compounds **(Compound 216)** (400 mg) and **(Compound 217)** (450 mg) both as white solid.

### Compound 230

### (*R)-2-((5-(2-(1-((2-amino-2-oxoethyl)amino)-4-methylpentan-3-yl)-2,6-diazaspiro[3.4]octan-6-yl)-1,2,4-triazin-6-yl)oxy)-N-ethyl-5-fluoro-N-isopropylbenzamide

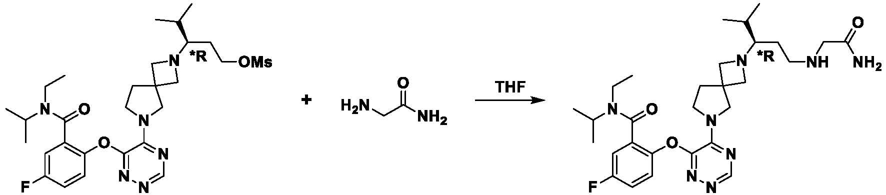

The solution of (*R)-3-(6-(6-(2-(ethyl(isopropyl)carbamoyl)-4-fluorophenoxy)-1,2,4-triazin-5-yl)-2,6-diazaspiro[3.4]octan-2-yl)-4-methylpentyl methanesulfonate **(intermediate 124)** (160 mg, crude) in THF (2 mL) was added to a solution 2-aminoacetamide (150 mg, 2.03 mmol) in THF (5 mL). The resulting mixture was stirred at RT for 2 h. The reaction mixture was filtered and washed with THF (20 mL). The filtrate was concentrated *in vacuo* to afford the crude product, which was purified by preparative HPLC over a Xtimate (column: C18 150x40mm 5um; eluent: ACN/H₂O (0.05% ammonia) from 25% to 55%, v/v) to afford the title compound (22.1 mg) as a white solid.

**LC-MS (ESI) (Method 1):** Rt = 2.849 min, m/z found 571.2 [M+H]⁺.

**SFC (Method 6):** Rt = 1.598 min.
**Compound 267, 269, 271, 272, 273, 277**
**(**R*)*-N-*ethyl-5-fluoro-2-((5-(2-(1-((2-hydroxyethyl)amino)-4-methylpentan-3-yl)-2,6-diazaspiro[3.4]octan-6-yl)-1,2,4-triazin-6-yl)oxy)-*N*-isopropylbenzamide**
**(**R*)-*N-*ethyl-5-fluoro-2-((5-(2-(1-((2-hydroxyethyl)(methyl)amino)-4-methylpentan-3-yl)-2,6-diazaspiro[3.4]octan-6-yl)-1,2,4-triazin-6-yl)oxy)-*N*-isopropylbenzamide**
**(**R*)-*N*-ethyl-5-fluoro-*N*-isopropyl-2-((5-(2-(1-((3-methoxypropyl)amino)-4-methylpentan-3-yl)-2,6-diazaspiro[3.4]octan-6-yl)-1,2,4-triazin-6-yl)oxy)benzamide**
**(**R*)-*N*-ethyl-2-((5-(2-(1-(ethylamino)-4-methylpentan-3-yl)-2,6-diazaspiro[3.4]octan-6-yl)-1,2,4-triazin-6-yl)oxy)-5-fluoro-*N*-isopropylbenzamide formate**
**(**R*)-*N*-ethyl-5-fluoro-2-((5-(2-(1-((3-hydroxypropyl)amino)-4-methylpentan-3-yl)-2,6-diazaspiro[3.4]octan-6-yl)-1,2,4-triazin-6-yl)oxy)-*N*-isopropylbenzamide**
**(**R*)-2-((5-(2-(1-((3-amino-3-oxopropyl)amino)-4-methylpentan-3-yl)-2,6-diazaspiro[3.4]octan-6-yl)-1,2,4-triazin-6-yl)oxy)-*N*-ethyl-5-fluoro-*N-*isopropylbenzamide**

**The following compounds were synthesized by an analogous method as described above for Compound 230**

| **Co. No.** | **Structure** | **Starting Material** | **Conditions** | **Spectra Details** |
|---|---|---|---|---|
| 267 | 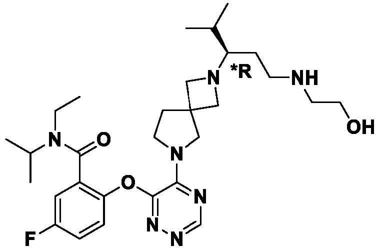 | intermediate 124, 2-aminoethan-1-ol | ACN, 60 °C | |
| 269 | 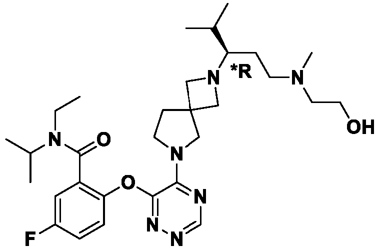 | intermediate 124, 2-(methyl-amino)ethan-1-ol | ACN, 60 °C | |
| 271 | 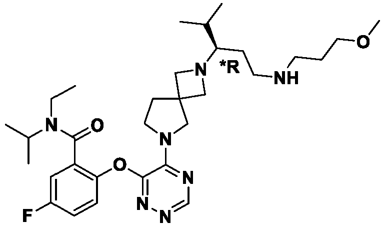 | intermediate 124, 3-methoxy-propan-1-amine | THF, RT | **LC-MS (ESI) (Method 2):** Rₜ = 1.995 min, m/z found 586.3 [M+H]⁺. |
| | | | | **SFC (Method 13):** Rₜ = 2.152 min. |
| 272 | 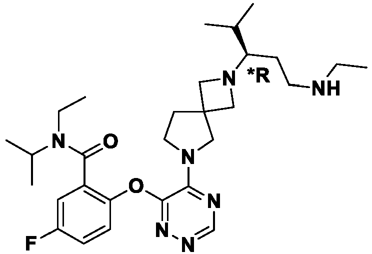 formate salt | intermediate 124, ethanamine | THF, RT | **LC-MS (ESI) (Method 2):** Rₜ = 1.892 min, m/z found 542.3 [M+H]⁺. |
| | | | | **SFC (Method 14):** Rₜ = 2.753 min. |
| 273 | 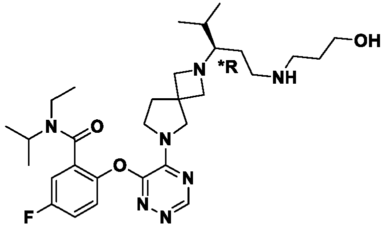 | intermediate 124, 3-aminopropan-1-ol | THF, RT | **LC-MS (ESI) (Method 1):** Rₜ = 2.894 min, m/z found 572.4 [M+H]⁺. |
| | | | | **SFC (Method 6):** Rₜ = 1.421 min. |
| 277 | 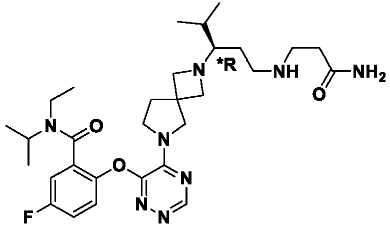 | intermediate 124, 3-aminopropana mide | THF, 60 °C | |

### Compound 236 and 237

### N-ethyl-5-fluoro-N-isopropyl-2-((5-(2-((3 *R,5 *R)-6-((2-methoxyethyl)(methyl)amino)-2,5-dimethylhexan-3-yl)-2,6-diazaspiro[3.4]octan-6-yl)-1,2,4-triazin-6-yl)oxy)benzamide

### N-ethyl-5-fluoro-N-isopropyl-2-((5-(2-((3*S,5*R)-6-((2-methoxyethyl)(methyl)amino)-2,5-dimethylhexan-3-yl)-2,6-diazaspiro[3.4]octan-6-yl)-1,2,4-triazin-6-yl)oxy)benzamide

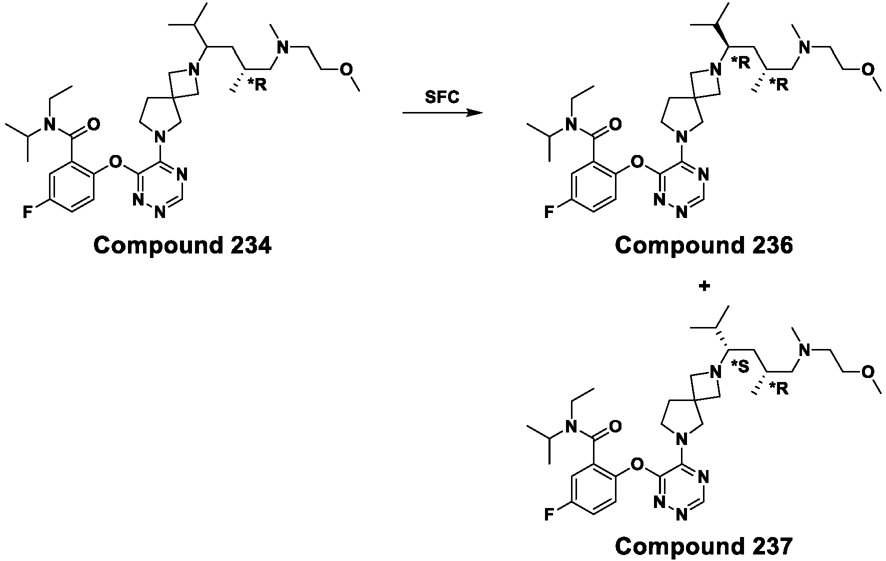

*N*-ethyl-5-fluoro-*N*-isopropyl-2-((5-(2-((5**R*)-6-((2-methoxyethyl)(methyl)amino)-2,5-**dimethylhexan-3-yl)-2,6-diazaspiro[3.4]octan-6-yl)-1,2,4-triazin-6-yl)oxy)benzamide (Compound 234)** (89.0 mg) was purified by SFC over DAICEL CHIRALPAK AD (column: 250x30 mm 10um; Mobile phase: A: Supercritical CO₂, B: IPA (0.1% ammonia), A:B = 80:20 at 60 mL/min) to afford the title compounds **(Compound 236)** (31.0 mg, 34% yield) and **(Compound 237)** (24.7 mg, 27% yield) both as yellow sticky solid.

### Compound 238 and 239

### N-ethyl-5-fluoro-N-isopropyl-2-((5-(2-((3*R,5*S)-6-((2-methoxyethyl)(methyl)amino)-2,5-dimethylhexan-3-yl)-2,6-diazaspiro[3.4]octan-6-yl)-1,2,4-triazin-6-yl)oxy)benzamide

### N-ethyl-5-fluoro-N-isopropyl-2-((5-(2-((3*S,5*S)-6-((2-methoxyethyl)(methyl)amino)-2,5-dimethylhexan-3-yl)-2,6-diazaspiro[3.4]octan-6-yl)-1,2,4-triazin-6-yl)oxy)benzamide

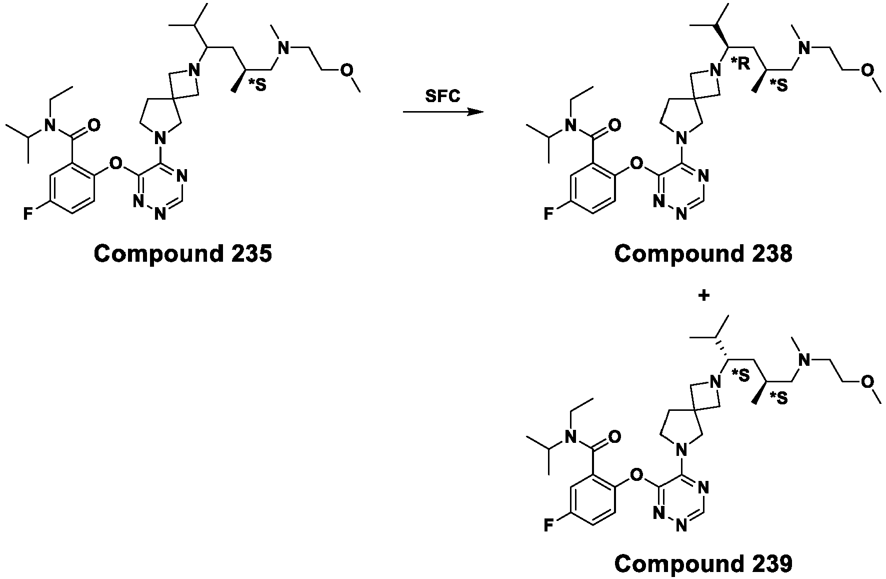

*N*-ethyl-5-fluoro-*N*-isopropyl-2-((5-(2-((5**S*)-6-((2-methoxyethyl)(methyl)amino)-2,5-dimethylhexan-3-yl)-2,6-diazaspiro[3.4]octan-6-yl)-1,2,4-triazin-6-yl)oxy)benzamide **(Compound 235)** (51 mg) was purified by SFC over DAICEL CHIRALCEL OD-H (column: 250x30mm 5um; Mobile phase: A: Supercritical CO₂, B: EtOH (0.1% ammonia), A:B = 85:15 at 60 mL/min) to afford the title compounds **(Compound 238)** (17.9 mg, 35%) and **(Compound 239)** (14.3 mg, 28%) both as white solid.

### Compound 248 and 249

### N-ethyl-5-fluoro-N-isopropyl-2-((5-(2-((R)-6-(((*R)-2-methoxypropyl)(methyl)amino)-2-methylhexan-3-yl)-2,6-diazaspiro[3.4]octan-6-yl)-1,2,4-triazin-6-yl)oxy)benzamide

### N-ethyl-5-fluoro-N-isopropyl-2-((5-(2-((R)-6-(((*S)-2-methoxypropyl)(methyl)amino)-2-methylhexan-3-yl)-2,6-diazaspiro[3.4]octan-6-yl)-1,2,4-triazin-6-yl)oxy)benzamide

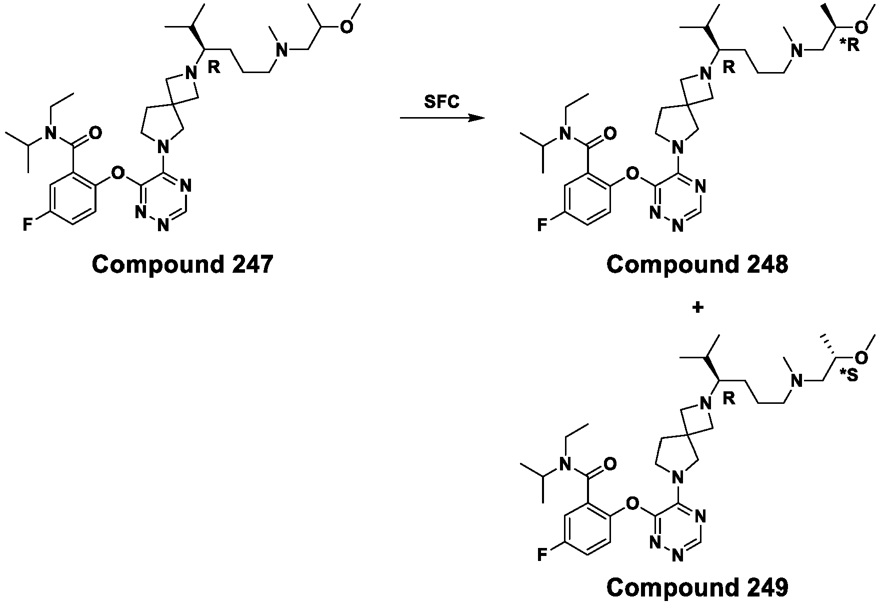

*N-ethyl-5-fluoro-N-isopropyl-2-((5-(2-((3R)-6-((2-methoxypropyl)(methyl)amino)-2-*methylhexan-3-yl)-2,6-diazaspiro[3.4]octan-6-yl)-1,2,4-triazin-6-yl)oxy)benzamide **(Compound 247)** (70 mg) was purified by SFC over DAICEL CHIRALPAK AD-H (column: 250x30mm 5µm; Mobile phase: A: supercritical CO₂, B: IPA (0.1% ammonia), A:B = 75%:25% at 60 mL/min) to afford the title compounds **(Compound 248)** (10 mg) and **(Compound 249)** (30 mg) both as light yellow sticky oil.

### Compound 261 and 262

### N-ethyl-5-fluoro-2-((5-(2-(6-hydroxy-2,4-dimethylhexan-3-yl)-2,6-diazaspiro[3.4]octan-6-yl)-1,2,4-triazin-6-yl)oxy)-N-isopropylbenzamide (mixture of R,S and S,R; or mixture of R,R and S,S)

### N-ethyl-5-fluoro-2-((5-(2-(6-hydroxy-2,4-dimethylhexan-3-yl)-2,6-diazaspiro[3.4]octan-6-yl)-1,2,4-triazin-6-yl)oxy)-N-isopropylbenzamide (mixture of R,R and S,S; or mixture of R,S and S,R)

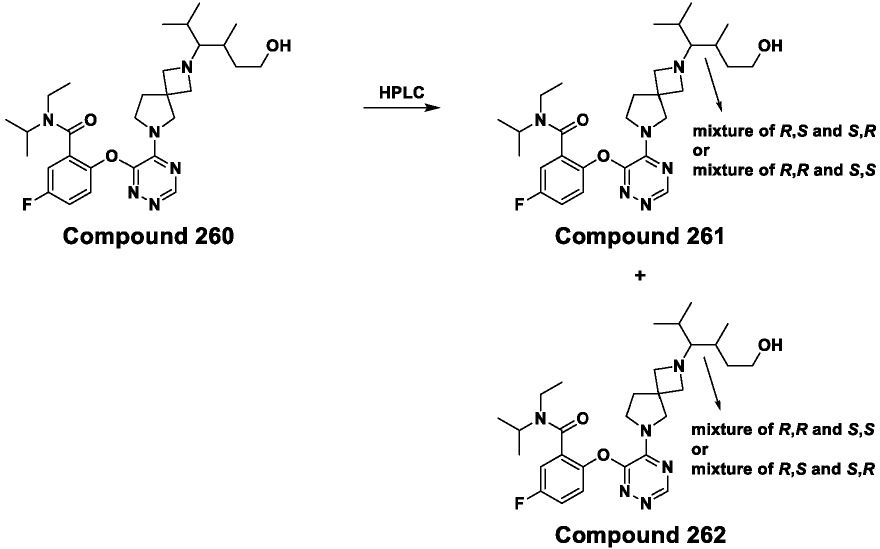

*N*-ethyl-5-fluoro-2-((5-(2-(6-hydroxy-2,4-dimethylhexan-3-yl)-2,6-diazaspiro[3.4]octan-6-yl)-1,2,4-triazin-6-yl)oxy)-*N*-isopropylbenzamide **(Compound 260)** (5.0 g, crude) was purified by HPLC (column: Xtimate C18 150x40 mm 5 µm; Mobile Phase: A: H₂O (0.05% ammonia), B: ACN, Flow rate: 60 mL/min, gradient: from 40% B to 60% B) to afford the title compounds **(Compound 261)** (220 mg) and **(Compound 262)** (300 mg) both as white solid.

### Compound 298

### N-ethyl-5-fluoro-2-((5-(2-((3R)-6-hydroxy-2-methylheptan-3-yl)-2,6-diazaspiro[3.4]octan-6-yl)-1,2,4-triazin-6-yl)oxy)-N-isopropylbenzamide

**The following compound was synthesized by an analogous method described above for intermediate 53**

| **Co. No.** | **Structure** | **Starting Materials** |
|---|---|---|
| 298 | 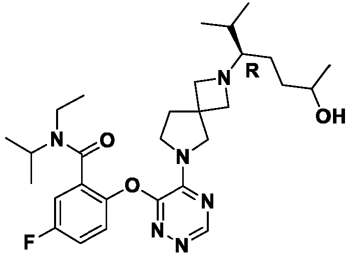 | intermediate 97, methylmagnesium bromide |

### Compound 301

### N-ethyl-5-fluoro-N-isopropyl-2-((5-(6-(6-((2-methoxyethyl)(methyl)amino)-2-methylhexan-3-yl)-2,6-diazaspiro[3.4]octan-2-yl)-1,2,4-triazin-6-yl)oxy)benzamide

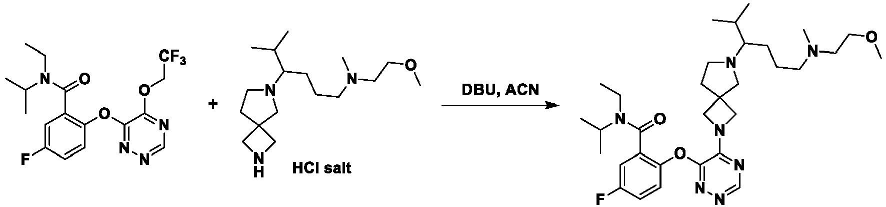

To a solution of *N*-(2-methoxyethyl)-N,5-dimethyl-4-(2,6-diazaspiro[3.4]octan-6-yl)hexan-1-amine hydrochloride **(intermediate 164)** (2.10 g, crude) and DBU (1.80 g, 11.8 mmol) in ACN (40 mL) was added N-ethyl-5-fluoro-N-isopropyl-2-((5-(2,2,2-trifluoroethoxy)-1,2,4-triazin-6-yl)oxy)benzamide **(intermediate 159)** (600 mg, 88% purity, 1.31 mmol) under N₂ atmosphere. The resulting mixture was stirred at 26 °C for 16 h. The reaction mixture was concentrated under reduced pressure and the residue was purified by preparative HPLC over Phenomenex Gemini-NX (column: 80×40 mm 3 µm, Mobile Phase: A: H₂O (0.05% ammonia), B: ACN, Flow rate: 30 mL/min, gradient condition: from 29% B to 99% B) to afford the title compound (130 mg) as colorless oil.
**Compound 319, 320, 321 and 322**
***N*-ethyl-5-fluoro-2-((5-(2-((3 **R,5* *R)-5-hydroxy-6-(isopropyl(methyl)amino)-2-methylhexan-3-yl)-2,6-diazaspiro[3.4]octan-6-yl)-1,2,4-triazin-6-yl)oxy)-*N-*isopropylbenzamide**
***N*-ethyl-5-fluoro-2-((5-(2-((3 **S,5***S*)-5-hydroxy-6-(isopropyl(methyl)amino)-2-methylhexan-3-yl)-2,6-diazaspiro[3.4]octan-6-yl)-1,2,4-triazin-6-yl)oxy)-*N-*isopropylbenzamide**
***N*-ethyl-5-fluoro-2-((5-(2-((3**R*,5**S*)-5-hydroxy-6-(isopropyl(methyl)amino)-2-methylhexan-3-yl)-2,6-diazaspiro[3.4]octan-6-yl)-1,2,4-triazin-6-yl)oxy)-*N-*isopropylbenzamide**
***N*-ethyl-5-fluoro-2-((5-(2-((3**S*,5**R*)-5-hydroxy-6-(isopropyl(methyl)amino)-2-methylhexan-3-yl)-2,6-diazaspiro[3.4]octan-6-yl)-1,2,4-triazin-6-yl)oxy)-*N-*isopropylbenzamide**

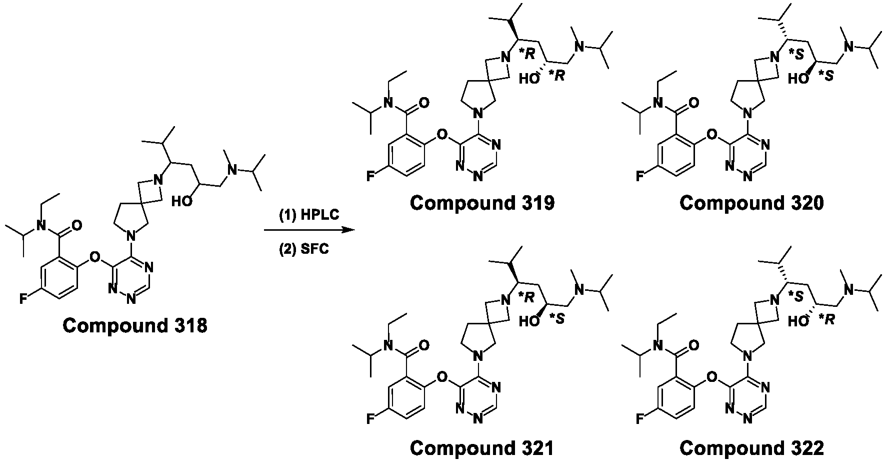

*N*-ethyl-5-fluoro-2-((5-(2-(5-hydroxy-6-(isopropyl(methyl)amino)-2-methylhexan-3-yl)-2,6-**diazaspiro[3.4]octan-6-yl)-1,2,4-triazin-6-yl)oxy)-*N*-isopropylbenzamide (Compund 318)** (235 mg, 91.5% purity) was first separated by preparative HPLC over Welch Xtimate (column: 150×25 mm 5 µm, Mobile Phase A: H₂O (0.2% FA), Mobile Phase B: ACN, Flow rate: 25 mL/min, gradient condition: from 2% B to 32%) to afford a mixture of **(Compound 319** and **Compound 320)** (95 mg, 88% purity by LCMS) and a mixture of **(Compound 321** and **Compound 322)** (97 mg, 81% purity by LCMS).

The mixture of **(Compound 319** and **Compound 320)** (95 mg, 88% purity by LCMS) and the mixture of **(Compound 321** and **Compound 322)** (97 mg, 81% purity by LCMS) were further separately purified by preparative HPLC over Welch Xtimate (column: C18 100×40 mm 3 µm, Mobile Phase A: H₂O (0.075% TFA), Mobile Phase B: ACN, Flow rate: 30 mL/min, gradient condition: from 10% B to 40% B) to afford a mixture of **(Compound 319** and **Compound 320)** (73 mg, 98.9% purity by LCMS) and a mixture of **(Compound 321** and **Compound 322)** (70 mg, 100% purity by LCMS) both as TFA salts.

The mixture of **(Compound 319** and **Compound 320)** (70 mg, 98.9% purity by LCMS, asTFA salt) was further separated by SFC over DAICEL CHIRALPAK IG (column: 250×30 mm, 10 um); Mobile phase: A: Supercritical CO₂, B: MeOH (0.1% ammonia), A:B = 40:60 at 80 mL/min) to affford **Compound 319** (15.5 mg) and **Compound 320** (16.2 mg) both as colorless sticky oil.

The mixture of **(Compound 321** and **Compound 322)** (65 mg, 100% purity by LCMS, as TFA salt) was further separated by SFC over DAICEL CHIRALPAK IG (column: 250×30 mm, 10 um; Mobile phase: A: Supercritical CO₂, B: MeOH (0.1% ammonia), A:B = 65:35 at 80 mL/min) to afford **Compound 322** (24 mg) and another fraction (22 mg) which was further separated by SFC over DAICEL CHIRALPAK AD (column: 250×30mm, 10um; Mobile phase: A: Supercritical CO₂, B: EtOH (0.1% ammonia), A:B = 75:25 at 60 mL/min) to afford **Compound 321** (16 mg).
**Compound 330, 331, 332, 333**
**2-((5-(2-((3**R,5***R*)-6-(diethylamino)-5-hydroxy-2-methylhexan-3-yl)-2,6-diazaspiro[3.4]octan-6-yl)-1,2,4-triazin-6-yl)oxy)-*N*-ethyl-5-fluoro-*N-*isopropylbenzamide**
**2-((5-(2-((3**S,*5**S*)-6-(diethylamino)-5-hydroxy-2-methylhexan-3-yl)-2,6-diazaspiro[3.4]octan-6-yl)-1,2,4-triazin-6-yl)oxy)-*N*-ethyl-5-fluoro-*N-*isopropylbenzamide**
**2-((5-(2-((3**S*,5**R*)-6-(diethylamino)-5-hydroxy-2-methylhexan-3-yl)-2,6-diazaspiro[3.4]octan-6-yl)-1,2,4-triazin-6-yl)oxy)-*N*-ethyl-5-fluoro-*N-*isopropylbenzamide**
**2-((5-(2-((3**R*,5**S*)-6-(diethylamino)-5-hydroxy-2-methylhexan-3-yl)-2,6-diazaspiro[3.4]octan-6-yl)-1,2,4-triazin-6-yl)oxy)-*N*-ethyl-5-fluoro-*N-*isopropylbenzamide**

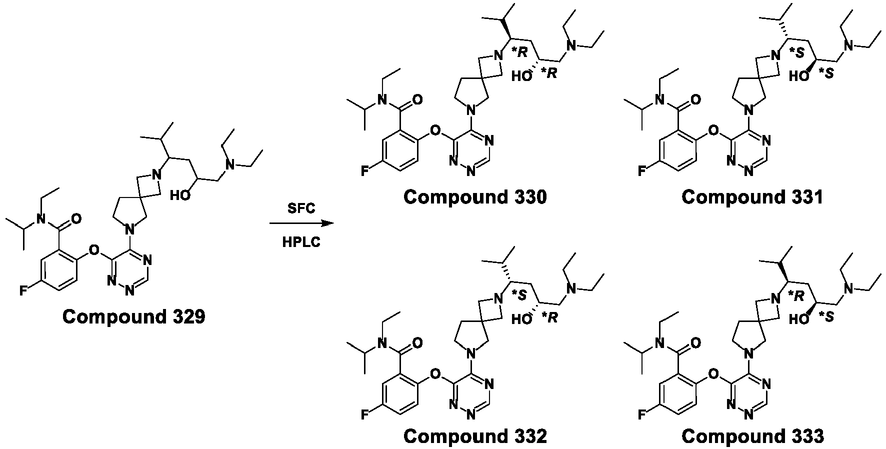

2-((5-(2-(6-(diethylamino)-5-hydroxy-2-methylhexan-3-yl)-2,6-diazaspiro[3.4]octan-6-yl)-1,2,4-triazin-6-yl)oxy)-*N*-ethyl-5-fluoro-*N*-isopropylbenzamide **(Compound 329)** (450 mg) was first separated by SFC over Daicel chiralpak AD (column: 250×30 mm, 10 µm, Mobile phase: A: Supercritical CO₂, B: EtOH (0.1% ammonia), A:B = 80:20 at 60 mL/min) to afford a mixture of **(Compound 330** and **Compound 331)** (200 mg), **Compound 332** (70 mg, 100% purity by LCMS) and **Compound 333** (170 mg, 88.9% purity by LCMS).

The **Compound 333** (170 mg, 88.9% purity by LCMS) was further purified by preparative HPLC over Phenomenex Gemini-NX (column: 75×30 mm, 3 um, Mobiel phase: A: H₂O (0.05% ammonia + 10 mM NH₄HCO₃), B: ACN, gradient condition: from 33% B to 63%, Flow rate: 25 mL/min) to afford **Compound 333** (69 mg, 97.5% purity by LCMS).

The mixture of **(Compound 330** and **Compound 331)** (200 mg) was further separated by chiral HPLC over DAICEL CHIRALPAK IG (column: 250×30 mm, 10 µm, Mobile phase: A: Heptane, B: EtOH (0.1% ammonia), gradient from 30% B to 50%, Flow rate: 25 mL/min) to afford **Compound 330** (60 mg, 75% purity by LCMS) and **Compound 331** (60 mg, 92% purity by LCMS).

The **Compound 330** (60 mg, 75% purity by LCMS) and **Compound 331** (60 mg, 92% purity by LCMS) were further separately purified by preparative HPLC over Welch Xtimate (column: 150×25 mm, 5 µm; Mobile phase: A: H₂O (0.2% FA), B: ACN, Flow rate: 25 mL/min, gradient condition: from 2% B to 32% B) and basified with ammonia to afford **Compound 330** (29 mg, 100% purity by LCMS) and **Compound 331** (23 mg, 100% purity by LCMS).

### Compound 340 and 341

### N-ethyl-2-((5-(2-((3*R,5S)-6-(ethyl(methyl)amino)-5-hydroxy-2-methylhexan-3-yl)-2,6-diazaspiro[3.4]octan-6-yl)-1,2,4-triazin-6-yl)oxy)-5-fluoro-N-isopropylbenzamide

### N-ethyl-2-((5-(2-((3*S,5S)-6-(ethyl(methyl)amino)-5-hydroxy-2-methylhexan-3-yl)-2,6-diazaspiro[3.4]octan-6-yl)-1,2,4-triazin-6-yl)oxy)-5-fluoro-N-isopropylbenzamide

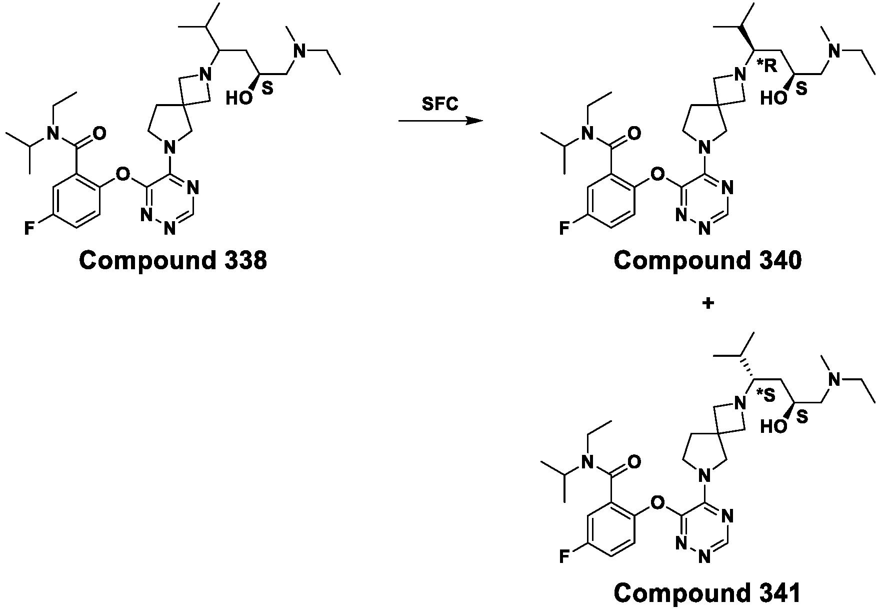

*N*-ethyl-2-((5-(2-((5*S*)-6-(ethyl(methyl)amino)-5-hydroxy-2-methylhexan-3-yl)-2,6-diazaspiro[3.4]octan-6-yl)-1,2,4-triazin-6-yl)oxy)-5-fluoro-*N*-isopropylbenzamide **(Compound 338)** (160 mg) was separated by SFC over DAICEL CHIRALPAK IG (column: 250×30 mm, 10 µm; Mobile phase: A: Supercritical CO₂, B: IPA (0.1% ammonia), A: B = 55:45 at 80 mL/min) to afford the title compounds **(Compound 340)** (30 mg) and **(Compound 341)** (66 mg) both as colorless oil.

### Compound 344 and 345

### N-ethyl-2-((5-(2-((3*R,5R)-6-(ethyl(methyl)amino)-5-hydroxy-2-methylhexan-3-yl)-2,6-diazaspiro[3.4]octan-6-yl)-1,2,4-triazin-6-yl)oxy)-5-fluoro-N-isopropylbenzamide

### N-ethyl-2-((5-(2-((3*S,5R)-6-(ethyl(methyl)amino)-5-hydroxy-2-methylhexan-3-yl)-2,6-diazaspiro[3.4]octan-6-yl)-1,2,4-triazin-6-yl)oxy)-5-fluoro-N-isopropylbenzamide

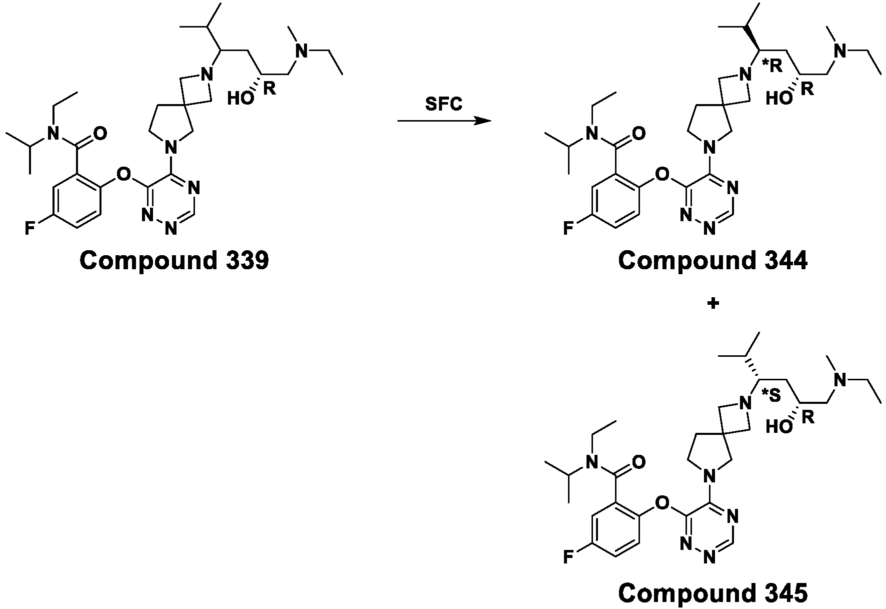

*N*-ethyl-2-((5-(2-((5*R*)-6-(ethyl(methyl)amino)-5-hydroxy-2-methylhexan-3-yl)-2,6-diazaspiro[3.4]octan-6-yl)-1,2,4-triazin-6-yl)oxy)-5-fluoro-*N*-isopropylbenzamide **(Compound 339)** (200 mg) was separated by SFC over DAICEL CHIRALPAK IG (column: 250×30 mm, 10 µm; Mobile phase: A: Supercritical CO₂, B: EtOH (0.1% ammonia), A:B = 45:55 at 80 mL/min) to afford **Compound 344** (100 mg, 98.4% purity by LCMS) and **Compound 345** (70 mg, 76% purity by LCMS) both as colorless sticky solid.

### Compound 347

### N-ethyl-2-((5-(2-((3*S,5R)-6-(ethyl(methyl)amino)-5-hydroxy-2-methylhexan-3-yl)-2,6-diazaspiro[3.4]octan-6-yl)-1,2,4-triazin-6-yl)oxy)-5-fluoro-N-isopropylbenzamide formate

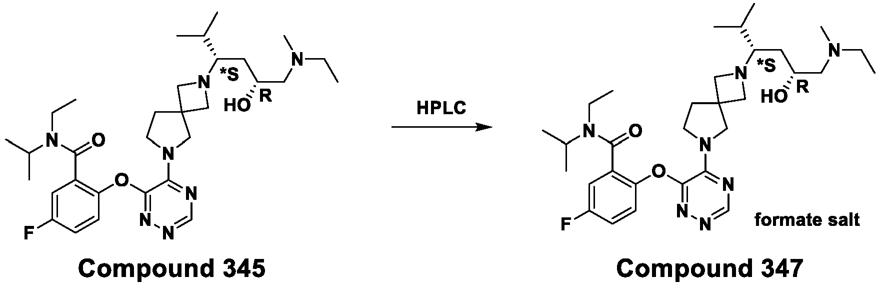

N-ethyl-2-((5-(2-((3**S*,5*R*)-6-(ethyl(methyl)amino)-5-hydroxy-2-methylhexan-3-yl)-2,6-diazaspiro[3.4]octan-6-yl)-1,2,4-triazin-6-yl)oxy)-5-fluoro-*N*-isopropylbenzamide **(Compound 345)** (70 mg, 76% purity by LCMS) was further purified by preparative HPLC over Phenomenex Gemini-NX (column: 150×30 mm, 5 um; Mobile Phase A: H₂O (0.225% FA), Mobile Phase B: ACN, Flow rate: 35 mL/min, gradient condition: from 15% B to 45% B) to afford the title compound (40.0 mg, 99.6% purity by LCMS) as a white solid.

**LC-MS (ESI) (Method 1):** Rt = 2.891 min, m/z found 586.4 [M+H]⁺.

**SFC (Method 8):** Rt = 2.652 min.

### Compound 350 and 351

### N-ethyl-5-fluoro-2-((5-(2-((3*R,5S)-5-hydroxy-6-((2-methoxyethyl)(methyl)amino)-2-methylhexan-3-yl)-2,6-diazaspiro[3.4]octan-6-yl)-1,2,4-triazin-6-yl)oxy)-N-isopropylbenzamide

### N-ethyl-5-fluoro-2-((5-(2-((3*S,5S)-5-hydroxy-6-((2-methoxyethyl)(methyl)amino)-2-methylhexan-3-yl)-2,6-diazaspiro[3.4]octan-6-yl)-1,2,4-triazin-6-yl)oxy)-N-isopropylbenzamide

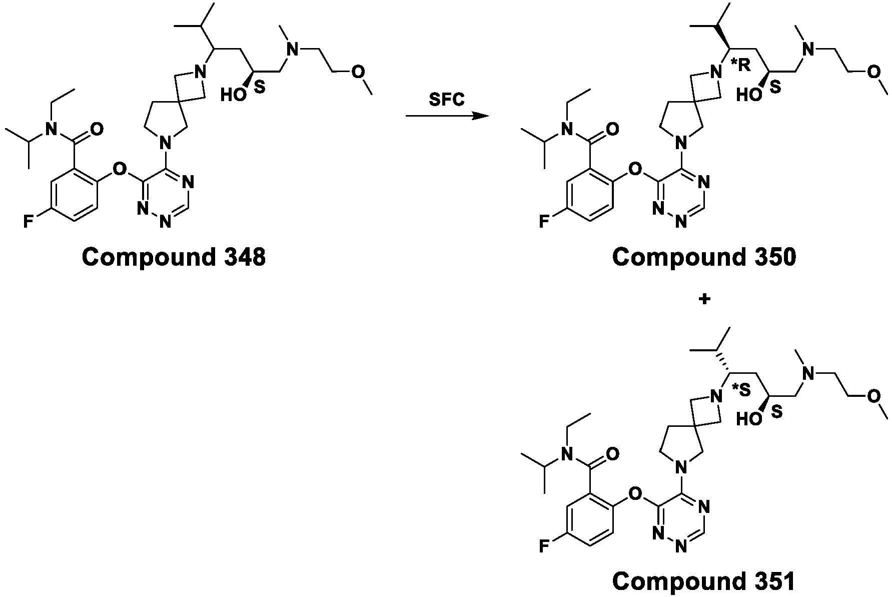

*N-ethyl-5-fluoro-2-((5-(2-((5S)-5-hydroxy-6-((2-methoxyethyl)(methyl)amino)-2-*methylhexan-3-yl)-2,6-diazaspiro[3.4]octan-6-yl)-1,2,4-triazin-6-yl)oxy)-*N-*isopropylbenzamide **(Compound 348)** (60 mg) was separated by SFC over DAICEL CHIRALPAK IG (column: 250×30 mm, 10 um; Mobile phase: A: Supercritical CO₂, B: EtOH (0.1% ammonia), A:B = 55:45 at 80 mL/min) to afford the title compounds **(Compound 350)** (22 mg) and **(Compound 351)** (27.7 mg).

### Compound 354 and 355

### N-ethyl-5-fluoro-2-((5-(2-((3*R,5R)-5-hydroxy-6-((2-methoxyethyl)(methyl)amino)-2-methylhexan-3-yl)-2,6-diazaspiro[3.4]octan-6-yl)-1,2,4-triazin-6-yl)oxy)-N isopropylbenzamide

### N-ethyl-5-fluoro-2-((5-(2-((3*S,5R)-5-hydroxy-6-((2-methoxyethyl)(methyl)amino)-2-methylhexan-3-yl)-2,6-diazaspiro[3.4]octan-6-yl)-1,2,4-triazin-6-yl)oxy)-N-isopropylbenzamide

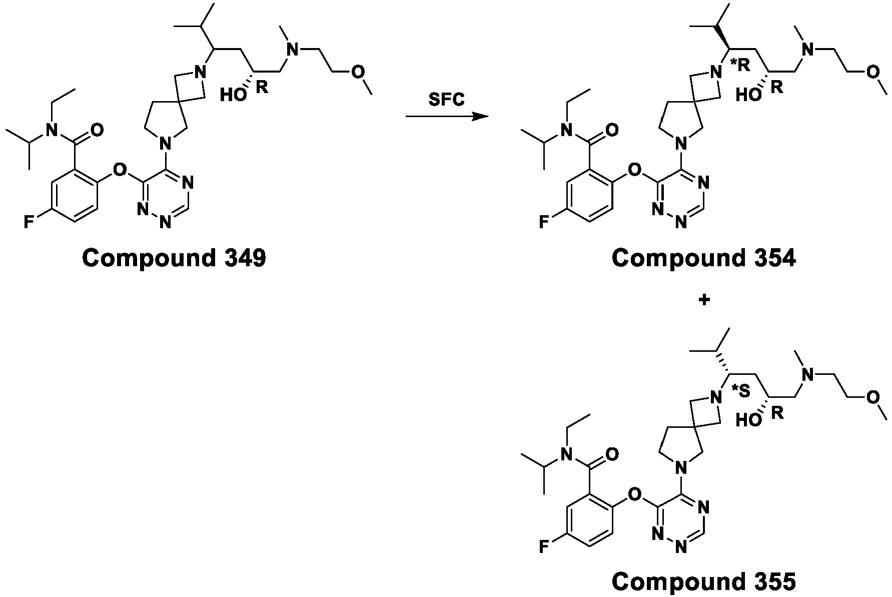

*N-ethyl-5-fluoro-2-((5-(2-((5R)-5-hydroxy-6-((2-methoxyethyl)(methyl)amino)-2-*methylhexan-3-yl)-2,6-diazaspiro[3.4]octan-6-yl)-1,2,4-triazin-6-yl)oxy)-*N-*isopropylbenzamide **(Compound 349)** (200 mg) was separated by SFC over DAICEL CHIRALPAK IG (column: 250×30 mm, 10 um; Mobile phase: A: Supercritical CO₂, B: EtOH (0.1% ammonia), A:B = 50:50 at 80 mL/min) to afford the title compounds **(Compound 354)** (100 mg) and **(Compound 355)** (70 mg) both as colorless sticky solid.

### Compound 361 and 362

### 2-((5-(2-((3*R)-6-(dimethylamino)-5-hydroxy-2-methylhexan-3-yl)-2,6-diazaspiro[3.4]octan-6-yl)-1,2,4-triazin-6-yl)oxy)-5-fluoro-N,N-diisopropylbenzamide

### 2-((5-(2-((3*S)-6-(dimethylamino)-5-hydroxy-2-methylhexan-3-yl)-2,6-diazaspiro[3.4]octan-6-yl)-1,2,4-triazin-6-yl)oxy)-5-fluoro-N,N-diisopropylbenzamide

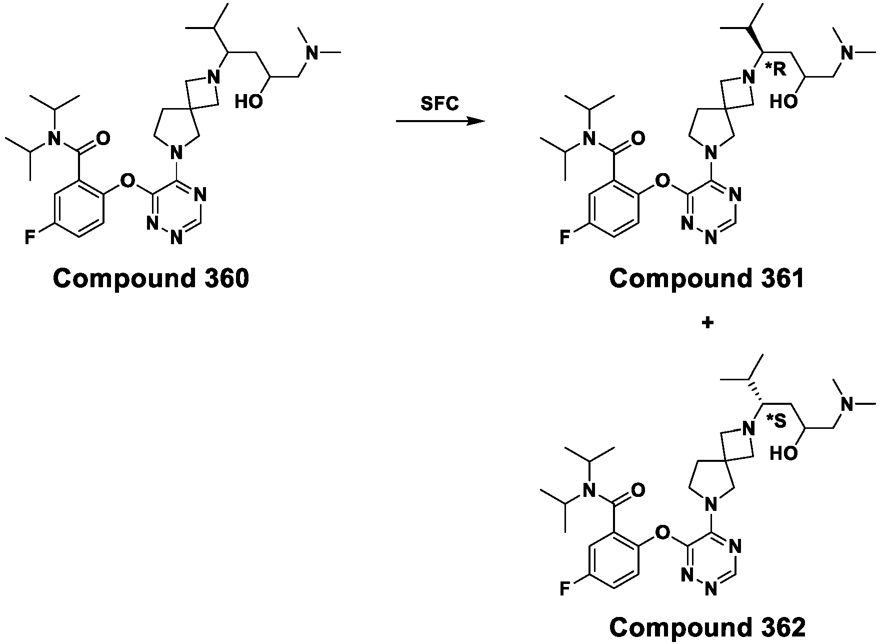

2-((5-(2-(6-(dimethylamino)-5-hydroxy-2-methylhexan-3-yl)-2,6-diazaspiro[3.4]octan-6-yl)-1,2,4-triazin-6-yl)oxy)-5-fluoro-*N,N*-diisopropylbenzamide **(Compound 360)** (250 mg) was separated by SFC over DAICEL CHIRALPAK IG (column: 250×30 mm, 10 um; Mobile phase: A: Supercritical CO₂, B: IPA (0.1% ammonia), A:B = 40:40 at 80 mL/min) to afford the title compounds **(Compound 361)** (105 mg) and **(Compound 362)** (120 mg) both as white solid.

### Compound 363 and 364

### 2-((5-(2-((3 *R,5*R)-6-(dimethylamino)-5-hydroxy-2-methylhexan-3-yl)-2,6-diazaspiro[3.4]octan-6-yl)-1,2,4-triazin-6-yl)oxy)-5-fluoro-N,N-diisopropylbenzamide

### 2-((5-(2-((3 *R,5 *S)-6-(dimethylamino)-5-hydroxy-2-methylhexan-3-yl)-2,6-diazaspiro[3.4]octan-6-yl)-1,2,4-triazin-6-yl)oxy)-5-fluoro-N,N-diisopropylbenzamide

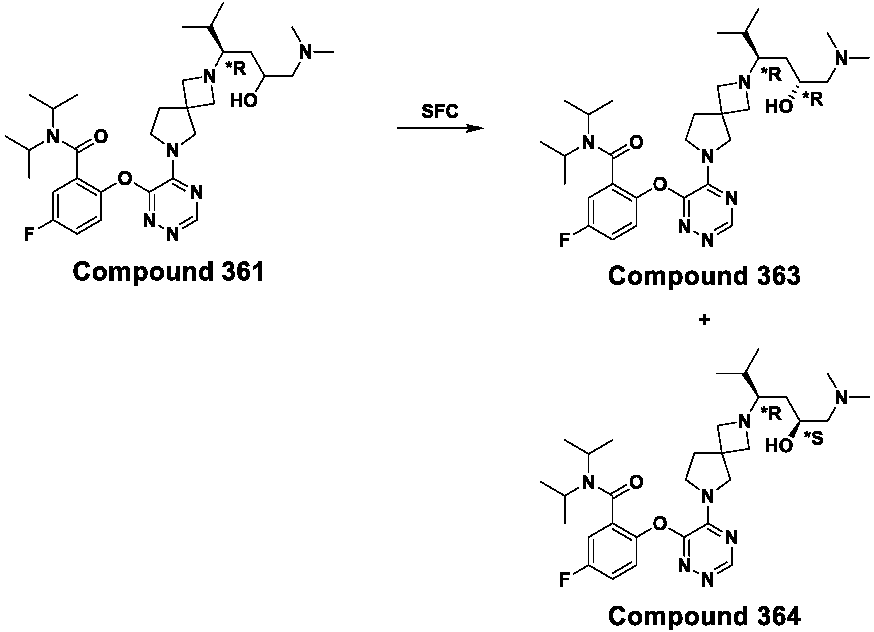

2-((5-(2-((3**R*)-6-(dimethylamino)-5-hydroxy-2-methylhexan-3-yl)-2,6-diazaspiro[3.4]octan-6-yl)-1,2,4-triazin-6-yl)oxy)-5-fluoro-N,N-diisopropylbenzamide **(Compound 361)** (105 mg) was separated by SFC over Phenomenex-Cellulose-2 (column: 250x30 mm, 10 um; Mobile phase: A: Supercritical CO₂, B: 0.1%NH₃H₂O EtOH (0.1% ammonia), A:B = 65:35 at 80 mL/min) to afford the title compounds **(Compound 363)** (45 mg) and **(Compound 364)** (35 mg) both as colorless sticky solid.

### Compound 367 and 368

### 2-((5-(2-((3 *S,5*S)-6-(dimethylamino)-5-hydroxy-2-methylhexan-3-yl)-2,6-diazaspiro[3.4]octan-6-yl)-1,2,4-triazin-6-yl)oxy)-5-fluoro-N,N-diisopropylbenzamide

### 2-((5-(2-((3 *S,5 *R)-6-(dimethylamino)-5-hydroxy-2-methylhexan-3-yl)-2,6-diazaspiro[3.4]octan-6-yl)-1,2,4-triazin-6-yl)oxy)-5-fluoro-N,N-diisopropylbenzamide

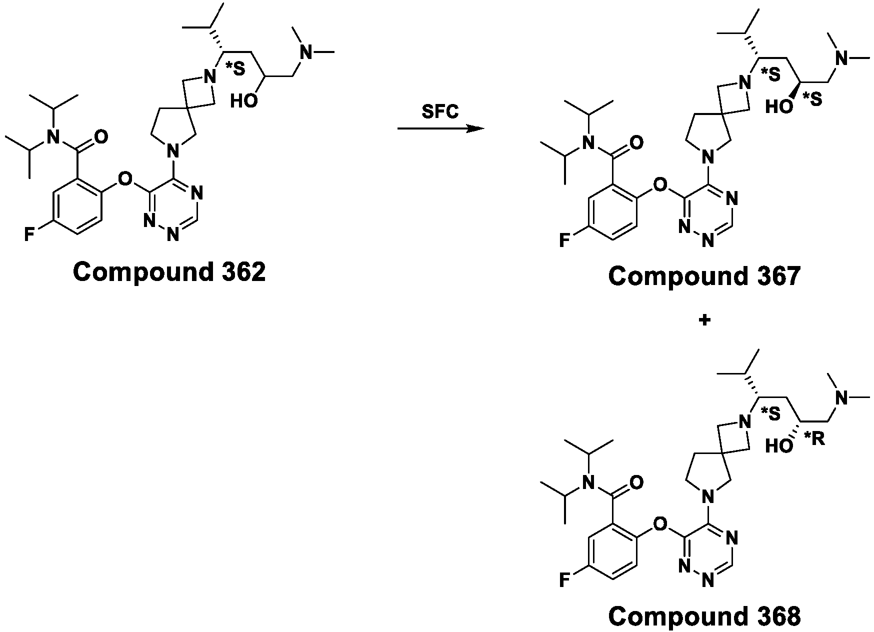

2-((5-(2-((3**S*)-6-(dimethylamino)-5-hydroxy-2-methylhexan-3-yl)-2,6-diazaspiro[3.4]octan-6-yl)-1,2,4-triazin-6-yl)oxy)-5-fluoro-*N,N*-diisopropylbenzamide **(Compuond 362)** (120 mg) was separated by SFC over DAICEL CHIRALPAK AS (column: 250×30 mm, 10um; Mobile phase: A: Supercritical CO₂, B: EtOH (0.1% ammonia), A:B = 75:25 at 60 mL/min) to afford the title compounds **(Compound 367)** (48 mg) and **(Compound 368)** (34 mg) both as colorless oil.

### Compound 384 and 385

### N-ethyl-5-fluoro-2-((5-(2-((3*R,5*R)-5-hydroxy-2-methyl-6-(methyl(propyl)amino)hexan-3-yl)-2,6-diazaspiro[3.4]octan-6-yl)-1,2,4-triazin-6-yl)oxy)-N-isopropylbenzamide

### N-ethyl-5-fluoro-2-((5-(2-((3*S,5*S)-5-hydroxy-2-methyl-6-(methyl(propyl)amino)hexan-3-yl)-2,6-diazaspiro[3.4]octan-6-yl)-1,2,4-triazin-6-yl)oxy)-N-isopropylbenzamide

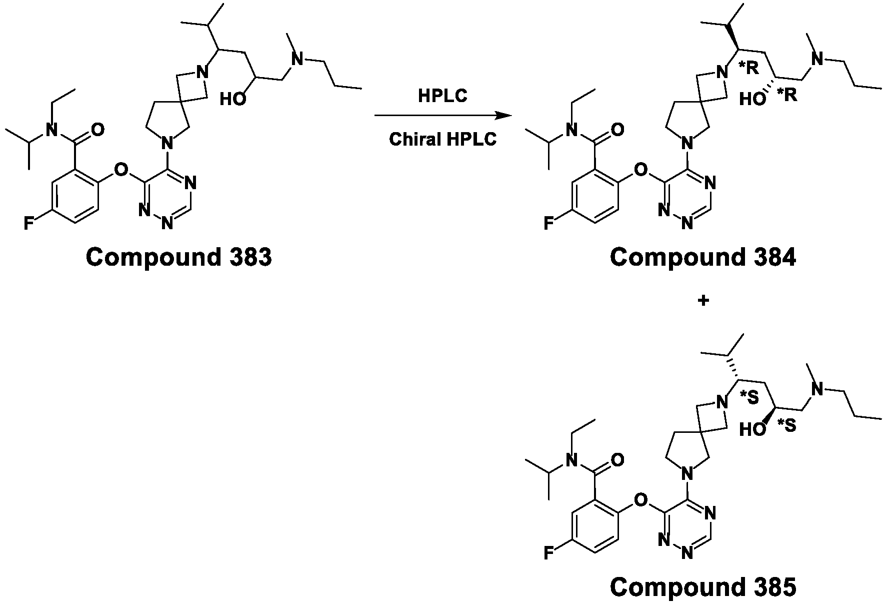

*N-ethyl-5-fluoro-2-((5-(2-(5-hydroxy-2-methyl-6-(methyl(propyl)amino)hexan-3-yl)-2,6-***diazaspiro[3.4]octan-6-yl)-1,2,4-triazin-6-yl)oxy)-*N*-isopropylbenzamide (Compound 383)** (432 mg) was purified by preparative HPLC over Welch Xtimate (column: C18 100×40 mm 3 µm, Mobile Phase A: H₂O (0.075%TFA), Mobile Phase B: ACN, Flow rate: 30 mL/min, gradient condition: from 10% B to 40% B) to afford a mixture of **Compound 384** and **Compound 385** (166 mg, as TFA salt).

The mixture of **Compound 384** and **Compound 385** (166 mg, TFA salt) was further separated by chiral HPLC over Daicel ChiralPak IG (column: 250×30 mm, 10 µm; Mobile phase: A: Heptane, B: EtOH (0.1% ammonia), Flow rate: 25mL/min, gradient condition: from 20% B to 50% B) to afford the title compounds **(Compound 384)** (30.7 mg) and **(Compound 385)** (14.4 mg) both as colorless sticky oil.

### Compound 389 and 390

### 2-((5-(2-((3 *R,5*S)-6-(ethyl(methyl)amino)-5-hydroxy-2-methylhexan-3-yl)-2,6-diazaspiro[3.4]octan-6-yl)-1,2,4-triazin-6-yl)oxy)-5-fluoro-N,N-diisopropylbenzamide

### 2-((5-(2-((3*S,5*S)-6-(ethyl(methyl)amino)-5-hydroxy-2-methylhexan-3-yl)-2,6-diazaspiro[3.4]octan-6-yl)-1,2,4-triazin-6-yl)oxy)-5-fluoro-N,N-diisopropylbenzamide

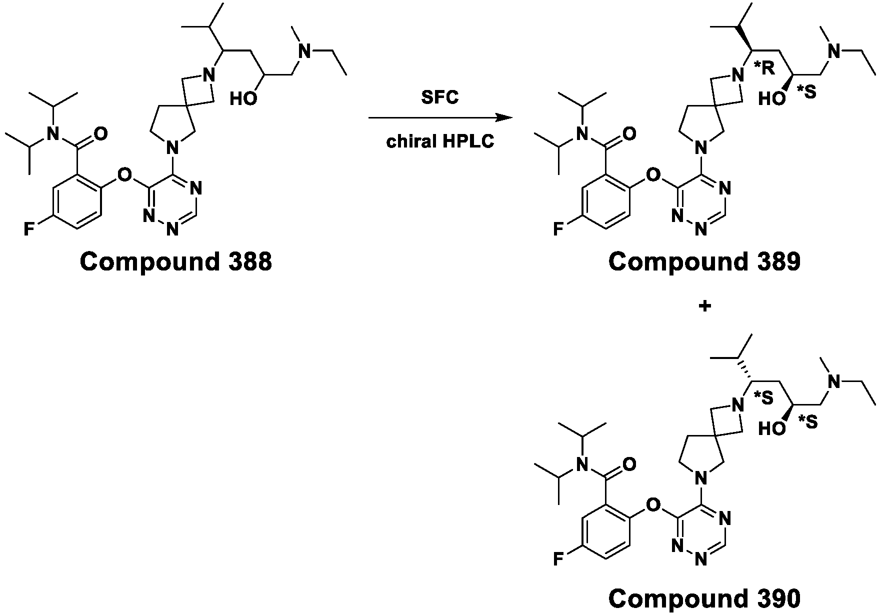

2-((5-(2-(6-(ethyl(methyl)amino)-5-hydroxy-2-methylhexan-3-yl)-2,6-diazaspiro[3.4]octan-6-yl)-1,2,4-triazin-6-yl)oxy)-5-fluoro-*N,N*-diisopropylbenzamide **(Compound 388)** (190 mg) was first separated by SFC over Daicel chiralpak IG (column: 250×30 mm, 10 µm; Mobile phase: A: Supercritical CO₂, B: EtOH (0.1% ammonia), A:B = 60:40; Flow rate: 80 mL/min) to afford **Compound 390** (45 mg) and a mixture of 3 diastereoisomers. (120 mg).

The mixture of 3 diastereoisomers (120 mg) was further separated by chiral HPLC over Daicel Daicel chiralpak IG (column: 250×30 mm, 10 µm), Mobile phase: A: Heptane, B: EtOH (0.1% ammonia), A:B = from 70:30 to 50:50, Flow rate: 25 mL/min) to afford **Compound 389** (22.0 mg, 86.6% purity by LCMS).

The **Compound 389** (22.0 mg, 86.6% purity by LCMS) was further purified by preparative HPLC over Welch Xtimate (column: C18 150×25 mm 5 µm, Mobile phase: A: H₂O (0.2% FA), B: ACN, gradient condition: from 2% B to 32%, Flow rate: 25 mL/min) and basified with ammonia to afford **Compound 389** (15.0 mg, 100% purity by LCMS).

### Compound 393

### (R)-2-((3-chloro-5-(2-(6-((2-methoxyethyl)(methyl)amino)-2-methylhexan-3-yl)-2,6-diazaspiro[3.4]octan-6-yl)-1,2,4-triazin-6-yl)oxy)-N-ethyl-5-fluoro-N-isopropylbenzamide

### Preparation Method A:

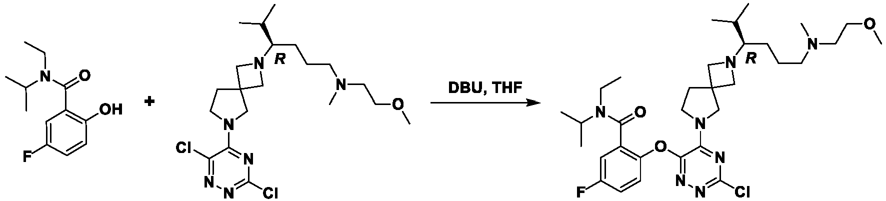

The mixture of N-ethyl-5-fluoro-2-hydroxy-N-isopropylbenzamide **(intermediate 28)** (1.10 g, 4.88 mmol), (R)-4-(6-(3,6-dichloro-1,2,4-triazin-5-yl)-2,6-diazaspiro[3.4]octan-2-yl)-N-(2-methoxyethyl)-*N*,5-dimethylhexan-1-amine **(intermediate 225)** (1.70 g, 3.82 mmol) and DBU (750 mg, 4.93 mmol) in anhydrous THF (15 mL) was stirred at 40 °C for 8 h. After cooled to RT, the mixture was concentrated under reduced pressure, the resulting residue was diluted with DCM (60 mL) and washed with H₂O (20 mL × 3). The organic layer was dried over anhydrous Na₂SO₄, filtered and concentrated under reduced pressure to give the crude product which was purified FCC (MeOH/DCM = 0% to 10%) to afford a yellow oil (1.40 g), which was further separated by SFC over DAICEL CHIRALPAK AD (column: 250×50 mm,10 um; Mobile phase: A: Supercritical CO₂, B: EtOH (0.1% ammonia), A:B = 50:50 at 70 mL/min; Column Temp: 38 °C; Nozzle Pressure: 100Bar; Nozzle Temp: 60 °C; Evaporator Temp: 20 °C; Trimmer Temp: 25 °C; Wavelength: 220nm) to afford the title compound (1.0 g).^{.}

### Preparation Method B:

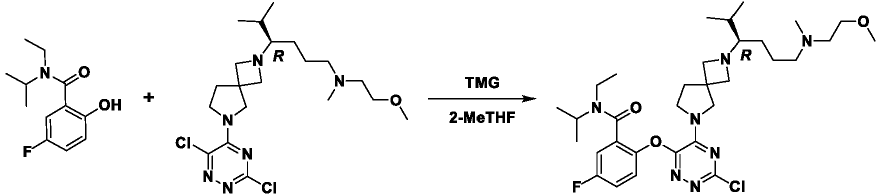

To a 2-MeTHF solution of (*R*)-4-(6-(3,6-dichloro-1,2,4-triazin-5-yl)-2,6-diazospiro[3.4]octan-2-yl)-*N*-(2-methoxyethyl)-*N*,5-dimethylhexan-1-amine **(intermediate 225)** (676g of a 14.8 wt% solution in 2-MeTHF, 100g corrected of **intermediate 225)** and N-ethyl-5-fluoro-2-hydroxy-N-isopropylbenzamide **(intermediate 28)** (50.6 g) in 2-MeTHF (40 g) at 20 to 30°C was added tetramethylguanidine (31 g) and the mixture was stirred for 40 to 48 h. A 7% NaHCO₃ aqueous solution (500g) was added and the mixture was stirred for 30 to 60 min. The aqueous layer was removed and the organic layer was washed with twice with 4% NaOH aqueous solution (2 x 500 g) and once with 10% Na₂SO₄ aqueous solution (500 g). The organic layer was concentrated under reduced pressure (<40°C) to 2.2~3.0 vol. and flushed three times with MeOH (1 × 790g and 2 × 395g) until both 2-MeTHF and water content were both ≤1.0% to afford the desired compound in 86% assay yield as a 60.1 wt% solution in methanol.

### Compound 400, 414

### (R)-2-((3-chloro-5-(2-(6-((2-methoxyethyl)(methyl)amino)-2-methylhexan-3-yl)-2,6-diazaspiro[3.4]octan-6-yl)-1,2,4-triazin-6-yl)oxy)-N-(ethyl-¹³C₂)-5-fluoro-N-(propan-2-yl-¹³C₃)benzamide

### (R)-2-((3-chloro-5-(2-(6-((2-methoxyethyl)(methyl)amino)-2-methylhexan-3-yl)-2,6-diazaspiro[3.4]octan-6-yl)-1,2,4-triazin-6-yl)oxy)-5-fluoro-N-isopropyl-N-methylbenzamide

**The following compounds were synthesized by an analogous method described above for compound 393 by method A**

| **Co. No.** | **Structure** | **Starting Materials** |
|---|---|---|
| 400 | 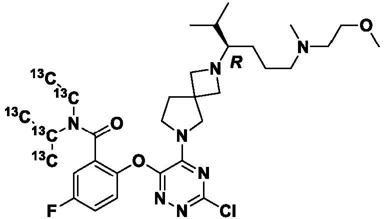 | intermediate 237, intermediate 225 |
| 414 | 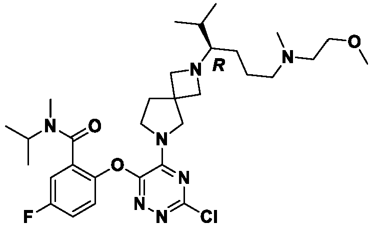 | intermediate 247, intermediate 225 |

### Compound 395

### (R)-N-ethyl-5-fluoro-N-isopropyl-2-((5-(2-(6-((2-methoxyethyl)(methyl)amino)-2-methylhexan-3-yl)-2,6-diazaspiro[3.4]octan-6-yl)-3-(methylamino)-1,2,4-triazin-6-yl)oxy)benzamide formate

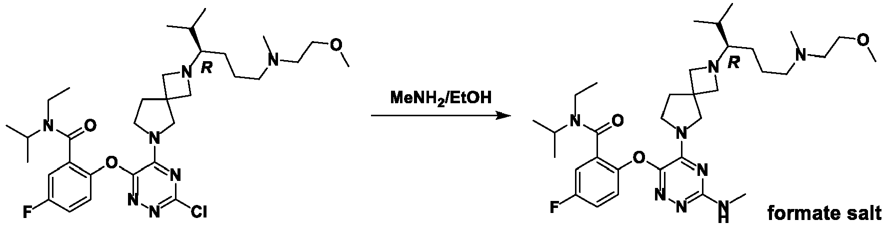

The mixture of (*R*)-2-((3-chloro-5-(2-(6-((2-methoxyethyl)(methyl)amino)-2-methylhexan-3-yl)-2,6-diazaspiro[3.4]octan-6-yl)-1,2,4-triazin-6-yl)oxy)-N-ethyl-5-fluoro-N-isopropylbenzamide **(Compound 393)** (100 mg, 0.158 mmol) and methanamine (1 mL, 33% in EtOH) was stirred at 90 °C for 1 h. After cooled to RT, the mixture was concentrated under reduced pressure to give the crude product which was purified by preparative HPLC (Column: Welch Xtimate C18 150×25 mm 5 um, Mobile Phase A: H₂O (0.2% FA), Mobile Phase B: ACN, Flow rate: 25 mL/min, gradient condition: from 5% B to 35%) to afford the title compound (49.8 mg, 43.6% yield) as sticky solid.

**LC-MS (ESI) (Method 2):** Rt = 1.997 min, m/z found 629.4 [M+H]⁺.

**SFC (Method 6):** Rt = 1.228 min.

### Compound 406 and 407

### (R)-2-((5-(2-(6-amino-2-methylhexan-3-yl)-2,6-diazaspiro[3.4]octan-6-yl)-3-chloro-1,2,4-triazin-6-yl)oxy)-N-ethyl-5-fluoro-N-isopropylbenzamide formate

### (R)-2-((5-(2-(6-amino-2-methylhexan-3-yl)-2,6-diazaspiro[3.4]octan-6-yl)-3-methoxy-1,2,4-triazin-6-yl)oxy)-N-ethyl-5-fluoro-N-isopropylbenzamide formate

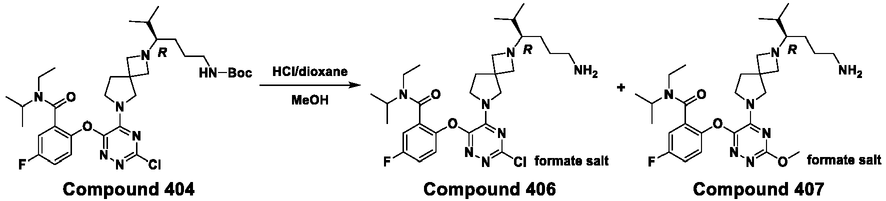

To a solution of *tert-*butyl (*R)*-(4-(6-(3-chloro-6-(2-(ethyl(isopropyl)carbamoyl)-4-fluorophenoxy)-1,2,4-triazin-5-yl)-2,6-diazaspiro[3.4]octan-2-yl)-5-methylhexyl)carbamate **(Compound 404)** (1.10 g, 1.66 mmol) in MeOH (15.0 mL) was added HCl/dioxane (15.0 mL, 60.0 mmol, 4M) and the resulting mixture was stirred at 20 °C for 12 h. The reaction mixture was concentrated under reduced pressure to give a residue which was purified by preparative HPLC over Welch Xtimate (column: C18 150×25 mm, 5um, Mobile Phase A: H₂O (0.2%FA), Mobile Phase B: ACN, Flow rate: 25 mL/min, gradient condition from 3% B to 33% B) to afford the title compounds **(Compound 406)** (360 mg) and **(Compound 407)** (160 mg) both as sticky oil.

**(Compound 406)** (60 mg) was further purified by preparative HPLC over Boston Green ODS (column: 150×30 mm, 5 um; Mobile Phase A: H₂O (0.225%FA), Mobile Phase B: ACN, Flow rate: 35 mL/min, gradient condition from 5% B to 35% B) to afford the title compound **(Compound 406)** (40 mg).

### Compound 406

**LC-MS (ESI) (Method 1):** Rt = 3.400 min, m/z found 562.3 [M+H]⁺.
**SFC (Method 32):** Rt = 2.093 min.

### Compound 407

**LC-MS (ESI) (Method 1):** Rt = 2.028 min, m/z found 558.3 [M+H]⁺.
**SFC (Method 6):** Rt = 1.42 min.

### Compound 416

### (R)-N-ethyl-5-fluoro-N-isopropyl-2-((3-methoxy-5-(2-(6-((2-methoxyethyl)(methyl)amino)-2-methylhexan-3-yl)-2,6-diazaspiro[3.4]octan-6-yl)-1,2,4-triazin-6-yl)oxy)benzamide formate

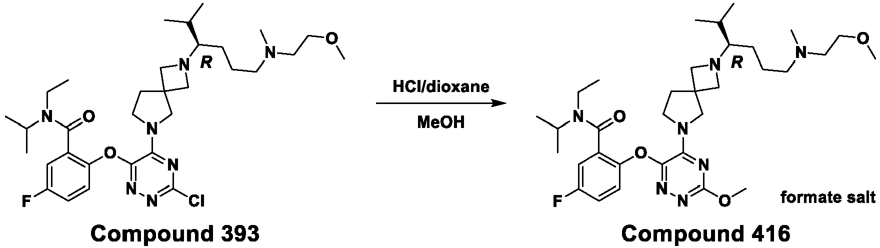

To the solution of *(R*)-2-((3-chloro-5-(2-(6-((2-methoxyethyl)(methyl)amino)-2-methylhexan-3-yl)-2,6-diazaspiro[3.4]octan-6-yl)-1,2,4-triazin-6-yl)oxy)-*N*-ethyl-5-fluoro-*N-*isopropylbenzamide **(Compound 393)** (100 mg, 0.158 mmol) in anhydrous MeOH (2 mL) was added HCl (1.6 mL, 6.40 mmol, 4 M in dioxane). The resulting mixture was stirred at 25 °C for 60 h. The mixture was concentrated under reduced pressure to give the crude product which was purified by preparative HPLC (Column: Boston Green ODS 150×30 mm 5 um, Mobile Phase A: H₂O (0.225% FA), Mobile Phase B: ACN, Flow rate: 35 mL/min, gradient condition from 12% B to 42% B) to afford the title compound (70.6 mg, 65.2% yield) as yellow sticky solid.

**LC-MS (ESI) (Method 2):** Rt = 2.096 min, m/z found 630.4 [M+H]⁺.

**SFC (Method 33):** Rt = 2.587 min.

### Compound 286

### (R)-N-ethyl-5-fluoro-2-((5-(2-(6-((2-hydroxyethyl)(methyl)amino)-2-methylhexan-3-yl)-2,6-diazaspiro[3.4]octan-6-yl)-1,2,4-triazin-6-yl)oxy)-N-isopropylbenzamide

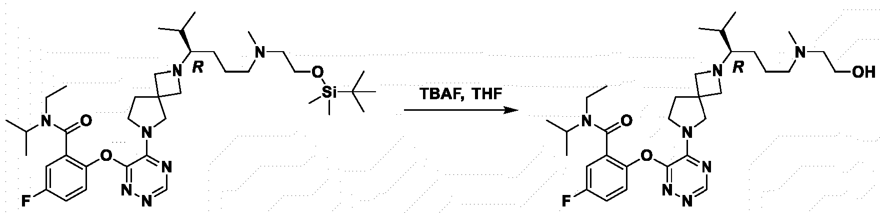

TBAF (79 µL; 0.079 mmol) was added dropwise to a solution of (*R*)-2-((5-(2-(6-((2-((*tert*butyldimethylsilyl)oxy)ethyl)(methyl)amino)-2-methylhexan-3-yl)-2,6-diazaspiro[3.4]octan-6-yl)-1,2,4-triazin-6-yl)oxy)-*N*-ethyl-5-fluoro*-N*-isopropylbenzamide **(intermediate 245)** (46 mg, 0.066 mmol) in THF (2 mL) at RT. The reaction mixture was stirred at RT for 20 h, then poured out into ice water and EtOAc was added. The mixture was basified with a 10% aqueous solution of K₂CO₃ and the organic layer was separated, washed with brine, dried over MgSO₄ and filtered. The solvent was evaporated to dryness to give a crude (45 mg) which was purified by silica gel chromatography (Stationary phase: irregular bare silica 4g, Mobile phase: 0.7% NH₄OH, 93% DCM, 7% MeOH). The fractions containing the product were mixed and concentrated. The resulting product was freeze-dried with ACN/H₂O 20/80 to give the title compound (30mg, 78% yield).

**LC-MS (ESI) (Method 4):** Rt = 3.048 min, m/z found 586.6 [M+H]⁺; 644.6 [M+CH3COO]⁻

### ANALYTICAL METHODS

The analytical information in the Compounds above or in the Tables below, was generated by using the analytical methods described below.

### NMR-Methods

Some NMR experiments were carried out using a Bruker Avance III 400 spectrometer at ambient temperature (298.6 K), using internal deuterium lock and equipped with BBO 400MHz S1 5 mm probe head with z gradients and operating at 400 MHz for the proton and 100MHz for carbon. Chemical shifts (δ) are reported in parts per million (ppm). J values are expressed in Hz.

Some NMR experiments were carried out using a Varian 400-MR spectrometer at ambient temperature (298.6 K), using internal deuterium lock and equipped with Varian 400 4NUC PFG probe head with z gradients and operating at 400 MHz for the proton and 100MHz for carbon. Chemical shifts (δ) are reported in parts per million (ppm). J values are expressed in Hz.

Some NMR experiments were carried out using a Varian 400-VNMRS spectrometer at ambient temperature (298.6 K), using internal deuterium lock and equipped with Varian 400 ASW PFG probe head with z gradients and operating at 400 MHz for the proton and 100MHz for carbon. Chemical shifts (δ) are reported in parts per million (ppm). J values are expressed in Hz.

Some NMR experiments were carried out using a Bruker AVANCE III HD 300 spectrometer at ambient temperature (298.6 K), using internal deuterium lock and equipped with PA BBO 300S1 BBF-H-D-05 Z 5 mm probe head with z gradients and operating at 300 MHz for the proton and 75 MHz for carbon. Chemical shifts (d) are reported in parts per million (ppm). J values are expressed in Hz.

### LCMS (Liquid chromatography/Mass spectrometry)

### General procedure

The High Performance Liquid Chromatography (HPLC) measurement was performed using a LC pump, a diode-array (DAD) or a UV detector and a column as specified in the respective methods. If necessary, additional detectors were included (see table of methods below).

Flow from the column was brought to the Mass Spectrometer (MS) which was configured with an atmospheric pressure ion source. It is within the knowledge of the skilled person to set the tune parameters (e.g. scanning range, dwell time...) in order to obtain ions allowing the identification of the compound's nominal monoisotopic molecular weight (MW). Data acquisition was performed with appropriate software.

Compounds are described by their experimental retention times (Rt) and ions. If not specified differently in the table of data, the reported molecular ion corresponds to the [M+H]⁺ (protonated molecule) and/or [M-H]⁻ (deprotonated molecule). In case the compound was not directly ionizable the type of adduct is specified (i.e. [M+NH₄]⁺, [M+HCOO]⁻, etc...). For molecules with multiple isotopic patterns (Br, Cl..), the reported value is the one obtained for the lowest isotope mass. All results were obtained with experimental uncertainties that are commonly associated with the method used.

Hereinafter, "SQD" means Single Quadrupole Detector, "RT" room temperature, "BEH" bridged ethylsiloxane/silica hybrid, "HSS" High Strength Silica, "DAD" Diode Array Detector.

**Table 1a. LCMS Method Codes (Flow expressed in mL/min; column temperature (T) in °C; Run time in minutes).**

| Method code | Instrument | Column | Mobile phase | Gradient | | | $\frac{Flow}{Column T}$ | Run time |
|---|---|---|---|---|---|---|---|---|
| 1 | Agilent | Waters XBridge C18 (2.0x50 mm, 5 uM) | mobile phase A: H₂O with 0.04 % TFA; mobile phase B: ACN with 0.02 % TFA | 100%A was held for 1 min, A gradient from 100% A to 40% A is applied in 4 min, and 40%A down to 15%A in 2.5 min. And then return to 100%A in 2 min and held for 0.5 min. The post time is 0.5 min. | | | $\frac{0.8}{50}$ | 10 |
| 2 | Agilent | Waters XBridge C18 (2.0x50 mm, 5 um) | mobile phase A: H₂O with 0.04 % TFA; mobile phase B: ACN with 0.02 % TFA | First, 90% A was held for 0.8 min. Then a gradient was applied to 20% A and 80% B in 3.7 min and held for 3 min. And then return to 90% A in 2 min and held for 0.5 min. The post time is 0.5 min. | | | $\frac{0.8}{50}$ | 10 |
| 3 | Agilent | Waters XBridge Shield RP18 (2.1x50 mm, 5 um) | mobile phase A: H₂O with 0.05% ammonia; mobile phase B: ACN | First, 100% A was held for min. Then a gradient was applied to 40% A and 60 % B in 4 min and then to 5% A and 95% B in 2.5 min. Finally return to 100% A in 2 min and held for 0.5 min. Post Time is 0.5 min. | 1 | | $\frac{0.8}{40}$ | 10 |
| 4 | Waters: Acquity UPLC^{®}-DAD and Quattro Micro^{™} | Waters BEH C18 (2.1x100 mm, 1.7 uM) | mobile phase A: 95% CH₃COONH₄ 7mM / 5%ACN mobile phase B: ACN | 84.2% A for 0.49min, to 10.5% A in 2.18min, held for 1.94min, back to 84.2% A in 0.73min, held for 0.73min. | | | $\frac{0.343}{40}$ | 6.2 |
| 5 | Agilent 1260/6120 | Waters Sunfire C18 (2.0x30 mm, 2.5 uM) | mobile phase A: H₂O with 0.1 % FA; mobile phase B: ACN | gradient from 5% B to 95% B is applied in 2.5 min, and held for 1.0 min. The post time is 0.8 min. | | | $\frac{1}{40}$ | 3.5 |
| 6 | Agilent 1260/6120 | Waters Sunfire C18 (2.0x30 mm, 2.5 uM) | mobile phase A: H₂O with 0.1 % FA; mobile phase B: ACN | 5% B was held for 1 min, gradient from 5% B to 30% B is applied in 2 min, and 30% B to 95% B in 0.5 min, and held for 1.0 min. The post time is 0.8 min. | | | $\frac{1}{40}$ | 4.5 |
| 7 | Agilent LC 1260 with MS6120 | XBridge C18, 4.6 ×150 mm, 3.5 µm | Mobile phase A 0.05% TFA in H₂O Mobile phase B 0.05 % TFA in ACN | Time (min) | A% | B% | $\frac{1.5}{45}$ | 20 |
| | | | | Initial | 95 | 5 | | |
| | | | | 11.0 | 65 | 35 | | |
| | | | | 13.0 | 5 | 95 | | |
| | | | | 15.0 | 5 | 95 | | |
| | | | | 16.0 | 95 | 5 | | |
| | | | | 20.0 | 95 | 5 | | |
| 8 | Waters: Acquity^{®} H-Class - DAD and SQD₂^{™} | Waters BEH^{®} C18 (1.7µm, 2.1x50mm) | A: CH₃COONH₄ 7mM 95%/ CH₃CN 5%, B: CH₃CN | From 95% A/5%B to 5% Ain 1min, held for 1.6min, back to 95% A/5%B in 0.2min, held for 0.5min. | | | $\frac{0.5}{40}$ | 3.5 |

### Analytical SFC

### General procedure for SFC methods

The SFC measurement was performed using an Analytical Supercritical fluid chromatography (SFC) system composed by a binary pump for delivering carbon dioxide (CO₂) and modifier, an autosampler, a column oven, a diode array detector equipped with a high-pressure flow cell standing up to 400 bars. If configured with a Mass Spectrometer (MS) the flow from the column was brought to the (MS). It is within the knowledge of the skilled person to set the tune parameters (e.g. scanning range, dwell time...) in order to obtain ions allowing the identification of the compound's nominal monoisotopic molecular weight (MW). Data acquisition was performed with appropriate software.

**Table 2a. Analytical SFC Methods (Flow expressed in mL/min; column temperature (T) in °C; Run time in minutes, Backpressure (BPR) in bars or pound-force per square inch (psi). "ACN" means acetonitrile; "MeOH" means methanol; "EtOH" means ethanol; "DEA" means diethylamine. All other abbreviations used in the table below are as defined before)**

| **Method code** | **column** | **mobile phase** | **gradient** | **Flow** | **Run time** |
|---|---|---|---|---|---|
| | | | | **Col T** | **BPR** |
| 1 | Waters UPCC with PDA (Chiralpak IG-3 100×4.6 mm I.D., 3 um) | A: Supercritical CO₂ | 40% of MeOH (0.05% DEA) in CO₂ | 3.2 | 10 |
| | | | | 35 | 1500 psi |
| | | B: MeOH (0.05% DEA) | | | |
| 2 | Waters UPCC with PDA (Chiralpak IG-3 100×4.6 mm I.D., 3 um) | A: Supercritical CO₂ | from 5% to 40% of B in 4 min and hold 40% for 2.5 min, then 5% of B for 1.5 min | 2.8 | 8 |
| | | | | 35 | 1500 psi |
| | | B: IPA (0.05% DEA) | | | |
| 3 | Agilent 1260 with DAD (ChiralPak AD-3 150×4.6 mm I.D., 3 um) | A: Supercritical CO₂ B: EtOH (0.05% DEA) | from 5% to 40% of B in 5.5 min and hold 40% for 3 min, then 5% of B for 1.5 min | 2.5 | 10 |
| | | | | 40 | 100 bar |
| 4 | Waters UPCC with PDA (Chiralpak AD-3 50×4.6 mm I.D., 3 um) | A: Supercritical CO₂ | from 5% to 40% of B in 2 min and hold 40% for 1.2 min, then 5% of B for 0.8 min | 4 | 4 |
| | | | | 35 | 1500 psi |
| | | B: IPA (0.05% DEA) | | | |
| 5 | Waters UPCC with PDA (Chiralpak IG-3 100×4.6 mm I.D., 3 um) | A: Supercritical CO₂ | 40% of MeOH (0.05% DEA) in CO₂ | 3.2 | 3 |
| | | | | 35 | 1500 psi |
| | | B: MeOH (0.05% DEA) | | | |
| 6 | Waters UPCC with PDA (Chiralpak AD-3 50×4.6 mm I.D., 3 um) | A: Supercritical CO₂ B: EtOH (0.05% DEA) | from 5% to 40% of B in 2 min and hold 40% for 1.2 min, then 5% of B for 0.8 min | 4 | 4 |
| | | | | 35 | 1500 psi |
| 7 | Waters UPCC with PDA (Chiralpak IG-3 100×4.6 mm I.D., 3 um) | A: Supercritical CO₂ | 25% of MeOH (0.05% DEA) in CO₂ | 3.5 | 9 |
| | | | | 35 | 1500 psi |
| | | B: MeOH (0.05% DEA) | | | |
| 8 | Waters UPCC with PDA (Chiralpak IG-3 50×4.6 mm I.D., 3 um) | A: Supercritical CO₂ B: EtOH (0.05% DEA) | from 5% to 40% of B in 2 min and hold 40% for 1.2 min, then 5% of B for 0.8 min | 4 | 4 |
| | | | | 35 | 1500 psi |
| 9 | Waters UPCC with PDA (Cellulose 2 150×4.6 mm I.D., 5um) | A: Supercritical CO₂ B: EtOH (0.05% DEA) | 50% B hold for 10 min | 2.5 | 11 |
| | | | | 35 | 1500 psi |
| 10 | Agilent 1260 with DAD (ChiralPak AS-3 150×4.6 mm I.D., 3 um) | A: Supercritical CO₂ B: EtOH (0.05% DEA) | from 5% to 40% of B in 5.5min, then 5% of B for 1.5 min | 2.5 | 7 |
| | | | | 40 | 100 bar |
| 11 | Waters UPCC with PDA (Chiralpak IG-3 100×4.6 mm I.D., 3 um) | A: Supercritical CO₂ B: EtOH (0.05% DEA) | from 5% to 40% of B in 4 min and hold 40% for 2.5 min, then 5% of B for 1.5 min | 2.8 | 8 |
| | | | | 35 | 1500 psi. |
| 12 | Agilent 1260 with DAD (ChiralPak IG-3 100×4.6 mm I.D., 3 um) | A: Supercritical CO₂ B: MeOH (0.1% Ethanolamine) | Isocratic: 40% B | 3 | 3 |
| | | | | 40 | 100 bar |
| 13 | Waters UPCC with PDA (Chiralpak AS-3 100×4.6 mm I.D., 3 um) | A: Supercritical CO₂ | from 5% to 40% of B in 4 min and hold 40% for 2.5 min, then 5% of B for 1.5 min | 2.8 | 8 |
| | | | | 35 | 1500 psi |
| | | B: EtOH (0.05% DEA) | | | |
| 14 | Waters UPCC with PDA (Chiralcel OD-3 100×4.6 mm I.D., 3 um) | A: Supercritical CO₂ | from 5% to 40% of B in 4 min and hold 40% for 2.5 min, then 5% of B for 1.5min | 2.8 | 8 |
| | | | | 35 | 1500 psi |
| | | B: EtOH (0.05% DEA) | | | |
| 15 | Waters UPCC with PDA (Chiralcel OD-3 50×4.6 mm I.D., 3 um) | A: Supercritical CO₂ | from 5% to 40% of B in 2 min and hold 40% for 1.2 min, then 5% of B for 0.8 min | 4 | 4 |
| | | | | 35 | 1500 psi |
| | | B: EtOH (0.05% DEA) | | | |
| 16 | Waters UPCC with PDA (Chiralpak AD-3 150×4.6 mm I.D., 3 um) | A: Supercritical CO₂ | from 5% to 40% of B in 5 min and from 40% to 5% of B in 0.5min, hold 5% of B for 1.5 min | 2.5 | 7 |
| | | | | 35 | 1500 psi |
| | | B: IPA (0.05% DEA) | | | |
| 17 | Waters UPCC with PDA (Cellulose-4 100×4.6 mm I.D., 3 um) | A: Supercritical CO₂ | Isocratic: 40% B | 28 | 6 |
| | | | | 35 | 1500 psi |
| | | B: EtOH (0.05% DEA) | | | |
| 18 | Waters UPCC with PDA (Chiralpak IG-3 50×4.6 mm I.D., 3 um) | A: Supercritical CO₂ | from 5% to 40% of B in 2 min and hold 40% for 1.2 min, then 5% of B for 0.8 min | 1 | 10 |
| | | | | 35 | 1500 psi |
| | | B: MeOH (0.05% DEA) | | | |
| 19 | Agilent 1260 with DAD (ChiralPak IG-3 100×4.6 mm I.D., 3 um) | A: Supercritical CO₂ | Isocratic: 40% B | 2.5 | 7 |
| | | | | 40 | 1500 psi |
| | | B: IPA (0.05% DEA) | | | |
| 20 | Agilent 1260 with DAD (ChiralPak AD-3 150×4.6 mm I.D., 3 um) | A: Supercritical CO₂ | Isocratic: 40% B | 2.5 | 5 |
| | | | | 40 | 1500 psi |
| | | B: EtOH (0.05% DEA) | | | |
| 21 | Waters UPCC with PDA (Chiralpak AD-3 150×4.6 mm I.D., 3 um) | A: Supercritical CO₂ | from 5% to 40% of B in 5 min and hold 40% for 2.5 min, then 5% of B for 2.5 min | 2.5 | 10 |
| | | | | 35 | 1500 psi |
| | | B: IPA (0.05% DEA) | | | |
| 22 | Waters UPCC with PDA (Chiralpak IG-3 100×4.6 mm I.D., 3 um) | A: Supercritical CO₂ | from 5% to 40% of B in 4 min and hold 40% for 2.5 min, then hold 5% of B for 1.5 min | 2.8 | 8 |
| | | | | 35 | 1500 psi |
| | | B: MeOH (0.05% DEA) | | | |
| 23 | Agilent 1260 with DAD (ChiralPak AD-3 150×4.6 mm I.D., 3 um) | A: Supercritical CO₂ | from 5% to 40% of B in 5.5min and hold 40% for 3 min, then 5% of B for 1.5 min | 2.5 | 10 |
| | | | | 40 | 100 bar |
| | | B: IPA (0.05% DEA) | | | |
| 24 | Waters UPCC with PDA (Chiralcel OJ-3 150×4.6 mm I.D., 3 um) | A: Supercritical CO₂ | from 5% to 40% of B in 5min and from 40% to 5% of B in 0.5min, hold 5% of B for 1.5 min | 2.5 | 7 |
| | | | | 35 | 1500 psi |
| | | B: EtOH (0.05% DEA) | | | |
| 25 | Waters UPCC with PDA (Chiralpak AS-3 100×4.6 mm I.D., 3 um) | A: Supercritical CO₂ | from 5% to 40% of B in 2 min and hold 40% for 2.5 min, then 5% of B for 1.5 min | 2.8 | 6 |
| | | | | 35 | 1500 psi |
| | | B: EtOH (0.05% DEA) | | | |
| 26 | Waters UPCC with PDA (Chiralpak AD-3 150×4.6 mm I.D., 3 um) | A: Supercritical CO₂ | from 5% to 40% of B in 5 min and hold 40% for 2.5 min, then 5% of B for 2.5 min | 2.5 | 10 |
| | | | | 35 | 1500 psi |
| | | B: EtOH (0.05% DEA) | | | |
| 27 | Waters UPCC with PDA (Chiralpak IG-3 100×4.6 mm I.D., 3um) | A: Supercritical CO₂ | Isocratic: 40% B | 3.2 | 9 |
| | | | | 35 | 1500 psi |
| | | B: EtOH (0.05% DEA) | | | |
| 28 | Agilent 1260 with DAD (ChiralPak IG-3 100×4.6 mm I.D., 3 um) | A: Supercritical CO₂ | Isocratic: 40% B | 2.5 | 6 |
| | | | | 40 | 100 bar |
| | | B:MeOH (0.05% DEA) | | | |
| 29 | Waters UPCC with PDA (Chiralpak AD-3 150×4.6 mm I.D., 3 um) | A: Supercritical CO₂ | from 5% to 40% of B in 5 min and from 40% to 5% of B in 0.5min, hold 5% of B for 1.5 min | 2.5 | 7 |
| | | | | 35 | 1500 psi |
| | | B: EtOH (0.05% DEA) | | | |
| 30 | Waters UPCC with PDA (Cellulose-2 100×4.6 mm I.D., 3 um) | A: Supercritical CO₂ | Isocratic: 40% B | 2.8 | 5 |
| | | | | 35 | 1500 psi |
| | | B: EtOH (0.05% DEA) | | | |
| 31 | Agilent 1260 with DAD (ChiralPak IG-3 100×4.6 mm I.D., 3 um) | A: Supercritical CO₂ | Isocratic: 40% B | 2.5 | 6 |
| | | | | 40 | 100 bar |
| | | B: EtOH (0.05% DEA) | | | |
| 32 | Waters UPCC with PDA | A: Supercritical CO₂ | from 5% to 40% of B in 4 min and hold 40% for 2.5 min, then 5% of B for 1.5 min | 2.8 | 8 |
| | | | | 35 | 1500 psi |
| | Chiralcel OJ-3 100×4.6 mm I.D., 3 um | B: EtOH (0.05% DEA) | | | |
| 33 | Waters UPCC with PDA | A: Supercritical CO₂ | from 5% to 40% of B in 4 min and hold 40% for 0.5 min, then 5% of B for 1.5 min | 2.8 | 6 |
| | | | | 35 | 1500 psi |
| | Chiralcel OD-3 100×4.6 mm I.D., 3 um | B: EtOH (0.05% DEA) | | | |

### Analytical chiral HPLC

### General method

The Chiral HPLC measurement was performed using a Chiral High Performance Liquid Chromatography (Chiral HPLC) system composed by a LC pump, a diode-array (DAD) or a UV detector and a chiral column as specified in the respective methods. Data acquisition was performed with appropriate software.

**Table 2b. Analytical chiral HPLC Methods (Flow expressed in mL/min; column temperature (T) in °C; Run time in minutes, Backpressure (BPR) in bars or pound-force per square inch (psi). "ACN" means acetonitrile; "MeOH" means methanol; "EtOH" means ethanol; "DEA" means diethylamine. All other abbreviations used in the table below are as defined before)**

| **Method code** | **column** | **mobile phase** | **gradient** | **Flow** | **Run time** |
|---|---|---|---|---|---|
| | | | | **Col T** | **BPR** |
| 1 | Shimadzu LC-20AB with PDA (Lux Cellulose 2 150×4.6 mm I.D., 3 um) | A: Hexane (0.1% DEA) | Isocratic: A:B = 70:30 | 1 | 15 |
| | | | | 35 | 1500 psi |
| | | B:EtOH (0.1% DEA) | | | |
| 2 | Agilent 1260 with DAD (IE-3 150×4.6 mm I.D., 3 um) | MeOH (0.05% DEA) | hold 100% of MeOH (0.05% DEA) for 20 min | 1 | 20 |
| | | | | 25 | 100 bar |
| 3 | Shimadzu LC-20AT (CHIRALPAK AD-3 150×4.6 mm I.D., 5 um) | A: Hexane | Isocratic: A:B = 90:10 | 1 | 11 |
| | | | | 35 | 1500 psi |
| | | B: EtOH | | | |
| 4 | Shimadzu LC-20AB with PDA (Chiralpak IG-3 50×4.6 mm I.D., 3 um) | A: Hexane (0.1% DEA) | Isocratic: A:B = 80:20 | 1 | 15 |
| | | | | 35 | 1500 psi |
| | | B: IPA | | | |
| 5 | Shimadzu LC-20AT CP-HPLC-09 (CHIRALPAK AD-H 150×4.6 mm I.D., 5 um) | A: Hexane (0.1% DEA) | Isocratic: A:B = 90 : 10 | 1 | 15 |
| | | | | 35 | 1500 psi |
| | | B: EtOH (0.1% DEA) | | | |
| 6 | Shimadzu LC-20AB with PDA (Chiralpak IG-3 50×4.6 mm I.D., 3 um) | A: Hexane (0.1% DEA) | Isocratic: A:B = 70:30 | 1 | 10 |
| | | | | 35 | 1500 psi |
| | | B: EtOH (0.1% DEA) | | | |
| 7 | Shimadzu LC-20AB with PDA (Chiralpak IG-3 50×4.6 mm I.D., 3 um) | A: Hexane (0.1% DEA) | Isocratic: A:B = 80 : 20 | 1 | 10 |
| | | | | 35 | 1500 psi |
| | | B: EtOH (0.1% DEA) | | | |
| 8 | Shimadzu LC-20AD with PDA (Chirapak IE 100×4.6 mm I.D., 3 um) | A: Hexane (0.1% DEA) | Isocratic: A:B = 40:60 | 1 | 15 |
| | | | | 35 | 1500 psi |
| | | B: EtOH (0.1% DEA) | | | |
| 9 | Shimadzu LC-20AD with PDA (Chirapak ID 100×4.6 mm I.D., 3 um) | A: Hexane (0.1%DEA) | Isocratic: A:B = 60:40 | 1 | 10 |
| | | | | 35 | 1500 psi |
| | | B: EtOH (0.1% DEA) | | | |

### PHARMACOLOGICAL PART

### 1) Menin/MLL homogenous time-resolved fluorescence (HTRF) assay

To an untreated, white 384-well microtiter plate was added 40 nL 200X test compound in DMSO and 4 µL 2X terbium chelate-labeled menin (vide infra for preparation) in assay buffer (40 mM Tris·HCl, pH 7.5, 50 mM NaCl, 1 mM DTT (dithiothreitol) and 0.05% Pluronic F-127). After incubation of test compound and terbium chelate-labeled menin for 30 min at ambient temperature, 4 µL 2X FITC-MBM1 peptide (FITC-β-alanine-SARWRFPARPGT-NH₂) ("FITC" means fluorescein isothiocyanate) in assay buffer was added, the microtiter plate centrifuged at 1000 rpm for 1 min and the assay mixtures incubated for 15 min at ambient temperature. The relative amount of menin·FITC-MBM1 complex present in an assay mixture is determined by measuring the homogenous time-resolved fluorescence (HTRF) of the terbium/FITC donor /acceptor fluorphore pair using an EnVision microplate reader (ex. 337 nm/terbium em. 490 nm/FITC em. 520 nm) at ambient temperature. The degree of fluorescence resonance energy transfer (the HTRF value) is expressed as the ratio of the fluorescence emission intensities of the FITC and terbium fluorophores (*F*^{em} 520 nm/*F*^{em} 490 nm). The final concentrations of reagents in the binding assay are 200 pM terbium chelate-labeled menin, 75 nM FITC-MBM1 peptide and 0.5% DMSO in assay buffer. Dose-response titrations of test compounds are conducted using an 11 point, four-fold serial dilution scheme, starting typically at 10 µM.

Compound potencies were determined by first calculating % inhibition at each compound concentration according to equation 1:$% inhibition = \left(\left(HC-LC\right)-\left({HTRF}^{compound}-LC\right)\right) / \left(HC-LC\right) * 100$ (Eqn 1)

Where LC and HC are the HTRF values of the assay in the presence or absence of a saturating concentration of a compound that competes with FITC-MBM1 for binding to menin, and HTRF^{compound} is the measured HTRF value in the presence of the test compound. HC and LC HTRF values represent an average of at least 10 replicates per plate. For each test compound, % inhibition values were plotted *vs.* the logarithm of the test compound concentration, and the *IC*₅₀ value derived from fitting these data to equation 2:$% inhibition = Bottom + \left(Top-Bottom\right) / \left(1+{10}^{∧}\left(\left(log{IC}_{50}-log\left[cmpd\right]\right)*h\right)\right)$ (Eqn 2)

Where Bottom and Top are the lower and upper asymptotes of the dose-response curve, respectively, *IC*₅₀ is the concentration of compound that yields 50% inhibition of signal and *h* is the Hill coefficient.

Preparation of Terbium cryptate labeling of Menin: Menin (a.a 1-610-6xhis tag, 2.3 mg/mL in 20mM Hepes (2-[4-(2-Hydroxyethyl)-1-piperazinyl]ethane sulfonic acid), 80 mM NaCl, 5mM DTT (Dithiothreitol), pH 7.5) was labeled with terbium cryptate as follows. 200 µg of Menin was buffer exchanged into 1x Hepes buffer. 6.67 µM Menin was incubated with 8-fold molar excess NHS (N-hydroxysuccinimide)-terbium cryptate for 40 minutes at room temperature. Half of the labeled protein was purified away from free label by running the reaction over a NAP5 column with elution buffer (0.1M Hepes, pH 7 + 0.1% BSA (bovine serum albumin)). The other half was eluted with 0.1M phosphate buffered saline (PBS), pH7. 400 µl of eluent was collected for each, aliquoted and frozen at -80°C. The final concentration of terbium-labeled Menin protein was 115 µg/mL in Hepes buffer and 85 µg/mL in PBS buffer, respectively.

### MENIN Protein Sequence (SEQ ID NO: 1):

MGLKAAQKTLFPLRSIDDVVRLFAAELGREEPDLVLLSLVLGFVEHFLAVNRVIPTNVPELT
FQPSPAPDPPGGLTYFPVADLSIIAALYARFTAQIRGAVDLSLYPREGGVSSRELVKKVSDV
IWNSLSRSYFKDRAHIQSLFSFITGTKLDSSGVAFAVVGACQALGLRDVHLALSEDHAWVVF
GPNGEQTAEVTWHGKGNEDRRGQTVNAGVAERSWLYLKGSYMRCDRKMEVAFMVCAINPSID
LHTDSLELLQLQQKLLWLLYDLGHLERYPMALGNLADLEELEPTPGRPDPLTLYHKGIASAK
TYYRDEHIYPYMYLAGYHCRNRNVREALQAWADTATVIQDYNYCREDEEIYKEFFEVANDVI
PNLLKEAASLLEAGEERPGEQSQGTQSQGSALQDPECFAHLLRFYDGICKWEEGSPTPVLHV
GWATFLVQSLGRFEGQVRQKVRIVSREAEAAEAEEPWGEEAREGRRRGPRRESKPEEPPPPK
KPALDKGLGTGQGAVSGPPRKPPGTVAGTARGPEGGSTAQVPAPAASPPPEGPVLTFQSEKM
KGMKELLVATKINSSAIKLQLTAQSQVQMKKQKVSTPSDYTLSFLKRQRKGLHHHHHH

### 2a) Proliferation assay

The anti-proliferative effect of menin/MLL protein/protein interaction inhibitor test compounds was assessed in human leukemia cell lines. The cell line MOLM14 harbors a MLL translocation and expresses the MLL fusion protein MLL-AF9, respectively, as well as the wildtype protein from the second allele. OCI-AML3 cells that carry the NPM1c gene mutation were also tested. MLL rearranged cell lines (e.g. MOLM14) and NPM1c mutated cell lines exhibit stem cell-like HOXA/MEIS1 gene expression signatures. KO-52 was used as a control cell line containing two *MLL (KMT2A)* wildtype alleles in order to exclude compounds that display general cytotoxic effects.

MOLM14 cells were cultured in RPMI-1640 (Sigma Aldrich) supplemented with 10% heat-inactivated fetal bovine serum (HyClone), 2 mM L-glutamine (Sigma Aldrich) and 50µg/ml gentamycin (Gibco). KO-52 and OCI-AML3 cell lines were propagated in alpha-MEM (Sigma Aldrich) supplemented with 20% heat-inactivated fetal bovine serum (HyClone), 2 mM L-glutamine (Sigma Aldrich) and 50µg/ml gentamycin (Gibco). Cells were kept at 0.3 - 2.5 million cells per ml during culturing and passage numbers did not exceed 20.

In order to assess the anti-proliferative effects, 200 MOLM14 cells, 200 OCI-AML3 cells or 300 KO-52 cells were seeded in 200µl media per well in 96-well round bottom, ultra-low attachment plates (Costar, catalogue number 7007). Cell seeding numbers were chosen based on growth curves to ensure linear growth throughout the experiment. Test compounds were added at different concentrations and the DMSO content was normalized to 0.3%. Cells were incubated for 8 days at 37°C and 5% CO₂. Spheroid like growth was measured in real-time by live-cell imaging (IncuCyteZOOM, Essenbio, 4x objective) acquiring images at day 8. Confluence (%) as a measure of spheroid size was determined using an integrated analysis tool.

In order to determine the effect of the test compounds over time, the confluence in each well as a measure of spheroid size, was calculated. Confluence of the highest dose of a reference compound was used as baseline for the LC (Low control) and the confluence of DMSO treated cells was used as 0% cytotoxicity (High Control, HC).

Absolute IC₅₀ values were calculated as percent change in confluence as follows:
LC = Low Control: cells treated with e.g. 1 µM of the cytotoxic agent staurosporin, or e.g. cells treated with a high concentration of an alternative reference compound $HC = High Control : Mean confluence \left(%\right) \left(DMSO treated cells\right)$ $% Effect = 100 - \left(100*\left(Sample-LC\right)/\left(HC-LC\right)\right)$

GraphPad Prism (version 7.00) was used to calculate the IC₅₀. Dose-response equation was used for the plot of % Effect vs Log10 compound concentration with a variable slope and fixing the maximum to 100% and the minimum to 0%.

### 2b) MEIS1 mRNA expression assay

MEIS1 mRNA expression upon treatment of compound was examined by Quantigene Singleplex assay (Thermo Fisher Scientific). This technology allows for direct quantification of mRNA targets using probes hybridizing to defined target sequences of interest and the signal is detected using a Multimode plate reader Envision (PerkinElmer). The MOLM14 cell line was used for this experiment. Cells were plated in 96-well plates at 3,750 cells/well in the presence of increasing concentrations of compounds. After incubation of 48 hours with compounds, cells were lysed in lysis buffer and incubated for 45 minutes at 55°C. Cell lysates were mixed with human MEIS 1 specific capture probe or human RPL28 (Ribosomal Protein L28) specific probe as a normalization control, as well as blocking probes. Cell lysates were then transferred to the custom assay hybridization plate (Thermo Fisher Scientific) and incubated for 18 to 22 hours at 55°C. Subsequently, plates were washed to remove unbound materials followed by sequential addition of preamplifiers, amplifiers, and label probe. Signals (= gene counts) were measured with a Multimode plate reader Envision. IC₅₀s were calculated by dose-response modelling using appropriate software. For all non-housekeeper genes response equal counts corrected for background and relative expression. For each sample, each test gene signal (background subtracted) was divided by the normalization gene signal (RPL28: background subtracted). Fold changes were calculated by dividing the normalized values for the treated samples by the normalized values for the DMSO treated sample. Fold changes of each target gene were used for the calculation of IC₅₀s.

**Table 3. Biological data - HTRF assay, proliferation assay, and MEIS1 mRNA expression assay**

| **Compound Number** | **HTRF-30min incubation IC₅₀ (nM)** | **MEIS1 IC₅₀ (µM)** | **spheroid assay_OneTime MOLM14 IC₅₀ (µM)** | **OCI-AML3 IC₅₀ (µM)** | **spheroid assay_OneTime KO-52 IC₅₀ (µM)** |
|---|---|---|---|---|---|
| 1 | 0.13 | 0.075 | 0.23 | 0.17 | >15 |
| 2 | 3.31 | >2.5 | 2.22 | | >15 |
| 3 | 0.10 | 0.095 | 0.042 | 0.31 | >15 |
| 4 | 0.095 | 0.02 | 0.03 | 0.39 | >15 |
| 5 | 2.26 | ~0.99 | 0.84 | | >15 |
| 6 | 0.61 | 0.36 | 0.48 | 1.86 | >15 |
| 7 | 86.20 | >2.5 | >3.75 | | >15 |
| 8 | 0.43 | ~0.65 | 1.02 | 2.15 | >15 |
| 9 | 44.66 | | >3.75 | | >15 |
| 10 | 0.18 | 0.41 | 0.34 | 1.68 | >15 |
| 11 | 0.11 | 0.018 | 0.021 | 0.21 | 8.38 |
| 12 | 0.12 | 0.039 | 0.036 | 0.63 | 8.24 |
| 13 | 0.40 | 0.077 | 0.061 | 0.21 | >15 |
| 14 | 0.05 | 0.0085 | 0.01 | 0.069 | 3.10 |
| 15 | 0.25 | 0.071 | 0.10 | 0.46 | 3.70 |
| 16 | 1.85 | ~0.67 | 0.37 | | 10.51 |
| 17 | 0.61 | 0.24 | 0.30 | 1.44 | 6.79 |
| 18 | 0.30 | 0.15 | 0.12 | 0.66 | >15 |
| 19 | 0.11 | 0.033 | 0.058 | 0.16 | >15 |
| 20 | 4.19 | >1 | >0.94 | | >15 |
| 21 | 10.41 | >1 | >0.94 | | >15 |
| 22 | 1.79 | ~0.76 | 0.36 | | 12.82 |
| 23 | 0.22 | 0.15 | 0.17 | 0.81 | 13.82 |
| 24 | 0.97 | ~0.84 | 0.69 | 2.68 | >15 |
| 25 | 10.52 | ~1.2 | >3.75 | | >15 |
| 26 | 0.28 | 0.49 | 0.27 | 1.34 | >15 |
| 27 | 0.09 | 0.02 | 0.021 | 0.091 | 6.85 |
| 28 | 2.75 | >1 | 0.60 | 1.36 | >15 |
| 29 | 2.42 | ~2.25 | 0.97 | | >15 |
| 30 | 0.067 | 0.099 | 0.13 | 0.47 | >15 |
| 31 | 2.28 | >1 | >0.94 | | >15 |
| 32 | 0.10 | 0.088 | 0.058 | 0.28 | 12.9 |
| 33 | 2.84 | >1 | >0.94 | | >15 |
| 34 | 0.87 | >1 | 3.84 | 3.44 | >15 |
| 35 | 0.15 | 0.12 | 0.20 | 0.49 | >15 |
| 36 | 2.60 | >1 | >0.94 | | >15 |
| 37 | 0.12 | 0.039 | 0.04 | 0.43 | >15 |
| 38 | 1.16 | ~1.1 | 0.52 | | >15 |
| 39 | 0.26 | 0.044 | 0.027 | 0.14 | >15 |
| 40 | 0.40 | 0.019 | 0.019 | 0.074 | 9.54 |
| 41 | 0.36 | 0.024 | 0.011 | 0.17 | >15 |
| 42 | 15.00 | | | | |
| 43 | 0.55 | ~0.24 | 0.29 | 1.20 | >15 |
| 44 | 46.62 | >2.5 | >3.75 | | >15 |
| 45 | 0.37 | 0.21 | 0.22 | 1.25 | >15 |
| 46 | 59.70 | >2.5 | >3.75 | | >15 |
| 47 | 0.31 | 0.14 | 0.22 | 0.6 | 10.84 |
| 48 | 70.39 | >1 | >0.94 | | >15 |
| 69 | 0.099 | 0.016 | 0.02 | 0.087 | 7.18 |
| 70 | 0.098 | 0.017 | 0.017 | 0.12 | 7.75 |
| 70a | 0.18 | 0.017 | 0.011 | 0.08 | |
| 71 | | 0.38 | 0.56 | 1.47 | 12.81 |
| 74 | 0.51 | 0.26 | 0.14 | 1.29 | 10.57 |
| 75 | 0.21 | 0.14 | 0.053 | 0.41 | >15 |
| 76 | 2.56 | | 0.34 | 2.39 | |
| 77 | 0.51 | 0.12 | 0.10 | 1.32 | 13.66 |
| 78 | 3.69 | >1 | >0.94 | 12.18 | >15 |
| 83 | 0.14 | 0.092 | 0.17 | 0.42 | 6.24 |
| 84 | 5.90 | >1 | >0.94 | 7.17 | >15 |
| 88 | 0.083 | 0.13 | 0.12 | 0.81 | 14 |
| 89 | 3.79 | | | | |
| 90 | 0.16 | | 0.04 | 0.19 | 1 |
| 94 | 0.12 | 0.0082 | 0.01 | 0.096 | 4.75 |
| 95 | 4.98 | ~0.62 | 0.39 | 1.62 | >15 |
| 99 | 0.20 | 0.026 | 0.0085 | 0.082 | 11.5 |
| 100 | 2.05 | | | | |
| 102 | 0.11 | 0.033 | 0.016 | 0.11 | >15 |
| 103 | 2.43 | 0.42 | 0.29 | 1.36 | >15 |
| 104 | 0.074 | 0.015 | 0.0067 | 0.15 | 10.74 |
| 112 | 0.049 | 0.009 | 0.0098 | 0.081 | >15 |
| 114 | 0.05 | 0.022 | 0.014 | 0.042 | 1.69 |
| 122 | 0.065 | 0.034 | 0.021 | 0.15 | >15 |
| 123 | 3.98 | >1 | >0.94 | 3.55 | >15 |
| 127 | 0.089 | 0.044 | 0.026 | 0.14 | 11.27 |
| 128 | 1.64 | ~0.68 | 0.63 | 1.99 | 11.39 |
| 132 | 0.11 | 0.015 | 0.033 | 0.19 | 10.41 |
| 133 | 2.13 | >1 | >0.94 | 3.50 | >15 |
| 135 | 0.057 | 0.016 | 0.013 | 0.23 | 9.69 |
| 137 | 0.18 | 0.025 | 0.045 | 0.16 | 7.29 |
| 140 | 0.093 | 0.028 | 0.11 | 0.33 | 5.75 |
| 142 | 0.071 | 0.051 | 0.012 | 0.22 | >15 |
| 145 | 0.07 | 0.021 | 0.014 | 0.11 | >15 |
| 146 | 0.081 | 0.44 | 0.32 | 1.79 | >15 |
| 148 | 0.35 | 0.06 | 0.041 | 0.34 | >15 |
| 150 | 0.26 | 0.013 | 0.014 | 0.16 | 8.70 |
| 152 | 0.19 | 0.087 | 0.037 | 0.35 | >15 |
| 154 | 0.051 | 0.027 | 0.022 | 0.067 | >15 |
| 157 | 0.12 | 0.022 | 0.021 | 0.083 | 13.80 |
| 159 | 0.10 | ~0.017 | 0.01 | 0.012 | 6.30 |
| 161 | 0.23 | 0.041 | 0.016 | 0.055 | >15 |
| 162 | 0.096 | ~0.019 | 0.013 | 0.026 | >15 |
| 163 | 0.089 | 0.02 | 0.018 | 0.12 | >15 |
| 165 | 0.06 | 0.023 | 0.03 | 0.099 | >15 |
| 167 | 0.16 | 0.018 | 0.017 | 0.065 | >15 |
| 168 | 0.10 | 0.074 | 0.049 | 0.37 | >15 |
| 170 | 0.10 | 0.037 | 0.025 | 0.041 | >15 |
| 172 | 0.13 | 0.013 | 0.023 | 0.038 | 13.4 |
| 176 | 0.13 | 0.017 | 0.038 | 0.12 | >15 |
| 177 | 0.062 | 0.0073 | 0.024 | 0.064 | >15 |
| 179 | 0.13 | 0.027 | 0.033 | 0.059 | >15 |
| 181 | 0.079 | 0.017 | 0.023 | 0.044 | 14.02 |
| 184 | ~0.36 | 0.11 | 0.23 | 0.19 | 3.00 |
| 185 | 0.20 | 0.044 | 0.079 | 0.061 | 1.7 |
| 188 | 0.43 | 0.034 | 0.055 | 0.34 | >15 |
| 189 | 0.22 | 0.019 | 0.03 | 0.19 | >15 |
| 191 | 0.28 | 0.22 | 0.28 | 0.56 | >15 |
| 193 | ~0.26 | 0.12 | 0.17 | 0.29 | >15 |
| 195 | ~0.4 | 0.056 | 0.07 | 0.058 | >15 |
| 197 | 0.30 | 0.036 | 0.045 | 0.036 | >15 |
| 199 | 0.13 | ~0.26 | 0.46 | 1.91 | >15 |
| 201 | 0.13 | 0.052 | 0.04 | 0.065 | >15 |
| 203 | 0.30 | 0.042 | 0.038 | 0.075 | >15 |
| 205 | 0.21 | 0.035 | 0.029 | 0.043 | >15 |
| 209 | 0.27 | 0.13 | 0.27 | 0.24 | >15 |
| 210 | 5.57 | >1 | >0.94 | 4.63 | |
| 211 | 0.35 | 0.11 | 0.15 | 0.097 | >15 |
| 212 | 9.45 | | | | |
| 214 | 0.49 | 0.14 | 0.12 | 0.38 | >15 |
| 215 | 88.84 | >1 | 1.49 | 9.24 | |
| 219 | 0.27 | ~0.23 | 0.13 | 0.43 | >15 |
| 223 | 0.40 | 0.20 | 0.18 | 0.99 | >15 |
| 225 | 8.31 | >1 | >0.94 | 7.09 | |
| 227 | 0.15 | 0.076 | 0.046 | 0.13 | >15 |
| 229 | 0.37 | 0.36 | 0.32 | 0.87 | >15 |
| 230 | ~0.88 | ~0.94 | >0.94 | 1.16 | >15 |
| 231 | 1.77 | ~0.56 | 0.79 | 1.80 | |
| 233 | 0.058 | 0.019 | 0.026 | 0.15 | 5.03 |
| 240 | 12.31 | | | | |
| 241 | 1.36 | 0.28 | 0.18 | 1.12 | |
| 242 | 0.26 | 0.23 | 0.17 | 1.07 | >15 |
| 243 | 7.39 | | | | |
| 245 | 0.26 | 0.14 | 0.073 | 0.43 | >15 |
| 246 | 0.063 | 0.18 | 0.06 | 0.38 | >15 |
| 250 | 0.18 | 0.091 | 0.22 | 0.41 | 7.05 |
| 251 | 0.18 | 0.019 | 0.052 | 0.13 | 3.90 |
| 252 | 0.065 | 0.053 | 0.032 | 0.64 | 7.26 |
| 254 | 4.16 | | 0.59 | 4.69 | >15 |
| 256 | 0.15 | 0.13 | 0.084 | 0.36 | >15 |
| 258 | 0.33 | 0.026 | 0.027 | 0.74 | 5.44 |
| 259 | 0.16 | 0.065 | 0.046 | 0.25 | >15 |
| 265 | 0.29 | | 0.028 | 0.061 | |
| 266 | 0.92 | 0.039 | 0.029 | 0.049 | >15 |
| 268 | 0.29 | 0.34 | 0.20 | 0.53 | >15 |
| 270 | 0.54 | 0.22 | 0.14 | 0.48 | >15 |
| 271 | 0.27 | 0.1 | 0.065 | 0.16 | >15 |
| 272 | 0.58 | 0.17 | 0.13 | 0.44 | >15 |
| 273 | 0.21 | ~0.39 | 0.39 | 0.83 | >15 |
| 274 | 0.38 | 0.23 | 0.20 | 0.64 | >15 |
| 275 | 0.66 | 0.1 | 0.078 | 0.33 | >15 |
| 276 | 0.50 | 0.39 | 0.24 | 1.61 | >15 |
| 278 | 0.068 | >1 | >0.94 | 3.21 | >15 |
| 280 | 0.20 | 1.07 | 0.69 | 0.92 | >15 |
| 281 | 0.24 | 0.038 | 0.048 | 0.09 | >15 |
| 283 | 0.054 | 0.01 | 0.014 | 0.046 | >15 |
| 284 | ~0.36 | 0.05 | 0.023 | 0.073 | >15 |
| 285 | 1.02 | 0.27 | 0.16 | 0.13 | >15 |
| 286 | | 0.096 | 0.076 | 0.29 | 22.94 |
| 300 | 0.066 | 0.029 | 0.036 | 0.071 | >15 |
| 302 | 0.26 | 0.16 | 0.097 | 0.77 | |
| 311 | 1.01 | ~0.63 | 0.51 | 2.88 | >15 |
| 313 | 1.99 | ~0.26 | | | |
| 314 | 1.53 | 0.16 | 0.30 | | |
| 315 | 0.51 | ~0.33 | 0.29 | 1.09 | >15 |
| 323 | 0.49 | 0.062 | 0.11 | 0.17 | 13.39 |
| 324 | 18.4 | | | | |
| 325 | ~0.16 | 0.015 | 0.016 | 0.19 | >15 |
| 326 | 18.51 | | | | |
| 334 | 0.079 | 0.011 | 0.012 | 0.13 | >15 |
| 335 | 5.49 | | | | |
| 336 | 0.21 | 0.061 | 0.07 | 0.87 | >15 |
| 337 | 4.57 | >1 | | | |
| 342 | 0.17 | 0.078 | 0.01 | 0.025 | 0.023 |
| 343 | ~3.93 | 0.53 | | | |
| 346 | 0.058 | 0.033 | 0.036 | 0.13 | >15 |
| 347 | 12.02 | >1 | >0.94 | 4.98 | |
| 352 | 0.077 | 0.13 | 0.018 | 0.15 | >15 |
| 353 | 4.19 | | | | |
| 356 | 0.053 | ~0.0082 | 0.007 | 0.046 | >15 |
| 357 | 7.77 | | | | |
| 365 | 0.097 | 0.01 | 0.011 | 0.037 | >15 |
| 366 | 0.088 | 0.02 | 0.017 | 0.076 | >15 |
| 369 | 1.82 | ~0.36 | 0.42 | | |
| 370 | 5.42 | | | | |
| 377 | 15.41 | | | | |
| 378 | 27.28 | | | | |
| 379 | 14.21 | | | | |
| 380 | 40.55 | | | | |
| 382 | 0.41 | 0.15 | 0.14 | 0.40 | 11.82 |
| 386 | ~0.59 | 0.061 | 0.16 | 0.81 | 9.6 |
| 387 | 9.52 | | | | |
| 391 | 0.90 | 0.16 | 0.49 | 2.10 | >15 |
| 392 | 10.34 | | | | |
| 394 | 0.23 | 0.049 | 0.11 | 0.33 | >15 |
| 395 | 0.20 | 0.14 | 0.056 | 0.28 | |
| 396 | 0.08 | 0.017 | 0.023 | 0.14 | 8.51 |
| 397 | 0.08 | 0.018 | 0.015 | 0.027 | >15 |
| 398 | 0.24 | 0.043 | 0.064 | 0.22 | >15 |
| 402 | 0.067 | 0.018 | 0.007 | 0.04 | |
| 406 | 0.033 | 0.13 | 0.22 | 0.49 | |
| 407 | 0.086 | 0.38 | | | |
| 409 | 0.12 | ~0.44 | 0.64 | 1.85 | |
| 410 | 0.12 | 0.12 | 0.16 | 0.38 | |
| 411 | 0.33 | 0.11 | 0.21 | 0.68 | |
| 413 | 0.051 | ~0.59 | 0.72 | 1.49 | |
| 415 | 0.084 | | | | |
| 416 | ~0.035 | 0.049 | | | |

### 3) Mouse PK (In vivo T1/2 and oral bioavailability)

In vivo pharmacokinetics (PK) were assessed in fasted male CD-1 mice (age 6-8 weeks) following a single intravenous (IV, 0.5 or 1.0 mg/kg administered at 2.5 ml/kg) or oral (PO, 5 mg/kg administered at 10 ml solution/kg) dose of test article formulated in a 20% (w:vol) HP-β-CD solution or in Pyrogen free water.

Plasma and/or whole blood samples were collected from the dorsal metatarsal vein at desired timepoints via serial capillary microsampling (approx. 0.03 mL) using EDTA as an anticoagulant. Concentrations of compound in the plasma and blood samples were analyzed using a qualified LC-MS/MS method. In silico analysis of main pharmacokinetic parameters was performed using WinNonlin (PhoenixTM, version 6.1) or similar software. (Results see Table 4)

### 4) Metabolic stability in human/mouse liver microsomes

### Experimental Procedure

The objective of this study is to measure *in vitro* metabolic stability of test compound(s) in human and mouse liver microsomes and provide quantitative information on the rate of metabolic turnover (i.e. determination of the apparent intrinsic clearance of test).

Test items were prepared at a stock concentration of 10 mM in DMSO. For determination of metabolic turnover, a final working solution was prepared by adding 2 µL of 10 mM DMSO stock solution for test compound or positive control compounds to 198 µL of acetonitrile (100 µM final concentration).

Incubations were performed as follows: First, liver microsomes were thawed on ice and a master solution containing liver microsomes in 100 mM PBS (phosphate-buffered saline) at pH 7.4 is prepared. Next, the liver microsomes solution was added to the incubation plates and 10 mM NADPH (Nicotinamide-adenine dinucleotide phosphate) was added (MW: 833.4 g/mol; Roche Diagnostics GmbH, Germany. Dissolved in phosphate buffer (100 mmol/L, pH 7.4)). The mixture was mixed for 10 seconds and pre-warmed in the incubation plate at 37°C for 10 minutes. The metabolic reaction was initiated with the addition of 5 µL of the 100 µM working solution for test compound or positive control compounds to incubation plate (final test item concentration = 1 µM). The reaction final mixture should contain 1 mM NADPH, 0.5 mg/mL microsomes protein and 1 µM test compound or positive control compound in 100 mM PBS at pH 7.4. The percentage of organic solvent in incubation mixture is 1% with DMSO ≤ 0.02%.

The reaction was quenched by transferring 50 µL of the incubated mixture at selected time points into the quenching plate containing 200 µL of cold methanol. After sampling of all the timepoints the quenching plate was centrifuged at 4000 rpm for 40 minutes to precipitate protein. A total of 90 µL of the supernatant was transferred to an analysis plate and ultra-pure H₂O water is added into each well for LC/MS/MS analysis. All incubations and analysis were performed in duplicate.

### Data analysis

All calculations were carried out using Microsoft Excel. The slope value, k, was determined by linear regression of the natural logarithm of the remaining percentage of the parent drug vs. incubation time curve.

The *in vitro* half-life *(in vitro* t_{1/2}) was determined from the slope value: $in vitro {t}_{1 / 2} = - \left(0.693/k\right)$

Conversion of the *in vitro* t_{1/2} (in min) into the *in vitro* intrinsic clearance (*in vitro* CLᵢₙₜ, in µL/min/mg proteins) was done using the following equation: $in vitro {Cl}_{int} = \left(\frac{0.693}{{t}_{\frac{1}{2}}}\right) ∗ \left(\frac{volume of incubation \left(μL\right)}{amount of proteins \left(mg\right)}\right)$

Results see Table 4

**Table 4: Mouse PK and metabolic stability ("NA" means not analyzed)**

| **Example number** | **Formulating agent** | **In vivo T1/2 (IV) (h)** | **Bioavailability (PO) (%)** | **Human LM Clint (µl/min/mg)** | **Mouse LM Clint (µl/min/mg)** |
|---|---|---|---|---|---|
| **27** | HP-β-CD | 6.7 | 17 | 19 | <7.5 |
| **70** | Pyrogen free water | 9.0 | 34 | 19 | <7.5 |
| **346** | HP-β-CD | 5.2 | 5.1 | 11 | <7.5 |
| **102** | HP-β-CD | 11 | 9.7 | NA | NA |
| **396** | | NA | NA | 22 | <7.5 |
| **104** | HP-β-CD | 8.7 | 6.1 | 19 | <7.5 |
| **114** | HP-β-CD | 9.5 | 8.7 | 26 | 15 |
| **1** | HP-β-CD | 15 | <1 | <7.5 | 21 |
| **112** | HP-β-CD | 6.2 | 4.0 | 17 | <7.5 |
| **245** | HP-β-CD | 7.0 | <1 | 18 | <7.5 |
| **37** | HP-β-CD | >12 | <1 | <7.5 | <7.5 |
| **6** | HP-β-CD | NA | NA | 35 | 28 |
| **45** | HP-β-CD | NA | NA | 43 | 110 |
| **13** | HP-β-CD | NA | NA | 75 | 44 |
| **47** | HP-β-CD | NA | NA | 38 | 47 |
| **83** | HP-β-CD | NA | NA | 539 | >1000 |
| **161** | HP-β-CD | NA | NA | 70 | 55 |
| **214** | HP-β-CD | NA | NA | 44 | 80 |
| **99** | HP-β-CD | NA | NA | 27 | 13 |
| **397** | HP-β-CD | NA | NA | 14 | <7.5 |
| **11** | HP-β-CD | NA | NA | 14 | <7.5 |

### 5) Protocol for pharmacodynamics (PD) activity in subcutaneous (sc or SC) xenografts of MOLM-14 or OCI-AML3 cells

### Test Agents and Controls

Compound 70 was formulated in 20% hydroxypropyl-beta-cyclodextrin (HP-β-CD) and prepared to reach a total volume of 0.2 mL (10 mL/kg) per dose for a 20 g animal. Doses were adjusted by individual body weight each day. Working stocks of Compound 70 were prepared once per week for each study and stored at room temperature. Compound 70 was administered orally (PO), daily.

### Assay

The in vivo pharmacodynamics (PD) activity of compounds was evaluated in subcutaneous (SC) xenografts of MOLM14 cells or OCI-AML3. Nude NMRI mice (Crl:NMRI-Foxn1nu/-) harboring MOLM14 or OCI-AML3 tumors were treated with 3 daily doses of vehicle or compounds. Plasma samples were collected at 23 hours after day 2 dose, 0.5 hours post final dose, and 16 hours post final dose and tumor samples were collected 16 hours post final dose. To examine the effects of compounds on the expression of multiple Menin-MLL target genes (e.g. MEIS1, MEF2C, FLT3) QuantiGene Plex technology (Thermo Fisher Scientific) was used. Frozen tumors were homogenized and transferred to individual lysing matrix tubes in lysis buffer and incubated for 30 minutes at 55°C. Cell lysates were mixed with target-specific capture probes, Luminex beads, and blocking probes, transferred to the custom assay hybridization plate (Thermo Fisher Scientific) and incubated for 18 to 22 hours at 54°C. Subsequently, plates were transferred to a magnetic separation plate and washed to remove unbound materials from beads followed by sequential hybridization of preamplifiers, amplifiers, and label probe and subsequent streptavidin phycoerythrin binding. Signals from the beads were measured with a Luminex FlexMap three-dimensional instrument. For all non-housekeeper genes response equal counts corrected for background and relative expression. For each sample, each test gene signal (background subtracted) was divided by the normalization gene signal (RPL19, RPL28, ATP6V1A: background subtracted). Fold changes were calculated by dividing the normalized values for the treated samples by the normalized values for the DMSO treated sample.

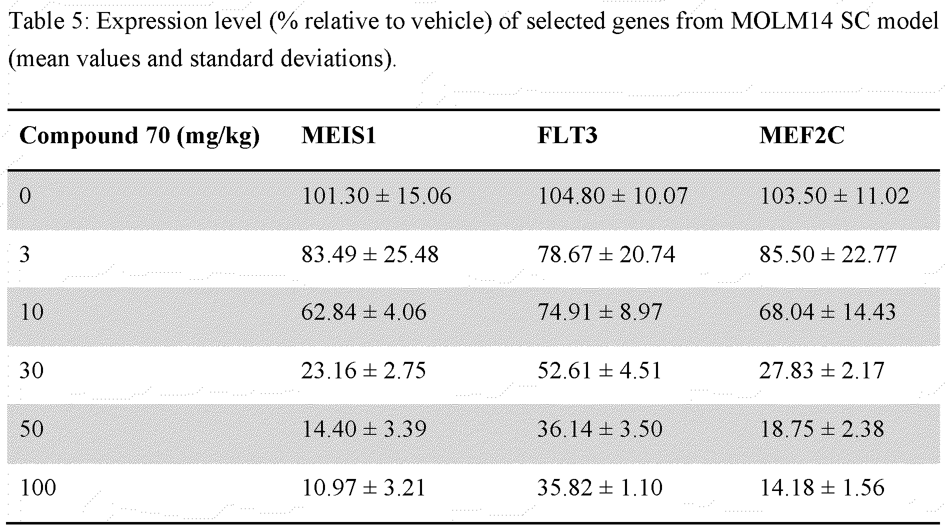

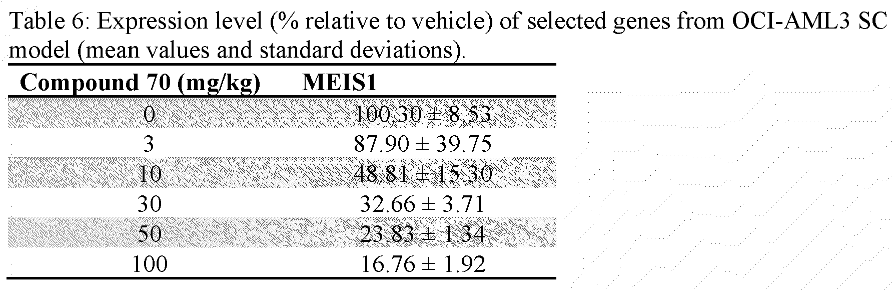

### 6) Efficacy study in MOLM-14 subcutaneous model

### Test Agents and Controls

Compound 70 was formulated in 20% hydroxypropyl-beta-cyclodextrin (HP-β-CD) and prepared to reach a total volume of 0.2 mL (10 mL/kg) per dose for a 20 g animal. Doses were adjusted by individual body weight each day. Working stocks of Compound 70 were prepared once per week for each study and stored at 25°C.

### Animals

Female NMRI Nude mice (MOLM-14 SC) were used when they were approximately 6 to 8 weeks of age and weighed approximately 25 g. All animals could acclimate and recover from any shipping-related stress for a minimum of 7 days prior to experimental use. Autoclaved water and irradiated food were provided ad libitum, and the animals were maintained on a 12hour light and dark cycle. Cages, bedding, and water bottles were autoclaved before use and changed weekly.

| Tissue Culture and Cell Injection Reagents |
|---|
| DPBS (Dulbecco's phosphate-buffered saline) |
| Heat-inactivated fetal bovine serum |
| RPMI 1640 medium |
| L-glutamine |
| Gentamycin |
| T175 Culture Flask |
| Roller Bottle |

### Tumor Model and Cell Culture Method

Human AML cells MOLM-14 were cultured at 37°C, 5% CO₂ in the indicated complete culture media (RPMI 1640 + 10% HI-FBS + 2mM L-glutamine + 50ug/ml Gentamycin ). Cells were harvested while in logarithmic growth and resuspended in cold (4°C) Roswell Park Memorial Institute (RPMI) 1640 in serum-free medium.

Each mouse received 5×10⁶ MOLM-14 cells in 50% Matrigel in the right flank, in a total volume of 0.2 mL using a 1cc syringe and a 27-gauge needle.

### Study Designs

Compound 70 was administered orally (PO), daily.

Day 0 is the day of tumor cell implantation and study initiation

Mice bearing SC MOLM-14 tumors were randomized on Day 16 post-tumor implantation and assigned to treatment groups according to tumor volume (mean of ~130 mm³; n=10/group). Treatment with vehicle or Compound 70 (at 30 and 100 mg/kg) was initiated on the same day, with daily oral dosing for 21 days. Plasma was collected at 1, 2, 4, 8, and 23 hours after the last dose (n=4-5/group/time point) for PK (pharmacokinetics) analysis.

### Animal Monitoring

SC tumor volume were measured for each animal 2 to 3 times per week or more throughout the study.

### Calculations

Tumor volume was calculated using the formula: $Tumore volume \left({mm}^{3}\right) = \left(D\times {d}^{2}/2\right) ;$ where 'D' represents the larger diameter and 'd' the smaller diameter of the tumor as determined by caliper measurements. Tumor volume data was graphed as the mean tumor volume ± SEM.

The % ΔTGI was defined as the difference between mean tumor burden of the treatment and control groups, calculated as % ΔTGI= ([(TV_{c}TVc₀)(TVₜTVₜ₀)]/(TV_{c}TVc₀))×100 where 'TV_{c}' is the mean tumor burden of a given control group, 'TVc₀' is the mean initial tumor burden of a given control group, 'TVₜ' is the mean tumor burden of the treatment group, and 'TVₜ₀' is the mean initial tumor burden of the treatment group. % TGI was defined as the difference between Mean tumor volumes of the treated and control groups, calculated as: $% TGI = \left(\left({TV}_{c}{TV}_{t}\right)/{TV}_{c}\right) \times 100$ where 'TV_{c}' is the mean tumor volume of the control group and 'TVₜ' is the mean tumor volume of the treatment group. As defined by National Cancer Institute criteria, ≥60% TGI is considered biologically significant.

The % Tumor Regression (TR), quantified to reflect the treatment-related reduction of tumor volume as compared to baseline independent of the control group, was calculated as %TR= (1-mean (TVₜi/TVₜ₀i)) x 100 where 'TVₜi' is the tumor burden of individual animals in a treatment group, and 'TVₜ₀i' is the initial tumor burden of the animal.

### Data Analysis

Tumor volume were graphed using Prism software (GraphPad version 7 or 8). Statistical significance for most studies was evaluated for Compound 70 -treated groups compared with HPβCD vehicle-treated controls on the last day of the study when 2/3 or more mice remained in each group. Differences between groups were considered significant when p≤0.05.

Statistical significance for animal tumor volume was calculated using the linear mixed-effects (LME) analysis in R software version 3.4.2 (using Janssen's internally developed Shiny application version 4.0), with treatment and time as fixed effects and animal as random effect. Logaritmic transformation was performed if individual longitudinal response trajectories were not linear.

The information derived from this model was used to make pairwise treatment comparisons of tumor volumes to that of the control group or between all the treatment groups.

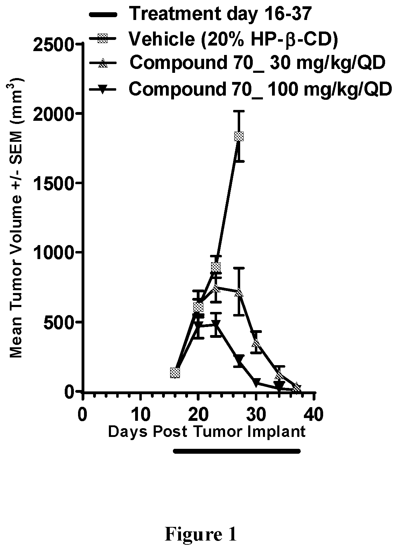

Results in Fig. 1.

### 7) Cardio-electrophysiological effects of the testing compounds in synchronously beating human pluripotent stem cell-derived cardiomyocytes (hSC-CMs) using a Ca²⁺-fluorescence assay (CTCM human)

### Protocol

Compounds were tested in the 96-well plates

Compounds were tested at 0.1 µM, 0.2 µM, 0.5 µM, 1 µM, 2.5 µM and 5 µM (n = 4 per dose) on Cor.4U ^{®}-Cardiomyocytes or on iCell^{®} Cardiomyocytes2

Alternatively, compounds were tested at 0.1 µM, 0.3 µM; 1 µM, 3 µM,10 µM and 30 µM (n = 4 per dose) mostly on iCell^{®} Cardiomyocytes2

### Positive and Negative controls

| | |
|---|---|
| Dofetilide | at 3 nM |
| Isoproterenol | at 100 nM |
| Nimodipine | at 100-300 nM |
| Cetirizine | at 3 µM |

### Vehicle control:

Dimethylsulfoxide (DMSO). The solutions of the compound in DMSO or its solvent (final concentration of 0.1% DMSO; n = 8)

### Preparation of Test Article and Controls

Tested compounds were dissolved in DMSO at 1000-fold the intended concentrations. A compound "mother-plate" was made, containing the test compounds and positive and negative controls at 1000-fold the final concentrations. At the experiment day, these stock solutions were diluted with Tyrode (Sigma), supplemented with 10 mM HEPES (Gibco), to 2-fold the intended concentration (in round bottom compound plates). Final DMSO concentration in test solutions and vehicle control was 0.1%.

### Cells

hSC-CMs (Cor.4U^{®} Cardiomyocytes) were obtained from CDI (Ncardia, Germany). Cells are pre-plated and seeded in fibronectin-coated 96-well plates at a density suited to form a monolayer and maintained in culture in a stage incubator (37°C, 5% CO₂), according to the instructions of the cell provider.

Second line hSC derived cardiomyocyte called iCell^{®} Cardiomyocytes2 were purchased from FUJIFILM Cellular Dynamics (USA). The experiments with test drugs are carried out 5 to 7 days after plating the cells onto the plate to have a living, beating monolayer of hiPSC-derived cardiomyocytes. The beating monolayer in 96-well-plates are normally taken from 2 Vials of frozen iCell^{®} Cardiomyocytes2 (≈5 million cells/vial), which will be plated onto three 96-well plates (≈50K/well).

### Before start of experiment

At least one hour before the start of the experiments the normal cell medium was replaced with Tyrode solution with Calcium dye (see below).

Cal 520 dye (AAT Bioquest) was dissolved in 11 ml of Tyrode supplemented with 10 mM HEPES and warmed up to 37 C before adding to the cells.

35 µl cell culture medium was removed from each well and replaced with 35 µl of pre-warmed Cal 520 dye solution and cell plate was incubated for 45 min at 37 °C / 5% CO₂. Cells were incubated for 5 min at 37 °C.

### Experiment

Spontaneous electrical activity is recorded, using Cal520^{™} (AAT Bioquest) calcium fluorescence-dye signaling. This dye integrates the total intracellular calcium activity over the whole well. A bottle of Cal520 dye (50µg, MW: 1103/mol) is dissolved with 50 µl DMSO as a stock solution of 0.9 mM. 50 µL of the stock solution of the dye was added to 10 ml Tryodes solution to have dye concentration of 4.5 µM. Subsequently, 35 µl of this dye solution was added into each well, to have a final dye concentration of 1.58 µM. The current dye protocol on this CTCM human assay was established recently (Ivan Kopljar et al, Journal of Pharmacological and toxicological methods 2018. 91: 80-86; Lu et al., Tox Sci 2019. 170 (2): 345-356).

Fluorescent signals (Ca²⁺ transient morphology) were measured using the Functional Drug Screen System (FDSS/µCell; Hamamatsu, Japan) and the recordings were subsequently analyzed off-line, using appropriate software e.g. Notocord.

The cell plate was loaded into the FDSS/µCell for a test run: Ca²⁺ transients were measured for 4 minutes to check for synchronous beating of the cardiomyocytes in each well. All 96 wells were measured simultaneously (sampling interval: 0.06 s, short exposure time: 10 ms; excitation wavelength 480 nm; emission wavelength 540 nm; FDSS/µCell warmed to 37°C). When all showed synchronous beating, the 96-well plate was measured repeatedly for 3 times (to verify synchronous beating in all 96-well at baseline, wells that did not meet the preset criteria were excluded from the study and not treated with compound):
T = 0: control period (-5 to -1 min) + compound addition, followed for 3 min.
T = 30: measured from 29 to 34 min after compound addition

During the compound addition step, 100 µl of the respective double-concentrated test solutions was pipetted into each well simultaneously.

Data were analyzed off-line using appropriate software e.g. Notocord-Hem (version 4.3).

The following parameters of the Ca²⁺ transient morphology were measured:
- beat rate (BR)
- amplitude of the Ca²⁺ transient (Amp),
- CTD₉₀: Ca²⁺ transient duration at 90% (time to 90% of the initial base value).

The presence of various *'arrhythmia-like'* activities were also noted during the experimental periods. These included:
- *'early afterdepolarization-like '* (EAD-*like*) events (defined as "an extra small peak of the transient waveform following the initial peak of the transient"),
- *'ventricular tachycardia-like'* (VT-*like*) events (defined as a very fast beating rate) or
- *'ventricular fibrillation-like'* (VF-*like*) events (defined as "small amplitude, fastrate Ca²⁺ waveforms with irregularities and non-measurable transient potentials)
- *'cessation of beating'* of the cells (no Ca²⁺ transients observed).

If compound-induced changes on the calcium transient signal could not be analyzed by the software, then these signals were identified as BQL (below quality analyses level).

### Data Analysis

Data, measured from the FDSS-µCell, were copied for off-line analysis and were analyzed and uploaded in SPEC-II (our operational management system) for further analysis. The values of the variables before and after administration of the compound were collected and transferred into an Excel workbook.

All values (actual units and percentage changes from the baseline values) are expressed as median (minimum and maximum). Changes versus the corresponding baseline values (in actual units) observed in the compound group were compared with those in the solvent control group using the Wilcoxon-Mann-Whitney Test. Two-tailed tests with Bonferroni correction for multiplicity adjustment were conducted. Since there are 10 treatment groups each compared to the solvent group, alpha level of 0.05/10 (0.005) was considered to reflect a statistically significant difference from the solvent group. All statistical analysis was performed using appropriate software e.g. R software version 3.5.2.

### Quality Control of the hiPSC-CMs in the plate:

Plates were rejected if they did not meet following criteria:
- Stable regular beating
- Amplitude > 500 relative units
- Beat rate between 25 and 80 beats per minute
- CTD₉₀ between 300 and 800 ms

In the present study, the hiPSC-CMs in the plates met the above criteria.

These parameters combined with incidence of arrhythmia or cessation of beating were used to calculate the potential hazard level using a weighted scoring method (based on Kopljar et al., Stem Cell Reports 2018. 11, 1365-1377). This hazard score is calculated per concentration by adding weighted points based on the Tolerance Intervals (TI) on the changes of CTD₉₀, the beat rate and amplitude (ΔΔ%) and incidence of beating stop and early afterdepolarization (EAD). Consequently, for each concentration one of four different hazard levels will be generated. This will be done after 30-min of incubated with compound. The hazard levels are:
No hazard: within the vehicle effect levels or small non-relevant changes.
Low hazard: relevant effect but potentially low risk for cardiac liabilities.
High hazard: relative high risk for cardiac liabilities.
Very high hazard: very high risk due to arrhythmic like events (EAD's).

The *'Hazard Score'* results provide an identification for potential acute cardiac drug-induced effects at free drug equivalent (*as no plasma proteins are added to the wells*). Evaluation of hazard identification is conducted using a 'scoring reference book' called *CTCM Scoring_version 1* (Kopljar et al., Stem Cell Reports 2018. 11: 1365-1377), and levels are indicated according to the following color scheme:

| Color | Hazard identification legend |
|---|---|
| Green | No concern |
| Yellow | Low concern |
| Red | High concern |
| Black | Very high concern due to arrhythmic events |

Ranking of a testing compound according to hazard score severity on the Ca²⁺ transient assay measured in HiPSc-CMs as listed above in different colors and in the associated table.

### RESULTS

### Using iCell^{®} Cardiomyocytes2 as cell line

### Positive and negative controls:

The positive and negative controls all had expected pharmacological effects in this assay Compounds:

| Compound | Color @ 0.1 µM | Color @ 0.2µM | Color @ 0.5µM | Color @ 1µM | Color @ 2.5µM | Color @ 5µM |
|---|---|---|---|---|---|---|
| 70 | Green | Green | Green | Green | Green | Green |
| 246 | Green | Green | Green | Green | Green | Green |

| Compound | Color @ 0.1µM | Color @ 0.3µM | Color @ 1µM | Color @ 3µM | Color @ 10µM | Color @ 30µM |
|---|---|---|---|---|---|---|
| 70a | Green | Green | Green | Green | Green | yellow |
| 398 | Green | Green | Green | Green | Green | Green |
| 11 | Green | Green | Green | Green | Green | Green |
| 286 | Green | Green | Green | Green | Green | Green |

For compound 70a: with an efficacious dose in mouse xenograft models of 30 mpk (mg/kg), CTCM human concentration vs free Cmax would be estimated as followed
Margin CTCM human 10 µM vs free Cmax >16 (mouse, human)
Margin CTCM human 30 µM vs free Cmax >45 (mouse, human)
Using Cor.4U ^{®}-Cardiomyocytes as cell line

| Compounds | Color @ 0.1µM | Color @ 0.2µM | Color @ 0.5µM | Color @ 1µM | Color @ 2.5µM | Color @ 5µM |
|---|---|---|---|---|---|---|
| 37 | Green | Green | Green | Green | Green | Green |
| 19 | Green | Green | Green | Green | Green | Green |

### 8) Effect on the membrane potassium current I_{Kr} in hERG transfected cell lines

### Protocol 1:

### List of abbreviations

### Abbreviations

- CHO: Chinese hamster ovary cell line
- DMSO: Dimethylsulfoxide
- hERG: human *ether-à-go-go*-related gene
- I_{Kr}: rapidly activating delayed-rectifier K⁺ current

### Methods

Experiments were performed using CHO cells stably expressing the hERG potassium channel. Cells were grown at 37°C and 5% CO₂ in culture flasks in Ham's F12 Medium supplemented with 10% heat-inactivated fetal calf serum, hygromycin B (100 µg/ml) and geneticin (100 µg/ml). For use in the automated patch-clamp system QPatch (Sophion) cells were harvested to obtain cell suspension of single cells.

Solutions: The bath solution contained (in mM) 145 NaCl, 4 KCl, 10 glucose, 10 HEPES ((4-(2-hydroxyethyl)-1-piperazineethanesulfonic acid), 2 CaCl₂ and 1 MgCl₂ (pH 7.4 with NaOH). The pipette solution contained (in mM) 120 KCl, 10 EGTA (Ethylene glycol-*bis*(2-aminoethylether)-*N,N,N',N'*-tetraacetic acid), 10 HEPES, 5.374 CaCl₂ and 1.75 MgCl₂ (pH 7.2 with KOH).

Patch-clamp experiments were performed in the voltage-clamp mode and whole-cell currents were recorded with an automated patch-clamp assay utilizing the QPatch system (Sophion). Current signals were amplified and digitized, stored and analyzed by using the QPatch assay software.

The holding potential was -80 mV. The hERG current (K⁺-selective outward current) was determined as the maximal tail current at -40 mV after a 2 second depolarization to +60 mV. Pulse cycling rate was 15 s. A short pulse (90 ms) to -40 mV served as a baseline step to calculate the tail current amplitude. After establishing whole-cell configuration and a stability period, the solvent control (0.3% DMSO) was applied for 5 minutes followed by the test substance by four increasing concentrations of 3 x 10⁻⁷ M, 3 x 10⁻⁶ M, 10⁻⁵ M and 3 x 10⁻⁵ M. Each concentration of the test substance was applied twice. The effect of each concentration was determined after 5 min as an average current of 3 sequential voltage pulses. To determine the extent of block the residual current was compared with vehicle pre-treatment. Concentration/response relations were calculated by non-linear least-squares fits to the individual data points. The half-maximal inhibiting concentration (IC50) was calculated by the fitting routine.

### Protocol 2:

### Cells

The compound, vehicle control and positive control were tested on hERG-transfected HEK293 cells. A human embryonic kidney cell line (HEK293) with a stable transfection of hERG (Zhou Z et al. Biophysical Journal 1998. 74, 230-241; McDonald T.V.et al, Nature 1997. 388, 289-292) was used (University of Wisconsin, Madison, USA). The cells were kept in culture in MEM (Minimum Essential Medium, Gibco) which was supplemented with (amounts indicated added to 500 ml MEM): 5 ml L-Glutamine-Penicillin-Streptomycin (Sigma), 50 ml Fetal Bovine serum (Bio-Whittaker), 5 ml Nonessential Amino Acids 100x (Gibco), 5 ml sodium pyruvate 100 mM (Gibco) and 4 ml geneticin 50 mg/ml (Gibco) using T175 flasks. The cells were incubated at 37°C in 5% CO₂ atmosphere (in air).

### Cell Harvesting for assay

Cells were harvested as described below using accumax^{™} (Sigma) as the dissociating reagent. Cells were then resuspended in a mixture of 33% DMEM/F12 (Dulbecco's Modified Eagle Medium/Nutrient Mixture F-12 - Sigma) media/67% extracellular physiological solution.

The flasks were washed twice carefully with ~5-10 ml phosphate buffered saline (PBS) (Gibco^{™}) containing 2mM EDTA (Ethylenediaminetetraacetic acid) (Sigma). The cells were dissociated using ~3 ml of accumax^{™} (cell detachment solution) and incubated for ~5 to 10 min. at 37°C. Cold external physiological solution (2-5 ml) was added and the flasks are incubated at ~4 °C for 5-10 min. Then, the cell suspension in each flask was gently dissociated with a 5ml pipette. The cell suspension was transferred to a low binding petri-dish (~10 mm diameter). Each flask was washed with ~ additional 5 ml cold external physiological solution and this solution was also added to the petri-dish. The petri-dish was then incubated for another 5 to 10 minutes at ~4 °C. After another gentle dissociation of the cell suspension in the petri dish, the cells were transferred to a reservoir kept on an orbital shaker at 200 rpm at 16°C. Before experiments were performed, the cells recovered for ~20 min.

### Compounds

A 10 mM solution of the compound was used and plated in a 384 well plate. Aliquots of the stock solutions are diluted with the recording solution (see section 3) using automated liquid handling (Biomek FXP; final DMSO concentration: 0.03 to 0.3 %). A standard range of screening concentrations was used ranging from 1 µM to 30 µM.

A positive control (E-4031) was included within each run to evaluate the sensitivity of the assay.

### External and intracellular solutions used in the experiments

In the table below the composition of the intracellular and external buffer solutions is shown in [mM] ("NMDG" means N-methyl-D-glucamine)

| Intracellular Solution | | | Extracellular Physiological Solution / Chip Fill Solution | | | Seal Enhancer Solution | | | Recording Solution | |
|---|---|---|---|---|---|---|---|---|---|---|
| KCl | 10 | | NaCl | 140 | | NMDG | 60 | | NMDG | 60 |
| KF | 110 | | KCl | 4 | | NaCl | 80 | | NaCl | 80 |
| NaCl | 10 | | Glucose | 5 | | KCl | 4 | | KCl | 4 |
| HEPES | 10 | | HEPES | 10 | | CaCl₂ | 10 | | CaCl₂ | 2 |
| EGTA | 10 | | CaCl₂ | 2 | | MgCl₂ | 1 | | MgCl₂ | 1 |
| | | | MgCl₂ | 1 | | Glucose | 5 | | Glucose | 5 |
| | | | | | | HEPES | 10 | | HEPES | 10 |
| pH 7.2 (KOH) | | | pH 7.4 (NaOH) | | | pH 7.4 (HCL) | | | pH 7.4 (HCL) | |

### Study design

The whole cell patch clamp technique on transfected cells allows the study of ion-channels with no - or limited interference from other ion-channels. The effects of the compounds on the hERG current were studied with an automated planar patch clamp system, SyncroPatch 384PE (Obergrussberger et al, Journal of Laboratory Automation 2016. 21 (6), 779-793). All cells were recorded in the whole cell mode of the patch clamp technique. The module is incorporated in a liquid handling pipetting robot system, Biomek FXP, for application of cells and compounds, vehicle control and positive control.

The different concentrations of the compounds were applied in two cumulatively increasing concentrations for the compunds (1 µM and 10 µM, and 3 µM and 30 µM, respectively). The hERG current was determined as the maximal tail current at -30 mV and percent inhibition upon compound or vehicle and positive control addition was reported.

After cells are caught onto the individual holes of the recording chips using the chip fill solution, the seal is increased with the seal enhancer solution (increased [Ca²⁺]; then the cells were washed twice with recording solution before using a pressure protocol to go into the whole cell mode.

After the whole cell mode was achieved, test pulses were given for ~10 minutes to quantify the hERG current in control conditions. During this control period vehicle control solution (recording solution containing 0.03% DMSO) was added three times into the individual wells. While continuing the pulse protocol, cumulatively increasing concentrations of the vehicle control, compound or positive control was added. The effect of the vehicle, compound and positive control was measured after 5 minutes of drug application. Two concentrations of the compound were tested per cell.

The use of the internal and recording solutions will result in ~10 mV liquid junction potential and the command voltage step will take this into account.

Electrophysiological measurements: The membrane current of the cells was measured at distinct membrane potentials with the patch clamp technique by means of an automated patch clamp system. The holding potential is -70 mV. The hERG current (K⁺-selective outward current) was determined as the maximal tail current at -30 mV after a 2 second depolarization to +70 mV (refs. 1, 4). Pulse cycling rate was 15 s.

### Data analysis

The leak corrected hERG current (K⁺-selective outward current) was determined as the maximal tail current at -30 mV after a 2-second of depolarization to +70 mV measured between 2336.3 ms and 3083.6 ms. The median of three current amplitudes was taken at the end of the control period and at the end of each addition of compound, vehicle and positive control to calculate the percent inhibition.

QC parameters were set in the SyncroPatch 384PE PatchControl384 software to automatically exclude wells from the analysis if values fall outside the range. The QC criteria are dependent on the type of recording plate (chip). Typically, a 4xChip (medium size hole) was used to record from hERG-transfected HEK293 cells. QC criteria 4-6 were set before the first addition of the compound; QC criteria 4 and 5 were also set at the end of each compound addition.

QC Criteria and acceptable ranges:
1. Board Check: -500pA - 500pA
2. Contact seal resistance: -100kOhm - 10MOhm
3. Junction potential offset: 0 - 100mV
4. Rseal ≥ 100 MOhm
5. Rseries: between 1 - 25 MOhm
6. hERG tail current ≥ 0.2 nA before compound addition

Each compound was replicated on the same plate in at least 5 wells. Percent inhibition of at least 2-3 replicates per concentration will be reported as median.

### Results:

### Protocol 1

| Compound Number | hERG- IC₅₀ (µM) |
|---|---|
| 17 | 4.1 |
| 40 | 5.0 |
| 83 | 6.0 |
| 41 | 6.3 |
| 39 | 7.6 |
| 45 | 12.6 |
| 14 | 20.0 |
| 22 | 20.9 |
| 13 | 25.7 |
| 94 | 30.9 |
| 1 | >30.2 |
| 4 | >30.2 |
| 37 | >30.2 |
| 30 | >30.2 |
| 19 | >30.2 |
| 12 | >30.2 |
| 18 | >30.2 |
| 11 | >30.2 |
| 34 | >30.2 |
| 49 | >30.2 |
| 47 | >30.2 |
| 35 | >30.2 |
| 27 | >30.2 |
| 32 | >30.2 |
| 23 | >30.2 |
| 10 | >30.2 |
| 71 | >30.2 |
| 258 | >30.2 |
| 75 | >30.2 |
| 252 | >30.2 |
| 396 | >30.2 |

### Protocol 2:

| Example Number | hERG IC₅₀ µM |
|---|---|
| 148 | 12.3 |
| 11 | >30.2 |
| 246 | >30.2 |
| 99 | >30.2 |
| 132 | >30.2 |
| 233 | >30.2 |
| 104 | >30.2 |
| 242 | >30.2 |
| 146 | >30.2 |
| 112 | >30.2 |
| 114 | >30.2 |
| 245 | >30.2 |
| 223 | >30.2 |
| 227 | >30.2 |

### 9) Efficacy study in disseminated OCI-AML3 model

### Test Agents and Controls

Compound 70 was formulated in 20% hydroxypropyl-beta-cyclodextrin (HP-β-CD) and prepared to reach a total volume of 0.2 mL (10 mL/kg) per dose for a 20 g animal. Doses were adjusted by individual body weight each day. Working stocks of Compound 70 were prepared once per week for each study and stored at 25°C.

### Animals

Female SCID beige mice (CB17.Cg-PrkdcscidLystbg-J/Crl/-) were used when they were approximately 6 to 8 weeks of age and weighed approximately 25 g. All animals could acclimate and recover from any shipping-related stress for a minimum of 7 days prior to experimental use. Autoclaved water and irradiated food were provided ad libitum, and the animals were maintained on a 12hour light and dark cycle. Cages, bedding, and water bottles were autoclaved before use and changed weekly.

| Tissue Culture and Cell Injection Reagents |
|---|
| DPBS (Dulbecco's phosphate-buffered saline) |
| Heat-inactivated fetal bovine serum |
| MEM Alpha medium |
| L-glutamine |
| Gentamycin |
| T175 Culture Flask |
| Roller Bottle |

### Tumor Model and Cell Culture Method

Human AML cell line OCI-AML3 was cultured at 37°C, 5% CO₂ in the indicated complete culture media (MEM Alpha + 20% HI-FBS (Heat-Inactivated Fetal Bovine Serum) + 2mM L-glutamine + 50ug/ml Gentamycin). Cells were harvested while in logarithmic growth and resuspended in cold (4°C) MEM ((Minimum Essential Medium) Alpha in serum-free medium. For the disseminated OCI-AML3 model, each mouse received 5x10⁵ cells via IV injection in a total volume of 0.2 mL using a 26-gauge needle.

### Study Designs

Compound 70 was administered orally (PO), daily.

Day 0 is the day of tumor cell implantation and study initiation

In the efficacy study, mice bearing IV OCI-AML3 xenograft tumors were randomly assigned to treatment groups 3 days post-tumor cell engraftment. Treatment with vehicle or Compound 70 (at 30, 50,100 mg/kg) was initiated on the same day, with daily dosing for 28 days.

### Animal Monitoring

Animals were monitored daily for clinical signs related to either compound toxicity or tumor burden (i.e., hind limb paralysis, lethargy, etc.).

### Calculations

For survival assessment, results were plotted as the percentage survival against days post tumor implant. Negative clinical signs and/or ≥20% body weight loss was used as a surrogate endpoint for death. Median survival was determined utilizing Kaplan-Meier survival analysis. The percent increased life span (ILS) was calculated as: ((median survival day of treated group - median survival day of control group) / median survival day of control group) × 100. Animals failing to reach the surrogate endpoint due to adverse clinical signs (such as ulcerated tumors, body weight loss, etc.) or death unrelated to treatment were censored for the survival assessment. As defined by NCI criteria, ≥25% ILS is considered biologically significant. (Johnson JI et al. Br J Cancer. 2001. 84(10), 1424-1431).

### Data Analysis

Survival and body weight data were graphically represented utilizing Prism (Version 7). Statistical significance for body weights was evaluated as described above. Statistical significance was evaluated for Kaplan-Meier survival plots comparing therapeutic treatment group vs. appropriate vehicle-treated control using log-rank (Mantel-Cox) test in R software version 3.4.2. Differences between groups were considered significant when the p value was ≤0.05.

### Survival

The Kaplan-Meier survival curve is shown in below figure. Mice bearing established OCI-AML3 tumors were orally dosed daily with Compound 70 at 30, 50, 100 mg/kg in 20% HP-β-CD formulation for a total of 28 days (n=9-10/group). For Compound 70 treated groups, the median days of survival were reached at the following days for 30mg/kg at day 75.5, for 50mg/kg at day 58.5 and for 100mg/kg at day 75 this compared to a median survival of 38.5 days for the vehicle-treated control group. Compound 70 treatment resulted in statistically significant increased lifespan of OCI-AML3 tumor-bearing mice by 96.1%, 51.9% and 94.8% (at the 30, 50 and 100 mg/kg dose levels) as compared to that of control mice, (p≤0.001). This was a biologically significant ILS as per NCI criteria threshold of ≥25% ILS (Johnson JI et al. Br J Cancer. 2001. 84(10), 1424-1431).

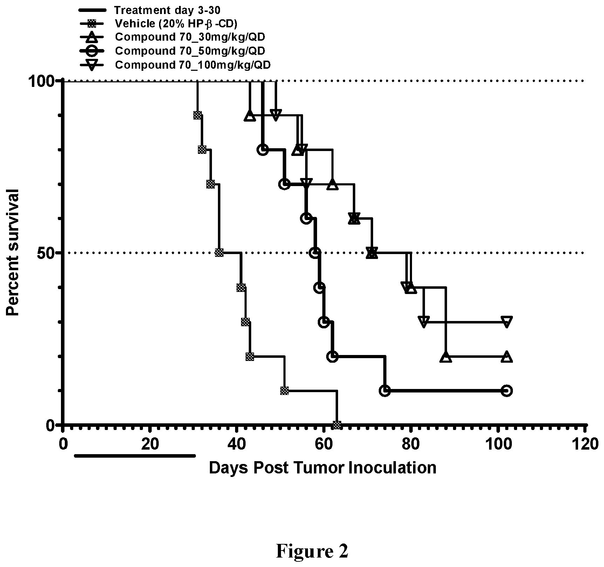

Results in Fig. 2.

## Claims

1. A compound wherein the compound is 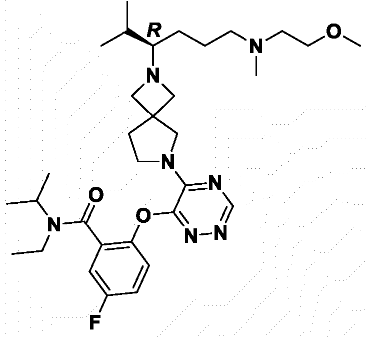 or a pharmaceutically acceptable salt or a solvate thereof.

2. The compound according to claim 1, wherein the compound is a pharmaceutically acceptable salt.

3. The compound according to claim 1, wherein the compound is a solvate.

4. The compound according to claim 1, wherein the compound is a solvate of a pharmaceutically acceptable salt.

5. The compound according to claim 1, wherein the compound is

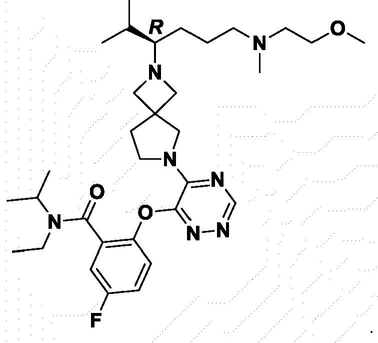

6. The compound according to claim 1, wherein the compound is

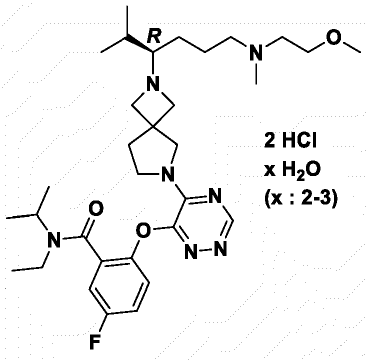

7. The compound according to claim 6, wherein the compound is

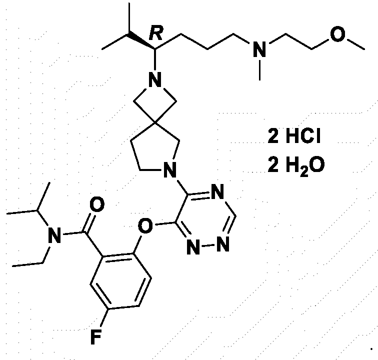

8. The compound according to claim 1, wherein the compound is

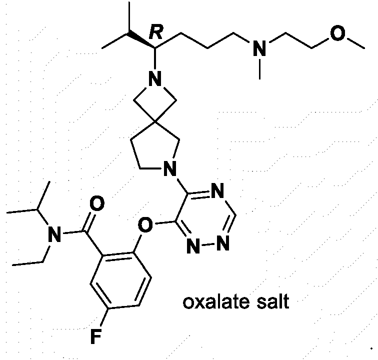

9. A pharmaceutical composition comprising a compound as claimed in any one of claims 1 to 8 and a pharmaceutically acceptable carrier or diluent.

10. A process for preparing a pharmaceutical composition as defined in claim 9 comprising mixing a pharmaceutically acceptable carrier with a therapeutically effective amount of a compound according to any one of claims 1 to 8.

11. A compound as claimed in any one of claims 1 to 8 or a pharmaceutical composition as claimed in claim 9 for use as a medicament.

12. A compound as claimed in any one of claims 1 to 8 or a pharmaceutical composition as claimed in claim 9 for use in the prevention or treatment of cancer.

13. A compound as claimed in any one of claims 1 to 8 or a pharmaceutical composition as claimed in claim 9 for use in the prevention or treatment of leukemia, myelodysplastic syndrome (MDS), or myeloproliferative neoplasms (MPN).

14. The compound or pharmaceutical composition for use according to claim 13, in the treatment of leukemia wherein the leukemia is (*NPM1*)-mutated leukemia.

15. The compound or pharmaceutical composition for use according to claim 13, in the prevention or treatment of leukemia wherein the leukemia is selected from acute leukemias, chronic leukemias, myeloid leukemias, myelogenous leukemias, lymphoblastic leukemias, and lymphocytic leukemias.

16. The compound or pharmaceutical composition for use according to claim 13, in the prevention or treatment of leukemia wherein the leukemia is selected from Acute myelogenous leukemias (AML), Chronic myelogenous leukemias (CML), Acute lymphoblastic leukemias (ALL), Chronic lymphocytic leukemias (CLL), T cell prolymphocytic leukemias (T-PLL), Large granular lymphocytic leukemia, Hairy cell leukemia (HCL), *KMT2A-*rearranged leukemias, *KMT2A-*PTD leukemias, *KMT2A* amplified leukemias, *KMT2A*-positive leukemias, and leukemias exhibiting *HOX*/*MEIS1* gene expression signatures.

17. The compound or pharmaceutical composition for use according to claim 13, in the treatment of leukemia wherein the leukemia is an acute leukemia.

18. The compound or pharmaceutical composition for use according to claim 17, wherein the acute leukemia is AML.

19. The compound or pharmaceutical composition for use according to claim 17, wherein the acute leukemia is ALL.

20. The compound or pharmaceutical composition for use according to any one of claims 17-19, wherein the acute leukemia has KMT2A gene alterations or *NPM1* mutations.

21. The compound or pharmaceutical composition for use according to any one of claims 17-19, wherein the acute leukemia has KMT2A gene rearrangements.

22. The compound or pharmaceutical composition for use according to any one of claims 17-19, wherein the acute leukemia has *NPM1* mutations.

23. The compound or pharmaceutical composition for use according to claim 22, wherein the *NPM1* mutation is *NPM1c.*

24. The compound or pharmaceutical composition for use according to claim 14, wherein the (*NPM1*)-mutated leukemia is *NPM1c.*

## Patentansprüche

1. Verbindung, wobei die Verbindung 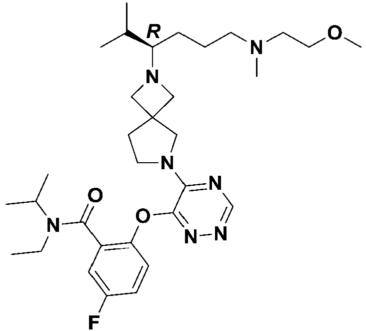 oder ein pharmazeutisch akzeptables Salz oder ein Solvat davon ist.

2. Verbindung nach Anspruch 1, wobei die Verbindung ein pharmazeutisch akzeptables Salz ist.

3. Verbindung nach Anspruch 1, wobei die Verbindung ein Solvat ist.

4. Verbindung nach Anspruch 1, wobei die Verbindung ein Solvat eines pharmazeutisch akzeptablen Salzes ist.

5. Verbindung nach Anspruch 1, wobei die Verbindung ist

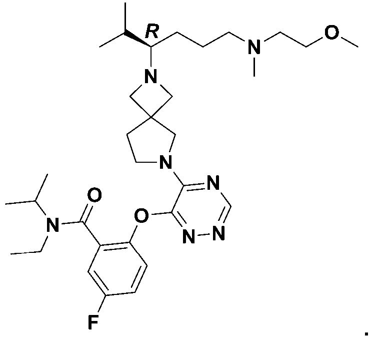

6. Verbindung nach Anspruch 1, wobei die Verbindung ist

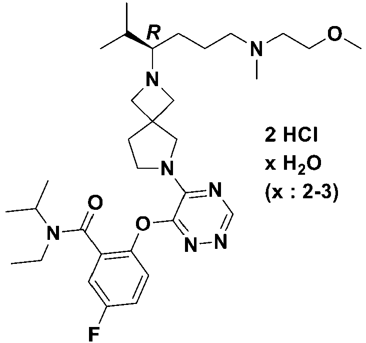

7. Verbindung nach Anspruch 6, wobei die Verbindung ist

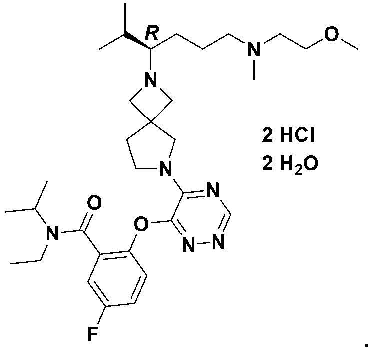

8. Verbindung nach Anspruch 1, wobei die Verbindung ist

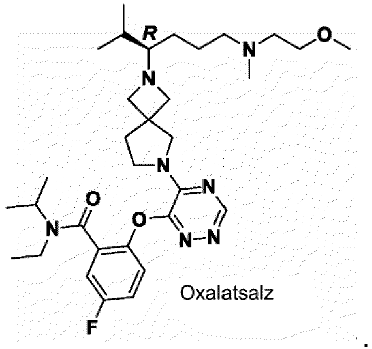

9. Pharmazeutische Zusammensetzung, umfassend eine Verbindung nach einem der Ansprüche 1 bis 8 und eine pharmazeutisch akzeptable Trägersubstanz oder ein pharmazeutisch akzeptables Verdünnungsmittel.

10. Verfahren zum Herstellen einer pharmazeutischen Zusammensetzung wie in Anspruch 9 definiert, umfassend ein Mischen einer pharmazeutisch akzeptablen Trägersubstanz mit einer therapeutisch wirksamen Menge einer Verbindung nach einem der Ansprüche 1 bis 8.

11. Verbindung nach einem der Ansprüche 1 bis 8 oder pharmazeutische Zusammensetzung nach Anspruch 9 zur Verwendung als ein Medikament.

12. Verbindung nach einem der Ansprüche 1 bis 8 oder pharmazeutische Zusammensetzung nach Anspruch 9 zur Verwendung bei einer Vorbeugung oder Behandlung von Krebs.

13. Verbindung nach einem der Ansprüche 1 bis 8 oder pharmazeutische Zusammensetzung nach Anspruch 9 zur Verwendung bei der Vorbeugung oder Behandlung von Leukämie, myelodysplastischem Syndrom (MDS) oder myeloproliferativen Neoplasien (MPN).

14. Verbindung oder pharmazeutische Zusammensetzung zur Verwendung nach Anspruch 13 bei der Behandlung von Leukämie, wobei die Leukämie eine (NPM1)-mutierte Leukämie ist.

15. Verbindung oder pharmazeutische Zusammensetzung zur Verwendung nach Anspruch 13 bei der Vorbeugung oder Behandlung von Leukämie, wobei die Leukämie aus akuten Leukämien, chronischen Leukämien, myeloiden Leukämien, myeloischen Leukämien, lymphoblastischen Leukämien und lymphozytischen Leukämien ausgewählt ist.

16. Verbindung oder pharmazeutische Zusammensetzung zur Verwendung nach Anspruch 13 bei der Vorbeugung oder Behandlung von Leukämie, wobei die Leukämie aus akuten myeloischen Leukämien (AML), chronischen myeloischen Leukämien (CML), akuten lymphoblastischen Leukämien (ALL), chronischen lymphozytischen Leukämien (CLL), prolymphozytischen T-Zell-Leukämien (T-PLL), Leukämie der großen granulären Lymphozyten, Haarzell-Leukämie (HCL), KMT2A-umgelagerten Leukämien, KMT2A-PTD-Leukämien, KMT2A-amplifizierten Leukämien, KMT2A-positiven Leukämien, und Leukämien, die *HOX*/*MEIS1-*Genexpressionssignaturen vorweisen, ausgewählt ist.

17. Verbindung oder pharmazeutische Zusammensetzung zur Verwendung nach Anspruch 13 bei der Behandlung von Leukämie, wobei die Leukämie eine akute Leukämie ist.

18. Verbindung oder pharmazeutische Zusammensetzung zur Verwendung nach Anspruch 17, wobei die akute Leukämie AML ist.

19. Verbindung oder pharmazeutische Zusammensetzung zur Verwendung nach Anspruch 17, wobei die akute Leukämie ALL ist.

20. Verbindung oder pharmazeutische Zusammensetzung zur Verwendung nach einem der Ansprüche 17 bis 19, wobei die akute Leukämie KMT2A-Genveränderungen oder NPM1-Mutationen aufweist.

21. Verbindung oder pharmazeutische Zusammensetzung zur Verwendung nach einem der Ansprüche 17 bis 19, wobei die akute Leukämie KMT2A-Genumlagerungen aufweist.

22. Verbindung oder pharmazeutische Zusammensetzung zur Verwendung nach einem der Ansprüche 17 bis 19, wobei die akute Leukämie *NPM1-*Mutationen aufweist.

23. Verbindung oder pharmazeutische Zusammensetzung zur Verwendung nach Anspruch 22, wobei die NPM1-Mutation *NPM1c* ist.

24. Verbindung oder pharmazeutische Zusammensetzung zur Verwendung nach Anspruch 14, wobei die (NPM1)-mutierte Leukämie *NPM1c* ist.

## Revendications

1. Composé dans lequel le composé est 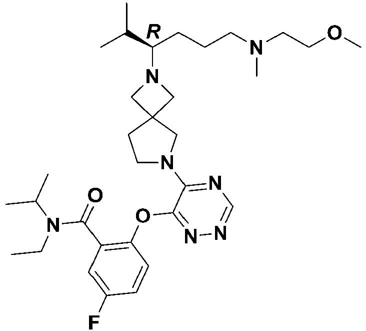 ou un sel ou un solvate pharmaceutiquement acceptable de celui-ci.

2. Composé selon la revendication 1, dans lequel le composé est un sel pharmaceutiquement acceptable.

3. Composé selon la revendication 1, dans lequel le composé est un solvate.

4. Composé selon la revendication 1, dans lequel le composé est un solvate d'un sel pharmaceutiquement acceptable.

5. Composé selon la revendication 1, dans lequel le composé est

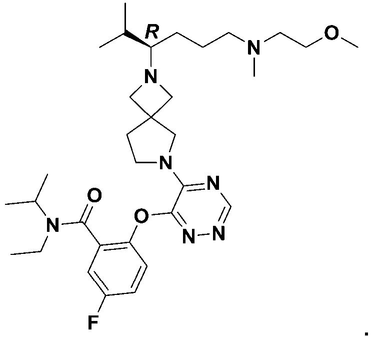

6. Composé selon la revendication 1, dans lequel le composé est

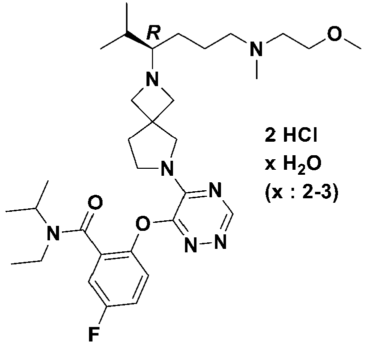

7. Composé selon la revendication 6, dans lequel le composé est

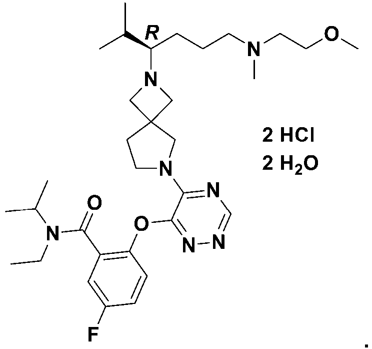

8. Composé selon la revendication 1, dans lequel le composé est

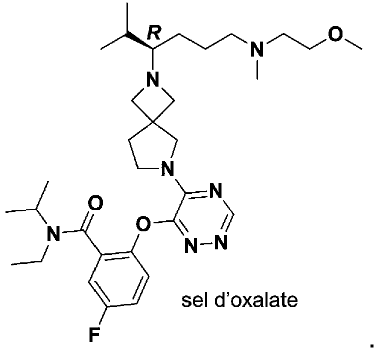

9. Composition pharmaceutique comprenant un composé selon l'une quelconque des revendications 1 à 8 et un support ou diluant pharmaceutiquement acceptable.

10. Procédé pour la préparation d'une composition pharmaceutique selon la revendication 9, comprenant le mélange d'un support pharmaceutiquement acceptable avec une quantité thérapeutiquement efficace d'un composé selon l'une quelconque des revendications 1 à 8.

11. Composé selon l'une quelconque des revendications 1 à 8 ou composition pharmaceutique selon la revendication 9 pour une utilisation comme médicament.

12. Composé selon l'une quelconque des revendications 1 à 8 ou composition pharmaceutique selon la revendication 9 pour une utilisation dans la prévention ou le traitement d'un cancer.

13. Composé selon l'une quelconque des revendications 1 à 8 ou composition pharmaceutique selon la revendication 9 pour une utilisation dans la prévention ou le traitement de la leucémie, d'un syndrome myélodysplasique (SMD) ou de néoplasmes myéloprolifératifs (NMP).

14. Composé ou composition pharmaceutique pour une utilisation selon la revendication 13, dans le traitement de la leucémie, dans lequel la leucémie est une leucémie avec mutation (*NPM1*).

15. Composé ou composition pharmaceutique pour une utilisation selon la revendication 13, dans la prévention ou le traitement de la leucémie, dans lequel la leucémie est choisie parmi leucémies aiguës, leucémies chroniques, leucémies myéloïdes, leucémies myélogènes, leucémies lymphoblastiques et leucémies lymphocytaires.

16. Composé ou composition pharmaceutique pour une utilisation selon la revendication 13, dans la prévention ou le traitement de la leucémie, dans lequel la leucémie est choisie parmi leucémies myélogènes aiguës (LMA), leucémies myélogènes chroniques (LMC), leucémies lymphoblastiques aiguës (LAL), leucémies lymphocytaires chroniques (LLC), leucémies prolymphocytaires à cellules T (T-PLL), leucémie à grands lymphocytes granuleux, leucémie à tricholeucocytes (HCL), leucémies avec réarrangement de *KMT2A,* leucémies KMT2A-PTD, les leucémies avec amplification de *KMT2A,* leucémies positives pour *KMT2A,* et leucémies présentant des signatures d'expression génique *HOXIMEIS1.*

17. Composé ou composition pharmaceutique pour une utilisation selon la revendication 13, dans le traitement d'une leucémie, dans lequel la leucémie est une leucémie aiguë.

18. Composé ou composition pharmaceutique pour une utilisation selon la revendication 17, dans lequel la leucémie aiguë est la LMA.

19. Composé ou composition pharmaceutique pour une utilisation selon la revendication 17, dans lequel la leucémie aiguë est une LAL.

20. Composé ou composition pharmaceutique pour une utilisation selon l'une quelconque des revendications 17 à 19, dans lequel la leucémie aiguë présente des altérations du gène KMT2A ou des mutations de *NPM1.*

21. Composé ou composition pharmaceutique pour une utilisation selon l'une quelconque des revendications 17 à 19, dans lequel la leucémie aiguë présente des réarrangements du gène KMT2A.

22. Composé ou composition pharmaceutique pour une utilisation selon l'une quelconque des revendications 17 à 19, dans lequel la leucémie aiguë présente des mutations de *NPM1.*

23. Composé ou composition pharmaceutique pour une utilisation selon la revendication 22, dans lequel la mutation *NPM1* est *NPM1c.*

24. Composé ou composition pharmaceutique pour une utilisation selon la revendication 14, dans lequel la leucémie avec une mutation *(NPM1)* est *NPM1c.*
